# EUROPEAN PATENT APPLICATION

(11) **EP 2 050 335 A1**
(43) Date of publication of application: **22.04.2009**
(21) Application number: 08020669.1
(22) Date of filing: 09.02.2007
(51) Int. Cl.: A01K 67/027, A61K 39/395, C07K 14/47, C07K 14/515, C07K 16/18, G01N 33/50

(54) **GENE DISRUPTIONS, COMPOSITIONS AND METHODS RELATING THERETO**

(30) Priority: 17.02.2006 US 774895 P
(62) Divisional of application: 07756828.5
(71) Applicant: Genentech, Inc., South San Francisco CA 94080-4990 (US); Lexicon Pharmaceuticals, Inc., The Woodlands, TX 77381 (US)
(72) Inventor: Bollinger, Kristi Rae, Tomball, Texas 77375 (US); Horner, Allison Anne Byers, Dickinson, Texas 77539 (US); Combs, Katherin E., Spring, TX 77380 (US); Culbertson, Ling Ling, Spring, TX 77386 (US); Cunningham, Jaime-jo, The Woodlands, TX 77382 (US); Desauvage, Frederic, Forster City, California 94404 (US); Edwards, Joel, The Woodlands, Texas 77382 (US); Girgis, Rosemary, Houston, Texas 77064 (US); Green, Leslie, Conroe, TX 77304 (US); McLain, Dina Rebecca, San Antonio, TX 77493 (US); Minze, Laurie Jeanette, Katy, TX 77493 (US); Montgomery, Charles A., Jay, Oklahoma 74346 (US); Payne, Bobby Joe, The Woodlands, TX 77381 (US); Phillips, Heidi, Palo Alto, California 94303 (US); Shi, Zheng-Zheng, The Woddlands, TX 77382 (US); Sparks, Mary Jean, Magnolia, TX 77354 (US); Stala, Joy, Dallas, TX 75204 (US); Tang, Tracy Tsu-Ling, Redwood City, CA 94065 (US); Townsend, Teresa Gail, Cypress, TX 77429 (US); Vogel, Peter, The Woodlands, TX 77381 (US); Willis-Sevaux, Tracy Ellen, Conroe, TX 77304 (US)
(74) Representative: Denison, Christopher Marcus

(57) **Abstract**

The present invention relates to transgenic animals, as wells as compositions and methods relating to the characterization of gene function. Specifically, the present invention provides transgenic mice comprising disruptions in PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PR021184, PRO7434, PRO9822, PRO8933, PRO9836, PRO9854, PRO9863, PRO10284, PRO37510, PRO35444, PRO20473, PRO21054 or PRO35246 genes. Such in vivo studies and characterizations may provide valuable identification and discovery of therapeutics and/or treatments useful in the prevention, amelioration, or correction of diseases or dysfunctions associated with gene disruptions associated with gene disruptions such as neurological disorders; cardiovascular, endothelial or angiogenic disorders; eye abnormalities; immunological disorders; oncological disorders; bone metabolic abnormalities or disorders; lipid metabolic disorder; or developmental abnormalities.

Sequence Listing

<110> Genentech, Inc. et al.

<120> NOVEL GENE DISRUPTIONS, COMPOSITIONS AND METHODS
      RELATING THERETO

<130> CMD/FP6579783

<150> EP 07756828.5
<151> 2007-02-09

<150> PCT/US2007/061927
<151> 2007-02-09

<150> US 60/774,895
<151> 2006-02-17

<160> 174

<210> 1
<211> 3355
<212> DNA
<213> Homo sapiens

<400> 1
gcagctggtt actgcatttc tccatgtggc agacagagca aagccacaac 50

gctttctctg ctggattaaa gacggcccac agaccagaac ttccactata 100

ctacttaaaa ttacataggt ggcttgtcaa attcaattga ttagtattgt 150

aaaaggaaaa agaagttcct tcttacagct tggattcaac ggtccaaaac 200

aaaaatgcag ctgccattaa agtctcagat gaacaaactt ctacactgat 250

ttttaaaatc aagaataagg gcagcaagtt tctggattca ctgaatcaac 300

agacacaaaa agctggcaat atagcaacta tgaagagaaa agctactaat 350

aaaattaacc caacgcatag aagacttttt tttctcttct aaaaacaact 400

aagtaaagac ttaaatttaa acacatcatt ttacaacctc atttcaaaat 450

gaagactttt acctggaccc taggtgtgct attcttccta ctagtggaca 500

ctggacattg cagaggtgga caattcaaaa ttaaaaaaat aaaccagaga 550

agataccctc gtgccacaga tggtaaagag gaagcaaaga aatgtgcata 600

cacattcctg gtacctgaac aaagaataac agggccaatc tgtgtcaaca 650

ccaaggggca agatgcaagt accattaaag acatgatcac caggatggac 700

cttgaaaacc tgaaggatgt gctctccagg cagaagcggg agatagatgt 750

tctgcaactg gtggtggatg tagatggaaa cattgtgaat gaggtaaagc 800

tgctgagaaa ggaaagccgt aacatgaact ctcgtgttac tcaactctat 850

atgcaattat tacatgagat tatccgtaag agggataatt cacttgaact 900

ttcccaactg gaaaacaaaa tcctcaatgt caccacagaa atgttgaaga 950

tggcaacaag atacagggaa ctagaggtga aatacgcttc cttgactgat 1000

cttgtcaata accaatctgt gatgatcact ttgttggaag aacagtgctt 1050

gaggatattt tcccgacaag acacccatgt gtctccccca cttgtccagg 1100

tggtgccaca acatattcct aacagccaac agtatactcc tggtctgctg 1150

ggaggtaacg agattcagag ggatccaggt tatcccagag atttaatgcc 1200

accacctgat ctggcaactt ctcccaccaa aagccctttc aagataccac 1250

cggtaacttt catcaatgaa ggaccattca aagactgtca gcaagcaaaa 1300

gaagctgggc attcggtcag tgggatttat atgattaaac ctgaaaacag 1350

caatggacca atgcagttat ggtgtgaaaa cagtttggac cctgggggtt 1400

ggactgttat tcagaaaaga acagacggct ctgtcaactt cttcagaaat 1450

tgggaaaatt ataagaaagg gtttggaaac attgacggag aatactggct 1500

tggactggaa aatatctata tgcttagcaa tcaagataat tacaagttat 1550

tgattgaatt agaagactgg agtgataaaa aagtctatgc agaatacagc 1600

agctttcgtc tggaacctga aagtgaattc tatagactgc gcctgggaac 1650

ttaccaggga aatgcagggg attctatgat gtggcataat ggtaaacaat 1700

tcaccacact ggacagagat aaagatatgt atgcaggaaa ctgcgcccac 1750

tttcataaag gaggctggtg gtacaatgcc tgtgcacatt ctaacctaaa 1800

tggagtatgg tacagaggag gccattacag aagcaagcac caagatggaa 1850

ttttctgggc cgaatacaga ggcgggtcat actccttaag agcagttcag 1900

atgatgatca agcctattga ctgaagagag acactcgcca atttaaatga 1950

cacagaactt tgtacttttc agctcttaaa aatgtaaatg ttacatgtat 2000

attacttggc acaatttatt tctacacaga aagtttttaa aatgaatttt 2050

accgtaacta taaaagggaa cctataaatg tagtttcatc tgtcgtcaat 2100

tactgcagaa aattatgtgt atccacaacc tagttatttt aaaaattatg 2150

ttgactaaat acaaagtttg ttttctaaaa tgtaaatatt tgccacaatg 2200

taaagcaaat cttagctata ttttaaatca taaataacat gttcaagata 2250

cttaacaatt tatttaaaat ctaagattgc tctaacgtct agtgaaaaaa 2300

atatttttta aatttcagcc aaataatgca ttttatttta taaaaataca 2350

gacagaaaat tagggagaaa cttctagttt tgccaataga aaatgttctt 2400

ccattgaata aaagttattt caaattgaat ttgtgccttt cacacgtaat 2450

gattaaatct gaattcttaa taatatatcc tatgctgatt ttcccaaaac 2500

atgacccata gtattaaata catatcattt ttaaaaataa aaaaaaaccc 2550

aaaaataatg catgcataat ttaaatggtc aatttataaa gacaaatcta 2600

tgaatgaatt tttcagtgtt atcttcatat gatatgctga acaccaaaat 2650

ctccagaaat gcattttatg tagttctaaa atcagcaaaa tattggtatt 2700

acaaaaatgc agaatattta gtgtgctaca gatctgaatt atagttctaa 2750

tttattatta ctttttttct aatttactga tcttactact acaaagaaaa 2800

aaaaacccaa cccatctgca attcaaatca gaaagtttgg acagctttac 2850

aagtattagt gcatgctcag aacaggtggg actaaaacaa actcaaggaa 2900

ctgttggctg ttttcccgat actgagaatt caacagctcc agagcagaag 2950

ccacaggggc atagcttagt ccaaactgct aatttcattt tacagtgtat 3000

gtaacgctta gtctcacagt gtctttaact catctttgca atcaacaact 3050

ttactagtga ctttctggaa caatttcctt tcaggaatac atattcactg 3100

cttagaggtg accttgcctt aatatatttg tgaagttaaa attttaaaga 3150

tagctcatga aacttttgct taagcaaaaa gaaaacctcg aattgaaatg 3200

tgtgaggcaa actatgcatg ggaatagctt aatgtgaaga taatcatttg 3250

gacaactcaa atccatcaac atgaccaatg tttttcatct gccacatctc 3300

aaaataaaac ttctggtgaa acaaattaaa caaaatatcc aaacctcaaa 3350

aaaaa 3355

<210> 2
<211> 491
<212> PRT
<213> Homo sapiens

<400> 2
Met Lys Thr Phe Thr Trp Thr Leu Gly Val Leu Phe Phe Leu Leu
1               5                   10                  15

Val Asp Thr Gly His Cys Arg Gly Gly Gln Phe Lys Ile Lys Lys
                20                  25                  30

Ile Asn Gln Arg Arg Tyr Pro Arg Ala Thr Asp Gly Lys Glu Glu
                35                  40                  45

Ala Lys Lys Cys Ala Tyr Thr Phe Leu Val Pro Glu Gln Arg Ile
                50                  55                  60

Thr Gly Pro Ile Cys Val Asn Thr Lys Gly Gln Asp Ala Ser Thr
                65                  70                  75

Ile Lys Asp Met Ile Thr Arg Met Asp Leu Glu Asn Leu Lys Asp
                80                  85                  90

Val Leu Ser Arg Gln Lys Arg Glu Ile Asp Val Leu Gln Leu Val
                95                  100                 105

Val Asp Val Asp Gly Asn Ile Val Asn Glu Val Lys Leu Leu Arg
                110                 115                 120

Lys Glu Ser Arg Asn Met Asn Ser Arg Val Thr Gln Leu Tyr Met
                125                 130                 135

Gln Leu Leu His Glu Ile Ile Arg Lys Arg Asp Asn Ser Leu Glu
                140                 145                 150

Leu Ser Gln Leu Glu Asn Lys Ile Leu Asn Val Thr Thr Glu Met
                155                 160                 165

Leu Lys Met Ala Thr Arg Tyr Arg Glu Leu Glu Val Lys Tyr Ala
                170                 175                 180

Ser Leu Thr Asp Leu Val Asn Asn Gln Ser Val Met Ile Thr Leu
                185                 190                 195

Leu Glu Glu Gln Cys Leu Arg Ile Phe Ser Arg Gln Asp Thr His
                200                 205                 210

Val Ser Pro Pro Leu Val Gln Val Val Pro Gln His Ile Pro Asn
                215                 220                 225

Ser Gln Gln Tyr Thr Pro Gly Leu Leu Gly Gly Asn Glu Ile Gln
                230                 235                 240

Arg Asp Pro Gly Tyr Pro Arg Asp Leu Met Pro Pro Pro Asp Leu
                245                 250                 255

Ala Thr Ser Pro Thr Lys Ser Pro Phe Lys Ile Pro Pro Val Thr
                260                 265                 270

Phe Ile Asn Glu Gly Pro Phe Lys Asp Cys Gln Gln Ala Lys Glu
                275                 280                 285

Ala Gly His Ser Val Ser Gly Ile Tyr Met Ile Lys Pro Glu Asn
                290                 295                 300

Ser Asn Gly Pro Met Gln Leu Trp Cys Glu Asn Ser Leu Asp Pro
                305                 310                 315

Gly Gly Trp Thr Val Ile Gln Lys Arg Thr Asp Gly Ser Val Asn

320                 325                 330

Phe Phe Arg Asn Trp Glu Asn Tyr Lys Lys Gly Phe Gly Asn Ile
                335                 340                 345

Asp Gly Glu Tyr Trp Leu Gly Leu Glu Asn Ile Tyr Met Leu Ser
                350                 355                 360

Asn Gln Asp Asn Tyr Lys Leu Leu Ile Glu Leu Glu Asp Trp Ser
                365                 370                 375

Asp Lys Lys Val Tyr Ala Glu Tyr Ser Ser Phe Arg Leu Glu Pro
                380                 385                 390

Glu Ser Glu Phe Tyr Arg Leu Arg Leu Gly Thr Tyr Gln Gly Asn
                395                 400                 405

Ala Gly Asp Ser Met Met Trp His Asn Gly Lys Gln Phe Thr Thr
                410                 415                 420

Leu Asp Arg Asp Lys Asp Met Tyr Ala Gly Asn Cys Ala His Phe
                425                 430                 435

His Lys Gly Gly Trp Trp Tyr Asn Ala Cys Ala His Ser Asn Leu
                440                 445                 450

Asn Gly Val Trp Tyr Arg Gly Gly His Tyr Arg Ser Lys His Gln
                455                 460                 465

Asp Gly Ile Phe Trp Ala Glu Tyr Arg Gly Gly Ser Tyr Ser Leu
                470                 475                 480

Arg Ala Val Gln Met Met Ile Lys Pro Ile Asp
                485                 490

<210> 3
<211> 2477
<212> DNA
<213> Homo sapiens

<400> 3
cgagggcttt tccggctccg gaatggcaca tgtgggaatc ccagtcttgt 50

tggctacaac atttttccct ttcctaacaa gttctaacag ctgttctaac 100

agctagtgat caggggttct tcttgctgga gaagaaaggg ctgagggcag 150

agcagggcac tctcactcag ggtgaccagc tccttgcctc tctgtggata 200

acagagcatg agaaagtgaa gagatgcagc ggagtgaggt gatggaagtc 250

taaaatagga aggaattttg tgtgcaatat cagactctgg gagcagttga 300

cctggagagc ctgggggagg gcctgcctaa caagctttca aaaaacagga 350

gcgacttcca ctgggctggg ataagacgtg ccggtaggat agggaagact 400

gggtttagtc ctaatatcaa attgactggc tgggtgaact tcaacagcct 450

tttaacctct ctgggagatg aaaacgatgg cttaaggggc cagaaataga 500

gatgctttgt aaaataaaat tttaaaaaaa gcaagtattt tatagcataa 550

aggctagaga ccaaaataga taacaggatt ccctgaacat tcctaagagg 600

gagaaagtat gttaaaaata gaaaaaccaa aatgcagaag gaggagactc 650

acagagctaa accaggatgg ggaccctggg tcaggccagc ctctttgctc 700

ctcccggaaa ttatttttgg tctgaccact ctgccttgtg ttttgcagaa 750

tcatgtgagg gccaaccggg gaaggtggag cagatgagca cacacaggag 800

ccgtctcctc accgccgccc ctctcagcat ggaacagagg cagccctggc 850

cccgggccct ggaggtggac agccgctctg tggtcctgct ctcagtggtc 900

tgggtgctgc tggccccccc agcagccggc atgcctcagt tcagcacctt 950

ccactctgag aatcgtgact ggaccttcaa ccacttgacc gtccaccaag 1000

ggacgggggc cgtctatgtg ggggccatca accgggtcta taagctgaca 1050

ggcaacctga ccatccaggt ggctcataag acagggccag aagaggacaa 1100

caagtctcgt tacccgcccc tcatcgtgca gccctgcagc gaagtgctca 1150

ccctcaccaa caatgtcaac aagctgctca tcattgacta ctctgagaac 1200

cgcctgctgg cctgtgggag cctctaccag ggggtctgca agctgctgcg 1250

gctggatgac ctcttcatcc tggtggagcc atcccacaag aaggagcact 1300

acctgtccag tgtcaacaag acgggcacca tgtacggggt gattgtgcgc 1350

tctgagggtg aggatggcaa gctcttcatc ggcacggctg tggatgggaa 1400

gcaggattac ttcccgaccc tgtccagccg gaagctgccc cgagaccctg 1450

agtcctcagc catgctcgac tatgagctac acagcgattt tgtctcctct 1500

ctcatcaaga tcccttcaga caccctggcc ctggtctccc actttgacat 1550

cttctacatc tacggctttg ctagtggggg ctttgtctac tttctcactg 1600

tccagcccga gacccctgag ggtgtggcca tcaactccgc tggagacctc 1650

ttctacacct cacgcatcgt gcggctctgc aaggatgacc ccaagttcca 1700

ctcatacgtg tccctgccct tcggctgcac ccgggccggg gtggaatacc 1750

gcctcctgca ggctgcttac ctggccaagc ctggggactc actggcccag 1800

gccttcaata tcaccagcca ggacgatgta ctctttgcca tcttctccaa 1850

agggcagaag cagtatcacc acccgcccga tgactctgcc ctgtgtgcct 1900

tccctatccg ggccatcaac ttgcagatca aggagcgcct gcagtcctgc 1950

taccagggcg agggcaacct ggagctcaac tggctgctgg ggaaggacgt 2000

ccagtgcacg aaggcgcctg tccccatcga tgataacttc tgtggactgg 2050

acatcaacca gcccctggga ggctcaactc cagtggaggg cctgaccctg 2100

tacaccacca gcaggaccgc catgacctct gtggcctcct acgtttacaa 2150

cggctacagc gtggtttttg tggggactaa gagtggcaag ctgaaaaagg 2200

taagagtcta tgagttcaga tgctccaatg ccattcacct cctcagcaaa 2250

gagtccctct tggaaggtag ctattggtgg agatttaact ataggcaact 2300

ttattttctt ggggaacaaa ggtgaaatgg ggaggtaaga aggggttaat 2350

tttgtgactt agcttctagc tacttcctcc agccatcagt cattgggtat 2400

gtaaggaatg caagcgtatt tcaatatttc ccaaacttta agaaaaaact 2450

ttaagaaggt acatctgcaa  aagcaaa 2477

<210> 4
<211> 552
<212> PRT
<213> Homo sapiens

<400> 4

<210> 5
<211> 2755
<212> DNA
<213> Homo sapiens

<400> 5
gggggttagg gaggaaggaa tccaccccca cccccccaaa cccttttctt 50

ctcctttcct ggcttcggac attggagcac taaatgaact tgaattgtgt 100

ctgtggcgag caggatggtc gctgttactt tgtgatgaga tcggggatga 150

attgctcgct ttaaaaatgc tgctttggat tctgttgctg gagacgtctc 200

tttgttttgc cgctggaaac gttacagggg acgtttgcaa agagaagatc 250

tgttcctgca atgagataga aggggaccta cacgtagact gtgaaaaaaa 300

gggcttcaca agtctgcagc gtttcactgc cccgacttcc cagttttacc 350

atttatttct gcatggcaat tccctcactc gacttttccc taatgagttc 400

gctaactttt ataatgcggt tagtttgcac atggaaaaca atggcttgca 450

tgaaatcgtt ccgggggctt ttctggggct gcagctggtg aaaaggctgc 500

acatcaacaa caacaagatc aagtcttttc gaaagcagac ttttctgggg 550

ctggacgatc tggaatatct ccaggctgat tttaatttat tacgagatat 600

agacccgggg gccttccagg acttgaacaa gctggaggtg ctcattttaa 650

atgacaatct catcagcacc ctacctgcca acgtgttcca gtatgtgccc 700

atcacccacc tcgacctccg gggtaacagg ctgaaaacgc tgccctatga 750

ggaggtcttg gagcaaatcc ctggtattgc ggagatcctg ctagaggata 800

acccttggga ctgcacctgt gatctgctct ccctgaaaga atggctggaa 850

aacattccca agaatgccct gatcggccga gtggtctgcg aagcccccac 900

cagactgcag ggtaaagacc tcaatgaaac caccgaacag gacttgtgtc 950

ctttgaaaaa ccgagtggat tctagtctcc cggcgccccc tgcccaagaa 1000

gagacctttg ctcctggacc cctgccaact cctttcaaga caaatgggca 1050

agaggatcat gccacaccag ggtctgctcc aaacggaggt acaaagatcc 1100

caggcaactg gcagatcaaa atcagaccca cagcagcgat agcgacgggt 1150

agctccagga acaaaccctt agctaacagt ttaccctgcc ctgggggctg 1200

cagctgcgac cacatcccag ggtcgggttt aaagatgaac tgcaacaaca 1250

ggaacgtgag cagcttggct gatttgaagc ccaagctctc taacgtgcag 1300

gagcttttcc tacgagataa caagatccac agcatccgaa aatcgcactt 1350

tgtggattac aagaacctca ttctgttgga tctgggcaac aataacatcg 1400

ctactgtaga gaacaacact ttcaagaacc ttttggacct caggtggcta 1450

tacatggata gcaattacct ggacacgctg tcccgggaga aattcgcggg 1500

gctgcaaaac ctagagtacc tgaacgtgga gtacaacgct atccagctca 1550

tcctcccggg cactttcaat gccatgccca aactgaggat cctcattctc 1600

aacaacaacc tgctgaggtc cctgcctgtg gacgtgttcg ctggggtctc 1650

gctctctaaa ctcagcctgc acaacaatta cttcatgtac ctcccggtgg 1700

caggggtgct ggaccagtta acctccatca tccagataga cctccacgga 1750

aacccctggg agtgctcctg cacaattgtg cctttcaagc agtgggcaga 1800

acgcttgggt tccgaagtgc tgatgagcga cctcaagtgt gagacgccgg 1850

tgaacttctt tagaaaggat ttcatgctcc tctccaatga cgagatctgc 1900

cctcagctgt acgctaggat ctcgcccacg ttaacttcgc acagtaaaaa 1950

cagcactggg ttggcggaga ccgggacgca ctccaactcc tacctagaca 2000

ccagcagggt gtccatctcg gtgttggtcc cgggactgct gctggtgttt 2050

gtcacctccg ccttcaccgt ggtgggcatg ctcgtgttta tcctgaggaa 2100

ccgaaagcgg tccaagagac gagatgccaa ctcctccgcg tccgagatta 2150

attccctaca gacagtctgt gactcttcct actggcacaa tgggccttac 2200

aacgcagatg gggcccacag agtgtatgac tgtggctctc actcgctctc 2250

agactaagac cccaacccca ataggggagg gcagagggaa ggcgatacat 2300

ccttccccac cgcaggcacc ccgggggctg gaggggcgtg tacccaaatc 2350

cccgcgccat cagcctggat gggcataagt agataaataa ctgtgagctc 2400

gcacaaccga aagggcctga ccccttactt agctccctcc ttgaaacaaa 2450

gagcagactg tggagagctg ggagagcgca gccagctcgc tctttgctga 2500

gagccccttt tgacagaaag cccagcacga ccctgctgga agaactgaca 2550

gtgccctcgc cctcggcccc ggggcctgtg gggttggatg ccgcggttct 2600

atacatatat acatatatcc acatctatat agagagatag atatctattt 2650

ttcccctgtg gattagcccc gtgatggctc cctgttggct acgcagggat 2700

gggcagttgc acgaaggcat gaatgtattg taaataagta actttgactt 2750

ctgac 2755

<210> 6
<211> 696
<212> PRT
<213> Homo sapiens

<400> 6
Met Leu Leu Trp Ile Leu Leu Leu Glu Thr Ser Leu Cys Phe Ala
1               5                   10                  15

Ala Gly Asn Val Thr Gly Asp Val Cys Lys Glu Lys Ile Cys Ser
                20                  25                  30

Cys Asn Glu Ile Glu Gly Asp Leu His Val Asp Cys Glu Lys Lys
                35                  40                  45

Gly Phe Thr Ser Leu Gln Arg Phe Thr Ala Pro Thr Ser Gln Phe
                50                  55                  60

Tyr His Leu Phe Leu His Gly Asn Ser Leu Thr Arg Leu Phe Pro
                65                  70                  75

Asn Glu Phe Ala Asn Phe Tyr Asn Ala Val Ser Leu His Met Glu
                80                  85                  90

Asn Asn Gly Leu His Glu Ile Val Pro Gly Ala Phe Leu Gly Leu
                95                  100                 105

Gln Leu Val Lys Arg Leu His Ile Asn Asn Asn Lys Ile Lys Ser
                110                 115                 120

Phe Arg Lys Gln Thr Phe Leu Gly Leu Asp Asp Leu Glu Tyr Leu
                125                 130                 135

Gln Ala Asp Phe Asn Leu Leu Arg Asp Ile Asp Pro Gly Ala Phe
                140                 145                 150

Gln Asp Leu Asn Lys Leu Glu Val Leu Ile Leu Asn Asp Asn Leu
                155                 160                 165

Ile Ser Thr Leu Pro Ala Asn Val Phe Gln Tyr Val Pro Ile Thr
                170                 175                 180

His Leu Asp Leu Arg Gly Asn Arg Leu Lys Thr Leu Pro Tyr Glu
                185                 190                 195

Glu Val Leu Glu Gln Ile Pro Gly Ile Ala Glu Ile Leu Leu Glu
                200                 205                 210

Asp Asn Pro Trp Asp Cys Thr Cys Asp Leu Leu Ser Leu Lys Glu
                215                 220                 225

Trp Leu Glu Asn Ile Pro Lys Asn Ala Leu Ile Gly Arg Val Val
                230                 235                 240

Cys Glu Ala Pro Thr Arg Leu Gln Gly Lys Asp Leu Asn Glu Thr
                245                 250                 255

Thr Glu Gln Asp Leu Cys Pro Leu Lys Asn Arg Val Asp Ser Ser
                260                 265                 270

Leu Pro Ala Pro Pro Ala Gln Glu Glu Thr Phe Ala Pro Gly Pro
                275                 280                 285

Leu Pro Thr Pro Phe Lys Thr Asn Gly Gln Glu Asp His Ala Thr
                290                 295                 300

Pro Gly Ser Ala Pro Asn Gly Gly Thr Lys Ile Pro Gly Asn Trp
                305                 310                 315

Gln Ile Lys Ile Arg Pro Thr Ala Ala Ile Ala Thr Gly Ser Ser
                320                 325                 330

Arg Asn Lys Pro Leu Ala Asn Ser Leu Pro Cys Pro Gly Gly Cys
                335                 340                 345

Ser Cys Asp His Ile Pro Gly Ser Gly Leu Lys Met Asn Cys Asn
                350                 355                 360

Asn Arg Asn Val Ser Ser Leu Ala Asp Leu Lys Pro Lys Leu Ser
                365                 370                 375

Asn Val Gln Glu Leu Phe Leu Arg Asp Asn Lys Ile His Ser Ile
                380                 385                 390

Arg Lys Ser His Phe Val Asp Tyr Lys Asn Leu Ile Leu Leu Asp
                395                 400                 405

Leu Gly Asn Asn Asn Ile Ala Thr Val Glu Asn Asn Thr Phe Lys

                410                 415                 420

Asn Leu Leu Asp Leu Arg Trp Leu Tyr Met Asp Ser Asn Tyr Leu
                425                 430                 435

Asp Thr Leu Ser Arg Glu Lys Phe Ala Gly Leu Gln Asn Leu Glu
                440                 445                 450

Tyr Leu Asn Val Glu Tyr Asn Ala Ile Gln Leu Ile Leu Pro Gly
                455                 460                 465

Thr Phe Asn Ala Met Pro Lys Leu Arg Ile Leu Ile Leu Asn Asn
                470                 475                 480

Asn Leu Leu Arg Ser Leu Pro Val Asp Val Phe Ala Gly Val Ser
                485                 490                 495

Leu Ser Lys Leu Ser Leu His Asn Asn Tyr Phe Met Tyr Leu Pro
                500                 505                 510

Val Ala Gly Val Leu Asp Gln Leu Thr Ser Ile Ile Gln Ile Asp
                515                 520                 525

Leu His Gly Asn Pro Trp Glu Cys Ser Cys Thr Ile Val Pro Phe
                530                 535                 540

Lys Gln Trp Ala Glu Arg Leu Gly Ser Glu Val Leu Met Ser Asp
                545                 550                 555

Leu Lys Cys Glu Thr Pro Val Asn Phe Phe Arg Lys Asp Phe Met
                560                 565                 570

Leu Leu Ser Asn Asp Glu Ile Cys Pro Gln Leu Tyr Ala Arg Ile
                575                 580                 585

Ser Pro Thr Leu Thr Ser His Ser Lys Asn Ser Thr Gly Leu Ala
                590                 595                 600

Glu Thr Gly Thr His Ser Asn Ser Tyr Leu Asp Thr Ser Arg Val
                605                 610                 615

Ser Ile Ser Val Leu Val Pro Gly Leu Leu Leu Val Phe Val Thr
                620                 625                 630

Ser Ala Phe Thr Val Val Gly Met Leu Val Phe Ile Leu Arg Asn
                635                 640                 645

Arg Lys Arg Ser Lys Arg Arg Asp Ala Asn Ser Ser Ala Ser Glu
                650                 655                 660

Ile Asn Ser Leu Gln Thr Val Cys Asp Ser Ser Tyr Trp His Asn
                665                 670                 675

Gly Pro Tyr Asn Ala Asp Gly Ala His Arg Val Tyr Asp Cys Gly
                680                 685                 690

Ser His Ser Leu Ser Asp
                695

<210> 7
<211> 1679
<212> DNA
<213> Homo sapiens

<400> 7
gttgtgtcct tcagcaaaac agtggattta aatctccttg cacaagcttg 50

agagcaacac aatctatcag gaaagaaaga aagaaaaaaa ccgaacctga 100

caaaaaagaa gaaaaagaag aagaaaaaaa atcatgaaaa ccatccagcc 150

aaaaatgcac aattctatct cttgggcaat cttcacgggg ctggctgctc 200

tgtgtctctt ccaaggagtg cccgtgcgca gcggagatgc caccttcccc 250

aaagctatgg acaacgtgac ggtccggcag ggggagagcg ccaccctcag 300

gtgcactatt gacaaccggg tcacccgggt ggcctggcta aaccgcagca 350

ccatcctcta tgctgggaat gacaagtggt gcctggatcc tcgcgtggtc 400

cttctgagca acacccaaac gcagtacagc atcgagatcc agaacgtgga 450

tgtgtatgac gagggccctt acacctgctc ggtgcagaca gacaaccacc 500

caaagacctc tagggtccac ctcattgtgc aagtatctcc caaaattgta 550

gagatttctt cagatatctc cattaatgaa gggaacaata ttagcctcac 600

ctgcatagca actggtagac cagagcctac ggttacttgg agacacatct 650

ctcccaaagc ggttggcttt gtgagtgaag acgaatactt ggaaattcag 700

ggcatcaccc gggagcagtc aggggactac gagtgcagtg cctccaatga 750

cgtggccgcg cccgtggtac ggagagtaaa ggtcaccgtg aactatccac 800

catacatttc agaagccaag ggtacaggtg tccccgtggg acaaaagggg 850

acactgcagt gtgaagcctc agcagtcccc tcagcagaat tccagtggta 900

caaggatgac aaaagactga ttgaaggaaa gaaaggggtg aaagtggaaa 950

acagaccttt cctctcaaaa ctcatcttct tcaatgtctc tgaacatgac 1000

tatgggaact acacttgcgt ggcctccaac aagctgggcc acaccaatgc 1050

cagcatcatg ctatttggtc caggcgccgt cagcgaggtg agcaacggca 1100

cgtcgaggag ggcaggctgc gtctggctgc tgcctcttct ggtcttgcac 1150

ctgcttctca aattttgatg tgagtgccac ttccccaccc gggaaaggct 1200

gccgccacca ccaccaccaa cacaacagca atggcaacac cgacagcaac 1250

caatcagata tatacaaatg aaattagaag aaacacagcc tcatgggaca 1300

gaaatttgag ggaggggaac aaagaatact ttggggggaa aagagtttta 1350

aaaaagaaat tgaaaattgc cttgcagata tttaggtaca atggagtttt 1400

cttttcccaa acgggaagaa cacagcacac ccggcttgga cccactgcaa 1450

gctgcatcgt gcaacctctt tggtgccagt gtgggcaagg gctcagcctc 1500

tctgcccaca gagtgccccc acgtggaaca ttctggagct ggccatccca 1550

aattcaatca gtccatagag acgaacagaa tgagaccttc cggcccaagc 1600

gtggcgctgc gggcactttg gtagactgtg ccaccacggc gtgtgttgtg 1650

aaacgtgaaa taaaaagagc  aaaaaaaaa 1679

<210> 8
<211> 344
<212> PRT
<213> Homo sapiens

<400> 8
Met Lys Thr Ile Gln Pro Lys Met His Asn Ser Ile Ser Trp Ala
1               5                   10                  15

Ile Phe Thr Gly Leu Ala Ala Leu Cys Leu Phe Gln Gly Val Pro
                20                  25                  30

Val Arg Ser Gly Asp Ala Thr Phe Pro Lys Ala Met Asp Asn Val
                35                  40                  45

Thr Val Arg Gln Gly Glu Ser Ala Thr Leu Arg Cys Thr Ile Asp
                50                  55                  60

Asn Arg Val Thr Arg Val Ala Trp Leu Asn Arg Ser Thr Ile Leu
                65                  70                  75

Tyr Ala Gly Asn Asp Lys Trp Cys Leu Asp Pro Arg Val Val Leu
                80                  85                  90

Leu Ser Asn Thr Gln Thr Gln Tyr Ser Ile Glu Ile Gln Asn Val
                95                  100                 105

Asp Val Tyr Asp Glu Gly Pro Tyr Thr Cys Ser Val Gln Thr Asp
                110                 115                 120

Asn His Pro Lys Thr Ser Arg Val His Leu Ile Val Gln Val Ser
                125                 130                 135

Pro Lys Ile Val Glu Ile Ser Ser Asp Ile Ser Ile Asn Glu Gly
                140                 145                 150

Asn Asn Ile Ser Leu Thr Cys Ile Ala Thr Gly Arg Pro Glu Pro
                155                 160                 165

Thr Val Thr Trp Arg His Ile Ser Pro Lys Ala Val Gly Phe Val
                170                 175                 180

Ser Glu Asp Glu Tyr Leu Glu Ile Gln Gly Ile Thr Arg Glu Gln
                185                 190                 195

Ser Gly Asp Tyr Glu Cys Ser Ala Ser Asn Asp Val Ala Ala Pro
                200                 205                 210

Val Val Arg Arg Val Lys Val Thr Val Asn Tyr Pro Pro Tyr Ile
                215                 220                 225

Ser Glu Ala Lys Gly Thr Gly Val Pro Val Gly Gln Lys Gly Thr
                230                 235                 240

Leu Gln Cys Glu Ala Ser Ala Val Pro Ser Ala Glu Phe Gln Trp
                245                 250                 255

Tyr Lys Asp Asp Lys Arg Leu Ile Glu Gly Lys Lys Gly Val Lys
                260                 265                 270

Val Glu Asn Arg Pro Phe Leu Ser Lys Leu Ile Phe Phe Asn Val
                275                 280                 285

Ser Glu His Asp Tyr Gly Asn Tyr Thr Cys Val Ala Ser Asn Lys
                290                 295                 300

Leu Gly His Thr Asn Ala Ser Ile Met Leu Phe Gly Pro Gly Ala
                305                 310                 315

Val Ser Glu Val Ser Asn Gly Thr Ser Arg Arg Ala Gly Cys Val
                320                 325                 330

Trp Leu Leu Pro Leu Leu Val Leu His Leu Leu Leu Lys Phe
                335                 340

<210> 9
<211> 2284
<212> DNA
<213> Homo sapiens

<400> 9
   gcggagcatc cgctgcggtc ctcgccgaga cccccgcgcg gattcgccgg 50
   tccttcccgc gggcgcgaca gagctgtcct cgcacctgga tggcagcagg 100
   ggcgccgggg tcctctcgac gccagagaga aatctcatca tctgtgcagc 150
   cttcttaaag caaactaaga ccagagggag gattatcctt gacctttgaa 200
   gaccaaaact aaactgaaat ttaaaatgtt cttcggggga gaagggagct 250
   tgacttacac tttggtaata atttgcttcc tgacactaag gctgtctgct 300
   agtcagaatt gcctcaaaaa gagtctagaa gatgttgtca ttgacatcca 350
   gtcatctctt tctaagggaa tcagaggcaa tgagcccgta tatacttcaa 400
   ctcaagaaga ctgcattaat tcttgctgtt caacaaaaaa catatcaggg 450
   gacaaagcat gtaacttgat gatcttcgac actcgaaaaa cagctagaca 500
   acccaactgc tacctatttt tctgtcccaa cgaggaagcc tgtccattga 550
   aaccagcaaa aggacttatg agttacagga taattacaga ttttccatct 600
   ttgaccagaa atttgccaag ccaagagtta ccccaggaag attctctctt 650
   acatggccaa ttttcacaag cagtcactcc cctagcccat catcacacag 700
   attattcaaa gcccaccgat atctcatgga gagacacact ttctcagaag 750
   tttggatcct cagatcacct ggagaaacta tttaagatgg atgaagcaag 800
   tgcccagctc cttgcttata aggaaaaagg ccattctcag agttcacaat 850
   tttcctctga tcaagaaata gctcatctgc tgcctgaaaa tgtgagtgcg 900
   ctcccagcta cggtggcagt tgcttctcca cataccacct cggctactcc 950
   aaagcccgcc acccttctac ccaccaatgc ttcagtgaca ccttctggga 1000
   cttcccagcc acagctggcc accacagctc cacctgtaac cactgtcact 1050
   tctcagcctc ccacgaccct catttctaca gtttttacac gggctgcggc 1100
   tacactccaa gcaatggcta caacagcagt tctgactacc acctttcagg 1150
   cacctacgga ctcgaaaggc agcttagaaa ccataccgtt tacagaaatc 1200
   tccaacttaa ctttgaacac agggaatgtg tataacccta ctgcactttc 1250
   tatgtcaaat gtggagtctt ccactatgaa taaaactgct tcctgggaag 1300
   gtagggaggc cagtccaggc agttcctccc agggcagtgt tccagaaaat 1350
   cagtacggcc ttccatttga aaaatggctt cttatcgggt ccctgctctt 1400
   tggtgtcctg ttcctggtga taggcctcgt cctcctgggt agaatccttt 1450
   cggaatcact ccgcaggaaa cgttactcaa gactggatta tttgatcaat 1500
   gggatctatg tggacatcta aggatggaac tcggtgtctc ttaattcatt 1550
   tagtaaccag aagcccaaat gcaatgagtt tctgctgact tgctagtctt 1600
   agcaggaggt tgtattttga agacaggaaa atgccccctt ctgctttcct 1650
   tttttttttt ggagacagag tcttgctctg ttgcccaggc tggagtgcag 1700
   tagcacgatc tcggctctca ccgcaacctc cgtctcctgg gttcaagcga 1750
   ttctcctgcc tcagcctcct aagtatctgg gattacaggc atgtgccacc 1800
   acacctgggt gatttttgta tttttagtag agacggggtt tcaccatgtt 1850
   ggtcaggctg gtctcaaact cctgacctag tgatccaccc tcctcggcct 1900
   cccaaagtgc tgggattaca ggcatgagcc accacagctg gcccccttct 1950
   gttttatgtt tggtttttga gaaggaatga agtgggaacc aaattaggta 2000
   attttgggta atctgtctct aaaatattag ctaaaaacaa agctctatgt 2050
   aaagtaataa agtataattg ccatataaat ttcaaaattc aactggcttt 2100
   tatgcaaaga aacaggttag gacatctagg ttccaattca ttcacattct 2150
   tggttccaga taaaatcaac tgtttatatc aatttctaat ggatttgctt 2200
   ttctttttat atggattcct ttaaaactta ttccagatgt agttccttcc 2250
   aattaaatat ttgaataaat cttttgttac tcaa 2284

<210> 10
<211> 431
<212> PRT
<213> Homo sapiens

<400> 10
Met Phe Phe Gly Gly Glu Gly Ser Leu Thr Tyr Thr Leu Val Ile
1               5                   10                  15

Ile Cys Phe Leu Thr Leu Arg Leu Ser Ala Ser Gln Asn Cys Leu
                20                  25                  30

Lys Lys Ser Leu Glu Asp Val Val Ile Asp Ile Gln Ser Ser Leu
                35                  40                  45

Ser Lys Gly Ile Arg Gly Asn Glu Pro Val Tyr Thr Ser Thr Gln
                50                  55                  60

Glu Asp Cys Ile Asn Ser Cys Cys Ser Thr Lys Asn Ile Ser Gly
                65                  70                  75

Asp Lys Ala Cys Asn Leu Met Ile Phe Asp Thr Arg Lys Thr Ala
                80                  85                  90

Arg Gln Pro Asn Cys Tyr Leu Phe Phe Cys Pro Asn Glu Glu Ala
                95                  100                 105

Cys Pro Leu Lys Pro Ala Lys Gly Leu Met Ser Tyr Arg Ile Ile
                110                 115                 120

Thr Asp Phe Pro Ser Leu Thr Arg Asn Leu Pro Ser Gln Glu Leu
                125                 130                 135

Pro Gln Glu Asp Ser Leu Leu His Gly Gln Phe Ser Gln Ala Val
                140                 145                 150

Thr Pro Leu Ala His His His Thr Asp Tyr Ser Lys Pro Thr Asp
                155                 160                 165

Ile Ser Trp Arg Asp Thr Leu Ser Gln Lys Phe Gly Ser Ser Asp
                170                 175                 180

His Leu Glu Lys Leu Phe Lys Met Asp Glu Ala Ser Ala Gln Leu
                185                 190                 195

Leu Ala Tyr Lys Glu Lys Gly His Ser Gln Ser Ser Gln Phe Ser
                200                 205                 210

Ser Asp Gln Glu Ile Ala His Leu Leu Pro Glu Asn Val Ser Ala
                215                 220                 225

Leu Pro Ala Thr Val Ala Val Ala Ser Pro His Thr Thr Ser Ala
                230                 235                 240

Thr Pro Lys Pro Ala Thr Leu Leu Pro Thr Asn Ala Ser Val Thr
                245                 250                 255

Pro Ser Gly Thr Ser Gln Pro Gln Leu Ala Thr Thr Ala Pro Pro
                260                 265                 270

Val Thr Thr Val Thr Ser Gln Pro Pro Thr Thr Leu Ile Ser Thr
                275                 280                 285

Val Phe Thr Arg Ala Ala Ala Thr Leu Gln Ala Met Ala Thr Thr
                290                 295                 300

Ala Val Leu Thr Thr Thr Phe Gln Ala Pro Thr Asp Ser Lys Gly
                305                 310                 315

Ser Leu Glu Thr Ile Pro Phe Thr Glu Ile Ser Asn Leu Thr Leu
                320                 325                 330

Asn Thr Gly Asn Val Tyr Asn Pro Thr Ala Leu Ser Met Ser Asn
                335                 340                 345

Val Glu Ser Ser Thr Met Asn Lys Thr Ala Ser Trp Glu Gly Arg
                350                 355                 360

Glu Ala Ser Pro Gly Ser Ser Ser Gln Gly Ser Val Pro Glu Asn
                365                 370                 375

Gln Tyr Gly Leu Pro Phe Glu Lys Trp Leu Leu Ile Gly Ser Leu
                380                 385                 390

Leu Phe Gly Val Leu Phe Leu Val Ile Gly Leu Val Leu Leu Gly
                395                 400                 405

Arg Ile Leu Ser Glu Ser Leu Arg Arg Lys Arg Tyr Ser Arg Leu
                410                 415                 420

Asp Tyr Leu Ile Asn Gly Ile Tyr Val Asp Ile
                425                 430

<210> 11
<211> 3121
<212> DNA
<213> Homo sapiens

<400> 11
gcgccctgag ctccgcctcc gggcccgata gcggcatcga gagcgcctcc 50

gtcgaggacc aggcggcgca gggggccggc gggcgaaagg aggatgaggg 100

ggcgcagcag ctgctgaccc tgcagaacca ggtggcgcgg ctggaggagg 150

agaaccgaga ctttctggct gcgctggagg acgccatgga gcagtacaaa 200

ctgcagagcg accggctgcg tgagcagcag gaggagatgg tggaactgcg 250

gctgcggtta gagctggtgc ggccaggctg ggggggcctg cggctcctga 300

atggcctgcc tcccgggtcc tttgtgcctc gacctcatac agcccccctg 350

gggggtgccc acgcccatgt gctgggcatg gtgccgcctg cctgcctccc 400

tggagatgaa gttggctctg agcagagggg agagcaggtg acaaatggca 450

gggaggctgg agctgagttg ctgactgagg tgaacaggct gggaagtggc 500

tcttcagctg cttcagagga ggaagaggag gaggaggagc cgcccaggcg 550

gaccttacac ctgcgcagaa ataggatcag caactgcagt cagagggcgg 600

gggcacgccc agggagtctg ccagagagga agggcccaga gctttgcctt 650

gaggagttgg atgcagccat tccagggtcc agagcagttg gtgggagcaa 700

ggcccgagtt caggcccgcc aggtcccccc tgccacagcc tcagagtggc 750

ggctggccca ggcccagcag aagatccggg agctggctat caacatccgc 800

atgaaggagg agcttattgg cgagctggtc cgcacaggaa aggcagctca 850

ggccctgaac cgccagcaca gccagcgtat ccgggagctg gagcaggagg 900

cagagcaggt gcgggccgag ctgagtgaag gccagaggca gctgcgggag 950

ctcgagggca aggagctcca ggatgctggc gagcggtctc ggctccagga 1000

gttccgcagg agggtcgctg cggcccagag ccaggtgcag gtgctgaagg 1050

agaagaagca ggctacggag cggctggtgt cactgtcggc ccagagtgag 1100

aagcgactgc aggagctcga gcggaacgtg cagctcatgc ggcagcagca 1150

gggacagctg cagaggcggc ttcgcgagga gacggagcag aagcggcgcc 1200

tggaggcaga aatgagcaag cggcagcacc gcgtcaagga gctggagctg 1250

aagcatgagc aacagcagaa gatcctgaag attaagacgg aagagatcgc 1300

ggccttccag aggaagaggc gcagtggcag caacggctct gtggtcagcc 1350

tggaacagca gcagaagatt gaggagcaga agaagtggct ggaccaggag 1400

atggagaagg tgctacagca gcggcgggcg ctggaggagc tgggggagga 1450

gctccacaag cgggaggcca tcctggccaa gaaggaggcc ctgatgcagg 1500

agaagacggg gctggagagc aagcgcctga gatccagcca ggccctcaac 1550

gaggacatcg tgcgagtgtc cagccggctg gagcacctgg agaaggagct 1600

gtccgagaag agcgggcagc tgcggcaggg cagcgcccag agccagcagc 1650

agatccgcgg ggagatcgac agcctgcgcc aggagaagga ctcgctgctc 1700

aagcagcgcc tggagatcga cggcaagctg aggcagggga gtctgctgtc 1750

ccccgaggag gagcggacgc tgttccagtt ggatgaggcc atcgaggccc 1800

tggatgctgc cattgagtat aagaatgagg ccatcacatg ccgccagcgg 1850

gtgcttcggg cctcagcctc gttgctgtcc cagtgcgaga tgaacctcat 1900

ggccaagctc agctacctct catcctcaga gaccagagcc ctcctctgca 1950

agtattttga caaggtggtg acgctccgag aggagcagca ccagcagcag 2000

attgccttct cggaactgga gatgcagctg gaggagcagc agaggctggt 2050

gtactggctg gaggtggccc tggagcggca gcgcctggag atggaccgcc 2100

agctgaccct gcagcagaag gagcacgagc agaacatgca gctgctcctg 2150

cagcagagtc gagaccacct cggtgaaggg ttagcagaca gcaggaggca 2200

gtatgaggcc cggattcaag ctctggagaa ggaactgggc cgttacatgt 2250

ggataaacca ggaactgaaa cagaagctcg gcggtgtgaa cgctgtaggc 2300

cacagcaggg gtggggagaa gaggagcctg tgctcggagg gcagacaggc 2350

tcctggaaat gaagatgagc tccacctggc acccgagctt ctctggctgt 2400

cccccctcac tgagggggcc ccccgcaccc gggaggagac gcgggacttg 2450

gtccacgctc cgttaccctt gacctggaaa cgctcgagcc tgtgtggtga 2500

ggagcagggg tcccccgagg aactgaggca gcgggaggcg gctgagcccc 2550

tggtggggcg ggtgcttcct gtgggtgagg caggcctgcc ctggaacttt 2600

gggcctttgt ccaagccccg gcgggaactg cgacgagcca gcccggggat 2650

gattgatgtc cggaaaaacc ccctgtaagc cctcggggca gaccctgcct 2700

tggagggaga ctccgagcct gctgaaaggg gcagctgcct gttttgcttc 2750

tgtgaagggc agtccttacc gcacacccta aatccaggcc ctcatctgta 2800

ccctcactgg gatcaacaaa tttgggccat ggcccaaaag aactggaccc 2850

tcatttaaca aaataatatg caaattccca ccacttactt ccatgaagct 2900

gtggtaccca attgccgcct tgtgtcttgc tcgaatctca ggacaattct 2950

ggtttcaggc gtaaatggat gtgcttgtag ttcaggggtt tggccaagaa 3000

tcatcacgaa agggtcggtg gcaaccaggt tgtggtttaa atggtcttat 3050

gtatataggg gaaactggga gactttagga tcttaaaaaa ccatttaata 3100

aaaaaaaatc tttgaaggga c 3121

<210> 12
<211> 830
<212> PRT
<213> Homo sapiens

<400> 12
Met Glu Gln Tyr Lys Leu Gln Ser Asp Arg Leu Arg Glu Gln Gln
1               5                   10                  15

Glu Glu Met Val Glu Leu Arg Leu Arg Leu Glu Leu Val Arg Pro
                20                  25                  30

Gly Trp Gly Gly Leu Arg Leu Leu Asn Gly Leu Pro Pro Gly Ser
                35                  40                  45

Phe Val Pro Arg Pro His Thr Ala Pro Leu Gly Gly Ala His Ala
                50                  55                  60

His Val Leu Gly Met Val Pro Pro Ala Cys Leu Pro Gly Asp Glu
                65                  70                  75

Val Gly Ser Glu Gln Arg Gly Glu Gln Val Thr Asn Gly Arg Glu
                80                  85                  90

Ala Gly Ala Glu Leu Leu Thr Glu Val Asn Arg Leu Gly Ser Gly
                95                  100                 105

Ser Ser Ala Ala Ser Glu Glu Glu Glu Glu Glu Glu Glu Pro Pro
                110                 115                 120

Arg Arg Thr Leu His Leu Arg Arg Asn Arg Ile Ser Asn Cys Ser
                125                 130                 135

Gln Arg Ala Gly Ala Arg Pro Gly Ser Leu Pro Glu Arg Lys Gly
                140                 145                 150

Pro Glu Leu Cys Leu Glu Glu Leu Asp Ala Ala Ile Pro Gly Ser
                155                 160                 165

Arg Ala Val Gly Gly Ser Lys Ala Arg Val Gln Ala Arg Gln Val
                170                 175                 180

Pro Pro Ala Thr Ala Ser Glu Trp Arg Leu Ala Gln Ala Gln Gln
                185                 190                 195

Lys Ile Arg Glu Leu Ala Ile Asn Ile Arg Met Lys Glu Glu Leu
                200                 205                 210

Ile Gly Glu Leu Val Arg Thr Gly Lys Ala Ala Gln Ala Leu Asn

215                 220                 225

Arg Gln His Ser Gln Arg Ile Arg Glu Leu Glu Gln Glu Ala Glu
                230                 235                 240

Gln Val Arg Ala Glu Leu Ser Glu Gly Gln Arg Gln Leu Arg Glu
                245                 250                 255

Leu Glu Gly Lys Glu Leu Gln Asp Ala Gly Glu Arg Ser Arg Leu
                260                 265                 270

Gln Glu Phe Arg Arg Arg Val Ala Ala Ala Gln Ser Gln Val Gln
                275                 280                 285

Val Leu Lys Glu Lys Lys Gln Ala Thr Glu Arg Leu Val Ser Leu
                290                 295                 300

Ser Ala Gln Ser Glu Lys Arg Leu Gln Glu Leu Glu Arg Asn Val
                305                 310                 315

Gln Leu Met Arg Gln Gln Gln Gly Gln Leu Gln Arg Arg Leu Arg
                320                 325                 330

Glu Glu Thr Glu Gln Lys Arg Arg Leu Glu Ala Glu Met Ser Lys
                335                 340                 345

Arg Gln His Arg Val Lys Glu Leu Glu Leu Lys His Glu Gln Gln
                350                 355                 360

Gln Lys Ile Leu Lys Ile Lys Thr Glu Glu Ile Ala Ala Phe Gln
                365                 370                 375

Arg Lys Arg Arg Ser Gly Ser Asn Gly Ser Val Val Ser Leu Glu
                380                 385                 390

Gln Gln Gln Lys Ile Glu Glu Gln Lys Lys Trp Leu Asp Gln Glu
                395                 400                 405

Met Glu Lys Val Leu Gln Gln Arg Arg Ala Leu Glu Glu Leu Gly
                410                 415                 420

Glu Glu Leu His Lys Arg Glu Ala Ile Leu Ala Lys Lys Glu Ala
                425                 430                 435

Leu Met Gln Glu Lys Thr Gly Leu Glu Ser Lys Arg Leu Arg Ser
                440                 445                 450

Ser Gln Ala Leu Asn Glu Asp Ile Val Arg Val Ser Ser Arg Leu
                455                 460                 465

Glu His Leu Glu Lys Glu Leu Ser Glu Lys Ser Gly Gln Leu Arg
                470                 475                 480

Gln Gly Ser Ala Gln Ser Gln Gln Gln Ile Arg Gly Glu Ile Asp
                485                 490                 495

Ser Leu Arg Gln Glu Lys Asp Ser Leu Leu Lys Gln Arg Leu Glu
                500                 505                 510

Ile Asp Gly Lys Leu Arg Gln Gly Ser Leu Leu Ser Pro Glu Glu
                515                 520                 525

Glu Arg Thr Leu Phe Gln Leu Asp Glu Ala Ile Glu Ala Leu Asp
                530                 535                 540

Ala Ala Ile Glu Tyr Lys Asn Glu Ala Ile Thr Cys Arg Gln Arg
                545                 550                 555

Val Leu Arg Ala Ser Ala Ser Leu Leu Ser Gln Cys Glu Met Asn
                560                 565                 570

Leu Met Ala Lys Leu Ser Tyr Leu Ser Ser Ser Glu Thr Arg Ala
                575                 580                 585

Leu Leu Cys Lys Tyr Phe Asp Lys Val Val Thr Leu Arg Glu Glu
                590                 595                 600

Gln His Gln Gln Gln Ile Ala Phe Ser Glu Leu Glu Met Gln Leu
                605                 610                 615

Glu Glu Gln Gln Arg Leu Val Tyr Trp Leu Glu Val Ala Leu Glu
                620                 625                 630

Arg Gln Arg Leu Glu Met Asp Arg Gln Leu Thr Leu Gln Gln Lys
                635                 640                 645

Glu His Glu Gln Asn Met Gln Leu Leu Leu Gln Gln Ser Arg Asp
                650                 655                 660

His Leu Gly Glu Gly Leu Ala Asp Ser Arg Arg Gln Tyr Glu Ala
                665                 670                 675

Arg Ile Gln Ala Leu Glu Lys Glu Leu Gly Arg Tyr Met Trp Ile
                680                 685                 690

Asn Gln Glu Leu Lys Gln Lys Leu Gly Gly Val Asn Ala Val Gly
                695                 700                 705

His Ser Arg Gly Gly Glu Lys Arg Ser Leu Cys Ser Glu Gly Arg
                710                 715                 720

Gln Ala Pro Gly Asn Glu Asp Glu Leu His Leu Ala Pro Glu Leu
                725                 730                 735

Leu Trp Leu Ser Pro Leu Thr Glu Gly Ala Pro Arg Thr Arg Glu
                740                 745                 750

Glu Thr Arg Asp Leu Val His Ala Pro Leu Pro Leu Thr Trp Lys
                755                 760                 765

Arg Ser Ser Leu Cys Gly Glu Glu Gln Gly Ser Pro Glu Glu Leu
                770                 775                 780

Arg Gln Arg Glu Ala Ala Glu Pro Leu Val Gly Arg Val Leu Pro
                785                 790                 795

Val Gly Glu Ala Gly Leu Pro Trp Asn Phe Gly Pro Leu Ser Lys
                800                 805                 810

Pro Arg Arg Glu Leu Arg Arg Ala Ser Pro Gly Met Ile Asp Val
                815                 820                 825

Arg Lys Asn Pro Leu
                830

<210> 13
<211> 2854
<212> DNA
<213> Homo sapiens

<400> 13
   ctaagaggac aagatgaggc ccggcctctc atttctccta gcccttctgt 50
   tcttccttgg ccaagctgca ggggatttgg gggatgtggg acctccaatt 100
   cccagccccg gcttcagctc tttcccaggt gttgactcca gctccagctt 150
   cagctccagc tccaggtcgg gctccagctc cagccgcagc ttaggcagcg 200
   gaggttctgt gtcccagttg ttttccaatt tcaccggctc cgtggatgac 250
   cgtgggacct gccagtgctc tgtttccctg ccagacacca cctttcccgt 300
   ggacagagtg gaacgcttgg aattcacagc tcatgttctt tctcagaagt 350
   ttgagaaaga actttctaaa gtgagggaat atgtccaatt aattagtgtg 400
   tatgaaaaga aactgttaaa cctaactgtc cgaattgaca tcatggagaa 450
   ggataccatt tcttacactg aactggactt cgagctgatc aaggtagaag 500
   tgaaggagat ggaaaaactg gtcatacagc tgaaggagag ttttggtgga 550
   agctcagaaa ttgttgacca gctggaggtg gagataagaa atatgactct 600
   cttggtagag aagcttgaga cactagacaa aaacaatgtc cttgccattc 650
   gccgagaaat cgtggctctg aagaccaagc tgaaagagtg tgaggcctct 700
   aaagatcaaa acacccctgt cgtccaccct cctcccactc cagggagctg 750
   tggtcatggt ggtgtggtga acatcagcaa accgtctgtg gttcagctca 800
   actggagagg gttttcttat ctatatggtg cttggggtag ggattactct 850
   ccccagcatc caaacaaagg actgtattgg gtggcgccat tgaatacaga 900
   tgggagactg ttggagtatt atagactgta caacacactg gatgatttgc 950
   tattgtatat aaatgctcga gagttgcgga tcacctatgg ccaaggtagt 1000
   ggtacagcag tttacaacaa caacatgtac gtcaacatgt acaacaccgg 1050
   gaatattgcc agagttaacc tgaccaccaa cacgattgct gtgactcaaa 1100
   ctctccctaa tgctgcctat aataaccgct tttcatatgc taatgttgct 1150
   tggcaagata ttgactttgc tgtggatgag aatggattgt gggttattta 1200
   ttcaactgaa gccagcactg gtaacatggt gattagtaaa ctcaatgaca 1250
   ccacacttca ggtgctaaac acttggtata ccaagcagta taaaccatct 1300
   gcttctaacg ccttcatggt atgtggggtt ctgtatgcca cccgtactat 1350
   gaacaccaga acagaagaga tttttactta ttatgacaca aacacaggga 1400
   aagagggcaa actagacatt gtaatgcata agatgcagga aaaagtgcag 1450
   agcattaact ataacccttt tgaccagaaa ctttatgtct ataacgatgg 1500
   ttaccttctg aattatgatc tttctgtctt gcagaagccc cagtaagctg 1550
   tttaggagtt agggtgaaag agaaaatgtt tgttgaaaaa atagtcttct 1600
   ccacttactt agatatctgc aggggtgtct aaaagtgtgt tcattttgca 1650
   gcaatgttta ggtgcatagt tctaccacac tagagatcta ggacatttgt 1700
   cttgatttgg tgagttctct tgggaatcat ctgcctcttc aggcgcattt 1750
   tgcaataaag tctgtctagg gtgggattgt cagaggtcta ggggcactgt 1800
   gggcctagtg aagcctactg tgaggaggct tcactagaag ccttaaatta 1850
   ggaattaagg aacttaaaac tcagtatggc gtctagggat tctttgtaca 1900
   ggaaatattg cccaatgact agtcctcatc catgtagcac cactaattct 1950
   tccatgcctg gaagaaacct ggggacttag ttaggtagat taatatctgg 2000
   agctcctcga gggaccaaat ctccaacttt tttttcccct cactagcacc 2050
   tggaatgatg ctttgtatgt ggcagataag taaatttggc atgcttatat 2100
   attctacatc tgtaaagtgc tgagttttat ggagagaggc ctttttatgc 2150
   attaaattgt acatggcaaa taaatcccag aaggatctgt agatgaggca 2200
   cctgcttttt cttttctctc attgtccacc ttactaaaag tcagtagaat 2250
   cttctacctc ataacttcct tccaaaggca gctcagaaga ttagaaccag 2300
   acttactaac caattccacc ccccaccaac ccccttctac tgcctacttt 2350
   aaaaaaatta atagttttct atggaactga tctaagatta gaaaaattaa 2400
   ttttctttaa tttcattatg gacttttatt tacatgactc taagactata 2450
   agaaaatctg atggcagtga caaagtgcta gcatttattg ttatctaata 2500
   aagaccttgg agcatatgtg caacttatga gtgtatcagt tgttgcatgt 2550
   aatttttgcc tttgtttaag cctggaactt gtaagaaaat gaaaatttaa 2600
   tttttttttc taggacgagc tatagaaaag ctattgagag tatctagtta 2650
   atcagtgcag tagttggaaa ccttgctggt gtatgtgatg tgcttctgtg 2700
   cttttgaatg actttatcat ctagtctttg tctatttttc ctttgatgtt 2750
   caagtcctag tctataggat tggcagttta aatgctttac tccccctttt 2800
   aaaataaatg attaaaatgt gctttgaaaa aaaaaaaaaa aaaaaaaaaa 2850
   aaaa 2854

<210> 14
<211> 510
<212> PRT
<213> Homo sapiens

<400> 14
Met Arg Pro Gly Leu Ser Phe Leu Leu Ala Leu Leu Phe Phe Leu
1               5                   10                  15

Gly Gln Ala Ala Gly Asp Leu Gly Asp Val Gly Pro Pro Ile Pro
                20                  25                  30

Ser Pro Gly Phe Ser Ser Phe Pro Gly Val Asp Ser Ser Ser Ser
                35                  40                  45

Phe Ser Ser Ser Ser Arg Ser Gly Ser Ser Ser Ser Arg Ser Leu
                50                  55                  60

Gly Ser Gly Gly Ser Val Ser Gln Leu Phe Ser Asn Phe Thr Gly
                65                  70                  75

Ser Val Asp Asp Arg Gly Thr Cys Gln Cys Ser Val Ser Leu Pro
                80                  85                  90

Asp Thr Thr Phe Pro Val Asp Arg Val Glu Arg Leu Glu Phe Thr
                95                  100                 105

Ala His Val Leu Ser Gln Lys Phe Glu Lys Glu Leu Ser Lys Val
                110                 115                 120

Arg Glu Tyr Val Gln Leu Ile Ser Val Tyr Glu Lys Lys Leu Leu
                125                 130                 135

Asn Leu Thr Val Arg Ile Asp Ile Met Glu Lys Asp Thr Ile Ser
                140                 145                 150

Tyr Thr Glu Leu Asp Phe Glu Leu Ile Lys Val Glu Val Lys Glu
                155                 160                 165

Met Glu Lys Leu Val Ile Gln Leu Lys Glu Ser Phe Gly Gly Ser
                170                 175                 180

Ser Glu Ile Val Asp Gln Leu Glu Val Glu Ile Arg Asn Met Thr

                185                 190                 195

Leu Leu Val Glu Lys Leu Glu Thr Leu Asp Lys Asn Asn Val Leu
                200                 205                 210

Ala Ile Arg Arg Glu Ile Val Ala Leu Lys Thr Lys Leu Lys Glu
                215                 220                 225

Cys Glu Ala Ser Lys Asp Gln Asn Thr Pro Val Val His Pro Pro
                230                 235                 240

Pro Thr Pro Gly Ser Cys Gly His Gly Gly Val Val Asn Ile Ser
                245                 250                 255

Lys Pro Ser Val Val Gln Leu Asn Trp Arg Gly Phe Ser Tyr Leu
                260                 265                 270

Tyr Gly Ala Trp Gly Arg Asp Tyr Ser Pro Gln His Pro Asn Lys
                275                 280                 285

Gly Leu Tyr Trp Val Ala Pro Leu Asn Thr Asp Gly Arg Leu Leu
                290                 295                 300

Glu Tyr Tyr Arg Leu Tyr Asn Thr Leu Asp Asp Leu Leu Leu Tyr
                305                 310                 315

Ile Asn Ala Arg Glu Leu Arg Ile Thr Tyr Gly Gln Gly Ser Gly
                320                 325                 330

Thr Ala Val Tyr Asn Asn Asn Met Tyr Val Asn Met Tyr Asn Thr
                335                 340                 345

Gly Asn Ile Ala Arg Val Asn Leu Thr Thr Asn Thr Ile Ala Val
                350                 355                 360

Thr Gln Thr Leu Pro Asn Ala Ala Tyr Asn Asn Arg Phe Ser Tyr
                365                 370                 375

Ala Asn Val Ala Trp Gln Asp Ile Asp Phe Ala Val Asp Glu Asn
                380                 385                 390

Gly Leu Trp Val Ile Tyr Ser Thr Glu Ala Ser Thr Gly Asn Met
                395                 400                 405

Val Ile Ser Lys Leu Asn Asp Thr Thr Leu Gln Val Leu Asn Thr
                410                 415                 420

Trp Tyr Thr Lys Gln Tyr Lys Pro Ser Ala Ser Asn Ala Phe Met
                425                 430                 435

Val Cys Gly Val Leu Tyr Ala Thr Arg Thr Met Asn Thr Arg Thr
                440                 445                 450

Glu Glu Ile Phe Tyr Tyr Tyr Asp Thr Asn Thr Gly Lys Glu Gly
                455                 460                 465

Lys Leu Asp Ile Val Met His Lys Met Gln Glu Lys Val Gln Ser
                470                 475                 480

Ile Asn Tyr Asn Pro Phe Asp Gln Lys Leu Tyr Val Tyr Asn Asp
                485                 490                 495

Gly Tyr Leu Leu Asn Tyr Asp Leu Ser Val Leu Gln Lys Pro Gln
                500                 505                 510

<210> 15
<211> 1830
<212> DNA
<213> Homo sapiens

<400> 15
gtggaggccg ccgacgatgg cggggccgac ggaggccgag acggggttgg 50

ccgagccccg ggccctgtgc gcgcagcggg gccaccgcac ctacgcgcgc 100

cgctgggtgt tcctgctcgc gatcagcctg ctcaactgct ccaacgccac 150

gctgtggctc agctttgcac ctgtggctga cgtcattgct gaggacttgg 200

tcctgtccat ggagcagatc aactggctgt cactggtcta cctcgtggta 250

tccaccccat ttggcgtggc ggccatctgg atcctggact ccgtcgggct 300

ccgtgcggcg accatcctgg gtgcgtggct gaactttgcc gggagtgtgc 350

tacgcatggt gccctgcatg gttgttggga cccaaaaccc atttgccttc 400

ctcatgggtg gccagagcct ctgtgccctt gcccagagcc tggtcatctt 450

ctctccagcc aagctggctg ccttgtggtt cccagagcac cagcgagcca 500

cggccaacat gctcgccacc atgtcgaacc ctctgggcgt ccttgtggcc 550

aatgtgctgt cccctgtgct ggtcaagaag ggtgaggaca ttccgttaat 600

gctcggtgtc tataccatcc ctgctggcgt cgtctgcctg ctgtccacca 650

tctgcctgtg ggagagtgtg ccccccaccc cgccctctgc cggggctgcc 700

agctccacct cagagaagtt cctggatggg ctcaagctgc agctcatgtg 750

gaacaaggcc tatgtcatcc tggctgtgtg cttgggggga atgatcggga 800

tctctgccag cttctcagcc ctcctggagc agatcctctg tgcaagcggc 850

cactccagtg ggttttccgg cctctgtggc gctctcttca tcacgtttgg 900

gatcctgggg gcactggctc tcggccccta tgtggaccgg accaagcact 950

tcactgaggc caccaagatt ggcctgtgcc tgttctctct ggcctgcgtg 1000

ccctttgccc tggtgtccca gctgcaggga cagacccttg ccctggctgc 1050

cacctgctcg ctgctcgggc tgtttggctt ctcggtgggc cccgtggcca 1100

tggagttggc ggtcgagtgt tccttccccg tgggggaggg ggctgccaca 1150

ggcatgatct ttgtgctggg gcaggccgag ggaatactca tcatgctggc 1200

aatgacggca ctgactgtgc gacgctcgga gccgtccttg tccacctgcc 1250

agcaggggga ggatccactt gactggacag tgtctctgct gctgatggcc 1300

ggcctgtgca ccttcttcag ctgcatcctg gcggtcttct tccacacccc 1350

ataccggcgc ctgcaggccg agtctgggga gcccccctcc acccgtaacg 1400

ccgtgggcgg cgcagactca gggccgggtg tggaccgagg gggagcagga 1450

agggctgggg tcctggggcc cagcacggcg actccggagt gcacggcgag 1500

gggggcctcg ctagaggacc ccagagggcc cgggagcccc cacccagcct 1550

gccaccgagc gactccccgt gcgcaaggcc cagcagccac cgacgcgccc 1600

tcccgccccg gcagactcgc aggcagggtc caagcgtcca ggtttattga 1650

cccggctggg tctcactcct ccttctcctc cccgtgggtg atcacgtagc 1700

tgagcgcctt gtagtccagg ttgcccgcca catcgatgga ggcgaactgg 1750

aacatctggt ccacctgcgg gcgggggcga aaggctcctt tgcgggctcc 1800

gggagcgaat tacaagcgcg cacctgaaaa 1830

<210> 16
<211> 560
<212> PRT
<213> Homo sapiens

<400> 16
Met Ala Gly Pro Thr Glu Ala Glu Thr Gly Leu Ala Glu Pro Arg
1               5                   10                  15

Ala Leu Cys Ala Gln Arg Gly His Arg Thr Tyr Ala Arg Arg Trp
                20                  25                  30

Val Phe Leu Leu Ala Ile Ser Leu Leu Asn Cys Ser Asn Ala Thr
                35                  40                  45

Leu Trp Leu Ser Phe Ala Pro Val Ala Asp Val Ile Ala Glu Asp
                50                  55                  60

Leu Val Leu Ser Met Glu Gln Ile Asn Trp Leu Ser Leu Val Tyr
                65                  70                  75

Leu Val Val Ser Thr Pro Phe Gly Val Ala Ala Ile Trp Ile Leu
                80                  85                  90

Asp Ser Val Gly Leu Arg Ala Ala Thr Ile Leu Gly Ala Trp Leu
                95                  100                 105

Asn Phe Ala Gly Ser Val Leu Arg Met Val Pro Cys Met Val Val
                110                 115                 120

Gly Thr Gln Asn Pro Phe Ala Phe Leu Met Gly Gly Gln Ser Leu
                125                 130                 135

Cys Ala Leu Ala Gln Ser Leu Val Ile Phe Ser Pro Ala Lys Leu
                140                 145                 150

Ala Ala Leu Trp Phe Pro Glu His Gln Arg Ala Thr Ala Asn Met
                155                 160                 165

Leu Ala Thr Met Ser Asn Pro Leu Gly Val Leu Val Ala Asn Val
                170                 175                 180

Leu Ser Pro Val Leu Val Lys Lys Gly Glu Asp Ile Pro Leu Met
                185                 190                 195

Leu Gly Val Tyr Thr Ile Pro Ala Gly Val Val Cys Leu Leu Ser
                200                 205                 210

Thr Ile Cys Leu Trp Glu Ser Val Pro Pro Thr Pro Pro Ser Ala
                215                 220                 225

Gly Ala Ala Ser Ser Thr Ser Glu Lys Phe Leu Asp Gly Leu Lys
                230                 235                 240

Leu Gln Leu Met Trp Asn Lys Ala Tyr Val Ile Leu Ala Val Cys
                245                 250                 255

Leu Gly Gly Met Ile Gly Ile Ser Ala Ser Phe Ser Ala Leu Leu
                260                 265                 270

Glu Gln Ile Leu Cys Ala Ser Gly His Ser Ser Gly Phe Ser Gly
                275                 280                 285

Leu Cys Gly Ala Leu Phe Ile Thr Phe Gly Ile Leu Gly Ala Leu
                290                 295                 300

Ala Leu Gly Pro Tyr Val Asp Arg Thr Lys His Phe Thr Glu Ala
                305                 310                 315

Thr Lys Ile Gly Leu Cys Leu Phe Ser Leu Ala Cys Val Pro Phe
                320                 325                 330

Ala Leu Val Ser Gln Leu Gln Gly Gln Thr Leu Ala Leu Ala Ala
                335                 340                 345

Thr Cys Ser Leu Leu Gly Leu Phe Gly Phe Ser Val Gly Pro Val
                350                 355                 360

Ala Met Glu Leu Ala Val Glu Cys Ser Phe Pro Val Gly Glu Gly
                365                 370                 375

Ala Ala Thr Gly Met Ile Phe Val Leu Gly Gln Ala Glu Gly Ile
                380                 385                 390

Leu Ile Met Leu Ala Met Thr Ala Leu Thr Val Arg Arg Ser Glu
                395                 400                 405

Pro Ser Leu Ser Thr Cys Gln Gln Gly Glu Asp Pro Leu Asp Trp
                410                 415                 420

Thr Val Ser Leu Leu Leu Met Ala Gly Leu Cys Thr Phe Phe Ser
                425                 430                 435

Cys Ile Leu Ala Val Phe Phe His Thr Pro Tyr Arg Arg Leu Gln
                440                 445                 450

Ala Glu Ser Gly Glu Pro Pro Ser Thr Arg Asn Ala Val Gly Gly
                455                 460                 465

Ala Asp Ser Gly Pro Gly Val Asp Arg Gly Gly Ala Gly Arg Ala
                470                 475                 480

Gly Val Leu Gly Pro Ser Thr Ala Thr Pro Glu Cys Thr Ala Arg
                485                 490                 495

Gly Ala Ser Leu Glu Asp Pro Arg Gly Pro Gly Ser Pro His Pro
                500                 505                 510

Ala Cys His Arg Ala Thr Pro Arg Ala Gln Gly Pro Ala Ala Thr
                515                 520                 525

Asp Ala Pro Ser Arg Pro Gly Arg Leu Ala Gly Arg Val Gln Ala
                530                 535                 540

Ser Arg Phe Ile Asp Pro Ala Gly Ser His Ser Ser Phe Ser Ser
                545                 550                 555

Pro Trp Val Ile Thr
                560

<210> 17
<211> 2749
<212> DNA
<213> Homo sapiens

<220>
<221> Unsure
<222> 1869,1887
<223> Unknown base

<400> 17
ctcccacggt gtccagcgcc cagaatgcgg cttctggtcc tgctatgggg 50

ttgcctgctg ctcccaggtt atgaagccct ggagggccca gaggaaatca 100

gcgggttcga aggggacact gtgtccctgc agtgcaccta cagggaagag 150

ctgagggacc accggaagta ctggtgcagg aagggtggga tcctcttctc 200

tcgctgctct ggcaccatct atgcagaaga agaaggccag gagacaatga 250

agggcagggt gtccatccgt gacagccgcc aggagctctc gctcattgtg 300

accctgtgga acctcaccct gcaagacgct ggggagtact ggtgtggggt 350

cgaaaaacgg ggccccgatg agtctttact gatctctctg ttcgtctttc 400

caggaccctg ctgtcctccc tccccttctc ccaccttcca gcctctggct 450

acaacacgcc tgcagcccaa ggcaaaagct cagcaaaccc agcccccagg 500

attgacttct cctgggctct acccggcagc caccacagcc aagcagggga 550

agacaggggc tgaggcccct ccattgccag ggacttccca gtacgggcac 600

gaaaggactt ctcagtacac aggaacctct cctcacccag cgacctctcc 650

tcctgcaggg agctcccgcc cccccatgca gctggactcc acctcagcag 700

aggacaccag tccagctctc agcagtggca gctctaagcc cagggtgtcc 750

atcccgatgg tccgcatact ggccccagtc ctggtgctgc tgagccttct 800

gtcagccgca ggcctgatcg ccttctgcag ccacctgctc ctgtggagaa 850

aggaagctca acaggccacg gagacacaga ggaacgagaa gttctggctc 900

tcacgcttga ctgcggagga aaaggaagcc ccttcccagg cccctgaggg 950

ggacgtgatc tcgatgcctc ccctccacac atctgaggag gagctgggct 1000

tctcgaagtt tgtctcagcg tagggcagga ggccctcctg gccaggccag 1050

cagtgaagca gtatggctgg ctggatcagc accgattccc gaaagctttc 1100

cacctcagcc tcagagtcca gctgcccgga ctccagggct ctccccaccc 1150

tccccaggct ctcctcttgc atgttccagc ctgacctaga agcgtttgtc 1200

agccctggag cccagagcgg tggccttgct cttccggctg gagactggga 1250

catccctgat aggttcacat ccctgggcag agtaccaggc tgctgaccct 1300

cagcagggcc agacaaggct cagtggatct ggtctgagtt tcaatctgcc 1350

aggaactcct gggcctcatg cccagtgtcg gaccctgcct tcctcccact 1400

ccagacccca ccttgtcttc cctccctggc gtcctcagac ttagtcccac 1450

ggtctcctgc atcagctggt gatgaagagg agcatgctgg ggtgagactg 1500

ggattctggc ttctctttga accacctgca tccagccctt caggaagcct 1550

gtgaaaaacg tgattcctgg ccccaccaag acccaccaaa accatctctg 1600

ggcttggtgc aggactctga attctaacaa tgcccagtga ctgtcgcact 1650

tgagtttgag ggccagtggg cctgatgaac gctcacaccc cttcagctta 1700

gagtctgcat ttgggctgtg acgtctccac ctgccccaat agatctgctc 1750

tgtctgcgac accagatcca cgtggggact cccctgaggc ctgctaagtc 1800

caggccttgg tcaggtcagg tgcacattgc aggataagcc caggaccggc 1850

acagaagtgg ttgcctttnc catttgccct ccctggncca tgccttcttg 1900

cctttggaaa aaatgatgaa gaaaaccttg gctccttcct tgtctggaaa 1950

gggttacttg cctatgggtt ctggtggcta gagagaaaag tagaaaacca 2000

gagtgcacgt aggtgtctaa cacagaggag agtaggaaca gggcggatac 2050

ctgaaggtga ctccgagtcc agccccctgg agaaggggtc gggggtggtg 2100

gtaaagtagc acaactacta ttttttttct ttttccatta ttattgtttt 2150

ttaagacaga atctcgtgct gctgcccagg ctggagtgca gtggcacgat 2200

ctgcaaactc cgcctcctgg gttcaagtga ttcttctgcc tcagcctccc 2250

gagtagctgg gattacaggc acgcaccacc acacctggct aatttttgta 2300

cttttagtag agatggggtt tcaccatgtt ggccaggctg gtcttgaact 2350

cctgacctca aatgagcctc ctgcttcagt ctcccaaatt gccgggatta 2400

caggcatgag ccactgtgtc tggccctatt tcctttaaaa agtgaaatta 2450

agagttgttc agtatgcaaa acttggaaag atggaggaga aaaagaaaag 2500

gaagaaaaaa atgtcaccca tagtctcacc agagactatc attatttcgt 2550

tttgttgtac ttccttccac tcttttcttc ttcacataat ttgccggtgt 2600

tctttttaca gagcaattat cttgtatata caactttgta tcctgccttt 2650

tccaccttat cgttccatca ctttattcca gcacttctct gtgttttaca 2700

gaccttttta taaataaaat gttcatcagc tgcataaaaa aaaaaaaaa 2749

<210> 18
<211> 332
<212> PRT
<213> Homo sapiens

<400> 18

<210> 19
<211> 1395
<212> DNA

<213> Homo sapiens

<400> 19
cggacgcgtg ggcggacgcg tgggggctgt gagaaagtgc caataaatac 50

atcatgcaac cccacggccc accttgtgaa ctcctcgtgc ccagggctga 100

tgtgcgtctt ccagggctac tcatccaaag gcctaatcca acgttctgtc 150

ttcaatctgc aaatctatgg ggtcctgggg ctcttctgga cccttaactg 200

ggtactggcc ctgggccaat gcgtcctcgc tggagccttt gcctccttct 250

actgggcctt ccacaagccc caggacatcc ctaccttccc cttaatctct 300

gccttcatcc gcacactccg ttaccacact gggtcattgg catttggagc 350

cctcatcctg acccttgtgc agatagcccg ggtcatcttg gagtatattg 400

accacaagct cagaggagtg cagaaccctg tagcccgctg catcatgtgc 450

tgtttcaagt gctgcctctg gtgtctggaa aaatttatca agttcctaaa 500

ccgcaatgca tacatcatga tcgccatcta cgggaagaat ttctgtgtct 550

cagccaaaaa tgcgttcatg ctactcatgc gaaacattgt cagggtggtc 600

gtcctggaca aagtcacaga cctgctgctg ttctttggga agctgctggt 650

ggtcggaggc gtgggggtcc tgtccttctt ttttttctcc ggtcgcatcc 700

cggggctggg taaagacttt aagagccccc acctcaacta ttactggctg 750

cccatcatga cctccatcct gggggcctat gtcatcgcca gcggcttctt 800

cagcgttttc ggcatgtgtg tggacacgct cttcctctgc ttcctggaag 850

acctggagcg gaacaacggc tccctggacc ggccctacta catgtccaag 900

agccttctaa agattctggg caagaagaac gaggcgcccc cggacaacaa 950

gaagaggaag aagtgacagc tccggccctg atccaggact gcaccccacc 1000

cccaccgtcc agccatccaa cctcacttcg ccttacaggt ctccattttg 1050

tggtaaaaaa aggttttagg ccaggcgccg tggctcacgc ctgtaatcca 1100

acactttgag aggctgaggc gggcggatca cctgagtcag gagttcgaga 1150

ccagcctggc caacatggtg aaacctccgt ctctattaaa aatacaaaaa 1200

ttagccgaga gtggtggcat gcacctgtca tcccagctac tcgggaggct 1250

gaggcaggag aatcgcttga acccgggagg cagaggttgc agtgagccga 1300

gatcgcgcca ctgcactcca acctgggtga cagactctgt ctccaaaaca 1350

aaacaaacaa acaaaaagat tttattaaag atattttgtt aactc 1395

<210> 20
<211> 288
<212> PRT
<213> Homo sapiens

<400> 20
Met Cys Val Phe Gln Gly Tyr Ser Ser Lys Gly Leu Ile Gln Arg
1               5                   10                  15

Ser Val Phe Asn Leu Gln Ile Tyr Gly Val Leu Gly Leu Phe Trp
                20                  25                  30

Thr Leu Asn Trp Val Leu Ala Leu Gly Gln Cys Val Leu Ala Gly
                35                  40                  45

Ala Phe Ala Ser Phe Tyr Trp Ala Phe His Lys Pro Gln Asp Ile
                50                  55                  60

Pro Thr Phe Pro Leu Ile Ser Ala Phe Ile Arg Thr Leu Arg Tyr
                65                  70                  75

His Thr Gly Ser Leu Ala Phe Gly Ala Leu Ile Leu Thr Leu Val
                80                  85                  90

Gln Ile Ala Arg Val Ile Leu Glu Tyr Ile Asp His Lys Leu Arg
                95                  100                 105

Gly Val Gln Asn Pro Val Ala Arg Cys Ile Met Cys Cys Phe Lys
                110                 115                 120

Cys Cys Leu Trp Cys Leu Glu Lys Phe Ile Lys Phe Leu Asn Arg
                125                 130                 135

Asn Ala Tyr Ile Met Ile Ala Ile Tyr Gly Lys Asn Phe Cys Val
                140                 145                 150

Ser Ala Lys Asn Ala Phe Met Leu Leu Met Arg Asn Ile Val Arg
                155                 160                 165

Val Val Val Leu Asp Lys Val Thr Asp Leu Leu Leu Phe Phe Gly
                170                 175                 180

Lys Leu Leu Val Val Gly Gly Val Gly Val Leu Ser Phe Phe Phe
                185                 190                 195

Phe Ser Gly Arg Ile Pro Gly Leu Gly Lys Asp Phe Lys Ser Pro
                200                 205                 210

His Leu Asn Tyr Tyr Trp Leu Pro Ile Met Thr Ser Ile Leu Gly
                215                 220                 225

Ala Tyr Val Ile Ala Ser Gly Phe Phe Ser Val Phe Gly Met Cys
                230                 235                 240

Val Asp Thr Leu Phe Leu Cys Phe Leu Glu Asp Leu Glu Arg Asn
                245                 250                 255

Asn Gly Ser Leu Asp Arg Pro Tyr Tyr Met Ser Lys Ser Leu Leu
                260                 265                 270

Lys Ile Leu Gly Lys Lys Asn Glu Ala Pro Pro Asp Asn Lys Lys
                275                 280                 285

Arg Lys Lys

<210> 21
<211> 2673
<212> DNA
<213> Homo sapiens

<400> 21
   cggggtcacg ggcagttgca gccgcggccg agcagccagc cgctaagaaa 50
   gagctcgccg ctgccgctcc cggagccgcc gaggccagct tcgcggcgct 100
   gccccgcggc gggagaggag gctgcagaag agcggaggcg gccagcggga 150
   gcggcggggc tcagcgcgca cactcagcgg ccggggagcc tcccgagctc 200
   tgcgcccgca cgcgccagcc gcggctcgcg cctttcttgg cctccgggcg 250
   cccgacctct cctcccccgc gccggctcgc cggggccgcg gcggcccaag 300
   gagcagcatg aatctgcggc tctgcgtgca ggcgctcctg ctgctctggc 350
   tctccttgac cgcggtgtgt ggagggtccc tgatgccgct tcccgatggg 400
   aatgggctgg aagacggcaa tgtccgccac ctggtgcagc ccagagggtc 450
   aaggaatggg ccagggccct ggcagggagg tcggaggaaa ttccgccgcc 500
   agcggccccg cctctcccat aagggaccca tgcctttctg aagcaggact 550
   gaaggggccc ccaagtgccc acccccggcg gttatgtctc ctccatagat 600
   tggtctgctt ctctggaggc ctcacgtcca ttcagctctc acctcgcacc 650
   tgctgtagcc accagtgggc ccagctcttc tcacctgcct gcttccccca 700
   gtggcgtgct cctggctgta gtttggatga ttcccgttct ctcacaagaa 750
   tccgtccagt ccatcttcct ggcccctccc tggactgact ttggagacct 800
   agccccagaa agcctccctt cttctccagg tcccctccgc cctagtccct 850
   gcctgtctca tctaacgccc caaaccttca tttgggcctt ccttcctcat 900
   gtctgccctg agcgcggggt ggaagtgctc ccttctgtgg gctccagcag 950
   atcccttgtt ttcctgtcag ttggacccct cacctggcct ccagggaaga 1000
   atgcagagaa aagcaaggag agactctagt taagaggtgc tggctgccgg 1050
   gatccagaca gggcacattg gggcatggaa gtgccagggt tggttttcag 1100
   gagctctggt gaagtgggtg gagcatcagc gtttgctcag ttaagggaga 1150
   ggtagagagg ggcccgtgaa gtcctttgtc acttctcttg ccttagtgtg 1200
   cctcccaata ctcccttctt cctgccccca caccccatcc ccagctagcc 1250
   caagctccag gtcaggaggg gagggtgctg ggcctgacat ggctatatac 1300
   cctcccagga gtaaaagcca agcaagaggt tgtttttgcc aagaatcaca 1350
   gaatgttaga gctgacagga cccttgaagg tcacttagcc ttcttaggca 1400
   aacgcctgca aaacagaagc ctggagaggg gagtgacctg ctcagagtca 1450
   ttgcagagcc gggatgggga ccaggtctcc catctcctac tttatgacgc 1500
   cctcttccct cttgatgatg tcttttcaaa gcaaatgaag tgccttttcc 1550
   cgaggctggg gctgggggtg gctgggaggg gaagggaagg gagaggcaag 1600
   ctggctgtga actgtcctgt tgtggggctg gagctgctcc cacctccctg 1650
   acctacccct gctgcaccat tcccccagct gggctggaag gttccataac 1700
   tggccagctg cccccataac tggcagcatt cccagaccca gggtactcta 1750
   ataggggcgg ctcaggcact gagactaccg ctcaacccca gggtggtttt 1800
   caggagtccg aggtagcctt caatcactgg actccatggc cttcccttcg 1850
   tgttgaccgg accttccttc cagggctttt cctttggggg aggcggagag 1900
   gggagaagaa ggaagggaag ggcagaagga aggagggaag aaaagaaagc 1950
   aaaggaacag aaggaaggaa agaaagatgg gaggaagtgc agcaggaata 2000
   gcaccctctc cccgggaggc cctagcttcc gtgaggggcc atcaccagcc 2050
   attccttgga gggggctttc tccccttttg cttgagcagg gttcccagga 2100
   gggagaaaga gaagacaaga gcctgatgcc caactttgtg tgtgtgggga 2150
   cgggggagtc agggcccccc aagtcccaca atagccccaa tgtttgccta 2200
   tccacctccc ccaagcccct ttacctatgc tgctgctaac gctgctgctg 2250
   ctgctgctgc tgcttaaagg ctcatgcttg gagtggggac tggtcggtgc 2300
   ccagaaagtc tcttctgcca ctgacgcccc catcagggat tgggccttct 2350
   ttcccccttc ctttctgtgt ctcctgcctc atcggcctgc catgacctgc 2400
   agccaagccc agccccgtgg ggaaggggag aaagtggggg atggctaaga 2450
   aagctgggag atagggaaca gaagagggta gtgggtgggc tagggggcct 2500
   gccttattta aagtggttgt ttatgattct tatactaatt tatacaaaga 2550
   tattaaggcc ctgttcatta agaaattgtt cccttcccct gtgttcaatg 2600
   tttgtaaaga ttgttctgtg taaatatgtc tttataataa acagttaaaa 2650
   gctgaaaaaa aaaaaaaaaa aaa 2673

<210> 22
<211> 77
<212> PRT
<213> Homo sapiens

<400> 22
Met Asn Leu Arg Leu Cys Val Gln Ala Leu Leu Leu Leu Trp Leu
1               5                   10                  15

Ser Leu Thr Ala Val Cys Gly Gly Ser Leu Met Pro Leu Pro Asp
                20                  25                  30

Gly Asn Gly Leu Glu Asp Gly Asn Val Arg His Leu Val Gln Pro
                35                  40                  45

Arg Gly Ser Arg Asn Gly Pro Gly Pro Trp Gln Gly Gly Arg Arg
                50                  55                  60

Lys Phe Arg Arg Gln Arg Pro Arg Leu Ser His Lys Gly Pro Met
                65                  70                  75

Pro Phe

<210> 23
<211> 2478
<212> DNA
<213> Homo sapiens

<400> 23
atctggttga actacttaag cttaatttgt taaactccgg taagtaccta 50

gcccacatga tttgactcag agattctctt ttgtccacag acagtcatct 100

caggggcaga aagaaaagag ctcccaaatg ctatatctat tcaggggctc 150

tcaagaacaa tggaatatca tcctgattta gaaaatttgg atgaagatgg 200

atatactcaa ttacacttcg actctcaaag caataccagg atagctgttg 250

tttcagagaa aggatcgtgt gctgcatctc ctccttggcg cctcattgct 300

gtaattttgg gaatcctatg cttggtaata ctggtgatag ctgtggtcct 350

gggtaccatg gggttctttt ccagcccttg tcctcctaat tggattatat 400

atgagaagag ctgttatcta ttcagcatgt cactaaattc ctgggatgga 450

agtaaaagac aatgctggca actgggctct aatctcctaa agatagacag 500

ctcaaatgaa ttgggattta tagtaaaaca agtgtcttcc caacctgata 550

attcattttg gataggcctt tctcggcccc agactgaggt accatggctc 600

tgggaggatg gatcaacatt ctcttctaac ttatttcaga tcagaaccac 650

agctacccaa gaaaacccat ctccaaattg tgtatggatt cacgtgtcag 700

tcatttatga ccaactgtgt agtgtgccct catatagtat ttgtgagaag 750

aagttttcaa tgtaagagga agggtggaga aggagagaga aatatgtgag 800

gtagtaagga ggacagaaaa cagaacagaa aagagtaaca gctgaggtca 850

agataaatgc agaaaatgtt tagagagctt ggccaactgt aatcttaacc 900

aagaaattga agggagaggc tgtgatttct gtatttgtcg acctacaggt 950

aggctagtat tatttttcta gttagtagat ccctagacat ggaatcaggg 1000

cagccaagct tgagttttta ttttttattt atttattttt ttgagatagg 1050

gtctcacttt gttacccagg ctggagtgca gtggcacaat ctcgactcac 1100

tgcagctatc tctcgcctca gcccctcaag tagctgggac tacaggtgca 1150

tgccaccatg ccaggctaat ttttggtgtt ttttgtagag actgggtttt 1200

gccatgttga ccaagctggt ctctaactcc tgggcttaag tgatctgccc 1250

gccttggcct cccaaagtgc tgggattaca gatgtgagcc accacacctg 1300

gccccaagct tgaattttca ttctgccatt gacttggcat ttaccttggg 1350

taagccataa gcgaatctta atttctggct ctatcagagt tgtttcatgc 1400

tcaacaatgc cattgaagtg cacggtgtgt tgccacgatt tgaccctcaa 1450

cttctagcag tatatcagtt atgaactgag ggtgaaatat atttctgaat 1500

agctaaatga agaaatggga aaaaatcttc accacagtca gagcaatttt 1550

attattttca tcagtatgat cataattatg attatcatct tagtaaaaag 1600

caggaactcc tactttttct ttatcaatta aatagctcag agagtacatc 1650

tgccatatct ctaatagaat cttttttttt tttttttttt tttgagacag 1700

agtttcgctc ttgttgccca ggctggagtg caacggcacg atctcggctc 1750

accgcaacct ccgccccctg ggttcaagca attctcctgc ctcagcctcc 1800

caagtagctg ggattacagt caggcaccac cacacccggc taattttgta 1850

tttttttagt agagacaggg tttctccatg tcggtcaggg tagtcccgaa 1900

ctcctgacct caagtgatct gcctgcctcg gcctcccaag tgctgggatt 1950

acaggcgtga gccactgcac ccagcctaga atcttgtata atatgtaatt 2000

gtagggaaac tgctctcata ggaaagtttt ctgcttttta aatacaaaaa 2050

tacataaaaa tacataaaat ctgatgatga atataaaaaa gtaaccaacc 2100

tcattggaac aagtattaac attttggaat atgttttatt agttttgtga 2150

tgtactgttt tacaattttt accatttttt tcagtaatta ctgtaaaatg 2200

gtattattgg aatgaaacta tatttcctca tgtgctgatt tgtcttattt 2250

ttttcatact ttcccactgg tgctattttt atttccaatg gatatttctg 2300

tattactagg gaggcattta cagtcctcta atgttgatta atatgtgaaa 2350

agaaattgta ccaattttac taaattatgc agtttaaaat ggatgatttt 2400

atgttatgtg gatttcattt caataaaaaa aaactcttat caaaaaaaaa 2450

aaaaaaaaaa aaaaaaaaaa aaaaaaaa 2478

<210> 24
<211> 201
<212> PRT
<213> Homo sapiens

<400> 24
Met Glu Tyr His Pro Asp Leu Glu Asn Leu Asp Glu Asp Gly Tyr
1               5                   10                  15

Thr Gln Leu His Phe Asp Ser Gln Ser Asn Thr Arg Ile Ala Val
                20                  25                  30

Val Ser Glu Lys Gly Ser Cys Ala Ala Ser Pro Pro Trp Arg Leu
                35                  40                  45

Ile Ala Val Ile Leu Gly Ile Leu Cys Leu Val Ile Leu Val Ile
                50                  55                  60

Ala Val Val Leu Gly Thr Met Gly Val Leu Ser Ser Pro Cys Pro
                65                  70                  75

Pro Asn Trp Ile Ile Tyr Glu Lys Ser Cys Tyr Leu Phe Ser Met
                80                  85                  90

Ser Leu Asn Ser Trp Asp Gly Ser Lys Arg Gln Cys Trp Gln Leu
                95                  100                 105

Gly Ser Asn Leu Leu Lys Ile Asp Ser Ser Asn Glu Leu Gly Phe
                110                 115                 120

Ile Val Lys Gln Val Ser Ser Gln Pro Asp Asn Ser Phe Trp Ile
                125                 130                 135

Gly Leu Ser Arg Pro Gln Thr Glu Val Pro Trp Leu Trp Glu Asp
                140                 145                 150

Gly Ser Thr Phe Ser Ser Asn Leu Phe Gln Ile Arg Thr Thr Ala

                155                 160                 165

Thr Gln Glu Asn Pro Ser Pro Asn Cys Val Trp Ile His Val Ser
                170                 175                 180

Val Ile Tyr Asp Gln Leu Cys Ser Val Pro Ser Tyr Ser Ile Cys
                185                 190                 195

Glu Lys Lys Phe Ser Met
                200

<210> 25
<211> 496
<212> DNA
<213> Homo sapiens

<400> 25
cgatgcgcgg acccgggcac cccctcctcc tggggctgct gctggtgctg 50

gggccttcgc cggagcagcg agtggaaatt gttcctcgag atctgaggat 100

gaaggacaag tttctaaaac accttacagg ccctctttat tttagtccaa 150

agtgcagcaa acacttccat agactttatc acaacaccag agactgcacc 200

attcctgcat actataaaag atgcgccagg cttcttaccc ggctggctgt 250

cagtccagtg tgcatggagg ataagtgagc agaccgtaca ggagcagcac 300

accaggagcc atgagaagtg ccttggaaac caacagggaa acagaactat 350

ctttatacac atcccctcat ggacaagaga tttatttttg cagacagact 400

cttccataag tcctttgagt tttgtatgtt gttgacagtt tgcagatata 450

tattcgataa atcagtgtac ttgacagtgt tatctgtcac ttattt 496

<210> 26
<211> 91
<212> PRT
<213> Homo sapiens

<400> 26
Met Arg Gly Pro Gly His Pro Leu Leu Leu Gly Leu Leu Leu Val
1               5                   10                  15

Leu Gly Pro Ser Pro Glu Gln Arg Val Glu Ile Val Pro Arg Asp
                20                  25                  30

Leu Arg Met Lys Asp Lys Phe Leu Lys His Leu Thr Gly Pro Leu
                35                  40                  45

Tyr Phe Ser Pro Lys Cys Ser Lys His Phe His Arg Leu Tyr His
                50                  55                  60

Asn Thr Arg Asp Cys Thr Ile Pro Ala Tyr Tyr Lys Arg Cys Ala
                65                  70                  75

Arg Leu Leu Thr Arg Leu Ala Val Ser Pro Val Cys Met Glu Asp
                80                  85                  90

Lys

<210> 27
<211> 1371
<212> DNA
<213> Homo sapiens

<400> 27
aactcaaact cctctctctg ggaaaacgcg gtgcttgctc ctcccggagt 50

ggccttggca gggtgttgga gccctcggtc tgccccgtcc ggtctctggg 100

gccaaggctg ggtttccctc atgtatggca agagctctac tcgtgcggtg 150

cttcttctcc ttggcataca gctcacagct ctttggccta tagcagctgt 200

ggaaatttat acctcccggg tgctggaggc tgttaatggg acagatgctc 250

ggttaaaatg cactttctcc agctttgccc ctgtgggtga tgctctaaca 300

gtgacctgga attttcgtcc tctagacggg ggacctgagc agtttgtatt 350

ctactaccac atagatccct tccaacccat gagtgggcgg tttaaggacc 400

gggtgtcttg ggatgggaat cctgagcggt acgatgcctc catccttctc 450

tggaaactgc agttcgacga caatgggaca tacacctgcc aggtgaagaa 500

cccacctgat gttgatgggg tgatagggga gatccggctc agcgtcgtgc 550

acactgtacg cttctctgag atccacttcc tggctctggc cattggctct 600

gcctgtgcac tgatgatcat aatagtaatt gtagtggtcc tcttccagca 650

ttaccggaaa aagcgatggg ccgaaagagc tcataaagtg gtggagataa 700

aatcaaaaga agaggaaagg ctcaaccaag agaaaaaggt ctctgtttat 750

ttagaagaca cagactaaca attttagatg gaagctgaga tgatttccaa 800

gaacaagaac cctagtattt cttgaagtta atggaaactt ttctttggct 850

tttccagttg tgacccgttt tccaaccagt tctgcagcat attagattct 900

agacaagcaa cacccctctg gagccagcac agtgctcctc catatcacca 950

gtcatacaca gcctcattat taaggtctta tttaatttca gagtgtaaat 1000

tttttcaagt gctcattagg ttttataaac aagaagctac atttttgccc 1050

ttaagacact acttacagtg ttatgacttg tatacacata tattggtatc 1100

aaaggggata aaagccaatt tgtctgttac atttcctttc acgtatttct 1150

tttagcagca cttctgctac taaagttaat gtgtttactc tctttccttc 1200

ccacattctc aattaaaagg tgagctaagc ctcctcggtg tttctgatta 1250

acagtaaatc ctaaattcaa actgttaaat gacattttta tttttatgtc 1300

tctccttaac tatgagacac atcttgtttt actgaatttc tttcaatatt 1350

ccaggtgata gatttttgtc  g 1371

<210> 28
<211> 215
<212> PRT
<213> Homo sapiens

<400> 28
Met Tyr Gly Lys Ser Ser Thr Arg Ala Val Leu Leu Leu Leu Gly
1               5                   10                  15

Ile Gln Leu Thr Ala Leu Trp Pro Ile Ala Ala Val Glu Ile Tyr
                20                  25                  30

Thr Ser Arg Val Leu Glu Ala Val Asn Gly Thr Asp Ala Arg Leu
                35                  40                  45

Lys Cys Thr Phe Ser Ser Phe Ala Pro Val Gly Asp Ala Leu Thr
                50                  55                  60

Val Thr Trp Asn Phe Arg Pro Leu Asp Gly Gly Pro Glu Gln Phe
                65                  70                  75

Val Phe Tyr Tyr His Ile Asp Pro Phe Gln Pro Met Ser Gly Arg
                80                  85                  90

Phe Lys Asp Arg Val Ser Trp Asp Gly Asn Pro Glu Arg Tyr Asp
                95                  100                 105

Ala Ser Ile Leu Leu Trp Lys Leu Gln Phe Asp Asp Asn Gly Thr
                110                 115                 120

Tyr Thr Cys Gln Val Lys Asn Pro Pro Asp Val Asp Gly Val Ile
                125                 130                 135

Gly Glu Ile Arg Leu Ser Val Val His Thr Val Arg Phe Ser Glu
                140                 145                 150

Ile His Phe Leu Ala Leu Ala Ile Gly Ser Ala Cys Ala Leu Met
                155                 160                 165

Ile Ile Ile Val Ile Val Val Val Leu Phe Gln His Tyr Arg Lys
                170                 175                 180

Lys Arg Trp Ala Glu Arg Ala His Lys Val Val Glu Ile Lys Ser
                185                 190                 195

Lys Glu Glu Glu Arg Leu Asn Gln Glu Lys Lys Val Ser Val Tyr
                200                 205                 210

Leu Glu Asp Thr Asp
                215

<210> 29
<211> 759
<212> DNA
<213> Homo sapiens

<400> 29
ctagatttgt cggcttgcgg ggagacttca ggagtcgctg tctctgaact 50

tccagcctca gagaccgccg cccttgtccc cgagggccat gggccgggtc 100

tcagggcttg tgccctctcg cttcctgacg ctcctggcgc atctggtggt 150

cgtcatcacc ttattctggt cccgggacag caacatacag gcctgcctgc 200

ctctcacgtt cacccccgag gagtatgaca agcaggacat tcagctggtg 250

gccgcgctct ctgtcaccct gggcctcttt gcagtggagc tggccggttt 300

cctctcagga gtctccatgt tcaacagcac ccagagcctc atctccattg 350

gggctcactg tagtgcatcc gtggccctgt ccttcttcat attcgagcgt 400

tgggagtgca ctacgtattg gtacattttt gtcttctgca gtgcccttcc 450

agctgtcact gaaatggctt tattcgtcac cgtctttggg ctgaaaaaga 500

aacccttctg attaccttca tgacgggaac ctaaggacga agcctacagg 550

ggcaagggcc gcttcgtatt cctggaagaa ggaaggcata ggcttcggtt 600

ttcccctcgg aaactgcttc tgctggagga tatgtgttgg aataattacg 650

tcttgagtct gggattatcc gcattgtatt tagtgctttg taataaaata 700

tgttttgtag taacattaag acttatatac agttttaggg gacaattaaa 750

aaaaaaaaa 759

<210> 30
<211> 140
<212> PRT
<213> Homo sapiens

<400> 30
Met Gly Arg Val Ser Gly Leu Val Pro Ser Arg Phe Leu Thr Leu
1               5                   10                  15

Leu Ala His Leu Val Val Val Ile Thr Leu Phe Trp Ser Arg Asp
                20                  25                  30

Ser Asn Ile Gln Ala Cys Leu Pro Leu Thr Phe Thr Pro Glu Glu
                35                  40                  45

Tyr Asp Lys Gln Asp Ile Gln Leu Val Ala Ala Leu Ser Val Thr
                50                  55                  60

Leu Gly Leu Phe Ala Val Glu Leu Ala Gly Phe Leu Ser Gly Val
                65                  70                  75

Ser Met Phe Asn Ser Thr Gln Ser Leu Ile Ser Ile Gly Ala His
                80                  85                  90

Cys Ser Ala Ser Val Ala Leu Ser Phe Phe Ile Phe Glu Arg Trp
                95                  100                 105

Glu Cys Thr Thr Tyr Trp Tyr Ile Phe Val Phe Cys Ser Ala Leu
                110                 115                 120

Pro Ala Val Thr Glu Met Ala Leu Phe Val Thr Val Phe Gly Leu
                125                 130                 135

Lys Lys Lys Pro Phe
                140

<210> 31
<211> 753
<212> DNA
<213> Homo sapiens

<400> 31
ggatgcagga cgctcccctg agctgcctgt caccgactag gtggagcagt 50

gtttcttccg cagactcaac tgagaagtca gcctctgggg caggcaccag 100

gaatctgcct tttcagttct gtctccggca ggctttgagg atgaaggctg 150

cgggcattct gaccctcatt ggctgcctgg tcacaggcgc cgagtccaaa 200

atctacactc gttgcaaact ggcaaaaata ttctcgaggg ctggcctgga 250

caattactgg ggcttcagcc ttggaaactg gatctgcatg gcatattatg 300

agagcggcta caacaccaca gccccgacgg tcctggatga cggcagcatc 350

gactatggca tcttccagat caacagcttc gcgtggtgca gacgcggaaa 400

gctgaaggag aacaaccact gccatgtcgc ctgctcagcc ttgatcactg 450

atgacctcac agatgcaatt atctgtgcca ggaaaattgt taaagagaca 500

caaggaatga actattggca aggctggaag aaacattgtg agggcagaga 550

cctgtccgag tggaaaaaag gctgtgaggt ttcctaaact ggaactggac 600

ccaggatgct ttgcagcaac gccctaggat ttgcagtgaa tgtccaaatg 650

cctgtgtcat cttgtcccgt ttcctcccaa tattccttct caaacttgga 700

gagggaaaat taagctatac ttttaagaaa ataaatattt ccatttaaat 750

gtc 753

<210> 32

<211> 148
<212> PRT
<213> Homo sapiens

<400> 32
Met Lys Ala Ala Gly Ile Leu Thr Leu Ile Gly Cys Leu Val Thr
1               5                   10                  15

Gly Ala Glu Ser Lys Ile Tyr Thr Arg Cys Lys Leu Ala Lys Ile
                20                  25                  30

Phe Ser Arg Ala Gly Leu Asp Asn Tyr Trp Gly Phe Ser Leu Gly
                35                  40                  45

Asn Trp Ile Cys Met Ala Tyr Tyr Glu Ser Gly Tyr Asn Thr Thr
                50                  55                  60

Ala Pro Thr Val Leu Asp Asp Gly Ser Ile Asp Tyr Gly Ile Phe
                65                  70                  75

Gln Ile Asn Ser Phe Ala Trp Cys Arg Arg Gly Lys Leu Lys Glu
                80                  85                  90

Asn Asn His Cys His Val Ala Cys Ser Ala Leu Ile Thr Asp Asp
                95                  100                 105

Leu Thr Asp Ala Ile Ile Cys Ala Arg Lys Ile Val Lys Glu Thr
                110                 115                 120

Gln Gly Met Asn Tyr Trp Gln Gly Trp Lys Lys His Cys Glu Gly
                125                 130                 135

Arg Asp Leu Ser Glu Trp Lys Lys Gly Cys Glu Val Ser
                140                 145

<210> 33
<211> 1091
<212> DNA
<213> Homo sapiens

<400> 33
caagcaggtc atccccttgg tgaccttcaa agagaagcag agagggcaga 50

ggtggggggc acagggaaag ggtgacctct gagattcccc ttttccccca 100

gactttggaa gtgacccacc atggggctca gcatcttttt gctcctgtgt 150

gttcttgggc tcagccaggc agccacaccg aagattttca atggcactga 200

gtgtgggcgt aactcacagc cgtggcaggt ggggctgttt gagggcacca 250

gcctgcgctg cgggggtgtc cttattgacc acaggtgggt cctcacagcg 300

gctcactgca gcggcagcag gtactgggtg cgcctggggg aacacagcct 350

cagccagctc gactggaccg agcagatccg gcacagcggc ttctctgtga 400

cccatcccgg ctacctggga gcctcgacga gccacgagca cgacctccgg 450

ctgctgcggc tgcgcctgcc cgtccgcgta accagcagcg ttcaacccct 500

gcccctgccc aatgactgtg caaccgctgg caccgagtgc cacgtctcag 550

gctggggcat caccaaccac ccacggaacc cattcccgga tctgctccag 600

tgcctcaacc tctccatcgt ctcccatgcc acctgccatg gtgtgtatcc 650

cgggagaatc acgagcaaca tggtgtgtgc aggcggcgtc ccggggcagg 700

atgcctgcca gggtgattct gggggccccc tggtgtgtgg gggagtcctt 750

caaggtctgg tgtcctgggg gtctgtgggg ccctgtggac aagatggcat 800

ccctggagtc tacacctata tttgcaagta tgtggactgg atccggatga 850

tcatgaggaa caactgacct gtttcctcca cctccacccc caccccttaa 900

cttgggtacc cctctggccc tcagagcacc aatatctcct ccatcacttc 950

ccctagctcc actcttgttg gcctgggaac ttcttggaac tttaactcct 1000

gccagccctt ctaagaccca cgagcggggt gagagaagtg tgcaatagtc 1050

tggaataaat ataaatgaag gaggggcaaa aaaaaaaaaa a 1091

<210> 34
<211> 248
<212> PRT
<213> Homo sapiens

<400> 34
Met Gly Leu Ser Ile Phe Leu Leu Leu Cys Val Leu Gly Leu Ser
1               5                   10                  15

Gln Ala Ala Thr Pro Lys Ile Phe Asn Gly Thr Glu Cys Gly Arg
                20                  25                  30

Asn Ser Gln Pro Trp Gln Val Gly Leu Phe Glu Gly Thr Ser Leu
                35                  40                  45

Arg Cys Gly Gly Val Leu Ile Asp His Arg Trp Val Leu Thr Ala
                50                  55                  60

Ala His Cys Ser Gly Ser Arg Tyr Trp Val Arg Leu Gly Glu His
                65                  70                  75

Ser Leu Ser Gln Leu Asp Trp Thr Glu Gln Ile Arg His Ser Gly
                80                  85                  90

Phe Ser Val Thr His Pro Gly Tyr Leu Gly Ala Ser Thr Ser His
                95                  100                 105

Glu His Asp Leu Arg Leu Leu Arg Leu Arg Leu Pro Val Arg Val
                110                 115                 120

Thr Ser Ser Val Gln Pro Leu Pro Leu Pro Asn Asp Cys Ala Thr

                125                 130                 135

Ala Gly Thr Glu Cys His Val Ser Gly Trp Gly Ile Thr Asn His
                140                 145                 150

Pro Arg Asn Pro Phe Pro Asp Leu Leu Gln Cys Leu Asn Leu Ser
                155                 160                 165

Ile Val Ser His Ala Thr Cys His Gly Val Tyr Pro Gly Arg Ile
                170                 175                 180

Thr Ser Asn Met Val Cys Ala Gly Gly Val Pro Gly Gln Asp Ala
                185                 190                 195

Cys Gln Gly Asp Ser Gly Gly Pro Leu Val Cys Gly Gly Val Leu
                200                 205                 210

Gln Gly Leu Val Ser Trp Gly Ser Val Gly Pro Cys Gly Gln Asp
                215                 220                 225

Gly Ile Pro Gly Val Tyr Thr Tyr Ile Cys Lys Tyr Val Asp Trp
                230                 235                 240

Ile Arg Met Ile Met Arg Asn Asn
                245

<210> 35
<211> 2498
<212> DNA
<213> Homo sapiens

<400> 35
cgtctctgcg ttcgccatgc gtcccggggc gccagggcca ctctggcctc 50

tgccctgggg ggccctggct tgggccgtgg gcttcgtgag ctccatgggc 100

tcggggaacc ccgcgcccgg tggtgtttgc tggctccagc agggccagga 150

ggccacctgc agcctggtgc tccagactga tgtcacccgg gccgagtgct 200

gtgcctccgg caacattgac accgcctggt ccaacctcac ccacccgggg 250

aacaagatca acctcctcgg cttcttgggc cttgtccact gccttccctg 300

caaagattcg tgcgacggcg tggagtgcgg cccgggcaag gcgtgccgca 350

tgctgggggg ccgcccgcgc tgcgagtgcg cgcccgactg ctcggggctc 400

ccggcgcggc tgcaggtctg cggctcagac ggcgccacct accgcgacga 450

gtgcgagctg cgcgccgcgc gctgccgcgg ccacccggac ctgagcgtca 500

tgtaccgggg ccgctgccgc aagtcctgtg agcacgtggt gtgcccgcgg 550

ccacagtcgt gcgtcgtgga ccagacgggc agcgcccact gcgtggtgtg 600

tcgagcggcg ccctgccctg tgccctccag ccccggccag gagctttgcg 650

gcaacaacaa cgtcacctac atctcctcgt gccacatgcg ccaggccacc 700

tgcttcctgg gccgctccat cggcgtgcgc cacgcgggca gctgcgcagg 750

cacccctgag gagccgccag gtggtgagtc tgcagaagag gaagagaact 800

tcgtgtgagc ctgcaggaca ggcctgggcc tggtgcccga ggccccccat 850

catcccctgt tatttattgc cacagcagag tctaatttat atgccacgga 900

cactccttag agcccggatt cggaccactt ggggatccca gaacctccct 950

gacgatatcc tggaaggact gaggaaggga ggcctggggg ccggctggtg 1000

ggtgggatag acctgcgttc cggacactga gcgcctgatt tagggccctt 1050

ctctaggatg ccccagcccc taccctaaga cctattgccg gggaggattc 1100

cacacttccg ctcctttggg gataaaccta ttaattattg ctactatcaa 1150

gagggctggg cattctctgc tggtaattcc tgaagaggca tgactgcttt 1200

tctcagcccc aagcctctag tctgggtgtg tacggagggt ctagcctggg 1250

tgtgtacgga gggtctagcc tgggtgagta cggagggtct agcctgggtg 1300

agtacggagg gtctagcctg ggtgagtacg gagggtctag cctgggtgtg 1350

tatggaggat ctagcctggg tgagtatgga gggtctagcc tgggtgagta 1400

tggagggtct agcctgggtg tgtatggagg gtctagcctg ggtgagtatg 1450

gagggtctag cctgggtgtg tatggagggt ctagcctggg tgagtatgga 1500

gggtctagcc tgggtgtgta cggagggtct agtctgagtg cgtgtgggga 1550

cctcagaaca ctgtgacctt agcccagcaa gccaggccct tcatgaaggc 1600

caagaaggct gccaccattc cctgccagcc caagaactcc agcttcccca 1650

ctgcctctgt gtgccccttt gcgtcctgtg aaggccattg agaaatgccc 1700

agtgtgcccc ctgggaaagg gcacggcctg tgctcctgac acgggctgtg 1750

cttggccaca gaaccaccca gcgtctcccc tgctgctgtc cacgtcagtt 1800

catgaggcaa cgtcgcgtgg tctcagacgt ggagcagcca gcggcagctc 1850

agagcagggc actgtgtccg gcggagccaa gtccactctg ggggagctct 1900

ggcggggacc acgggccact gctcacccac tggccccgag gggggtgtag 1950

acgccaagac tcacgcatgt gtgacatccg gagtcctgga gccgggtgtc 2000

ccagtggcac cactaggtgc ctgctgcctc cacagtgggg ttcacaccca 2050

gggctccttg gtcccccaca acctgccccg gccaggcctg cagacccaga 2100

ctccagccag acctgcctca cccaccaatg cagccggggc tggcgacacc 2150

agccaggtgc tggtcttggg ccagttctcc cacgacggct caccctcccc 2200

tccatctgcg ttgatgctca gaatcgccta cctgtgcctg cgtgtaaacc 2250

acagcctcag accagctatg gggagaggac aacacggagg atatccagct 2300

tccccggtct ggggtgagga atgtggggag cttgggcatc ctcctccagc 2350

ctcctccagc ccccaggcag tgccttacct gtggtgccca gaaaagtgcc 2400

cctaggttgg tgggtctaca ggagcctcag ccaggcagcc caccccaccc 2450

tggggccctg cctcaccaag gaaataaaga ctcaagccat aaaaaaaa 2498

<210> 36
<211> 263
<212> PRT
<213> Homo sapiens

<400> 36
Met Arg Pro Gly Ala Pro Gly Pro Leu Trp Pro Leu Pro Trp Gly
1               5                   10                  15

Ala Leu Ala Trp Ala Val Gly Phe Val Ser Ser Met Gly Ser Gly
                20                  25                  30

Asn Pro Ala Pro Gly Gly Val Cys Trp Leu Gln Gln Gly Gln Glu
                35                  40                  45

Ala Thr Cys Ser Leu Val Leu Gln Thr Asp Val Thr Arg Ala Glu
                50                  55                  60

Cys Cys Ala Ser Gly Asn Ile Asp Thr Ala Trp Ser Asn Leu Thr
                65                  70                  75

His Pro Gly Asn Lys Ile Asn Leu Leu Gly Phe Leu Gly Leu Val
                80                  85                  90

His Cys Leu Pro Cys Lys Asp Ser Cys Asp Gly Val Glu Cys Gly
                95                  100                 105

Pro Gly Lys Ala Cys Arg Met Leu Gly Gly Arg Pro Arg Cys Glu
                110                 115                 120

Cys Ala Pro Asp Cys Ser Gly Leu Pro Ala Arg Leu Gln Val Cys
                125                 130                 135

Gly Ser Asp Gly Ala Thr Tyr Arg Asp Glu Cys Glu Leu Arg Ala
                140                 145                 150

Ala Arg Cys Arg Gly His Pro Asp Leu Ser Val Met Tyr Arg Gly
                155                 160                 165

Arg Cys Arg Lys Ser Cys Glu His Val Val Cys Pro Arg Pro Gln
                170                 175                 180

Ser Cys Val Val Asp Gln Thr Gly Ser Ala His Cys Val Val Cys
                185                 190                 195

Arg Ala Ala Pro Cys Pro Val Pro Ser Ser Pro Gly Gln Glu Leu
                200                 205                 210

Cys Gly Asn Asn Asn Val Thr Tyr Ile Ser Ser Cys His Met Arg
                215                 220                 225

Gln Ala Thr Cys Phe Leu Gly Arg Ser Ile Gly Val Arg His Ala
                230                 235                 240

Gly Ser Cys Ala Gly Thr Pro Glu Glu Pro Pro Gly Gly Glu Ser
                245                 250                 255

Ala Glu Glu Glu Glu Asn Phe Val
                260

<210> 37
<211> 2668
<212> DNA
<213> Homo sapiens

<400> 37
gactttgctt gaatgtttac attttctgct cgctgtccta catatcacaa 50

tatagtgttc acgttttgtt aaaactttgg ggtgtcagga gttgagcttg 100

ctcagcaagc cagcatggct aggatgagct ttgttatagc agcttgccaa 150

ttggtgctgg gcctactaat gacttcatta accgagtctt ccatacagaa 200

tagtgagtgt ccacaacttt gcgtatgtga aattcgtccc tggtttaccc 250

cacagtcaac ttacagagaa gccaccactg ttgattgcaa tgacctccgc 300

ttaacaagga ttcccagtaa cctctctagt gacacacaag tgcttctctt 350

acagagcaat aacatcgcga agactgtgga tgagctgcag cagcttttca 400

acttgactga actagatttc tcccaaaaca actttactaa cattaaggag 450

gtcgggctgg caaacctaac ccagctcaca acgctgcatt tggaggaaaa 500

tcagattacc gagatgactg attactgtct acaagacctc agcaaccttc 550

aagaactcta catcaaccac aaccaaatta gcactatttc tgctcatgct 600

tttgcaggct taaaaaatct attaaggctc cacctgaact ccaacaaatt 650

gaaagttatt gatagtcgct ggtttgattc tacacccaac ctggaaattc 700

tcatgatcgg agaaaaccct gtgattggaa ttctggatat gaacttcaaa 750

cccctcgcaa atttgagaag cttagttttg gcaggaatgt atctcactga 800

tattcctgga aatgctttgg tgggtctgga tagccttgag agcctgtctt 850

tttatgataa caaactggtt aaagtccctc aacttgccct gcaaaaagtt 900
ccaaatttga aattcttaga cctcaacaaa aaccccattc acaaaatcca 950
agaaggggac ttcaaaaata tgcttcggtt aaaagaactg ggaatcaaca 1000
atatgggcga gctcgtttct gtcgaccgct atgccctgga taacttgcct 1050
gaactcacaa agctggaagc caccaataac cctaaactct cttacatcca 1100
ccgcttggct ttccgaagtg tccctgctct ggaaagcttg atgctgaaca 1150
acaatgcctt gaatgccatt taccaaaaga cagtcgaatc cctccccaat 1200
ctgcgtgaga tcagtatcca tagcaatccc ctcaggtgtg actgtgtgat 1250
ccactggatt aactccaaca aaaccaacat ccgcttcatg gagcccctgt 1300
ccatgttctg tgccatgccg cccgaatata aagggcacca ggtgaaggaa 1350
gttttaatcc aggattcgag tgaacagtgc ctcccaatga tatctcacga 1400
cagcttccca aatcgtttaa acgtggatat cggcacgacg gttttcctag 1450
actgtcgagc catggctgag ccagaacctg aaatttactg ggtcactccc 1500
attggaaata agataactgt ggaaaccctt tcagataaat acaagctaag 1550
tagcgaaggt accttggaaa tatctaacat acaaattgaa gactcaggaa 1600
gatacacatg tgttgcccag aatgtccaag ggcagacact tcgggtggca 1650
acaattaagg ttaacgggac ccttctggat ggtacccagg tgctaaaaat 1700
atacgtcaag cagacagaat cccattccat cttagtgtcc tggaaagtta 1750
attccaatgt catgacgtca aacttaaaat ggtcgtctgc caccatgaag 1800
attgataacc ctcacataac atatactgcc agggtcccag tcgatgtcca 1850
tgaatacaac ctaacgcatc tgcagccttc cacagattat gaagtgtgtc 1900
tcacagtgtc caatattcat cagcagactc aaaagtcatg cgtaaatgtc 1950
acaaccaaaa atgccgcctt cgcagtggac atctctgatc aagaaaccag 2000
tacagccctt gctgcagtaa tggggtctat gtttgccgtc attagccttg 2050
cgtccattgc tgtgtacttt gccaaaagat ttaagagaaa aaactaccac 2100
cactcattaa aaaagtatat gcaaaaaacc tcttcaatcc cactaaatga 2150
gctgtaccca ccactcatta acctctggga aggtgacagc gagaaagaca 2200
aagatggttc tgcagacacc aagccaaccc aggtcgacac atccagaagc 2250
tattacatgt ggtaactcag aggatatttt gcttctggta gtaaggagca 2300

caaagacgtt tttgctttat tctgcaaaag tgaacaagtt gaagactttt 2350

gtatttttga ctttgctagt ttgtggcaga gtggagagga cgggtggata 2400

tttcaaattt ttttagtata gcgtatcgca agggtttgac acggctgcca 2450

gcgactctag gcttccagtc tgtgtttggt ttttattctt atcattatta 2500

tgattgttat tatattatta ttttatttta gttgttgtgc taaactcaat 2550

aatgctgttc taactacagt gctcaataaa atgattaatg acaggaaaaa 2600

aaaaaaaaaa aaaaaaaaaa aaaaaaaaaa aaaaaaaaaa aaaaaaaaaa 2650

aaaaaaaaaa  aaaaaaaa 2668

<210> 38
<211> 716
<212> PRT
<213> Homo sapiens

<400> 38
Met Ala Arg Met Ser Phe Val Ile Ala Ala Cys Gln Leu Val Leu
1               5                   10                  15

Gly Leu Leu Met Thr Ser Leu Thr Glu Ser Ser Ile Gln Asn Ser
                20                  25                  30

Glu Cys Pro Gln Leu Cys Val Cys Glu Ile Arg Pro Trp Phe Thr
                35                  40                  45

Pro Gln Ser Thr Tyr Arg Glu Ala Thr Thr Val Asp Cys Asn Asp
                50                  55                  60

Leu Arg Leu Thr Arg Ile Pro Ser Asn Leu Ser Ser Asp Thr Gln
                65                  70                  75

Val Leu Leu Leu Gln Ser Asn Asn Ile Ala Lys Thr Val Asp Glu
                80                  85                  90

Leu Gln Gln Leu Phe Asn Leu Thr Glu Leu Asp Phe Ser Gln Asn
                95                  100                 105

Asn Phe Thr Asn Ile Lys Glu Val Gly Leu Ala Asn Leu Thr Gln
                110                 115                 120

Leu Thr Thr Leu His Leu Glu Glu Asn Gln Ile Thr Glu Met Thr
                125                 130                 135

Asp Tyr Cys Leu Gln Asp Leu Ser Asn Leu Gln Glu Leu Tyr Ile
                140                 145                 150

Asn His Asn Gln Ile Ser Thr Ile Ser Ala His Ala Phe Ala Gly
                155                 160                 165

Leu Lys Asn Leu Leu Arg Leu His Leu Asn Ser Asn Lys Leu Lys
                170                 175                 180

Val Ile Asp Ser Arg Trp Phe Asp Ser Thr Pro Asn Leu Glu Ile
                185                 190                 195

Leu Met Ile Gly Glu Asn Pro Val Ile Gly Ile Leu Asp Met Asn
                200                 205                 210

Phe Lys Pro Leu Ala Asn Leu Arg Ser Leu Val Leu Ala Gly Met
                215                 220                 225

Tyr Leu Thr Asp Ile Pro Gly Asn Ala Leu Val Gly Leu Asp Ser
                230                 235                 240

Leu Glu Ser Leu Ser Phe Tyr Asp Asn Lys Leu Val Lys Val Pro
                245                 250                 255

Gln Leu Ala Leu Gln Lys Val Pro Asn Leu Lys Phe Leu Asp Leu
                260                 265                 270

Asn Lys Asn Pro Ile His Lys Ile Gln Glu Gly Asp Phe Lys Asn
                275                 280                 285

Met Leu Arg Leu Lys Glu Leu Gly Ile Asn Asn Met Gly Glu Leu
                290                 295                 300

Val Ser Val Asp Arg Tyr Ala Leu Asp Asn Leu Pro Glu Leu Thr
                305                 310                 315

Lys Leu Glu Ala Thr Asn Asn Pro Lys Leu Ser Tyr Ile His Arg
                320                 325                 330

Leu Ala Phe Arg Ser Val Pro Ala Leu Glu Ser Leu Met Leu Asn
                335                 340                 345

Asn Asn Ala Leu Asn Ala Ile Tyr Gln Lys Thr Val Glu Ser Leu
                350                 355                 360

Pro Asn Leu Arg Glu Ile Ser Ile His Ser Asn Pro Leu Arg Cys
                365                 370                 375

Asp Cys Val Ile His Trp Ile Asn Ser Asn Lys Thr Asn Ile Arg
                380                 385                 390

Phe Met Glu Pro Leu Ser Met Phe Cys Ala Met Pro Pro Glu Tyr
                395                 400                 405

Lys Gly His Gln Val Lys Glu Val Leu Ile Gln Asp Ser Ser Glu
                410                 415                 420

Gln Cys Leu Pro Met Ile Ser His Asp Ser Phe Pro Asn Arg Leu
                425                 430                 435

Asn Val Asp Ile Gly Thr Thr Val Phe Leu Asp Cys Arg Ala Met
                440                 445                 450

Ala Glu Pro Glu Pro Glu Ile Tyr Trp Val Thr Pro Ile Gly Asn
                455                 460                 465

Lys Ile Thr Val Glu Thr Leu Ser Asp Lys Tyr Lys Leu Ser Ser

                470                 475                 480

Glu Gly Thr Leu Glu Ile Ser Asn Ile Gln Ile Glu Asp Ser Gly
                485                 490                 495

Arg Tyr Thr Cys Val Ala Gln Asn Val Gln Gly Ala Asp Thr Arg
                500                 505                 510

Val Ala Thr Ile Lys Val Asn Gly Thr Leu Leu Asp Gly Thr Gln
                515                 520                 525

Val Leu Lys Ile Tyr Val Lys Gln Thr Glu Ser His Ser Ile Leu
                530                 535                 540

Val Ser Trp Lys Val Asn Ser Asn Val Met Thr Ser Asn Leu Lys
                545                 550                 555

Trp Ser Ser Ala Thr Met Lys Ile Asp Asn Pro His Ile Thr Tyr
                560                 565                 570

Thr Ala Arg Val Pro Val Asp Val His Glu Tyr Asn Leu Thr His
                575                 580                 585

Leu Gln Pro Ser Thr Asp Tyr Glu Val Cys Leu Thr Val Ser Asn
                590                 595                 600

Ile His Gln Gln Thr Gln Lys Ser Cys Val Asn Val Thr Thr Lys
                605                 610                 615

Asn Ala Ala Phe Ala Val Asp Ile Ser Asp Gln Glu Thr Ser Thr
                620                 625                 630

Ala Leu Ala Ala Val Met Gly Ser Met Phe Ala Val Ile Ser Leu
                635                 640                 645

Ala Ser Ile Ala Val Tyr Phe Ala Lys Arg Phe Lys Arg Lys Asn
                650                 655                 660

Tyr His His Ser Leu Lys Lys Tyr Met Gln Lys Thr Ser Ser Ile
                665                 670                 675

Pro Leu Asn Glu Leu Tyr Pro Pro Leu Ile Asn Leu Trp Glu Gly
                680                 685                 690

Asp Ser Glu Lys Asp Lys Asp Gly Ser Ala Asp Thr Lys Pro Thr
                695                 700                 705

Gln Val Asp Thr Ser Arg Ser Tyr Tyr Met Trp
                710                 715

<210> 39
<211> 3680
<212> DNA
<213> Homo sapiens

<400> 39
gaacgtgcca ccatgcccag ctaatttttg tatttttagt agagacgggg 50

tttcaccatg ttggccaggc tggtcttgaa ctcgtgacct catgatccgc 100
tcacctcggc ctcccaaagt gctgggatta caggcatgag ccactgacgc 150
ctggccagcc tatgcatttt taagaaatta ttctgtatta ggtgctgtgc 200
taaacattgg gcactacagt gaccaaaaca gactgaattc cccaagagcc 250
aaagaccagt gagggagacc aacaagaaac aggaaatgca aaagagacca 300
ttattactca ctatgactaa gggtcacaaa tggggtacgt tgatggagag 350
tgatttgtta agagactaca gagggaggac agactaccaa gaggggggcc 400
aggaaagctc ctctgacgag gtggtatttc agcccaaact ggaagaatga 450
gaaagagcta gccagccatc agaatagtcc agaagagatg gggagcacta 500
cactcactac actttggcct gagaaaatag catgggattg gaggaggctg 550
ggggaacacc acttctgccg acctgggcag gaggcattga gggcttgaga 600
aagggcaatg gcagtagcag tagaaaggac agggtaggag cagggacttt 650
gcaggtggaa tcattaggtc ttatcaacag atatgggcaa gcaaagccag 700
gggagaattg atggtaatgc tgaggtttgg agccaggcta gatgggacag 750
tggtgggtga tgcaaaggaa agaggtcagg aagcagggcc agacgtgggg 800
agaaggtgtg ggggtttggt ttccatcttg ccgagtctgc cggaatgtgg 850
atgggaagac caagaggagg agcaaggggc agaggggaag ggaatcttaa 900
agaagtcctg gatgccacac tcttcttcct tcctcctctt ccctctcctc 950
agaggtctca ctcgtggttc ttcatttcct gccctgcctc catctcctct 1000
gggtgctggg aaagtggagg attagctgaa gttttgcttc tcggggcctg 1050
tctgaatctc cattgctttc tgggaggaca taattcacct gtcctagctt 1100
cttatcatct tacatttccc tgtagccact gggacatatg tggtgttcct 1150
tcctagctcc tgtctcctcc tcatgccttt gctgggtatg ggcatgttag 1200
ggggaaggtc attgctgtca gaggggcact gactttctaa tggtgttacc 1250
caaggtgaat gttggagaca cagtcgcgat gctgcccaag tcccggcgag 1300
ccctaactat ccaggagatc gctgcgctgg ccaggtcctc cctgcatggt 1350
atgcagcccc tcccatgttt ctggccactt tgtcctttct cctcccgttt 1400
gcacatccct ttggaactgt ttcctgtgag tacatgctgg ggtctcccct 1450
ttcttccctt gctcaggtga atctcagccc cttctcccac ccaaaggttc 1500

acatggatcc taactactgc cacccttcca cctccctgca cctgtgctcc 1550

ctggcctggt cctttaccag gcttctccac cctcccctat ctccaggtat 1600

ttcccaggtg gtgaaggacc acgtgaccaa gcctaccgcc atggcccagg 1650

gccgagtggc tcacctcatt gagtggaagg gctggagcaa gccgagtgac 1700

tcacctgctg ccctggaatc agccttttcc tcctattcag acctcagcga 1750

gggcgaacaa gaggctcgct ttgcagcagg agtggctgag cagtttgcca 1800

tcgcggaagc caagctccga gcatggtctt cggtggatgg cgaggactcc 1850

actgatgact cctatgatga ggactttgct gggggaatgg acacagacat 1900

ggctgggcag ctgcccctgg ggccgcacct ccaggacctg ttcaccggcc 1950

accggttctc ccggcctgtg cgccagggct ccgtggagcc tgagagcgac 2000

tgctcacaga ccgtgtcccc agacaccctg tgctctagtc tgtgcagcct 2050

ggaggatggg ttgttgggct ccccggcccg gctggcctcc cagctgctgg 2100

gcgatgagct gcttctcgcc aaactgcccc ccagccggga aagtgccttc 2150

cgcagcctgg gcccactgga ggcccaggac tcactctaca actcgcccct 2200

cacagagtcc tgcctttccc ccgcggagga ggagccagcc ccctgcaagg 2250

actgccagcc actctgccca ccactaacgg gcagctggga acggcagcgg 2300

caagcctctg acctggcctc ttctggggtg gtgtccttag atgaggatga 2350

ggcagagcca gaggaacagt gacccacatc atgcctggca gtggcatgca 2400

tcccccggct gctgccaggg gcagagcctc tgtgcccaag tgtgggctca 2450

aggctcccag cagagctcca cagcctagag ggctcctggg agcgctcgct 2500

tctccgttgt gtgttttgca tgaaagtgtt tggagaggag gcaggggctg 2550

ggctgggggc gcatgtcctg cccccactcc cggggcttgc cgggggttgc 2600

ccggggcctc tggggcatgg ctacagctgt ggcagacagt gatgttcatg 2650

ttcttaaaat gccacacaca catttcctcc tcggataatg tgaaccacta 2700

agggggttgt gactgggctg tgtgagggtg gggtgggagg gggcccagca 2750

accccccacc ctccccatgc ctctctcttc tctgcttttc ttctcacttc 2800

cgagtccatg tgcagtgctt gatagaatca cccccacctg gaggggctgg 2850

ctcctgccct cccggagcct atgggttgag ccgtccctca agggcccctg 2900

cccagctggg ctcgtgctgt gcttcattca cctctccatc gtctctaaat 2950

cttcctcttt tttcctaaag acagaaggtt tttggtctgt tttttcagtc 3000

ggatcttctc ttctctggga ggctttggaa tgatgaaagc atgtaccctc 3050

cacccttttc ctggccccct aatggggcct gggccctttc ccaacccctc 3100

ctaggatgtg cgggcagtgt gctggcgcct cacagccagc cgggctgccc 3150

attcacgcag agctctctga gcgggaggtg gaagaaagga tggctctggt 3200

tgccacagag ctgggacttc atgttcttct agagagggcc acaagagggc 3250

cacaggggtg gccgggagtt gtcagctgat gcctgctgag aggcaggaat 3300

tgtgccagtg agtgacagtc atgagggagt gtctcttctt ggggaggaaa 3350

gaaggtagag cctttctgtc tgaatgaaag gccaaggcta cagtacaggg 3400

ccccgcccca gccagggtgt taatgcccac gtagtggagg cctctggcag 3450

atcctgcatt ccaaggtcac tggactgtac gtttttatgg ttgtgggaag 3500

ggtgggtggc tttagaatta agggccttgt aggctttggc aggtaagagg 3550

gcccaaggta agaacgagag ccaacgggca caagcattct atatataagt 3600

ggctcattag gtgtttattt tgttctattt aagaatttgt tttattaaat 3650

taatataaaa atctttgtaa atctctaaaa 3680

<210> 40
<211> 335
<212> PRT
<213> Homo sapiens

<400> 40
Met Phe Leu Ala Thr Leu Ser Phe Leu Leu Pro Phe Ala His Pro
1               5                   10                  15

Phe Gly Thr Val Ser Cys Glu Tyr Met Leu Gly Ser Pro Leu Ser
                20                  25                  30

Ser Leu Ala Gln Val Asn Leu Ser Pro Phe Ser His Pro Lys Val
                35                  40                  45

His Met Asp Pro Asn Tyr Cys His Pro Ser Thr Ser Leu His Leu
                50                  55                  60

Cys Ser Leu Ala Trp Ser Phe Thr Arg Leu Leu His Pro Pro Leu
                65                  70                  75

Ser Pro Gly Ile Ser Gln Val Val Lys Asp His Val Thr Lys Pro
                80                  85                  90

Thr Ala Met Ala Gln Gly Arg Val Ala His Leu Ile Glu Trp Lys
                95                  100                 105

Gly Trp Ser Lys Pro Ser Asp Ser Pro Ala Ala Leu Glu Ser Ala

                110                 115                 120

Phe Ser Ser Tyr Ser Asp Leu Ser Glu Gly Glu Gln Glu Ala Arg
                125                 130                 135

Phe Ala Ala Gly Val Ala Glu Gln Phe Ala Ile Ala Glu Ala Lys
                140                 145                 150

Leu Arg Ala Trp Ser Ser Val Asp Gly Glu Asp Ser Thr Asp Asp
                155                 160                 165

Ser Tyr Asp Glu Asp Phe Ala Gly Gly Met Asp Thr Asp Met Ala
                170                 175                 180

Gly Gln Leu Pro Leu Gly Pro His Leu Gln Asp Leu Phe Thr Gly
                185                 190                 195

His Arg Phe Ser Arg Pro Val Arg Gln Gly Ser Val Glu Pro Glu
                200                 205                 210

Ser Asp Cys Ser Gln Thr Val Ser Pro Asp Thr Leu Cys Ser Ser
                215                 220                 225

Leu Cys Ser Leu Glu Asp Gly Leu Leu Gly Ser Pro Ala Arg Leu
                230                 235                 240

Ala Ser Gln Leu Leu Gly Asp Glu Leu Leu Leu Ala Lys Leu Pro
                245                 250                 255

Pro Ser Arg Glu Ser Ala Phe Arg Ser Leu Gly Pro Leu Glu Ala
                260                 265                 270

Gln Asp Ser Leu Tyr Asn Ser Pro Leu Thr Glu Ser Cys Leu Ser
                275                 280                 285

Pro Ala Glu Glu Glu Pro Ala Pro Cys Lys Asp Cys Gln Pro Leu
                290                 295                 300

Cys Pro Pro Leu Thr Gly Ser Trp Glu Arg Gln Arg Gln Ala Ser
                305                 310                 315

Asp Leu Ala Ser Ser Gly Val Val Ser Leu Asp Glu Asp Glu Ala
                320                 325                 330

Glu Pro Glu Glu Gln
                335

<210> 41
<211> 1969
<212> DNA
<213> Homo sapiens

<400> 41
ggaggaggga gggcgggcag gcgccagccc agagcagccc cgggcaccag 50

cacggactct ctcttccagc ccaggtgccc cccactctcg ctccattcgg 100

cgggagcacc cagtcctgta cgccaaggaa ctggtcctgg gggcaccatg 150

gtttcggcgg cagcccccag cctcctcatc cttctgttgc tgctgctggg 200

gtctgtgcct gctaccgacg cccgctctgt gcccctgaag gccacgttcc 250

tggaggatgt ggcgggtagt ggggaggccg agggctcgtc ggcctcctcc 300

ccgagcctcc cgccaccctg gaccccggcc ctcagcccca catcgatggg 350

gccccagccc acaaccctgg ggggcccatc accccccacc aacttcctgg 400

atgggatagt ggacttcttc cgccagtacg tgatgctgat tgctgtggtg 450

ggctccctgg cctttctgct gatgttcatc gtctgtgccg cggtcatcac 500

ccggcagaag cagaaggcct cggcctatta cccatcgtcc ttccccaaga 550

agaagtacgt ggaccagagt gaccgggccg gggcccccgg ggccttcagt 600

gaggtccccg acagagcccc cgacagcagg cccgaggaag ccctggattc 650

ctcccggcag ctccaggccg acatcttggc cgccacccag aacctcaagt 700

cccccaccag ggctgcactg ggcggtgggg acggagccag gatggtggag 750

ggcaggggcg cagaggaaga ggagaagggc agccaggagg gggaccagga 800

agtccaggga catggggtcc cagtggagac accagaggcg caggaggagc 850

cgtgctcagg ggtccttgag ggggctgtgg tggccggtga gggccaaggg 900

gagctggaag ggtctctctt gttagcccag gaagcccagg gaccagtggg 950

tccccccgaa agcccctgtg cttgcagcag tgtccacccc agtgtctaac 1000

agtcctcccg ggctgccagc cctgactgtc gggcccccaa gtggtcacct 1050

ccccgtgtat gaaaaggcct tcagccctga ctgcttcctg acactccctc 1100

cttggcctcc ctgtggtgcc aatcccagca tgtgctgatt ctacagcagg 1150

cagaaatgct ggtccccggt gccccggagg aatcttacca agtgccatca 1200

tccttcacct cagcagcccc aaagggctac atcctacagc acagctcccc 1250

tgacaaagtg agggagggca cgtgtccctg tgacagccag gataaaacat 1300

cccccaaagt gctgggatta caggcgtgag ccaccgtgcc cggcccaaac 1350

tactttttaa aacagctaca gggtaaaatc ctgcagcacc cactctggaa 1400

aatactgctc ttaattttcc tgaaggtggc cccctgtttc tagttggtcc 1450

aggattaggg atgtggggta tagggcattt aaatcctctc aagcgctctc 1500

caagcacccc cggcctgggg gtgagtttct catcccgcta ctgctgctgg 1550

gatcaggttg aatgaatgga actcttcctg tctggcctcc aaagcagcct 1600

agaagctgag gggctgtgtt tgaggggacc tccaccctgg ggaagtccga 1650

ggggctgggg aagggtttct gacgcccagc ctggagcagg ggggccctgg 1700

ccaccccctg ttgctcacac attgtctggc agcctgtgtc cacaatattc 1750

gtcagtcctc gacagggagc ctgggctccg tcctgcttta gggaggctct 1800

ggcaggaggt cctctccccc atccctccat ctggggctcc cccaacctct 1850

gcacagctct ccaggtgctg agatataatg caccagcaca ataaaccttt 1900

attccggcct gaaaaaaaaa aaaaaaaaaa aaaaaaaaaa aaaaaaaaaa 1950

aaaaaaaaaa aaaaaaaga 1969

<210> 42
<211> 283
<212> PRT
<213> Homo sapiens

<400> 42
Met Val Ser Ala Ala Ala Pro Ser Leu Leu Ile Leu Leu Leu Leu
1               5                   10                  15

Leu Leu Gly Ser Val Pro Ala Thr Asp Ala Arg Ser Val Pro Leu
                20                  25                  30

Lys Ala Thr Phe Leu Glu Asp Val Ala Gly Ser Gly Glu Ala Glu
                35                  40                  45

Gly Ser Ser Ala Ser Ser Pro Ser Leu Pro Pro Pro Trp Thr Pro
                50                  55                  60

Ala Leu Ser Pro Thr Ser Met Gly Pro Gln Pro Thr Thr Leu Gly
                65                  70                  75

Gly Pro Ser Pro Pro Thr Asn Phe Leu Asp Gly Ile Val Asp Phe
                80                  85                  90

Phe Arg Gln Tyr Val Met Leu Ile Ala Val Val Gly Ser Leu Ala
                95                  100                 105

Phe Leu Leu Met Phe Ile Val Cys Ala Ala Val Ile Thr Arg Gln
                110                 115                 120

Lys Gln Lys Ala Ser Ala Tyr Tyr Pro Ser Ser Phe Pro Lys Lys
                125                 130                 135

Lys Tyr Val Asp Gln Ser Asp Arg Ala Gly Gly Pro Arg Ala Phe
                140                 145                 150

Ser Glu Val Pro Asp Arg Ala Pro Asp Ser Arg Pro Glu Glu Ala
                155                 160                 165

Leu Asp Ser Ser Arg Gln Leu Gln Ala Asp Ile Leu Ala Ala Thr
                170                 175                 180

Gln Asn Leu Lys Ser Pro Thr Arg Ala Ala Leu Gly Gly Gly Asp
                185                 190                 195

Gly Ala Arg Met Val Glu Gly Arg Gly Ala Glu Glu Glu Glu Lys
                200                 205                 210

Gly Ser Gln Glu Gly Asp Gln Glu Val Gln Gly His Gly Val Pro
                215                 220                 225

Val Glu Thr Pro Glu Ala Gln Glu Glu Pro Cys Ser Gly Val Leu
                230                 235                 240

Glu Gly Ala Val Val Ala Gly Glu Gly Gln Gly Glu Leu Glu Gly
                245                 250                 255

Ser Leu Leu Leu Ala Gln Glu Ala Gln Gly Pro Val Gly Pro Pro
                260                 265                 270

Glu Ser Pro Cys Ala Cys Ser Ser Val His Pro Ser Val
                275                 280

<210> 43
<211> 2314
<212> DNA
<213> Homo sapiens

<400> 43
ggtctctgtc cttggctgtg gctcctgcgc tctggctgag ccatgttcct 50

tctcctcgcc ctcctcactg agcttggaag actgcaagcc cacgaaggtt 100

ctgaaggaat atttctgcat gtcacagttc cacggaagat taagtcaaat 150

gacagtgaag tttcagagag gaagatgatt tacatcatta caattgatgg 200

acaaccttac actctacatc tcggaaaaca atcattctta ccccagaact 250

tttggtttta tacatataat gaaactggat ctttgcattc tgtgtctcca 300

tattttatga tgcattgcca ttaccaagga tatgctgccg aatttccaaa 350

ttcatttgtg acactcagta tatgttctgg tctcagggga tttctccagt 400

ttgaaaatat cagttatgga attgaaccag tagaatcttc agcaagattt 450

gagcatataa tttatcaaat gaaaaataat gatccaaatg tatccatttt 500

agcagtaaat tacagtcata tttggcagaa agaccagccc tacaaagttc 550

ctttaaactc acagataaaa aatctttcaa aactattacc ccaatatctg 600

gaaatataca ttatagtgga aaaagctttg atgtttaccc agttcaaatt 650

gactgttata ctgtcttcct tggaattgtg gtcaaatgaa aaccagattt 700

ccaccagtgg ggatgctgat gatatattac aaagattttt ggcatggaaa 750

cgggactatc tcatcctacg gccccatgac atagcatact tacttgttta 800

caggaaacat cctaaatatg tgggagcaac atttcctggc accgtatgca 850

ataaaagcta tgatgcaggt attgctatgt atccagatgc aataggtttg 900

gagggatttt cggttattat agctcaactg cttggcctta atgtaggatt 950

aacatatgat gacatcactc agtgtttctg tctgagagct acatgcatca 1000

tgaatcatga agcagtgagt gccagtggta gaaagatttt tagcaactgc 1050

agcatgcacg actatagata ttttgtttca aaatttgaga ctaaatgcct 1100

tcagaagctt tcaaatttgc aaccattaca tcaaaatcaa ccagtgtgtg 1150

gtaatgggat tttggaatcc aatgaagaat gtgactgtgg taataaaaat 1200

gaatgtcaat ttaagaagtg ctgtgattat aacacatgta aactgaaggg 1250

ctcagtaaaa tgtggttctg gaccatgttg tacatcaaag tgtgagttgt 1300

caatagcagg cactccatgt agaaagagta ttgatccaga gtgtgatttt 1350

acagagtact gcaatggaac ctctagtaat tgtgttcctg acacttatgc 1400

actgaatggc cgtttgtgca agttgggaac tgcctattgc tataacggac 1450

aatgtcaaac tactgataac cagtgtgcca agatatttgg aaaaggtgct 1500

caaggtgctc catttgcctg ttttaaagaa gttaattctc tgcatgaaag 1550

atctgaaaac tgtggtttta aaaattcaca accattacct tgtgaacgga 1600

aggatgttct ctgtggaaaa ttagcttgtg ttcagccaca taaaaatgct 1650

aataaaagtg acgctcaatc tacagtttat tcatatattc aagaccatgt 1700

atgtgtatct atagccactg gttcctccat gagatcagat ggaacagaca 1750

atgcctatgt ggctgatggc accatgtgtg gtccagaaat gtactgtgta 1800

aataaaacct gcagaaaagt tcatttaatg ggatataact gtaatgccac 1850

cacaaaatgc aaagggaaag ggatatgtaa taattttggt aattgtcaat 1900

gcttccctgg acatagacct ccagattgta aattccagtt tggttcccca 1950

gggggtagta ttgatgatgg aaattttcag aaatctggtg acttttatac 2000

tgaaaaaggc tacaatacac actggaacaa ctggtttatt ctgagtttct 2050

gcatttttct gccgtttttc atagttttca ccactgtgat ctttaaaaga 2100

aatgaaataa gtaaatcatg taacagagag aatgcagagt ataatcgtaa 2150

ttcatccgtt gtatcagaaa gcgatgacgt gggacattaa tattgcacag 2200

aacttccata gcaaataacc taaaggaacg aatgtgcttt atttataacc 2250

ttacgttatc cccaatgcat tgtaaatgtc aaacttttgg aaaataaagc 2300

ctgcgtgccc  tccc 2314

<210> 44
<211> 715
<212> PRT
<213> Homo sapiens

<400> 44
Met Phe Leu Leu Leu Ala Leu Leu Thr Glu Leu Gly Arg Leu Gln
1               5                   10                  15

Ala His Glu Gly Ser Glu Gly Ile Phe Leu His Val Thr Val Pro
                20                  25                  30

Arg Lys Ile Lys Ser Asn Asp Ser Glu Val Ser Glu Arg Lys Met
                35                  40                  45

Ile Tyr Ile Ile Thr Ile Asp Gly Gln Pro Tyr Thr Leu His Leu
                50                  55                  60

Gly Lys Gln Ser Phe Leu Pro Gln Asn Phe Leu Val Tyr Thr Tyr
                65                  70                  75

Asn Glu Thr Gly Ser Leu His Ser Val Ser Pro Tyr Phe Met Met
                80                  85                  90

His Cys His Tyr Gln Gly Tyr Ala Ala Glu Phe Pro Asn Ser Phe
                95                  100                 105

Val Thr Leu Ser Ile Cys Ser Gly Leu Arg Gly Phe Leu Gln Phe
                110                 115                 120

Glu Asn Ile Ser Tyr Gly Ile Glu Pro Val Glu Ser Ser Ala Arg
                125                 130                 135

Phe Glu His Ile Ile Tyr Gln Met Lys Asn Asn Asp Pro Asn Val
                140                 145                 150

Ser Ile Leu Ala Val Asn Tyr Ser His Ile Trp Gln Lys Asp Gln
                155                 160                 165

Pro Tyr Lys Val Pro Leu Asn Ser Gln Ile Lys Asn Leu Ser Lys
                170                 175                 180

Leu Leu Pro Gln Tyr Leu Glu Ile Tyr Ile Ile Val Glu Lys Ala
                185                 190                 195

Leu Met Phe Thr Gln Phe Lys Leu Thr Val Ile Leu Ser Ser Leu
                200                 205                 210

Glu Leu Trp Ser Asn Glu Asn Gln Ile Ser Thr Ser Gly Asp Ala
                215                 220                 225

Asp Asp Ile Leu Gln Arg Phe Leu Ala Trp Lys Arg Asp Tyr Leu

                230                 235                 240

Ile Leu Arg Pro His Asp Ile Ala Tyr Leu Leu Val Tyr Arg Lys
                245                 250                 255

His Pro Lys Tyr Val Gly Ala Thr Phe Pro Gly Thr Val Cys Asn
                260                 265                 270

Lys Ser Tyr Asp Ala Gly Ile Ala Met Tyr Pro Asp Ala Ile Gly
                275                 280                 285

Leu Glu Gly Phe Ser Val Ile Ile Ala Gln Leu Leu Gly Leu Asn
                290                 295                 300

Val Gly Leu Thr Tyr Asp Asp Ile Thr Gln Cys Phe Cys Leu Arg
                305                 310                 315

Ala Thr Cys Ile Met Asn His Glu Ala Val Ser Ala Ser Gly Arg
                320                 325                 330

Lys Ile Phe Ser Asn Cys Ser Met His Asp Tyr Arg Tyr Phe Val
                335                 340                 345

Ser Lys Phe Glu Thr Lys Cys Leu Gln Lys Leu Ser Asn Leu Gln
                350                 355                 360

Pro Leu His Gln Asn Gln Pro Val Cys Gly Asn Gly Ile Leu Glu
                365                 370                 375

Ser Asn Glu Glu Cys Asp Cys Gly Asn Lys Asn Glu Cys Gln Phe
                380                 385                 390

Lys Lys Cys Cys Asp Tyr Asn Thr Cys Lys Leu Lys Gly Ser Val
                395                 400                 405

Lys Cys Gly Ser Gly Pro Cys Cys Thr Ser Lys Cys Glu Leu Ser
                410                 415                 420

Ile Ala Gly Thr Pro Cys Arg Lys Ser Ile Asp Pro Glu Cys Asp
                425                 430                 435

Phe Thr Glu Tyr Cys Asn Gly Thr Ser Ser Asn Cys Val Pro Asp
                440                 445                 450

Thr Tyr Ala Leu Asn Gly Arg Leu Cys Lys Leu Gly Thr Ala Tyr
                455                 460                 465

Cys Tyr Asn Gly Gln Cys Gln Thr Thr Asp Asn Gln Cys Ala Lys
                470                 475                 480

Ile Phe Gly Lys Gly Ala Gln Gly Ala Pro Phe Ala Cys Phe Lys
                485                 490                 495

Glu Val Asn Ser Leu His Glu Arg Ser Glu Asn Cys Gly Phe Lys
                500                 505                 510

Asn Ser Gln Pro Leu Pro Cys Glu Arg Lys Asp Val Leu Cys Gly
                515                 520                 525

Lys Leu Ala Cys Val Gln Pro His Lys Asn Ala Asn Lys Ser Asp
                530                 535                 540

Ala Gln Ser Thr Val Tyr Ser Tyr Ile Gln Asp His Val Cys Val
                545                 550                 555

Ser Ile Ala Thr Gly Ser Ser Met Arg Ser Asp Gly Thr Asp Asn
                560                 565                 570

Ala Tyr Val Ala Asp Gly Thr Met Cys Gly Pro Glu Met Tyr Cys
                575                 580                 585

Val Asn Lys Thr Cys Arg Lys Val His Leu Met Gly Tyr Asn Cys
                590                 595                 600

Asn Ala Thr Thr Lys Cys Lys Gly Lys Gly Ile Cys Asn Asn Phe
                605                 610                 615

Gly Asn Cys Gln Cys Phe Pro Gly His Arg Pro Pro Asp Cys Lys
                620                 625                 630

Phe Gln Phe Gly Ser Pro Gly Gly Ser Ile Asp Asp Gly Asn Phe
                635                 640                 645

Gln Lys Ser Gly Asp Phe Tyr Thr Glu Lys Gly Tyr Asn Thr His
                650                 655                 660

Trp Asn Asn Trp Phe Ile Leu Ser Phe Cys Ile Phe Leu Pro Phe
                665                 670                 675

Phe Ile Val Phe Thr Thr Val Ile Phe Lys Arg Asn Glu Ile Ser
                680                 685                 690

Lys Ser Cys Asn Arg Glu Asn Ala Glu Tyr Asn Arg Asn Ser Ser
                695                 700                 705

Val Val Ser Glu Ser Asp Asp Val Gly His
                710                 715

<210> 45
<211> 2570
<212> DNA
<213> Homo sapiens

<400> 45
ccaggaccag ggcgcaccgg ctcagcctct cacttgtcag aggccgggga 50

agagaagcaa agcgcaacgg tgtggtccaa gccggggctt ctgcttcgcc 100

tctaggacat acacgggacc ccctaacttc agtcccccaa acgcgcaccc 150

tcgaagtctt gaactccagc cccgcacatc cacgcgcggc acaggcgcgg 200

caggcggcag gtcccggccg aaggcgatgc gcgcaggggg tcgggcagct 250

gggctcgggc ggcgggagta gggcccggca gggaggcagg gaggctgcat 300

attcagagtc gcgggctgcg ccctgggcag aggccgccct cgctccacgc 350

aacacctgct gctgccaccg cgccgcgatg agccgcgtgg tctcgctgct 400

gctgggcgcc gcgctgctct gcggccacgg agccttctgc cgccgcgtgg 450

tcagcggcca aaaggtgtgt tttgctgact tcaagcatcc ctgctacaaa 500

atggcctact tccatgaact gtccagccga gtgagctttc aggaggcacg 550

cctggcttgt gagagtgagg gaggagtcct cctcagcctt gagaatgaag 600

cagaacagaa gttaatagag agcatgttgc aaaacctgac aaaacccggg 650

acagggattt ctgatggtga tttctggata gggctttgga ggaatggaga 700

tgggcaaaca tctggtgcct gcccagatct ctaccagtgg tctgatggaa 750

gcaattccca gtaccgaaac tggtacacag atgaaccttc ctgcggaagt 800

gaaaagtgtg ttgtgatgta tcaccaacca actgccaatc ctggccttgg 850

gggtccctac ctttaccagt ggaatgatga caggtgtaac atgaagcaca 900

attatatttg caagtatgaa ccagagatta atccaacagc ccctgtagaa 950

aagccttatc ttacaaatca accaggagac acccatcaga atgtggttgt 1000

tactgaagca ggtataattc ccaatctaat ttatgttgtt ataccaacaa 1050

tacccctgct cttactgata ctggttgctt ttggaacctg ttgtttccag 1100

atgctgcata aaagtaaagg aagaacaaaa actagtccaa accagtctac 1150

actgtggatt tcaaagagta ccagaaaaga aagtggcatg gaagtataat 1200

aactcattga cttggttcca gaattttgta attctggatc tgtataagga 1250

atggcatcag aacaatagct tggaatggct tgaaatcaca aaggatctgc 1300

aagatgaact gtaagctccc ccttgaggca aatattaaag taatttttat 1350

atgtctatta tttcatttaa agaatatgct gtgctaataa tggagtgaga 1400

catgcttatt ttgctaaagg atgcacccaa acttcaaact tcaagcaaat 1450

gaaatggaca atgcagataa agttgttatc aacacgtcgg gagtatgtgt 1500

gttagaagca attcctttta tttctttcac ctttcataag ttgttatcta 1550

gtcaatgtaa tgtatattgt attgaaattt acagtgtgca aaagtatttt 1600

acctttgcat aagtgtttga taaaaatgaa ctgttctaat atttattttt 1650

atggcatctc atttttcaat acatgctctt ttgattaaag aaacttatta 1700

ctgttgtcaa ctgaattcac acacacacaa atatagtacc atagaaaaag 1750

tttgttttct cgaaataatt catctttcag cttctctgct tttggtcaat 1800

gtctaggaaa tctcttcaga aataagaagc tatttcatta agtgtgatat 1850

aaacctcctc aaacatttta cttagaggca aggattgtct aatttcaatt 1900

gtgcaagaca tgtgccttat aattattttt agcttaaaat taaacagatt 1950

ttgtaataat gtaactttgt taataggtgc ataaacacta atgcagtcaa 2000

tttgaacaaa agaagtgaca tacacaatat aaatcatatg tcttcacacg 2050

ttgcctatat aatgagaagc agctctctga gggttctgaa atcaatgtgg 2100

tccctctctt gcccactaaa caaagatggt tgttcggggt ttgggattga 2150

cactggaggc agatagttgc aaagttagtc taaggtttcc ctagctgtat 2200

ttagcctctg actatattag tatacaaaga ggtcatgtgg ttgagaccag 2250

gtgaatagtc actatcagtg tggagacaag cacagcacac agacatttta 2300

ggaaggaaag gaactacgaa atcgtgtgaa aatgggttgg aacccatcag 2350

tgatcgcata ttcattgatg agggtttgct tgagatagaa aatggtggct 2400

cctttctgtc ttatctccta gtttcttcaa tgcttacgcc ttgttcttct 2450

caagagaaag ttgtaactct ctggtcttca tatgtccctg tgctcctttt 2500

aaccaaataa agagttcttg tttctggggg aaaaaaaaaa aaaaaaaaaa 2550

aaaaaaaaaa aaaaaaaaaa 2570

<210> 46
<211> 273
<212> PRT
<213> Homo sapiens

<400> 46
Met Ser Arg Val Val Ser Leu Leu Leu Gly Ala Ala Leu Leu Cys
1               5                   10                  15

Gly His Gly Ala Phe Cys Arg Arg Val Val Ser Gly Gln Lys Val
                20                  25                  30

Cys Phe Ala Asp Phe Lys His Pro Cys Tyr Lys Met Ala Tyr Phe
                35                  40                  45

His Glu Leu Ser Ser Arg Val Ser Phe Gln Glu Ala Arg Leu Ala
                50                  55                  60

Cys Glu Ser Glu Gly Gly Val Leu Leu Ser Leu Glu Asn Glu Ala
                65                  70                  75

Glu Gln Lys Leu Ile Glu Ser Met Leu Gln Asn Leu Thr Lys Pro
                80                  85                  90

Gly Thr Gly Ile Ser Asp Gly Asp Phe Trp Ile Gly Leu Trp Arg
                95                  100                 105

Asn Gly Asp Gly Gln Thr Ser Gly Ala Cys Pro Asp Leu Tyr Gln
                110                 115                 120

Trp Ser Asp Gly Ser Asn Ser Gln Tyr Arg Asn Trp Tyr Thr Asp
                125                 130                 135

Glu Pro Ser Cys Gly Ser Glu Lys Cys Val Val Met Tyr His Gln
                140                 145                 150

Pro Thr Ala Asn Pro Gly Leu Gly Gly Pro Tyr Leu Tyr Gln Trp
                155                 160                 165

Asn Asp Asp Arg Cys Asn Met Lys His Asn Tyr Ile Cys Lys Tyr
                170                 175                 180

Glu Pro Glu Ile Asn Pro Thr Ala Pro Val Glu Lys Pro Tyr Leu
                185                 190                 195

Thr Asn Gln Pro Gly Asp Thr His Gln Asn Val Val Val Thr Glu
                200                 205                 210

Ala Gly Ile Ile Pro Asn Leu Ile Tyr Val Val Ile Pro Thr Ile
                215                 220                 225

Pro Leu Leu Leu Leu Ile Leu Val Ala Phe Gly Thr Cys Cys Phe
                230                 235                 240

Gln Met Leu His Lys Ser Lys Gly Arg Thr Lys Thr Ser Pro Asn
                245                 250                 255

Gln Ser Thr Leu Trp Ile Ser Lys Ser Thr Arg Lys Glu Ser Gly
                260                 265                 270

Met Glu Val

<210> 47
<211> 1240
<212> DNA
<213> Homo sapiens

<400> 47
tgcatcagtg cccaggcaag cccaggagtt gacatttctc tgcccagcca 50

tgggcctcac cctgctcttg ctgctgctcc tgggactaga aggtcagggc 100

atagttggca gcctccctga ggtgctgcag gcacccgtgg gaagctccat 150

tctggtgcag tgccactaca ggctccagga tgtcaaagct cagaaggtgt 200

ggtgccggtt cttgccggag gggtgccagc ccctggtgtc ctcagctgtg 250

gatcgcagag ctccagcggg caggcgtacg tttctcacag acctgggtgg 300

gggcctgctg caggtggaaa tggttaccct gcaggaagag gatgctggcg 350

agtatggctg catggtggat ggggccaggg ggccccagat tttgcacaga 400

gtctctctga acatactgcc cccagaggaa gaagaagaga cccataagat 450

tggcagtctg gctgagaacg cattctcaga ccctgcaggc agtgccaacc 500

ctttggaacc cagccaggat gagaagagca tccccttgat ctggggtgct 550

gtgctcctgg taggtctgct ggtggcagcg gtggtgctgt ttgctgtgat 600

ggccaagagg aaacaagaat ccctcctcag tggtccacca cgtcagtgac 650

tctggaccgg ctgctgaatt gcctttggat gtaccacaca ttaggcttga 700

ctcaccacct tcatttgaca ataccaccta caccagccta cctcttgatt 750

ccccatcagg aaaaccttca ctcccagctc catcctcatt gccccctcta 800

cctcctaagg tcctggtctg ctccaagcct gtgacatatg ccacagtaat 850

cttcccggga gggaacaagg gtggagggac ctcgtgtggg ccagcccaga 900

atccacctaa caatcagact ccatccagct aagctgctca tcacacttta 950

aactcatgag gaccatccct aggggttctg tgcatccatc cagccagctc 1000

atgccctagg atccttagga tatctgagca accagggact ttaagatcta 1050

atccaatgtc ctaactttac tagggaaagt gacgctcaga catgactgag 1100

atgtcttggg gaagacctcc ctgcacccaa ctcccccact ggttcttcta 1150

ccattacaca ctgggctaaa taaaccctaa taatgatgtg caaaaaaaaa 1200

aaaaaaaaaa aaaaaaaaaa aaaaaaaaaa aaaaaaaaaa 1240

<210> 48
<211> 199
<212> PRT
<213> Homo sapiens

<400> 48
Met Gly Leu Thr Leu Leu Leu Leu Leu Leu Leu Gly Leu Glu Gly
1               5                   10                  15

Gln Gly Ile Val Gly Ser Leu Pro Glu Val Leu Gln Ala Pro Val
                20                  25                  30

Gly Ser Ser Ile Leu Val Gln Cys His Tyr Arg Leu Gln Asp Val
                35                  40                  45

Lys Ala Gln Lys Val Trp Cys Arg Phe Leu Pro Glu Gly Cys Gln
                50                  55                  60

Pro Leu Val Ser Ser Ala Val Asp Arg Arg Ala Pro Ala Gly Arg
                65                  70                  75

Arg Thr Phe Leu Thr Asp Leu Gly Gly Gly Leu Leu Gln Val Glu
                80                  85                  90

Met Val Thr Leu Gln Glu Glu Asp Ala Gly Glu Tyr Gly Cys Met
                95                  100                 105

Val Asp Gly Ala Arg Gly Pro Gln Ile Leu His Arg Val Ser Leu
                110                 115                 120

Asn Ile Leu Pro Pro Glu Glu Glu Glu Glu Thr His Lys Ile Gly
                125                 130                 135

Ser Leu Ala Glu Asn Ala Phe Ser Asp Pro Ala Gly Ser Ala Asn
                140                 145                 150

Pro Leu Glu Pro Ser Gln Asp Glu Lys Ser Ile Pro Leu Ile Trp
                155                 160                 165

Gly Ala Val Leu Leu Val Gly Leu Leu Val Ala Ala Val Val Leu
                170                 175                 180

Phe Ala Val Met Ala Lys Arg Lys Gln Glu Ser Leu Leu Ser Gly
                185                 190                 195

Pro Pro Arg Gln

<210> 49
<211> 2776
<212> DNA
<213> Homo sapiens

<400> 49
   ccggccgtgt ggaaccaaac ctgcgcgcgt ggccgggccg tgggacaacg 50
   aggccgcgga gacgaaggcg caatggcgag gaagttatct gtaatcttga 100
   tcctgacctt tgccctctct gtcacaaatc cccttcatga actaaaagca 150
   gctgctttcc cccagaccac tgagaaaatt agtccgaatt gggaatctgg 200
   cattaatgtt gacttggcaa tttccacacg gcaatatcat ctacaacagc 250
   ttttctaccg ctatggagaa aataattctt tgtcagttga agggttcaga 300
   aaattacttc aaaatatagg catagataag attaaaagaa tccatataca 350
   ccatgaccac gaccatcact cagaccacga gcatcactca gaccatgagc 400
   gtcactcaga ccatgagcat cactcagacc acgagcatca ctctgaccat 450
   gatcatcact cccaccataa tcatgctgct tctggtaaaa ataagcgaaa 500
   agctctttgc ccagaccatg actcagatag ttcaggtaaa gatcctagaa 550
   acagccaggg gaaaggagct caccgaccag aacatgccag tggtagaagg 600
   aatgtcaagg acagtgttag tgctagtgaa gtgacctcaa ctgtgtacaa 650
   cactgtctct gaaggaactc actttctaga gacaatagag actccaagac 700
   ctggaaaact cttccccaaa gatgtaagca gctccactcc acccagtgtc 750
   acatcaaaga gccgggtgag ccggctggct ggtaggaaaa caaatgaatc 800
   tgtgagtgag ccccgaaaag gctttatgta ttccagaaac acaaatgaaa 850
   atcctcagga gtgtttcaat gcatcaaagc tactgacatc tcatggcatg 900
   ggcatccagg ttccgctgaa tgcaacagag ttcaactatc tctgtccagc 950
   catcatcaac caaattgatg ctagatcttg tctgattcat acaagtgaaa 1000
   agaaggctga aatccctcca aagacctatt cattacaaat agcctgggtt 1050
   ggtggtttta tagccatttc catcatcagt ttcctgtctc tgctgggggt 1100
   tatcttagtg cctctcatga atcgggtgtt tttcaaattt ctcctgagtt 1150
   tccttgtggc actggccgtt gggactttga gtggtgatgc ttttttacac 1200
   cttcttccac attctcatgc aagtcaccac catagtcata gccatgaaga 1250
   accagcaatg gaaatgaaaa gaggaccact tttcagtcat ctgtcttctc 1300
   aaaacataga agaaagtgcc tattttgatt ccacgtggaa gggtctaaca 1350
   gctctaggag gcctgtattt catgtttctt gttgaacatg tcctcacatt 1400
   gatcaaacaa tttaaagata agaagaaaaa gaatcagaag aaacctgaaa 1450
   atgatgatga tgtggagatt aagaagcagt tgtccaagta tgaatctcaa 1500
   ctttcaacaa atgaggagaa agtagataca gatgatcgaa ctgaaggcta 1550
   tttacgagca gactcacaag agccctccca ctttgattct cagcagcctg 1600
   cagtcttgga agaagaagag gtcatgatag ctcatgctca tccacaggaa 1650
   gtctacaatg aatatgtacc cagagggtgc aagaataaat gccattcaca 1700
   tttccacgat acactcggcc agtcagacga tctcattcac caccatcatg 1750
   actaccatca tattctccat catcaccacc accaaaacca ccatcctcac 1800
   agtcacagcc agcgctactc tcgggaggag ctgaaagatg ccggcgtcgc 1850
   cactttggcc tggatggtga taatgggtga tggcctgcac aatttcagcg 1900
   atggcctagc aattggtgct gcttttactg aaggcttatc aagtggttta 1950
   agtacttctg ttgctgtgtt ctgtcatgag ttgcctcatg aattaggtga 2000
   ctttgctgtt ctactaaagg ctgacatgac cgttaagcag gctgtccttt 2050
   ataatgcatt gtcagccatg ctggcgtatc ttggaatggc aacaggaatt 2100
   ttcattggtc attatgctga aaatgtttct atgtggatat ttgcacttac 2150
   tgctggctta ttcatgtatg ttgctctggt tgatatggta cctgaaatgc 2200
   tgcacaatga tgctagtgac catggatgta gccgctgggg gtatttcttt 2250
   ttacagaatg ctgggatgct tttgggtttt ggaattatgt tacttatttc 2300
   catatttgaa cataaaatcg tgtttcgtat aaatttctag ttaaggttta 2350
   aatgctagag tagcttaaaa agttgtcata gtttcagtag gtcataggga 2400
   gatgagtttg tatgctgtac tatgcagcgt ttaaagttag tgggttttgt 2450
   gatttttgta ttgaatattg ctgtctgtta caaagtcagt taaaggtacg 2500
   ttttaatatt taagttattc tatcttggag ataaaatctg tatgtgcaat 2550
   tcaccggtat taccagttta ttatgtaaac aagagatttg gcatgacatg 2600
   ttctgtatgt ttcagggaaa aatgtcttta atgctttttc aagaactaac 2650
   acagttattc ctatactgga ttttaggtct ctgaagaact gctggtgttt 2700
   aggaataaga atgtgcatga agcctaaaat accaagaaag cttatactga 2750
   atttaagcaa aaaaaaaaaa aaaaaa 2776

<210> 50
<211> 755
<212> PRT
<213> Homo sapiens

<400> 50
Met Ala Arg Lys Leu Ser Val Ile Leu Ile Leu Thr Phe Ala Leu
1               5                   10                  15

Ser Val Thr Asn Pro Leu His Glu Leu Lys Ala Ala Ala Phe Pro
                20                  25                  30

Gln Thr Thr Glu Lys Ile Ser Pro Asn Trp Glu Ser Gly Ile Asn
                35                  40                  45

Val Asp Leu Ala Ile Ser Thr Arg Gln Tyr His Leu Gln Gln Leu
                50                  55                  60

Phe Tyr Arg Tyr Gly Glu Asn Asn Ser Leu Ser Val Glu Gly Phe
                65                  70                  75

Arg Lys Leu Leu Gln Asn Ile Gly Ile Asp Lys Ile Lys Arg Ile
                80                  85                  90

His Ile His His Asp His Asp His His Ser Asp His Glu His His
                95                  100                 105

Ser Asp His Glu Arg His Ser Asp His Glu His His Ser Asp His
                110                 115                 120

Glu His His Ser Asp His Asp His His Ser His His Asn His Ala
                125                 130                 135

Ala Ser Gly Lys Asn Lys Arg Lys Ala Leu Cys Pro Asp His Asp
                140                 145                 150

Ser Asp Ser Ser Gly Lys Asp Pro Arg Asn Ser Gln Gly Lys Gly
                155                 160                 165

Ala His Arg Pro Glu His Ala Ser Gly Arg Arg Asn Val Lys Asp
                170                 175                 180

Ser Val Ser Ala Ser Glu Val Thr Ser Thr Val Tyr Asn Thr Val
                185                 190                 195

Ser Glu Gly Thr His Phe Leu Glu Thr Ile Glu Thr Pro Arg Pro
                200                 205                 210

Gly Lys Leu Phe Pro Lys Asp Val Ser Ser Ser Thr Pro Pro Ser
                215                 220                 225

Val Thr Ser Lys Ser Arg Val Ser Arg Leu Ala Gly Arg Lys Thr
                230                 235                 240

Asn Glu Ser Val Ser Glu Pro Arg Lys Gly Phe Met Tyr Ser Arg
                245                 250                 255

Asn Thr Asn Glu Asn Pro Gln Glu Cys Phe Asn Ala Ser Lys Leu
                260                 265                 270

Leu Thr Ser His Gly Met Gly Ile Gln Val Pro Leu Asn Ala Thr
                275                 280                 285

Glu Phe Asn Tyr Leu Cys Pro Ala Ile Ile Asn Gln Ile Asp Ala
                290                 295                 300

Arg Ser Cys Leu Ile His Thr Ser Glu Lys Lys Ala Glu Ile Pro
                305                 310                 315

Pro Lys Thr Tyr Ser Leu Gln Ile Ala Trp Val Gly Gly Phe Ile
                320                 325                 330

Ala Ile Ser Ile Ile Ser Phe Leu Ser Leu Leu Gly Val Ile Leu
                335                 340                 345

Val Pro Leu Met Asn Arg Val Phe Phe Lys Phe Leu Leu Ser Phe
                350                 355                 360

Leu Val Ala Leu Ala Val Gly Thr Leu Ser Gly Asp Ala Phe Leu
                365                 370                 375

His Leu Leu Pro His Ser His Ala Ser His His His Ser His Ser
                380                 385                 390

His Glu Glu Pro Ala Met Glu Met Lys Arg Gly Pro Leu Phe Ser
                395                 400                 405

His Leu Ser Ser Gln Asn Ile Glu Glu Ser Ala Tyr Phe Asp Ser
                410                 415                 420

Thr Trp Lys Gly Leu Thr Ala Leu Gly Gly Leu Tyr Phe Met Phe
                425                 430                 435

Leu Val Glu His Val Leu Thr Leu Ile Lys Gln Phe Lys Asp Lys
                440                 445                 450

Lys Lys Lys Asn Gln Lys Lys Pro Glu Asn Asp Asp Asp Val Glu
                455                 460                 465

Ile Lys Lys Gln Leu Ser Lys Tyr Glu Ser Gln Leu Ser Thr Asn
                470                 475                 480

Glu Glu Lys Val Asp Thr Asp Asp Arg Thr Glu Gly Tyr Leu Arg
                485                 490                 495

Ala Asp Ser Gln Glu Pro Ser His Phe Asp Ser Gln Gln Pro Ala
                500                 505                 510

Val Leu Glu Glu Glu Glu Val Met Ile Ala His Ala His Pro Gln
                515                 520                 525

Glu Val Tyr Asn Glu Tyr Val Pro Arg Gly Cys Lys Asn Lys Cys
                530                 535                 540

His Ser His Phe His Asp Thr Leu Gly Gln Ser Asp Asp Leu Ile
                545                 550                 555

His His His His Asp Tyr His His Ile Leu His His His His His
                560                 565                 570

Gln Asn His His Pro His Ser His Ser Gln Arg Tyr Ser Arg Glu
                575                 580                 585

Glu Leu Lys Asp Ala Gly Val Ala Thr Leu Ala Trp Met Val Ile
                590                 595                 600

Met Gly Asp Gly Leu His Asn Phe Ser Asp Gly Leu Ala Ile Gly
                605                 610                 615

Ala Ala Phe Thr Glu Gly Leu Ser Ser Gly Leu Ser Thr Ser Val
                620                 625                 630

Ala Val Phe Cys His Glu Leu Pro His Glu Leu Gly Asp Phe Ala
                635                 640                 645

Val Leu Leu Lys Ala Asp Met Thr Val Lys Gln Ala Val Leu Tyr
                650                 655                 660

Asn Ala Leu Ser Ala Met Leu Ala Tyr Leu Gly Met Ala Thr Gly
                665                 670                 675

Ile Phe Ile Gly His Tyr Ala Glu Asn Val Ser Met Trp Ile Phe
                680                 685                 690

Ala Leu Thr Ala Gly Leu Phe Met Tyr Val Ala Leu Val Asp Met

                695                 700                 705

Val Pro Glu Met Leu His Asn Asp Ala Ser Asp His Gly Cys Ser
                710                 715                 720

Arg Trp Gly Tyr Phe Phe Leu Gln Asn Ala Gly Met Leu Leu Gly
                725                 730                 735

Phe Gly Ile Met Leu Leu Ile Ser Ile Phe Glu His Lys Ile Val
                740                 745                 750

Phe Arg Ile Asn Phe
                755

<210> 51
<211> 3915
<212> DNA
<213> Homo sapiens

<400> 51
gtggggtggg gtggggctgg gggcttgtcg ccctttcagg ctccaccctt 50

tgcggagatt ataaatagtc atgatcccag cgagacccag agatgcctgt 100

aatggtgaga ctttggatcc ttcctgagga cgtggagaaa actttctgct 150

gagaaggaca ttttgaaggt tttgttggct gaaaaagctg tttctggaat 200

cacccctaga tctttcttga agacttgaat tagattacag cgatggggac 250

acagaaggtc accccagctc tgatatttgc catcacagtt gctacaatcg 300

gctctttcca atttggctac aacactgggg tcatcaatgc tcctgagaag 350

atcataaagg aatttatcaa taaaactttg acggacaagg gaaatgcccc 400

accctctgag gtgctgctca cgtctctctg gtccttgtct gtggccatat 450

tttccgtcgg gggtatgatc ggctcctttt ccgtcggact cttcgtcaac 500

cgctttggca ggcgcaattc aatgctgatt gtcaacctgt tggctgtcac 550

tggtggctgc tttatgggac tgtgtaaagt agctaagtcg gttgaaatgc 600

tgatcctggg tcgcttggtt attggcctct tctgcggact ctgcacaggt 650

tttgtgccca tgtacattgg agagatctcg cctactgccc tgcggggtgc 700

ctttggcact ctcaaccagc tgggcatcgt tgttggaatt ctggtggccc 750

agatctttgg tctggaattc atccttgggt ctgaagagct atggccgctg 800

ctactgggtt ttaccatcct tcctgctatc ctacaaagtg cagcccttcc 850

attttgccct gaaagtccca gatttttgct cattaacaga aaagaagagg 900

agaatgctaa gcagatcctc cagcggttgt ggggcaccca ggatgtatcc 950

caagacatcc aggagatgaa agatgagagt gcaaggatgt cacaagaaaa 1000

gcaagtcacc gtgctagagc tctttagagt gtccagctac cgacagccca 1050

tcatcatttc cattgtgctc cagctctctc agcagctctc tgggatcaat 1100

gctgtgttct attactcaac aggaatcttc aaggatgcag gtgttcaaga 1150

gcccatctat gccaccatcg gcgcgggtgt ggttaatact atcttcactg 1200

tagtttctct atttctggtg gaaagggcag gaagaaggac tctgcatatg 1250

ataggccttg gagggatggc tttttgttcc acgctcatga ctgtttcttt 1300

gttattaaag gataactata atgggatgag ctttgtctgt attggggcta 1350

tcttggtctt tgtagccttc tttgaaattg gaccaggccc cattccctgg 1400

tttattgtgg ccgaactctt cagccagggc ccccgcccag ctgcgatggc 1450

agtggccggc tgctccaact ggacctccaa cttcctagtc ggattgctct 1500

tcccctccgc tgctcactat ttaggagcct acgtttttat tatcttcacc 1550

ggcttcctca ttaccttctt ggcttttacc ttcttcaaag tccctgagac 1600

ccgtggcagg acttttgagg atatcacacg ggcctttgaa gggcaggcac 1650

acggtgcaga tagatctgga aaggacggcg tcatggagat gaacagcatc 1700

gagcctgcta aggagaccac caccaatgtc taagtcgtgc ctccttccac 1750

ctccctcccg gcatgggaaa gccacctctc cctcaacaag ggagagacct 1800

catcaggatg aacccaggac gcttctgaat gctgctactt aattcctttc 1850

tcatcccacg cactccatga gcaccccaag gctgcggttt gttggatctt 1900

caatggcttt ttaaatttta tttcctggac atcctcttct gcttaggaga 1950

gaccgagtga acctaccttc atttcaggag ggattggccg cttggcacat 2000

gacaactttg ccagcttttc ctcccttggg ttctgatatt gccgcactag 2050

gggatatagg agaggaaaag taaggtgcag ttcccccaac ctcagactta 2100

ccaggaagca gatacatatg agtgtggaag ccggagggtg tttatgtaag 2150

agcaccttcc tcacttccat acagctctac gtggcaaatt aacttgagtt 2200

ttatttattt tatcctctgg tttaattaca taattttttt ttttttactt 2250

taagtttcag gatacatgtg ccgaatgtgc aggtttgtta cataggtata 2300

tatatgccat gatggaaata tttatttttt taagcgtaat tttgccaaat 2350

aataaaaaca gaaggaaatt gagattagag ggaggtgttt aaagagaggt 2400

tatagagtag aagatttgat gctggagagg ttaaggtgca ataagaattt 2450
agggagaaat gttgttcatt attggagggt aaatgatgtg gtgcctgagg 2500
tctgtacgtt acctcttaac aatttctgtc cttcagatgg aaactcttta 2550
acttctcgta aaagtcatat acctatataa taaagctact gatttccttg 2600
gagctttttt ctttaagata atagtttaca tgtagtagta cttgaaatct 2650
aggattatta actaatatgg gcattgtagt taatgatggt tgatgggttc 2700
taattttgga tggagtccag ggaagagaaa gtgatttcta gaaagcctgt 2750
tcccctcact ggatgaaata actccttctt gtagtagtct cattactttt 2800
gaagtaatcc cgccacctat ctcgtgggag agccatccaa ataagaaacc 2850
taaaataatt ggttcttggt agagattcat tatttttcca ctttgttctt 2900
taggagattt taggtgttga ttttctgttg tattttaact cataccttta 2950
aaggaattcc ccaaagaatg tttatagcaa acttggaatt tgtaacctca 3000
gctctgggag aggatttttt tctgagcgat tattatctaa agtgtgttgt 3050
tgctttaggc tcacggcacg cttgcgtatg tctgttacca tgtcactgtg 3100
gtcctatgcc gaatgccctc aggggacttg aatctttcca ataaaccagg 3150
tttagacagt atgagtcaat gtgcagtgta gcccacactt gagaggatga 3200
atgtatgtgc actgtcactt tgctctgggt ggaagtacgt tattgttgac 3250
ttattttctc tgtgtttgtt cctacagccc ctttttcata tgttgctcag 3300
tctccctttc ccttcttggt gcttacacat ctcagaccct ttagccaaac 3350
ccttgtcagt gacagtattt tggttcttag ttctcactgt tccctctgct 3400
cctggagcct ttgaataaaa atgcacgtag ctgaggccgg atgcggtggc 3450
tcacgcctgt aatcccagca ctttgggagg cctaggcggg cggtcagggg 3500
ttcgagacca gtctggccaa catcgtgaaa ccctgtctct actaaaaatg 3550
caaaaattag ccgggcgtgg tggcgggcgc ctgtaatccc agctacttgg 3600
gaagctgagg cgggagaatc atgtgaaccc gggacgcagg ggttgcagtg 3650
agcggagatc gcatcattgc actctagcct gggccacagg gcgagactcc 3700
gtctcaaaaa aaaaaaaatg cacatagcta tcgagtgtgc tttagcttga 3750
aaaggtgacc ttgcaacttc atgtcaactt tctggctcct caaacagtag 3800
gttggcagta aggcagggtc ccatttctca ctgagaagat tgtgaatatt 3850

tccatatgga ttttctattg ttactctggt tctttgtttt aaaataaaaa 3900

ttctgaatgt acacg 3915

<210> 52
<211> 496
<212> PRT
<213> Homo sapiens

<400> 52
Met Gly Thr Gln Lys Val Thr Pro Ala Leu Ile Phe Ala Ile Thr
1               5                   10                  15

Val Ala Thr Ile Gly Ser Phe Gln Phe Gly Tyr Asn Thr Gly Val
                20                  25                  30

Ile Asn Ala Pro Glu Lys Ile Ile Lys Glu Phe Ile Asn Lys Thr
                35                  40                  45

Leu Thr Asp Lys Gly Asn Ala Pro Pro Ser Glu Val Leu Leu Thr
                50                  55                  60

Ser Leu Trp Ser Leu Ser Val Ala Ile Phe Ser Val Gly Gly Met
                65                  70                  75

Ile Gly Ser Phe Ser Val Gly Leu Phe Val Asn Arg Phe Gly Arg
                80                  85                  90

Arg Asn Ser Met Leu Ile Val Asn Leu Leu Ala Val Thr Gly Gly
                95                  100                 105

Cys Phe Met Gly Leu Cys Lys Val Ala Lys Ser Val Glu Met Leu
                110                 115                 120

Ile Leu Gly Arg Leu Val Ile Gly Leu Phe Cys Gly Leu Cys Thr
                125                 130                 135

Gly Phe Val Pro Met Tyr Ile Gly Glu Ile Ser Pro Thr Ala Leu
                140                 145                 150

Arg Gly Ala Phe Gly Thr Leu Asn Gln Leu Gly Ile Val Val Gly
                155                 160                 165

Ile Leu Val Ala Gln Ile Phe Gly Leu Glu Phe Ile Leu Gly Ser
                170                 175                 180

Glu Glu Leu Trp Pro Leu Leu Leu Gly Phe Thr Ile Leu Pro Ala
                185                 190                 195

Ile Leu Gln Ser Ala Ala Leu Pro Phe Cys Pro Glu Ser Pro Arg
                200                 205                 210

Phe Leu Leu Ile Asn Arg Lys Glu Glu Glu Asn Ala Lys Gln Ile
                215                 220                 225

Leu Gln Arg Leu Trp Gly Thr Gln Asp Val Ser Gln Asp Ile Gln
                230                 235                 240

Glu Met Lys Asp Glu Ser Ala Arg Met Ser Gln Glu Lys Gln Val
                245                 250                 255

Thr Val Leu Glu Leu Phe Arg Val Ser Ser Tyr Arg Gln Pro Ile
                260                 265                 270

Ile Ile Ser Ile Val Leu Gln Leu Ser Gln Gln Leu Ser Gly Ile
                275                 280                 285

Asn Ala Val Phe Tyr Tyr Ser Thr Gly Ile Phe Lys Asp Ala Gly
                290                 295                 300

Val Gln Glu Pro Ile Tyr Ala Thr Ile Gly Ala Gly Val Val Asn
                305                 310                 315

Thr Ile Phe Thr Val Val Ser Leu Phe Leu Val Glu Arg Ala Gly
                320                 325                 330

Arg Arg Thr Leu His Met Ile Gly Leu Gly Gly Met Ala Phe Cys
                335                 340                 345

Ser Thr Leu Met Thr Val Ser Leu Leu Leu Lys Asp Asn Tyr Asn
                350                 355                 360

Gly Met Ser Phe Val Cys Ile Gly Ala Ile Leu Val Phe Val Ala
                365                 370                 375

Phe Phe Glu Ile Gly Pro Gly Pro Ile Pro Trp Phe Ile Val Ala
                380                 385                 390

Glu Leu Phe Ser Gln Gly Pro Arg Pro Ala Ala Met Ala Val Ala
                395                 400                 405

Gly Cys Ser Asn Trp Thr Ser Asn Phe Leu Val Gly Leu Leu Phe
                410                 415                 420

Pro Ser Ala Ala His Tyr Leu Gly Ala Tyr Val Phe Ile Ile Phe
                425                 430                 435

Thr Gly Phe Leu Ile Thr Phe Leu Ala Phe Thr Phe Phe Lys Val
                440                 445                 450

Pro Glu Thr Arg Gly Arg Thr Phe Glu Asp Ile Thr Arg Ala Phe
                455                 460                 465

Glu Gly Gln Ala His Gly Ala Asp Arg Ser Gly Lys Asp Gly Val
                470                 475                 480

Met Glu Met Asn Ser Ile Glu Pro Ala Lys Glu Thr Thr Thr Asn
                485                 490                 495

Val

<210> 53
<211> 1401
<212> DNA
<213> Homo sapiens

<400> 53
cgtattgctc ggcccgggga gtttcgcccc ctgcccggct ccgcggcgcg 50

gaggatggcg tggaaacggc tgggcgcgct ggtgatgttc cctctacaga 100

tgatctatct ggtggtgaaa gcagccgtcg gactggtgct gcccgccaag 150

ctgcgggacc tgtcgcggga gaacgtcctc atcaccggcg gcgggagagg 200

catcgggcgt cagctcgccc gcgagttcgc ggagcgcggc gccagaaaga 250

ttgttctctg gggccggact gagaaatgcc tgaaggagac gacggaggag 300

atccggcaga tgggcactga gtgccattac ttcatctgtg atgtgggcaa 350

ccgggaggag gtgtaccaga cggccaaggc cgtccgggag aaggtgggtg 400

acatcaccat cctggtgaac aatgccgccg tggtccatgg gaagagccta 450

atggacagtg atgatgatgc cctcctcaag tcccaacaca tcaacaccct 500

gggccagttc tggaccacca aggccttcct gccgcgtatg ctggagctgc 550

agaatggcca catcgtgtgc ctcaactccg tgctggcact gtctgccatc 600

cccggtgcca tcgactactg cacatccaaa gcgtcagcct tcgccttcat 650

ggagagcctg accctggggc tgctggactg tccgggagtc agcgccacca 700

cagtgctgcc cttccacacc agcaccgaga tgttccaggg catgagagtc 750

aggtttccca acctctttcc cccactgaag ccggagacgg tggcccggag 800

gacagtggaa gctgtgcagc tcaaccaggc cctcctcctc ctcccatgga 850

caatgcatgc cctcgttatc ttgaaaagca tacttccaca ggctgcactc 900

gaggagatcc acaaattctc aggaacctac acctgcatga acactttcaa 950

agggcggaca tagagacagg atgaagacat gcttgaggag ccacggagtt 1000

tgggggccac agcacctggg cacacacccg agcacctgtc cattggcatg 1050

cttctgctgg gtgagcagga cagctcctgt ccccagcgaa gaatccggct 1100

gcccctgggc cagtcccagg acctttgcac aggactgatg ggtgtaactg 1150

acccccacag ggaggcagga aaacagccag aagccacctt gacacttttg 1200

aacatttcca gttctgtaga gtttattgtc aattgcttct caagtctaac 1250

cagcctcagc agtgtgcata gaccatttcc aggagggtct gtccccagat 1300

gctctgcctc ccgttccaaa acccactcat cctcagcttg cacaaactgg 1350

ttgaacggca ggaatgaaaa ataaagagag atggcttttg tgaaaaaaaa 1400

a 1401

<210> 54
<211> 302
<212> PRT
<213> Homo sapiens

<400> 54
Met Ala Trp Lys Arg Leu Gly Ala Leu Val Met Phe Pro Leu Gln
1               5                   10                  15

Met Ile Tyr Leu Val Val Lys Ala Ala Val Gly Leu Val Leu Pro
                20                  25                  30

Ala Lys Leu Arg Asp Leu Ser Arg Glu Asn Val Leu Ile Thr Gly
                35                  40                  45

Gly Gly Arg Gly Ile Gly Arg Gln Leu Ala Arg Glu Phe Ala Glu
                50                  55                  60

Arg Gly Ala Arg Lys Ile Val Leu Trp Gly Arg Thr Glu Lys Cys
                65                  70                  75

Leu Lys Glu Thr Thr Glu Glu Ile Arg Gln Met Gly Thr Glu Cys
                80                  85                  90

His Tyr Phe Ile Cys Asp Val Gly Asn Arg Glu Glu Val Tyr Gln
                95                  100                 105

Thr Ala Lys Ala Val Arg Glu Lys Val Gly Asp Ile Thr Ile Leu
                110                 115                 120

Val Asn Asn Ala Ala Val Val His Gly Lys Ser Leu Met Asp Ser
                125                 130                 135

Asp Asp Asp Ala Leu Leu Lys Ser Gln His Ile Asn Thr Leu Gly
                140                 145                 150

Gln Phe Trp Thr Thr Lys Ala Phe Leu Pro Arg Met Leu Glu Leu
                155                 160                 165

Gln Asn Gly His Ile Val Cys Leu Asn Ser Val Leu Ala Leu Ser
                170                 175                 180

Ala Ile Pro Gly Ala Ile Asp Tyr Cys Thr Ser Lys Ala Ser Ala
                185                 190                 195

Phe Ala Phe Met Glu Ser Leu Thr Leu Gly Leu Leu Asp Cys Pro
                200                 205                 210

Gly Val Ser Ala Thr Thr Val Leu Pro Phe His Thr Ser Thr Glu
                215                 220                 225

Met Phe Gln Gly Met Arg Val Arg Phe Pro Asn Leu Phe Pro Pro
                230                 235                 240

Leu Lys Pro Glu Thr Val Ala Arg Arg Thr Val Glu Ala Val Gln
                245                 250                 255

Leu Asn Gln Ala Leu Leu Leu Leu Pro Trp Thr Met His Ala Leu
                260                 265                 270

Val Ile Leu Lys Ser Ile Leu Pro Gln Ala Ala Leu Glu Glu Ile
                275                 280                 285

His Lys Phe Ser Gly Thr Tyr Thr Cys Met Asn Thr Phe Lys Gly
                290                 295                 300

Arg Thr

<210> 55
<211> 2997
<212> DNA
<213> Homo sapiens

<400> 55
cggacgcgtg ggcgggcgcg ccgggaggga ccggcggcgg catgggccgg 50

gggccctggg atgcgggccc gtctcgccgc ctgctgccgc tgttgctgct 100

gctcggcctg gcccgcggcg ccgcgggagc gccgggcccc gacggtttag 150

acgtctgtgc cacttgccat gaacatgcca catgccagca aagagaaggg 200

aagaagatct gtatttgcaa ctatggattt gtagggaacg ggaggactca 250

gtgtgttgat aaaaatgagt gccagtttgg agccactctt gtctgtggga 300

accacacatc ttgccacaac acccccgggg gcttctattg catttgcctg 350

gaaggatatc gagccacaaa caacaacaag acattcattc ccaacgatgg 400

caccttttgt acagacatag atgagtgtga agtttctggc ctgtgcaggc 450

atggagggcg atgcgtgaac actcatggga gctttgaatg ctactgtatg 500

gatggatact tgccaaggaa tggacctgaa cctttccacc cgaccaccga 550

tgccacatca tgcacagaaa tagactgtgg tacccctcct gaggttccag 600

atggctatat cataggaaat tatacgtcta gtctgggcag ccaggttcgt 650

tatgcttgca gagaaggatt cttcagtgtt ccagaagata cagtttcaag 700

ctgcacaggc ctgggcacat gggagtcccc aaaattacat tgccaagaga 750

tcaactgtgg caaccctcca gaaatgcggc acgccatctt ggtaggaaat 800

cacagctcca ggctgggcgg tgtggctcgc tatgtctgtc aagagggctt 850

tgagagccct ggaggaaaga tcacttctgt ttgcacagag aaaggcacct 900

ggagagaaag tactttaaca tgcacagaaa ttctgacaaa gattaatgat 950

gtatcactgt ttaatgatac ctgtgtgaga tggcaaataa actcaagaag 1000

aataaacccc aagatctcat atgtgatatc cataaaagga caacggttgg 1050

accctatgga atcagttcgt gaggagacag tcaacttgac cacagacagc 1100

aggaccccag aagtgtgcct agccctgtac ccaggcacca actacaccgt 1150

gaacatctcc acagcacctc ccaggcgctc gatgccagcc gtcatcggtt 1200

tccagacagc tgaagttgat ctcttagaag atgatggaag tttcaatatt 1250

tcaatattta atgaaacttg tttgaaattg aacaggcgtt ctaggaaagt 1300

tggatcagaa cacatgtacc aatttaccgt tctgggtcag aggtggtatc 1350

tggctaactt ttctcatgca acatcgttta acttcacaac gagggaacaa 1400

gtgcctgtag tgtgtttgga tctgtaccct acgactgatt atacggtgaa 1450

tgtgaccctg ctgagatctc ctaagcggca ctcagtgcaa ataacaatag 1500

caactccccc agcagtaaaa cagaccatca gtaacatttc aggatttaat 1550

gaaacctgct tgagatggag aagcatcaag acagctgata tggaggagat 1600

gtatttattc cacatttggg gccagagatg gtatcagaag gaatttgccc 1650

aggaaatgac ctttaatatc agtagcagca gccgagatcc cgaggtgtgc 1700

ttggacctac gtccgggtac caactacaat gtcagtctcc gggctctgtc 1750

ttcggaactt cctgtggtca tctccctgac aacccagata acagagcctc 1800

ccctcccgga agtagaattt tttacggtgc acagaggacc tctaccacgc 1850

ctcagactga ggaaagccaa ggagaaaaat ggaccaatca gttcatatca 1900

ggtgttagtg cttcccctgg ccctccaaag cacattttct tgtgattctg 1950

aaggcgcttc ctccttcttt agcaacgcct ctgatgctga tggatacgtg 2000

gctgcagaac tactggccaa agatgttcca gatgatgcca tggagatacc 2050

tataggagac aggctgtact atggggaata ttataatgca cccttgaaaa 2100

gagggagtga ttactgcatt atattacgaa tcacaagtga atggaataag 2150

gtgagaagac actcctgtgc agtttgggct caggtgaaag attcgtcact 2200

catgctgctg cagatggcgg gtgttggact gggttccctg gctgttgtga 2250

tcattctcac attcctctcc ttctcagcgg tgtgatggca gatggacact 2300

gagtggggag gatgcactgc tgctgggcag gtgttctggc agcttctcag 2350

gtgcccgcac agaggctccg tgtgacttcc gtccagggag catgtgggcc 2400

tgcaactttc tccattccca gctgggcccc attcctggat ttaagatggt 2450

ggctatccct gaggagtcac cataaggaga aaactcagga attctgagtc 2500

ttccctgcta caggaccagt tctgtgcaat gaacttgaga ctcctgatgt 2550

acactgtgat attgaccgaa ggctacatac agatctgtga atcttggctg 2600

ggacttcctc tgagtgatgc ctgagggtca gctcctctag acattgactg 2650

caagagaatc tctgcaacct cctatataaa agcatttctg ttaattcatt 2700

cagaatccat tctttacaat atgcagtgag atgggcttaa gtttgggcta 2750

gagtttgact ttatgaagga ggtcattgaa aaagagaaca gtgacgtagg 2800

caaatgtttc aagcacttta gaaacagtac ttttcctata attagttgat 2850

atactaatga gaaaatatac tagcctggcc atgccaataa gtttcctgct 2900

gtgtctgtta ggcagcattg ctttgatgca atttctattg tcctatatat 2950

tcaaaagtaa tgtctacatt ccagtaaaaa tatcccgtaa ttaaaaa 2997

<210> 56
<211> 747
<212> PRT
<213> Homo sapiens

<400> 56
Met Gly Arg Gly Pro Trp Asp Ala Gly Pro Ser Arg Arg Leu Leu
1               5                   10                  15

Pro Leu Leu Leu Leu Leu Gly Leu Ala Arg Gly Ala Ala Gly Ala
                20                  25                  30

Pro Gly Pro Asp Gly Leu Asp Val Cys Ala Thr Cys His Glu His
                35                  40                  45

Ala Thr Cys Gln Gln Arg Glu Gly Lys Lys Ile Cys Ile Cys Asn
                50                  55                  60

Tyr Gly Phe Val Gly Asn Gly Arg Thr Gln Cys Val Asp Lys Asn
                65                  70                  75

Glu Cys Gln Phe Gly Ala Thr Leu Val Cys Gly Asn His Thr Ser
                80                  85                  90

Cys His Asn Thr Pro Gly Gly Phe Tyr Cys Ile Cys Leu Glu Gly
                95                  100                 105

Tyr Arg Ala Thr Asn Asn Asn Lys Thr Phe Ile Pro Asn Asp Gly
                110                 115                 120

Thr Phe Cys Thr Asp Ile Asp Glu Cys Glu Val Ser Gly Leu Cys
                125                 130                 135

Arg His Gly Gly Arg Cys Val Asn Thr His Gly Ser Phe Glu Cys
                140                 145                 150

Tyr Cys Met Asp Gly Tyr Leu Pro Arg Asn Gly Pro Glu Pro Phe
                155                 160                 165

His Pro Thr Thr Asp Ala Thr Ser Cys Thr Glu Ile Asp Cys Gly
                170                 175                 180

Thr Pro Pro Glu Val Pro Asp Gly Tyr Ile Ile Gly Asn Tyr Thr
                185                 190                 195

Ser Ser Leu Gly Ser Gln Val Arg Tyr Ala Cys Arg Glu Gly Phe
                200                 205                 210

Phe Ser Val Pro Glu Asp Thr Val Ser Ser Cys Thr Gly Leu Gly
                215                 220                 225

Thr Trp Glu Ser Pro Lys Leu His Cys Gln Glu Ile Asn Cys Gly
                230                 235                 240

Asn Pro Pro Glu Met Arg His Ala Ile Leu Val Gly Asn His Ser
                245                 250                 255

Ser Arg Leu Gly Gly Val Ala Arg Tyr Val Cys Gln Glu Gly Phe
                260                 265                 270

Glu Ser Pro Gly Gly Lys Ile Thr Ser Val Cys Thr Glu Lys Gly
                275                 280                 285

Thr Trp Arg Glu Ser Thr Leu Thr Cys Thr Glu Ile Leu Thr Lys
                290                 295                 300

Ile Asn Asp Val Ser Leu Phe Asn Asp Thr Cys Val Arg Trp Gln
                305                 310                 315

Ile Asn Ser Arg Arg Ile Asn Pro Lys Ile Ser Tyr Val Ile Ser
                320                 325                 330

Ile Lys Gly Gln Arg Leu Asp Pro Met Glu Ser Val Arg Glu Glu
                335                 340                 345

Thr Val Asn Leu Thr Thr Asp Ser Arg Thr Pro Glu Val Cys Leu
                350                 355                 360

Ala Leu Tyr Pro Gly Thr Asn Tyr Thr Val Asn Ile Ser Thr Ala
                365                 370                 375

Pro Pro Arg Arg Ser Met Pro Ala Val Ile Gly Phe Gln Thr Ala
                380                 385                 390

Glu Val Asp Leu Leu Glu Asp Asp Gly Ser Phe Asn Ile Ser Ile
                395                 400                 405

Phe Asn Glu Thr Cys Leu Lys Leu Asn Arg Arg Ser Arg Lys Val
                410                 415                 420

Gly Ser Glu His Met Tyr Gln Phe Thr Val Leu Gly Gln Arg Trp
                425                 430                 435

Tyr Leu Ala Asn Phe Ser His Ala Thr Ser Phe Asn Phe Thr Thr
                440                 445                 450

Arg Glu Gln Val Pro Val Val Cys Leu Asp Leu Tyr Pro Thr Thr
                455                 460                 465

Asp Tyr Thr Val Asn Val Thr Leu Leu Arg Ser Pro Lys Arg His
                470                 475                 480

Ser Val Gln Ile Thr Ile Ala Thr Pro Pro Ala Val Lys Gln Thr
                485                 490                 495

Ile Ser Asn Ile Ser Gly Phe Asn Glu Thr Cys Leu Arg Trp Arg
                500                 505                 510

Ser Ile Lys Thr Ala Asp Met Glu Glu Met Tyr Leu Phe His Ile
                515                 520                 525

Trp Gly Gln Arg Trp Tyr Gln Lys Glu Phe Ala Gln Glu Met Thr
                530                 535                 540

Phe Asn Ile Ser Ser Ser Ser Arg Asp Pro Glu Val Cys Leu Asp
                545                 550                 555

Leu Arg Pro Gly Thr Asn Tyr Asn Val Ser Leu Arg Ala Leu Ser
                560                 565                 570

Ser Glu Leu Pro Val Val Ile Ser Leu Thr Thr Gln Ile Thr Glu
                575                 580                 585

Pro Pro Leu Pro Glu Val Glu Phe Phe Thr Val His Arg Gly Pro
                590                 595                 600

Leu Pro Arg Leu Arg Leu Arg Lys Ala Lys Glu Lys Asn Gly Pro
                605                 610                 615

Ile Ser Ser Tyr Gln Val Leu Val Leu Pro Leu Ala Leu Gln Ser
                620                 625                 630

Thr Phe Ser Cys Asp Ser Glu Gly Ala Ser Ser Phe Phe Ser Asn
                635                 640                 645

Ala Ser Asp Ala Asp Gly Tyr Val Ala Ala Glu Leu Leu Ala Lys
                650                 655                 660

Asp Val Pro Asp Asp Ala Met Glu Ile Pro Ile Gly Asp Arg Leu
                665                 670                 675

Tyr Tyr Gly Glu Tyr Tyr Asn Ala Pro Leu Lys Arg Gly Ser Asp
                680                 685                 690

Tyr Cys Ile Ile Leu Arg Ile Thr Ser Glu Trp Asn Lys Val Arg
                695                 700                 705

Arg His Ser Cys Ala Val Trp Ala Gln Val Lys Asp Ser Ser Leu
                710                 715                 720

Met Leu Leu Gln Met Ala Gly Val Gly Leu Gly Ser Leu Ala Val

725                 730                 735

Val Ile Ile Leu Thr Phe Leu Ser Phe Ser Ala Val
                740                 745

<210> 57
<211> 3010
<212> DNA
<213> Homo sapiens

<400> 57
gcccccagca tggcttggca gggctggccc gcggcgtggc agtgggtcgc 50

cggctgctgg ctcctcctcg tccttgtcct cgtcctactt gtgagccccc 100

gcggctgccg agcgcggcgg ggcctccgcg gtctgctcat ggcgcacagc 150

cagcggctgc tcttccgaat cgggtacagc ctgtacaccc gcacctggct 200

cgggtacctc ttctaccgac agcagctgcg cagggctcgg aatcgctacc 250

ctaaaggcca ctcgaaaacc cagccccgcc tcttcaatgg agtgaaggtg 300

cttcccatcc ctgtcctctc ggacaactac agctacctca tcatcgacac 350

ccaggcccag ctggctgtgg ctgtggaccc ttcagaccct cgggctgtgc 400

aggcttccat tgaaaaggaa ggggtcacct tggtcgccat tctgtgtact 450

cacaagcact gggaccacag tggagggaac cgtgacctca gccggcggca 500

ccgggactgt cgggtgtacg ggagccctca ggacggcatc ccctacctca 550

cccatcccct gtgtcatcaa gatgtggtca gcgtgggacg gcttcagatc 600

cgggccctgg ctacacctgg ccacacacaa ggccatctgg tctacctact 650

ggatggggag ccctacaagg gtccctcctg cctcttctca ggggacctgc 700

tcttcctctc tggctgtggg cggacctttg agggcaatgc agagaccatg 750

ctgagctcac tggacactgt gctggggcta ggggatgaca cccttctgtg 800

gcctggtcat gagtatgcag aggagaacct gggctttgca ggtgtggtgg 850

agcccgagaa cctggcccgg gagaggaaga tgcagtgggt gcagcggcag 900

cggctggagc gcaagggcac gtgcccatct accctgggag aggagcgctc 950

ctacaacccg ttcctgagaa cccactgcct ggcgctacag gaggctctgg 1000

ggccggggcc gggccccact ggggatgatg actactcccg ggcccagctc 1050

ctggaagagc tccgccggct gaaggatatg cacaagagca agtgatgccc 1100

ccagcgcccc cagcccagcc cactccccgc atggggaggc cgccaccacc 1150

aacacctcat catccttctc atcgctaaca ccaccacctc catcggcacc 1200

caagcgggca tcatcccccc acactgctca ggggagggga gggatcaggc 1250
gatgagactg tgaggccaaa agaagggggc ctgttggagg ctgggaaccc 1300
cgcagcgcga ggctgcctca tcaacggcaa gaggaaagga ggggtctcgg 1350
gacatctcca gaccctacca actgggaggg tcccctcctc cttccctact 1400
cctgggacgg cagcaaggac atgggggctg ctgttagctt ctccgtcagg 1450
aggcctcatc tcactgtagc cctggaaccc agggtccatc ttgcccttcc 1500
cccatccatg gttgggaaag aagctcagcc cctcacagtg gcctcaagtg 1550
tgatgcctta caaaagcacc actcagatgg gcagctggac tctggtgtcc 1600
tgagactctg ccctcttccc acagcctccc tgccccaccc atccctgcaa 1650
agccattttt cagacagagc cattcctaag aacactgaag ggctggaatg 1700
ctggctggcc actctctgcc tcagtggcct ccctacagcc tggaagaagg 1750
agggtcctga ttgccaagga aacctcctca ttgggctaag gagacactgg 1800
agtctggagt gtggagcccc acagtcttgc aggtcacatg ctctccttgc 1850
acatctggcc tggttgtacc cactggcctc tgcctctgcc ctgggccaaa 1900
agggcccctc cttgccaggg gagagacagc cacggtcctc tttggccgat 1950
gctgtattct cattttggcc cttgttctta ggcccgtctg cccgccctcc 2000
tccatctaac ctttcctgtt ttatccgcag cccttttctt ctttgagtta 2050
gtaaagattt attctgtaac ctgacactca tctggccctt tgcagtttgc 2100
cagccatatt cccatgtgat ttcccactgg atccaggccc ccatccggct 2150
ggcaggaggg ggctctgacg tacaggttgg aaatcagaag tctgtgagag 2200
cgcgggagtg catggcagct ctgggtccca gacctggccc gacccctctg 2250
cttcacctcc agctctgctg ctcctctact cttgggtcga gatccctttg 2300
gagccacagc gaggaaccct gtggtcctca ggcaggtgta ccttgagtca 2350
gccaggagcc ctcttttcct gtgtcaaagc ctgccctcgg gctctgctca 2400
cctctggtga ccctccaaga tgcccctgcc ctcagtttcc cctcatgatc 2450
tggcctctgc ccccttctct agccacagcc tctagtacac tttagcaata 2500
ccaccagact agttagagtt ccccactcac caagcaagac atgcagtttc 2550
atgcctctgt gccttcgctc atgctgtttc ttccgactgg aatgccttcc 2600
cctgctcctc ctgccttgtc tgcctggcaa gttcatctct cacgatcccc 2650

tcaaaggccc cctcctccag gaaggcaacc cctgtgcccc tcccctccag 2700

gctacctctg cactttgtca atgcttctct tgtggcactt atcacactgt 2750

attttacttg tttacatgtt tgtctcccct tctagactgt gaatccttaa 2800

gggcatggac tgtatcttat gcatctctgt atttctgcgc ctagcacggt 2850

gcctagcaca cagtaggcgc tcaataaatg ttgaatgaat gaaaaaaaaa 2900

aaaaaaaaaa aaaaaaaaaa aaaaaaaaaa aaaaaaaaaa aaaaaaaaaa 2950

aaaaaaaaaa aaaaaaaaaa aaaaaaaaaa aaaaaaaaaa aaaaaaaaaa 3000

aaaaaaaaaa 3010

<210> 58
<211> 361
<212> PRT
<213> Homo sapiens

<400> 58
Met Ala Trp Gln Gly Trp Pro Ala Ala Trp Gln Trp Val Ala Gly
1               5                   10                  15

Cys Trp Leu Leu Leu Val Leu Val Leu Val Leu Leu Val Ser Pro
                20                  25                  30

Arg Gly Cys Arg Ala Arg Arg Gly Leu Arg Gly Leu Leu Met Ala
                35                  40                  45

His Ser Gln Arg Leu Leu Phe Arg Ile Gly Tyr Ser Leu Tyr Thr
                50                  55                  60

Arg Thr Trp Leu Gly Tyr Leu Phe Tyr Arg Gln Gln Leu Arg Arg
                65                  70                  75

Ala Arg Asn Arg Tyr Pro Lys Gly His Ser Lys Thr Gln Pro Arg
                80                  85                  90

Leu Phe Asn Gly Val Lys Val Leu Pro Ile Pro Val Leu Ser Asp
                95                  100                 105

Asn Tyr Ser Tyr Leu Ile Ile Asp Thr Gln Ala Gln Leu Ala Val
                110                 115                 120

Ala Val Asp Pro Ser Asp Pro Arg Ala Val Gln Ala Ser Ile Glu
                125                 130                 135

Lys Glu Gly Val Thr Leu Val Ala Ile Leu Cys Thr His Lys His
                140                 145                 150

Trp Asp His Ser Gly Gly Asn Arg Asp Leu Ser Arg Arg His Arg
                155                 160                 165

Asp Cys Arg Val Tyr Gly Ser Pro Gln Asp Gly Ile Pro Tyr Leu
                170                 175                 180

Thr His Pro Leu Cys His Gln Asp Val Val Ser Val Gly Arg Leu
                185                 190                 195

Gln Ile Arg Ala Leu Ala Thr Pro Gly His Thr Gln Gly His Leu
                200                 205                 210

Val Tyr Leu Leu Asp Gly Glu Pro Tyr Lys Gly Pro Ser Cys Leu
                215                 220                 225

Phe Ser Gly Asp Leu Leu Phe Leu Ser Gly Cys Gly Arg Thr Phe
                230                 235                 240

Glu Gly Asn Ala Glu Thr Met Leu Ser Ser Leu Asp Thr Val Leu
                245                 250                 255

Gly Leu Gly Asp Asp Thr Leu Leu Trp Pro Gly His Glu Tyr Ala
                260                 265                 270

Glu Glu Asn Leu Gly Phe Ala Gly Val Val Glu Pro Glu Asn Leu
                275                 280                 285

Ala Arg Glu Arg Lys Met Gln Trp Val Gln Arg Gln Arg Leu Glu
                290                 295                 300

Arg Lys Gly Thr Cys Pro Ser Thr Leu Gly Glu Glu Arg Ser Tyr
                305                 310                 315

Asn Pro Phe Leu Arg Thr His Cys Leu Ala Leu Gln Glu Ala Leu
                320                 325                 330

Gly Pro Gly Pro Gly Pro Thr Gly Asp Asp Asp Tyr Ser Arg Ala
                335                 340                 345

Gln Leu Leu Glu Glu Leu Arg Arg Leu Lys Asp Met His Lys Ser
                350                 355                 360

Lys

<210> 59
<211> 2695
<212> DNA
<213> Homo sapiens

<400> 59
   gctagaccga gccctgggag gctacgggct cccccggaaa ccctgccagg 50
   ggagccgggt tttgagctca ggcgcctcta gcggcggccc ccagaaatct 100
   gactcgcgag gccagagttg cagggactga atagcaaact gaggctgagt 150
   agggaacaga ccatgaggtc agtgcagatc ttcctctccc aatgccgttt 200
   gctccttcta ctagttccga caatgctcct taagtctctt ggcgaagatg 250
   taatttttca ccctgaaggg gagtttgact cgtatgaagt caccattcct 300
   gagaagctga gcttccgggg agaggtgcag ggtgtggtca gtcccgtgtc 350
   ctacctactg cagttaaaag gcaagaagca cgtcctccat ttgtggccca 400
   agagacttct gttgccccga catctgcgcg ttttctcctt cacagaacat 450
   ggggaactgc tggaggatca tccttacata ccaaaggact gcaactacat 500
   gggctccgtg aaagagtctc tggactctaa agctactata agcacatgca 550
   tggggggtct ccgaggtgta tttaacattg atgccaaaca ttaccaaatt 600
   gagcccctca aggcctctcc cagttttgaa catgtcgtct atctcctgaa 650
   gaaagagcag tttgggaatc aggtttgtgg cttaagtgat gatgaaatag 700
   aatggcagat ggccccttat gagaataagg cgaggctaag ggactttcct 750
   ggatcctata aacacccaaa gtacttggaa ttgatcctac tctttgatca 800
   aagtaggtat aggtttgtga acaacaatct ttctcaagtc atacatgatg 850
   ccattctttt gactgggatt atggacacct actttcaaga tgttcgtatg 900
   aggatacact taaaggctct tgaagtatgg acagatttta acaaaatacg 950
   cgttggatat ccagagttag ctgaagtttt aggcagattt gtaatatata 1000
   aaaaaagtgt attaaatgct cgcctgtcat cagattgggc acatttatat 1050
   cttcaaagaa aatataatga tgctcttgca tggtcgtttg gaaaagtgtg 1100
   ttctctagaa tatgctggat cagtgagtac tttactagat acaaatatcc 1150
   ttgcccctgc tacctggtct gctcatgagc tgggtcatgc tgtaggaatg 1200
   tcacatgatg aacaatactg ccaatgtagg ggtaggctta attgcatcat 1250
   gggctcagga cgcactgggt ttagcaattg cagttatatc tcttttttta 1300
   aacatatctc ttcgggagca acatgtctaa ataatatccc aggactaggt 1350
   tatgtgctta agagatgtgg aaacaaaatt gtggaggaca atgaggaatg 1400
   tgactgtggt tccacagagg agtgtcagaa agatcggtgt tgccaatcaa 1450
   attgtaagtt gcaaccaggt gccaactgta gcattggact ttgctgtcat 1500
   gattgtcggt ttcgtccatc tggatacgtg tgtaggcagg aaggaaatga 1550
   atgtgacctt gcagagtact gcgacgggaa ttcaagttcc tgcccaaatg 1600
   acgtttataa gcaggatgga accccttgca agtatgaagg ccgttgtttc 1650
   aggaaggggt gcagatccag atatatgcag tgccaaagca tttttggacc 1700
   tgatgccatg gaggctccta gtgagtgcta tgatgcagtt aacttaatag 1750
   gtgatcaatt tggtaactgt gagattacag gaattcgaaa ttttaaaaag 1800
   tgtgaaagtg caaattcaat atgtggcagg ctacagtgta taaatgttga 1850
   aaccatccct gatttgccag agcatacgac tataatttct actcatttac 1900
   aggcagaaaa tctcatgtgc tggggcacag gctatcatct atccatgaaa 1950
   cccatgggaa tacctgacct aggtatgata aatgatggca cctcctgtgg 2000
   agaaggccgg gtatgtttta aaaaaaattg cgtcaatagc tcagtcctgc 2050
   agtttgactg tttgcctgag aaatgcaata cccggggtgt ttgcaacaac 2100
   agaaaaaact gccactgcat gtatgggtgg gcacctccat tctgtgagga 2150
   agtggggtat ggaggaagca ttgacagtgg gcctccagga ctgctcagag 2200
   gggcgattcc ctcgtcaatt tgggttgtgt ccatcataat gtttcgcctt 2250
   attttattaa tcctttcagt ggtttttgtg tttttccggc aagtgatagg 2300
   aaaccactta aaacccaaac aggaaaaaat gccactatcc aaagcaaaaa 2350
   ctgaacagga agaatctaaa acaaaaactg tacaggaaga atctaaaaca 2400
   aaaactggac aggaagaatc tgaagcaaaa actggacagg aagaatctaa 2450
   agcaaaaact ggacaggaag aatctaaagc aaacattgaa agtaaacgac 2500
   ccaaagcaaa gagtgtcaag aaacaaaaaa agtaaccggg caatccatac 2550
   tcattcagta acacaggctc atttatttaa ccagctaatc atttatccaa 2600
   aggctttcca ttcttctccc aatatttttt tactttaatt tttcccacaa 2650
   gttttgatca gcaaataaac agcattcttg ttttggaaac aaaaa 2695

<210> 60
<211> 790
<212> PRT
<213> Homo sapiens

<400> 60
Met Arg Ser Val Gln Ile Phe Leu Ser Gln Cys Arg Leu Leu Leu
1               5                   10                  15

Leu Leu Val Pro Thr Met Leu Leu Lys Ser Leu Gly Glu Asp Val
                20                  25                  30

Ile Phe His Pro Glu Gly Glu Phe Asp Ser Tyr Glu Val Thr Ile
                35                  40                  45

Pro Glu Lys Leu Ser Phe Arg Gly Glu Val Gln Gly Val Val Ser
                50                  55                  60

Pro Val Ser Tyr Leu Leu Gln Leu Lys Gly Lys Lys His Val Leu
                65                  70                  75

His Leu Trp Pro Lys Arg Leu Leu Leu Pro Arg His Leu Arg Val
                80                  85                  90

Phe Ser Phe Thr Glu His Gly Glu Leu Leu Glu Asp His Pro Tyr
                95                  100                 105

Ile Pro Lys Asp Cys Asn Tyr Met Gly Ser Val Lys Glu Ser Leu
                110                 115                 120

Asp Ser Lys Ala Thr Ile Ser Thr Cys Met Gly Gly Leu Arg Gly
                125                 130                 135

Val Phe Asn Ile Asp Ala Lys His Tyr Gln Ile Glu Pro Leu Lys
                140                 145                 150

Ala Ser Pro Ser Phe Glu His Val Val Tyr Leu Leu Lys Lys Glu
                155                 160                 165

Gln Phe Gly Asn Gln Val Cys Gly Leu Ser Asp Asp Glu Ile Glu
                170                 175                 180

Trp Gln Met Ala Pro Tyr Glu Asn Lys Ala Arg Leu Arg Asp Phe
                185                 190                 195

Pro Gly Ser Tyr Lys His Pro Lys Tyr Leu Glu Leu Ile Leu Leu
                200                 205                 210

Phe Asp Gln Ser Arg Tyr Arg Phe Val Asn Asn Asn Leu Ser Gln
                215                 220                 225

Val Ile His Asp Ala Ile Leu Leu Thr Gly Ile Met Asp Thr Tyr
                230                 235                 240

Phe Gln Asp Val Arg Met Arg Ile His Leu Lys Ala Leu Glu Val
                245                 250                 255

Trp Thr Asp Phe Asn Lys Ile Arg Val Gly Tyr Pro Glu Leu Ala
                260                 265                 270

Glu Val Leu Gly Arg Phe Val Ile Tyr Lys Lys Ser Val Leu Asn
                275                 280                 285

Ala Arg Leu Ser Ser Asp Trp Ala His Leu Tyr Leu Gln Arg Lys
                290                 295                 300

Tyr Asn Asp Ala Leu Ala Trp Ser Phe Gly Lys Val Cys Ser Leu
                305                 310                 315

Glu Tyr Ala Gly Ser Val Ser Thr Leu Leu Asp Thr Asn Ile Leu
                320                 325                 330

Ala Pro Ala Thr Trp Ser Ala His Glu Leu Gly His Ala Val Gly
                335                 340                 345

Met Ser His Asp Glu Gln Tyr Cys Gln Cys Arg Gly Arg Leu Asn
                350                 355                 360

Cys Ile Met Gly Ser Gly Arg Thr Gly Phe Ser Asn Cys Ser Tyr
                365                 370                 375

Ile Ser Phe Phe Lys His Ile Ser Ser Gly Ala Thr Cys Leu Asn
                380                 385                 390

Asn Ile Pro Gly Leu Gly Tyr Val Leu Lys Arg Cys Gly Asn Lys
                395                 400                 405

Ile Val Glu Asp Asn Glu Glu Cys Asp Cys Gly Ser Thr Glu Glu
                410                 415                 420

Cys Gln Lys Asp Arg Cys Cys Gln Ser Asn Cys Lys Leu Gln Pro
                425                 430                 435

Gly Ala Asn Cys Ser Ile Gly Leu Cys Cys His Asp Cys Arg Phe
                440                 445                 450

Arg Pro Ser Gly Tyr Val Cys Arg Gln Glu Gly Asn Glu Cys Asp
                455                 460                 465

Leu Ala Glu Tyr Cys Asp Gly Asn Ser Ser Ser Cys Pro Asn Asp
                470                 475                 480

Val Tyr Lys Gln Asp Gly Thr Pro Cys Lys Tyr Glu Gly Arg Cys
                485                 490                 495

Phe Arg Lys Gly Cys Arg Ser Arg Tyr Met Gln Cys Gln Ser Ile
                500                 505                 510

Phe Gly Pro Asp Ala Met Glu Ala Pro Ser Glu Cys Tyr Asp Ala
                515                 520                 525

Val Asn Leu Ile Gly Asp Gln Phe Gly Asn Cys Glu Ile Thr Gly
                530                 535                 540

Ile Arg Asn Phe Lys Lys Cys Glu Ser Ala Asn Ser Ile Cys Gly
                545                 550                 555

Arg Leu Gln Cys Ile Asn Val Glu Thr Ile Pro Asp Leu Pro Glu
                560                 565                 570

His Thr Thr Ile Ile Ser Thr His Leu Gln Ala Glu Asn Leu Met
                575                 580                 585

Cys Trp Gly Thr Gly Tyr His Leu Ser Met Lys Pro Met Gly Ile
                590                 595                 600

Pro Asp Leu Gly Met Ile Asn Asp Gly Thr Ser Cys Gly Glu Gly
                605                 610                 615

Arg Val Cys Phe Lys Lys Asn Cys Val Asn Ser Ser Val Leu Gln
                620                 625                 630

Phe Asp Cys Leu Pro Glu Lys Cys Asn Thr Arg Gly Val Cys Asn
                635                 640                 645

Asn Arg Lys Asn Cys His Cys Met Tyr Gly Trp Ala Pro Pro Phe

                650                 655                 660

Cys Glu Glu Val Gly Tyr Gly Gly Ser Ile Asp Ser Gly Pro Pro
                665                 670                 675

Gly Leu Leu Arg Gly Ala Ile Pro Ser Ser Ile Trp Val Val Ser
                680                 685                 690

Ile Ile Met Phe Arg Leu Ile Leu Leu Ile Leu Ser Val Val Phe
                695                 700                 705

Val Phe Phe Arg Gln Val Ile Gly Asn His Leu Lys Pro Lys Gln
                710                 715                 720

Glu Lys Met Pro Leu Ser Lys Ala Lys Thr Glu Gln Glu Glu Ser
                725                 730                 735

Lys Thr Lys Thr Val Gln Glu Glu Ser Lys Thr Lys Thr Gly Gln
                740                 745                 750

Glu Glu Ser Glu Ala Lys Thr Gly Gln Glu Glu Ser Lys Ala Lys
                755                 760                 765

Thr Gly Gln Glu Glu Ser Lys Ala Asn Ile Glu Ser Lys Arg Pro
                770                 775                 780

Lys Ala Lys Ser Val Lys Lys Gln Lys Lys
                785                 790

<210> 61
<211> 2024
<212> DNA
<213> Homo sapiens

<400> 61
caggcgggcc cccgcgcggc agggccctgg acccgcgcgg ctcccgggga 50

tggtgagcaa ggcgctgctg cgcctcgtgt ctgccgtcaa ccgcaggagg 100

atgaagctgc tgctgggcat cgccttgctg gcctacgtcg cctctgtttg 150

gggcaacttc gttaatatga ggtctatcca ggaaaatggt gaactaaaaa 200

ttgaaagcaa gattgaagag atggttgaac cactaagaga gaaaatcaga 250

gatttagaaa aaagctttac ccagaaatac ccaccagtaa agtttttatc 300

agaaaaggat cggaaaagaa ttttgataac aggaggcgca gggttcgtgg 350

gctcccatct aactgacaaa ctcatgatgg acggccacga ggtgaccgtg 400

gtggacaatt tcttcacggg caggaagaga aacgtggagc actggatcgg 450

acatgagaac ttcgagttga ttaaccacga cgtggtggag cccctctaca 500

tcgaggttga ccagatatac catctggcat ctccagcctc ccctccaaac 550

tacatgtata atcctatcaa gacattaaag accaatacga ttgggacatt 600

aaacatgttg gggctggcaa aacgagtcgg tgcccgtctg ctcctggcct 650
ccacatcgga ggtgtatgga gatcctgaag tccaccctca aagtgaggat 700
tactggggcc acgtgaatcc aataggacct cgggcctgct acgatgaagg 750
caaacgtgtt gcagagacca tgtgctatgc ctacatgaag caggaaggcg 800
tggaagtgcg agtggccaga atcttcaaca cctttgggcc acgcatgcac 850
atgaacgatg ggcgagtagt cagcaacttc atcctgcagg cgctccaggg 900
ggagccactc acggtatacg gatccgggtc tcagacaagg gcgttccagt 950
acgtcagcga tctagtgaat ggcctcgtgg ctctcatgaa cagcaacgtc 1000
agcagcccgg tcaacctggg gaacccagaa gaacacacaa tcctagaatt 1050
tgctcagtta attaaaaacc ttgttggtag cggaagtgaa attcagtttc 1100
tctccgaagc ccaggatgac ccacagaaaa gaaaaccaga catcaaaaaa 1150
gcaaagctga tgctggggtg ggagcccgtg gtcccgctgg aggaaggttt 1200
aaacaaagca attcactact tccgtaaaga actcgagtac caggcaaata 1250
atcagtacat ccccaaacca aagcctgcca gaataaagaa aggacggact 1300
cgccacagct gaactcctca cttttaggac acaagactac cattgtacac 1350
ttgatgggat gtatttttgg cttttttttg ttgtcgttta aagaaagact 1400
ttaacaggtg tcatgaagaa caaactggaa tttcattctg aagcttgctt 1450
taatgaaatg gatgtgccta aaagctcccc tcaaaaaact gcagattttg 1500
ccttgcactt tttgaatctc tctttttatg taaaatagcg tagatgcatc 1550
tctgcgtatt ttcaagtttt tttatcttgc tgtgagagca tatgttgtga 1600
ctgtcgttga cagttttatt tactggtttc tttgtgaagc tgaaaaggaa 1650
cattaagcgg gacaaaaaat gccgatttta tttataaaag tgggtactta 1700
ataaatgagt cgttatacta tgcataaaga aaaatcctag cagtattgtc 1750
aggtggtggt gcgccggcat tgattttagg gcagataaaa gaattctgtg 1800
tgagagcttt atgtttctct tttaattcag agtttttcca aggtctactt 1850
ttgagttgca aacttgactt tgaaatattc ctgttggtca tgatcaagga 1900
tatttgaaat cactactgtg ttttgctgcg tatctggggc gggggcaggt 1950
tggggggcac aaagttaaca tattcttggt taaccatggt taaatatgct 2000
attttaataa aatattgaaa ctca 2024

<210> 62
<211> 420
<212> PRT
<213> Homo sapiens

<400> 62
Met Val Ser Lys Ala Leu Leu Arg Leu Val Ser Ala Val Asn Arg
1               5                   10                  15

Arg Arg Met Lys Leu Leu Leu Gly Ile Ala Leu Leu Ala Tyr Val
                20                  25                  30

Ala Ser Val Trp Gly Asn Phe Val Asn Met Arg Ser Ile Gln Glu
                35                  40                  45

Asn Gly Glu Leu Lys Ile Glu Ser Lys Ile Glu Glu Met Val Glu
                50                  55                  60

Pro Leu Arg Glu Lys Ile Arg Asp Leu Glu Lys Ser Phe Thr Gln
                65                  70                  75

Lys Tyr Pro Pro Val Lys Phe Leu Ser Glu Lys Asp Arg Lys Arg
                80                  85                  90

Ile Leu Ile Thr Gly Gly Ala Gly Phe Val Gly Ser His Leu Thr
                95                  100                 105

Asp Lys Leu Met Met Asp Gly His Glu Val Thr Val Val Asp Asn
                110                 115                 120

Phe Phe Thr Gly Arg Lys Arg Asn Val Glu His Trp Ile Gly His
                125                 130                 135

Glu Asn Phe Glu Leu Ile Asn His Asp Val Val Glu Pro Leu Tyr
                140                 145                 150

Ile Glu Val Asp Gln Ile Tyr His Leu Ala Ser Pro Ala Ser Pro
                155                 160                 165

Pro Asn Tyr Met Tyr Asn Pro Ile Lys Thr Leu Lys Thr Asn Thr
                170                 175                 180

Ile Gly Thr Leu Asn Met Leu Gly Leu Ala Lys Arg Val Gly Ala
                185                 190                 195

Arg Leu Leu Leu Ala Ser Thr Ser Glu Val Tyr Gly Asp Pro Glu
                200                 205                 210

Val His Pro Gln Ser Glu Asp Tyr Trp Gly His Val Asn Pro Ile
                215                 220                 225

Gly Pro Arg Ala Cys Tyr Asp Glu Gly Lys Arg Val Ala Glu Thr
                230                 235                 240

Met Cys Tyr Ala Tyr Met Lys Gln Glu Gly Val Glu Val Arg Val
                245                 250                 255

Ala Arg Ile Phe Asn Thr Phe Gly Pro Arg Met His Met Asn Asp
                260                 265                 270

Gly Arg Val Val Ser Asn Phe Ile Leu Gln Ala Leu Gln Gly Glu
                275                 280                 285

Pro Leu Thr Val Tyr Gly Ser Gly Ser Gln Thr Arg Ala Phe Gln
                290                 295                 300

Tyr Val Ser Asp Leu Val Asn Gly Leu Val Ala Leu Met Asn Ser
                305                 310                 315

Asn Val Ser Ser Pro Val Asn Leu Gly Asn Pro Glu Glu His Thr
                320                 325                 330

Ile Leu Glu Phe Ala Gln Leu Ile Lys Asn Leu Val Gly Ser Gly
                335                 340                 345

Ser Glu Ile Gln Phe Leu Ser Glu Ala Gln Asp Asp Pro Gln Lys
                350                 355                 360

Arg Lys Pro Asp Ile Lys Lys Ala Lys Leu Met Leu Gly Trp Glu
                365                 370                 375

Pro Val Val Pro Leu Glu Glu Gly Leu Asn Lys Ala Ile His Tyr
                380                 385                 390

Phe Arg Lys Glu Leu Glu Tyr Gln Ala Asn Asn Gln Tyr Ile Pro
                395                 400                 405

Lys Pro Lys Pro Ala Arg Ile Lys Lys Gly Arg Thr Arg His Ser
                410                 415                 420

<210> 63
<211> 1123
<212> DNA
<213> Homo sapiens

<400> 63
cgtctgtaga gatatcatga acttcaactt agctttggta ctttcttccc 50

tgaagacaga gggcagaact ctgagttcca gaaccatttt caactgtatt 100

ggggaccaat cacttgactc tattcttgtc tctctgacag atgacgctac 150

actctcctct gaataatgga caccatttct aaaactgaat cctgctacta 200

aaataattca gatgatatat ttttccaatt ctacaatctt gctttgtttt 250

atttagttgt tttctctctc tcttcccagt tttccagaga ctggagctaa 300

actgggcttt caacatcatc atgaagttta tcctcctctg ggccctcttg 350

aatctgactg ttgctttggc ctttaatcca gattacacag tcagctccac 400

tcccccttac ttggtctatt tgaaatctga ctacttgccc tgcgctggag 450

tcctgatcca cccgctttgg gtgatcacag ctgcacactg caatttacca 500

aagcttcggg tgatattggg ggttacaatc ccagcagact ctaatgaaaa 550

gcatctgcaa gtgattggct atgagaagat gattcatcat ccacacttct 600

cagtcacttc tattgatcat gacatcatgc taatcaagct gaaaacagag 650

gctgaactca atgactatgt gaaattagcc aacctgccct accaaactat 700

ctctgaaaat accatgtgct ctgtctctac ctggagctac aatgtgtgtg 750

atatctacaa agagcccgat tcactgcaaa ctgtgaacat ctctgtaatc 800

tccaagcctc agtgtcgcga tgcctataaa acctacaaca tcacggaaaa 850

tatgctgtgt gtgggcattg tgccaggaag gaggcagccc tgcaaggaag 900

tttctgctgc cccggcaatc tgcaatggga tgcttcaagg aatcctgtct 950

tttgcggatg gatgtgtttt gagagccgat gttggcatct atgccaaaat 1000

tttttactat ataccctgga ttgaaaatgt aatccaaaat aactgagctg 1050

tggcagttgt ggaccatatg acacagcttg tccccatcgt tcacctttag 1100

aattaaatat aaattaactc ctc 1123

<210> 64
<211> 241
<212> PRT
<213> Homo sapiens

<400> 64
Met Lys Phe Ile Leu Leu Trp Ala Leu Leu Asn Leu Thr Val Ala
1               5                   10                  15

Leu Ala Phe Asn Pro Asp Tyr Thr Val Ser Ser Thr Pro Pro Tyr
                20                  25                  30

Leu Val Tyr Leu Lys Ser Asp Tyr Leu Pro Cys Ala Gly Val Leu
                35                  40                  45

Ile His Pro Leu Trp Val Ile Thr Ala Ala His Cys Asn Leu Pro
                50                  55                  60

Lys Leu Arg Val Ile Leu Gly Val Thr Ile Pro Ala Asp Ser Asn
                65                  70                  75

Glu Lys His Leu Gln Val Ile Gly Tyr Glu Lys Met Ile His His
                80                  85                  90

Pro His Phe Ser Val Thr Ser Ile Asp His Asp Ile Met Leu Ile
                95                  100                 105

Lys Leu Lys Thr Glu Ala Glu Leu Asn Asp Tyr Val Lys Leu Ala
                110                 115                 120

Asn Leu Pro Tyr Gln Thr Ile Ser Glu Asn Thr Met Cys Ser Val

125                 130                 135

Ser Thr Trp Ser Tyr Asn Val Cys Asp Ile Tyr Lys Glu Pro Asp
                140                 145                 150

Ser Leu Gln Thr Val Asn Ile Ser Val Ile Ser Lys Pro Gln Cys
                155                 160                 165

Arg Asp Ala Tyr Lys Thr Tyr Asn Ile Thr Glu Asn Met Leu Cys
                170                 175                 180

Val Gly Ile Val Pro Gly Arg Arg Gln Pro Cys Lys Glu Val Ser
                185                 190                 195

Ala Ala Pro Ala Ile Cys Asn Gly Met Leu Gln Gly Ile Leu Ser
                200                 205                 210

Phe Ala Asp Gly Cys Val Leu Arg Ala Asp Val Gly Ile Tyr Ala
                215                 220                 225

Lys Ile Phe Tyr Tyr Ile Pro Trp Ile Glu Asn Val Ile Gln Asn
                230                 235                 240

Asn

<210> 65
<211> 1333
<212> DNA
<213> Homo sapiens

<400> 65
gttttattga caatacatgc atcatatctt ttgactttga aggatatctc 50

atgtcaaagg aatcaagtta tgatttatag aggattcagc tggaatacct 100

tgtgggtgct ggctgagggt ggcaaaacgc ctaccgagac atgaaggttt 150

tagccactag ttttgtcctt gggagcctgg ggttggcctt ctacctgcct 200

ttggtggtga ctacacctaa aacactggcc atccctgaga agctgcaaga 250

agctgtgggg aaagttatca tcaatgccac aacctgtact gtcacctgtg 300

gccttggcta taaggaggag accgtctgtg aggtgggccc tgatggagtg 350

agaaggaaat gtcagactca gcgcttagaa tgtctgacca actggatctg 400

tgggatgctc catttcacca ttctcattgg caaggaattt gagcttagct 450

gtctgagttc agacatcttg gagtttggac aggaagcttt ccggttcacc 500

tggagacttg ctcgaggtgt catctccact gacgatgagg tcttcaaacc 550

ctttcaagcc aactcccact ttgtgaagtt taaatatgct caggagtatg 600

actctgggac atatcgctgt gatgtgcagc tggtaaaaaa cttgagactt 650

gtcaagaggc tctattttgg gttgagggtc cttcctccta acttggtgaa 700

tctgaatttc catcagtcac ttactgagga tcagaagtta atagatgagg 750

gattggaagt taatctggac agctactcca agcctcacca cccaaagtgg 800

aaaaagaagg tggcgtcagc cttgggaata ggaattgcca ttggagtggt 850

tggtggcgtg ttggtgagga ttgtcctctg tgcgctaagg gggggcctgc 900

agcagtgaca gcttcaagaa cttaacagcc ttgctcctga agaactggct 950

gcccaggaag ccaagctagc tttttagggg agtgttccag ctgctggtag 1000

tggatcagct tagagggaac actcccacag ccaaaagaat gagtgggaga 1050

aatggagggg acaatctcct gggagctatg cgcagtaacc taacttcctt 1100

atgtcccatg gatctcttcc tgatcttccc tgcccattgg gtacccagga 1150

aactgcaagc attgcctgtg ttcctgggaa gagttctaag aagcttgcat 1200

tcattttcta ccctttatga cttggatgcc tccccacctc catttcccct 1250

cttctgagct gtgtattcat gtagagggat gtattcagcc tttttagtga 1300

acattttttt tcaataaaag taattcacag taa 1333

<210> 66
<211> 255
<212> PRT
<213> Homo sapiens

<400> 66
Met Lys Val Leu Ala Thr Ser Phe Val Leu Gly Ser Leu Gly Leu
1               5                   10                  15

Ala Phe Tyr Leu Pro Leu Val Val Thr Thr Pro Lys Thr Leu Ala
                20                  25                  30

Ile Pro Glu Lys Leu Gln Glu Ala Val Gly Lys Val Ile Ile Asn
                35                  40                  45

Ala Thr Thr Cys Thr Val Thr Cys Gly Leu Gly Tyr Lys Glu Glu
                50                  55                  60

Thr Val Cys Glu Val Gly Pro Asp Gly Val Arg Arg Lys Cys Gln
                65                  70                  75

Thr Gln Arg Leu Glu Cys Leu Thr Asn Trp Ile Cys Gly Met Leu
                80                  85                  90

His Phe Thr Ile Leu Ile Gly Lys Glu Phe Glu Leu Ser Cys Leu
                95                  100                 105

Ser Ser Asp Ile Leu Glu Phe Gly Gln Glu Ala Phe Arg Phe Thr
                110                 115                 120

Trp Arg Leu Ala Arg Gly Val Ile Ser Thr Asp Asp Glu Val Phe
                125                 130                 135

Lys Pro Phe Gln Ala Asn Ser His Phe Val Lys Phe Lys Tyr Ala
                140                 145                 150

Gln Glu Tyr Asp Ser Gly Thr Tyr Arg Cys Asp Val Gln Leu Val
                155                 160                 165

Lys Asn Leu Arg Leu Val Lys Arg Leu Tyr Phe Gly Leu Arg Val
                170                 175                 180

Leu Pro Pro Asn Leu Val Asn Leu Asn Phe His Gln Ser Leu Thr
                185                 190                 195

Glu Asp Gln Lys Leu Ile Asp Glu Gly Leu Glu Val Asn Leu Asp
                200                 205                 210

Ser Tyr Ser Lys Pro His His Pro Lys Trp Lys Lys Lys Val Ala
                215                 220                 225

Ser Ala Leu Gly Ile Gly Ile Ala Ile Gly Val Val Gly Gly Val
                230                 235                 240

Leu Val Arg Ile Val Leu Cys Ala Leu Arg Gly Gly Leu Gln Gln
                245                 250                 255

<210> 67
<211> 1677
<212> DNA
<213> Homo sapiens

<400> 67
aatagaagtc ctcaggacgg agcagaggtg gccggcgggc ccggctgact 50

gcgcctctgc tttctttcca taaccttttc tttcggactc gaatcacggc 100

tgctgcgaag ggtctagttc cggacactag ggtgcccgaa cgcgctgatg 150

ccccgagtgc tcgcagggct tcccgctaac catgctgccg ccgccgcggc 200

ccgcagctgc cttggcgctg cctgtgctcc tgctactgct ggtggtgctg 250

acgccgcccc cgaccggcgc aaggccatcc ccaggcccag attacctgcg 300

gcgcggctgg atgcggctgc tagcggaggg cgagggctgc gctccctgcc 350

ggccagaaga gtgcgccgcg ccgcggggct gcctggcggg cagggtgcgc 400

gacgcgtgcg gctgctgctg ggaatgcgcc aacctcgagg gccagctctg 450

cgacctggac cccagtgctc acttctacgg gcactgcggc gagcagcttg 500

agtgccggct ggacacaggc ggcgacctga gccgcggaga ggtgccggaa 550

cctctgtgtg cctgtcgttc gcagagtccg ctctgcgggt ccgacggtca 600

cacctactcc cagatctgcc gcctgcagga ggcggcccgc gctcggcccg 650

atgccaacct cactgtggca cacccggggc cctgcgaatc ggggccccag 700

atcgtgtcac atccatatga cacttggaat gtgacagggc aggatgtgat 750

ctttggctgt gaagtgtttg cctaccccat ggcctccatc gagtggagga 800

aggatggctt ggacatccag ctgccagggg atgaccccca catctctgtg 850

cagtttaggg gtggacccca gaggtttgag gtgactggct ggctgcagat 900

ccaggctgtg cgtcccagtg atgagggcac ttaccgctgc cttggccgca 950

atgccctggg tcaagtggag gcccctgcta gcttgacagt gctcacacct 1000

gaccagctga actctacagg catcccccag ctgcgatcac taaacctggt 1050

tcctgaggag gaggctgaga gtgaagagaa tgacgattac tactaggtcc 1100

agagctctgg cccatggggg tgggtgagcg gctatagtgt tcatccctgc 1150

tcttgaaaag acctggaaag gggagcaggg tcccttcatc gactgctttc 1200

atgctgtcag tagggatgat catgggaggc ctatttgact ccaaggtagc 1250

agtgtggtag gatagagaca aaagctggag gagggtaggg agagaagctg 1300

agaccaggac cggtggggta caaaggggcc catgcaggag atgccctggc 1350

cagtaggacc tccaacaggt tgtttcccag gctggggtgg gggcctgagc 1400

agacacagag gtgcaggcac caggattctc cacttcttcc agccctgctg 1450

ggccacagtt ctaactgccc ttcctcccag gccctggttc ttgctatttc 1500

ctggtcccca acgtttatct agcttgtttg ccctttcccc aaactcatct 1550

tccagaactt ttccctctct cctaagcccc agttgcacct actaactgca 1600

gtcccttttg ctgtctgccg tcttttgtac aagagagaga acagcggagc 1650

atgacttagt tcagtgcaga gagattt 1677

<210> 68
<211> 304
<212> PRT
<213> Homo sapiens

<400> 68
Met Leu Pro Pro Pro Arg Pro Ala Ala Ala Leu Ala Leu Pro Val
1               5                   10                  15

Leu Leu Leu Leu Leu Val Val Leu Thr Pro Pro Pro Thr Gly Ala
                20                  25                  30

Arg Pro Ser Pro Gly Pro Asp Tyr Leu Arg Arg Gly Trp Met Arg
                35                  40                  45

Leu Leu Ala Glu Gly Glu Gly Cys Ala Pro Cys Arg Pro Glu Glu
                50                  55                  60

Cys Ala Ala Pro Arg Gly Cys Leu Ala Gly Arg Val Arg Asp Ala
                65                  70                  75

Cys Gly Cys Cys Trp Glu Cys Ala Asn Leu Glu Gly Gln Leu Cys
                80                  85                  90

Asp Leu Asp Pro Ser Ala His Phe Tyr Gly His Cys Gly Glu Gln
                95                  100                 105

Leu Glu Cys Arg Leu Asp Thr Gly Gly Asp Leu Ser Arg Gly Glu
                110                 115                 120

Val Pro Glu Pro Leu Cys Ala Cys Arg Ser Gln Ser Pro Leu Cys
                125                 130                 135

Gly Ser Asp Gly His Thr Tyr Ser Gln Ile Cys Arg Leu Gln Glu
                140                 145                 150

Ala Ala Arg Ala Arg Pro Asp Ala Asn Leu Thr Val Ala His Pro
                155                 160                 165

Gly Pro Cys Glu Ser Gly Pro Gln Ile Val Ser His Pro Tyr Asp
                170                 175                 180

Thr Trp Asn Val Thr Gly Gln Asp Val Ile Phe Gly Cys Glu Val
                185                 190                 195

Phe Ala Tyr Pro Met Ala Ser Ile Glu Trp Arg Lys Asp Gly Leu
                200                 205                 210

Asp Ile Gln Leu Pro Gly Asp Asp Pro His Ile Ser Val Gln Phe
                215                 220                 225

Arg Gly Gly Pro Gln Arg Phe Glu Val Thr Gly Trp Leu Gln Ile
                230                 235                 240

Gln Ala Val Arg Pro Ser Asp Glu Gly Thr Tyr Arg Cys Leu Gly
                245                 250                 255

Arg Asn Ala Leu Gly Gln Val Glu Ala Pro Ala Ser Leu Thr Val
                260                 265                 270

Leu Thr Pro Asp Gln Leu Asn Ser Thr Gly Ile Pro Gln Leu Arg
                275                 280                 285

Ser Leu Asn Leu Val Pro Glu Glu Glu Ala Glu Ser Glu Glu Asn
                290                 295                 300

Asp Asp Tyr Tyr

<210> 69
<211> 882
<212> DNA
<213> Homo sapiens

<400> 69
gcgtggtgcg ggggcgtggg gaaatcgggt tgccccagcc gttactggtc 50

cgcgcagtca gggcatcctc cgcatcctcc acatccttcc atggctctga 100

agaataaatt cagttgttta tggatcttgg gtctgtgttt ggtagccact 150

acatcttcca aaatcccatc catcactgac ccacacttta tagacaactg 200

catagaagcc cacaacgaat ggcgtggcaa agtcaaccct cccgcggccg 250

acatgaaata catgatttgg gataaaggtt tagcaaagat ggctaaagca 300

tgggcaaacc agtgcaaatt tgaacataat gactgtttgg ataaatcata 350

taaatgctat gcagcttttg aatatgttgg agaaaatatc tggttaggtg 400

gaataaagtc attcacacca agacatgcca ttacggcttg gtataatgaa 450

acccaatttt atgattttga tagtctatca tgctccagag tctgtggcca 500

ttatacacag ttagtttggg ccaattcatt ttatgtcggt tgtgcagttg 550

caatgtgtcc taaccttggg ggagcttcaa ctgcaatatt tgtatgcaac 600

tacggacctg caggaaattt tgcaaatatg cctccttacg caagaggaga 650

atcttgctct ctctgctcaa aagaagagaa atgtgtaaag aacctctgca 700

ggactccaca acttattata cctaaccaaa atccatttct gaagccaacg 750

gggagagcac ctcagcagac agcctttaat ccattcagct taggttttct 800

tcttctgaga atcttttaat gtcatttata tacaaaagaa attctcaaat 850

gttaaaataa aggaatagtt tattgcttaa ta 882

<210> 70
<211> 484
<212> PRT
<213> Homo sapiens

<400> 70

<210> 71
<211> 957
<212> DNA
<213> Homo sapiens

<400> 71
gtgagacttc tttcttcatt gtggctagct ttgaaaagac cctctgaact 50

tcctaaagat atcaagatga tatcaccaga cttgcccttt ttgacaattg 100

tcttgatcat agttagttgg acaacttgtg gagcactagc catacttctt 150

tcttatcttt actatgtgtt taaggttgtt catctgcaag ccagcttaac 200

aacttttaag aatagccagc ctgtgaatcc caaacactct agaagaagtg 250

aaaagaaatc caatcatcat aaagactcct caatacacca tcttcgttta 300

tctgccaacg atgctgaaga tagccttcgc atgcacagta ctgtgattaa 350

cttactaaca tggattgtat tactcagcat gccttctcta atttattggc 400

taaagaatct taggtattat tttaaactta atcctgatcc atgtaaacct 450

ttggcattta tccttattcc gactatggca attcttggaa atacttacac 500

tgtttcaata aaatcaagta aattgttgaa gactacttca caatttccac 550

ttcctctggc tgttggtgtg attgcttttg ggtcagcaca tttatatagg 600

cttccatgct ttgtcttcat tcctctttta ctccatgcat tatgcaactt 650

tatgtaagat tggacttaag gaatgatgaa gataatttat gtgtttaggg 700

ccagtgataa gagggaacac acagatccat cagtatggac agcaagatcc 750

tttggagaag acaagtctat ttttacaata ttgaaaatag gaaattagtt 800

ttgtaatgtt tgagggaagt agttgaagca tggttttgtt ttgtggtgtg 850

gaatccatgt actaatcatt tttgaaaaat tcatgaaggg atatatggtg 900

atcactatca ttgaggactc ctgtgcatat aaaatagtct gttttatcaa 950

ctgtaaa 957

<210> 72
<211> 196
<212> PRT
<213> Homo sapiens

<400> 72
Met Ile Ser Pro Asp Leu Pro Phe Leu Thr Ile Val Leu Ile Ile
1               5                   10                  15

Val Ser Trp Thr Thr Cys Gly Ala Leu Ala Ile Leu Leu Ser Tyr
                20                  25                  30

Leu Tyr Tyr Val Phe Lys Val Val His Leu Gln Ala Ser Leu Thr
                35                  40                  45

Thr Phe Lys Asn Ser Gln Pro Val Asn Pro Lys His Ser Arg Arg
                50                  55                  60

Ser Glu Lys Lys Ser Asn His His Lys Asp Ser Ser Ile His His
                65                  70                  75

Leu Arg Leu Ser Ala Asn Asp Ala Glu Asp Ser Leu Arg Met His
                80                  85                  90

Ser Thr Val Ile Asn Leu Leu Thr Trp Ile Val Leu Leu Ser Met
                95                  100                 105

Pro Ser Leu Ile Tyr Trp Leu Lys Asn Leu Arg Tyr Tyr Phe Lys
                110                 115                 120

Leu Asn Pro Asp Pro Cys Lys Pro Leu Ala Phe Ile Leu Ile Pro
                125                 130                 135

Thr Met Ala Ile Leu Gly Asn Thr Tyr Thr Val Ser Ile Lys Ser
                140                 145                 150

Ser Lys Leu Leu Lys Thr Thr Ser Gln Phe Pro Leu Pro Leu Ala
                155                 160                 165

Val Gly Val Ile Ala Phe Gly Ser Ala His Leu Tyr Arg Leu Pro
                170                 175                 180

Cys Phe Val Phe Ile Pro Leu Leu Leu His Ala Leu Cys Asn Phe

                185                 190                 195

Met

<210> 73
<211> 2473
<212> DNA
<213> Homo sapiens

<400> 73
cccagccccg cgttcggctg ctctcgagga ggccggagtc cccggagacg 50

atgcgccccg cgcagccgcc tgcgcctgcg ggagccggct gcccttgaga 100

tggagttgct gcctctttgg ctctgcctgg gttttcactt cctgaccgtg 150

ggctggagga acagaagcgg aacagccaca gcagcctccc aaggagtctg 200

caagttggtg ggtggagccg ctgactgccg agggcagagc ctcgcttcgg 250

tgcccagcag cctcccgccc cacgcccgga tgctcaccct ggatgccaac 300

cctctcaaga ccctgtggaa tcactccctc cagccttacc ctctcctgga 350

gagcctcagc ctgcacagct gccacctgga gcgcatcagc cgcggcgcct 400

tccaggagca aggtcacctg cgcagcctgg tcctggggga caactgcctc 450

tcagagaact acgaagagac ggcagccgcc ctccacgccc tgccgggcct 500

gcggaggctg gacttgtcag gaaacgccct gacggaggac atggcagcgc 550

tcatgctcca gaacctctcc tcgctgcggt ccgtgtccct ggcggggaac 600

accatcatgc ggctggacga ctccgtcttc gagggcctgg agcgtctccg 650

ggagctggat ctgcagagga actacatctt cgagatcgag ggcggcgctt 700

tcgacggcct ggctgagctg aggcacctca acctggcctt caacaacctc 750

ccctgcatcg tggacttcgg gctcacgcgg ctgcgggtcc tcaacgtcag 800

ctacaacgtc ctggagtggt tcctcgcgac cgggggagag gctgccttcg 850

agctggagac gctggacctg tctcacaacc agctgctgtt cttcccgctg 900

ctgccccagt acagcaagtt gcggaccctc ctgctgcgcg acaacaacat 950

gggcttctac cgggacctgt acaacacctc gtcgccgagg gagatggtgg 1000

cccagttcct cctcgtggac ggcaacgtga ccaacatcac caccgtcagc 1050

ctctgggaag aattctcctc cagcgacctc gcagatctcc gcttcctgga 1100

catgagccag aaccagttcc agtacctgcc agacggcttc ctgaggaaaa 1150

tgccttccct ctcccacctg aacctccacc agaattgcct gatgacgctt 1200

cacattcggg agcacgagcc ccccggagcg ctcaccgagc tggacctgag 1250

ccacaaccag ctgtcggagc tgcacctggc tccggggctg gccagctgcc 1300

tgggcagcct gcgcttgttc aacctgagct ccaaccagct cctgggcgtc 1350

ccccctggcc tcttcgccaa tgctaggaac atcactacac ttgacatgag 1400

ccacaatcag atctcacttt gtcccctgcc agctgcctcg gaccgggtgg 1450

gcccccctag ctgtgtggat ttcaggaata tggcatcttt aaggagcctg 1500

tctctggagg gctgtggcct gggggcattg ccagactgcc cattccaagg 1550

gacctccctg acctacttag acctctcaag caactggggg gttctgaatg 1600

ggagcctcgc cccactccag gatgttgccc ccatgttaca ggtcctgtct 1650

ctcaggaaca tgggcctcca ctccagcttt atggcgttgg acttctctgg 1700

gtttgggaat ctcagggact tagatctgtc ggggaattgc ttgaccacct 1750

tcccaaggtt tgggggcagc ctggccctgg agaccctgga tctccgtaga 1800

aactcgctca cagcccttcc ccagaaggct gtgtctgagc agctctcgag 1850

aggtctgcgg accatctacc tcagtcagaa tccatatgac tgctgtgggg 1900

tggatggctg gggggccctg cagcatgggc agacggtggc cgactgggcc 1950

atggtcacct gcaacctctc ctccaagatc atccgcgtga cggagctgcc 2000

cggaggtgtg cctcgggact gcaagtggga gcggctggac ctgggcctgc 2050

tctacctcgt gctcatcctc cccagctgcc tcaccctgct ggtggcctgc 2100

actgtcatcg tcctcacttt taagaagcct ctgcttcagg tcatcaagag 2150

ccgctgccac tggtcctccg tttactgacc tggctgtgtg ccaagactcg 2200

aaattcggtc cgcacacaac aggacacttt ctctgccagc tttcaagatg 2250

tgatgcagag gccaagtctg acgaattgaa gtttcaatta aaatttaata 2300

tgtttccatt cctcatcgcc caccccaccc ccgcccccac caccgcccaa 2350

gttctttttc catcattata attcatcctt attatcttgg taaaatattt 2400

attaagtgac tttttcagaa ataaaaggca acgtgtctca taaatatttt 2450

ttaaaaaaaa aaaaaaaaaa aaa 2473

<210> 74
<211> 692
<212> PRT
<213> Homo sapiens

<400> 74
Met Glu Leu Leu Pro Leu Trp Leu Cys Leu Gly Phe His Phe Leu
1               5                   10                  15

Thr Val Gly Trp Arg Asn Arg Ser Gly Thr Ala Thr Ala Ala Ser
                20                  25                  30

Gln Gly Val Cys Lys Leu Val Gly Gly Ala Ala Asp Cys Arg Gly
                35                  40                  45

Gln Ser Leu Ala Ser Val Pro Ser Ser Leu Pro Pro His Ala Arg
                50                  55                  60

Met Leu Thr Leu Asp Ala Asn Pro Leu Lys Thr Leu Trp Asn His
                65                  70                  75

Ser Leu Gln Pro Tyr Pro Leu Leu Glu Ser Leu Ser Leu His Ser
                80                  85                  90

Cys His Leu Glu Arg Ile Ser Arg Gly Ala Phe Gln Glu Gln Gly
                95                  100                 105

His Leu Arg Ser Leu Val Leu Gly Asp Asn Cys Leu Ser Glu Asn
                110                 115                 120

Tyr Glu Glu Thr Ala Ala Ala Leu His Ala Leu Pro Gly Leu Arg
                125                 130                 135

Arg Leu Asp Leu Ser Gly Asn Ala Leu Thr Glu Asp Met Ala Ala
                140                 145                 150

Leu Met Leu Gln Asn Leu Ser Ser Leu Arg Ser Val Ser Leu Ala
                155                 160                 165

Gly Asn Thr Ile Met Arg Leu Asp Asp Ser Val Phe Glu Gly Leu
                170                 175                 180

Glu Arg Leu Arg Glu Leu Asp Leu Gln Arg Asn Tyr Ile Phe Glu
                185                 190                 195

Ile Glu Gly Gly Ala Phe Asp Gly Leu Ala Glu Leu Arg His Leu
                200                 205                 210

Asn Leu Ala Phe Asn Asn Leu Pro Cys Ile Val Asp Phe Gly Leu
                215                 220                 225

Thr Arg Leu Arg Val Leu Asn Val Ser Tyr Asn Val Leu Glu Trp
                230                 235                 240

Phe Leu Ala Thr Gly Gly Glu Ala Ala Phe Glu Leu Glu Thr Leu
                245                 250                 255

Asp Leu Ser His Asn Gln Leu Leu Phe Phe Pro Leu Leu Pro Gln
                260                 265                 270

Tyr Ser Lys Leu Arg Thr Leu Leu Leu Arg Asp Asn Asn Met Gly
                275                 280                 285

Phe Tyr Arg Asp Leu Tyr Asn Thr Ser Ser Pro Arg Glu Met Val
                290                 295                 300

Ala Gln Phe Leu Leu Val Asp Gly Asn Val Thr Asn Ile Thr Thr
                305                 310                 315

Val Ser Leu Trp Glu Glu Phe Ser Ser Ser Asp Leu Ala Asp Leu
                320                 325                 330

Arg Phe Leu Asp Met Ser Gln Asn Gln Phe Gln Tyr Leu Pro Asp
                335                 340                 345

Gly Phe Leu Arg Lys Met Pro Ser Leu Ser His Leu Asn Leu His
                350                 355                 360

Gln Asn Cys Leu Met Thr Leu His Ile Arg Glu His Glu Pro Pro
                365                 370                 375

Gly Ala Leu Thr Glu Leu Asp Leu Ser His Asn Gln Leu Ser Glu
                380                 385                 390

Leu His Leu Ala Pro Gly Leu Ala Ser Cys Leu Gly Ser Leu Arg
                395                 400                 405

Leu Phe Asn Leu Ser Ser Asn Gln Leu Leu Gly Val Pro Pro Gly
                410                 415                 420

Leu Phe Ala Asn Ala Arg Asn Ile Thr Thr Leu Asp Met Ser His
                425                 430                 435

Asn Gln Ile Ser Leu Cys Pro Leu Pro Ala Ala Ser Asp Arg Val
                440                 445                 450

Gly Pro Pro Ser Cys Val Asp Phe Arg Asn Met Ala Ser Leu Arg
                455                 460                 465

Ser Leu Ser Leu Glu Gly Cys Gly Leu Gly Ala Leu Pro Asp Cys
                470                 475                 480

Pro Phe Gln Gly Thr Ser Leu Thr Tyr Leu Asp Leu Ser Ser Asn
                485                 490                 495

Trp Gly Val Leu Asn Gly Ser Leu Ala Pro Leu Gln Asp Val Ala
                500                 505                 510

Pro Met Leu Gln Val Leu Ser Leu Arg Asn Met Gly Leu His Ser
                515                 520                 525

Ser Phe Met Ala Leu Asp Phe Ser Gly Phe Gly Asn Leu Arg Asp
                530                 535                 540

Leu Asp Leu Ser Gly Asn Cys Leu Thr Thr Phe Pro Arg Phe Gly
                545                 550                 555

Gly Ser Leu Ala Leu Glu Thr Leu Asp Leu Arg Arg Asn Ser Leu
                560                 565                 570

Thr Ala Leu Pro Gln Lys Ala Val Ser Glu Gln Leu Ser Arg Gly

                575                 580                 585

Leu Arg Thr Ile Tyr Leu Ser Gln Asn Pro Tyr Asp Cys Cys Gly
                590                 595                 600

Val Asp Gly Trp Gly Ala Leu Gln His Gly Gln Thr Val Ala Asp
                605                 610                 615

Trp Ala Met Val Thr Cys Asn Leu Ser Ser Lys Ile Ile Arg Val
                620                 625                 630

Thr Glu Leu Pro Gly Gly Val Pro Arg Asp Cys Lys Trp Glu Arg
                635                 640                 645

Leu Asp Leu Gly Leu Leu Tyr Leu Val Leu Ile Leu Pro Ser Cys
                650                 655                 660

Leu Thr Leu Leu Val Ala Cys Thr Val Ile Val Leu Thr Phe Lys
                665                 670                 675

Lys Pro Leu Leu Gln Val Ile Lys Ser Arg Cys His Trp Ser Ser
                680                 685                 690

Val Tyr

<210> 75
<211> 2025
<212> DNA
<213> Homo sapiens

<400> 75
gcccggtgga gaattaggtg ctgctgggag ctcctgcctc ccacaggatt 50

ccagctgcag ggagcctcag ggactctggg ccgcacggag ttgggggcat 100

tccccagaga gcgtcgccat ggtctgcagg gagcagttat caaagaatca 150

ggtcaagtgg gtgtttgccg gcattacctg tgtgtctgtg gtggtcattg 200

ccgcaatagt ccttgccatc accctgcggc ggccaggctg tgagctggag 250

gcctgcagcc ctgatgccga catgctggac tacctgctga gcctgggcca 300

gatcagccgg cgagatgcct tggaggtcac ctggtaccac gcagccaaca 350

gcaagaaagc catgacagct gccctgaaca gcaacatcac agtcctggag 400

gctgacgtca atgtagaagg gctcggcaca gccaatgaga caggagttcc 450

catcatggca caccccccca ctatctacag tgacaacaca ctggagcagt 500

ggctggacgc tgtgctgggc tcttcccaaa agggcatcaa actggacttc 550

aagaacatca aggcagtggg cccctccctg gacctcctgc ggcagctgac 600

agaggaaggc aaagtccggc ggcccatatg gatcaacgct gacatcttaa 650

agggccccaa catgctcatc tcaactgagg tcaatgccac acagttcctg 700

gccctggtcc aggagaagta tcccaaggct accctatctc caggctggac 750

caccttctac atgtccacgt ccccaaacag gacgtacacc caagccatgg 800

tggagaagat gcacgagctg gtgggaggag tgccccagag ggtcaccttc 850

cctgtacggt cttccatggt gcgggctgcc tggccccact tcagctggct 900

gctgagccaa tctgagaggt acagcctgac gctgtggcag gctgcctcgg 950

accccatgtc ggtggaagat ctgctctacg tccgggataa cactgctgtc 1000

caccaagtct actatgacat ctttgagcct ctcctgtcac agttcaagca 1050

gctggccttg aatgccacac ggaaaccaat gtactacacg ggaggcagcc 1100

tgatccctct tctccagctg cctggggatg acggtctgaa tgtggagtgg 1150

ctggttcctg acgtccaggg cagcggtaaa acagcaacaa tgaccctccc 1200

agacacagaa ggcatgatcc tgctgaacac tggcctcgag ggaactgtgg 1250

ctgaaaaccc cgtgcccatt gttcatactc caagtggcaa catcctgacg 1300

ctggagtcct gcctgcagca gctggccaca catcccggac actggggcat 1350

ccatttgcaa atagtggagc ccgcagccct ccggccatcc ctggccttgc 1400

tggcacgcct ctccagcctt ggcctcttgc attggcctgt gtgggttggg 1450

gccaaaatct cccacgggag tttttcggtc cccggccatg tggctggcag 1500

agagctgctt acagctgtgg ctgaggtctt cccccacgtg actgtggcac 1550

caggctggcc tgaggaggtg ctgggcagtg gctacaggga acagctgctc 1600

acagatatgc tagagttgtg ccaggggctc tggcaacctg tgtccttcca 1650

gatgcaggcc atgctgctgg gccacagcac agctggagcc ataggcaggc 1700

tgctggcatc ctcccccggg gccaccgtca cagtggagca caacccagct 1750

gggggcgact atgcctctgt gaggacagca ttgctggcag ctagggctgt 1800

ggacaggacc cgagtctact acaggctacc ccagggctac cacaaggact 1850

tgctggctca tgttggtaga aactgagcac ccaggggtgg tgggccagcg 1900

gacctcaggg cggaggcttc ccacggggag gcaggaagaa ataaaggtct 1950

ttggctttct ccaggcaaaa aaaaaaaaaa aaaaaaaaaa aaaaaaaaaa 2000

aaaaaaaaaa aaaaaaaaaa aaaag 2025

<210> 76
<211> 585

<212> PRT
<213> Homo sapiens

<400> 76

<210> 77
<211> 2012
<212> DNA
<213> Homo sapiens

<400> 77
ttcctagaag gtgagcgcgg acggtatgca aagttgtgaa tccagtggtg 50

acagtgcgga tgaccctctc agtcgcggcc tacggagaag gggacagcct 100

cgtgtggtgg tgatcggcgc cggcttggct ggcctggctg cagccaaagc 150

acttcttgag cagggtttca cggatgtcac tgtgcttgag gcttccagcc 200

acatcggagg ccgtgtgcag agtgtgaaac ttggacacgc cacctttgag 250

ctgggagcca cctggatcca tggctcccat gggaacccta tctatcatct 300

agcagaagcc aacggcctcc tggaagagac aaccgatggg gaacgcagcg 350

tgggccgcat cagcctctat tccaagaatg gcgtggcctg ctaccttacc 400

aaccacggcc gcaggatccc caaggacgtg gttgaggaat tcagcgattt 450

atacaacgag gtctataact tgacccagga gttcttccgg cacgataaac 500

cagtcaatgc tgaaagtcaa aatagcgtgg gggtgttcac ccgagaggag 550

gtgcgtaacc gcatcaggaa tgaccctgac gacccagagg ctaccaagcg 600

cctgaagctc gccatgatcc agcagtacct gaaggtggag agctgtgaga 650

gcagctcaca cagcatggac gaggtgtccc tgagcgcctt cggggagtgg 700

accgagatcc ccggcgctca ccacatcatc ccctcgggct tcatgcgggt 750

tgtggagctg ctggcggagg gcatccctgc ccacgtcatc cagctaggga 800

aacctgtccg ctgcattcac tgggaccagg cctcagcccg ccccagaggc 850

cctgagattg agccccgggg tgagggcgac cacaatcacg acactgggga 900

gggtggccag ggtggagagg agccccgggg gggcaggtgg gatgaggatg 950

agcagtggtc ggtggtggtg gagtgcgagg actgtgagct gatcccggcg 1000

gaccatgtga ttgtgaccgt gtcgctaggt gtgctaaaga ggcagtacac 1050

cagtttcttc cggccaggcc tgcccacaga gaaggtggct gccatccacc 1100

gcctgggcat tggcaccacc gacaagatct ttctggaatt cgaggagccc 1150

ttctggggcc ctgagtgcaa cagcctacag tttgtgtggg aggacgaagc 1200

agagagccac accctcacct acccacctga gctctggtac cgcaagatct 1250

gcggctttga tgtcctctac ccgcctgagc gctacggcca tgtgctgagc 1300

ggctggatct gcggggagga ggccctcgtc atggagaagt gtgatgacga 1350

ggcagtggcc gagatctgca cggagatgct gcgtcagttc acaggaaacc 1400

ccaacattcc aaaacctcgg cgaatcttgc gctcggcctg gggcagcaac 1450

ccttacttcc gcggctccta ttcatacacg caggtgggct ccagcggggc 1500

ggatgtggag aagctggcca agcccctgcc gtacacagag agctcaaaga 1550

cagcgcccat gcaggtgctg ttttccggtg aggccaccta ccgcaagtac 1600

tattccacca cccacggtgc tctgctgtcc ggccagcgtg aggctgcccg 1650

cctcattgag atgtaccgag acctcttcca gcaggggacc tgagggctgt 1700

cctcgctgct gagaagagcc actaactcgt gacctccagc ctgccccttg 1750

ctgccgtgtg ctcctgcctt cctgatcctc tgtagaaagg atttttatct 1800

tctgtagagc tagccgccct gactgccttc agacctggcc ctgtagcttt 1850

tctttttctc caggctgggc cgtgagcagg tgggccgttg agttacctct 1900

gtgctggatc ccgtgccccc acttgcctac cctctgtcct gccttgttat 1950

tgtaagtgcc ttcaatactt tgcattttgg gataataaaa aaggctccct 2000

cccctgccaa aa 2012

<210> 78
<211> 555
<212> PRT
<213> Homo sapiens

<400> 78

<210> 79
<211> 994
<212> DNA
<213> Homo sapiens

<220>
<221> Unsure
<222> 992
<223> Unknown base

<400> 79
gttgggcagc agccacccgc tcacctccat ccccaggact tagagggacg 50

cagggcgttg ggaacagagg acactccagg cgctgaccct gggaggccag 100

gaccagggcc aaagtcccgt gggcaagagg agtcctcaga ggtccttcat 150

tcagcggttc cgggaggtct gggaagccca cggcctggct ggggcagggt 200

caacgccgcc aggccgccat ggtcctgtgc tggctgctgc ttctggtgat 250

ggctctgccc ccaggcacga cgggcgtcaa ggactgcgtc ttctgtgagc 300

tcaccgactc catgcagtgt cctggtacct acatgcactg tggcgatgac 350

gaggactgct tcacaggcca cggggtcgcc ccgggcactg gtccggtcat 400

caacaaaggc tgcctgcgag ccaccagctg cggccttgag gaacccgtca 450

gctacagggg cgtcacctac agcctcacca ccaactgctg caccggccgc 500

ctgtgtaaca gagccccgag cagccagaca gtgggggcca ccaccagcct 550

ggcactgggg ctgggtatgc tgcttcctcc acgtttgctg tgaccaacag 600

ggaggacagg gcctgggact gttctcccag atccgccact ccccatgtcc 650

ccatgtcctt cccccactaa atggccagag aggccctgga caacctcttg 700

cggccctggc ttcatccctt ctaaggctgt ccaccaggag cccggtgcta 750

ggggaagcat ccccaggcct gactgagcgg caggggagca cggcccgtgg 800

gtttgattgt attactctgt tccactggtt ctaagacgca gagcttctca 850

catctcaatc aggatgcttc tctccattgg tagcacttta gagtccatga 900

aatatggtaa aaaatatata tatatcataa taaatgacag ctgatgttca 950

tgggggaaaa aaaaaaaaaa aaaaaaaaaa aaaaaaaaaa anga 994

<210> 80
<211> 124
<212> PRT
<213> Homo sapiens

<400> 80
Met Val Leu Cys Trp Leu Leu Leu Leu Val Met Ala Leu Pro Pro
1               5                   10                  15

Gly Thr Thr Gly Val Lys Asp Cys Val Phe Cys Glu Leu Thr Asp
                20                  25                  30

Ser Met Gln Cys Pro Gly Thr Tyr Met His Cys Gly Asp Asp Glu
                35                  40                  45

Asp Cys Phe Thr Gly His Gly Val Ala Pro Gly Thr Gly Pro Val
                50                  55                  60

Ile Asn Lys Gly Cys Leu Arg Ala Thr Ser Cys Gly Leu Glu Glu
                65                  70                  75

Pro Val Ser Tyr Arg Gly Val Thr Tyr Ser Leu Thr Thr Asn Cys
                80                  85                  90

Cys Thr Gly Arg Leu Cys Asn Arg Ala Pro Ser Ser Gln Thr Val
                95                  100                 105

Gly Ala Thr Thr Ser Leu Ala Leu Gly Leu Gly Met Leu Leu Pro
                110                 115                 120

Pro Arg Leu Leu

<210> 81
<211> 3976
<212> DNA
<213> Homo sapiens

<400> 81
caagtccgtt gaggctgcca ggcgagtcag gtctctctgg acctcgcctg 50

actcggctgg gctgtgcctg aaattgaccc agctccacca tactccttga 100

ttatgagaaa acaaggagta agctcaaagc ggctgcaatc ttccggccgc 150

agccagtcta aggggcggcg cggggcctcc ctcgcccggg agccggaggt 200

agaggaggag gtggaaaagt cggtcctagg cggcgggaaa ctgccaaggg 250

gcgcctggag gtcctccccg gggaggatcc aaagtctgaa agagcgaaaa 300

ggcttggagc tagaggtggt ggccaagacc tttcttctcg gccccttcca 350

gttcgtccgt aattccctgg cgcagctccg ggaaaaggtg caggaactgc 400

aggcgcggcg gttctccagc agaaccactc tcggcatcgc tgtctttgtg 450

gcaattttac attggttaca tttagtaaca ctttttgaaa atgatcgtca 500

tttctctcac ctctcatctt tggaacggga gatgactttt cgcactgaaa 550

tgggacttta ttattcatac ttcaagacca ttattgaagc accttcgttt 600

ttggaaggac tgtggatgat tatgaatgac aggcttactg aatatcctct 650

tataattaat gcaataaaac gcttccatct ttatccagag gtaatcatag 700

cctcctggta ttgcacattc atgggaataa tgaatttatt tggactagaa 750

actaagacct gctggaatgt caccagaata gaacctctta atgaagttca 800

aagctgtgaa ggattgggag atcctgcttg cttttatgtt ggtgtaatct 850

ttattttaaa tggactaatg atgggattgt tcttcatgta tggagcatac 900

ctgagtggga ctcaactggg aggtcttatt acagtactgt gcttcttttt 950

caaccatgga gaggccaccc gtgtgatgtg gacaccacct ctccgtgaaa 1000

gtttttccta tcctttcctt gtacttcaga tgtgtatttt aactttgatt 1050

ctcaggacct caagcaatga tagaaggccc ttcattgcac tctgtctttc 1100

caatgttgct tttatgcttc cctggcaatt tgctcagttt atacttttta 1150

cacagatagc atcattattt cccatgtatg ttgtgggata cattgaacca 1200

agcaaatttc agaagatcat ttatatgaac atgatttcag ttacccttag 1250
tttcattttg atgtttggaa attcaatgta cttatcttct tattattctt 1300
catctttgtt aatgacgtgg gcaataattc taaagagaaa tgaaattcaa 1350
aaactgggag tatctaaact caacttttgg ctaattcaag gtagtgcctg 1400
gtggtgtgga acaatcattt tgaaatttct gacatctaaa atcttaggcg 1450
tttcagacca cattcgcctg agtgatctta tagcagccag aatcttaagg 1500
tatacagatt ttgatacttt aatatatacc tgtgctcccg aatttgactt 1550
catggaaaaa gcgactccgc tgagatacac aaagacatta ttgcttccag 1600
ttgttatggt gattacatgt tttatcttta aaaagactgt tcgtgatatt 1650
tcatatgttt tagctacaaa catttatcta agaaaacagc tccttgaaca 1700
cagtgagctg gcttttcaca cattgcagtt gttagtgttt actgcccttg 1750
ccattttaat tatgaggcta aagatgtttt tgacaccgca catgtgtgtt 1800
atggcttcct tgatatgctc tcgacagctc tttggctggc tttttcgcag 1850
agttcgtttt gagaaggtta tctttggcat tttaacagtg atgtcaatac 1900
aaggttatgc aaacctccgt aatcaatgga gcataatagg agaatttaat 1950
aatttgcctc aggaagaact tttacagtgg atcaaataca gtaccacatc 2000
agatgctgtc tttgcaggtg ccatgcctac aatggcaagc atcaagctgt 2050
ctacacttca tcccattgtg aatcatccac attacgaaga tgcagacttg 2100
agggctcgga caaaaatagt ttattctaca tatagtcgaa aatctgccaa 2150
agaagtaaga gataaattgt tggagttaca tgtgaattat tatgttttag 2200
aagaggcatg gtgtgttgtg agaactaagc ctggttgcag tatgcttgaa 2250
atctgggatg tggaagaccc ttccaatgca gctaaccctc ccttatgtag 2300
cgtcctgctc gaagacgcca ggccttactt caccacagta tttcagaata 2350
gtgtgtacag agtattaaag gttaactgag aaggatacta cccattttac 2400
tatggcacaa tgccgtgtgt caaaaacaat caccctttgg cttattcaca 2450
ttaataaaaa tcacaagctt taataacaga cacttaaaaa taagataaaa 2500
atggattgga aatttttctg attactaaaa ggtaaattac ttttctgttc 2550
attgaatgtc agccttatta agcttgtcat ataagttatt aaatcattca 2600
tgtcatactg cataaacaaa tgttcatttc agaattttaa agagaaatgt 2650

atataaagaa cmatgatttt aataatcagg ggtatgtaag tcctttttca 2700

tccaactagg tgaattgctt cagattttct ctagtaccag agggtacctc 2750

ctcaaactct ttgaaccact taaggcagaa gaatgcaagc tctgaaatga 2800

catccttaaa atgctgatac tggtcacagc ctctttacct ctgtgaggaa 2850

attgtaacag tgtgtctttt aaggtgtttt tattttacca gcccttaaga 2900

aagatctcta atacctttta atactttttt ttaataattt caagttgaag 2950

tgtttttaaa aacactttgt tttgtaatgt tttgaatctc ttgagatgtg 3000

tttaccccac tagatacata tttgccactg gttagttctc catctaagct 3050

caagaggtta ttcatctctc tttagattcc agtggctttt cttttaacat 3100

ccaggtaaaa cagaaactgc tatggtatac aaccaagttt tggggttaaa 3150

cataatcaga aaagaaaatc cagttaaatt tatgaagtga gattttcaga 3200

tcctagatct tgaataaagg aaaggtcttt tcatcttgat ggccccaaag 3250

cttgttggtc atggtcttta tttctggcca ctatcttctt aaataatata 3300

tttttaagcc ctcatttatt tttggttttg ggtgaggaaa gtcatgtttt 3350

ctaagtcctc tcccctaata aaacctaccc aacaatagtg ctttgaaaag 3400

tggtagttat cttgaagata ctcttgccaa atgcaaagat aaacattctt 3450

tttgtctgct ttataaatat gaaatatgcc agatctatag tattttaatg 3500

tgcatctact ttaaatgagt catcttgggg tttttataat tcccttatgt 3550

tcttgcccct ctacacttga aataacaaaa tgccttaatt ttatggatta 3600

gttctcttat agtagacagg cagctatatg cagcaaaacc aataaagtta 3650

tttttcaact ttcatagttg taaaatatct tataccagaa tacaaaacag 3700

ctaagaaaac atgccacatt ttattttagc attttcaaat aatttgtttt 3750

tggtgtaagc acaggataaa aaaggagagc gtcaaagaaa agagacataa 3800

cacctaacat tcataaaaat taacaaagta tattttggat gatgttttta 3850

caggaaatat tttaaataag ttggtagaac ttttaaaatg gtactgtatt 3900

agctaataaa atattcagta caaatatatg tttggattta tgcattaaaa 3950

aactaataaa attatttcca acttta 3976

<210> 82
<211> 758
<212> PRT
<213> Homo sapiens

<400> 82
Met Arg Lys Gln Gly Val Ser Ser Lys Arg Leu Gln Ser Ser Gly
1               5                   10                  15

Arg Ser Gln Ser Lys Gly Arg Arg Gly Ala Ser Leu Ala Arg Glu
                20                  25                  30

Pro Glu Val Glu Glu Glu Val Glu Lys Ser Val Leu Gly Gly Gly
                35                  40                  45

Lys Leu Pro Arg Gly Ala Trp Arg Ser Ser Pro Gly Arg Ile Gln
                50                  55                  60

Ser Leu Lys Glu Arg Lys Gly Leu Glu Leu Glu Val Val Ala Lys
                65                  70                  75

Thr Phe Leu Leu Gly Pro Phe Gln Phe Val Arg Asn Ser Leu Ala
                80                  85                  90

Gln Leu Arg Glu Lys Val Gln Glu Leu Gln Ala Arg Arg Phe Ser
                95                  100                 105

Ser Arg Thr Thr Leu Gly Ile Ala Val Phe Val Ala Ile Leu His
                110                 115                 120

Trp Leu His Leu Val Thr Leu Phe Glu Asn Asp Arg His Phe Ser
                125                 130                 135

His Leu Ser Ser Leu Glu Arg Glu Met Thr Phe Arg Thr Glu Met
                140                 145                 150

Gly Leu Tyr Tyr Ser Tyr Phe Lys Thr Ile Ile Glu Ala Pro Ser
                155                 160                 165

Phe Leu Glu Gly Leu Trp Met Ile Met Asn Asp Arg Leu Thr Glu
                170                 175                 180

Tyr Pro Leu Ile Ile Asn Ala Ile Lys Arg Phe His Leu Tyr Pro
                185                 190                 195

Glu Val Ile Ile Ala Ser Trp Tyr Cys Thr Phe Met Gly Ile Met
                200                 205                 210

Asn Leu Phe Gly Leu Glu Thr Lys Thr Cys Trp Asn Val Thr Arg
                215                 220                 225

Ile Glu Pro Leu Asn Glu Val Gln Ser Cys Glu Gly Leu Gly Asp
                230                 235                 240

Pro Ala Cys Phe Tyr Val Gly Val Ile Phe Ile Leu Asn Gly Leu
                245                 250                 255

Met Met Gly Leu Phe Phe Met Tyr Gly Ala Tyr Leu Ser Gly Thr
                260                 265                 270

Gln Leu Gly Gly Leu Ile Thr Val Leu Cys Phe Phe Phe Asn His
                275                 280                 285

Gly Glu Ala Thr Arg Val Met Trp Thr Pro Pro Leu Arg Glu Ser
                290                 295                 300

Phe Ser Tyr Pro Phe Leu Val Leu Gln Met Cys Ile Leu Thr Leu
                305                 310                 315

Ile Leu Arg Thr Ser Ser Asn Asp Arg Arg Pro Phe Ile Ala Leu
                320                 325                 330

Cys Leu Ser Asn Val Ala Phe Met Leu Pro Trp Gln Phe Ala Gln
                335                 340                 345

Phe Ile Leu Phe Thr Gln Ile Ala Ser Leu Phe Pro Met Tyr Val
                350                 355                 360

Val Gly Tyr Ile Glu Pro Ser Lys Phe Gln Lys Ile Ile Tyr Met
                365                 370                 375

Asn Met Ile Ser Val Thr Leu Ser Phe Ile Leu Met Phe Gly Asn
                380                 385                 390

Ser Met Tyr Leu Ser Ser Tyr Tyr Ser Ser Ser Leu Leu Met Thr
                395                 400                 405

Trp Ala Ile Ile Leu Lys Arg Asn Glu Ile Gln Lys Leu Gly Val
                410                 415                 420

Ser Lys Leu Asn Phe Trp Leu Ile Gln Gly Ser Ala Trp Trp Cys
                425                 430                 435

Gly Thr Ile Ile Leu Lys Phe Leu Thr Ser Lys Ile Leu Gly Val
                440                 445                 450

Ser Asp His Ile Arg Leu Ser Asp Leu Ile Ala Ala Arg Ile Leu
                455                 460                 465

Arg Tyr Thr Asp Phe Asp Thr Leu Ile Tyr Thr Cys Ala Pro Glu
                470                 475                 480

Phe Asp Phe Met Glu Lys Ala Thr Pro Leu Arg Tyr Thr Lys Thr
                485                 490                 495

Leu Leu Leu Pro Val Val Met Val Ile Thr Cys Phe Ile Phe Lys
                500                 505                 510

Lys Thr Val Arg Asp Ile Ser Tyr Val Leu Ala Thr Asn Ile Tyr
                515                 520                 525

Leu Arg Lys Gln Leu Leu Glu His Ser Glu Leu Ala Phe His Thr
                530                 535                 540

Leu Gln Leu Leu Val Phe Thr Ala Leu Ala Ile Leu Ile Met Arg
                545                 550                 555

Leu Lys Met Phe Leu Thr Pro His Met Cys Val Met Ala Ser Leu
                560                 565                 570

Ile Cys Ser Arg Gln Leu Phe Gly Trp Leu Phe Arg Arg Val Arg
                575                 580                 585

Phe Glu Lys Val Ile Phe Gly Ile Leu Thr Val Met Ser Ile Gln
                590                 595                 600

Gly Tyr Ala Asn Leu Arg Asn Gln Trp Ser Ile Ile Gly Glu Phe
                605                 610                 615

Asn Asn Leu Pro Gln Glu Glu Leu Leu Gln Trp Ile Lys Tyr Ser
                620                 625                 630

Thr Thr Ser Asp Ala Val Phe Ala Gly Ala Met Pro Thr Met Ala
                635                 640                 645

Ser Ile Lys Leu Ser Thr Leu His Pro Ile Val Asn His Pro His
                650                 655                 660

Tyr Glu Asp Ala Asp Leu Arg Ala Arg Thr Lys Ile Val Tyr Ser
                665                 670                 675

Thr Tyr Ser Arg Lys Ser Ala Lys Glu Val Arg Asp Lys Leu Leu
                680                 685                 690

Glu Leu His Val Asn Tyr Tyr Val Leu Glu Glu Ala Trp Cys Val
                695                 700                 705

Val Arg Thr Lys Pro Gly Cys Ser Met Leu Glu Ile Trp Asp Val
                710                 715                 720

Glu Asp Pro Ser Asn Ala Ala Asn Pro Pro Leu Cys Ser Val Leu
                725                 730                 735

Leu Glu Asp Ala Arg Pro Tyr Phe Thr Thr Val Phe Gln Asn Ser
                740                 745                 750

Val Tyr Arg Val Leu Lys Val Asn
                755

<210> 83
<211> 1294
<212> DNA
<213> Homo sapiens

<400> 83
   taggtggcgg gcgggtactt aaggcgcggc caccgggctg gcagtgcgcc 50
   caacagcgga ctccgagacc agcggatctc ggcaaaccct ctttctcgac 100
   cacccaccta ccattcttgg aaccatggcg gcagtggcgg cggcctcggc 150
   tgaactgctc atcatcggct ggtacatctt ccgcgtgctg ctgcaggtgt 200
   tcctggaatg ctgcatttac tgggtaggat tcgcttttcg aaatcctcca 250
   gggacacagc ccattgcgag aagtgaggtg ttcaggtact ccctgcagaa 300
   gctggcatac acggtgtcgc ggaccgggcg gcaggtgttg ggggagcgca 350
   ggcagcgagc ccccaactga ggccccagct cccagccctg ggcggccgta 400
   tcatcaggtg ctcctgtgca tctcggccag cacgggagcc agtgccgcgc 450
   aggaatgtgg ggtcccctgt gttccctcgc cagagcactt ggcaaggtca 500
   gtgaggggcc agtagacccc cggagaagca gtaccgacaa tgacgaagat 550
   accagatccc ttcccaaccc ctttgcaccg gtcccactaa ggggcagggt 600
   cgagagagga ggggggatag ggggagcaga ccctgagatc tgggcatagg 650
   caccgcattc tgatctggac aaagtcggga cagcaccatc ccagccccga 700
   agcccgggcc atgccagcag gccccaccat ggaaatcaaa acaccgcacc 750
   agccagcaga atggacattc tgacatcgcc agccgacgcc ctgaatcttg 800
   gtgcagcacc caccgcgtgc ctgtgtggcg ggactggagg gcacagttga 850
   ggaaggaggg tggttaagaa atacagtggg gccctctcgc tgtcccttgc 900
   ccagggcact tgtattccag cctcgctgca tttgctctct cgattgcccc 950
   tttcctccta catgcctccc aagcccaccc tactccaaaa gtaatgtgtc 1000
   acttgatttg gaactattca agcagtaaaa gtaaatgaat cccaccttta 1050
   ctaaaacact ttctctgaac cccccttgcc cctcactgat cttgcttttc 1100
   cctggtctca gcagttgtgg tcaatattgt ggtaatcgct aattgtactg 1150
   attgtttaag tgtgcattag ttgtctctcc ccagctagat tgtaagctcc 1200
   tggaggacag ggaccacctc tacaaaaaat aaaaaaagta cctcccctgt 1250
   ctcgcacagt gtcccaggac cctgcggtgc agtagaggcg cacc 1294

<210> 84
<211> 81
<212> PRT
<213> Homo sapiens

<400> 84
Met Ala Ala Val Ala Ala Ala Ser Ala Glu Leu Leu Ile Ile Gly
1               5                   10                  15

Trp Tyr Ile Phe Arg Val Leu Leu Gln Val Phe Leu Glu Cys Cys
                20                  25                  30

Ile Tyr Trp Val Gly Phe Ala Phe Arg Asn Pro Pro Gly Thr Gln
                35                  40                  45

Pro Ile Ala Arg Ser Glu Val Phe Arg Tyr Ser Leu Gln Lys Leu
                50                  55                  60

Ala Tyr Thr Val Ser Arg Thr Gly Arg Gln Val Leu Gly Glu Arg

                65                  70                  75

Arg Gln Arg Ala Pro Asn
                80

<210> 85
<211> 3583
<212> DNA
<213> Homo sapiens

<400> 85
ctccccggcg ccgcaggcag cgtcctcctc cgaagcagct gcacctgcaa 50

ctgggcagcc tggaccctcg tgccctgttc ccgggacctc gcgcaggggg 100

cgccccggga caccccctgc gggccgggtg gaggaggaag aggaggagga 150

ggaagaagac gtggacaagg acccccatcc tacccagaac acctgcctgc 200

gctgccgcca cttctcttta agggagagga aaagagagcc taggagaacc 250

atggggggct gcgaagtccg ggaatttctt ttgcaatttg gtttcttctt 300

gcctctgctg acagcgtggc caggcgactg cagtcacgtc tccaacaacc 350

aagttgtgtt gcttgataca acaactgtac tgggagagct aggatggaaa 400

acatatccat taaatgggtg ggatgccatc actgaaatgg atgaacataa 450

taggcccatt cacacatacc aggtatgtaa tgtaatggaa ccaaaccaaa 500

acaactggct tcgtacaaac tggatctccc gtgatgcagc tcagaaaatt 550

tatgtggaaa tgaaattcac actaagggat tgtaacagca tcccatgggt 600

cttggggact tgcaaagaaa catttaatct gttttatatg gaatcagatg 650

agtcccacgg aattaaattc aagccaaacc agtatacaaa gatcgacaca 700

attgctgctg atgagagttt tacccagatg gatttgggtg atcgcatcct 750

caaactcaac actgaaattc gtgaggtggg gcctatagaa aggaaaggat 800

tttatctggc ttttcaagac attggggcgt gcattgccct ggtttcagtc 850

cgtgttttct acaagaaatg ccccttcact gttcgtaact tggccatgtt 900

tcctgatacc attccaaggg ttgattcctc ctctttggtt gaagtacggg 950

gttcttgtgt gaagagtgct gaagagcgtg acactcctaa actgtattgt 1000

ggagctgatg gagattggct ggttcctctt ggaaggtgca tctgcagtac 1050

aggatatgaa gaaattgagg gttcttgcca tgcttgcaga ccaggattct 1100

ataaagcttt tgctgggaac acaaaatgtt ctaaatgtcc tccacacagt 1150

ttaacataca tggaagcaac ttctgtctgt cagtgtgaaa agggttattt 1200

ccgagctgaa aaagacccac cttctatggc atgtaccagg ccaccttcag 1250

ctcctaggaa tgtggttttt aacatcaatg aaacagccct tattttggaa 1300

tggagcccac caagtgacac aggagggaga aaagatctca catacagtgt 1350

aatctgtaag aaatgtggct tagacaccag ccagtgtgag gactgtggtg 1400

gaggactccg cttcatccca agacatacag gcctgatcaa caattccgtg 1450

atagtacttg actttgtgtc tcacgtgaat tacacctttg aaatagaagc 1500

aatgaatgga gtttctgagt tgagtttttc tcccaagcca ttcacagcta 1550

ttacagtgac cacggatcaa gatgcacctt ccctgatagg tgtggtaagg 1600

aaggactggg catcccaaaa tagcattgcc ctatcatggc aagcacctgc 1650

tttttccaat ggagccattc tggactacga gatcaagtac tatgagaaag 1700

aacatgagca gctgacctac tcttccacaa ggtccaaagc ccccagtgtc 1750

atcatcacag gtcttaagcc agccaccaaa tatgtatttc acatccgagt 1800

gagaactgcg acaggataca gtggctacag tcagaaattt gaatttgaaa 1850

caggagatga aacttctgac atggcagcag aacaaggaca gattctcgtg 1900

atagccaccg ccgctgttgg cggattcact ctcctcgtca tcctcacttt 1950

attcttcttg atcactggga gatgtcagtg gtacataaaa gccaagatga 2000

agtcagaaga gaagagaaga aaccacttac agaatgggca tttgcgcttc 2050

ccgggaatta aaacttacat tgatccagat acatatgaag acccatccct 2100

agcagtccat gaatttgcaa aggagattga tccctcaaga attcgtattg 2150

agagagtcat tggggcaggt gaatttggag aagtctgtag tgggcgtttg 2200

aagacaccag ggaaaagaga gatcccagtt gccattaaaa ctttgaaagg 2250

tggccacatg gatcggcaaa gaagagattt tctaagagaa gctagtatca 2300

tgggccagtt tgaccatcca aacatcattc gcctagaagg ggttgtcacc 2350

aaaagatcct tcccggccat tggggtggag gcgttttgcc ccagcttcct 2400

gagggcaggg tttttaaata gcatccaggc cccgcatcca gtgccagggg 2450

gaggatcttt gccccccagg attcctgctg gcagaccagt aatgattgtg 2500

gtggaatata tggagaatgg atccctagac tcctttttgc ggaagcatga 2550

tggccacttc acagtcatcc agttggtcgg aatgctccga ggcattgcat 2600

caggcatgaa gtatctttct gatatgggtt atgttcatcg agacctagcg 2650

gctcggaata tactggtcaa tagcaactta gtatgcaaag tttctgattt 2700

tggtctctcc agagtgctgg aagatgatcc agaagctgct tatacaacaa 2750

ctggtggaaa aatccccata aggtggacag ccccagaagc catcgcctac 2800

agaaaattct cctcagcaag cgatgcatgg agctatggca ttgtcatgtg 2850

ggaggtcatg tcctatggag agagacctta ttgggaaatg tctaaccaag 2900

atgtcattct gtccattgaa gaagggtaca gacttccagc tcccatgggc 2950

tgtccagcat ctctacacca gctgatgctc cactgctggc agaaggagag 3000

aaatcacaga ccaaaattta ctgacattgt cagcttcctt gacaaactga 3050

tccgaaatcc cagtgccctt cacaccctgg tggaggacat ccttgtaatg 3100

ccagagtccc ctggtgaagt tccggaatat cctttgtttg tcacagttgg 3150

tgactggcta gattctataa agatggggca atacaagaat aacttcgtgg 3200

cagcagggtt tacaacattt gacctgattt caagaatgag cattgatgac 3250

attagaagaa ttggagtcat acttattgga caccagagac gaatagtcag 3300

cagcatacag actttacgtt tacacatgat gcacatacag gagaagggat 3350

ttcatgtatg aaagtaccac aagcacctgt gttttgtgcc tcagcatttc 3400

taaaatgaac gatatcctct ctactactct ctcttctgat tctccaaaca 3450

tcacttcaca aactgcagtc ttctgttcag actataggca cacaccttat 3500

gtttatgctt ccaaccagga ttttaaaatc atgctacata aatccgttct 3550

gaataacctg caactaaaaa aaaaaaaaaa aaa 3583

<210> 86
<211> 1036
<212> PRT
<213> Homo sapiens

<400> 86
Met Gly Gly Cys Glu Val Arg Glu Phe Leu Leu Gln Phe Gly Phe
1               5                   10                  15

Phe Leu Pro Leu Leu Thr Ala Trp Pro Gly Asp Cys Ser His Val
                20                  25                  30

Ser Asn Asn Gln Val Val Leu Leu Asp Thr Thr Thr Val Leu Gly
                35                  40                  45

Glu Leu Gly Trp Lys Thr Tyr Pro Leu Asn Gly Trp Asp Ala Ile
                50                  55                  60

Thr Glu Met Asp Glu His Asn Arg Pro Ile His Thr Tyr Gln Val

                65                  70                  75

Cys Asn Val Met Glu Pro Asn Gln Asn Asn Trp Leu Arg Thr Asn
                80                  85                  90

Trp Ile Ser Arg Asp Ala Ala Gln Lys Ile Tyr Val Glu Met Lys
                95                  100                 105

Phe Thr Leu Arg Asp Cys Asn Ser Ile Pro Trp Val Leu Gly Thr
                110                 115                 120

Cys Lys Glu Thr Phe Asn Leu Phe Tyr Met Glu Ser Asp Glu Ser
                125                 130                 135

His Gly Ile Lys Phe Lys Pro Asn Gln Tyr Thr Lys Ile Asp Thr
                140                 145                 150

Ile Ala Ala Asp Glu Ser Phe Thr Gln Met Asp Leu Gly Asp Arg
                155                 160                 165

Ile Leu Lys Leu Asn Thr Glu Ile Arg Glu Val Gly Pro Ile Glu
                170                 175                 180

Arg Lys Gly Phe Tyr Leu Ala Phe Gln Asp Ile Gly Ala Cys Ile
                185                 190                 195

Ala Leu Val Ser Val Arg Val Phe Tyr Lys Lys Cys Pro Phe Thr
                200                 205                 210

Val Arg Asn Leu Ala Met Phe Pro Asp Thr Ile Pro Arg Val Asp
                215                 220                 225

Ser Ser Ser Leu Val Glu Val Arg Gly Ser Cys Val Lys Ser Ala
                230                 235                 240

Glu Glu Arg Asp Thr Pro Lys Leu Tyr Cys Gly Ala Asp Gly Asp
                245                 250                 255

Trp Leu Val Pro Leu Gly Arg Cys Ile Cys Ser Thr Gly Tyr Glu
                260                 265                 270

Glu Ile Glu Gly Ser Cys His Ala Cys Arg Pro Gly Phe Tyr Lys
                275                 280                 285

Ala Phe Ala Gly Asn Thr Lys Cys Ser Lys Cys Pro Pro His Ser
                290                 295                 300

Leu Thr Tyr Met Glu Ala Thr Ser Val Cys Gln Cys Glu Lys Gly
                305                 310                 315

Tyr Phe Arg Ala Glu Lys Asp Pro Pro Ser Met Ala Cys Thr Arg
                320                 325                 330

Pro Pro Ser Ala Pro Arg Asn Val Val Phe Asn Ile Asn Glu Thr
                335                 340                 345

Ala Leu Ile Leu Glu Trp Ser Pro Pro Ser Asp Thr Gly Gly Arg
                350                 355                 360

Lys Asp Leu Thr Tyr Ser Val Ile Cys Lys Lys Cys Gly Leu Asp
                365                 370                 375

Thr Ser Gln Cys Glu Asp Cys Gly Gly Gly Leu Arg Phe Ile Pro
                380                 385                 390

Arg His Thr Gly Leu Ile Asn Asn Ser Val Ile Val Leu Asp Phe
                395                 400                 405

Val Ser His Val Asn Tyr Thr Phe Glu Ile Glu Ala Met Asn Gly
                410                 415                 420

Val Ser Glu Leu Ser Phe Ser Pro Lys Pro Phe Thr Ala Ile Thr
                425                 430                 435

Val Thr Thr Asp Gln Asp Ala Pro Ser Leu Ile Gly Val Val Arg
                440                 445                 450

Lys Asp Trp Ala Ser Gln Asn Ser Ile Ala Leu Ser Trp Gln Ala
                455                 460                 465

Pro Ala Phe Ser Asn Gly Ala Ile Leu Asp Tyr Glu Ile Lys Tyr
                470                 475                 480

Tyr Glu Lys Glu His Glu Gln Leu Thr Tyr Ser Ser Thr Arg Ser
                485                 490                 495

Lys Ala Pro Ser Val Ile Ile Thr Gly Leu Lys Pro Ala Thr Lys
                500                 505                 510

Tyr Val Phe His Ile Arg Val Arg Thr Ala Thr Gly Tyr Ser Gly
                515                 520                 525

Tyr Ser Gln Lys Phe Glu Phe Glu Thr Gly Asp Glu Thr Ser Asp
                530                 535                 540

Met Ala Ala Glu Gln Gly Gln Ile Leu Val Ile Ala Thr Ala Ala
                545                 550                 555

Val Gly Gly Phe Thr Leu Leu Val Ile Leu Thr Leu Phe Phe Leu
                560                 565                 570

Ile Thr Gly Arg Cys Gln Trp Tyr Ile Lys Ala Lys Met Lys Ser
                575                 580                 585

Glu Glu Lys Arg Arg Asn His Leu Gln Asn Gly His Leu Arg Phe
                590                 595                 600

Pro Gly Ile Lys Thr Tyr Ile Asp Pro Asp Thr Tyr Glu Asp Pro
                605                 610                 615

Ser Leu Ala Val His Glu Phe Ala Lys Glu Ile Asp Pro Ser Arg
                620                 625                 630

Ile Arg Ile Glu Arg Val Ile Gly Ala Gly Glu Phe Gly Glu Val
                635                 640                 645

Cys Ser Gly Arg Leu Lys Thr Pro Gly Lys Arg Glu Ile Pro Val
                650                 655                 660

Ala Ile Lys Thr Leu Lys Gly Gly His Met Asp Arg Gln Arg Arg
                665                 670                 675

Asp Phe Leu Arg Glu Ala Ser Ile Met Gly Gln Phe Asp His Pro
                680                 685                 690

Asn Ile Ile Arg Leu Glu Gly Val Val Thr Lys Arg Ser Phe Pro
                695                 700                 705

Ala Ile Gly Val Glu Ala Phe Cys Pro Ser Phe Leu Arg Ala Gly
                710                 715                 720

Phe Leu Asn Ser Ile Gln Ala Pro His Pro Val Pro Gly Gly Gly
                725                 730                 735

Ser Leu Pro Pro Arg Ile Pro Ala Gly Arg Pro Val Met Ile Val
                740                 745                 750

Val Glu Tyr Met Glu Asn Gly Ser Leu Asp Ser Phe Leu Arg Lys
                755                 760                 765

His Asp Gly His Phe Thr Val Ile Gln Leu Val Gly Met Leu Arg
                770                 775                 780

Gly Ile Ala Ser Gly Met Lys Tyr Leu Ser Asp Met Gly Tyr Val
                785                 790                 795

His Arg Asp Leu Ala Ala Arg Asn Ile Leu Val Asn Ser Asn Leu
                800                 805                 810

Val Cys Lys Val Ser Asp Phe Gly Leu Ser Arg Val Leu Glu Asp
                815                 820                 825

Asp Pro Glu Ala Ala Tyr Thr Thr Thr Gly Gly Lys Ile Pro Ile
                830                 835                 840

Arg Trp Thr Ala Pro Glu Ala Ile Ala Tyr Arg Lys Phe Ser Ser
                845                 850                 855

Ala Ser Asp Ala Trp Ser Tyr Gly Ile Val Met Trp Glu Val Met
                860                 865                 870

Ser Tyr Gly Glu Arg Pro Tyr Trp Glu Met Ser Asn Gln Asp Val
                875                 880                 885

Ile Leu Ser Ile Glu Glu Gly Tyr Arg Leu Pro Ala Pro Met Gly
                890                 895                 900

Cys Pro Ala Ser Leu His Gln Leu Met Leu His Cys Trp Gln Lys
                905                 910                 915

Glu Arg Asn His Arg Pro Lys Phe Thr Asp Ile Val Ser Phe Leu
                920                 925                 930

Asp Lys Leu Ile Arg Asn Pro Ser Ala Leu His Thr Leu Val Glu

                935                 940                 945

Asp Ile Leu Val Met Pro Glu Ser Pro Gly Glu Val Pro Glu Tyr
                950                 955                 960

Pro Leu Phe Val Thr Val Gly Asp Trp Leu Asp Ser Ile Lys Met
                965                 970                 975

Gly Gln Tyr Lys Asn Asn Phe Val Ala Ala Gly Phe Thr Thr Phe
                980                 985                 990

Asp Leu Ile Ser Arg Met Ser Ile Asp Asp Ile Arg Arg Ile Gly
                995                 1000                1005

Val Ile Leu Ile Gly His Gln Arg Arg Ile Val Ser Ser Ile Gln
                1010                1015                1020

Thr Leu Arg Leu His Met Met His Ile Gln Glu Lys Gly Phe His
                1025                1030                1035

Val

<210> 87
<211> 1322
<212> DNA
<213> Homo sapiens

<400> 87
ccaatgaatc cctgcggttg gctgggggca gtgggtccca cactgcctca 50

cttccctaaa tgggcagctt cacttttaga accccgggtc cttccctggc 100

aggcccaggt ggcacatcct gtgtcgggtg ggccctcacc ttggatctcc 150

aggcctgaca ctgcccagct ggatggaacc atggccccag ccttcctgct 200

gctgctgctg ctgtggccac agggttgcgt ctcaggcccc tctgctgaca 250

gtgtatacac aaaagtgagg ctccttgaag gggagactct gtctgtgcag 300

tgctcctata agggctacaa aaaccgcgtg gagggcaagg tttggtgcaa 350

aatcaggaag aagaagtgtg agcctggctt tgcccgagtc tgggtgaaag 400

ggccccgcta cttgctgcag gacgatgccc aggccaaggt ggtcaacatc 450

accatggtgg ccctcaagct ccaggactca ggccgatact ggtgcatgcg 500

caacacctct gggatcctgt accccttgat gggcttccag ctggatgtgt 550

ctccagctcc ccaaactgag aggaacattc ctttcacaca tctggacaac 600

atcctcaaga gtggaactgt cacaactggc caagccccta cctcaggccc 650

tgatgcccct tttaccactg gtgtgatggt gttcacccca ggactcatca 700

ccttgcctag gctcttagcc tccaccagac ctgcctccaa gacaggctac 750

agcttcactg ctaccagcac caccagccag ggacccagga ggaccatggg 800

gtcccagaca gtgaccgcgt ctcccagcaa tgccagggac tcctctgctg 850

gcccagaatc catctccact aagtctgggg acctcagcac cagatcgccc 900

accacagggc tctgcctcac cagcagatct ctcctcaaca gactaccctc 950

catgccctcc atcaggcacc aggatgttta ctccactgtg cttggggtgg 1000

tgctgaccct cctggtgctg atgctgatca tggtctatgg gttttggaag 1050

aagagacaca tggcaagcta cagcatgtgc agcgatcctt ctacacgtga 1100

cccacctgga agaccagagc cctatgtgga agtctacttg atctgaggcc 1150

acttaagcat ggggtgggga gcttctccca gagtggcccc agggggttag 1200

aggaggggtg aagattgggg ccagtatcga tcttatgaag ctggaggact 1250

tgtgcagtgc tggactcacc caggacttcc caaacccaga ggctgtcatc 1300

ctaagcagcc ccacagccca gt 1322

<210> 88
<211> 321
<212> PRT
<213> Homo sapiens

<400> 88
Met Ala Pro Ala Phe Leu Leu Leu Leu Leu Leu Trp Pro Gln Gly
1               5                   10                  15

Cys Val Ser Gly Pro Ser Ala Asp Ser Val Tyr Thr Lys Val Arg
                20                  25                  30

Leu Leu Glu Gly Glu Thr Leu Ser Val Gln Cys Ser Tyr Lys Gly
                35                  40                  45

Tyr Lys Asn Arg Val Glu Gly Lys Val Trp Cys Lys Ile Arg Lys
                50                  55                  60

Lys Lys Cys Glu Pro Gly Phe Ala Arg Val Trp Val Lys Gly Pro
                65                  70                  75

Arg Tyr Leu Leu Gln Asp Asp Ala Gln Ala Lys Val Val Asn Ile
                80                  85                  90

Thr Met Val Ala Leu Lys Leu Gln Asp Ser Gly Arg Tyr Trp Cys
                95                  100                 105

Met Arg Asn Thr Ser Gly Ile Leu Tyr Pro Leu Met Gly Phe Gln
                110                 115                 120

Leu Asp Val Ser Pro Ala Pro Gln Thr Glu Arg Asn Ile Pro Phe
                125                 130                 135

Thr His Leu Asp Asn Ile Leu Lys Ser Gly Thr Val Thr Thr Gly
                140                 145                 150

Gln Ala Pro Thr Ser Gly Pro Asp Ala Pro Phe Thr Thr Gly Val
                155                 160                 165

Met Val Phe Thr Pro Gly Leu Ile Thr Leu Pro Arg Leu Leu Ala
                170                 175                 180

Ser Thr Arg Pro Ala Ser Lys Thr Gly Tyr Ser Phe Thr Ala Thr
                185                 190                 195

Ser Thr Thr Ser Gln Gly Pro Arg Arg Thr Met Gly Ser Gln Thr
                200                 205                 210

Val Thr Ala Ser Pro Ser Asn Ala Arg Asp Ser Ser Ala Gly Pro
                215                 220                 225

Glu Ser Ile Ser Thr Lys Ser Gly Asp Leu Ser Thr Arg Ser Pro
                230                 235                 240

Thr Thr Gly Leu Cys Leu Thr Ser Arg Ser Leu Leu Asn Arg Leu
                245                 250                 255

Pro Ser Met Pro Ser Ile Arg His Gln Asp Val Tyr Ser Thr Val
                260                 265                 270

Leu Gly Val Val Leu Thr Leu Leu Val Leu Met Leu Ile Met Val
                275                 280                 285

Tyr Gly Phe Trp Lys Lys Arg His Met Ala Ser Tyr Ser Met Cys
                290                 295                 300

Ser Asp Pro Ser Thr Arg Asp Pro Pro Gly Arg Pro Glu Pro Tyr
                305                 310                 315

Val Glu Val Tyr Leu Ile
                320

<210> 89
<211> 1167
<212> DNA
<213> Homo sapiens

<400> 89
cgggatggga aaactatgcc tggggccgac gctctgcccg gctgctgccg 50

ctgaggaaag ccgggacgcg gagccccgcc gagagcttct ttgctccgga 100

cgcccctgga cgtggcgggc agccgcgagg gtaaccacca tgatcccctg 150

ggtgctcctg gcctgtgccc tcccctgtgc tgctgaccca ctgcttggcg 200

cctttgctcg cagggacttc cggaaaggct cccctcaact ggtctgcagc 250

ctgcctggcc cccagggccc acccggcccc ccaggagccc cagggccctc 300

aggaatgatg ggacgaatgg gctttcctgg caaagacggc caagatggac 350

acgacggcga ccggggggac agcggagagg aaggtccacc tggccggaca 400

ggtaaccggg gaaagccagg accaaagggc aaagccgggg ccattgggcg 450

ggctggcccc cgtggcccca agggggtcaa cggtaccccc gggaagcatg 500

gcacaccagg caagaagggg cccaagggca agaaagggga gccaggcctc 550

ccaggcccct gcagctgtgg cagtggccat accaagtcag ctttctcggt 600

ggcagtgacc aagagctacc cacgggagcg gctgcccatc aagtttgaca 650

agattctgat gaacgagggt ggccactaca atgcttccag cggcaagttc 700

gtctgcggcg tgcctgggat ctactacttc acctacgaca tcacgctggc 750

caacaagcac ctggccatcg gcctggtgca caacggccag taccgcatcc 800

ggacctttga tgccaacacc ggcaaccacg atgtggcctc aggctccacc 850

atcctggctc tcaagcaggg tgacgaagtt tggctgcaga tcttctactc 900

agagcagaac gggctcttct atgaccctta ctggacagac agcctcttta 950

cgggcttcct aatctatgcc gaccaggatg accccaacga ggtatagaca 1000

tgccacggcg gtcctccagg cagggaacaa gcttctggac ttgggcttac 1050

agagcaagac cccacaactg taggctgggg gtggggggtc gagtgagcgg 1100

ttctagcctc aggctcacct cctccgcctc tttttttccc cttcattaaa 1150

tccaaacctt tttattc 1167

<210> 90
<211> 330
<212> PRT
<213> Homo sapiens

<400> 90
Met Gly Lys Leu Cys Leu Gly Pro Thr Leu Cys Pro Ala Ala Ala
1               5                   10                  15

Ala Glu Glu Ser Arg Asp Ala Glu Pro Arg Arg Glu Leu Leu Cys
                20                  25                  30

Ser Gly Arg Pro Trp Thr Trp Arg Ala Ala Ala Arg Val Thr Thr
                35                  40                  45

Met Ile Pro Trp Val Leu Leu Ala Cys Ala Leu Pro Cys Ala Ala
                50                  55                  60

Asp Pro Leu Leu Gly Ala Phe Ala Arg Arg Asp Phe Arg Lys Gly
                65                  70                  75

Ser Pro Gln Leu Val Cys Ser Leu Pro Gly Pro Gln Gly Pro Pro
                80                  85                  90

Gly Pro Pro Gly Ala Pro Gly Pro Ser Gly Met Met Gly Arg Met
                95                  100                 105

Gly Phe Pro Gly Lys Asp Gly Gln Asp Gly His Asp Gly Asp Arg
                110                 115                 120

Gly Asp Ser Gly Glu Glu Gly Pro Pro Gly Arg Thr Gly Asn Arg
                125                 130                 135

Gly Lys Pro Gly Pro Lys Gly Lys Ala Gly Ala Ile Gly Arg Ala
                140                 145                 150

Gly Pro Arg Gly Pro Lys Gly Val Asn Gly Thr Pro Gly Lys His
                155                 160                 165

Gly Thr Pro Gly Lys Lys Gly Pro Lys Gly Lys Lys Gly Glu Pro
                170                 175                 180

Gly Leu Pro Gly Pro Cys Ser Cys Gly Ser Gly His Thr Lys Ser
                185                 190                 195

Ala Phe Ser Val Ala Val Thr Lys Ser Tyr Pro Arg Glu Arg Leu
                200                 205                 210

Pro Ile Lys Phe Asp Lys Ile Leu Met Asn Glu Gly Gly His Tyr
                215                 220                 225

Asn Ala Ser Ser Gly Lys Phe Val Cys Gly Val Pro Gly Ile Tyr
                230                 235                 240

Tyr Phe Thr Tyr Asp Ile Thr Leu Ala Asn Lys His Leu Ala Ile
                245                 250                 255

Gly Leu Val His Asn Gly Gln Tyr Arg Ile Arg Thr Phe Asp Ala
                260                 265                 270

Asn Thr Gly Asn His Asp Val Ala Ser Gly Ser Thr Ile Leu Ala
                275                 280                 285

Leu Lys Gln Gly Asp Glu Val Trp Leu Gln Ile Phe Tyr Ser Glu
                290                 295                 300

Gln Asn Gly Leu Phe Tyr Asp Pro Tyr Trp Thr Asp Ser Leu Phe
                305                 310                 315

Thr Gly Phe Leu Ile Tyr Ala Asp Gln Asp Asp Pro Asn Glu Val
                320                 325                 330

<210> 91
<211> 2977
<212> DNA
<213> Homo sapiens

<400> 91
ggatgcttgg cgctctacct cgccgcccct gagccttccc gtccgcctcg 50

ccacgcgccc ggacggcctg gggttgctgc ccgtcagtct cgaaaggtgt 100

ttttggggaa aaaaatcaca atctggacgt gagaaaggac atgaggagac 150
taaagacctg ggattttgtc aatcagaatg aaaccaatgt tgaaagactt 200
ttcaaatcta ttgttggtgg tactctgtga ctatgttctt ggagaagctg 250
aatatcttct cttgagagag ccaggccatg tagcactaag caacgacaca 300
gtgtatgtgg atttccagta ttttgatggt gctaatggga cactgaggaa 350
tgtatctgtc ctgctgttgg aggccaacac caatcagact gtaactacca 400
agtacctcct gaccaaccag tcccagggaa cactaaagtt tgagtgcttc 450
tatttcaagg aggctggtga ctactggttc acaatgactc cagaagcaac 500
agacaacagc actccattcc cctggtggga gaaaagtgcc tttctgaagg 550
tggaatggcc tgtctttcac gttgacttga ataggagtgc caaggcagca 600
gaaggcacct tccaagtggg cctatttacc agtcaaccac tgtgcccgtt 650
tcctgtggac aagcccaaca ttgtagtgga tgtcatcttc accaacagtc 700
ttcctgaggc aagaagaaat tcaagacagc cgctggaaat aagaaccagc 750
aaaaggacag aacttgctca aggtcagtgg gttgagtttg gctgtgcacc 800
cttggggcca gaagcctatg tcaccgtggc gctgaagctg cttgggcgag 850
actcagtcat tacctccaca ggacccattg acctggccca gaaatttgga 900
tacaaactgg tgatggtgcc agaactcaca tgtgagtccg gggtagaggt 950
gacagtgctg cctccaccat gcaccttcgt ccaaggagtg gtcactgtct 1000
tcaaggaggc ccccagatac cctgggaaga ggaccattca cttggctgaa 1050
aacagcctgc ccctgggaga gaggaggaca atttttaact gtactttgtt 1100
tgacatgggg aagaataagt actgctttga ctttggcatt tcaagcagaa 1150
gccatttttc tgcaaaggag gagtgcatgc taattcagag aaatacagaa 1200
acttggggac tgtggcagcc atggagccag tgtagtgcca catgtgggga 1250
tggtgtcaga gagcgtcgcc gagtgtgtct cacttccttc ccctccagtc 1300
ctgtctgccc tggaatgtcc ttggaggcct ccctgtgttc cctggaggag 1350
tgtgctgctt tccagccatc cagcccatct cctcttcagc cccagggtcc 1400
agtgaagtcc aacaacatcg tgactgtcac tggtatatcc ttgtgcttgt 1450
tcatcatcat tgccactgtg ctcatcacgc tgtggaggag gttcggccgg 1500
ccagccaagt gcagcacacc tgctcgacac aactccatcc actcccccag 1550

cttccggaag aactcggacg aggagaatat ctgcgagctg agcgagcagc 1600
gcgggagctt ctcggatggg ggagacgggc ccacggggag tccaggggac 1650
acaggcatcc ctctgaccta caggcggagc gggccggtac ctcccgagga 1700
tgatgcctct ggcagcgaga gcttccagtc caacgcccag aagataatcc 1750
cacctctgtt cagctaccgc cttgcccagc agcagttaaa ggagatgaaa 1800
aagaaaggtc tgacggaaac taccaaagtg tatcacgtgt ctcagagtcc 1850
cctgacagac actgccattg atgcggcccc cagcgctccc ttagatttgg 1900
aaagcccgga agaagctgca gcaaacaagt tccggatcaa atccccattt 1950
ccggagcagc ccgcggtcag tgccggggaa aggcctccct ccaggctgga 2000
tctaaatgtg actcaggcca gttgtgccat aagccccagc cagactctga 2050
tccgcaagtc acaggcaagg cacgtgggca gcagaggggg cccgtccgaa 2100
aggagccatg ccaggaacgc ccatttcagg aggacagcga gtttccatga 2150
agccaggcag gcccggccgt tccgagagag gagcatgtcc actctgactc 2200
cacggcaggc ccctgcctac agctctagga cgcggacctg cgagcaggca 2250
gaggacagat ttaggcctca gagtcgaggt gcccacctgt ttcctgaaaa 2300
actggagcat ttccaagagg caagtggaac ccgtggtcca ttaaaccctc 2350
tccctaaatc ctacactttg gggcagccct tgaggaaacc agaccttggg 2400
gatcaccagg caggattagt ggccggaatt gagagaacag agccccacag 2450
agctcgtcgg ggaccgtccc ccagtcacaa gagtgtctca aggaagcagt 2500
cttctcccat atcccccaaa gataactacc agagggtcag ttctctgagc 2550
ccttctcagt gtagaaaaga caagtgtcaa agcttcccca ctcaccctga 2600
gtttgccttc tatgacaata cgtcgtttgg cctcactgag gctgagcaga 2650
ggatgctgga cctcccagga tattttgggt caaatgaaga ggatgaaacc 2700
acaagtacac ttagcgtgga gaagctggtg atctagactg agaatcagcc 2750
tgagctttac acagctgggg tctgctactc gcgttttgta gacttttgtg 2800
taactatttg taccgtagga cagaatgtga ggaggaagta acacacagag 2850
gaggatgtgt gtgtatgcat gtgtttgaat tcacaaggaa gaaattattt 2900
atcttgagct ttttcctttg ttattcaatt tctattgatt tattagtaat 2950
aacaatgata ataaaatgta aatgagc 2977

<210> 92
<211> 852
<212> PRT
<213> Homo sapiens

<400> 92
Met Lys Pro Met Leu Lys Asp Phe Ser Asn Leu Leu Leu Val Val
1               5                   10                  15

Leu Cys Asp Tyr Val Leu Gly Glu Ala Glu Tyr Leu Leu Leu Arg
                20                  25                  30

Glu Pro Gly His Val Ala Leu Ser Asn Asp Thr Val Tyr Val Asp
                35                  40                  45

Phe Gln Tyr Phe Asp Gly Ala Asn Gly Thr Leu Arg Asn Val Ser
                50                  55                  60

Val Leu Leu Leu Glu Ala Asn Thr Asn Gln Thr Val Thr Thr Lys
                65                  70                  75

Tyr Leu Leu Thr Asn Gln Ser Gln Gly Thr Leu Lys Phe Glu Cys
                80                  85                  90

Phe Tyr Phe Lys Glu Ala Gly Asp Tyr Trp Phe Thr Met Thr Pro
                95                  100                 105

Glu Ala Thr Asp Asn Ser Thr Pro Phe Pro Trp Trp Glu Lys Ser
                110                 115                 120

Ala Phe Leu Lys Val Glu Trp Pro Val Phe His Val Asp Leu Asn
                125                 130                 135

Arg Ser Ala Lys Ala Ala Glu Gly Thr Phe Gln Val Gly Leu Phe
                140                 145                 150

Thr Ser Gln Pro Leu Cys Pro Phe Pro Val Asp Lys Pro Asn Ile
                155                 160                 165

Val Val Asp Val Ile Phe Thr Asn Ser Leu Pro Glu Ala Arg Arg
                170                 175                 180

Asn Ser Arg Gln Pro Leu Glu Ile Arg Thr Ser Lys Arg Thr Glu
                185                 190                 195

Leu Ala Gln Gly Gln Trp Val Glu Phe Gly Cys Ala Pro Leu Gly
                200                 205                 210

Pro Glu Ala Tyr Val Thr Val Ala Leu Lys Leu Leu Gly Arg Asp
                215                 220                 225

Ser Val Ile Thr Ser Thr Gly Pro Ile Asp Leu Ala Gln Lys Phe
                230                 235                 240

Gly Tyr Lys Leu Val Met Val Pro Glu Leu Thr Cys Glu Ser Gly
                245                 250                 255

Val Glu Val Thr Val Leu Pro Pro Pro Cys Thr Phe Val Gln Gly
                260                 265                 270

Val Val Thr Val Phe Lys Glu Ala Pro Arg Tyr Pro Gly Lys Arg
                275                 280                 285

Thr Ile His Leu Ala Glu Asn Ser Leu Pro Leu Gly Glu Arg Arg
                290                 295                 300

Thr Ile Phe Asn Cys Thr Leu Phe Asp Met Gly Lys Asn Lys Tyr
                305                 310                 315

Cys Phe Asp Phe Gly Ile Ser Ser Arg Ser His Phe Ser Ala Lys
                320                 325                 330

Glu Glu Cys Met Leu Ile Gln Arg Asn Thr Glu Thr Trp Gly Leu
                335                 340                 345

Trp Gln Pro Trp Ser Gln Cys Ser Ala Thr Cys Gly Asp Gly Val
                350                 355                 360

Arg Glu Arg Arg Arg Val Cys Leu Thr Ser Phe Pro Ser Ser Pro
                365                 370                 375

Val Cys Pro Gly Met Ser Leu Glu Ala Ser Leu Cys Ser Leu Glu
                380                 385                 390

Glu Cys Ala Ala Phe Gln Pro Ser Ser Pro Ser Pro Leu Gln Pro
                395                 400                 405

Gln Gly Pro Val Lys Ser Asn Asn Ile Val Thr Val Thr Gly Ile
                410                 415                 420

Ser Leu Cys Leu Phe Ile Ile Ile Ala Thr Val Leu Ile Thr Leu
                425                 430                 435

Trp Arg Arg Phe Gly Arg Pro Ala Lys Cys Ser Thr Pro Ala Arg
                440                 445                 450

His Asn Ser Ile His Ser Pro Ser Phe Arg Lys Asn Ser Asp Glu
                455                 460                 465

Glu Asn Ile Cys Glu Leu Ser Glu Gln Arg Gly Ser Phe Ser Asp
                470                 475                 480

Gly Gly Asp Gly Pro Thr Gly Ser Pro Gly Asp Thr Gly Ile Pro
                485                 490                 495

Leu Thr Tyr Arg Arg Ser Gly Pro Val Pro Pro Glu Asp Asp Ala
                500                 505                 510

Ser Gly Ser Glu Ser Phe Gln Ser Asn Ala Gln Lys Ile Ile Pro
                515                 520                 525

Pro Leu Phe Ser Tyr Arg Leu Ala Gln Gln Gln Leu Lys Glu Met
                530                 535                 540

Lys Lys Lys Gly Leu Thr Glu Thr Thr Lys Val Tyr His Val Ser

                545                 550                 555

Gln Ser Pro Leu Thr Asp Thr Ala Ile Asp Ala Ala Pro Ser Ala
                560                 565                 570

Pro Leu Asp Leu Glu Ser Pro Glu Glu Ala Ala Ala Asn Lys Phe
                575                 580                 585

Arg Ile Lys Ser Pro Phe Pro Glu Gln Pro Ala Val Ser Ala Gly
                590                 595                 600

Glu Arg Pro Pro Ser Arg Leu Asp Leu Asn Val Thr Gln Ala Ser
                605                 610                 615

Cys Ala Ile Ser Pro Ser Gln Thr Leu Ile Arg Lys Ser Gln Ala
                620                 625                 630

Arg His Val Gly Ser Arg Gly Gly Pro Ser Glu Arg Ser His Ala
                635                 640                 645

Arg Asn Ala His Phe Arg Arg Thr Ala Ser Phe His Glu Ala Arg
                650                 655                 660

Gln Ala Arg Pro Phe Arg Glu Arg Ser Met Ser Thr Leu Thr Pro
                665                 670                 675

Arg Gln Ala Pro Ala Tyr Ser Ser Arg Thr Arg Thr Cys Glu Gln
                680                 685                 690

Ala Glu Asp Arg Phe Arg Pro Gln Ser Arg Gly Ala His Leu Phe
                695                 700                 705

Pro Glu Lys Leu Glu His Phe Gln Glu Ala Ser Gly Thr Arg Gly
                710                 715                 720

Pro Leu Asn Pro Leu Pro Lys Ser Tyr Thr Leu Gly Gln Pro Leu
                725                 730                 735

Arg Lys Pro Asp Leu Gly Asp His Gln Ala Gly Leu Val Ala Gly
                740                 745                 750

Ile Glu Arg Thr Glu Pro His Arg Ala Arg Arg Gly Pro Ser Pro
                755                 760                 765

Ser His Lys Ser Val Ser Arg Lys Gln Ser Ser Pro Ile Ser Pro
                770                 775                 780

Lys Asp Asn Tyr Gln Arg Val Ser Ser Leu Ser Pro Ser Gln Cys
                785                 790                 795

Arg Lys Asp Lys Cys Gln Ser Phe Pro Thr His Pro Glu Phe Ala
                800                 805                 810

Phe Tyr Asp Asn Thr Ser Phe Gly Leu Thr Glu Ala Glu Gln Arg
                815                 820                 825

Met Leu Asp Leu Pro Gly Tyr Phe Gly Ser Asn Glu Glu Asp Glu
                830                 835                 840

Thr Thr Ser Thr Leu Ser Val Glu Lys Leu Val Ile
                845                 850

<210> 93
<211> 43
<212> DNA
<213> Artificial sequence

<220>
<223> oligonucleotide probe

<400> 93
tgtaaaacga cggccagtta aatagacctg caattattaa tct 43

<210> 94
<211> 41
<212> DNA
<213> Artificial sequence

<220>
<223> oligoneucleotide probe

<400> 94
caggaaacag ctatgaccac ctgcacacct gcaaatccat t 41

<210> 95
<211> 29
<212> DNA
<213> Artificial sequence

<220>
<223> oligoneucleotide probe

<400> 95
caggttatcc cagagattta atgccacca 29

<210> 96
<211> 34
<212> DNA
<213> Artificial sequence

<220>
<223> oligoneucleotide probe

<400> 96
ttggtgggag aagttgccag atcaggtggt ggca 34

<210> 97
<211> 25
<212> DNA
<213> Artificial sequence

<220>
<223> oligoneucleotide probe

<400> 97
ttcacaccat aactgcattg gtcca 25

<210> 98
<211> 20
<212> DNA
<213> Artificial sequence

<220>
<223> oligoneucleotide probe

<400> 98
 tggaataccg cctcctgcag 20

<210> 99
<211> 24
<212> DNA
<213> Artificial sequence

<220>
<223> oligoneucleotide probe

<400> 99
 cttctgccct ttggagaaga  tggc 24

<210> 100
<211> 43
<212> DNA
<213> Artificial sequence

<220>
<223> oligoneucleotide probe

<400> 100
 ggactcactg gcccaggcct tcaatatcac cagccaggac  gat 43

<210> 101
<211> 22
<212> DNA
<213> Artificial sequence

<220>
<223> oligoneucleotide probe

<400> 101
 gttggatctg ggcaacaata  ac 22

<210> 102
<211> 24
<212> DNA
<213> Artificial sequence

<220>
<223> oligoneucleotide probe

<400> 102
 attgttgtgc aggctgagtt  taag 24

<210> 103
<211> 45

<212> DNA
<213> Artificial sequence

<220>
<223> oligoneucleotide probe

<400> 103
ggtggctata catggatagc aattacctgg acacgctgtc ccggg 45

<210> 104
<211> 30
<212> DNA
<213> Artificial sequence

<220>
<223> oligoneucleotide probe

<400> 104
agggaggatt atccttgacc tttgaagacc 30

<210> 105
<211> 18
<212> DNA
<213> Artificial sequence

<220>
<223> oligoneucleotide probe

<400> 105
gaagcaagtg cccagctc 18

<210> 106
<211> 18
<212> DNA
<213> Artificial sequence

<220>
<223> oligoneucleotide probe

<400> 106
cgggtccctg ctctttgg 18

<210> 107
<211> 24
<212> DNA
<213> Artificial sequence

<220>
<223> oligoneucleotide probe

<400> 107
caccgtagct gggagcgcac tcac 24

<210> 108
<211> 18
<212> DNA
<213> Artificial sequence

<220>
<223> oligoneucleotide probe

<400> 108
agtgtaagtc aagctccc 18

<210> 109
<211> 49
<212> DNA
<213> Artificial sequence

<220>
<223> oligoneucleotide probe

<400> 109
gcttcctgac actaaggctg tctgctagtc agaattgcct caaaaagag 49

<210> 110
<211> 24
<212> DNA
<213> Artificial sequence

<220>
<223> oligoneucleotide probe

<400> 110
agctgtggtc atggtggtgt ggtg 24

<210> 111
<211> 24
<212> DNA
<213> Artificial sequence

<220>
<223> oligoneucleotide probe

<400> 111
ctaccttggc cataggtgat ccgc 24

<210> 112
<211> 42
<212> DNA
<213> Artificial sequence

<220>
<223> oligoneucleotide porbe

<400> 112
catcagcaaa ccgtctgtgg ttcagctcaa ctggagaggg tt 42

<210> 113
<211> 24
<212> DNA
<213> Artificial sequence

<220>
<223> oligoneucleotide probe

<400> 113
agcttctcag ccctcctgga gcag 24

<210> 114
<211> 25
<212> DNA
<213> Artificial sequence

<220>
<223> oligoneucleotide probe

<400> 114
cgggtcaata aacctggacg cttgg 25

<210> 115
<211> 43
<212> DNA
<213> Artificial sequence

<220>
<223> oligoneucleotide probe

<400> 115
tatgtggacc ggaccaagca cttcactgag gccaccaaga ttg 43

<210> 116
<211> 24
<212> DNA
<213> Artificial sequence

<220>
<223> oligoneucleotide probe

<400> 116
ccctgcagtg cacctacagg gaag 24

<210> 117
<211> 24
<212> DNA
<213> Artificial sequence

<220>
<223> oligoneucleotide probe

<400> 117
ctgtcttccc ctgcttggct gtgg 24

<210> 118
<211> 47
<212> DNA
<213> Artificial sequence

<220>
<223> oligoneucleotide probe

<400> 118
ggtgcaggaa gggtgggatc ctcttctctc gctgctctgg ccacatc 47

<210> 119
<211> 22
<212> DNA
<213> Artificial sequence

<220>
<223> oligoneucleotide probe

<400> 119
tcgtgcccag gggctgatgt gc 22

<210> 120
<211> 24
<212> DNA
<213> Artificial sequence

<220>
<223> oligoneucleotide probe

<400> 120
gtctttaccc agccccggga tgcg 24

<210> 121
<211> 50
<212> DNA
<213> Artificial sequence

<220>
<223> oligoneucleotide probe

<400> 121
ggcctaatcc aacgttctgt cttcaatctg caaatctatg gggtcctggg 50

<210> 122
<211> 27
<212> DNA
<213> Artificial sequence

<220>
<223> oligoneucleotide probe

<400> 122
gtccacagac agtcatctca ggagcag 27

<210> 123
<211> 20
<212> DNA
<213> Artificial sequence

<220>
<223> oligoneucleotide probe

<400> 123
acaagtgtct tcccaacctg 20

<210> 124
<211> 24
<212> DNA

<213> Artificial sequence

<220>
<223> oligoneucleotide probe

<400> 124
atcctcccag agccatggta cctc 24

<210> 125
<211> 51
<212> DNA
<213> Artificial sequence

<220>
<223> oligoneucleotide probe

<400> 125
ccaaggatag ctgttgtttc agagaaagga tcgtgtgctg catctcctcc 50

t 51

<210> 126
<211> 24
<212> DNA
<213> Artificial sequence

<220>
<223> oligoneucleotide probe

<400> 126
ccgaggccat ctagaggcca gagc 24

<210> 127
<211> 24
<212> DNA
<213> Artificial sequence

<220>
<223> oligoneucleotide probe

<400> 127
acaggcagag ccaatggcca gagc 24

<210> 128
<211> 45
<212> DNA
<213> Artificial sequence

<220>
<223> oligoneucleotide probe

<400> 128
gagaggactg cgggagtttg ggacctttgt gcagacgtgc tcatg 45

<210> 129
<211> 24
<212> DNA
<213> Artificial sequence

<220>
<223> oligoneucleotide probe

<400> 129
 gcaggctttg aggatgaagg  ctgc 24

<210> 130
<211> 24
<212> DNA
<213> Artificial sequence

<220>
<223> oligoneucleotide probe

<400> 130
 ctcattggct gcctggtcac  aggc 24

<210> 131
<211> 24
<212> DNA
<213> Artificial sequence

<220>
<223> oligoneucleotide probe

<400> 131
 ccagtcggac aggtctctcc  cctc 24

<210> 132
<211> 24
<212> DNA
<213> Artificial sequence

<220>
<223> oligoneucleotide probe

<400> 132
 tcagtgacca aggctgagca  ggcg 24

<210> 133
<211> 47
<212> DNA
<213> Artificial sequence

<220>
<223> oligoneucleotide probe

<400> 133
 ctacactcgt tgcaaactgg caaaaatatt ctcgagggct  ggcctgg 47

<210> 134
<211> 20
<212> DNA
<213> Artificial sequence

<220>
<223> oligoneucleotide probe

<400> 134
tcctgtgagc acgtggtgtg 20

<210> 135
<211> 18
<212> DNA
<213> Artificial sequence

<220>
<223> oligoneucleotide probe

<400> 135
gggtgggata  gacctgcg 18

<210> 136
<211> 18
<212> DNA
<213> Artificial sequence

<220>
<223> oligoneucleotide probe

<400> 136
aaggccaaga  aggctgcc 18

<210> 137
<211> 18
<212> DNA
<213> Artificial sequence

<220>
<223> oligoneucleotide probe

<400> 137
ccaggcctgc  agacccag 18

<210> 138
<211> 24
<212> DNA
<213> Artificial sequence

<220>
<223> oligoneucleotide probe

<400> 138
cttcctcagt ccttccagga  tatc 24

<210> 139
<211> 24
<212> DNA
<213> Artificial sequence

<220>
<223> oligoneucleotide probe

<400> 139
aagctggata tcctccgtgt  tgtc 24

<210> 140
<211> 27
<212> DNA
<213> Artificial sequence

<220>
<223> oligoneucleotide probe

<400> 140
cctgaagagg catgactgct tttctca 27

<210> 141
<211> 27
<212> DNA
<213> Artificial sequence

<220>
<223> oligoneucleotide probe

<400> 141
ggggataaac ctattaatta ttgctac 27

<210> 142
<211> 44
<212> DNA
<213> Artificial sequence

<220>
<223> oligoneucleotide probe

<400> 142
aacgtcacct acatctcctc gtgccacatg cgccaggcca cctg 44

<210> 143
<211> 25
<212> DNA
<213> Artificial sequence

<220>
<223> oligoneucleotide probe

<400> 143
tgtcctttgt cccagacttc tgtcc 25

<210> 144
<211> 50
<212> DNA
<213> Artificial sequence

<220>
<223> oligoneucleotide probe

<400> 144
ctggatgcta atgtgtccag taaatgatcc ccttatcccg tcgcgatgct 50

<210> 145
<211> 23

<212> DNA
<213> Artificial sequence

<220>
<223> oligoneucleotide probe

<400> 145
ggcgagccct aactatccag gag 23

<210> 146
<211> 39
<212> DNA
<213> Artificial sequence

<220>
<223> oligoneucleotide probe

<400> 146
ggagatcgct gcgctggcca ggtcctccct gcatggtat 39

<210> 147
<211> 22
<212> DNA
<213> Artificial sequence

<220>
<223> oligoneucleotide probe

<400> 147
ctgctgcaaa gcgagcctct tg 22

<210> 148
<211> 30
<212> DNA
<213> Artificial sequence

<220>
<223> oligoneucleotide probe

<400> 148
tgtgatttta cagagtactg caatggaacc 30

<210> 149
<211> 27
<212> DNA
<213> Artificial sequence

<220>
<223> oligoneucleotide probe

<400> 149
ttctttaaaa caggcaaatg gagcacc 27

<210> 150
<211> 40
<212> DNA
<213> Artificial sequence

<220>
<223> oligoneucleotide probe

<400> 150
 tgacacttat gcattgaatg gccgtttgtg caagttggga 40

<210> 151
<211> 24
<212> DNA
<213> Artificial sequence

<220>
<223> oligoneucleotide probe

<400> 151
 caccaaccaa ctgccaatcc  tggc 24

<210> 152
<211> 26
<212> DNA
<213> Artificial sequence

<220>
<223> oligoneucleotide probe

<400> 152
 accacattct gatgggtgtc  tcctgg 26

<210> 153
<211> 49
<212> DNA
<213> Artificial sequence

<220>
<223> oligoneucleotide probe

<400> 153
 gggtccctac ctttaccagt ggaatgatga caggtgtaac  atgaagcac 49

<210> 154
<211> 22
<212> DNA
<213> Artificial sequence

<220>
<223> oligoneucleotide probe

<400> 154
 ccacttgcca tgaacatgcc  ac 22

<210> 155
<211> 25
<212> DNA
<213> Artificial sequence

<220>
<223> oligoneucleotide probe

<400> 155
cctcttgaca gacatagcga gccac 25

<210> 156
<211> 43
<212> DNA
<213> Artificial sequence

<220>
<223> oligoneucleotide probe

<400> 156
cactcttgtc tgtgggaacc acacatcttg ccacaactgt ggc 43

<210> 157
<211> 31
<212> DNA
<213> Artificial sequence

<220>
<223> oligoneucleotide probe

<400> 157
gctctgtctc tacctggagc tacaatgtgt g 31

<210> 158
<211> 27
<212> DNA
<213> Artificial sequence

<220>
<223> oligoneucleotide probe

<400> 158
ttggcataga tgccaacatc agctctc 27

<210> 159
<211> 47
<212> DNA
<213> Artificial sequence

<220>
<223> oligoneucleotide probe

<400> 159
gagcccgatt cactgcaaac tgtgaacatc tctgtaatct ccaagcc 47

<210> 160
<211> 23
<212> DNA
<213> Artificial sequence

<220>
<223> oligoneucleotide probe

<400> 160
cctgtcgttc gcagagtccg ctc 23

<210> 161
<211> 24
<212> DNA
<213> Artificial sequence

<220>
<223> oligoneucleotide probe

<400> 161
cacacagagg ttccggcacc tctc 24

<210> 162
<211> 38
<212> DNA
<213> Artificial sequence

<220>
<223> oligoneucleotide probe

<400> 162
tcctgcaggc ggcagatctg ggagtaggtg tgaccgtc 38

<210> 163
<211> 24
<212> DNA
<213> Artificial sequence

<220>
<223> oligoneucleotide probe

<400> 163
acaactgcat agaagcccac aacg 24

<210> 164
<211> 31
<212> DNA
<213> Artificial sequence

<220>
<223> oligoneucleotide probe

<400> 164
acaactgcat agaagcccac aacgaatggc g 31

<210> 165
<211> 24
<212> DNA
<213> Artificial sequence

<220>
<223> oligoneucleotide probe

<400> 165
tctccccgtt ggcttcagaa atgg 24

<210> 166
<211> 31
<212> DNA

<213> Artificial sequence

<220>
<223> oligoneucleotide probe

<400> 166
cgccattcgt tgtgggcttc tatgcagttg t 31

<210> 167
<211> 50
<212> DNA
<213> Artificial sequence

<220>
<223> oligoneucleotide probe

<400> 167
ggttaggtgg aataaagtca ttcacaccaa gacatgccat tacggcttgg 50

<210> 168
<211> 24
<212> DNA
<213> Artificial sequence

<220>
<223> oligoneucleotide probe

<400> 168
gagctggatc tgcagaggaa ctac 24

<210> 169
<211> 24
<212> DNA
<213> Artificial sequence

<220>
<223> oligoneucleotide probe

<400> 169
aggaaccact ccaggacgtt gtag 24

<210> 170
<211> 46
<212> DNA
<213> Artificial sequence

<220>
<223> oligoneucleotide probe

<400> 170
ctttcgacgg cctggctgag ctgaggcacc tcaacctggc cttcaa 46

<210> 171
<211> 27
<212> DNA
<213> Artificial sequence

<220>

<223> oligoneucleotide probe

<400> 171
 cctataaggg ctacaaaaac  cgcgtgg 27

<210> 172
<211> 27
<212> DNA
<213> Artificial sequence

<220>
<223> oligoneucleotide probe

<400> 172
 agcactgcac agacagagtc  tcccctt 27

<210> 173
<211> 39
<212> DNA
<213> Artificial sequence

<220>
<223> oligoneucleotide probe

<400> 173
 caacatcacc atggtggccc tcaagctcca  ggactcagg 39

<210> 174
<211> 25
<212> DNA
<213> Artificial sequence

<220>
<223> oligoneucleotide probe

<400> 174
 ttccactcaa tgaggtgagc  cactc 25

## Description

### FIELD OF THE INVENTION

The present invention relates to compositions, including transgenic and knockout animals and methods of using such compositions for the diagnosis and treatment of diseases or disorders.

### BACKGROUND OF THE INVENTION

Extracellular proteins play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including as pharmaceuticals, diagnostics, biosensors and bioreactors. Most protein drugs available at present, such as thrombolytic agents, interferons, interleukins, erythropoietins, colony stimulating factors, and various other cytokines, are secretory proteins. Their receptors, which are membrane proteins, also have potential as therapeutic or diagnostic agents. Efforts are being undertaken by both industry and academia to identify new, native secreted proteins. Many efforts are focused on the screening of mammalian recombinant DNA libraries to identify the coding sequences for novel secreted proteins. Examples of screening methods and techniques are described in the literature [see, for example, Klein et al., Proc. Natl. Acad. Sci. 93:7108-7113 (1996); U.S. Patent No. 5,536,637)].

Membrane-bound proteins and receptors can play important roles in, among other things, the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. Such membrane-bound proteins and cell receptors include, but are not limited to, cytokine receptors, receptor kinases, receptor phosphatases, receptors involved in cell-cell interactions, and cellular adhesion molecules like selectins and integrins. For instance, transduction of signals that regulate cell growth and differentiation is regulated in part by phosphorylation of various cellular proteins. Protein tyrosine kinases, enzymes that catalyze that process, can also act as growth factor receptors. Examples include fibroblast growth factor receptor and nerve growth factor receptor.

Membrane-bound proteins and receptor molecules have various industrial applications, including as pharmaceutical and diagnostic agents. Receptor immuno-adhesions, for instance, can be employed as therapeutic agents to block receptor-ligand interactions. The membrane-bound proteins can also be employed for screening of potential peptide or small molecule inhibitors of the relevant receptor/ligand interaction.

Efforts are being undertaken by both industry and academia to identify new, native receptor or membrane-bound proteins. Many efforts are focused on the screening of mammalian recombinant DNA libraries to identify the coding sequences for novel receptor or membrane-bound proteins.

Given the importance of secreted and membrane-bound proteins in biological and disease processes, *in vivo* studies and characterizations may provide valuable identification and discovery of therapeutics and/or treatments useful in the prevention, amelioration or correction of diseases or dysfunctions. In this regard, genetically engineered mice have proven to be invaluable tools for the functional dissection ofbiological processes relevant to human disease, including immunology, cancer, neuro-biology, cardiovascular biology, obesity and many others. Gene knockouts can be viewed as modeling the biological mechanism of drug action by presaging the activity of highly specific antagonists *in vivo*. Knockout mice have been shown to model drug activity; phenotypes ofmice deficient for specific pharmaceutical target proteins canresemble the human clinical phenotype caused by the corresponding antagonist drug. Gene knockouts enable the discovery of the mechanism of action of the target, the predominant physiological role of the target, and mechanism-based side-effects that might result from inhibition of the target in mammals. Examples of this type include mice deficient in the angiotensin converting enzyme (ACE) [Esther, C.R. et al., Lab. Invest., 74:953-965 (1996)] and cyclooxygenase-1 (COX1) genes [Langenbach, R. et al., Cell, 83:483-492 (1995)]. Conversely, knocking the gene out in the mouse can have an opposite phenotypic effect to that observed in humans after administration of an agonist drug to the corresponding target. Examples include the erythropoietin knockout [Wu, C.S. et al., Cell, 83:59-67 (1996)], in which a consequence of the mutation is deficient red blood cell production, and the GABA(A)-R-β3 knockout [DeLorey, T.M., J. Neurosci., 18:8505-8514 (1998)], in which the mutant mice show hyperactivity and hyper-responsiveness. Both these phenotypes are opposite to the effects of erythropoietin and benzodiazepine administration in humans. A striking example of a target validated using mouse genetics is the ACC2 gene. Although the human ACC2 gene had been identified several years ago, interest in ACC2 as a target for drug development was stimulated only recently after analysis of ACC2 function using a knockout mouse. ACC2 mutant mice eat more than their wild-type littermates, yet burn more fat and store less fat in their adipocytes, making this enzyme a probable target for chemical antagonism in the treatment of obesity [Abu-Elheiga, L. et al., Science, 291:2613-2616 (2001)].

In the instant application, mutated gene disruptions have resulted in phenotypic observations related to various disease conditions or dysfunctions including: CNS/neurological disturbances or disorders such as anxiety; eye abnormalities and associated diseases; cardiovascular, endothelial or angiogenic disorders including atherosclerosis; abnormal metabolic disorders including diabetes and dyslipidemias associated with elevated serum triglycerides and cholesterol levels; immunological and inflammatory disorders; oncological disorders; bone metabolic abnormalities or disorders such as arthritis, osteoporosis and osteopetrosis; or a developmental disease such as embryonic lethality.

### SUMMARY OF THE INVENTION

### A. Embodiments

The invention provides an isolated nucleic acid molecule comprising a nucleotide sequence that encodes a PRO188, PR0235, PR0266, PR0337, PR0361, PR0539, PR0698, PR0717, PR0846, PR0874, PR098346, PRO1082, PRO1097, PRO1192, PR01268, PR01278, PRO1303, PRO1308, PR01338, PRO1378, PRO1415, PRO1867, PRO1890, PR03438, PR019835, PR036915, PR036029, PR04999, PR05778, PR05997, PR06079, PR06090, PR07178, PR021184, PR07434, PR09822, PRO9833, PR09836, PR09854, PR09862, PRO10284, PR037510, PR035444, PR020473, PR021054 or PR035246 polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO188, PR0235, PR0266, PR0337, PR0361, PR0539, PRO698, PR0717, PR0846, PR0874, PR098346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PR036029, PR04999, PR05778, PRO5997, PR06079, PR06090, PR07178, PR021184, PR07434, PR09822, PR09833, PR09836, PR09854, PR09862, PRO10284, PR037510, PRO35444, PR020473, PR021054 or PR035246 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide cDNA as disclosed herein, the coding sequence of aPRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide lacking the signal peptide as disclosed herein, the coding sequence of an extracellular domain of a transmembrane PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, with or without the signal peptide, as disclosed herein or the coding sequence of any other specifically defmed fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect of the invention provides an isolated nucleic acid molecule comprising a nucleotide sequence encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides are contemplated.

The invention also provides fragments of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide coding sequence, or the complement thereof, that may find use as, for example, hybridization probes, for encoding fragments of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide that may optionally encode a polypeptide comprising a binding site for an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody or as antisense oligonucleotide probes. Such nucleic acid fragments usually are or are at least about 10 nucleotides in length, alternatively are or are at least about 15 nucleotides in length, alternatively are or are at least about 20 nucleotides in length, alternatively are or are at least about 30 nucleotides in length, alternatively are or are at least about 40 nucleotides in length, alternatively are or are at least about 50 nucleotides in length, alternatively are or are at least about 60 nucleotides in length, alternatively are or are at least about 70 nucleotides in length, alternatively are or are at least about 80 nucleotides in length, alternatively are or are at least about 90 nucleotides in length, alternatively are or are at least about 100 nucleotides in length, alternatively are or are at least about 110 nucleotides in length, alternatively are or are at least about 120 nucleotides in length, alternatively are or are at least about 130 nucleotides in length, alternatively are or are at least about 140 nucleotides in length, alternatively are or are at least about 150 nucleotides in length, alternatively are or are at least about 160 nucleotides in length, alternatively are or are at least about 170 nucleotides in length, alternatively are or are at least about 180 nucleotides in length, alternatively are or are at least about 190 nucleotides in length, alternatively are or are at least about 200 nucleotides in length, alternatively are or are at least about 250 nucleotides in length, alternatively are or are at least about 300 nucleotides in length, alternatively are or are at least about 350 nucleotides in length, alternatively are or are at least about 400 nucleotides in length, alternatively are or are at least about 450 nucleotides in length, alternatively are or are at least about 500 nucleotides in length, alternatively are or are at least about 600 nucleotides in length, alternatively are or are at least about 700 nucleotides in length, alternatively are or are at least about 800 nucleotides in length, alternatively are or are at least about 900 nucleotides in length and alternatively are or are at least about 1000 nucleotides in length, wherein in this context the term "about" means the referenced nucleotide sequence length plus or minus 10% of that referenced length. It is noted that novel fragments of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding nucleotide sequence may be determined in a routine manner by aligning the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding nucleotide sequence with other known nucleotide sequences using any of a number of well known sequence alignment programs and determining which PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding nucleotide sequence fragment(s) are novel. All of such PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding nucleotide sequences are contemplated herein. Also contemplated are the PRO188, PRO235, PRO266, PRO337, PRO361, PRO539, PRO698, PRO717, PRO846, PRO874, PRO98346, PRO1082, PRO1097, PRO1192, PRO, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fragments encoded by these nucleotide molecule fragments, preferably those PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fragments that comprise a binding site for an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention provides isolated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides encoded by any of the isolated nucleic acid sequences hereinabove identified.

In a certain aspect, the invention concerns an isolated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In one aspect, the invention concerns PRO188, PRO235, PRO266, PRO337, PRO3 61, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polypeptides which are or are at least about 10 amino acids in length, alternatively are or are at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polypeptides will have or have no more than one conservative amino acid substitution as compared to the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence, alternatively will have or will have no more than 2,3,4, 5,6,7, 8, 9, or 10 conservative amino acid substitution as compared to the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence.

In a specific aspect, the invention provides an isolated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide and recovering the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide from the cell culture.

Another aspect the invention provides an isolated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and recovering the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO, PRO9833, PRO9836, PRO9854, PRO9862, PRO10284, PRO37510, PRO35444, PRO20473, PRO21054 or PRO35246 polypeptide from the cell culture.

The invention provides agonists and antagonists of a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as defined herein. In particular, the agonist or antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody or a small molecule.

The invention provides a method of identifying agonists or antagonists to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which comprise contacting the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with a candidate molecule and monitoring a biological activity mediated by said PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Preferably, the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

The invention provides a composition of matter comprising a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, or an agonistor antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as herein described, or an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, in combination with a carrier. Optionally, the carrier is a pharmaceutically acceptable carrier.

The invention provides the use of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, or an agonist or antagonist thereof as hereinbefore described, or an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, for the preparation of a medicament useful in the treatment of a condition which is responsive to the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention provides vectors comprising DNA encoding any of the herein described polypeptides. Host cell comprising any such vector are also provided. By way of example, the host cells may be CHO cells, *E*. *coli*, or yeast. A process for producing any of the herein described polypeptides is further provided and comprises culturing host cells under conditions suitable for expression of the desired polypeptide and recovering the desired polypeptide from the cell culture.

The invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

The invention provides an antibody which binds, preferably specifically, to any of the above or below described polypeptides. Optionally, the antibody is a monoclonal antibody, humanized antibody, antibody fragment or single-chain antibody.

The invention provides oligonucleotide probes which may be useful for isolating genomic and cDNA nucleotide sequences, measuring or detecting expression of an associated gene or as antisense probes, wherein those probes may be derived from any of the above or below described nucleotide sequences. Preferred probe lengths are described above.

The invention also provides a method of identifying a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) measuring a physiological characteristic of the non-human transgenic animal; and
(c) comparing the measured physiological characteristic with that of a gender matched wild-type animal,
wherein the physiological characteristic of the non-human transgenic animal that differs from the physiological characteristic of the wild-type animal is identified as a phenotype resulting from the gene disruption in the non-human transgenic animal. In one aspect, the non-human transgenic animal is a mammal. In another aspect, the mammal is a rodent. In still another aspect, the mammal is a rat or a mouse. In one aspect, the non-human transgenic animal is heterozygous for the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In another aspect, the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In still yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e.g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In still another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased prepulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages of B cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages of B cells; decreased CD4+ and CD8+ cells; decrease in natural killer cells; increase in monocytes; increased mean serum IgG1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.

The invention also provides an isolated cell derived from a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In one aspect, the isolated cell is a murine cell. In yet another aspect, the murine cell is an embryonic stem cell. In still another aspect, the isolated cell is derived from a non-human transgenic animal which exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality. The invention also provides a method of identifying an agent that modulates a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

In one aspect, the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In yet another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentiapigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In still another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased prepulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages of B cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages ofB cells; decreased CD4+ and CD8+ cells; decrease in natural killer cells; increase in monocytes; increased mean serum IgG1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.

The invention also provides an agent which modulates the phenotype associated with gene disruption. In one aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of identifying an agent that modulates a physiological characteristic associated with a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

In one aspect, the non-human transgenic animal exhibits at least one of the following physiological characteristics compared with gender matched wild-type littermates:

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased prepulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages of B cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages ofB cells; decreased CD4+ and CD8+ cells; decrease in natural killer cells; increase in monocytes; increased mean serum IgG 1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.

The invention also provides an agent that modulates a physiological characteristic which is associated with gene disruption. In one aspect, the agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of identifying an agent which modulates a behavior associated with a disruption ofthe gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PR3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

In one aspect, the observed behavior is an increased anxiety-like response during open field activity testing. In yet another aspect, the observed behavior is a decreased anxiety-like response during open field activity testing. In yet another aspect, the observed behavior is an abnormal circadian rhythm during home-cage activity testing. In yet another aspect, the observed behavior is an enhanced motor coordination during inverted screen testing. In yet another aspect, the observed behavior is impaired motor coordination during inverted screen testing. In yet another aspect, the observed behavior includes depression, generalized anxiety disorders, attention deficit disorder, sleep disorder, hyperactivity disorder, obsessive compulsive disorder, schizophrenia, cognitive disorders, hyperalgesia and sensory disorders. Such disorders include the category defmed as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The invention also provides an agent that modulates a behavior which is associated with gene disruption. In one aspect, the agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) administering a test agent to said non-human transgenic animal; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality associated with the gene disruption in the non-human transgenic animal.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defmed as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy ofthe retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism, or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

In another aspect, the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased prepulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages of B cells; decreased B1 to B2 ratio in peritoneal lavage; decreased peritoneal CD23- cells and corresponding increase in percentages of CD23+ cells; decrease in B220dim/CD43 dim cells; increase percentages ofB220dim/CD43dim cells in bone marrow; decrease CD11bhi cells and increased CD11bmed cells; increased CD62hiCD44 dim cells in lymph nodes; decreased percentages ofT cells and increased percentages of B cells; decreased CD4+ and CD8+ cells; decrease in natural killer cells; increase in monocytes; increased mean serum IgG1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption. In one aspect, the agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, ariti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a therapeutic agent for the treatment of a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a method of identifying an agent that modulates the expression of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) contacting a test agent with a host cell expressing a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide; and
(b) determining whether the test agent modulates the expression of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by the host cell.

The invention also provides an agent that modulates the expression of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In one aspect, the agent is an agonist or antagonist ofthe phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of evaluating a therapeutic agent capable of affecting a condition associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
(a) providing a non-human transgenic animal whose genome comprises a disruption of the gene which encodes for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

In one aspect, the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.

The invention also provides a therapeutic agent which is capable of affecting a condition associated with gene disruption. In one aspect, the agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO3 6029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a pharmaceutical composition comprising a therapeutic agent capable of affecting the condition associated with gene disruption.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject in need of such treatment whom may already have the disorder, or may be prone to have the disorder or may be in whom the disorder is to be prevented, a therapeutically effective amount of a therapeutic agent, or agonists or antagonists thereof, , thereby effectively treating or preventing or ameliorating said disorder or disease.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentiapigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft-versus-host disease.

In yet another aspect, the bone metabolic abnormality or disorder is arthritis, osteoporosis, osteopenia or osteopetrosis.

In another aspect the therapeutic agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agent is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO3 5444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PRO188, PRO235, PRO266, PRO3 37, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37S10, PRO35444, PRO20473, PRO21054 or PRO35246 polypeptide, the method comprising:
(a) providing a non-human transgenic animal cell culture, each cell of said culture comprising a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
(b) administering a test agent to said cell culture; and
(c) determining whether the test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said culture.

In yet another aspect, the neurological disorder is an increased anxiety-like response during open field activity testing. In yet another aspect, the neurological disorder is a decreased anxiety-like response during open field activity testing. In yet another aspect, the neurological disorder is an abnormal circadian rhythm during home-cage activity testing. In yet another aspect, the neurological disorder is an enhanced motor coordination during inverted screen testing. In yet another aspect, the neurological disorder is impaired motor coordination during inverted screen testing. In yet another aspect, the neurological disorder includes depression, generalized anxiety disorders, attention deficit disorder, sleep disorder, hyperactivity disorder, obsessive compulsive disorder, schizophrenia, cognitive disorders, hyperalgesia and sensory disorders. Such neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

In another aspect, the eye abnormality is a retinal abnormality. In still another aspect, the eye abnormality is consistent with vision problems or blindness. In yet another aspect, the retinal abnormality is consistent with retinitis pigmentosa or is characterized by retinal degeneration or retinal dysplasia.

In still another aspect, the retinal abnormalities are consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

In still another aspect, the eye abnormality is a cataract. In still yet another aspect, the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.

In still another aspect, the developmental abnormality comprises embryonic lethality or reduced viability.

In yet another aspect, the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.

In still yet another aspect, the immunological disorders are consistent with systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.

In yet another aspect, the bone metabolic abnormality or disorder is arthritis, osteoporosis, osteopenia or osteopetrosis.

The invention also provides an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality which is associated with gene disruption in said culture. In one aspect, the agent is an agonist or antagonist of the phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO1284, PRO37510, PRO35444, PRO20473, PRO21054 or PRO35246 polypeptide. In yet another aspect, the agent is an agonist or antagonistofa PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In yet another aspect, the agonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody. In still another aspect, the antagonist agent is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.

The invention also provides a method of modulating a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already have the phenotype, or may be prone to have the phenotype or may be in whom the phenotype is to be prevented, an effective amount of an agent identified as modulating said phenotype, or agonists or antagonists thereof, thereby effectively modulating the phenotype.

The invention also provides a method of modulating a physiological characteristic associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already exhibit the physiological characteristic, or may be prone to exhibit the physiological characteristic or may be in whom the physiological characteristic is to be prevented, an effective amount of an agent identified as modulating said physiological characteristic, or agonists or antagonists thereof, thereby effectively modulating the physiological characteristic.

The invention also provides a method of modulating a behavior associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already exhibit the behavior, or may be prone to exhibit the behavior or may be in whom the exhibited behavior is to be prevented, an effective amount of an agent identified as modulating said behavior, or agonists or antagonists thereof, thereby effectively modulating the behavior.

The invention also provides a method of modulating the expression of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a host cell expressing said PRO188, PRO, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, an effective amount of an agent identified as modulating said expression, or agonists or antagonists thereof, thereby effectively modulating the expression of said polypeptide.

The invention also provides a method of modulating a condition associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO, PRO20473, PRO21054 or PRO35246 polypeptide, the method comprising administering to a subject whom may have the condition, or may be prone to have the condition or may be in whom the condition is to be prevented, a therapeutically effective amount of a therapeutic agent identified as modulating said condition, or agonists or antagonists thereof, thereby effectively modulating the condition.

The invention also provides a method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO3 5246 polypeptide, the method comprising administering to a non-human transgenic animal cell culture, each cell of said culture comprising a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, an effective amount of an agent identified as treating or preventing or ameliorating said disorder, or agonists or antagonists thereof, thereby effectively treating or preventing or ameliorating said disorder.

### B. Further Embodiments

In yet further embodiments, the invention is directed to the following set of potential claims for this application:
1. A method of identifying a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) measuring a physiological characteristic of the non-human transgenic animal; and
   (c) comparing the measured physiological characteristic with that of a gender matched wild-type animal,
   wherein the physiological characteristic of the non-human transgenic animal that differs from the physiological characteristic of the wild-type animal is identified as a phenotype resulting from the gene disruption in the non-human transgenic animal.
2. The method of Claim 1, wherein the non-human transgenic animal is heterozygous for the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
3. The method of Claim 1, wherein the phenotype exhibited by the non-human transgenic animal as compared with gender matched wild-type littermates is at least one of the following: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
4. The method of Claim 3, wherein the neurological disorder is an increased anxiety-like response during open field activity testing.
5. The method of Claim 3, wherein the neurological disorder is a decreased anxiety-like response during open field activity testing.
6. The method of Claim 3, wherein the neurological disorder is an abnormal circadian rhythm during home-cage activity testing.
7. The method of Claim 3, wherein the neurological disorder is an enhanced motor coordination during inverted screen testing.
8. The method of Claim 3, wherein the neurological disorder is an impaired motor coordination during inverted screen testing.
9. The method of Claim 3, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
10. The method of Claim 3, wherein the eye abnormality is a retinal abnormality.
11. The method of Claim 3, wherein the eye abnormality is consistent with vision problems or blindness.
12. The method of Claim 10, wherein the retinal abnormality is consistent with retinitis pigmentosa.
13. The method of Claim 10, wherein the retinal abnormality is characterized by retinal degeneration or retinal dysplasia.
14. The method of Claim 10, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
15. The method of Claim 3, wherein the eye abnormality is a cataract.
16. The method of Claim 15, wherein the cataract is consistent with systemic diseases such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
17. The method of Claim 3, wherein the developmental abnormality comprises embryonic lethality or reduced viability.
18. The method of Claim 3, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
19. The method of Claim 3, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
20. The method of Claim 3, wherein the bone metabolic abnormality or disorder is arthritis, osteoporosis or osteopetrosis.
21. The method of Claim 1, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased pre-pulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages ofB cells in blood; decreased percentages of CD4 and CD8 cells and increased percentages ofB cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages ofB cells; decreased CD4+ and CD8+ cells; decrease in natural killer cells; increase in monocytes; increased mean serum IgG 1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG 1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.
22. An isolated cell derived from a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
23. The isolated cell of Claim 22 which is a murine cell.
24. The isolated cell of Claim 23, wherein the murine cell is an embryonic stem cell.
25. The isolated cell of Claim 22, wherein the non-human transgenic animal exhibits at least one of the following phenotypes compared with gender matched wild-type littermates: a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
26. A method of identifying an agent that modulates a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
   (d) administering a test agent to the non-human transgenic animal of (a); and
   (e) determining whether the test agent modulates the identified phenotype associated with gene disruption in the non-human transgenic animal.
27. The method of Claim 26, wherein the phenotype associated with the gene disruption comprises a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
28. The method of Claim 27, wherein the neurological disorder is an increased anxiety-like response during open field activity testing.
29. The method of Claim 27, wherein the neurological disorder is a decreased anxiety-like response during open field activity testing.
30. The method of Claim 27, wherein the neurological disorder is an abnormal circadian rhythm during home-cage activity testing.
31. The method of Claim 27, wherein the neurological disorder is an enhanced motor coordination during inverted screen testing.
32. The method of Claim 27, wherein the neurological disorder is an impaired motor coordination during inverted screen testing.
33. The method of Claim 27, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
34. The method of Claim 27, wherein the eye abnormality is a retinal abnormality.
35. The method of Claim 27, wherein the eye abnormality is consistent with vision problems or blindness.
36. The method of Claim 34, wherein the retinal abnormality is consistent with retinitis pigmentosa.
37. The method of Claim 34, wherein the retinal abnormality is characterized by retinal degeneration or retinal dysplasia.
38. The method of Claim 34, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
39. The method of Claim 27, wherein the eye abnormality is a cataract.
40. The method of Claim 39, wherein the cataract is consistent with systemic diseases such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
41. The method of Claim 27, wherein the developmental abnormality comprises embryonic lethality or reduced viability.
42. The method of Claim 27, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
43. The method of Claim 27, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation-associated diseases including graft rejection and graft -versus-host disease.
44. The method of Claim 27, wherein said bone metabolic abnormality or disorder is arthritis, osteoporosis or osteopetrosis.
45. The method of Claim 26, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased pre-pulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages ofB cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages ofB cells; decreased CD4+ and CD8+ cells; decrease in natural killer cells; increase in monocytes; increased mean serum IgG1 response to ovalbumin challenge; decreased mean serum IgG 1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.
46. An agent identified by the method of Claim 26.
47. The agent of Claim 46 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
48. The agent of Claim 47, wherein the agonist is an anti-PRO188, anti-PRO235, anti-PRO266, anti-PRO337,
   anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 oranti-PRO35246 antibody.
49. The agent of Claim 47, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
50. A method of identifying an agent that modulates a physiological characteristic associated with a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) measuring a physiological characteristic exhibited by the non-human transgenic animal of (a);
   (c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
   (d) administering a test agent to the non-human transgenic animal of (a); and
   (e) determining whether the physiological characteristic associated with gene disruption is modulated.
51. The method of Claim 50, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased pre-pulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages of B cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages ofB cells; decreased CD4+ and CD8+ cells; decrease in natural killer cells; increase in monocytes; increased mean serum IgG 1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.
52. An agent identified by the method of Claim 50.
53. The agent of Claim 52 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
54. The agent of Claim 53, wherein the agonist is an anti-PRO188, anti-PRO235, anti-PRO266, anti-PRO337,
   anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
55. The agent of Claim 53, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
56. A method of identifying an agent which modulates a behavior associated with a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) observing the behavior exhibited by the non-human transgenic animal of (a);
   (c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
   (d) administering a test agent to the non-human transgenic animal of (a); and
   (e) determining whether the agent modulates the behavior associated with gene disruption.
57. The method of Claim 56, wherein the behavior is an increased anxiety-like response during open field activity testing.
58. The method of Claim 56, wherein the behavior is a decreased anxiety-like response during open field activity testing.
59. The method of Claim 56, wherein the behavior is an abnormal circadian rhythm during home-cage activity testing.
60. The method of Claim 56, wherein the behavior is an enhanced motor coordination during inverted screen testing.
61. The method of Claim 56, wherein the behavior is an impaired motor coordination during inverted screen testing.
62. The method of Claim 56, wherein the behavior is depression, generalized anxiety disorders, attention deficit disorder, sleep disorder, hyperactivity disorder, obsessive compulsive disorder, schizophrenia, cognitive disorders, hyperalgesia or sensory disorders.
63. An agent identified by the method of Claim 56.
64. The agent of Claim 63 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
65. The agent of Claim 64, wherein the agonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
66. The agent of Claim 64, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
67. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) administering a test agent to said non-human transgenic animal; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in the non-human transgenic animal.
68. The method of Claim 67, wherein the neurological disorder is an increased anxiety-like response during open field activity testing.
69. The method of Claim 67, wherein the neurological disorder is a decreased anxiety-like response during open field activity testing.
70. The method of Claim 67, wherein the neurological disorder is an abnormal circadian rhythm during home-cage activity testing.
71. The method of Claim 67, wherein the neurological disorder is an enhanced motor coordination during inverted screen testing.
72. The method of Claim 67, wherein the neurological disorder is an impaired motor coordination during inverted screen testing.
73. The method of Claim 73, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
74. The method of Claim 67, wherein the eye abnormality is a retinal abnormality.
75. The method of Claim 67, wherein the eye abnormality is consistent with vision problems or blindness.
76. The method of Claim 74, wherein the retinal abnormality is consistent with retinitis pigmentosa.
77. The method of Claim 74, wherein the retinal abnormality is characterized by retinal degeneration or retinal dysplasia.
78. The method of Claim 74, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
79. The method of Claim 67, wherein the eye abnormality is a cataract.
80. The method of Claim 79, wherein the cataract is a systemic disease such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
81. The method of Claim 67, wherein the developmental abnormality comprises embryonic lethality or reduced viability.
82. The method of Claim 67, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
83. The method of Claim 67, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft-versus-host disease.
84. The method of Claim 67, wherein said bone metabolic abnormality or disorder is arthritis, osteoporosis or osteopetrosis.
85. The method of Claim 67, wherein the non-human transgenic animal exhibits at least one of the following physiological characteristics compared with gender matched wild-type littermates: increased anxiety-like response during open field testing; hyperactivity during open field testing; decreased anxiety during open field testing; decreased locomotor activity during open field testing; decreased hole-poke and rearing or decreased exploratory behavior in open field testing; abnormal circadian rhythm during home-cage activity testing (increased activity during the end of light phase/beginning of dark phase in circadian rhythm testing; altered sleep/wake cycle; abnormal circadian rhythm); during home-cage activity testing including decreased ambulatory counts; abnormal circadian rhythm during home-cage activity testing including increased ambulatory counts; decreased rearing; abnormal circadian rhythm with increased activity (dark to light phases); abnormal circadian rhythm with augmentation or increase in activity during the early part of dark phase; decreased sensitivity to stress induced hyperthermia; impaired motor coordination during inverted screen testing; enhanced motor coordination in inverted screen testing; increased pre-pulse inhibition response indicating enhanced sensorimotor gating/attention; decreased pre-pulse inhibition with impaired sensorimotor gating/attention; decreased immobility during tail suspension testing with decreased depressive-like response; decreased latency to respond in hot plate testing; opthamological abnormalities; increased artery to vein ratio; decreased heart rate; increased heart rate; decreased basal body temperature; decreased mean systolic blood pressure; increased mean fasting serum glucose levels; decreased mean serum glucose levels; decreased mean serum glucose levels in heterozygous mice; enhanced glucose tolerance; increased insulin sensitivity; increased mean serum cholesterol levels; increased mean serum triglyceride levels; impaired glucose tolerance; decreased uric acid levels; decreased calcium levels; increased mean serum alkaline phosphatase levels; decreased alkaline phosphatase levels; increased total bilirubin levels; hematauria in homozygous mice and heterozygous mice; increased total white blood cell (WBC) count; increased mean absolute lymphocyte count; increase in peripheral blood eosinophils; increased mean platelet count; increased mean platelet volume; increase in red blood cells (RBCs) with a decrease in corpuscular volume; decreased hemoglobin concentration and hematocrit; increased percentages of CD4 cells and decreased percentages of B cells in blood; decreased percentages of CD4 and CD8 cells and increased percentages ofB cells; decreased B1 to B2 ratio in peritoneal lavage; decreased peritoneal CD23- cells and corresponding increase in percentages of CD23+ cells; decrease in B220dim/CD43 dim cells; increase percentages of B220dim/CD43dim cells in bone marrow; decrease CD11bhi cells and increased CD11bmed cells; increased CD62hiCD44 dim cells in lymph nodes; decreased percentages ofT cells and increased percentages of B cells; decreased CD4+and CD8+ cells; decrease in natural killer cells; increase in monocytes; increased mean serum IgG1 response to ovalbumin challenge; decreased mean serum IgG1 response to ovalbumin challenge; increased mean serum IgG2a response to ovalbumin challenge; decreased mean serum IgG2a response to ovalbumin challenge; increased mean serum IL-6 response to LPS challenge; increased mean serum TNF alpha response to LPS challenge; increased mean serum MCP-1 response to LPS challenge; increased mean serum IgM level; increase mean serum IgG 1; increased mean serum IgG2a; increased mean serum IgG2b; decreased skin fibroblast proliferation rate; increased skin fibroblast proliferation rate; increased skin fibroblast proliferation rate in heterozygous mice; increased mean percent of total body fat and total fat mass; increased mean percent total body fat in heterozygous mice; increased mean body weight; increased mean body length; increased total tissue mass (TTM); increased total tissue mass (TTM) in heterozygous mice; increased in lean body mass (LBM); increased in lean body mass (LBM) in heterozygous mice; increased bone mineral density (BMD); increase in bone mineral content (BMC); increased mean femoral mid-shaft cortical thickness; increased mean femoral mid-shaft cross-sectional area; increased mean femoral mid-shaft cross-sectional area in heterozygous mice; increased mean trabecular bone volume, number and connectivity density; increased BMC/LBM ratio; increase in bone mineral content in heterozygous mice; increased BMC/LBM ratio in heterozygous mice; increase in total body bone mineral density; increase in total body vBMD; decreased mean percent of total body fat and total fat mass; decreased mean body weight; decreased mean body length; decreased mean body weight and length in heterozygous mice; decreased total tissue mass (TTM); decreased lean body mass (LBM); decreased lean body mass (LBM) in heterozygous mice; decreased femoral bone mineral density (BMD); decreased vertebral bone mineral density (BMD); decreased bone mineral density (BMD) in total body; decreased bone mineral content (BMC) in heterozygous mice; decreased bone mineral density (total body and vertebrae BMD) in heterozygous mice; decreased bone mineral content (BMC); decreased bone mineral density index (BMC/LBM); increased BMC/LBM; decreased total body volumetric bone mineral density (vBMD); decreased mean femoral mid-shaft cortical thickness; decreased mean femoral mid-shaft cross-sectional area; decreased mean vertebral trabecular bone volume, number and connectivity density; osteopetrosis; osteoporosis; minimal-to-moderate necrosis, inflammation and/or regeneration of skeletal muscle; defective spermatogenesis in the testes; hypospermia and defective spermatozoa in the epididymus; male infertility; testicular degeneration; decreased testes weight; abnormal urination; decreased brain weight; alterations in hematopoietic system: hypoplasia of lymphoid and hematopoietic cells in the spleen, cytoplasmic vacuolization in hepatocytes, lipid depletion in adipose tissue and reduced hematopoiesis in bone marrow; growth retardation; small mice and failure to thrive; reduced viability; exencephaly and perinatal lethality; embryonic lethality with cardiac defects marked by prominent ventricular and atrial septa defects; embryonic lethality with multiple craniofacial abnormalities, including absence of the nares, mouth and ear canals, with affected mutants lacking a lower jaw, tongue and associated structures (eyes and other structures of the face were hypoplastic and deformed, some with no facial features; and homozygous embryonic lethality.
86. An agent identified by the method of Claim 67.
87. The agent of Claim 86 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
88. The agent of Claim 87, wherein the agonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
89. The agent of Claim 87, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
90. A therapeutic agent identified by the method of Claim 67.
91. A method of identifying an agent that modulates the expression of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) contacting a test agent with a host cell expressing a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide; and
   (b) determining whether the test agent modulates the expression of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by the host cell.
92. An agent identified by the method of Claim 91.
93. The agent of Claim 92 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
94. The agent of Claim 93, wherein the agonist is an anti-PRO188, anti-PRO235, anti-PRO266, anti-PRO337,
   anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
95. The agent of Claim 93, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
96. A method of evaluating a therapeutic agent capable of affecting a condition associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal whose genome comprises a disruption of the gene which encodes for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) measuring a physiological characteristic of the non-human transgenic animal of (a);
   (c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
   (d) administering a test agent to the non-human transgenic animal of (a); and
   (e) evaluating the effects of the test agent on the identified condition associated with gene disruption in the non-human transgenic animal.
97. The method of Claim 96, wherein the condition is a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality.
98. A therapeutic agent identified by the method of Claim 96.
99. The therapeutic agent of Claim 98 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
100. The therapeutic agent of Claim 99, wherein the agonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
101. The therapeutic agent of Claim 99, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
102. A pharmaceutical composition comprising the therapeutic agent of Claim 98.
103. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject in need of such treatment whom may already have the disorder, or may be prone to have the disorder or may be in whom the disorder is to be prevented, a therapeutically effective amount of the therapeutic agent of Claim 94, or agonists or antagonists thereof, thereby effectively treating or preventing or ameliorating said disorder.
104. The method of Claim 103, wherein the neurological disorder is an increased anxiety-like response during open field activity testing.
105. The method of Claim 103, wherein the neurological disorder is a decreased anxiety-like response during open field activity testing.
106. The method of Claim 103, wherein the neurological disorder is an abnormal circadian rhythm during home-cage activity testing.
107. The method of Claim 103, wherein the neurological disorder is an enhanced motor coordination during inverted screen testing.
108. The method of Claim 103, wherein the neurological disorder is an impaired motor coordination during inverted screen testing.
109. The method of Claim 103, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
110. The method of Claim 103, wherein the eye abnormality is a retinal abnormality.
111. The method of Claim 103, wherein the eye abnormality is consistent with vision problems or blindness.
112. The method of Claim 110, wherein the retinal abnormality is consistent with retinitis pigmentosa.
113. The method of Claim 110, wherein the retinal abnormality is characterized by retinal degeneration or retinal dysplasia.
114. The method of Claim 110, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
115. The method of Claim 103, wherein the eye abnormality is a cataract.
116. The method of Claim 115, wherein the cataract is a systemic disease such as human Down's syndrome, Hallerman-Streiffsyndrome, Lowe syndrome, galactosemia, Marfan syndrome, Trismoy 13-15, Alport syndrome, myotonic dystrophy, Fabry disease, hypoparathroidism or Conradi syndrome.
117. The method of Claim 103, wherein the developmental abnormality comprises embryonic lethality or reduced viability.
118. The method of Claim 103, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
119. The method of Claim 103, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; or transplantation associated diseases including graft rejection and graft -versus-host disease.
120. The method of Claim 103, wherein said bone metabolic abnormality or disorder is arthritis, osteoporosis or osteopetrosis.
121. A method of identifying an agent that ameliorates or modulates a neurological disorder; a cardiovascular, endothelial or angiogenic disorder; an eye abnormality; an immunological disorder; an oncological disorder; a bone metabolic abnormality or disorder; a lipid metabolic disorder; or a developmental abnormality associated with a disruption in the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising:
   (a) providing a non-human transgenic animal cell culture, each cell of said culture comprising a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide;
   (b) administering a test agent to said cell culture; and
   (c) determining whether said test agent ameliorates or modulates the neurological disorder; cardiovascular, endothelial or angiogenic disorder; eye abnormality; immunological disorder; oncological disorder; bone metabolic abnormality or disorder; lipid metabolic disorder; or developmental abnormality in said cell culture.
122. The method of Claim 121, wherein the neurological disorder is an increased anxiety-like response during open field activity testing.
123. The method of Claim 121, wherein the neurological disorder is a decreased anxiety-like response during open field activity testing.
124. The method of Claim 121, wherein the neurological disorder is an abnormal circadian rhythm during home-cage activity testing.
125. The method of Claim 121, wherein the neurological disorder is an enhanced motor coordination during inverted screen testing.
126. The method of Claim 121, wherein the neurological disorder is an impaired motor coordination during inverted screen testing.
127. The method of Claim 121, wherein the neurological disorder is depression, generalized anxiety disorders, attention deficit disorder, sleep disorder, hyperactivity disorder, obsessive compulsive disorder, schizophrenia, cognitive disorders, hyperalgesia or sensory disorders.
128. The method of Claim 121, wherein the eye abnormality is a retinal abnormality.
129. The method of Claim 121, wherein the eye abnormality is consistent with vision problems or blindness.
130. The method of Claim 128, wherein the retinal abnormality is consistent with retinitis pigmentosa.
131. The method of Claim 128, wherein the retinal abnormality is characterized by retinal degeneration or retinal dysplasia.
132. The method of Claim 128, wherein the retinal abnormality is consistent with retinal dysplasia, various retinopathies, including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.
133. The method of Claim 121, wherein the eye abnormality is a cataract.
134. The method of Claim 133, wherein the cataract is a systemic disease such as human Down's syndrome, Hallerman-Streiff syndrome, Lowe syndrome, galactosemia, Marfan syndrome, Trismoy 13-15, Alport syndrome, myotonic dystrophy, Fabry disease, hypoparathroidism or Conradi syndrome.
135. The method of Claim 121, wherein the developmental abnormality comprises embryonic lethality or reduced viability.
136. The method of Claim 121, wherein the cardiovascular, endothelial or angiogenic disorders are arterial diseases, such as diabetes mellitus; papilledema; optic atrophy; atherosclerosis; angina; myocardial infarctions such as acute myocardial infarctions, cardiac hypertrophy, and heart failure such as congestive heart failure; hypertension; inflammatory vasculitides; Reynaud's disease and Reynaud's phenomenon; aneurysms and arterial restenosis; venous and lymphatic disorders such as thrombophlebitis, lymphangitis, and lymphedema; peripheral vascular disease; cancer such as vascular tumors, *e*.*g*., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma; tumor angiogenesis; trauma such as wounds, burns, and other injured tissue, implant fixation, scarring; ischemia reperfusion injury; rheumatoid arthritis; cerebrovascular disease; renal diseases such as acute renal failure, or osteoporosis.
137. The method of Claim 121, wherein the immunological disorders are systemic lupus erythematosis; rheumatoid arthritis; juvenile chronic arthritis; spondyloarthropathies; systemic sclerosis (scleroderma); idiopathic inflammatory myopathies (dermatomyositis, polymyositis); Sjögren's syndrome; systemic vasculitis; sarcoidosis; autoimmune hemolytic anemia (immune pancytopenia, paroxysmal nocturnal hemoglobinuria); autoimmune thrombocytopenia (idiopathic thrombocytopenic purpura, immune-mediated thrombocytopenia); thyroiditis (Grave's disease, Hashimoto's thyroiditis, juvenile lymphocytic thyroiditis, atrophic thyroiditis); diabetes mellitus; immune-mediated renal disease (glomerulonephritis, tubulointerstitial nephritis); demyelinating diseases of the central and peripheral nervous systems such as multiple sclerosis, idiopathic demyelinating polyneuropathy or Guillain-Barré syndrome, and chronic inflammatory demyelinating polyneuropathy; hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis; inflammatory bowel disease (ulcerative colitis: Crohn's disease); gluten-sensitive enteropathy, and Whipple's disease; autoimmune or immune-mediated skin diseases including bullous skin diseases, erythema multiforme and contact dermatitis, psoriasis; allergic diseases such as asthma, allergic rhinitis, atopic dermatitis, food hypersensitivity and urticaria; immunologic diseases of the lung such as eosinophilic pneumonia, idiopathic pulmonary fibrosis and hypersensitivity pneumonitis; ortransplantation associated diseases including graft rejection and graft -versus-host disease.
138. The method of Claim 121, wherein said bone metabolic abnormality or disorder is arthritis, osteoporosis or osteopetrosis.
139. An agent identified by the method of Claim 121.
140. The agent of Claim 139 which is an agonist or antagonist of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
141. The agent of Claim 140, wherein the agonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
142. The agent of Claim 140, wherein the antagonist is an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody.
143. A therapeutic agent identified by the method of Claim 121.
144. A method of modulating a phenotype associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already have the phenotype, or may be prone to have the phenotype or may be in whom the phenotype is to be prevented, an effective amount of the agent of Claim 46, or agonists or antagonists thereof, thereby effectively modulating the phenotype.
145. A method of modulating a physiological characteristic associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already exhibit the physiological characteristic, or may be prone to exhibit the physiological characteristic or may be in whom the physiological characteristic is to be prevented, an effective amount of the agent of Claim 52, or agonists or antagonists thereof, thereby effectively modulating the physiological characteristic.
146. A method of modulating a behavior associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may already exhibit the behavior, or may be prone to exhibit the behavior or may be in whom the exhibited behavior is to be prevented, an effective amount of the agent of Claim 63, or agonists or antagonists thereof, thereby effectively modulating the behavior.
147. A method of modulating the expression of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a host cell expressing said PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, an effective amount of the agent of Claim 92, or agonists or antagonists thereof, thereby effectively modulating the expression of said polypeptide.
148. A method of modulating a condition associated with a disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a subject whom may have the condition, or may be prone to have the condition or may be in whom the condition is to be prevented, a therapeutically effective amount of the therapeutic agent of Claim 98, or agonists or antagonists thereof, thereby effectively modulating the condition.
149. A method of treating or preventing or ameliorating a neurological disorder; cardiovascular, endothelial or angiogenic disorder; immunological disorder; oncological disorder; bone metabolic abnormality or disorder, or embryonic lethality associated with the disruption of a gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the method comprising administering to a non-human transgenic animal cell culture, each cell of said culture comprising a disruption of the gene which encodes for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, a therapeutically effective amount of the agent of Claim 139, or agonists or antagonists thereof, thereby effectively treating or preventing or ameliorating said disorder.

### BRIEF DESCRIPTION OF THE DRAWINGS

Figure 1 shows a nucleotide sequence (SEQ ID NO:1) of a native sequence PRO188 cDNA, wherein SEQ ID NO:1 is a clone designated herein as "DNA28497-1130" (UNQ162).
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PRO235 cDNA, wherein SEQ ID NO:3 is a clone designated herein as "DNA35558-1167" (UNQ209).
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PRO266 cDNA, wherein SEQ ID NO:5 is a clone designated herein as "DNA37150-1178" (UNQ233).
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PRO337 cDNA, wherein SEQ ID NO:7 is a clone designated herein as "DNA43316-1237" (UNQ297).
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PRO361 cDNA, wherein SEQ ID NO:9 is a clone designated herein as "DNA45410-1250" (UNQ316).
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PRO539 cDNA, wherein SEQ ID NO:11 is a clone designated herein as "DNA47465-1561" (UNQ340).
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PRO698 cDNA, wherein SEQ ID NO:13 is a clone designated herein as "DNA48320-1433" (UNQ362).
Figure 14 shows the amino acid sequence (SEQ ID NO:14) derived from the coding sequence of SEQ ID NO: 13 shown in Figure 13.
Figure 15 shows a nucleotide sequence (SEQ ID NO:15) of a native sequence PRO717 cDNA, wherein SEQ ID NO:15 is a clone designated herein as "DNA50988-1326" (UNQ385).
Figure 16 shows the amino acid sequence (SEQ ID NO:16) derived from the coding sequence of SEQ ID NO:15 shown in Figure 15.
Figure 17 shows a nucleotide sequence (SEQ ID NO:17) of a native sequence PRO846 cDNA, wherein SEQ ID NO:17 is a clone designated herein as "DNA44196-1353" (UNQ422).
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PRO874 cDNA, wherein SEQ ID NO:19 is a clone designated herein as "DNA40621-1440" (UNQ441).
Figure 20 shows the amino acid sequence (SEQ ID NO:20) derived from the coding sequence of SEQ ID NO:19 shown in Figure 19.

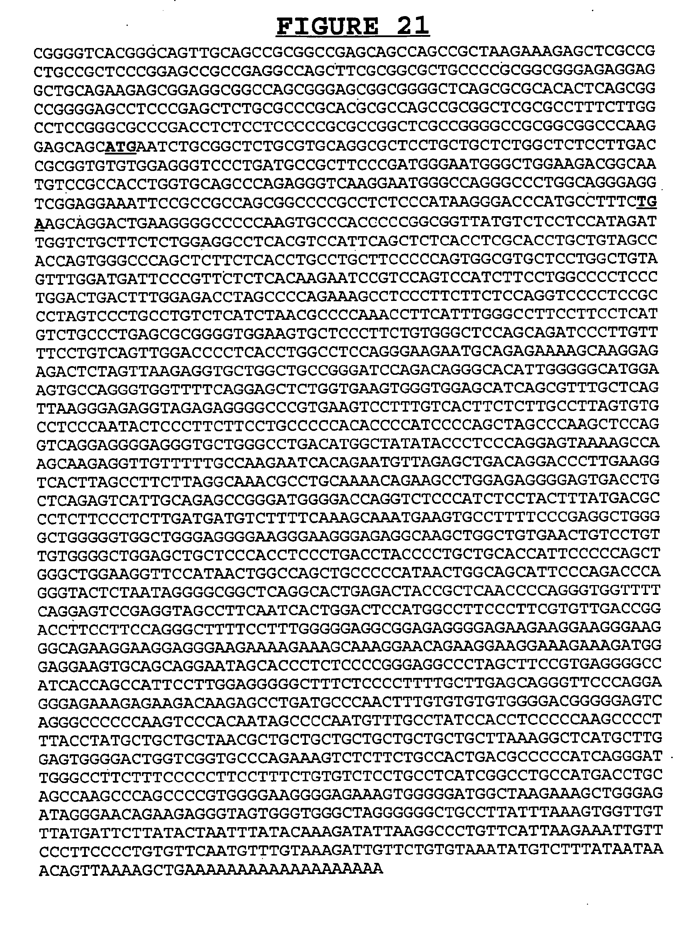

Figure 21 shows a nucleotide sequence (SEQ ID NO:21) of a native sequence PRO98346 cDNA, wherein SEQ ID NO:21 is a clone designated herein as "DNA349738" (UNQ471).

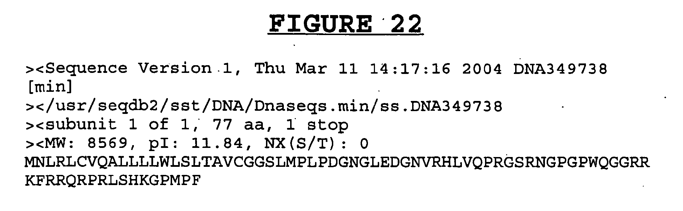

Figure 22 shows the amino acid sequence (SEQ ID NO:22) derived from the coding sequence of SEQ ID NO:21 shown in Figure 21.
Figure 23 shows a nucleotide sequence (SEQ ID NO:23) of a native sequence PRO1082 cDNA, wherein SEQ ID NO:23 is a clone designated herein as "DNA53912-1457" (UNQ539/589).
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PRO1097 cDNA, wherein SEQ ID NO:25 is a clone designated herein as "DNA59841-1460" (UNQ542).
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PRO1192 cDNA, wherein SEQ ID NO:27 is a clone designated herein as "DNA62814-1521" (UNQ606).
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PRO1268 cDNA, wherein SEQ ID NO:29 is a clone designated herein as "DNA66519-1535" (UNQ638).
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PRO1278 cDNA, wherein SEQ ID NO:31 is a clone designated herein as "DNA66304-1546" (UNQ648).
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PRO1303 cDNA, wherein SEQ ID NO:33 is a clone designated herein as "DNA65409-1566" (UNQ669).
Figure 34 shows the amino acid sequence (SEQ ID NO:34) derived from the coding sequence of SEQ ID NO:33 shown in Figure 33.
Figure 35 shows a nucleotide sequence (SEQ ID NO:35) of a native sequence PRO1308 cDNA, wherein SEQ ID NO:35 is a clone designated herein as "DNA62306-1570" (UNQ674).
Figure 36 shows the amino acid sequence (SEQ ID NO:36) derived from the coding sequence of SEQ ID NO:35 shown in Figure 35.
Figure 37 shows a nucleotide sequence (SEQ ID NO:37) of a native sequence PRO1338 cDNA, wherein SEQ ID NO:37 is a clone designated herein as "DNA66667-1596" (UNQ693).
Figure 38 shows the amino acid sequence (SEQ ID NO:38) derived from the coding sequence of SEQ ID NO:37 shown in Figure 37.
Figure 39 shows a nucleotide sequence (SEQ ID NO:39) of a native sequence PRO1378 cDNA, wherein SEQ ID NO:39 is a clone designated herein as "DNA58730-1607" (UNQ715).
Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PRO1415 cDNA, wherein SEQ ID NO:41 is a clone designated herein as "DNA58852-1637" (UNQ731).
Figure 42 shows the amino acid sequence (SEQ ID NO:42) derived from the coding sequence of SEQ ID NO:41 shown in Figure 41.
Figure 43 shows a nucleotide sequence (SEQ ID NO:43) of a native sequence PRO1867 cDNA, wherein SEQ ID NO:43 is a clone designated herein as "DNA84925-2514" (UNQ858).
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PRO1890 cDNA, wherein SEQ ID NO:45 is a clone designated herein as "DNA79230-2525" (UNQ872).
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PRO3438 cDNA, wherein SEQ ID NO:47 is a clone designated herein as "DNA82364-2538" (UNQ1825).
Figure 48 shows the amino acid sequence (SEQ ID NO:48) derived from the coding sequence of SEQ ID NO:47 shown in Figure 47.

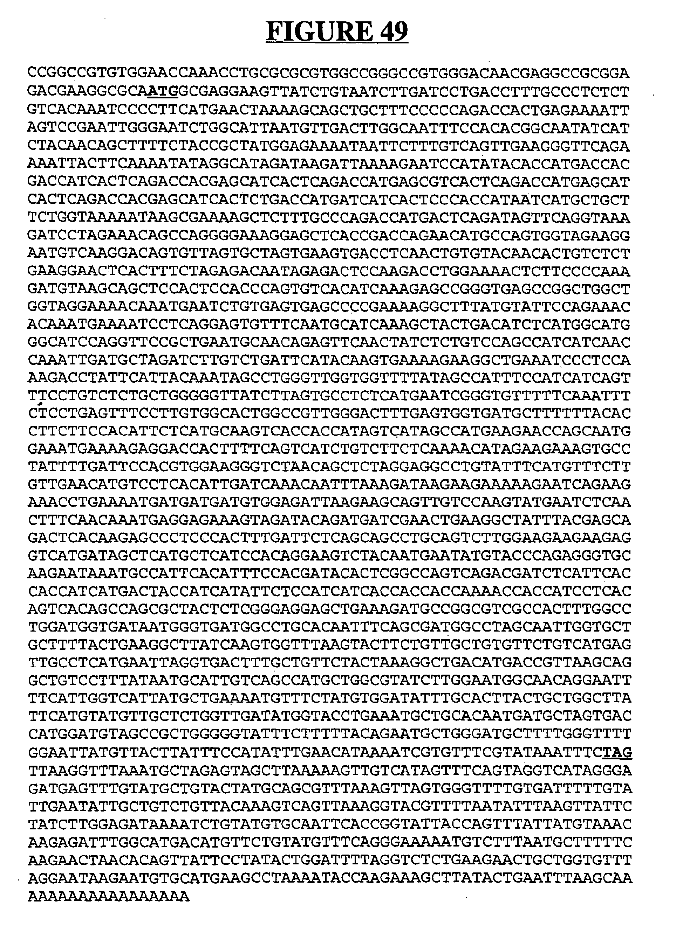

Figure 49 shows a nucleotide sequence (SEQ ID NO:49) of a native sequence PRO19835 cDNA, wherein SEQ ID NO:49 is a clone designated herein as "DNA164647" (UNQ2194).

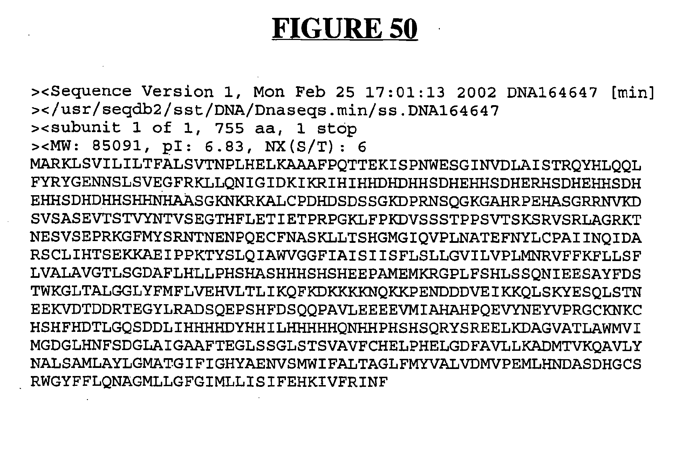

Figure 50 shows the amino acid sequence (SEQ ID NO:50) derived from the coding sequence of SEQ ID NO:49 shown in Figure 49.

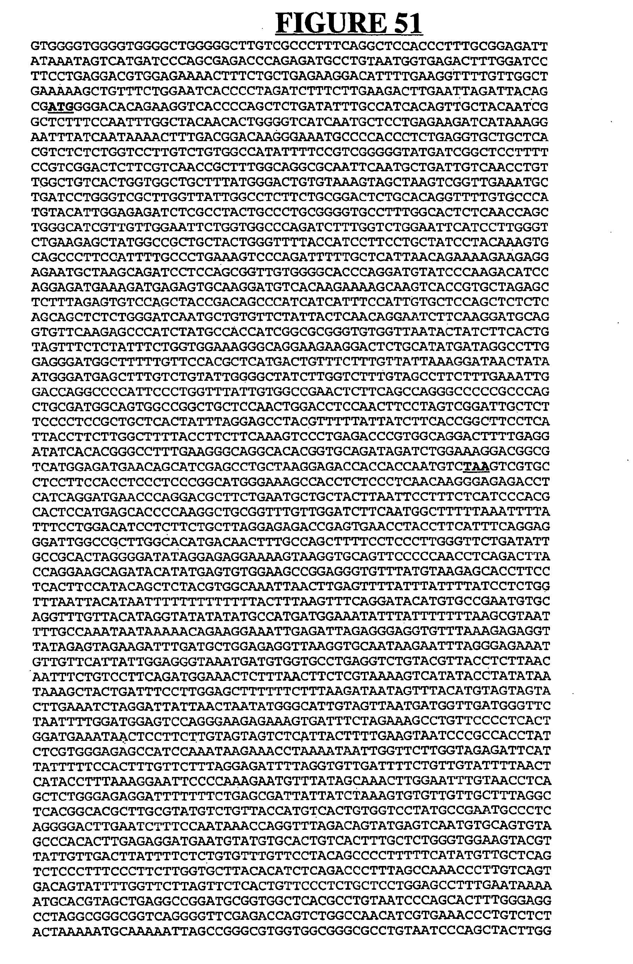

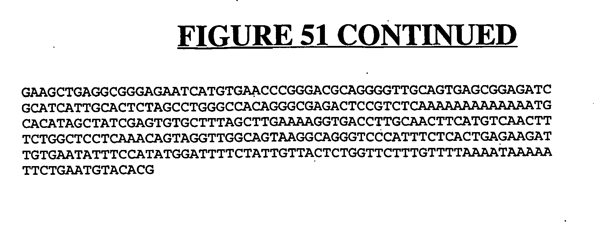

Figure 51 shows a nucleotide sequence (SEQ ID NO:51) of a native sequence PRO36915 cDNA, wherein SEQ ID NO:51 is a clone designated herein as "DNA226452" (UNQ2235).

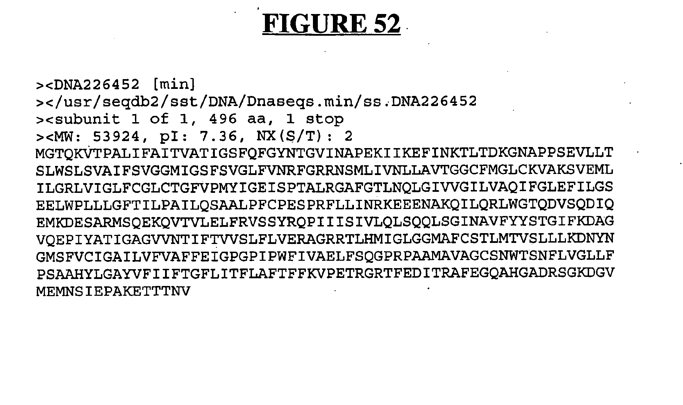

Figure 52 shows the amino acid sequence (SEQ ID NO:52) derived from the coding sequence of SEQ ID NO:51 shown in Figure 51.

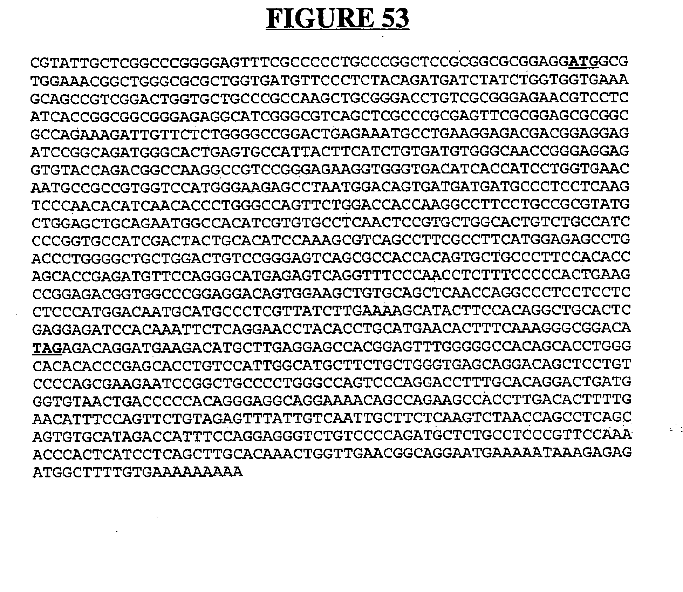

Figure 53 shows a nucleotide sequence (SEQ ID NO:53) ofa native sequence PRO36029 cDNA, wherein SEQ ID NO:53 is a clone designated herein as "DNA225566" (UNQ2424).

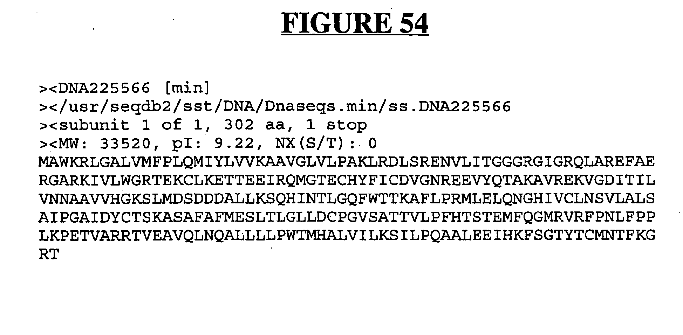

Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PRO4999 cDNA, wherein SEQ ID NO:55 is a clone designated herein as "DNA96031-2664" (UNQ2438).
Figure 56 shows the amino acid sequence (SEQ ID NO:56) derived from the coding sequence of SEQ ID NO:55 shown in Figure 55.
Figure 57 shows a nucleotide sequence (SEQ ID NO:57) of a native sequence PRO5778 cDNA, wherein SEQ ID NO:57 is a clone designated herein as "DNA96894-2675" (UNQ2491).
Figure 58 shows the amino acid sequence (SEQ ID NO:58) derived from the coding sequence of SEQ ID NO:57 shown in Figure 57.
Figure 59 shows a nucleotide sequence (SEQ ID NO:59) of a native sequence PRO5997 cDNA, wherein SEQ ID NO:59 is a clone designated herein as "DNA97005-2687" (UNQ2509).
Figure 60 shows the amino acid sequence (SEQ ID NO:60) derived from the coding sequence of SEQ ID NO:59 shown in Figure 59.
Figure 61 shows a nucleotide sequence (SEQ ID NO:61) of a native sequence PRO6079 cDNA, wherein SEQ ID NO:61 is a clone designated herein as "DNA111750-2706" (UNQ2538).
Figure 62 shows the amino acid sequence (SEQ ID NO:62) derived from the coding sequence of SEQ ID NO:61 shown in Figure 61.
Figure 63 shows a nucleotide sequence (SEQ ID NO:63) of a native sequence PRO6090 cDNA, wherein SEQ ID NO:63 is a clone designated herein as "DNA107781-2707" (UNQ2540).
Figure 64 shows the amino acid sequence (SEQ ID NO:64) derived from the coding sequence of SEQ ID NO:63 shown in Figure 63.
Figure 65 shows a nucleotide sequence (SEQ ID NO:65) of a native sequence PRO7178 cDNA, wherein SEQ ID NO:65 is a clone designated herein as "DNA108789-2748" (UNQ2788).
Figure 66 shows the amino acid sequence (SEQ ID NO:66) derived from the coding sequence of SEQ ID NO:65 shown in Figure 65.
Figure 67 shows a nucleotide sequence (SEQ ID NO:67) of a native sequence PRO21184 cDNA, wherein SEQ ID NO:67 is a clone designated herein as "DNA167678-2963" (UNQ2945).
Figure 68 shows the amino acid sequence (SEQ ID NO:68) derived from the coding sequence of SEQ ID NO:67 shown in Figure 67.
Figure 69 shows a nucleotide sequence (SEQ ID NO:69) of a native sequence PRO7434 cDNA, wherein SEQ ID NO:69 is a clone designated herein as "DNA123430-2755" (UNQ2972).
Figure 70 shows the amino acid sequence (SEQ ID NO:70) derived from the coding sequence of SEQ ID NO:69 shown in Figure 69.
Figure 71 shows a nucleotide sequence (SEQ ID NO:71) of a native sequence PRO9822 cDNA, wherein SEQ ID NO:71 is a clone designated herein as "DNA108738-2767" (UNQ3024).
Figure 72 shows the amino acid sequence (SEQ ID NO:72) derived from the coding sequence of SEQ ID NO:71 shown in Figure 71.
Figure 73 shows a nucleotide sequence (SEQ ID NO:73) of a native sequence PRO9833 cDNA, wherein SEQ ID NO:73 is a clone designated herein as "DNA130809-2769" (UNQ3030).
Figure 74 shows the amino acid sequence (SEQ ID NO:74) derived from the coding sequence of SEQ ID NO:73 shown in Figure 73.
Figure 75 shows a nucleotide sequence (SEQ ID NO:75) of a native sequence PRO9836 cDNA, wherein SEQ ID NO:75 is a clone designated herein as "DNA119514-2772" (UNQ3034).
Figure 76 shows the amino acid sequence (SEQ ID NO:76) derived from the coding sequence of SEQ ID NO:75 shown in Figure 75.
Figure 77 shows a nucleotide sequence (SEQ ID NO:77) of a native sequence PRO9854 cDNA, wherein SEQ ID NO:77 is a clone designated herein as "DNA108771-2776" (UNQ3039).
Figure 78 shows the amino acid sequence (SEQ ID NO:78) derived from the coding sequence of SEQ ID NO:77 shown in Figure 77.
Figure 79 shows a nucleotide sequence (SEQ ID NO:79) of a native sequence PRO9862 cDNA, wherein SEQ ID NO:79 is a clone designated herein as "DNA125148-2782" (UNQ3046).
Figure 80 shows the amino acid sequence (SEQ ID NO:80) derived from the coding sequence of SEQ ID NO:79 shown in Figure 79.
Figure 81 shows a nucleotide sequence (SEQ ID NO:81) of a native sequence PRO10284 cDNA, wherein SEQ ID NO:81 is a clone designated herein as "DNA138039-2828" (UNQ3127).
Figure 82 shows the amino acid sequence (SEQ ID NO:82) derived from the coding sequence of SEQ ID NO:81 shown in Figure 81.

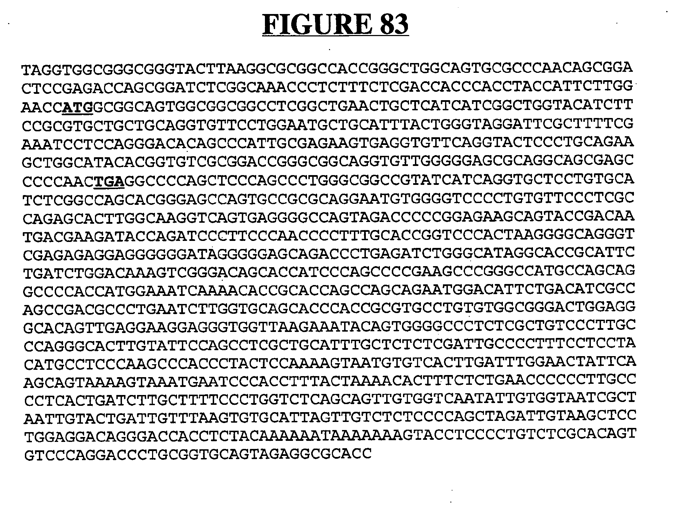

Figure 83 shows a nucleotide sequence (SEQ ID NO:83) ofanative sequence PRO37510 cDNA, wherein SEQ ID NO:83 is a clone designated herein as "DNA227047" (UNQ4430).

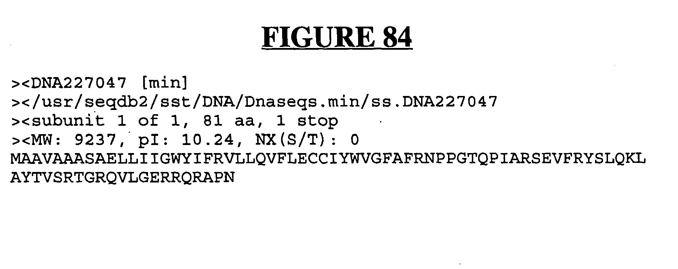

Figure 84 shows the amino acid sequence (SEQ ID NO:84) derived from the coding sequence of SEQ ID NO:83 shown in Figure 83.

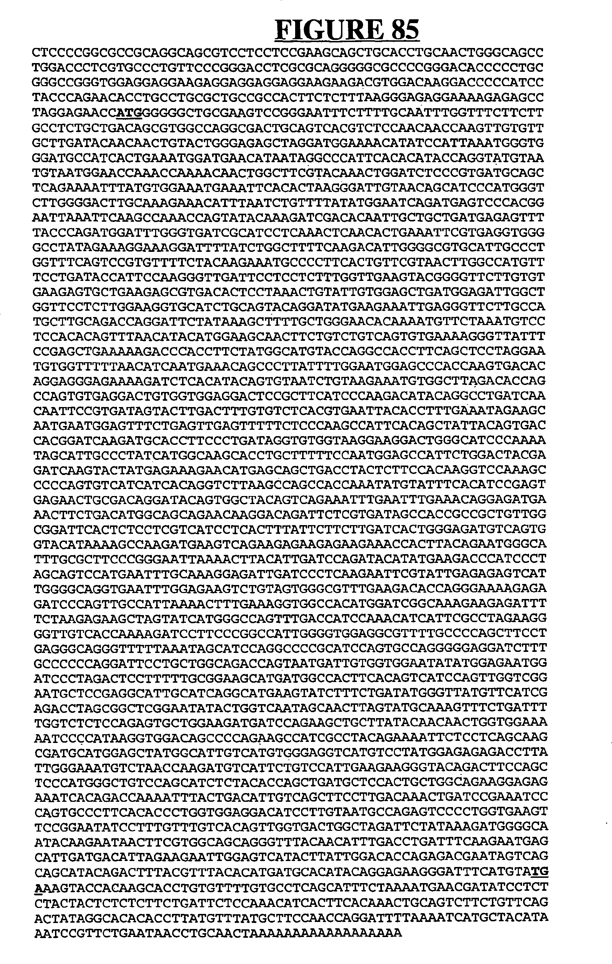

Figure 85 shows a nucleotide sequence (SEQ ID NO:85) of a native sequence PRO35444 cDNA, wherein SEQ ID NO:85 is a clone designated herein as "DNA222653" (UNQ6114).

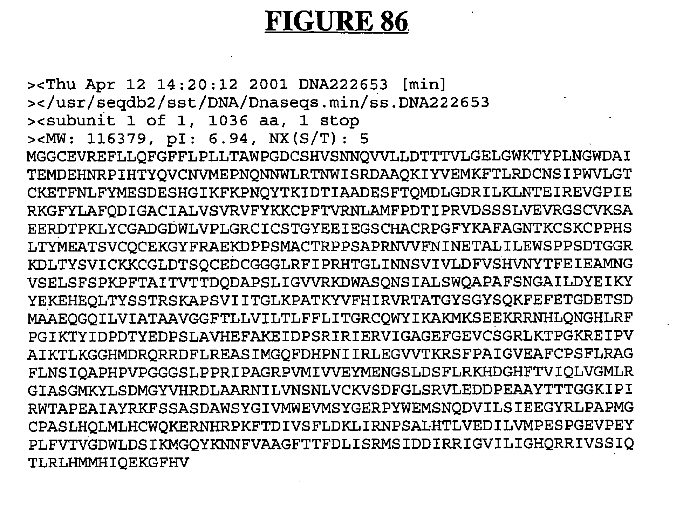

Figure 86 shows the amino acid sequence (SEQ ID NO:86) derived from the coding sequence of SEQ ID NO:85 shown in Figure 85.
Figure 87 shows a nucleotide sequence (SEQ ID NO:87) of a native sequence PRO20473 cDNA, wherein SEQ ID NO:87 is a clone designated herein as "DNA163134-2917" (UNQ6268).
Figure 88 shows the amino acid sequence (SEQ ID NO:88) derived from the coding sequence of SEQ ID NO:87 shown in Figure 87.
Figure 89 shows a nucleotide sequence (SEQ ID NO:89) of a native sequence PRO21054 cDNA, wherein SEQ ID NO:89 is a clone designated herein as "DNA143501-2922" (UNQ6349).
Figure 90 shows the amino acid sequence (SEQ ID NO:90) derived from the coding sequence of SEQ ID NO:89 shown in Figure 89.

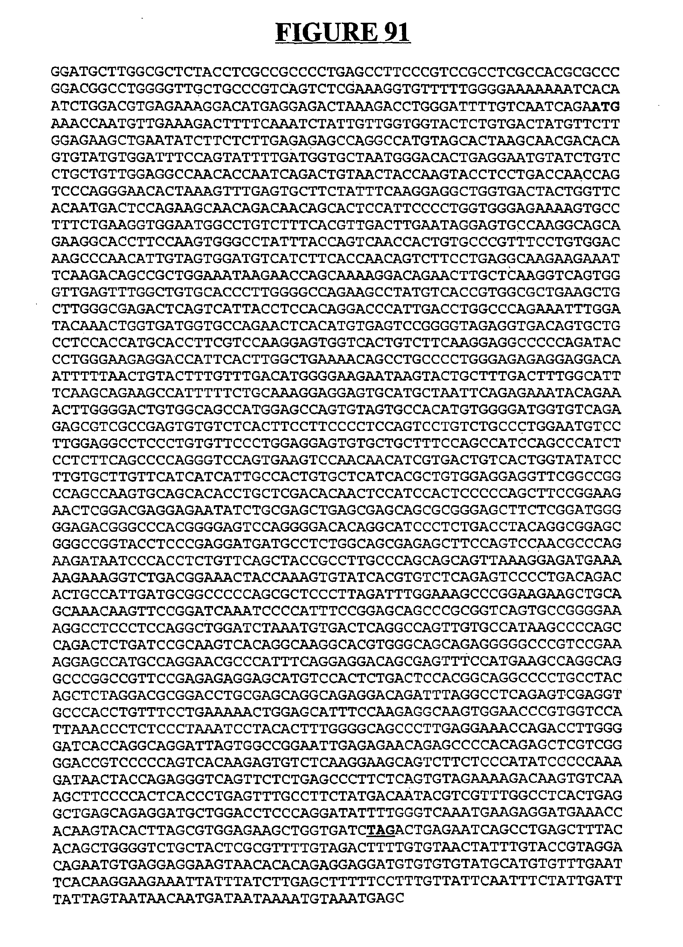

Figure 91 shows a nucleotide sequence (SEQ ID NO:91) of a native sequence PRO3 5246 cDNA, wherein SEQ ID NO:91 is a clone designated herein as "DNA129618" (UNQ3010).

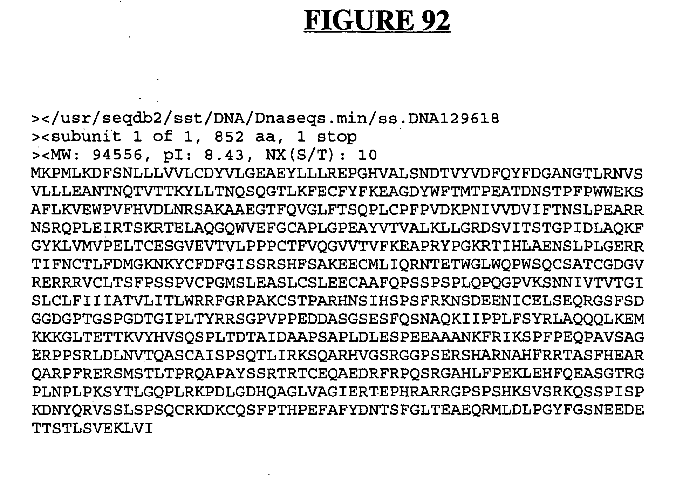

Figure 92 shows the amino acid sequence (SEQ ID NO:92) derived from the coding sequence of SEQ ID NO:91 shown in Figure 91.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide" comprises a polypeptide having the same amino acid sequence as the corresponding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide derived from nature. Such native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides can be isolated from nature or can be produced by recombinant or synthetic means. The term "native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide" specifically encompasses naturally-occurring truncated or secreted forms of the specific PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide (*e*.*g*., an extracellular domain sequence), naturally-occurring variant forms (*e*.*g*., alternatively spliced forms) and naturally-occurring allelic variants of the polypeptide. The invention provides native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides disclosed herein which are mature or full-length native sequence polypeptides comprising the full-length amino acids sequences shown in the accompanying figures. Start and stop codons are shown in bold font and underlined in the figures. However, while the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides.

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO375 10, PRO35444, PRO20473, PRO21054 or PRO35246 polypeptide "extracellular domain" or "ECD" refers to a form of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which is essentially free of the transmembrane and cytoplasmic domains. Ordinarily, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide ECD will have less than 1% of such transmembrane and/or cytoplasmic domains and preferably, will have less than 0.5% of such domains. It will be understood that anytransmembrane domains identified for the PRO188, PRO23 5, PRO266, PRO33 7, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.
"PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide variant" means a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, preferably an active PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide, as defmed herein having at least about 80% amino acid sequence identity with a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence lacking the signal peptide as disclosed herein, an extracellular domain of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, with or without the signal peptide, as disclosed herein or any other fragment of a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein (such as those encoded by a nucleic acid that represents only a portion of the complete coding sequence for a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide). Such PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide variants include, for instance, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides wherein one or more amino acid residues are added, or deleted, at the N- or C-terminus of the full-length native amino acid sequence. Ordinarily, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide variant will have or will have at least about 80% amino acid sequence identity, alternatively will have or will have at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence lacking the signal peptide as disclosed herein, an extracellular domain of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, with or without the signal peptide, as disclosed herein or any other specifically defmed fragment of a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein. Ordinarily, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polypeptides are or are at least about 10 amino acids in length, alternatively are or are at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polypeptides will have no more than one conservative amino acid substitution as compared to the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence, alternatively will have or will have no more than 2, 3, 4, 5, 6, 7, 8, 9, or 10 conservative amino acid substitution as compared to the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence.
"Percent (%) amino acid sequence identity" with respect to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequences identified herein is defmed as the percentage of amino acid residues in a candidate sequence that are identical with the amino acid residues in the specific PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence, after aligning the sequences and introducing gaps, if necessary, to achieve the maximum percent sequence identity, and not considering any conservative substitutions as part of the sequence identity. Alignment for purposes ofdetermining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U. S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues. Unless specifically stated otherwise, all % amino acid sequence identity values used herein are obtained as described in the immediately preceding paragraph using the ALIGN-2 computer program.
"PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polynucleotide" or "PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant nucleic acid sequence" means a nucleic acid molecule which encodes a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, preferably an active PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, as defined herein and which has at least about 80% nucleic acid sequence identity with a nucleotide acid sequence encoding a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein, a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence lacking the signal peptide as disclosed herein, an extracellular domain of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, with or without the signal peptide, as disclosed herein or any other fragment of a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein (such as those encoded by a nucleic acid that represents only a portion of the complete coding sequence for a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide). Ordinarily, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polynucleotide will have or will have at least about 80% nucleic acid sequence identity, alternatively will have or will have at least about 81 %, 82%, 83%, 84%, 85%, 86%, 87%, 88%, 89%, 90%, 91%, 92%, 93%, 94%, 95%, 96%, 97%, 98%, or 99% nucleic acid sequence identity with a nucleic acid sequence encoding a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein, a full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence lacking the signal peptide as disclosed herein, an extracellular domain of a PRO188, PRO235, PRO266, PRO33 7, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, with or without the signal sequence, as disclosed herein or any other fragment of a full-tength PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide sequence as disclosed herein. Variants do not encompass the native nucleotide sequence.

Ordinarily, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polynucleotides are or are at least about 5 nucleotides in length, alternatively are or are at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170,175, 180, 185, 190, 195, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.
"Percent (%) nucleic acid sequence identity" with respect to PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding nucleic acid sequences identified herein is defmed as the percentage of nucleotides in a candidate sequence that are identical with the nucleotides in the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acid sequence ofinterest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U. S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number ofnucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the PRO-DNA" nucleic acid molecule of interest is being compared, and "N", "L" and "V" each represent different hypothetical nucleotides. Unless specifically stated otherwise, all % nucleic acid sequence identity values used herein are obtained as described in the immediately preceding paragraph using the ALIGN-2 computer program.

The invention also provides PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polynucleotides which are nucleic acid molecules that encode a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and which are capable of hybridizing, preferably under stringent hybridization and wash conditions, to nucleotide sequences encoding a full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as disclosed herein. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polypeptides may be those that are encoded by a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant polynucleotide.

The term "full-length coding region" when used in reference to a nucleic acid encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide refers to the sequence of nucleotides which encode the full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide of the invention (which is often shown between start and stop codons, inclusive thereof, in the accompanying figures). The term "full-length coding region" when used in reference to an ATCC deposited nucleic acid refers to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding portion of the cDNA that is inserted into the vector deposited with the ATCC (which is often shown between start and stop codons, inclusive thereof, in the accompanying figures).
"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. The invention provides that the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide natural environment will not be present. Ordinarily, however, isolated polypeptide will be prepared by at least one purification step.

An "isolated" PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.
"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).
"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.
"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).
"Active" or "activity" for the purposes herein refers to form(s) of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which retain a biological and/or an immunological activity of native or naturally-occurring PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, wherein "biological" activity refers to a biological function (either inhibitory or stimulatory) caused by a native or naturally-occurring PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide other than the ability to induce the production of an antibody against an antigenic epitope possessed by a native or naturally-occurring PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867,PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and an "immunological" activity refers to the ability to induce the production of an antibody against an antigenic epitope possessed by a native or naturally-occurring PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

The term "antagonist" is used in the broadest sense [unless otherwise qualified], and includes any molecule that partially or fully blocks, inhibits, or neutralizes a biological activity of a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide disclosed herein. In a similar manner, the term "agonist" is used in the broadest sense [unless otherwise qualified] and includes any molecule that mimics a biological activity of a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide disclosed herein. Suitable agonist or antagonist molecules specifically include agonist or antagonist antibodies or antibody fragments, fragments or amino acid sequence variants ofnative PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides, peptides, antisense oligonucleotides, small organic molecules, etc. Methods for identifying agonists or antagonists of a PRO188, PRO235, PRO266, PRO3 3 7, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may comprise contacting a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with a candidate agonist or antagonist molecule and measuring a detectable change in one or more biological activities normally associated with the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.
"Treating" or "treatment" or "alleviation" refers to both therapeutic treatment and prophylactic or preventative measures, wherein the object is to prevent or slow down (lessen) the targeted pathologic condition or disorder. A subject in need of treatment may already have the disorder, or may be prone to have the disorder or may be in whom the disorder is to be prevented.
"Chronic" administration refers to administration of the agent(s) in a continuous mode as opposed to an acute mode, so as to maintain the initial therapeutic effect (activity) for an extended period oftime. "Intermittent" administration is treatment that is not consecutively done without interruption, but rather is cyclic in nature.
"Mammal" for purposes of treatment refers to any animal classified as a mammal, including humans, rodents such as rats or mice, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.
"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. Depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

An "effective amount" of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO3 5246 binding oligopeptide, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule or an agonist or antagonist thereof as disclosed herein is an amount sufficient to carry out a specifically stated purpose. An "effective amount" may be determined empirically and in a routine manner, in relation to the stated purpose.

The term "therapeutically effective amount" refers to an amount of an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide, a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule or other drug effective to "treat" a disease or disorder in a subject or mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. See the definition herein of "treating". To the extent the drug may prevent growth and/or kill existing cancer cells, it may be cytostatic and/or cytotoxic.

The phrases "cardiovascular, endothelial and angiogenic disorder", "cardiovascular, endothelial and angiogenic dysfunction", "cardiovascular, endothelial or angiogenic disorder" and "cardiovascular, endothelial or angiogenic dysfunction" are used interchangeably and refer in part to systemic disorders that affect vessels, such as diabetes mellitus, as well as diseases of the vessels themselves, such as of the arteries, capillaries, veins, and/or lymphatics. This would include indications that stimulate angiogenesis and/or cardiovascularization, and those that inhibit angiogenesis and/or cardiovascularization. Such disorders include, for example, arterial disease, such as atherosclerosis, hypertension, inflammatory vasculitides, Reynaud's disease and Reynaud's phenomenon, aneurysms, and arterial restenosis; venous and lymphatic disorders such as thrombophlebitis, lymphangitis, and lymphedema; and other vascular disorders such as peripheral vascular disease, cancer such as vascular tumors, e.g., hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma, tumor angiogenesis, trauma such as wounds, burns, and other injured tissue, implant fixation, scarring, ischemia reperfusion injury, rheumatoid arthritis, cerebrovascular disease, renal diseases such as acute renal failure, or osteoporosis. This would also include angina, myocardial infarctions such as acute myocardial infarctions, cardiac hypertrophy, and heart failure such as CHF.
"Hypertrophy", as used herein, is defined as an increase in mass of an organ or structure independent of natural growth that does not involve tumor formation. Hypertrophy of an organ or tissue is due either to an increase in the mass of the individual cells (true hypertrophy), or to an increase in the number of cells making up the tissue (hyperplasia), or both. Certain organs, such as the heart, lose the ability to divide shortly after birth. Accordingly, "cardiac hypertrophy" is defined as an increase in mass ofthe heart, which, in adults, is characterized by an increase in myocyte cell size and contractile protein content without concomitant cell division. The character ofthe stress responsible for inciting the hypertrophy, (e.g., increased preload, increased afterload, loss ofmyocytes, as in myocardial infarction, or primary depression of contractility), appears to play a critical role in determining the nature of the response. The early stage of cardiac hypertrophy is usually characterized morphologically by increases in the size of myofibrils and mitochondria, as well as by enlargement of mitochondria and nuclei. At this stage, while muscle cells are larger than normal, cellular organization is largely preserved. At a more advanced stage of cardiac hypertrophy, there are preferential increases in the size or number of specific organelles, such as mitochondria, and new contractile elements are added in localized areas ofthe cells, in an irregular manner. Cells subjected to long-standinghypertrophy showmore obvious disruptions in cellular organization, including markedly enlarged nuclei with highly lobulated membranes, which displace adjacent myofibrils and cause breakdown of normal Z-band registration. The phrase "cardiac hypertrophy" is used to include all stages of the progression of this condition, characterized by various degrees of structural damage of the heart muscle, regardless of the underlying cardiac disorder. Hence, the term also includes physiological conditions instrumental in the development of cardiac hypertrophy, such as elevated blood pressure, aortic stenosis, or myocardial infarction.
"Heart failure" refers to an abnormality of cardiac function where the heart does not pump blood at the rate needed for the requirements of metabolizing tissues. The heart failure can be caused by a number of factors, including ischemic, congenital, rheumatic, or idiopathic forms.
"Congestive heart failure" (CHF) is a progressive pathologic state where the heart is increasingly unable to supply adequate cardiac output (the volume of blood pumped by the heart over time) to deliver the oxygenated blood to peripheral tissues. As CHF progresses, structural and hemodynamic damages occur. While these damages have a variety of manifestations, one characteristic symptom is ventricular hypertrophy. CHF is a common end result of a number of various cardiac disorders.
"Myocardial infarction" generally results from atherosclerosis of the coronary arteries, often with superimposed coronary thrombosis. It may be divided into two major types: transmural infarcts, in which myocardial necrosis involves the full thickness ofthe ventricular wall, and subendocardial (nontransmural) infarcts, in which the necrosis involves the subendocardium, the intramural myocardium, or both, without extending all the way through the ventricular wall to the epicardium. Myocardial infarction is known to cause both a change in hemodynamic effects and an alteration in structure in the damaged and healthy zones of the heart. Thus, for example, myocardial infarction reduces the maximum cardiac output and the stroke volume of the heart. Also associated with myocardial infarction is a stimulation of the DNA synthesis occurring in the interstice as well as an increase in the formation of collagen in the areas of the heart not affected.

As a result of the increased stress or strain placed on the heart in prolonged hypertension due, for example, to the increased total peripheral resistance, cardiac hypertrophy has long been associated with "hypertension". A characteristic of the ventricle that becomes hypertrophic as a result of chronic pressure overload is an impaired diastolic performance. Fouad *et al*., J. Am. Coll. Cardiol., 4: 1500-1506 (1984); Smith *et al*., J. Am. Coll. Cardiol., 5: 869-874 (1985). A prolonged left ventricular relaxation has been detected in early essential hypertension, in spite of normal or supranormal systolic function. Hartford et al., Hypertension, 6: 329-338 (1984). However, there is no close parallelism between blood pressure levels and cardiac hypertrophy. Although improvement in left ventricular function in response to antihypertensive therapy has been reported in humans, patients variously treated with a diuretic (hydrochlorothiazide), a β-blocker (propranolol), or a calcium channel blocker (diltiazem), have shown reversal of left ventricular hypertrophy, without improvement in diastolic function. Inouye et al., Am. J. Cardiol., 53: 1583-7 (1984).

Another complex cardiac disease associated with cardiac hypertrophy is "hypertrophic cardiomyopathy". This condition is characterized by a great diversity of morphologic, functional, and clinical features (Maron et al., N. Engl. J. Med., 316: 780-789 (1987); Spirito et al., N. Engl. J. Med., 320: 749-755 (1989); Louie and Edwards, Prog. Cardiovasc. Dis., 36: 275-308 (1994); Wigle et al., Circulation, 92: 1680-1692 (1995)), the heterogeneity of which is accentuated by the fact that it afflicts patients of all ages. Spirito et al., N. Engl. J. Med., 336: 775-785 (1997). The causative factors of hypertrophic cardiomyopathy are also diverse and little understood. In general, mutations in genes encoding sarcomeric proteins are associated with hypertrophic cardiomyopathy. Recent data suggest that β-myosin heavy chain mutations may account for approximately 30 to 40 percent of cases of familial hypertrophic cardiomyopathy. Watkins et al., N. Engl. J. Med., 326: 1108-1114 (1992); Schwartz et al, Circulation, 91: 532-540 (1995); Marian and Roberts, Circulation, 92: 1336-1347 (1995); Thierfelder et al., Cell, 77: 701-712 (1994); Watkins et al., Nat. Gen., 11: 434-437 (1995). Besides β-myosin heavy chain, other locations of genetic mutations include cardiac troponin T, alpha topomyosin, cardiac myosin binding protein C, essential myosin light chain, and regulatory myosin light chain. *See*, Malik and Watkins, Curr. Opin. Cardiol., 12: 295-302 (1997).

Supravalvular "aortic stenosis" is an inherited vascular disorder characterized by narrowing of the ascending aorta, but other arteries, including the pulmonary arteries, may also be affected. Untreated aortic stenosis may lead to increased intracardiac pressure resulting in myocardial hypertrophy and eventually heart failure and death. The pathogenesis of this disorder is not fully understood, but hypertrophy and possibly hyperplasia of medial smooth muscle are prominent features of this disorder. It has been reported that molecular variants of the elastin gene are involved in the development and pathogenesis of aortic stenosis. U.S. Patent No. 5,650,282 issued July 22, 1997.
"Valvular regurgitation" occurs as a result of heart diseases resulting in disorders of the cardiac valves. Various diseases, like rheumatic fever, can cause the shrinking or pulling apart of the valve orifice, while other diseases may result in endocarditis, an inflammation of the endocardium or lining membrane of the atrioventricular orifices and operation of the heart. Defects such as the narrowing of the valve stenosis or the defective closing of the valve result in an accumulation of blood in the heart cavity or regurgitation of blood past the valve. If uncorrected, prolonged valvular stenosis or insufficiency may result in cardiac hypertrophy and associated damage to the heart muscle, which may eventually necessitate valve replacement.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, etc.

The term "T cell mediated disease" means a disease in which T cells directly or indirectly mediate or otherwise contribute to a morbidity in a mammal. The T cell mediated disease may be associated with cell mediated effects, lymphokine mediated effects, etc., and even effects associated with B cells if the B cells are stimulated, for example, by the lymphokines secreted by T cells.

Examples of immune-related and inflammatory diseases, some of which are immune or T cell mediated, include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonia, idiopathic pulmonary fibrosis and hypersensitivity pneumonitis, or transplantation associated diseases including graft rejection and graft -versus-host-disease. Infectious diseases including viral diseases such as AIDS (HIV infection), hepatitis A, B, C, D, and E, herpes, etc., bacterial infections, fungal infections, protozoal infections and parasitic infections.

An "autoimmune disease" herein is a disease or disorder arising from and directed against an individual's own tissues or organs or a co-segregate or manifestation thereofor resulting condition therefrom. In many of these autoimmune and inflammatory disorders, a number of clinical and laboratory markers may exist, including, but not limited to, hypergammaglobulinemia, high levels of autoantibodies, antigen-antibody complex deposits in tissues, benefit from corticosteroid or immunosuppressive treatments, and lymphoid cell aggregates in affected tissues. Without being limited to any one theory regarding B-cell mediated autoimmune disease, it is believed that B cells demonstrate a pathogenic effect in human autoimmune diseases through a multitude of mechanistic pathways, including autoantibody production, immune complex formation, dendritic and T-cell activation, cytokine synthesis, direct chemokine release, and providing a nidus for ectopic neo-lymphogenesis. Each of these pathways may participate to different degrees in the pathology of autoimmune diseases.
"Autoimmune disease" can be an organ-specific disease (i.e., the immune response is specifically directed against an organ system such as the endocrine system, the hematopoietic system, the skin, the cardiopulmonary system, the gastrointestinal and liver systems, the renal system, the thyroid, the ears, the neuromuscular system, the central nervous system, etc.) or a systemic disease which can affect multiple organ systems (for example, systemic lupus erythematosus (SLE), rheumatoid arthritis, polymyositis, etc.). Preferred such diseases include autoimmune rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), autoimmune gastrointestinal and liver disorders (such as, for example, inflammatory bowel diseases (e.g., ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g., Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.
Specific examples of other autoimmune diseases as defmed herein, which in some cases encompass those listed above, include, but are not limited to, arthritis (acute and chronic, rheumatoid arthritis including juvenile-onset rheumatoid arthritis and stages such as rheumatoid synovitis, gout or gouty arthritis, acute immunological arthritis, chronic inflammatory arthritis, degenerative arthritis, type II collagen-induced arthritis, infectious arthritis, Lyme arthritis, proliferative arthritis, psoriatic arthritis, Still's disease, vertebral arthritis, osteoarthritis, arthritis chronica progrediente, arthritis deformans, polyarthritis chronica primaria, reactive arthritis, menopausal arthritis, estrogen-depletion arthritis, and ankylosing spondylitis/rheumatoid spondylitis), autoimmune lymphoproliferative disease, inflammatory hyperproliferative skin diseases, psoriasis such as plaque psoriasis, gutatte psoriasis, pustular psoriasis, and psoriasis of the nails, atopy including atopic diseases such as hay fever and Job's syndrome, dermatitis including contact dermatitis, chronic contact dermatitis, exfoliative dermatitis, allergic dermatitis, allergic contact dermatitis, hives, dermatitis herpetiformis, nummular dermatitis, seborrheic dermatitis, non-specific dermatitis, primary irritant contact dermatitis, and atopic dermatitis, x-linked hyper IgM syndrome, allergic intraocular inflammatory diseases, urticaria such as chronic allergic urticaria and chronic idiopathic urticaria, including chronic autoimmune urticaria, myositis, polymyositis/dermatomyositis, juvenile dermatomyositis, toxic epidermal necrolysis, scleroderma (including systemic scleroderma), sclerosis such as systemic sclerosis, multiple sclerosis (MS) such as spino-optical MS, primary progressive MS (PPMS), and relapsing remitting MS (RRMS), progressive systemic sclerosis, atherosclerosis, arteriosclerosis, sclerosis disseminata, ataxic sclerosis, neuromyelitis optica (NMO), inflammatory bowel disease (IBD) (for example, Crohn's disease, autoimmune-mediated gastrointestinal diseases, gastrointestinal inflammation, colitis such as ulcerative colitis, colitis ulcerosa, microscopic colitis, collagenous colitis, colitis polyposa, necrotizing enterocolitis, and transmural colitis, and autoimmune inflammatory bowel disease), bowel inflammation, pyoderma gangrenosum, erythema nodosum, primary sclerosing cholangitis, respiratory distress syndrome, including adult or acute respiratory distress syndrome (ARDS), meningitis, inflammation of all or part of the uvea, iritis, choroiditis, an autoimmune hematological disorder, graft-versus-host disease, angioedema such as hereditary angioedema, cranial nerve damage as in meningitis, herpes gestationis, pemphigoid gestationis, pruritis scroti, autoimmune premature ovarian failure, sudden hearing loss due to an autoimmune condition, IgE-mediated diseases such as anaphylaxis and allergic and atopic rhinitis, encephalitis such as Rasmussen's encephalitis and limbic and/or brainstem encephalitis, uveitis, such as anterior uveitis, acute anterior uveitis, granulomatous uveitis, nongranulomatous uveitis, phacoantigenic uveitis, posterior uveitis, or autoimmune uveitis, glomerulonephritis (GN) with and without nephrotic syndrome such as chronic or acute glomerulonephritis such as primary GN, immune-mediated GN, membranous GN (membranous nephropathy), idiopathic membranous GN or idiopathic membranous nephropathy, membrano- or membranous proliferative GN (MPGN), including Type I and Type II, and rapidly progressive GN (RPGN), proliferative nephritis, autoimmune polyglandular endocrine failure, balanitis including balanitis circumscripta plasmacellularis, balanoposthitis, erythema annulare centrifugum, erythema dyschromicumperstans, eythema multiform, granuloma annulare, lichen nitidus, lichen sclerosus et atrophicus, lichen simplex chronicus, lichen spinulosus, lichen planus, lamellar ichthyosis, epidermolytic hyperkeratosis, premalignant keratosis, pyoderma gangrenosum, allergic conditions and responses, food allergies, drug allergies, insect allergies, rare allergic disorders such as mastocytosis, allergic reaction, eczema including allergic or atopic eczema, asteatotic eczema, dyshidrotic eczema, and vesicular palmoplantar eczema, asthma such as asthma bronchiale, bronchial asthma, and auto-immune asthma, conditions involving infiltration ofT cells and chronic inflammatory responses, immune reactions against foreign antigens such as fetal A-B-O blood groups during pregnancy, chronic pulmonary inflammatory disease, autoimmune myocarditis, leukocyte adhesion deficiency, lupus, including lupus nephritis, lupus cerebritis, pediatric lupus, non-renal lupus, extra-renal lupus, discoid lupus and discoid lupus erythematosus, alopecia lupus, SLE, such as cutaneous SLE or subacute cutaneous SLE, neonatal lupus syndrome (NLE), and lupus erythematosus disseminatus, juvenile onset (Type I) diabetes mellitus, including pediatric IDDM, adult onset diabetes mellitus (Type II diabetes), autoimmune diabetes, idiopathic diabetes insipidus, diabetic retinopathy, diabetic nephropathy, diabetic colitis, diabetic large-artery disorder, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitides, including vasculitis, large-vessel vasculitis (including polymyalgia rheumatica and giant-cell (Takayasu's) arteritis), medium-vessel vasculitis (including Kawasaki's disease and polyarteritis nodosa/periarteritis nodosa), microscopic polyarteritis, immunovasculitis, CNS vasculitis, cutaneous vasculitis, hypersensitivity vasculitis, necrotizing vasculitis such as systemic necrotizing vasculitis, and ANCA-associated vasculitis, such as Churg-Strauss vasculitis or syndrome (CSS) and ANCA-associated small-vessel vasculitis, temporal arteritis, aplastic anemia, autoimmune aplastic anemia, Coombs positive anemia, Diamond Blackfan anemia, hemolytic anemia or immune hemolytic anemia including autoimmune hemolytic anemia (AIHA), pernicious anemia (anemia perniciosa), Addison's disease, pure red cell anemia or aplasia (PRCA), Factor VIII deficiency, hemophilia A, autoimmune neutropenia(s), cytopenias such as pancytopenia, leukopenia, diseases involving leukocyte diapedesis, CNS inflammatory disorders, Alzheimer's disease, Parkinson's disease, multiple organ injury syndrome such as those secondary to septicemia, trauma or hemorrhage, antigen-antibody complex- mediated diseases, anti-glomerular basement membrane disease, anti-phospholipid antibody syndrome, motoneuritis, allergic neuritis, ²Behçet's disease/syndrome, Castleman's syndrome, Goodpasture's syndrome, Reynaud's syndrome, Sjögren's syndrome, Stevens-Johnson syndrome, pemphigoid such as pemphigoid bullous and skin pemphigoid, pemphigus (including pemphigus vulgaris, pemphigus foliaceus, pemphigus mucus-membrane pemphigoid, and pemphigus erythematosus), autoimmune polyendocrinopathies, Reiter's disease or syndrome, thermal injury due to an autoimmune condition, preeclampsia, an immune complex disorder such as immune complex nephritis, antibody-mediated nephritis, neuroinflammatory disorders, polyneuropathies, chronic neuropathy such as IgM polyneuropathies or IgM-mediated neuropathy, thrombocytopenia (as developed by myocardial infarction patients, for example), including thrombotic thrombocytopenic purpura (TTP), post-transfusion purpura (PTP), heparin-induced thrombocytopenia, and autoimmune or immune-mediated thrombocytopenia including, for example, idiopathic thrombocytopenic purpura (ITP) including chronic or acute ITP, scleritis such as idiopathic cerato-scleritis, episcleritis, autoimmune disease of the testis and ovary including autoimmune orchitis and oophoritis, primary hypothyroidism, hypoparathyroidism, autoimmune endocrine diseases including thyroiditis such as autoimmune thyroiditis, Hashimoto's disease, chronic thyroiditis (Hashimoto's thyroiditis), or subacute thyroiditis, autoimmune thyroid disease, idiopathic hypothyroidism, Grave's disease, polyglandular syndromes such as autoimmune polyglandular syndromes, for example, type I (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis or encephalomyelitis allergica and experimental allergic encephalomyelitis (EAE), myasthenia gravis such as thymoma-associated myasthenia gravis, cerebellar degeneration, neuromyotonia, opsoclonus or opsoclonus myoclonus syndrome (OMS), and sensory neuropathy, multifocal motor neuropathy, Sheehan's syndrome, autoimmune hepatitis, chronic hepatitis, lupoid hepatitis, giant-cell hepatitis, chronic active hepatitis or autoimmune chronic active hepatitis, pneumonitis such as lymphoid interstitial pneumonitis (LIP), bronchiolitis obliterans (non-transplant) vs NSIP, Guillain-Barré syndrome, Berger's disease (IgA nephropathy), idiopathic IgA nephropathy, linear IgAdermatosis, acute febrile neutrophilic dermatosis, subcorneal pustular dermatosis, transient acantholytic dermatosis, cirrhosis such as primary biliary cirrhosis and pneumonocirrhosis, autoimmune enteropathy syndrome, Celiac or Coeliac disease, celiac sprue (gluten enteropathy), refractory sprue, idiopathic sprue, cryoglobulinemia such as mixed cryoglobulinemia, amylotrophic lateral sclerosis (ALS; Lou Gehrig's disease), coronary artery disease, autoimmune ear disease such as autoimmune inner ear disease (AIED), autoimmune hearing loss, polychondritis such as refractory or relapsed or relapsing polychondritis, pulmonary alveolar proteinosis, Cogan's syndrome/nonsyphilitic interstitial keratitis, Bell's palsy, Sweet's disease/syndrome, rosacea autoimmune, zoster-associated pain, amyloidosis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal or segmental or focal segmental glomerulosclerosis (FSGS), endocrine ophthalmopathy, uveoretinitis, chorioretinitis, autoimmune hepatological disorder, fibromyalgia, multiple endocrine failure, Schmidt's syndrome, adrenalitis, gastric atrophy, presenile dementia, demyelinating diseases such as autoimmune demyelinating diseases and chronic inflammatory demyelinating polyneuropathy, Dressler's syndrome, alopecia areata, alopecia totalis, CREST syndrome (calcinosis, Raynaud's phenomenon, esophageal dysmotility, sclerodactyly, and telangiectasia), male and female autoimmune infertility, e.g., due to anti-spermatozoan antibodies, mixed connective tissue disease, Chagas' disease, rheumatic fever, recurrent abortion, farmer's lung, erythema multiforme, post-cardiotomy syndrome, Cushing's syndrome, bird-fancier's lung, allergic granulomatous angiitis, benign lymphocytic angiitis, Alport's syndrome, alveolitis such as allergic alveolitis and fibrosing alveolitis, interstitial lung disease, transfusion reaction, leprosy, malaria, parasitic diseases such as leishmaniasis, kypanosomiasis, schistosomiasis, ascariasis, aspergillosis, Sampter's syndrome, Caplan's syndrome, dengue, endocarditis, endomyocardial fibrosis, diffuse interstitial pulmonary fibrosis, interstitial lung fibrosis, fibrosing mediastinitis, pulmonary fibrosis, idiopathic pulmonary fibrosis, cystic fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, iridocyclitis (acute or chronic), or Fuch's cyclitis, Henoch-Schonlein purpura, human immunodeficiency virus (HIV) infection, SCID, acquired immune deficiency syndrome (AIDS), echovirus infection, sepsis (systemic inflammatory response syndrome (SIRS)), endotoxemia, pancreatitis, thyroxicosis, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, chorioiditis, giant-cell polymyalgia, chronic hypersensitivity pneumonitis, conjunctivitis, such as vernal catarrh, keratoconjunctivitis sicca, and epidemic keratoconjunctivitis, idiopathic nephritic syndrome, minimal change nephropathy, benign familial and ischemia-reperfusion injury, transplant organ reperfusion, retinal autoimmunity, joint inflammation, bronchitis, chronic obstructive airway/pulmonary disease, silicosis, aphthae, aphthous stomatitis, arteriosclerotic disorders (cerebral vascular insufficiency) such as arteriosclerotic encephalopathy and arteriosclerotic retinopathy, aspermiogenese, autoimmune hemolysis, Boeck's disease, cryoglobulinemia, Dupuytren's contracture, endophthalmia phacoanaphylactica, enteritis allergica, erythema nodosum leprosum, idiopathic facial paralysis, chronic fatigue syndrome, febris rheumatica, Hamman-Rich's disease, sensoneural hearing loss, haemoglobinuria paroxysmatica, hypogonadism, ileitis regionalis, leucopenia, mononucleosis infectiosa, traverse myelitis, primary idiopathic myxedema, nephrosis, ophthalmia symphatica, orchitis granulomatosa, pancreatitis, polyradiculitis acuta, pyoderma gangrenosum, Quervain's thyreoiditis, acquired spenic atrophy, non-malignant thymoma, lymphofollicular thymitis, vitiligo, toxic-shock syndrome, food poisoning, conditions involving infiltration ofT cells, leukocyte-adhesion deficiency, immune responses associated with acute and delayed hypersensitivity mediated by cytokines and T-lymphocytes, diseases involving leukocyte diapedesis, multiple organ injury syndrome, antigen-antibody complex-mediated diseases, antiglomerular basement membrane disease, autoimmune polyendocrinopathies, oophoritis, primary myxedema, autoimmune atrophic gastritis, sympathetic ophthalmia, rheumatic diseases, mixed connective tissue disease, nephrotic syndrome, insulitis, polyendocrine failure, autoimmune polyglandular syndromes, including polyglandular syndrome type I, adult-onset idiopathic hypoparathyroidism (AOIH), cardiomyopathy such as dilated cardiomyopathy, epidermolisis bullosa acquisita (EBA), hemochromatosis, myocarditis, nephrotic syndrome, primary sclerosing cholangitis, purulent or nonpurulent sinusitis, acute or chronic sinusitis, ethmoid, frontal, maxillary, or sphenoid sinusitis, allergic sinusitis, an eosinophil-related disorder such as eosinophilia, pulmonary infiltration eosinophilia, eosinophilia-myalgia syndrome, Loffler's syndrome, chronic eosinophilic pneumonia, tropical pulmonary eosinophilia, bronchopneumonic aspergillosis, aspergilloma, or granulomas containing eosinophils, anaphylaxis, spondyloarthropathies, seronegative spondyloarthritides, polyendocrine autoimmune disease, sclerosing cholangitis, sclera, episclera, chronic mucocutaneous candidiasis, Bruton's syndrome, transient hypogammaglobulinemia of infancy, Wiskott-Aldrich syndrome, ataxia telangiectasia syndrome, angiectasis, autoimmune disorders associated with collagen disease, rheumatism such as chronic arthrorheumatism, lymphadenitis, reduction in blood pressure response, vascular dysfunction, tissue injury, cardiovascular ischemia, hyperalgesia, renal ischemia, cerebral ischemia, and disease accompanying vascularization, allergic hypersensitivity disorders, glomerulonephritides, reperfusion injury, ischemic re-perfusion disorder, reperfusion injury of myocardial or other tissues, lymphomatous tracheobronchitis, inflammatory dermatoses, dermatoses with acute inflammatory components, multiple organ failure, bullous diseases, renal cortical necrosis, acute purulent meningitis or other central nervous system inflammatory disorders, ocular and orbital inflammatory disorders, granulocyte transfusion-associated syndromes, cytokine-induced toxicity, narcolepsy, acute serious inflammation, chronic intractable inflammation, pyelitis, endarterial hyperplasia, peptic ulcer, valvulitis, and endometriosis.

The phrase "anxiety related disorders" refers to disorders of anxiety, mood, and substance abuse, including but not limited to: depression, generalized anxiety disorders, attention deficit disorder, sleep disorder, hyperactivity disorder, obsessive compulsive disorder, schizophrenia, cognitive disorders, hyperalgesia and sensory disorders. Such disorders include the mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, social anxiety, autism, specific phobia, substance-induced anxiety disorder, acute alcohol withdrawal, obsessive compulsive disorder, agoraphobia, monopolar disorders, bipolar disorder I or II, bipolar disorder not otherwise specified, cyclothymic disorder, depressive disorder, major depressive disorder, mood disorder, substance-induced mood disorder, enhancement of cognitive function, loss of cognitive function associated with but not limited to Alzheimer's disease, stroke, or traumatic injury to the brain, seizures resulting from disease or injury including but not limited to epilepsy, learning disorders/disabilities, cerebral palsy. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

The term "lipid metabolic disorder" refers to abnormal clinical chemistry levels of cholesterol and triglycerides, wherein elevated levels of these lipids is an indication for atherosclerosis. Additionally, abnormal serum lipid levels may be an indication of various cardiovascular diseases including hypertension, stroke, coronary artery diseases, diabetes and/or obesity.

The phrase "eye abnormality" refers to such potential disorders of the eye as they may be related to atherosclerosis or various ophthalmological abnormalities. Such disorders include but are not limited to the following: retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstrom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis. Cataracts are also considered an eye abnormality and are associated with such systemic diseases as: Human Down's syndrome, Hallerman-Streiff syndrome, Lowe syndrome, galactosemia, Marfan syndrome, Trismoy 13-15 condition, Alport syndrome, myotonic dystrophy, Fabry disease, hypothroidisms, or Conradi syndrome. Other ocular developmental anomalies include: Aniridia, anterior segment and dysgenesis syndrome. Cataracts may also occur as a result of an intraocular infection or inflammation (uveitis).

A "growth inhibitory amount" of an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule is an amount capable of inhibiting the growth of a cell, especially tumor, e.g., cancer cell, either *in vitro* or *in vivo.* A "growth inhibitory amount" of an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338,PRO1378,PRO1415,PRO1867,PRO1890,PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule for purposes of inhibiting neoplastic cell growth may be determined empirically and in a routine manner.

A "cytotoxic amount" of an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule is an amount capable of causing the destruction of a cell, especially tumor, e.g., cancer cell, either *in vitro* or *in vivo*. A "cytotoxic amount" of an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO3 5246 antibody, PRO188, PRO23 5, PRO266, PRO337, PRO3 61, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule for purposes of inhibiting neoplastic cell growth may be determined empirically and in a routine manner.

The term "antibody" is used in the broadest sense and specifically covers, for example, single anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody monoclonal antibodies (including agonist, antagonist, and neutralizing antibodies), anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody compositions with polyepitopic specificity, polyclonal antibodies, single chain anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies, and fragments of anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies (see below) as long as they exhibit the desired biological or immunological activity. The term "immunoglobulin" (Ig) is used interchangeable with antibody herein.

An "isolated antibody" is one which has been identified and separated and/orrecovered from a component ofits natural environment. Contaminant components of its natural environment are materials which would interfere with diagnostic or therapeutic uses for the antibody, and may include enzymes, hormones, and other proteinaceous or nonproteinaceous solutes. The invention provides that the antibody will be purified (1) to greater than 95% by weight of antibody as determined by the Lowry method, and most preferably more than 99% by weight, (2) to a degree sufficient to obtain at least 15 residues ofN-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The basic 4-chain antibody unit is a heterotetrameric glycoprotein composed of two identical light (L) chains and two identical heavy (H) chains (an IgM antibody consists of 5 of the basic heterotetramer unit along with an additional polypeptide called J chain, and therefore contain 10 antigen binding sites, while secreted IgA antibodies can polymerize to form polyvalent assemblages comprising 2-5 of the basic 4-chain units along with J chain). In the case of IgGs, the 4-chain unit is generally about 150,000 daltons. Each L chain is linked to a H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H} 1). Particular amino acid residues are believed to form an interface between the light chain and heavy chain variable domains. The pairing of a V_{H} and V_{L} together forms a single antigen-binding site. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa and lambda, based on the amino acid sequences of their constant domains. Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated α, δ, ε, γ, and µ, respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The term "variable" refers to the fact that certain segments of the variable domains differ extensively in sequence among antibodies. The V domain mediates antigen binding and define specificity ofa particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β -sheet configuration, connected by three hypervariable regions, which form loops connecting, and in some cases forming part of, the β -sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the V_{L}, and around about 1-35 (H1), 50-65 (H2) and 95-102 (H3) in the V_{H}; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the V_{L}, and 26-32 (H1), 53-55 (H2) and 96-101 (H3) in the V_{H}; Chothia and Lesk J. Mol. Biol. 196:901-917 (1987)).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, e.g., U. S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

The monoclonal antibodies herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc), and human constant region sequences.

An "intact" antibody is one which comprises an antigen-binding site as well as a C_{L} and at least heavy chain constant domains, C_{H} 1, C_{H} 2 and C_{H} 3. The constant domains may be native sequence constant domains (e.g. human native sequence constant domains) or amino acid sequence variant thereof. Preferably, the intact antibody has one or more effector functions.
"Antibody fragments" comprise a portion ofan intact antibody, preferably the antigen binding or variable region of the intact antibody. Examples of antibody fragments include Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H} 1). Each Fab fragment is monovalent with respect to antigen binding, i.e., it has a single antigen-binding site. Pepsin treatment of an antibody yields a single large F(ab')₂ fragment which roughly corresponds to two disulfide linked Fab fragments having divalent antigen-binding activity and is still capable of cross-linking antigen. Fab' fragments differ from Fab fragments by having additional few residues at the carboxy terminus of the C_{H} 1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, which region is also the part recognized by Fc receptors (FcR) found on certain types of cells.
"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.
"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).
"Humanized" forms ofnon-human (e.g., rodent) antibodies are chimeric antibodies that contain minimal sequence derived from the non-human antibody. For the most part, humanized antibodies are human immunoglobulins (recipient antibody) in which residues from a hypervariable region of the recipient are replaced by residues from a hypervariable region of a non-human species (donor antibody) such as mouse, rat, rabbit or non-human primate having the desired antibody specificity, affinity, and capability. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "species-dependent antibody," e.g., a mammalian anti-human IgE antibody, is an antibody which has a stronger binding affinity for an antigen from a first mammalian species than it has for a homologue of that antigen from a second mammalian species. Normally, the species-dependent antibody "bind specifically" to a human antigen (i.e., has a binding affinity (Kd) value of no more than about 1 x 10⁻⁷ M, preferably no more than about 1 x 10⁻⁸ and most preferably no more than about 1 x 10⁻⁹ M) but has a binding affinity for a homologue of the antigen from a second non-human mammalian species which is at least about 50 fold, or at least about 500 fold, or at least about 1000 fold, weaker than its binding affinity for the human antigen. The species-dependent antibody can be of any of the various types of antibodies as defined above, but preferably is a humanized or human antibody.

A "PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptide" is an oligopeptide that binds, preferably specifically, to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as described herein. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptides may be chemically synthesized using known oligopeptide synthesis methodology or may be prepared and purified using recombinant technology. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptides usually are or are at least about 5 amino acids in length, alternatively are or are at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as described herein. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding oligopeptides may be identified without undue experimentation using well known techniques. In this regard, it is noted that techniques for screening oligopeptide libraries for oligopeptides that are capable of specifically binding to a polypeptide target are well known in the art (see, e.g., U.S. Patent Nos. 5,556,762, 5,750,373, 4,708,871, 4,833,092,5,223,409,5,403,484,5,571,689,5,663,143; PCT PublicationNos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J.Immunol., 140:611-616 (1998), Cwirla, S.E. et al. (1990) Proc. Natl. Acad. Sci. USA, 87:6378; Lowman, H.B. etal. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

A "PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO343 8, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecule" is an organic molecule other than an oligopeptide or antibody as defined herein that binds, preferably specifically, to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as described herein. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecules may be identified and chemically synthesized using known methodology (see, e.g., PCT Publication Nos. WO00/00823 and WO00/39585). PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding organic molecules are usually less than about 2000 daltons in size, alternatively less than about 1500, 750, 500, 250 or 200 daltons in size, wherein such organic molecules that are capable of binding, preferably specifically, to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as described herein may be identified without undue experimentation using well known techniques. In this regard, it is noted that techniques for screening organic molecule libraries for molecules that are capable of binding to a polypeptide target are well known in the art (see, e.g., PCT Publication Nos. WO00/00823 and WO00/39585).

An antibody, oligopeptide or other organic molecule "which binds" an antigen of interest, e.g. a tumor-associated polypeptide antigen target, is one that binds the antigen with sufficient affinity such that the antibody, oligopeptide or other organic molecule is preferably useful as a diagnostic and/or therapeutic agent in targeting a cell or tissue expressing the antigen, and does not significantly cross-react with other proteins. The extent of binding of the antibody, oligopeptide or other organic molecule to a "non-target" protein will be less than about 10% of the binding of the antibody, oligopeptide or other organic molecule to its particular target protein as determined by fluorescence activated cell sorting (FACS) analysis or radioimmunoprecipitation (RIA). With regard to the binding of an antibody, oligopeptide or other organic molecule to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target, for example, an excess of non-labeled target. In this case, specific binding is indicated if the binding of the labeled target to a probe is competitively inhibited by excess unlabeled target. The term "specific binding" or "specifically binds to" or is "specific for" a particular polypeptide or an epitope on a particular polypeptide target as used herein can be exhibited, for example, by a molecule having a Kd for the target of at least about 10⁻⁴ M, alternatively at least about 10⁻⁵ M, alternatively at least about 10⁻⁶ M, alternatively at least about 10⁻⁷ M, alternatively at least about 10⁻⁸ M, alternatively at least about 10⁻⁹ M, alternatively at least about 10⁻¹⁰ M, alternatively at least about 10⁻¹¹ M, alternatively at least about 10⁻¹² M, or greater. The term "specific binding" refers to binding where a molecule binds to a particular polypeptide or epitope on a particular polypeptide without substantially binding to any other polypeptide or polypeptide epitope.

An antibody, oligopeptide or other organic molecule that "inhibits the growth of tumor cells expressing a "PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246" or a "growth inhibitory" antibody, oligopeptide or other organic molecule is one which results in measurable growth inhibition of cancer cells expressing or overexpressing the appropriate PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be a transmembrane polypeptide expressed on the surface of a cancer cell or may be a polypeptide that is produced and secreted by a cancer cell. Preferred growth inhibitory anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies, oligopeptides or organic molecules inhibit growth of PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-expressing tumor cells by or by greater than 20%, preferably from about 20% to about 50%, and even more preferably, by or by greater than 50% (e.g., from about 50% to about 100%) as compared to the appropriate control, the control typically being tumor cells not treated with the antibody, oligopeptide or other organic molecule being tested. Growth inhibition can be measured at an antibody concentration of about 0.1 to 30 µg/ml or about 0.5 nM to 200 nM in cell culture, where the growth inhibition is determined 1-10 days after exposure of the tumor cells to the antibody. Growth inhibition of tumor cells *in vivo* can be determined in various ways. The antibody is growth inhibitory *in vivo* if administration of the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody at about 1 µg/kg to about 100 mg/kg body weight results in reduction in tumor size or tumor cell proliferation within about 5 days to 3 months from the first administration of the antibody, preferably within about 5 to 30 days.

An antibody, oligopeptide or other organic molecule which "induces apoptosis" is one which induces programmed cell death as determined by binding ofannexin V, fragmentation of DNA, cell shrinkage, dilation of endoplasmic reticulum, cell fragmentation, and/or formation of membrane vesicles (called apoptotic bodies). The cell is usually one which overexpresses a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Preferably the cell is a tumor cell, e.g., a prostate, breast, ovarian, stomach, endometrial, lung, kidney, colon, bladder cell. Various methods are available for evaluating the cellular events associated with apoptosis. For example, phosphatidyl serine (PS) translocation can be measured by annexin binding; DNA fragmentation can be evaluated through DNA laddering; and nuclear/chromatin condensation along with DNA fragmentation can be evaluated by any increase in hypodiploid cells. Preferably, the antibody, oligopeptide or other organic molecule which induces apoptosis is one which results in or in about 2 to 50 fold, preferably in or in about 5 to 50 fold, and most preferably in or in about 10 to 50 fold, induction of annexin binding relative to untreated cell in an annexin binding assay.

Antibody "effector functions" refer to those biological activities attributable to the Fc region (a native sequence Fc region or amino acid sequence variant Fc region) of an antibody, and vary with the antibody isotype. Examples of antibody effector functions include: C 1 q binding and complement dependent cytotoxicity; Fc receptor binding; antibody-dependent cell-mediated cytotoxicity (ADCC); phagocytosis; down regulation of cell surface receptors (e.g., B cell receptor); and B cell activation.
"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo*, e.g., in a animal model such as that disclosed in Clynes et al.Proc. Natl. Acad. Sci. U.S.A. 95:652-656 (1998).
"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif(ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol. 9:457-492 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).
"Human effector cells" are leukocytes which express one or more FcRs and perform effector functions. Preferably, the cells express at least FcγRIII and perform ADCC effector function. Examples ofhuman leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, e.g., from blood.
"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass) which are bound to their cognate antigen. To assess complement activation, a CDC assay, e.g., as described in Gazzano-Santoro et al., J. Immunol. Methods 202:163 (1996), may be performed.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD). Preferably, the cancer comprises a tumor that expresses an IGF receptor, more preferably breast cancer, lung cancer, colorectal cancer, or prostate cancer, and most preferably breast or prostate cancer.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and caticbeamicin omegaI1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; Ionidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE^{™} Cremopbor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6- thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON. toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; and pharmaceutically acceptable salts, acids or derivatives of any of the above.

The terms "cell proliferative disorder" and "proliferative disorder" refer to disorders that are associated with some degree of abnormal cell proliferation. In one aspect of the invention, the cell proliferative disorder is cancer.
"Tumor", as used herein, refers to all neoplastic cell growth and proliferation, whether malignant or benign, and all pre-cancerous and cancerous cells and tissues.

An antibody, oligopeptide or other organic molecule which "induces cell death" is one which causes a viable cell to become nonviable. The cell is one which expresses a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, preferably a cell that overexpresses a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as compared to a normal cell of the same tissue type. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be a transmembrane polypeptide expressed on the surface of a cancer cell or may be a polypeptide that is produced and secreted by a cancer cell. Preferably, the cell is a cancer cell, e.g., a breast, ovarian, stomach, endometrial, salivary gland, lung, kidney, colon, thyroid, pancreatic or bladder cell. Cell death *in vitro* may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody, oligopeptide or other organic molecule is able to induce cell death, loss of membrane integrity as evaluated by uptake of propidium iodide (PI), trypan blue (see Moore et al. Cytotechnology 17:1-11(1995)) or 7AAD can be assessed relative to untreated cells. Preferred cell death-inducing antibodies, oligopeptides or other organic molecules are those which induce PI uptake in the PI uptake assay in BT474 cells.

As used herein, the term "immunoadhesion" designates antibody-like molecules which combine the binding specificity of a heterologous protein (an "adhesion") with the effector functions of immunoglobulin constant domains. Structurally, the immunoadhesions comprise a fusion of an amino acid sequence with the desired binding specificity which is other than the antigen recognition and binding site of an antibody (i.e., is "heterologous"), and an immunoglobulin constant domain sequence. The adhesion part of an immunoadhesion molecule typically is a contiguous amino acid sequence comprising at least the binding site of a receptor or a ligand. The immunoglobulin constant domain sequence in the immunoadhesion may be obtained from any immunoglobulin, such as IgG-1, IgG-2, IgG-3, or IgG-4 subtypes, IgA (including IgA-1 and IgA-2), IgE, IgD or IgM.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.
"Replication-preventing agent" is an agent wherein replication, function, and/or growth of the cells is inhibited or prevented, or cells are destroyed, no matter what the mechanism, such as by apoptosis, angiostasis, cytosis, tumoricide, mytosis inhibition, blocking cell cycle progression, arresting cell growth, binding to tumors, acting as cellular mediators, etc. Such agents include a chemotherapeutic agent, cytotoxic agent, cytokine, growth-inhibitory agent, or anti-hormonal agent, e.g., an anti-estrogen compound such as tamoxifen, an anti-progesterone such as onapristone (see, EP 616 812); or an anti-androgen such as flutamide, as well as aromidase inhibitors, or a hormonal agent such as an androgen.

The term "cytotoxic agent" as used herein refers to a substance that inhibits or prevents the function of cells and/or causes destruction of cells. The term is intended to include radioactive isotopes (e.g., At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins ofbacterial, fungal, plant or animal origin, including fragments and/or variants thereof, and the various antitumor or anticancer agents disclosed below. Other cytotoxic agents are described below. A tumoricidal agent causes destruction of tumor cells.

Preferred cytotoxic agents herein for the specific tumor types to use in combination with the antagonists herein are as follows:
1. Prostate cancer: androgens, docetaxel, paclitaxel, estramustine, doxorubicin, mitoxantrone, antibodies to ErbB2 domain(s) such as 2C4 (WO 01 /00245; hybridoma ATCC HB-12697), which binds to a region in the extracellular domain of ErbB2 (e.g., any one or more residues in the region from about residue 22 to about residue 584 of ErbB2, inclusive), AVASTIN^{™} anti-vascular endothelial growth factor (VEGF), TARCEVA^{™}OSI-774 (erlotinib) (Genenetech and OSI Pharmaceuticals), or other epidermal growth factor receptor tyrosine kinase inhibitors (EGFR TKI's).
2. Stomach cancer: 5-fluorouracil (5FU), XELODA^{™} capecitabine, methotrexate, etoposide, cisplatin/carboplatin, pacliitaxel, docetaxel, gemcitabine, doxorubicin, and CPT-11 (camptothcin-11; irinotecan, USA Brand Name: CAMPTOSAR^{®}).
3. Pancreatic cancer: gemcitabine, 5FU, XELODA^{™} capecitabine, CT-11, docetaxel, paclitaxel, cisplatin, carboplatin, TARCEVA^{™} erlotinib, and other EGFR TKI's.
4. Colorectal cancer: 5FU, XELODA^{™} capecitabine, CPT-11, oxaliplatin, AVASTIN^{™} anti-VEGF, TARCEVA^{™} erlotinib and other EGFR TKI's, and ERBITUX^{™} (formerly known as IMC-C225) human:murine-chimerized monoclonal antibody that binds to EGFR and blocks the ability of EGF to initiate receptor activation and signaling to the tumor.
5. Renal cancer: IL-2, interferon alpha, AVASTIN^{™} anti-VEGF, MEGACE^{™} (Megestrol acetate) progestin, vinblastine, TARCEVA^{™} erlotinib, and other EGFR TKI's.

A "growth inhibitory agent" when used herein refers to a compound or composition which inhibits growth of a cell, especially a PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO375 10-, PRO35444-, PRO20473-, PRO21054- or PRO35246-expressing cancer cell, either *in vitro* or *in vivo*. Thus, the growth inhibitory agent may be one which significantly reduces the percentage of PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-expressing cells in S phase. Examples of growth inhibitory agents include agents that block cell cycle progression (at a place other than S phase), such as agents that induce G 1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE^{®}, Rhone-Poulenc Rorer), derived from the European yew, is a semisynthetic analogue ofpaclitaxel (TAXOL^{®}, Bristol-Myers Squibb). Paclitaxel and docetaxel promote the assembly of microtubules from tubulin dimers and stabilize microtubules by preventing depolymerization, which results in the inhibition of mitosis in cells.
"Doxorubicin" is an anthracycline antibiotic. The full chemical name of doxorubicin is (8S-cis)-10-[(3-amino-2,3,6-trideoxy-α-L-lyxo-hexapyranosyl)oxy]-7,8,9,10-tetrahydro-6,8,11-trihydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon -α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- la, IL-2, IL-3, IL-4, IL-5, IL-6, IL-7, IL-8, IL-9, IL-11, IL-12; a tumor necrosis factor such as TNF-α or TNF-B; and other polypeptide factors including LIF and kit ligand (KL). As used herein, the term cytokine includes proteins from natural sources or from recombinant cell culture and biologically active equivalents of the native sequence cytokines.

The term "package insert" is used to refer to instructions customarily included in commercial packages of therapeutic products, that contain information about the indications, usage, dosage, administration, contraindications and/or warnings concerning the use of such therapeutic products.

The term "gene" refers to (a) a gene containing at least one of the DNA sequences disclosed herein; (b) any DNA sequence that encodes the amino acid sequence encoded by the DNA sequences disclosed herein and/or; ©) any DNA sequence that hybridizes to the complement of the coding sequences disclosed herein. Preferably, the term includes coding as well as noncoding regions, and preferably includes all sequences necessary for normal gene expression.

The term "gene targeting" refers to a type of homologous recombination that occurs when a fragment of genomic DNA is introduced into a mammalian cell and that fragment locates and recombines with endogenous homologous sequences. Gene targeting by homologous recombination employs recombinant DNA technologies to replace specific genomic sequences with exogenous DNA of particular design.

The term "homologous recombination" refers to the exchange of DNA fragments between two DNA molecules or chromatids at the site of homologous nucleotide sequences.

The term "target gene" (alternatively referred to as "target gene sequence" or "target DNA sequence") refers to any nucleic acid molecule, polynucleotide, or gene to be modified by homologous recombination. The target sequence includes an intact gene, an exon or intron, a regulatory sequence or any region between genes. The target gene my comprise a portion of a particular gene or genetic locus in the individual's genomic DNA.
"Disruption" of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene occurs when a fragment of genomic DNA locates and recombines with an endogenous homologous sequence wherein the disruption is a deletion of the native gene or a portion thereof, or a mutation in the native gene or wherein the disruption is the functional inactivation of the native gene. Alternatively, sequence disruptions may be generated by nonspecific insertional inactivation using a gene trap vector (i.e. non-human transgenic animals containing and expressing a randomly inserted transgene; *see* for example U.S. Pat. No. 6,436,707 issued August 20, 2002). These sequence disruptions or modifications may include insertions, missense, frameshift, deletion, or substitutions, or replacements of DNA sequence, or any combination thereof. Insertions include the insertion of entire genes, which may be of animal, plant, fungal, insect, prokaryotic, or viral origin. Disruption, for example, can alter the normal gene product by inhibiting its production partially or completely or by enhancing the normal gene product's activity. Preferably, the disruption is a null disruption, wherein there is no significant expression of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene.

The term "native expression" refers to the expression of the full-length polypeptide encoded by the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene, at expression levels present in the wild-type mouse. Thus, a disruption in which there is "no native expression" of the endogenous PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO3 5246 gene refers to a partial or complete reduction of the expression of at least a portion of a polypeptide encoded by an endogenous PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene of a single cell, selected cells, or all of the cells of a mammal.

The term "knockout" refers to the disruption of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene wherein the disruption results in: the functional inactivation of the native gene; the deletion of the native gene or a portion thereof; or a mutation in the native gene.

The term "knock-in" refers to the replacement of the mouse ortholog (or other mouse gene) with a human cDNA encoding any of the specific human PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding genes or variants thereof (ie. the disruption results in a replacement of a native mouse gene with a native human gene).

The term "construct" or "targeting construct" refers to an artificially assembled DNA segment to be transferred into a target tissue, cell line or animal. Typically, the targeting construct will include a gene or a nucleic acid sequence of particular interest, a marker gene and appropriate control sequences. As provided herein, the targeting construct comprises a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 targeting construct. A "PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 targeting construct" includes a DNA sequence homologous to at least one portion of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene and is capable of producing a disruption in a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene in a host cell.

The term "transgenic cell" refers to a cell containing within its genome a PRO188, PRO235, PRO266, PRO337,PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene that has been disrupted, modified, altered, or replaced completely or partially by the method of gene targeting.

The term "transgenic animal" refers to an animal that contains within its genome a specific gene that has been disrupted or otherwise modified or mutated by the methods described herein or methods otherwise well known in the art. Preferably the non-human transgenic animal is a mammal. More preferably, the mammal is a rodent such as a rat or mouse. In addition, a "transgenic animal" may be a heterozygous animal (i.e., one defective allele and one wild-type allele) or a homozygous animal (i.e., two defective alleles). An embryo is considered to fall within the definition of an animal. The provision of an animal includes the provision of an embryo or foetus *in utero*, whether by mating or otherwise, and whether or not the embryo goes to term.

As used herein, the terms "selective marker" and position selection marker" refer to a gene encoding a product that enables only the cells that carry the gene to survive and/or grow under certain conditions. For example, plant and animal cells that express the introduced neomycin resistance (Neo^{r}) gene are resistant to the compound G418. Cells that do not carry the Neo^{r} gene marker are killed by G418. Other positive selection markers are known to, or are within the purview of, those of ordinary skill in the art.

The term "modulates" or "modulation" as used herein refers to the decrease, inhibition, reduction, amelioration, increase or enhancement of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene function, expression, activity, or alternatively a phenotype associated with PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene.

The term "ameliorates" or "amelioration" as used herein refers to a decrease, reduction or elimination of a condition, disease, disorder, or phenotype, including an abnormality or symptom.

The term "abnormality" refers to any disease, disorder, condition, or phenotype in which PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is implicated, includingpathological conditions and behavioral observations.

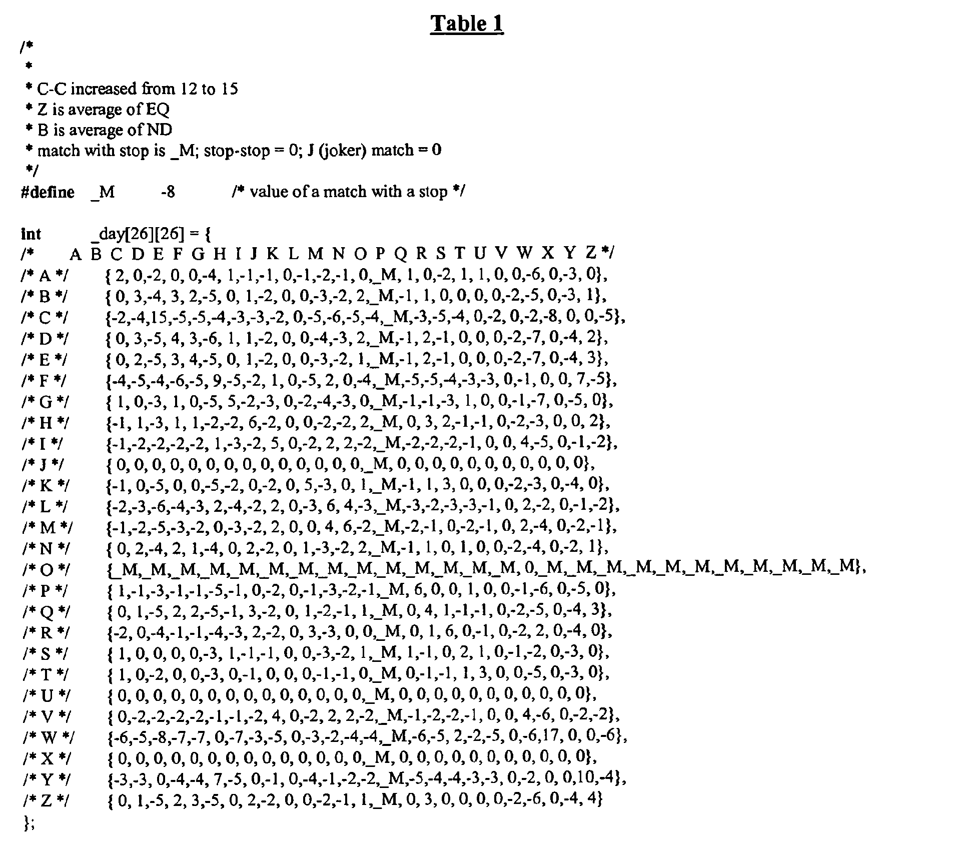

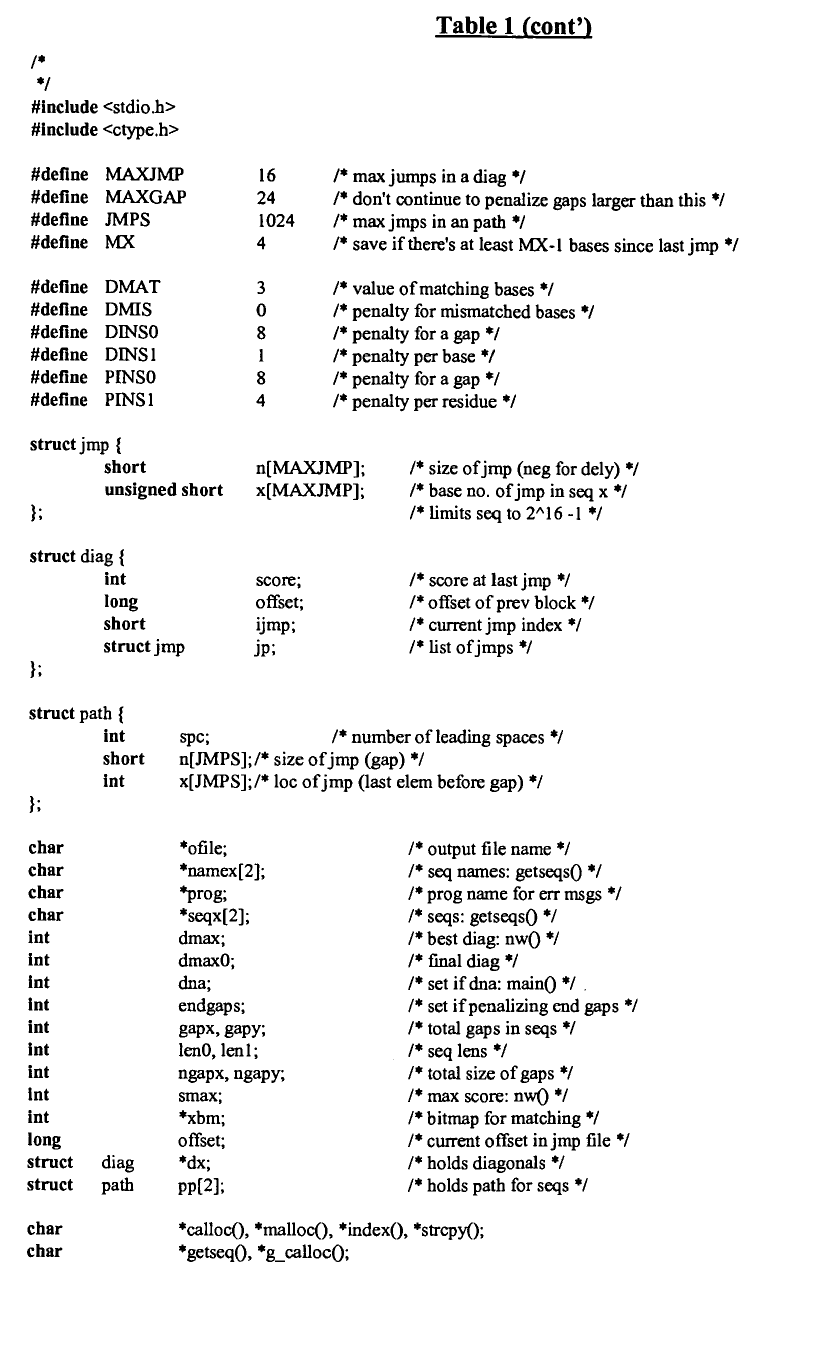

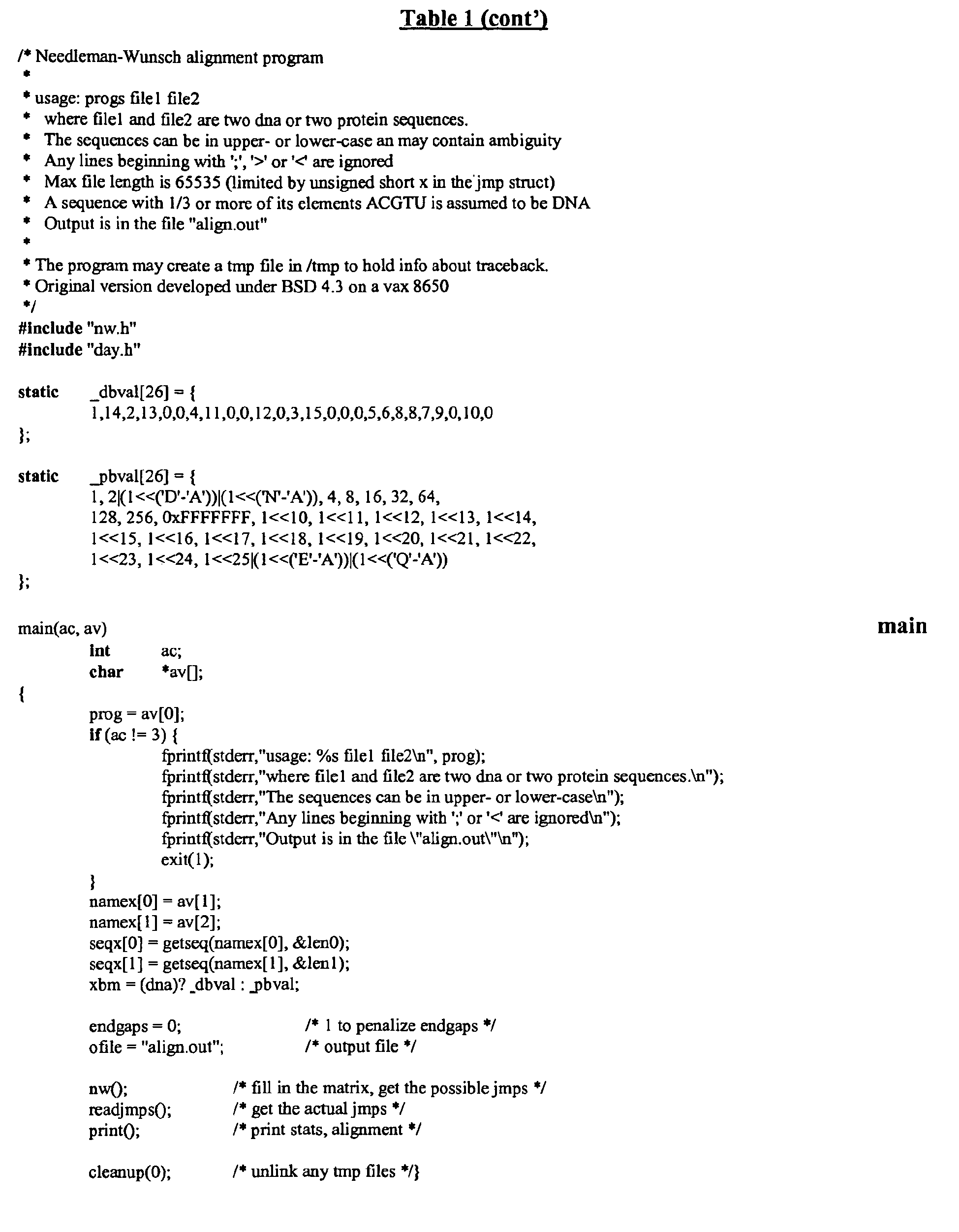

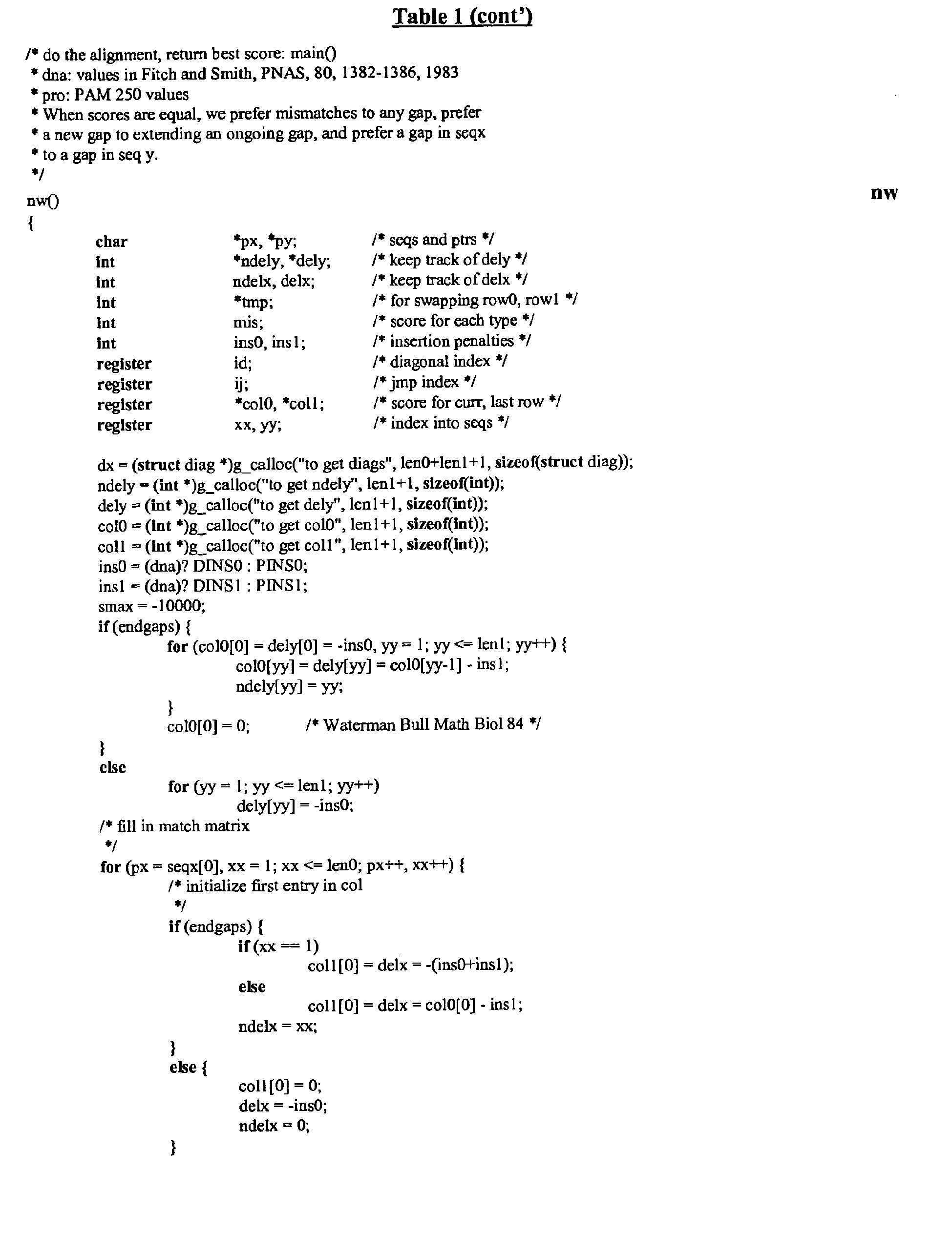

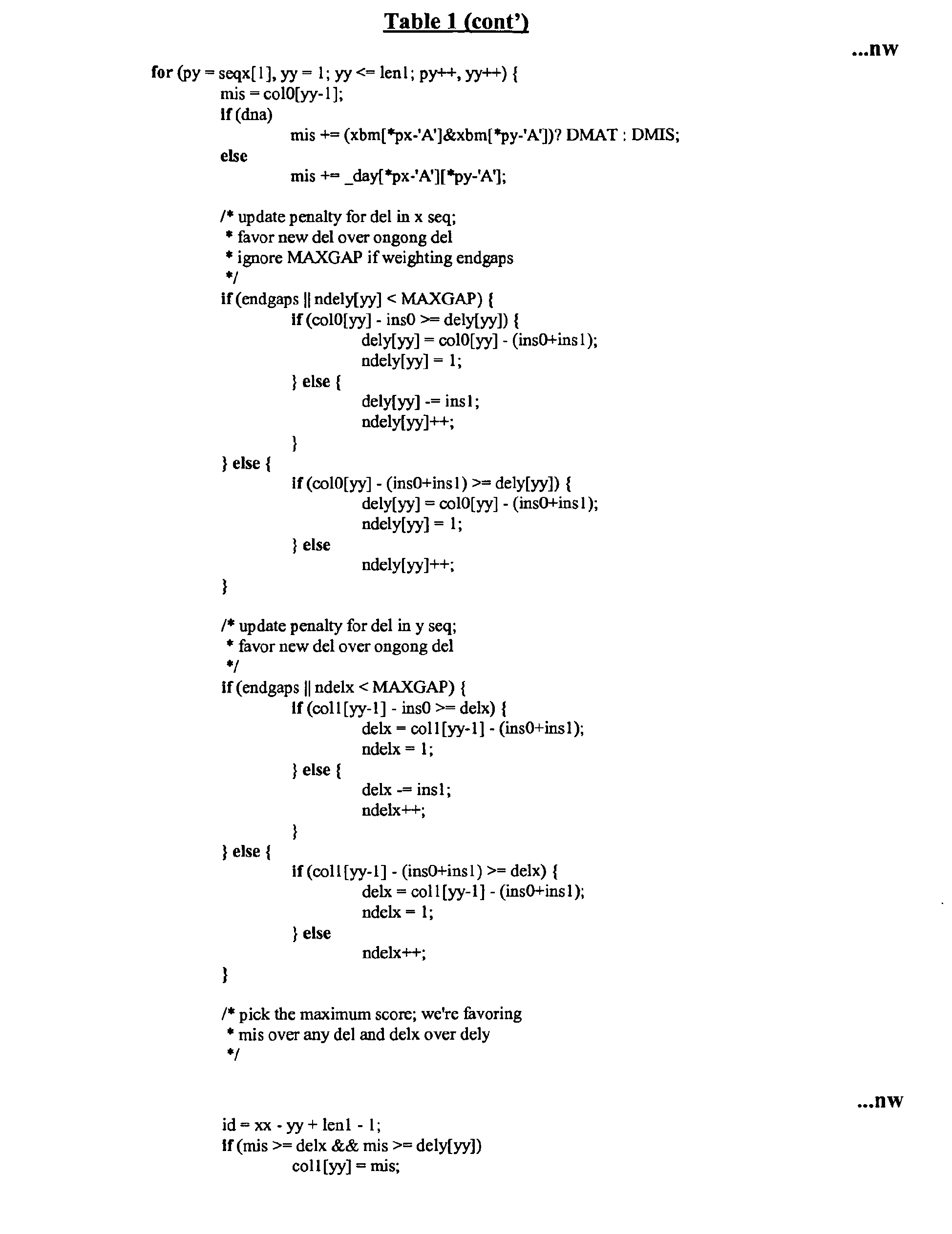

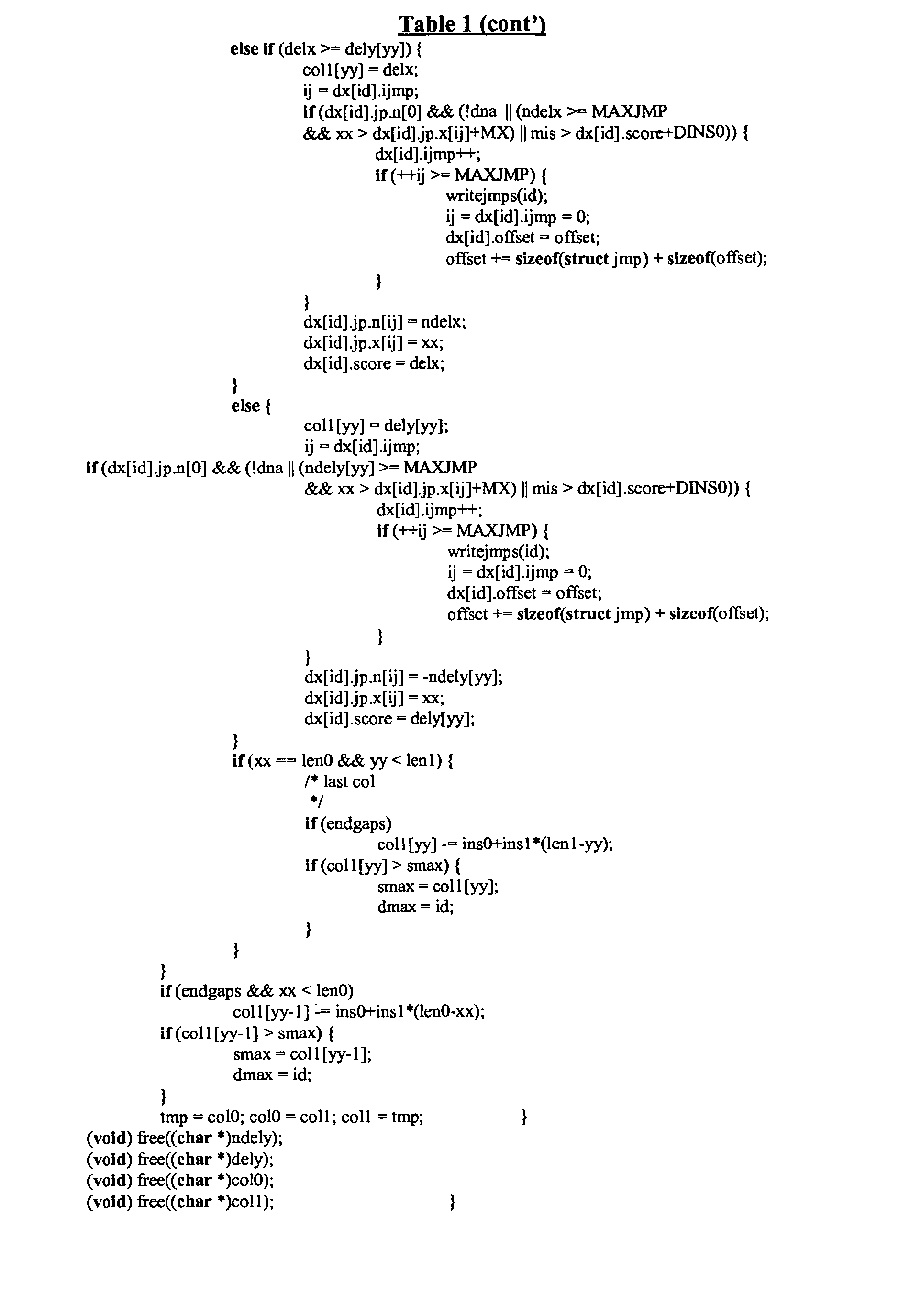

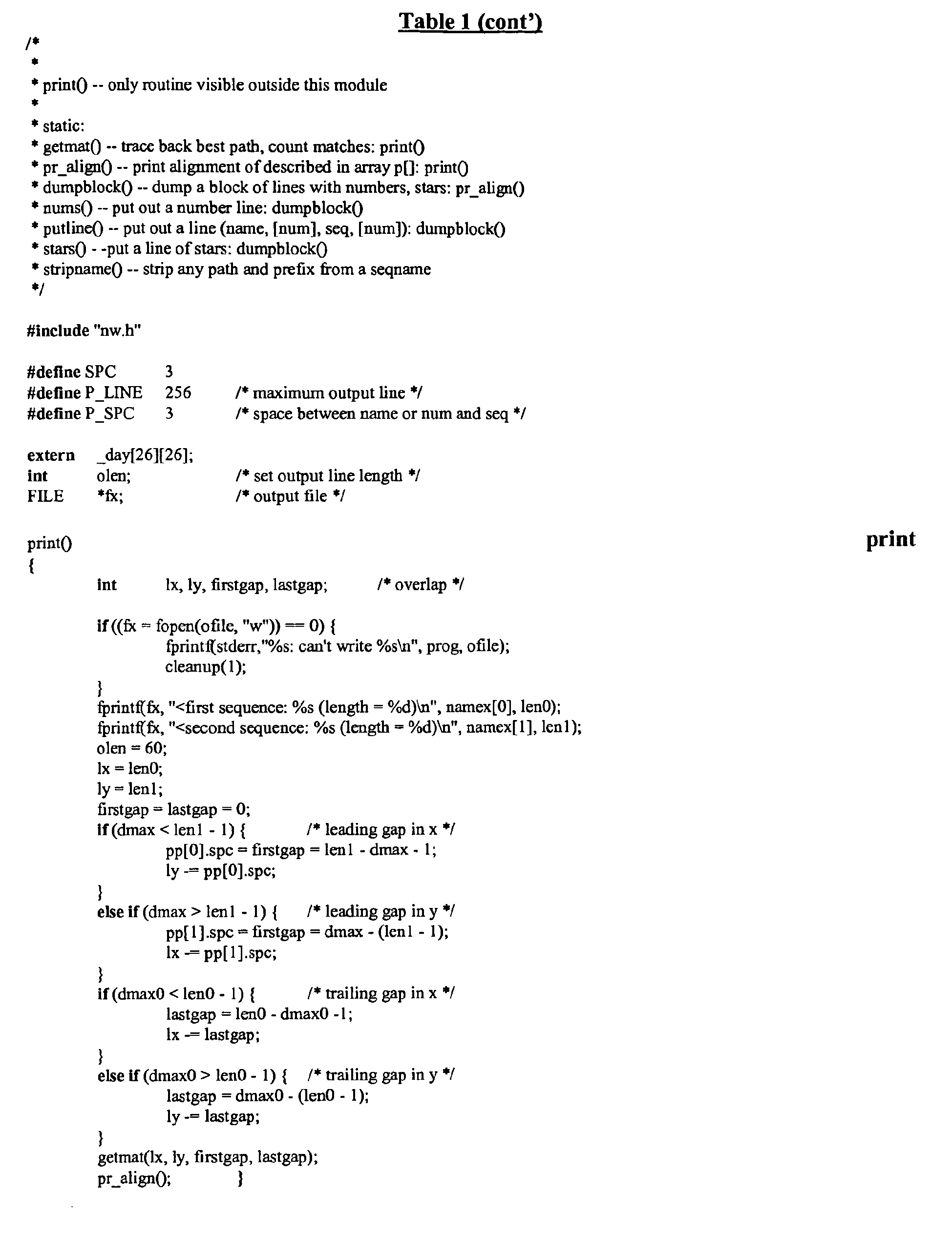

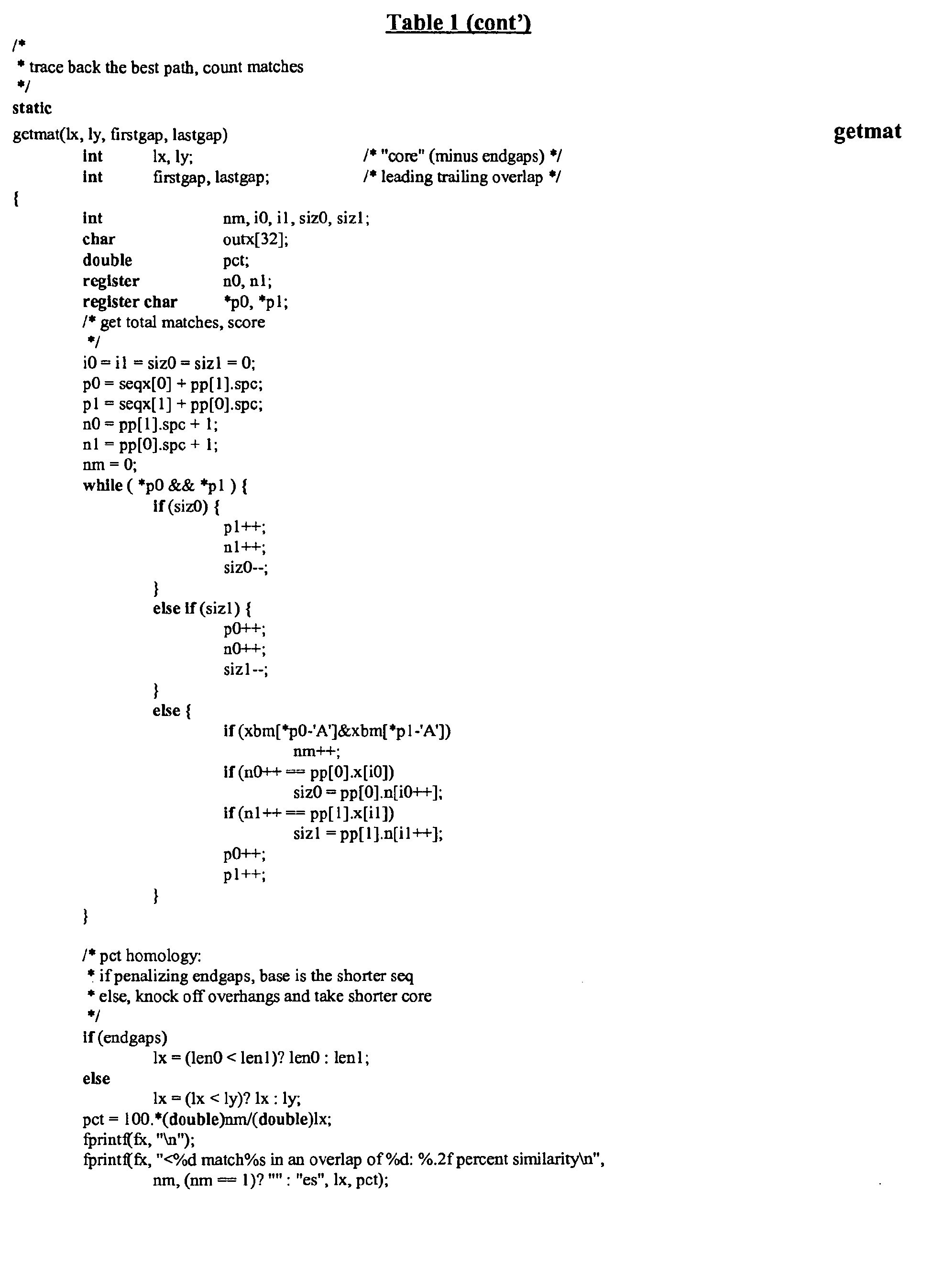

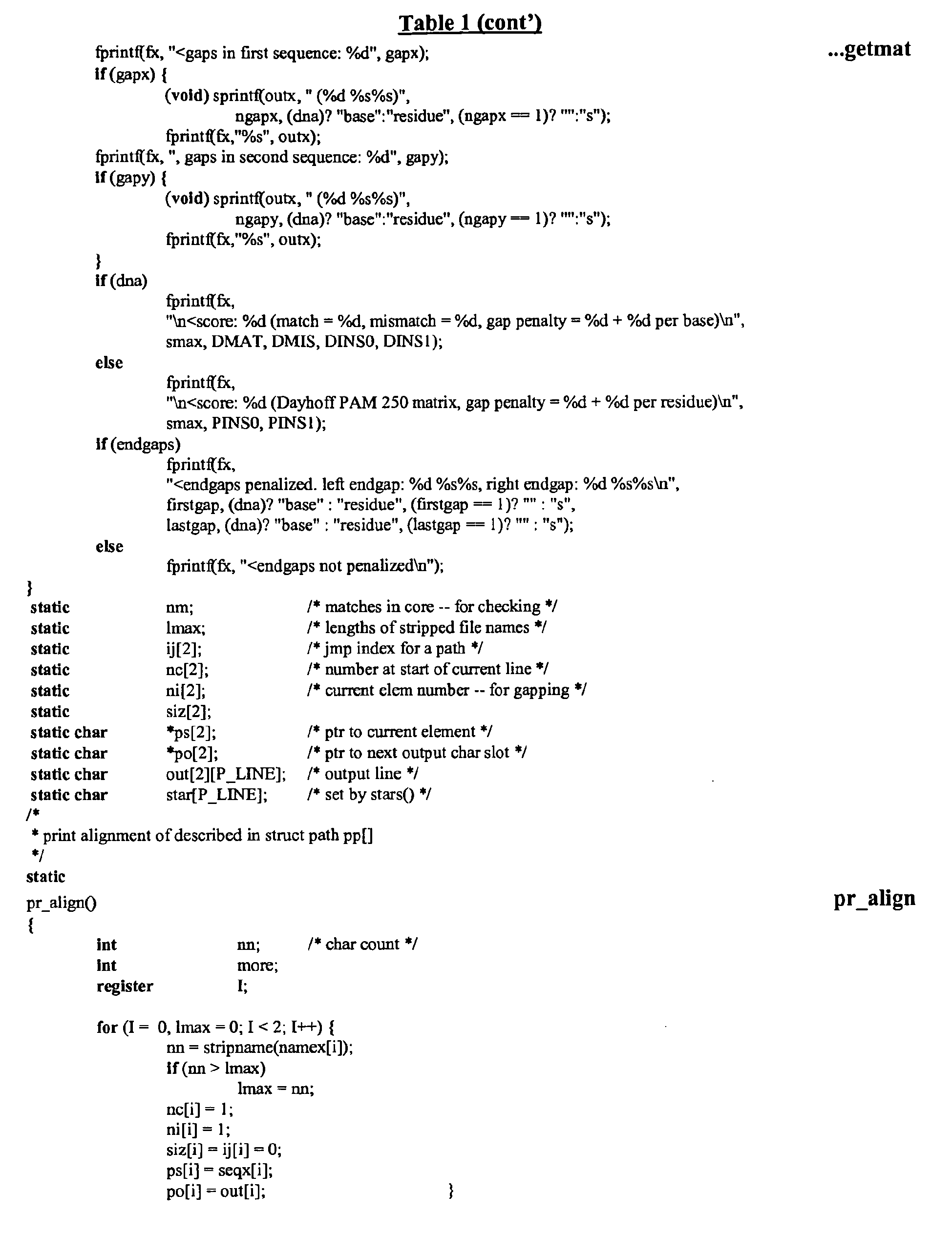

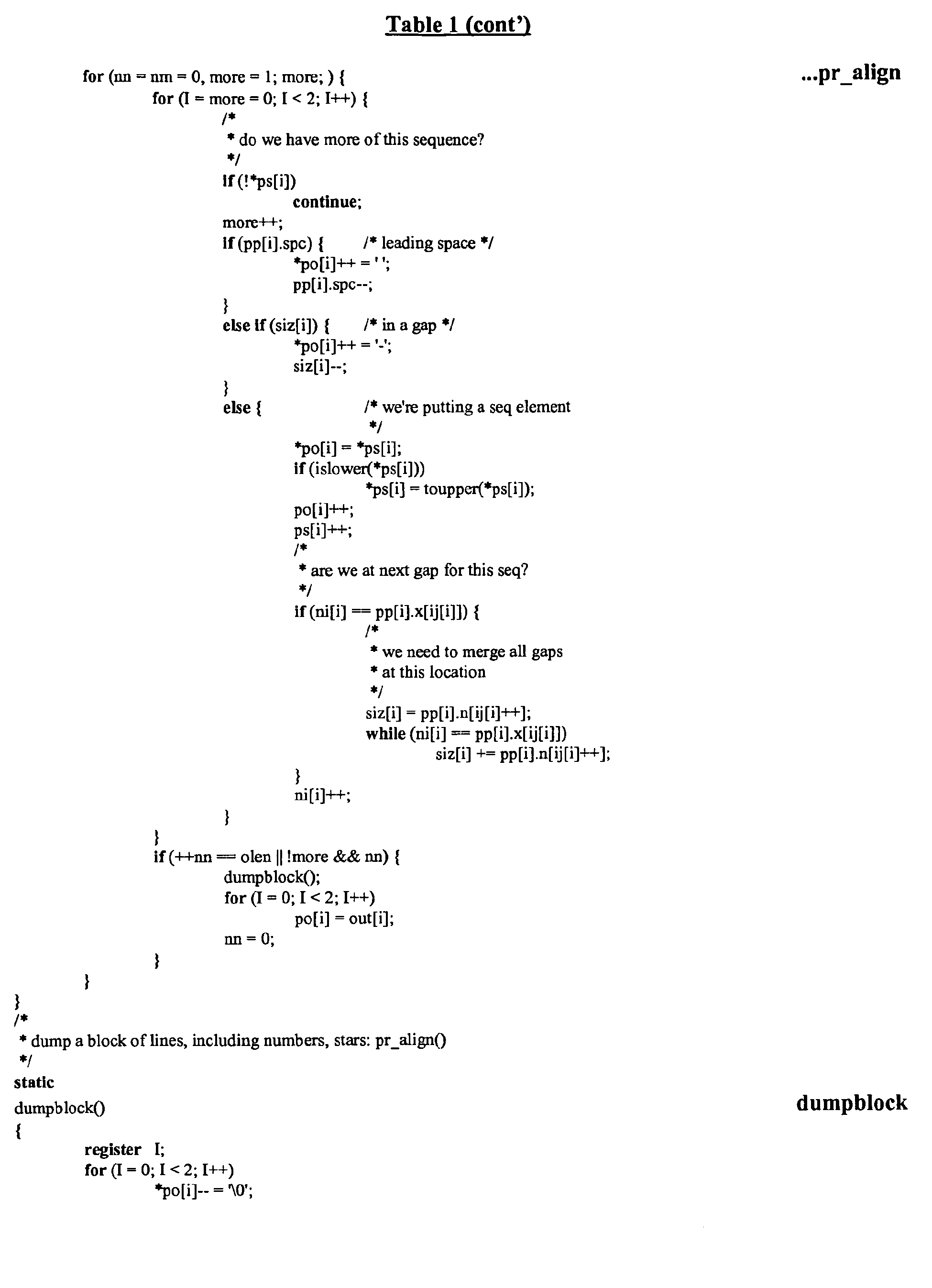

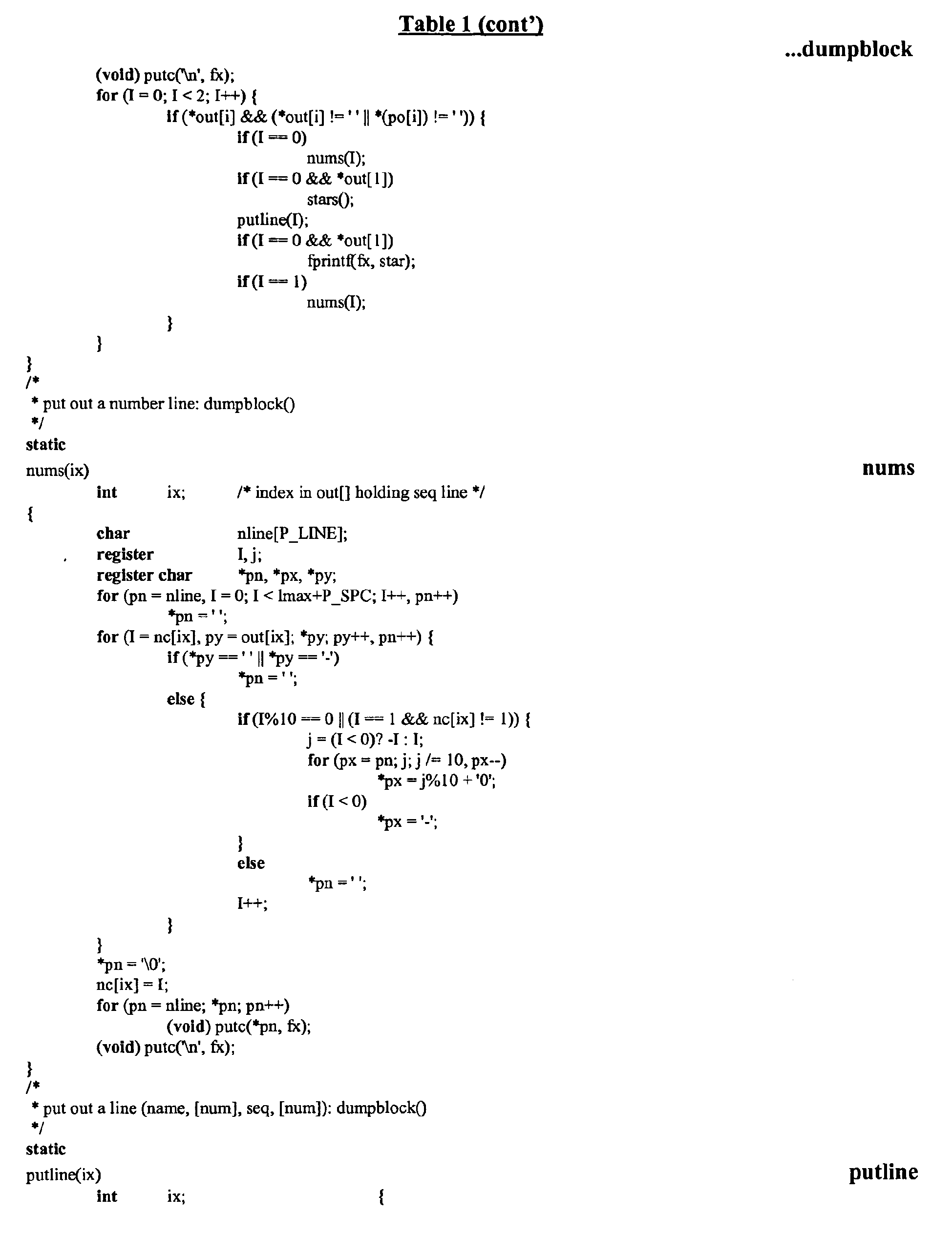

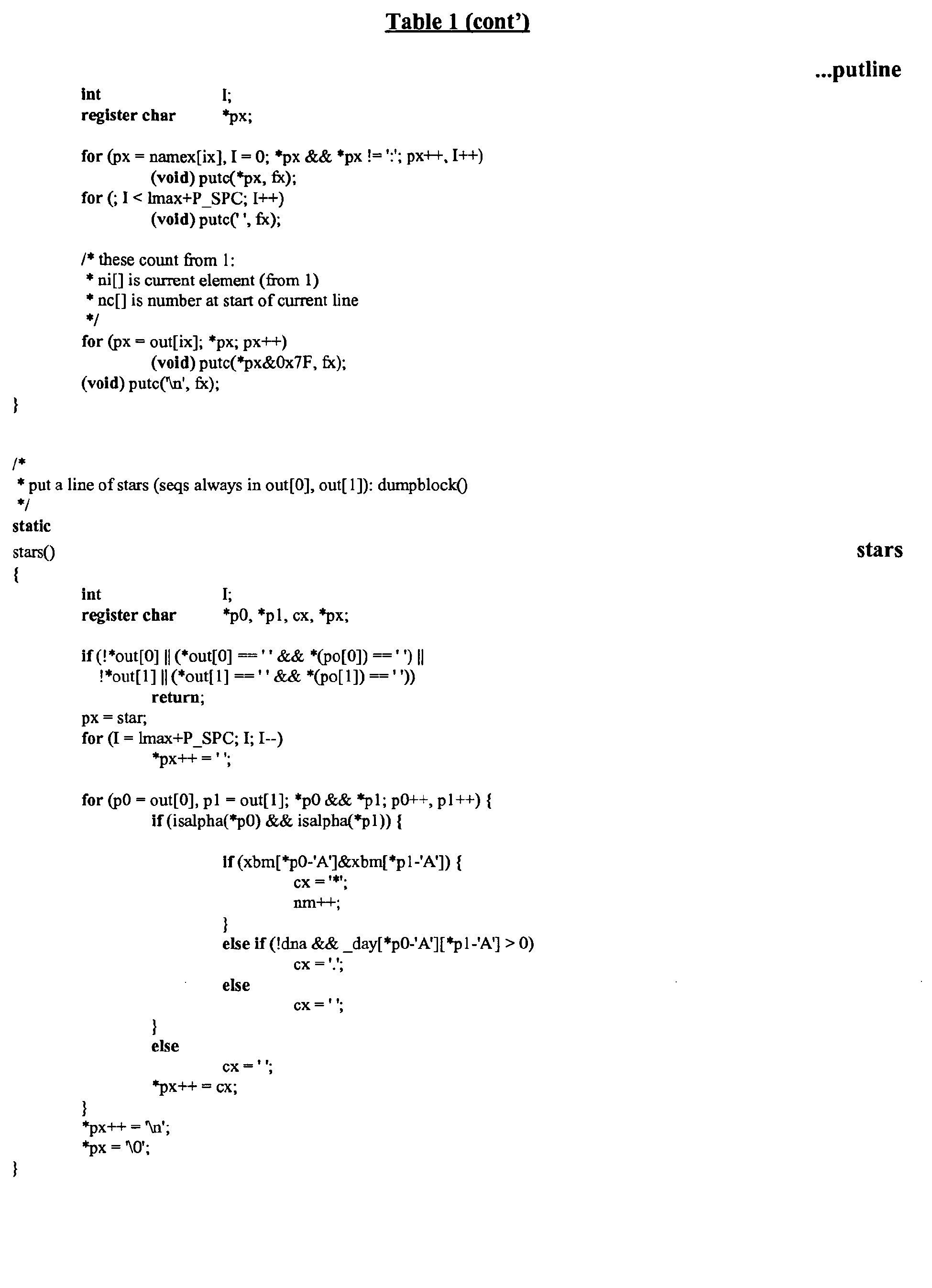

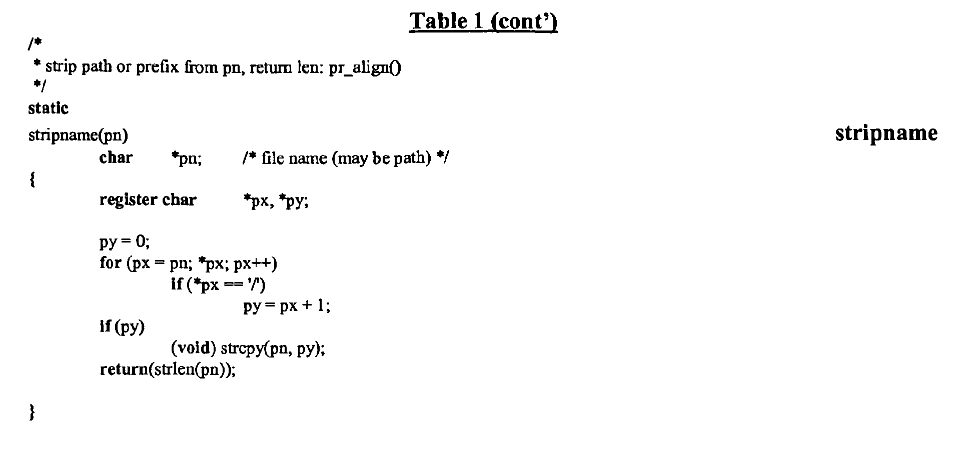

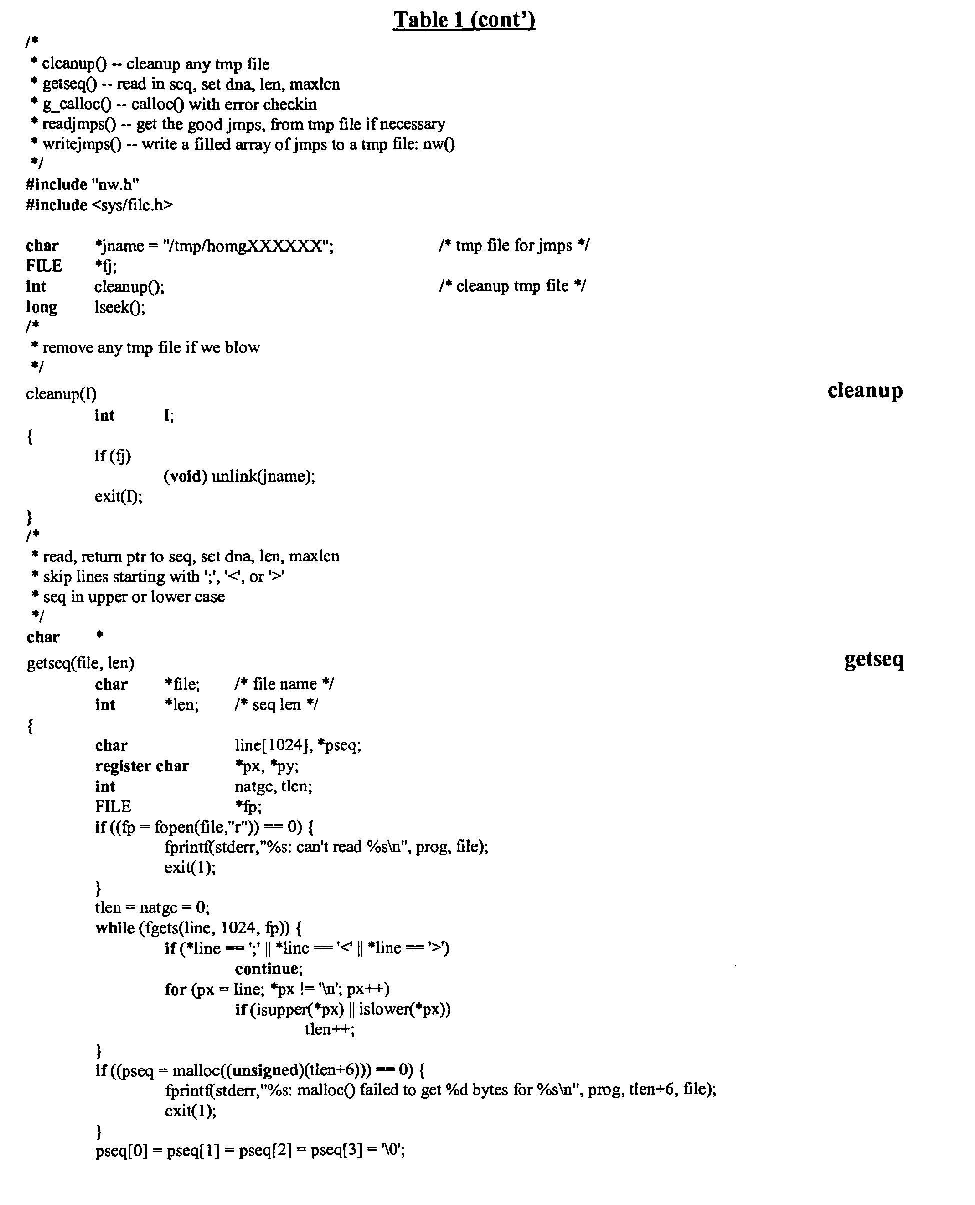

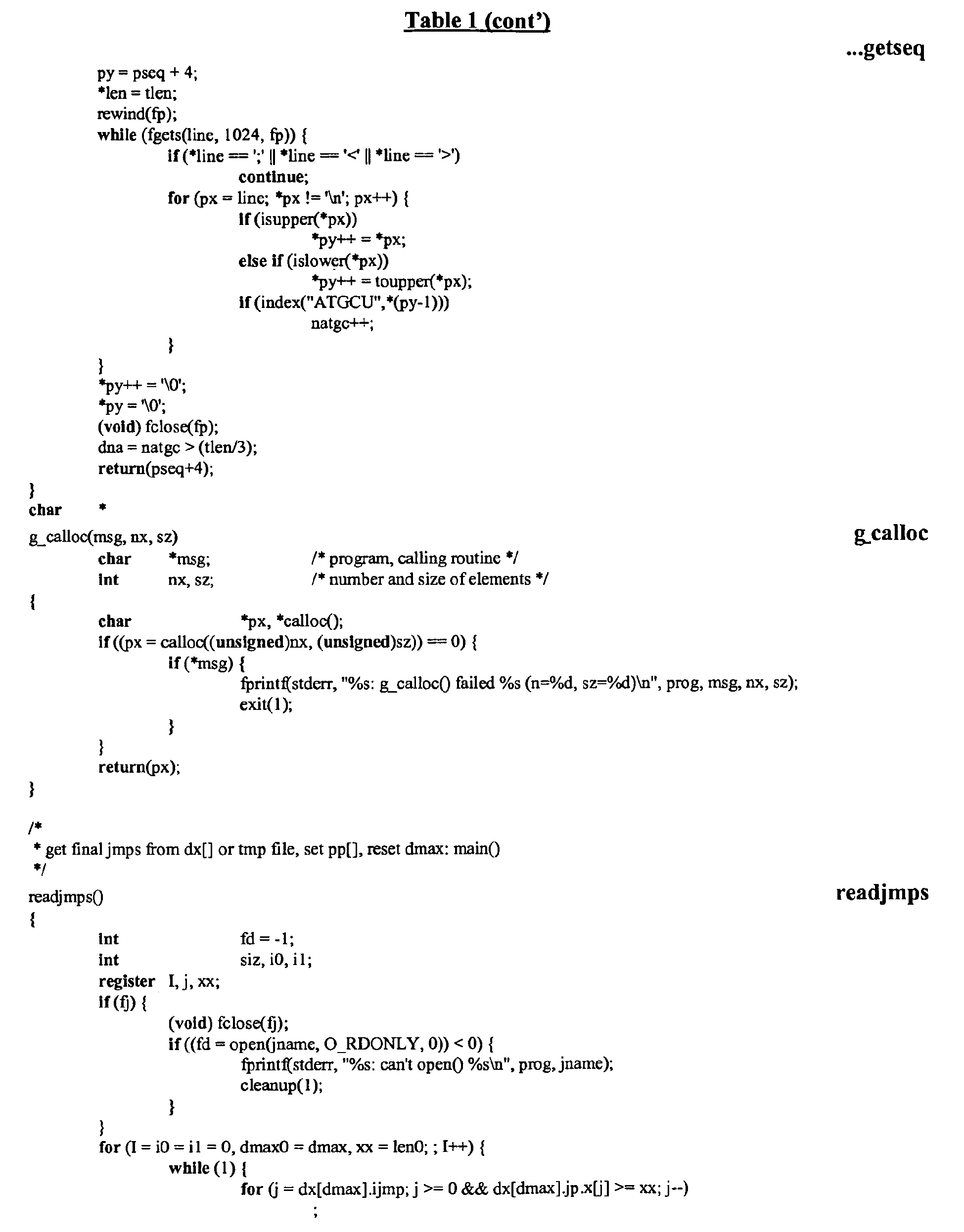

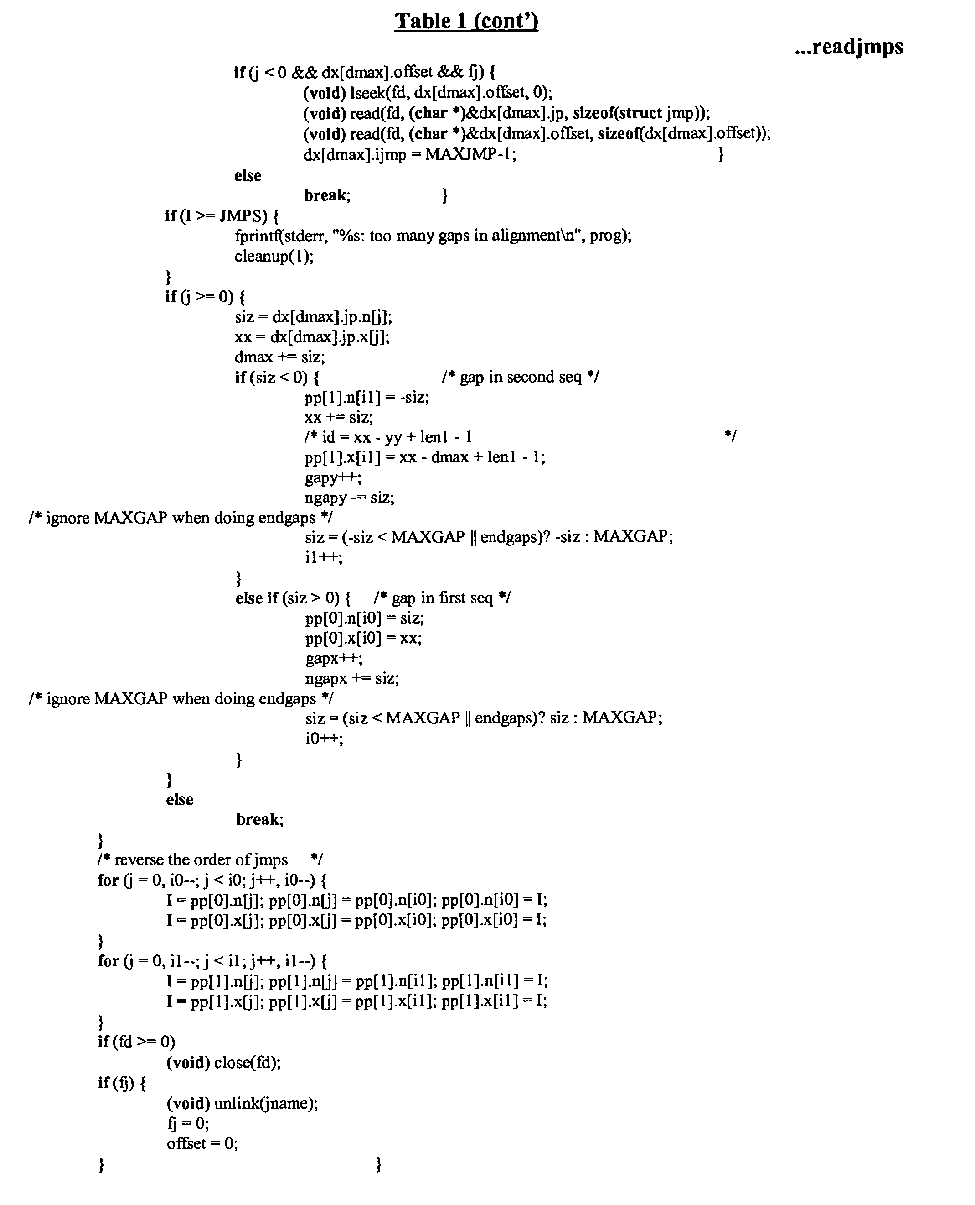

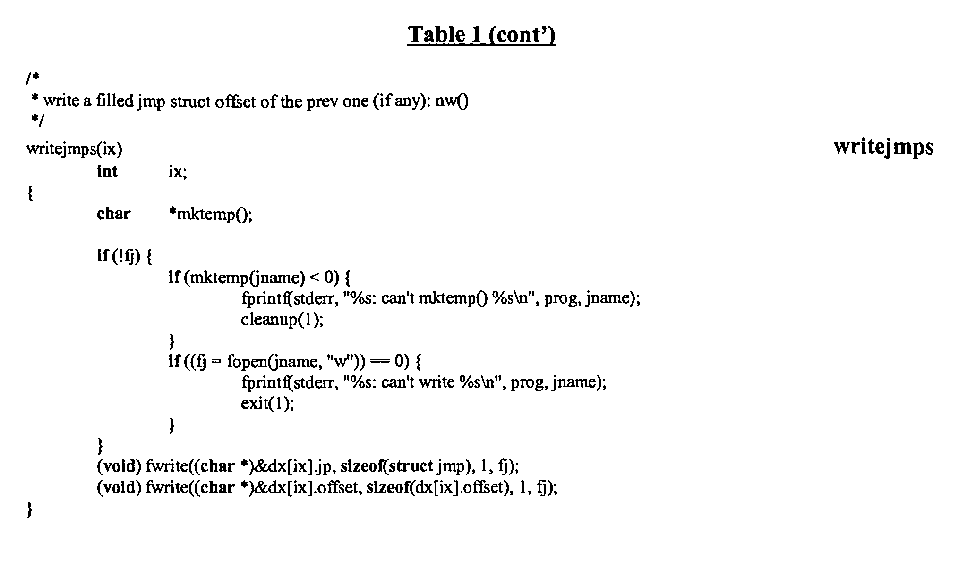

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity=
(the number of identically matching amino acid residues between the two polypeptide sequences as determined by ALIGN-2) divided by (the total number of amino acid residues of the PRO polypeptide)=
5 divided by 15=33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity=
(the number of identically matching amino acid residues between the two polypeptide sequences as determined by ALIGN-2) divided by (the total number of amino acid residues of the PRO polypeptide)=
5 divided by 10=50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% amino acid sequence identity=
(the number of identically matching nucleotides between the two nucleic acid sequences as determined by ALIGN-2) divided by (the total number of nucleotides of the PRO-DNA nucleic acid sequence)=
6 divided by 14=42.9%

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |

% amino acid sequence identity=
(the number of identically matching nucleotides between the two nucleic acid sequences as determined by ALIGN-2) divided by (the total number of nucleotides of the PRO-DNA nucleic acid sequence)=
4 divided by 12=33.3%

### II. Compositions and Methods of the Invention

### A. Full-Length PRO188, PRO235, PRO266, PRO337, PRO361, PRO539, PRO698, PRO717, PRO846, PRO874, PRO98346, PRO1082, PRO1097, PRO1192, PRO1268, PRO1278. PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides

The present invention provides newly identified and isolated nucleotide sequences encoding polypeptides referred to in the present application as PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. In particular, cDNAs encoding various PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides and encoding nucleic acids described herein, Applicants have identified what is believed to be the reading frame best identifiable with the sequence information available at the time.

### B. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptide Variants

In addition to the full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides described herein, it is contemplated that PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variants can be prepared. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variants can be prepared by introducing appropriate nucleotide changes into the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA, and/or by synthesis of the desired PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide. Those skilled in the art will appreciate that amino acid changes may alter post-translational processes of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, such as changing the number or position of glycosylation sites or altering the membrane anchoring characteristics.

Variations in the native full-length sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or in various domains of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide that results in a change in the amino acid sequence of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as compared with the native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide. Optionally the variation is by substitution of at least one amino acid with any other amino acid in one or more of the domains of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Guidance in determining which amino acid residue may be inserted, substituted or deleted without adversely affecting the desired activity may be found by comparing the sequence of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303 PRO1308, PRO1338, PRO1378 PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 fragments may be prepared by any of a number of conventional techniques. Desired peptide fragments may be chemically synthesized. An alternative approach involves generating PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that defme the desired termini of the DNA fragment are employed at the 5' and 3' primers in the PCR. Preferably, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fragments share at least one biological and/or immunological activity with the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide disclosed herein.

Conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are preferably introduced and the products screened.

**Table 6**

| **Original Residue** | **Exemplary Substitutions** | **Preferred Substitutions** |
|---|---|---|
| Ala (A) | Val; Leu; Ile | Val |
| Arg(R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| lie (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in function or immunological identity of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
Amino acids may be grouped according to similarities in the properties of their side chains (in A. L. Lehninger, in Biochemistry, second ed., pp. 73-75, Worth Publishers, New York (1975)):
   (1) non-polar: Ala (A), Val (V), Leu (L), Ile (I), Pro (P), Phe (F), Trp (W), Met (M)
   (2) uncharged polar: Gly (G), Ser (S), Thr (T), Cys (C), Tyr (Y), Asn (N), Gln (Q)
   (3) acidic: Asp (D), Glu (E)
   (4) basic: Lys (K), Arg (R), His(H)
Alternatively, naturally occurring residues may be divided into groups based on common side-chain properties:
   (1) hydrophobic: Norleucine, Met, Ala, Val, Leu, Ile;
   (2) neutral hydrophilic: Cys, Ser, Thr, Asn, Gln;
   (3) acidic: Asp, Glu;
   (4) basic: His, Lys, Arg;
   (5) residues that influence chain orientation: Gly, Pro;
   (6) aromatic: Trp, Tyr, Phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO343 8, PRO19835, PRO36916, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides

Covalent modifications of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides are included within the scope ofthis invention. One type of covalent modification includes reacting targeted amino acid residues of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with anorganic derivatizing agent that is capable of reacting with selected side chains or the N- or C- terminal residues of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Derivatization with bifunctional agents is useful, for instance, for crosslinking PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides to a water-insoluble support matrix or surface for use in the method for purifying anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide included within the scope of this invention comprises altering the native glycosylation pattern of the polypeptide. "Altering the native glycosylation pattern" is intended for purposes herein to mean deleting one or more carbohydrate moieties found in native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides (either by removing the underlying glycosylation site or by deleting the glycosylation by chemical and/or enzymatic means), and/or adding one or more glycosylation sites that are not present in the native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. In addition, the phrase includes qualitative changes in the glycosylation of the native proteins, involving a change in the nature and proportions of the various carbohydrate moieties present.

Addition ofglycosylation sites to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be accomplished by altering the amino acid sequence. The alteration may be made, for example, by the addition of, or substitution by, one or more serine or threonine residues to the native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 (for O-linked glycosylation sites). The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 amino acid sequence may optionally be altered through changes at the DNA level, particularly by mutating the DNA encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide at preselected bases such that codons are generated that will translate into the desired amino acids.

Another means of increasing the number of carbohydrate moieties on the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131(1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides comprises linking the PRO188, PRO235, PRO266, PRO337, PRO361, PRO539, PRO698, PRO717, PRO846, PRO874, PRO98346, PRO1082, PRO1097 PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides of the present invention may also be modified in a way to form a chimeric molecule comprising the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide fused to another, heterologous polypeptide or amino acid sequence.

Such a chimeric molecule comprises a fusion of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with a tag polypeptide which provides an epitope to which an anti-tag antibody can selectively bind. The epitope tag is generally placed at the amino- or carboxyl- terminus of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. The presence ofsuch epitope-tagged forms of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be detected using an antibody against the tag polypeptide. Also, provision of the epitope tag enables the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

The chimeric molecule may comprise a fusion of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide in place of at least one variable region within an Ig molecule. In a particularly preferred aspect of the invention, the immunoglobulin fusion includes the hinge, CH2 and CH3, or the hinge, CH 1, CH2 and CH3 regions of an IgG 1 molecule. For the production of immunoglobulin fusions see also US Patent No. 5,428,130 issued June 27, 1995.

### D. Preparation of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides

The description below relates primarily to production of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides by culturing cells transformed or transfected with a vector containing PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acid. It is, of course, contemplated that alternative methods, which are well known in the art, may be employed to prepare PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. For instance, the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions ofthe PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be chemically synthesized separately and combined using chemical or enzymatic methods to produce the full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

### 1. Isolation of DNA Encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides

DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides may be obtained from a cDNA library prepared from tissue believed to possess the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 mRNA and to express it at a detectable level. Accordingly, human PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA can be conveniently obtained from a cDNA library prepared from human tissue, such as described in the Examples. The PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding gene may also be obtained from a genomic library or by known synthetic procedures (e.g., automated nucleic acid synthesis).

Libraries can be screened with probes (such as antibodies to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO343 8, PRO19835, PRO36915, PRO3 6029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31, 446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia*, e.g., *E. coli, Enterobacter, Erwinia, Klebsiella, Proteus, Salmonella*, e.g., *Salmonella typhimurium, Serratia*, e.g., *Serratia marcescans*, and *Shigella*, as well as *Bacilli* such as *B. subtilis* and *B. licheniformis* (e.g., *B. licheniformis* 41P disclosed in DD 266,710 published 12 April 1989), *Pseudomonas* such as *P. aeruginosa*, and *Streptomyces*. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3*; *E. coli* W3110 strain 27C7 (ATCC 55,244), which has the complete genotype *tonA ptr3 phoA E15 (argF-lac)169 degP ompT kan^{r}*;*E. coli* W3110 strain 37D6, which has the complete genotype *tonA ptr3 phoA E15 (argF-lac)169 degP ompT rbs7 ilvG kan^{r}; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such asA. *nidulans* (Ballance et al., Biochem. Biophys. Res. Commun.,112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis*, and *Rhodotorula*. A list of specific species that are exemplary of this class of yeasts may be found in C. Anthony, The Biochemistry of Methylotrophs, 269 (1982).

Suitable host cells for the expression of glycosylated PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component ofthe vector, or it may be a part ofthe PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding DNA that is inserted into the vector. The signal sequence may be a prokaryotic signal sequence selected, for example, from the group of the alkaline phosphatase, penicillinase, 1pp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli*.

An example of suitable selectable markers for mammalian cells are those that enable the identification of cells competent to take up the PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO021054- or PRO35246-encoding nucleic acid, such as DHFR or thymidine kinase. Anappropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry,17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides.

Still other methods, vectors, and host cells suitable for adaptation to the synthesis of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription ofmRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or against a synthetic peptide based on the DNA sequences provided herein or against exogenous sequence fused to PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA and encoding a specific antibody epitope.

### 5. Purification of Polypeptide

Forms of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides may be recovered from culture medium or from host cell lysates. If membrane-bound, it can be released from the membrane using a suitable detergent solution (e.g. Triton-X 100) or by enzymatic cleavage. Cells employed in expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides can be disrupted by various physical or chemical means, such as freeze-thaw cycling, sonication, mechanical disruption, or cell lysing agents.

It may be desired to purify PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide produced.

### E. Uses for PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides

Nucleotide sequences (or their complement) encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides have various applications in the art of molecular biology, including uses as hybridization probes, in chromosome and gene mapping and in the generation of anti-sense RNA and DNA. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acid will also be useful for the preparation of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides by the recombinant techniques described herein.

The full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO3 5444, PRO20473, PRO21054 or PRO35246 gene, or portions thereof, may be used as hybridization probes for a cDNA library to isolate the full-length PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 cDNA or to isolate still other cDNAs (for instance, those encoding naturally-occurring variants of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides from other species) which have a desired sequence identity to the native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415 PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246. By way of example, a screening method will comprise isolating the coding region of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO9835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene using the known DNA sequence to synthesize a selected probe of about 40 bases. Hybridization probes may be labeled by a variety of labels, including radionucleotides such as ³²P or ³⁵S, or enzymatic labels such as alkaline phosphatase coupled to the probe via avidin/biotin coupling systems. Labeled probes having a sequence complementary to that of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene of the present invention can be used to screen libraries of human cDNA, genomic DNA or mRNA to determine which members of such libraries the probe hybridizes to. Hybridization techniques are described in further detail in the Examples below.

Any EST sequences disclosed in the present application may similarly be employed as probes, using the methods disclosed herein.

Other useful fragments of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acids include antisense or sense oligonucleotides comprising a singe-stranded nucleic acid sequence (either RNA or DNA) capable of binding to target PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 mRNA (sense) or PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA (antisense) sequences. Antisense or sense oligonucleotides, according to the present invention, comprise a fragment of the coding region of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. The antisense oligonucleotides thus may be used to block expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable ofresisting enzymatic degradation) but retain sequence specificity to be able to bind to target nucleotide sequences.

Other examples ofsense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, CaPO₄-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo*. Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

The probes may also be employed in PCR techniques to generate a pool of sequences for identification of closely related PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 coding sequences.

Nucleotide sequences encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can also be used to construct hybridization probes for mapping the gene which encodes that PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890,PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

When the coding sequences for PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 encode a protein which binds to another protein (for example, where the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is a receptor), the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can be used to isolate correlative ligand(s). Screening assays can be designed to fmd lead compounds that mimic the biological activity of a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or a receptor for PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode PRO188, PRO235, PRO266, PRO337, PRO361, PRO539, PRO698, PRO717, PRO846, PRO874, PRO98346, PRO1082, PROl097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. The invention provides cDNA encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308 PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide which can be used to clone genomic DNA encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915,P RO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide in accordance with established techniques and the genomic sequences used to generate transgenic animals that contain cells which express DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. Any technique known in the art may be used to introduce a target gene transgene into animals to produce the founder lines of transgenic animals. Such techniques include, but are not limited to pronuclear microinjection (U.S. Pat. Nos. 4,873,191, 4,736,866 and 4,870,009); retrovirus mediated gene transfer into germ lines (Van der Putten, et al., Proc. Natl. Acad. Sci..USA, 82:6148-6152 (1985)); gene targeting in embryonic stem cells (Thompson, et al., Cell, 56:313-321 (1989)); nonspecific insertional inactivation using a gene trap vector (U.S. Pat. No. 6,436,707); electroporation of embryos (Lo, Mol. Cell Biol., 3:1803-1814 (1983)); and sperm-mediated gene transfer (Lavitrano, et al., Cell, 57:717-723 (1989)); etc. Typically, particular cells would be targeted for a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 transgene incorporation with tissue-specific enhancers. Transgenic animals that include a copy of a transgene encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide introduced into the germ line of the animal at an embryonic stage can be used to examine the effect of increased expression of DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition. Altematively,non-humanhomologuesofPRO188,PRO235,PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides can be used to construct a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 "knock out" animal which has a defective or altered gene encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867,PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 proteins as a result of homologous recombination between the endogenous gene encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides and altered genomic DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides introduced into an embryonic stem cell of the animal. Preferably the knock out animal is a mammal. More preferably, the mammal is a rodent such as a rat or mouse. For example, cDNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides can be used to clone genomic DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides in accordance with established techniques. A portion of the genomic DNA encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be deleted or replaced with another gene, such as a gene encoding a selectable marker which can be used to monitor integration. Typically, several kilobases of unaltered flanking DNA (both at the 5'and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the gene encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

In addition, knockout mice can be highly informative in the discovery of gene function and pharmaceutical utility for a drug target, as well as in the determination of the potential on-target side effects associated with a given target. Gene function and physiology are so well conserved between mice and humans., since they are both mammals and contain similar numbers of genes, which are highly conserved between the species. It has recently been well documented, for example, that 98% of genes on mouse chromosome 16 have a human ortholog (Mural et al., Science 296:1661-71 (2002)).

Although gene targeting in embryonic stem (ES) cells has enabled the construction of mice with null mutations in many genes associated with human disease, not all genetic diseases are attributable to null mutations. One can design valuable mouse models ofhuman diseases by establishing a method for gene replacement (knock-in) which will disrupt the mouse locus and introduce a human counterpart with mutation. Subsequently one can conduct *in vivo* drug studies targeting the human protein (Kitamoto et. Al., Biochemical and Biophysical Res. Commun., 222:742-47 (1996)).

Nucleic acid encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo*. It has already been shown that short antisense oligonucleotides can be imported into cells where they act as inhibitors, despite their low intracellular concentrations caused by their restricted uptake by the cell membrane. (Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use ofliposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et at., Science 256, 808-813 (1992).

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415,PRO1867, PRO1890, PRO3438, PRO19835, PRO36915, PRO36029, PRO4999,PRO5778,PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides described herein may also be employed as molecular weight markers for protein electrophoresis purposes and the isolated nucleic acid sequences may be used for recombinantly expressing those markers.

The nucleic acid molecules encoding the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides or fragments thereof described herein are useful for chromosome identification. In this regard, there exists an ongoing need to identify new chromosome markers, since relatively few chromosome marking reagents, based upon actual sequence data are presently available. Each PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acid molecule of the present invention can be used as a chromosome marker.

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides and nucleic acid molecules of the present invention may also be used diagnostically for tissue typing, wherein the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides of the present invention may be differentially expressed in one tissue as compared to another, preferably in a diseased tissue as compared to a normal tissue of the same tissue type. PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 nucleic acid molecules will find use for generating probes for PCR, Northern analysis, Southern analysis and Western analysis.

The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides described herein may also be employed as therapeutic agents. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides of the present invention can be formulated according to known methods to prepare pharmaceutically useful compositions, whereby the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form oflyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, PLURONICS^{™} or PEG.

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or agonist or antagonist thereof is employed, normal dosage amounts may vary from about 10 ng/kg to up to 100 mg/kg of mammal body weight or more per day, preferably about 1 µg/kg/day to 10 mg/kg/day, depending upon the route of administration. Guidance as to particular dosages and methods ofdelivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is desired in a formulation with release characteristics suitable for the treatment of any disease or disorder requiring administration of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, microencapsulation of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is contemplated. Microencapsulation of recombinant proteins for sustained release has been successfully performed with human growth hormone (rhGH), interferon- (rhIFN- ), interleukin-2, and MN rgp120. Johnson et al., Nat. Med., 2:795-799 (1996); Yasuda, Biomed. Ther., 27:1221-1223 (1993); Hora et al., Bio/Technology, 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range ofbiodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that mimic the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890,PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide (agonists) or prevent the effect of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833,PRO9836,PRO9854, PRO9862, PRO10284, PRO37510, PRO35444, PRO20473, PRO21054 or PRO35246 polypeptide (antagonists). Agonists that mimic a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide would be especially valuable therapeutically in those instances where a negative phenotype is observed based on findings with the non-human transgenic animal whose genome comprises a disruption of the gene which encodes for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Antagonists that prevent the effects of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide would be especially valuable therapeutically in those instances where a positive phenotype is observed based upon observations with the non-human transgenic knockout animal. Screening assays for antagonist drug candidates are designed to identify compounds that bind or complex with the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378,PRO1415,PRO1867,PRO1890, PRO3438, PRO19835,PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide encoded by the genes identified herein, or otherwise interfere with the interaction of the encoded polypeptide with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide encoded by a nucleic acid identified herein under conditions and for a time sufficient to allow these two components to interact.

In binding assays, the interaction is binding and the complex formed can be isolated or detected in the reaction mixture. The PRO188 PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO343 8, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide encoded by the gene identified herein or the drug candidate is immobilized on a solid phase, e.g., on a microtiter plate, by covalent or non-covalent attachments. Non-covalent attachment generally is accomplished by coating the solid surface with a solution of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and drying. Alternatively, an immobilized antibody, e.g., a monoclonal antibody, specific for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide encoded by a gene identified herein, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction ofa gene encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be added to a cell along with the compound to be screened for a particular activity and the ability of the compound to inhibit the activity of interest in the presence of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide indicates that the compound is an antagonist to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Alternatively, antagonists may be detected by combining the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and a potential antagonist with membrane-bound PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide receptors or recombinant receptors under appropriate conditions for a competitive inhibition assay. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be labeled, such as by radioactivity, such that the number of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and a cDNA library created from this RNA is divided into pools and used to transfect COS cells or other cells that are not responsive to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Transfected cells that are grown on glass slides are exposed to labeled PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. ThePRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, the labeled PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

Another approach in assessing the effect of an antagonist to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, would be administering a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 antagonist to a wild-type mouse in order to mimic a known knockout phenotype. Thus, one would initially knockout the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene of interest and observe the resultant phenotype as a consequence of knocking out or disrupting the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene. Subsequently, one could then assess the effectiveness of an antagonist to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO3 5246 polypeptide by administering an antagonist to the PRO188, PRO23 5, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide to a wild-type mouse. An effective antagonist would be expected to mimic the phenotypic effect that was initially observed in the knockout animal.

Likewise, one could assess the effect of an agonist to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, by administering a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 agonist to a non-human transgenic mouse in order to ameliorate a known negative knockout phenotype. Thus, one would initially knockout the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene of interest and observe the resultant phenotype as a consequence of knocking out or disrupting the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 gene. Subsequently, one could then assess the effectiveness of an agonist to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by administering an agonist to the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide to a the non-human transgenic mouse. An effective agonist would be expected to ameliorate the negative phenotypic effect that was initially observed in the knockout animal.

In another assay for antagonists, mammalian cells or a membrane preparation expressing the receptor would be incubated with a labeled PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide in the presence of the candidate compound. The ability of the compound to enhance or block this interaction could then be measured.

More specific examples of potential antagonists include an oligonucleotide that binds to the fusions of immunoglobulin with the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO343 8, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide that recognizes the receptor but imparts no effect, thereby competitively inhibiting the action of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

Another potential PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO8867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. The antisense RNA oligonucleotide hybridizes to the mRNA *in vivo* and blocks translation of the mRNA molecule into the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, thereby blocking the normal biological activity of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. Examples ofsmall molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra*.

These small molecules can be identified by any one or more of the screening assays discussed hereinabove and/or by any other screening techniques well known for those skilled in the art.

Diagnostic and therapeutic uses of the herein disclosed molecules may also be based upon the positive functional assay hits disclosed and described below.

### F. Anti-PRO188, Anti-PRO235, Anti-PRO266, Anti-PRO337, Anti-PRO361, Anti-PRO539, Anti-PRO698, Anti-PRO717, Anti-PRO846, Anti-PRO874, Anti-PRO98346, Anti-PRO1082, Anti-PRO1097, Anti-PRO1192, Anti-PRO1268, Anti-PRO1278, Anti-PRO1303, Anti-PRO1308, Anti-PRO1338, Anti-PRO1378, Anti-PRO1415, Anti-PRO1867, Anti-PRO1890, Anti-PRO3438, Anti-PRO19835, Anti-PRO36915, Anti-PRO36029, Anti-PRO4999, Anti-PRO5778, Anti-PRO5997, Anti-PRO6079, Anti-PRO6090, Anti-PRO7178, Anti-PRO21184, Anti-PRO7434, Anti-PRO9822, Anti-PRO9833, Anti-PRO9836, Anti-PRO9854, Anti-PRO9862, Anti-PRO10284, Anti-PRO37510, Anti-PRO35444, Anti-PRO20473, Anti-PRO21054 or Anti-PRO35246 Antibodies

The present invention provides anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies which may find use herein as therapeutic and/or diagnostic agents. Exemplary antibodies include polyclonal, monoclonal, humanized, bispecific, and heteroconjugate antibodies.

### 1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal e.g,, by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as*E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

Monoclonal antibodies or antibody fragments can be isolated from antibody phage libraries generated using the techniques described in McCafferty et al., Nature, 348:552-554 (1990). Clackson et al., Nature, 3 52:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (C_{H} and C sequences for the homologous murine sequences (U.S. PatentNo. 4,816,567; and Morrison, et al., Proc. Natl Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### 3. Human and Humanized Antibodies

The anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all ofthe CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536(1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody are contemplated. For example, the humanized antibody may be an antibody fragment, such as a Fab, which is optionally conj ugated with one or more cytotoxic agent(s) in order to generate an immunoconjugate. Alternatively, the humanized antibody may be an intact antibody, such as an intact IgG1 antibody.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array into such germ-line mutant mice will result in the production ofhuman antibodies upon antigen challenge. See, e.g., Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all ofGenPharm); 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some ofthe properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### 4. Antibody fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Fab and F(ab')₂ fragment with increased in vivo half-life comprising a salvage receptor binding epitope residues are described in U.S. Patent No. 5,869,046. Other techniques for the production of antibody fragments will be apparent to the skilled practitioner. The antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Patent 5,641,870 for example. Such linear antibody fragments may be monospecific or bispecific.

### 5. Bispecific Antibodies

Bispecific antibodies are antibodies that have binding specificities for at least two different epitopes. Exemplary bispecific antibodies may bind to two different epitopes of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 protein as described herein. Other such antibodies may combine a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 binding site with a binding site for another protein. Alternatively, an anti-PRO188, anti-PRO235, anti-PRO266, anti-PRO337, anti-PRO361, anti-PRO539, anti-PRO698, anti-PRO7 17, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 arm may be combined with an arm which binds to a triggering molecule on a leukocyte such as a T-cell receptor molecule (e.g. CD3), or Fc receptors for IgG (FcγR), such as FcγRI (CD64), FcγRII (CD32) and FcγRIII (CD16), so as to focus and localize cellular defense mechanisms to the PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-expressing cell. Bispecific antibodies may also be used to localize cytotoxic agents to cells which express a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide. These antibodiespossess a PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-binding arm and an arm which binds the cytotoxic agent (e.g., saporin, anti-interferon-α, vinca alkaloid, ricin A chain, methotrexate or radioactive isotope hapten). Bispecific antibodies can be prepared as full length antibodies or antibody fragments (e.g., F(ab')₂ bispecific antibodies).

WO 96/16673 describes a bispecific anti-ErbB2/anti-FcγRIII antibody and U.S. Patent No. 5,837,234 discloses a bispecific anti-ErbB2/anti-FcγRI antibody. A bispecific anti-ErbB2/Fcα antibody is shown in WO98/02463. U.S. Patent No. 5,821,337 teaches a bispecific anti-ErbB2/anti-CD3 antibody.

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificity (antibody-antigen combining sites) are fused to immunoglobulin constant domain sequences. Preferably, the fusion is with an Ig heavy chain constant domain, comprising at least part of the hinge, C_{H}2, and C_{H}3 regions. It is preferred to have the first heavy-chain constant region (C_{H}1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

The invention provides bispecific antibodies which are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain. In this method, one or more small amino acid side chains from the interface ofthe first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli*, which can be chemically coupled to form bispecific antibodies. Shalaby et al., J. Exp. Med. 175: 217-225 (1992) describe the production of a fully humanized bispecific antibody F(ab')₂molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V_{H} connected to a V_{L} by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 6. Heteroconjugate Antibodies

Heteroconjugate antibodies are also within the scope ofthe present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples ofsuitable reagents for this purpose include iminothiolate and methyl-4-mercaptobutyrimidate and those disclosed, for example, in U.S. Patent No. 4,676,980.

### 7. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)ₙ-VD2-(X2)ₙ-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 8. Effector Function Engineering

It may be desirable to modify the antibody of the invention with respect to effector function, e.g., so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using beterobifunctional cross-linkers as described in Wolffet al., Cancer Research 53:2560-2565 (1993). Alternatively, an antibody can be engineered which has dual Fc regions and may thereby have enhanced complement lysis and ADCC capabilities. See Stevenson et al., Anti-Cancer Drug Design 3:219-230 (1989). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule.

### 9. Immunoconjugates

The invention also pertains to immunoconjugates comprising an antibody conjugated to a cytotoxic agent such as a chemotherapeutic agent, a growth inhibitory agent, a toxin (*e*.*g*., an enzymatically active toxin of bacterial, fungal, plant, or animal origin, or fragments thereof), or a radioactive isotope (*i*.*e*., a radioconjugate).

Chemotherapeutic agents useful in the generation of such immunoconj ugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothene, and CC1065, and the derivatives of these toxins that have toxin activity, are also contemplated herein.

### Maytansine and maytansinoids

The invention provides an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody (full length or fragments) which is conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533, the disclosures of which are hereby expressly incorporated by reference.

### Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1, the disclosures of which are hereby expressly incorporated by reference. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA.1-maytansonoid conjugate was tested *in vitro* on the human breast cancer cell line SK-BR-3, which expresses 3 x 10⁵ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice.

### Anti-PRO188, Anti-PRO235, Anti-PRO266, Anti-PRO337, Anti-PRO361, Anti-PRO539, Anti-PRO698, Anti-PRO717, Anti-PRO846, Anti-PRO874, Anti-PRO98346, Anti-PRO1082, Anti-PRO1097, Anti-PRO1192, Anti-PRO1268, Anti-PRO1278, Anti-PRO1303, Anti-PRO1308, Anti-PRO1338, Anti-PRO1378, Anti-PRO1415, Anti-PRO1867, Anti-PRO1890, Anti-PRO3438, Anti-PRO19835, Anti-PRO36915, Anti-PRO36029, Anti-PRO4999, Anti-PRO5778, Anti-PRO5997, Anti-PRO6079, Anti-PRO6090, Anti-PRO7178, Anti-PRO21184, Anti-PRO7434, Anti-PRO9822, Anti-PRO9833, Anti-PRO9836, Anti-PRO9854, Anti-PRO9862, Anti-PRO10284, Anti-PRO37510, Anti-PRO35444, Anti-PRO20473, Anti-PRO21054 or Anti-PRO35246 Antibody-Maytansinoid Conjugates (Immunoconjugates)

Anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody-maytansinoid conjugates are prepared by chemically linking an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disufide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. The linkage is formed at the C-3 position of maytansinol or a maytansinol analogue.

### Calicheamicin

Another immunoconjugate of interest comprises an anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody conjugated to one or more calicheamicin molecules. The calicheamicin family of antibiotics are capable ofproducing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, γ₁^{I}, α₂^{I}, α₃^{I}, N-acetyl-γ₁^{I}, PSAG and θ₁^{I}, (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

### Other cytotoxic agents

Other antitumor agents that can be conjugated to the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 oranti-PRO35246 antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcim *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc^{99m} or I¹²³, .Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238:1098(1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

Alternatively, a fusion protein comprising the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

The invention provides that the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

### 10. Immunoliposomes

The anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement ofbiological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 11. Pharmaceutical Compositions of Antibodies

Antibodies specifically binding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide identified herein, as well as other molecules identified by the screening assays disclosed hereinbefore, can be administered for the treatment of various disorders in the form of pharmaceutical compositions.

If the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e*.*g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT ^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### G. Uses forAnti-PRO188, Anti-PRO235, Anti-PRO266, Anti-PRO337, Anti-PRO361, Anti-PRO539, Anti-PRO698, Anti-PRO717, Anti-PRO846, Anti-PRO874, Anti-PRO98346, Anti-PRO1082, Anti-PRO1097, Anti-PRO1192, Anti-PRO1268, Anti-PRO1278, Anti-PRO1303, Anti-PRO1308, Anti-PRO1338, Anti-PRO1378, Anti-PRO1415, Anti-PRO1867, Anti-PRO1890, Anti-PRO3438, Anti-PRO19835, Anti-PRO36915, Anti-PRO36029 Anti-PRO4999, Anti-PRO5778, Anti-PRO5997, Anti-PRO6079, Anti-PRO6090, Anti-PRO7178, Anti-PRO21184, Anti-PRO7434, Anti-PRO9822, Anti-PRO9833, Anti-PRO9836, Anti-PRO9854 Anti-PRO9862, Anti-PRO10284, Anti-PRO37510, Anti-PRO35444, Anti-PRO20473, Anti-PRO21054 or Anti-PRO35246 Antibodies

The anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies of the invention have various therapeutic and/or diagnostic utilities for a neurological disorder; a cardiovascular, endothelial or angiogenic disorder; an immunological disorder; an oncological disorder; an embryonic developmental disorder or lethality, or a metabolic abnormality. For example, anti-PRO18 8, anti-PRO235, anti-PRO266, anti-PRO337, anti-PRO3 61, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies may be used in diagnostic assays for PRO188, PRO235, PRO266, PRO337, PRO361, PRO539, PRO698, PRO717, PRO846, PRO874, PRO98346, PRO1082, PRO1097, PRO1192, PRO1268 PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246, e.g., detecting its expression (and in some cases, differential expression) in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem, and Cytochem., 30:407 (1982).

Anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies also are useful for the affinity purification of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides from recombinant cell culture or natural sources. In this process, the antibodies against PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides are immobilized on a suitable support, such a Sephadex resin or filter paper, using methods well known in the art. The immobilized antibody then is contacted with a sample containing the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide to be purified, and thereafter the support is washed with a suitable solvent that will remove substantially all the material in the sample except the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, which is bound to the immobilized antibody. Finally, the support is washed with another suitable solvent that will release the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide from the antibody.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e*.*g*., Dayhoff, GenBank), and proprietary databases (e.g. LIFESEQ^{™}, Incyte Pharmaceuticals, Palo Alto, CA). The search was performed using the computer program BLAST or BLAST-2 (Altschul et al., Methods in Enzymology, 266:460-480 (1996)) as a comparison of the ECD protein sequences to a 6 frame translation of the EST sequences. Those comparisons with a BLAST score of 70 (or in some cases 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defmed orientation into a suitable cloning vector (such as pRKB or pRKD; pRK5B is a precursor of pRK5D that does not contain the SfiI site; *see*, Holmes et al., Science, 253:1278-1280 (1991)) in the unique XhoI and NotI sites.

### EXAMPLE 2: Isolation of cDNA clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, e.g. CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11, his3-15, MAL⁺, SUC⁺, GAL⁺. Preferably, yeast mutants can be employed that have deficient post-translational pathways. Such mutants may have translocation deficient alleles in *sec*71, *sec*72, *sec62,* with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OCCH₃), and resuspended into LiAc/TE (2.5 ml).

Transformation took place by mixing the prepared cells (100 µl) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl, 1 mM EDTA, 100 mM Li₂OOCCH₃, pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCl, 1 mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was:
5'-TGTAAAACGACGGCCAGTTAAATAGACCTGCAATTATTAATCT-3' (SEQ ID NO:93) The sequence of reverse oligonucleotide 2 was:
5'-CAGGAAACAGCTATGACCACCTGCACACCTGCAAATCCATT-3' (SEQ ID NO:94) PCR was then performed as follows:

| | | | | |
|---|---|---|---|---|
| a. | | Denature | 92°C, | 5 minutes |
| | | | | |
| b. | 3 cycles of: | Denature | 92°C, | 30 seconds |
| | | Anneal | 59°C, | 30 seconds |
| | | Extend | 72°C, | 60 seconds |
| | | | | |
| c. | 3 cycles of: | Denature | 92°C, | 30 seconds |
| | | Anneal | 57°C, | 30 seconds |
| | | Extend | 72°C, | 60 seconds |
| | | | | |
| d. | 25 cycles of: | Denature | 92°C, | 30 seconds |
| | | Anneal | 55°C, | 30 seconds |
| | | Extend | 72°C, | 60 seconds |
| | | | | |
| e. | | Hold | 4°C | |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl) was examined by agarose gel electrophoresis in a 1 % agarose gel using aTris-Borate-EDTA (TBE) buffering system as described by Sambrook *et al., supra.* Clones resulting in a single strong PCR product larger than 400 bp were further analyzed by DNA sequencing after purification with a 96 Qiaquick PCR clean-up column (Qiagen Inc., Chatsworth, CA).

### EXAMPLE 3: Isolation of cDNA Clones Using Signal Algorithm Analysis

Various polypeptide-encoding nucleic acid sequences were identified by applying a proprietary signal sequence finding algorithm developed by Genentech, Inc. (South San Francisco, CA) upon ESTs as well as clustered and assembled EST fragments from public (*e*.*g*., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

Using the techniques described in Examples 1 to 3 above, numerous full-length cDNA clones were identified as encoding PRO188, PRO235, PRO266, PRO337, PRO361, PRO539, PRO698, PRO717, PRO846, PRO874, PRO1082, PRO1097, PRO1192, PRO1268, PRO1278, PRO1303, PRO1308, PRO1338, PRO1378, PRO1415, PRO1867, PRO1890, PRO3438, PRO4999, PRO5778, PRO5997, PRO6079, PRO6090, PRO7178, PRO21184, PRO7434, PRO9822, PRO9833, PRO9836, PRO9854, PRO9862, PRO10284, PRO20473, or PRO21054 polypeptides as disclosed herein. These cDNAs were then deposited under the terms of the Budapest Treaty with the American Type Culture Collection, 10801 University Blvd., Manassas, VA 20110-2209, USA (ATCC) as shown in Table 7 below. In addition, the sequence of DNA349738 encoding PRO98346 polypeptides was identified from GenBank accession no.: BC021104; the sequence of DNA164647 encoding PRO19835 polypeptides was identified from GenBank accession no.: AX207207; the sequence of DNA226452 encoding PRO36915 polypeptides was identified from GenBank accession no.: M20681; the sequence of DNA225566 encoding PRO36029 polypeptides was identified from GenBank accession no.: AF061741; the sequence of DNA227047 encoding PRO37510 polypeptides was identified from GenBank accession no.: U2503 3; the sequence of DNA222653 encoding PRO35444 polypeptides was identified from GenBank accession no.: AX574576; and the sequence of DNA129618 encoding PRO35246 polypeptides was identified from GenBank accession no.: NM_018676.

**Table 7**

| Material | ATCC Dep. No. | Deposit Date |
|---|---|---|
| DNA28497-1130 | 209279 | September 18, 1997 |
| DNA35558-1167 | 209374 | October 16, 1997 |
| DNA37150-1178 | 209401 | October 17, 1997 |
| DNA43316-1237 | 209487 | November 21, 1997 |
| DNA45410-1250 | 209621 | February 5, 1998 |
| DNA47465-1561 | 203661 | February 9, 1999 |
| DNA48320-1433 | 209904 | May 27, 1998 |
| DNA50988-1326 | 209814 | April 28, 1998 |
| DNA44196-1353 | 209847 | May 6, 1998 |
| DNA40621-1440 | 209922 | June 2, 1998 |
| DNA53912-1457 | 209870 | May 14, 1998 |
| DNA59841-1460 | 203044 | July 1, 1998 |
| DNA62814-1521 | 203093 | August 4, 1998 |
| DNA66519-1535 | 203236 | September 15, 1998 |
| DNA66304-1546 | 203321 | October 6, 1998 |
| DNA65409-1566 | 203232 | September 15, 1998 |
| DNA62306-1570 | 203254 | September 9, 1998 |
| DNA66667-1596 | 203267 | September 22, 1998 |
| DNA58730-1607 | 203221 | September 15, 1998 |
| DNA58852-1637 | 203271 | September 22, 1998 |
| DNA84925-2514 | 203548 | December 22, 1998 |
| DNA79230-2525 | 203549 | December 22, 1998 |
| DNA82364-2538 | 203603 | January 20, 1999 |
| DNA96031-2664 | PTA-237 | June 15, 1999 |
| DNA96894-2675 | PTA-260 | June 22, 1999 |
| DNA97005-2687 | PTA-378 | July 20, 1999 |
| DNA111750-2706 | PTA-489 | August 3, 1999 |
| DNA107781-2707 | PTA-484 | August 3, 1999 |
| DNA108789-2748 | PTA-547 | August 17, 1999 |
| DNA167678-2963 | PTA-2302 | July 25, 2000 |
| DNA123430-2755 | PTA-614 | August 31, 1999 |
| DNA108738-2767 | PTA-862 | October 19, 1999 |
| DNA130809-2769 | PTA-949 | November 9, 1999 |
| DNA119514-2772 | PTA-946 | November 9, 1999 |
| DNA108771-2776 | PTA-948 | November 9, 1999 |
| DNA125148-2782 | PTA-955 | November 16, 1999 |
| DNA138039-2828 | PTA-1343 | February 8, 2000 |
| DNA163134-2917 | PTA-1842 | May 9, 2000 |
| DNA143501-2922 | PTA-1908 | May 23, 2000 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

### EXAMPLE 4: Isolation of cDNA clones Encoding Human PRO188 Polypeptides [UNQ162]

An expressed sequence tag (EST) DNA database ( LIFESEQ^{®}, Incyte Pharmaceuticals, Palo Alto, CA) was searched and an EST was identified which showed homology to the human TIE ligand family.

RNA for construction of cDNA libraries was then isolated from human fetal lung tissue. The cDNA libraries used to isolate the cDNA clones encoding human PRO188 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defmed orientation into a suitable cloning vector (such as pRKB or pRKD; pRK5B is a precursor of pRKSD that does not contain the SfiI site; *see*, Holmes *et al*., Science, 253:1278-1280 (1991)) in the unique XhoI and NotI.

Oligonucleotides probes based upon the above described EST sequence were then synthesized: 1) to identify by PCR a cDNA library that contained the sequence of interest, and 2) for use as probes to isolate a clone of the full-length coding sequence for PRO188. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra*, with the PCR primer pair. A positive library was then used to isolate clones encoding the gene of interest using the probe oligonucleotide and one of the primer pairs.

The oligonucleotide probes employed were as follows:
NL5.5-1:
   5'-CAGGTTATCCCAGAGATTTAATGCCACCA-3' (SEQ ID NO:95)
NL5.3-1:
   5'-TTGGTGGGAGAAGTTGCCAGATCAGGTGGTGGCA-3' (SEQ ID NO:96)
NL5.3-2:
   5'TTCACACCATAACTGCATTGGTCCA-3' (SEQ ID NO:97)

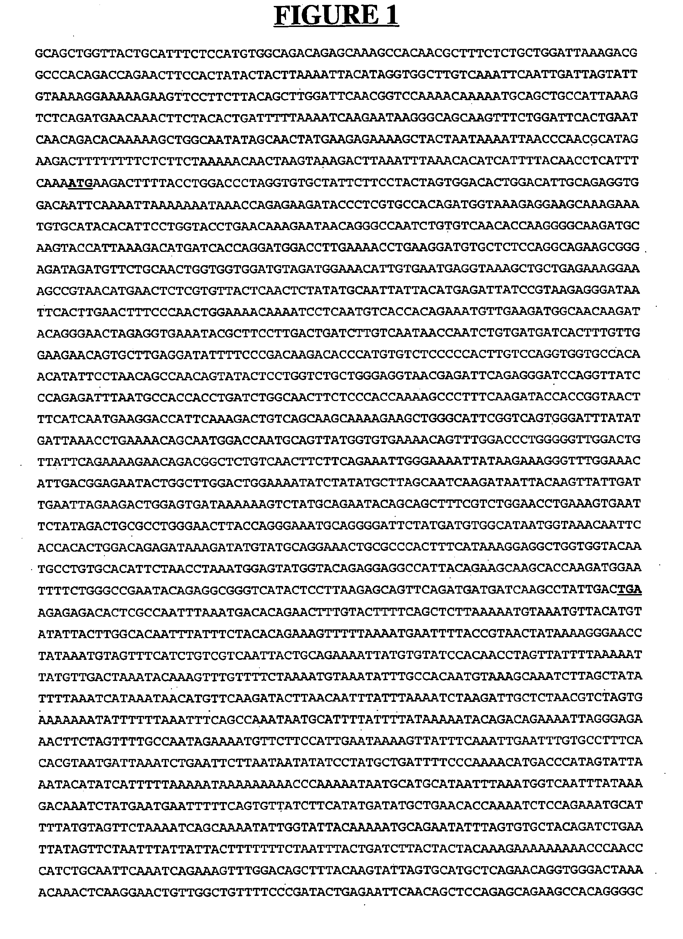

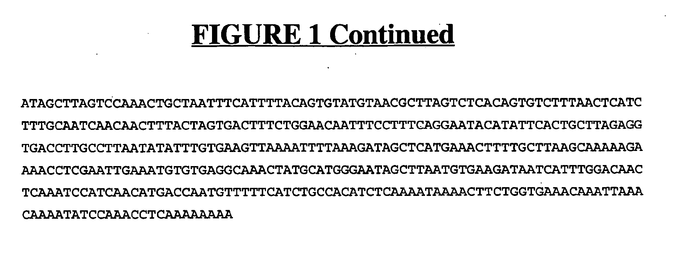

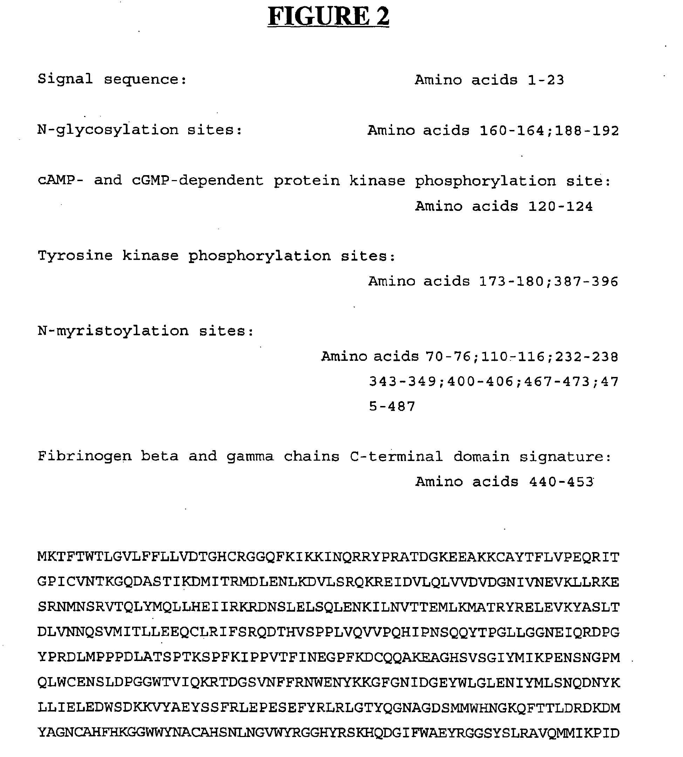

A full length clone [DNA28497-1130] was identified that contained a single open reading frame with an apparent translational initiation site at nucleotide positions 449-451 and a stop signal at nucleotide positions 1922-1924 (Figure 1, SEQ ID NO:1). The predicted polypeptide precursor is 491 amino acids long, and has a calculated molecular weight of approximately 56,720 daltons and an estimated pI of approximately 8.56. Analysis of the full-length PRO188 sequence shown in Figure 2 (SEQ ID NO:2) evidences the presence of a variety of important polypeptide domains as shown in Figure 2, wherein the locations given for those important polypeptide domains are approximate as described above. Analysis of the full-length PRO188 polypeptide shown in Figure 2 evidences the presence of the following: a signal peptide from about amino acid 1 to about amino acid 23; N-glycosylation sites from about amino acid 160 to about amino acid 164, and from about amino acid 188 to about amino acid 192; a cAMP- and cGMP-dependent protein kinase phosphorylation site from about amino acid 120 to about amino acid 124; tyrosine kinase phosphorylation sites from about amino acid 173 to about amino acid 180, and from about amino acid 387 to about amino acid 396; N-myristoylation sites from about amino acid 70 to about amino acid 76, from about amino acid 110 to about amino acid 116, from about amino acid 232 to about amino acid 238, from about amino acid 343 to about amino acid 349, from about amino acid 400 to about amino acid 406, from about amino acid 467 to about amino acid 473, and from about amino acid 475 to about amino acid 487; and a fibrinogen beta and gamma chains C-terminal domain signature from about amino acid 440 to about amino acid 453. Clone DNA28497-1130 has been deposited with ATCC on September 18, 1997 and is assigned ATCC deposit no. 209279.

Based on a BLAST and FastA sequence alignment analysis of the full-length sequence shown in Figure 2 (SEQ ID NO:2), PRO188 (herein designated NL5) shows 24% amino acid sequence identity to both ligand 1 and ligand 2 of the TIE2 receptor. Ligand 1 and ligand 2 of the TIE-2 receptor are 64% identical and 40-43% identical, respectively, to PRO188. The abbreviation "TIE" is an acronym which stands for "**t**yrosine kinase containing **I**g and **E**GF homology domains" and was coined to designate a new family of receptor tyrosine kinases.

### EXAMPLE 5: Isolation of cDNA clones Encoding Human PRO235 Polypeptides [UNQ209]

A consensus DNA sequence was assembled relative to other EST sequences using phrap as described in Example 1 above. This consensus sequence is herein designated "DNA30927". Based on the DNA30927 consensus sequence, oligonucleotides were synthesized: 1) to identify by PCR a cDNA library that contained the sequence of interest, and 2) for use as probes to isolate a clone of the full-length coding sequence for PRO235.

A pair of PCR primers (forward and reverse) were synthesized:
forward PCR primer 5'-TGGAATACCGCCTCCTGCAG-3' (SEQ ID NO:98)
reverse PCR primer 5'-CTTCTGCCCTTTGGAGAAGATGGC-3' (SEQ ID NO:99) Additionally, a synthetic oligonucleotide hybridization probe was constructed from the consensus DNA30927 sequence which had the following nucleotide sequence
hybridization probe
   5'-GGACTCACTGGCCCAGGCCTTCAATATCACCAGCCAGGACGAT-3' (SEQ ID NO:100)

In order to screen several libraries for a source of a full-length clone, DNA from the libraries was screened by PCR amplification with the PCR primer pair identified above. A positive library was then used to isolate clones encoding the PRO235 gene using the probe oligonucleotide and one of the PCR primers.

RNA for construction of the cDNA libraries was isolated from human fetal liver tissue.

DNA sequencing of the clones isolated as described above gave the full-length DNA sequence for PRO235 [herein designated as DNA35558-1167] (SEQ ID NO:3) and the derived protein sequence for PRO235.

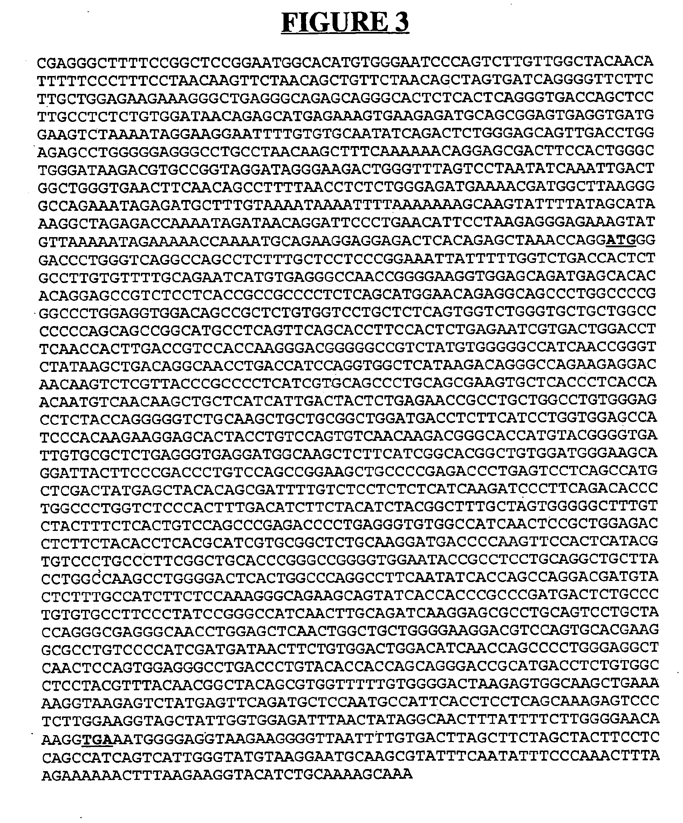

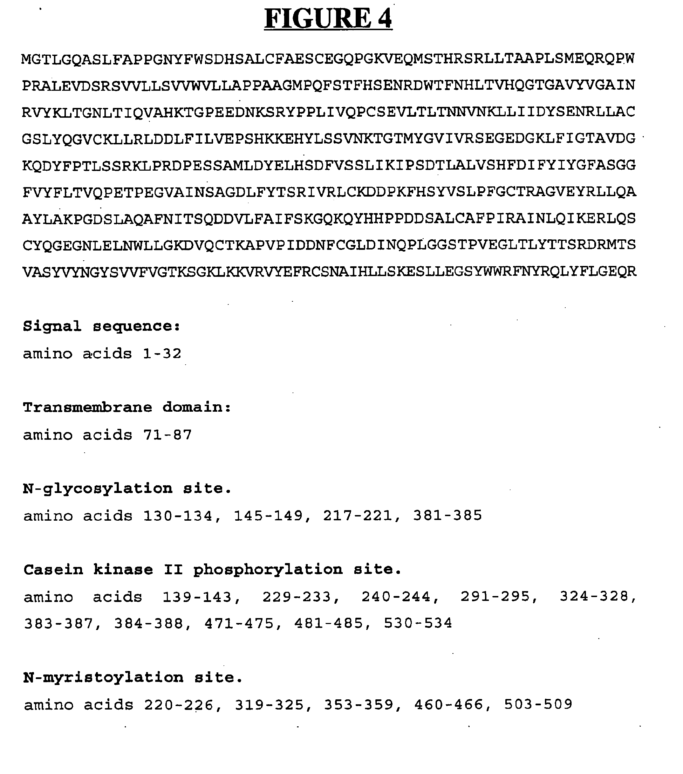

The entire nucleotide sequence of DNA35558-1167 is shown in Figure 3 (SEQ ID NO:3). Clone DNA35558-1167 contains a single open reading frame with an apparent translational initiation site at nucleotide positions 667-669 and ending at the stop codon at nucleotide positions 2323-2325 (Figure 3). The predicted polypeptide precursor is 552 amino acids long (Figure 4; SEQ ID NO:4). Clone DNA35558-1167 has been deposited with ATCC on October 16, 1997and is assigned ATCC deposit no. 209374.

Analysis of the amino acid sequence of the full-length PRO235 polypeptide suggests that portions of it possess significant homology to the human, mouse and Xenopus plexin protein, thereby indicating that PRO235 may be a novel plexin protein.

### EXAMPLE 6: Isolation of cDNA clones Encoding Human PRO266 Polypeptides [UNQ233]

An expressed sequence tag database was searched for ESTs having homology to SLIT, resulting in the identification of a single EST sequence designated herein as T73996. Based on the T73996 EST sequence, oligonucleotides were synthesized: 1) to identify by PCR a cDNA library that contained the sequence of interest, and 2) for use as probes to isolate a clone of the full-length coding sequence for PRO266.

A pair of PCR primers (forward and reverse) were synthesized:
forward PCR primer 5'-GTTGGATCTGGGCAACAATAAC-3' (SEQ ID NO:101)
reverse PCR primer 5'-ATTGTTGTGCAGGCTGAGTTTAAG-3' (SEQ ID NO:102) Additionally, a synthetic oligonucleotide hybridization probe was constructed which had the following nucleotide sequence
hybridization probe
   5'-GGTGGCTATACATGGATAGCAATTACCTGGACACGCTGTCCCGGG-3' (SEQ ID NO:103)

In order to screen several libraries for a source of a full-length clone, DNA from the libraries was screened by PCR amplification with the PCR primer pair identified above. A positive library was then used to isolate clones encoding the PRO266 gene using the probe oligonucleotide and one of the PCR primers.

RNA for construction ofthe cDNA libraries was isolated from human fetal brain tissue. DNA sequencing of the clones isolated as described above gave the full-length DNA sequence for PRO266 [herein designated as DNA37150-1178] (SEQ ID NO:5) and the derived protein sequence for PRO266.

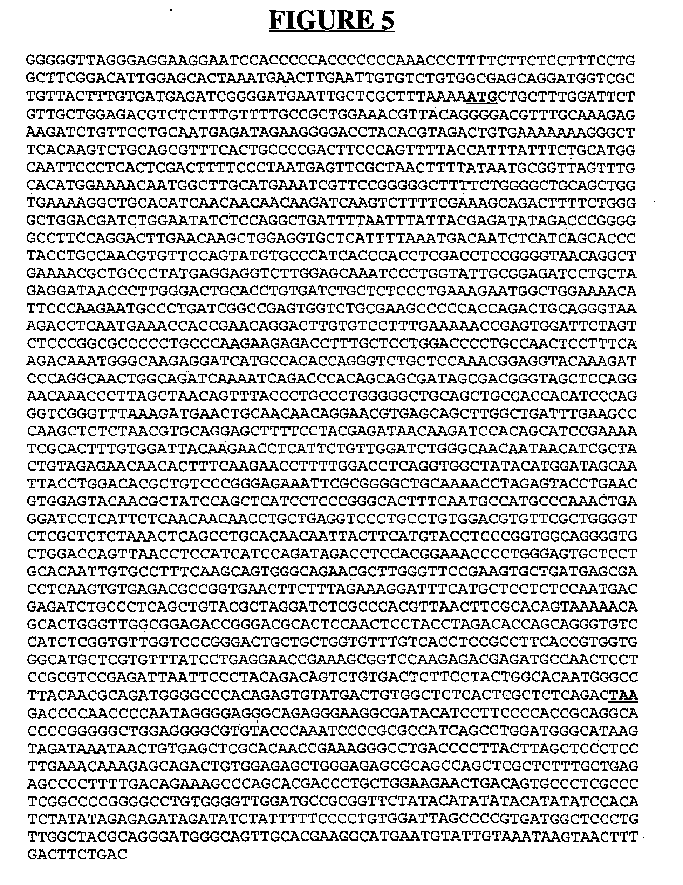

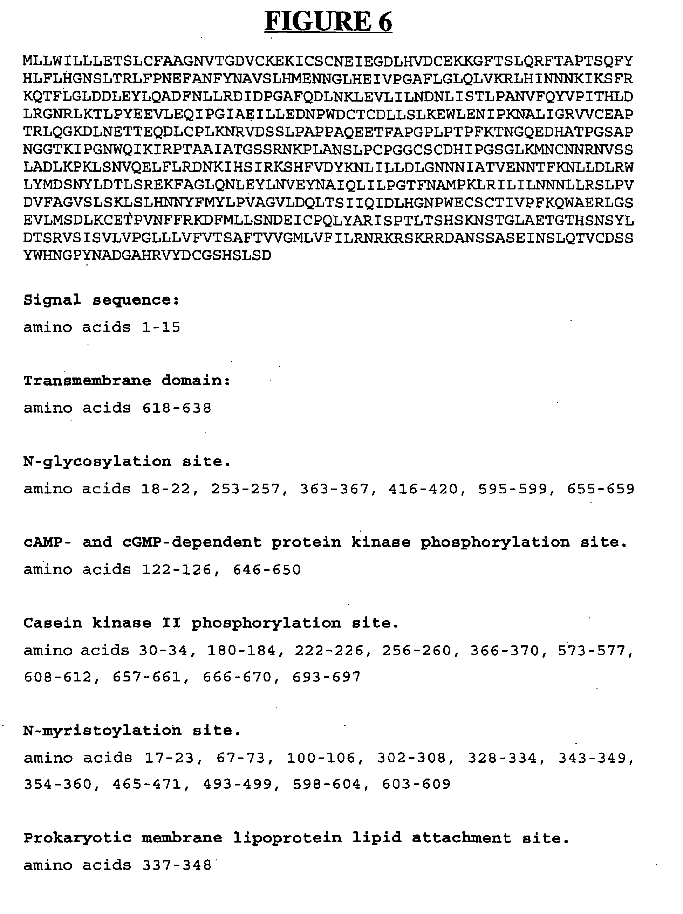

The entire nucleotide sequence of DNA37150-1178 is shown in Figure 5 (SEQ ID NO:5). Clone DNA37150-1178 contains a single open reading frame with an apparent translational initiation site at nucleotide positions 167-169 and ending at the stop codon after nucleotide position 2254 of SEQ ID NO:5. The predicted polypeptide precursor is 696 amino acids long (Figure 6; SEQ ID NO:6). Clone DNA37150-1178 has been deposited with ATCC on October 17, 1997and is assigned ATCC deposit no. ATCC 209401.

Analysis of the amino acid sequence of the full-length PRO266 polypeptide suggests that portions of it possess significant homology to the SLIT protein, thereby indicating that PRO266 may be a novel leucine rich repeat protein.

### EXAMPLE 7: Isolation of cDNA clones Encoding Human PRO337 Polypeptides [UNQ297]

A cDNA sequence identified in the amylase screen described in Example 2 above is herein designated DNA42301. The DNA42301 sequence was then compared to other EST sequences using phrap as described in Example 1 above and a consensus sequence designated herein as DNA28761 was identified. Based on this consensus sequence, oligonucleotides were synthesized: 1) to identify by PCR a cDNA library that contained the sequence of interest, and 2) for use as probes to isolate a clone of the full-length coding sequence. In order to screen several libraries for a source of a full-length clone, DNA from the libraries was screened by PCR amplification with the PCR primer pair identified above. A positive library was then used to isolate clones encoding the PRO337 gene using the probe oligonucleotide and one of the PCR primers. RNA for construction of the cDNA libraries was isolated from human fetal brain.

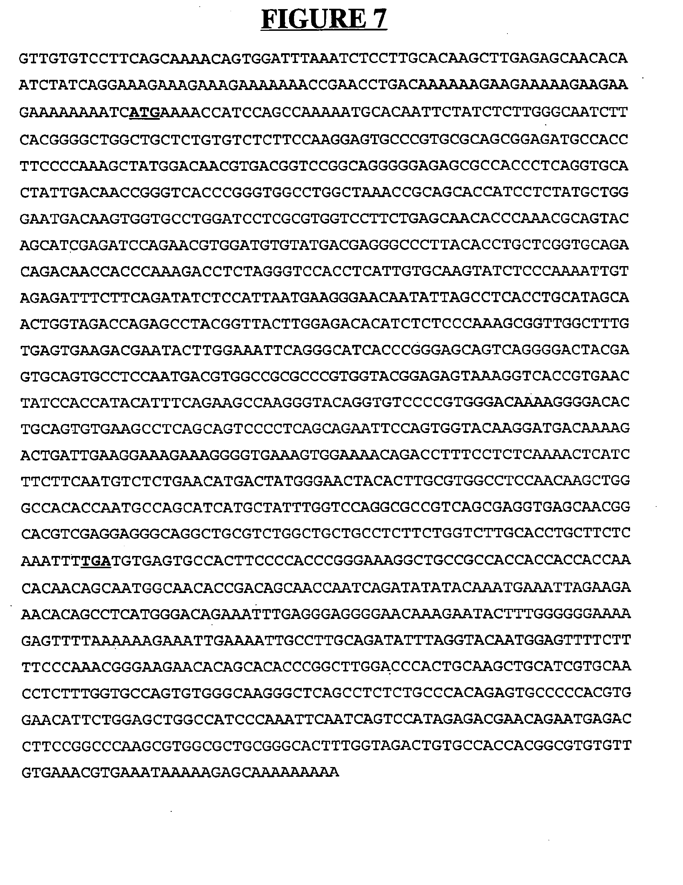

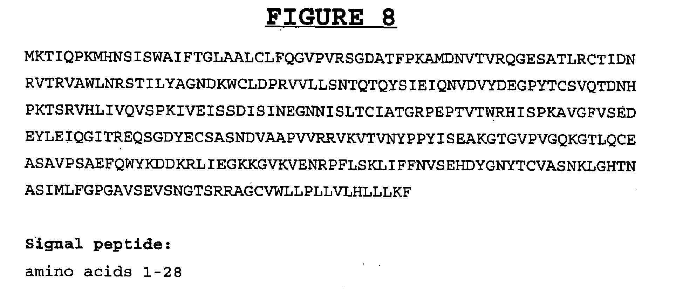

A cDNA clone was sequenced in its entirety. The full length nucleotide sequence of DNA43316-1237 is shown in Figure 7 (SEQ ID NO:7). Clone DNA43316-1237 contains a single open reading frame with an apparent translational initiation site at nucleotide positions 134-136 (Figure 7; SEQ ID NO:7). The predicted polypeptide precursor is 344 amino acids long (Figure 8; SEQ ID NO:8). Clone DNA43316-1237 has been deposited with ATCC on November 21, 1997and is assigned ATCC deposit no. 209487.

Based on a BLAST-2 and FastA sequence alignment analysis of the full-length sequence, PRO337 shows amino acid sequence identity to rat neurotrimin (97%).

### EXAMPLE 8: Isolation of cDNA clones Encoding Human PRO361 Polypeptides [UNQ316]

A consensus DNA sequence was assembled relative to other EST sequences using phrap as described in Example 1 above. This consensus sequence is herein designated DNA40654. Based on the DNA40654 consensus sequence, oligonucleotides were synthesized: 1) to identify by PCR a cDNA library that contained the sequence of interest, and 2) for use as probes to isolate a clone of the full-length coding sequence for PRO361.

Forward and reverse PCR primers were synthesized as follows:
forward PCR primer 5'-AGGGAGGATTATCCTTGACCTTTGAAGACC-3' (SEQ ID NO:104)
forward PCR primer 5'-GAAGCAAGTGCCCAGCTC-3' (SEQ ID NO:105)
forward PCR primer 5'-CGGGTCCCTGCTCTTTGG-3' (SEQ ID NO:106)
reverse PCR primer 5'-CACCGTAGCTGGGAGCGCACTCAC-3' (SEQ ID NO:107)
reverse PCR primer 5'-AGTGTAAGTCAAGCTCCC-3' (SEQ ID NO:108) Additionally, a synthetic oligonucleotide hybridization probe was constructed from the consensus DNA40654 sequence which had the following nucleotide sequence
hybridization probe
   5'- GCTTCCTGACACTAAGGCTGTCTGCTAGTCAGAATTGCCTCAAAAAGAG-3' (SEQ ID NO:109)

In order to screen several libraries for a source of a full-length clone, DNA from the libraries was screened by PCR amplification with one of the PCR primer pairs identified above. A positive library was then used to isolate clones encoding the PRO361 gene using the probe oligonucleotide. RNA for construction of the cDNA libraries was isolated from human fetal kidney tissue.

DNA sequencing of the clones isolated as described above gave the full-length DNA sequence for PRO361 [herein designated as DNA45410-1250] (SEQ ID NO:9) and the derived protein sequence for PRO361.

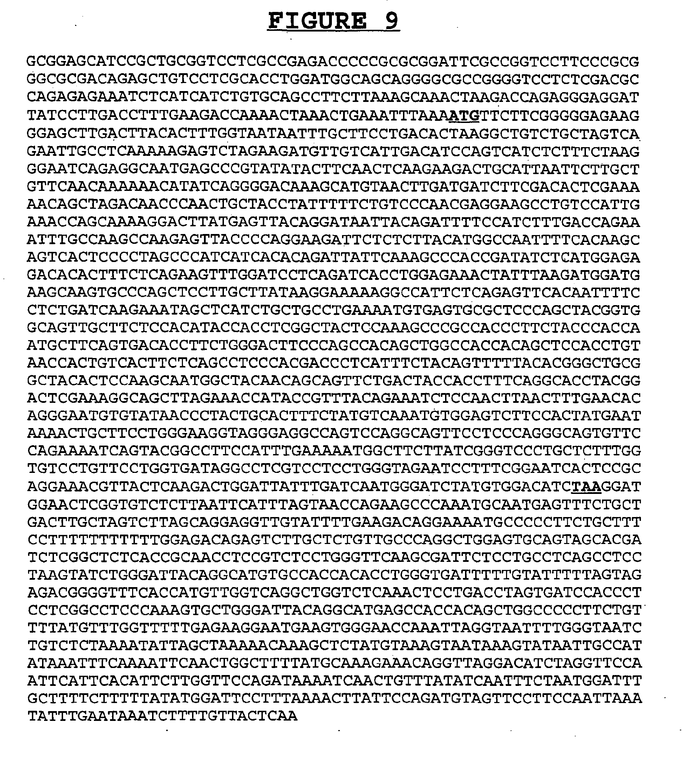

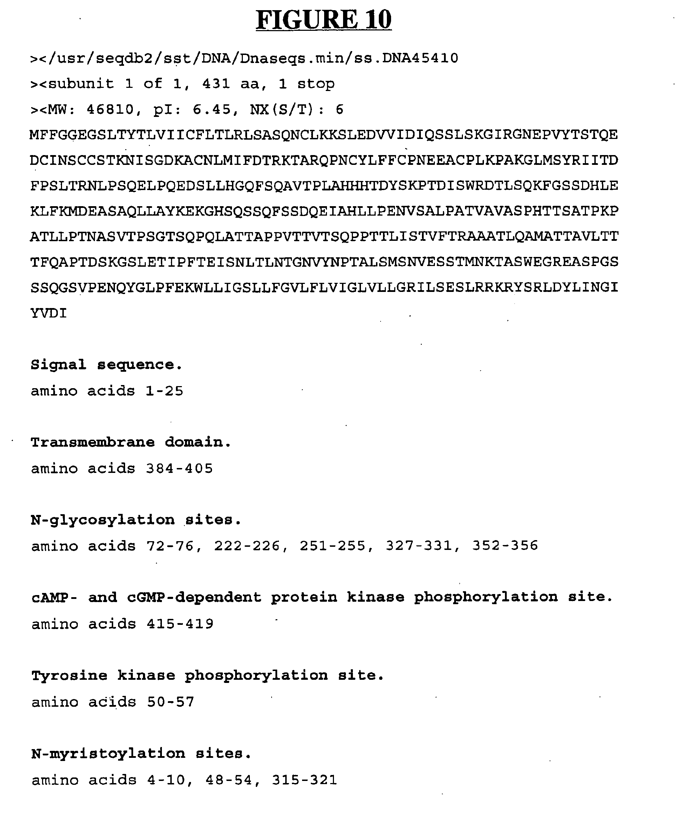

The entire nucleotide sequence of DNA45410-1250 is shown in Figure 9 (SEQ ID NO:9). Clone DNA45410-1250 contains a single open reading frame with an apparent translational initiation site at nucleotide positions 226-228 and ending at the stop codon at nucleotide positions 1519-1521 (Figure 9). The predicted polypeptide precursor is 431 amino acids long (Figure 10; SEQ ID NO:10). The full-length PRO361 protein shown in Figure 10 has an estimated molecular weight of about 46,810 daltons and a pI of about 6.45. In addition, regions of interest including the transmembrane domain (amino acids 380-409) and sequences typical of the arginase family of proteins (amino acids 3-14 and 39-57) are designated in Figure 10. Clone DNA45410-1250 has been deposited with ATCC on February 5, 1998 and is assigned ATCC deposit no. ATCC 209621.

Analysis of the amino acid sequence of the full-length PRO361 polypeptide suggests that portions of it possess significant homology to the mucin and/or chitinase proteins, thereby indicating that PRO361 may be a novel mucin and/or chitinase protein.

### EXAMPLE 9: Isolation of cDNA clones Encoding Human PRO539 Polypeptides [UNQ340]

A consensus DNA sequence was assembled relative to other EST sequences using phrap as described in Example 1. This consensus sequence is herein designated DNA41882. Based on the DNA41882 consensus sequence, oligonucleotides were synthesized: 1) to identify by PCR a cDNA library that contained the sequence of interest, and 2) for use as probes to isolate a clone of the full-length coding sequence for PRO539.

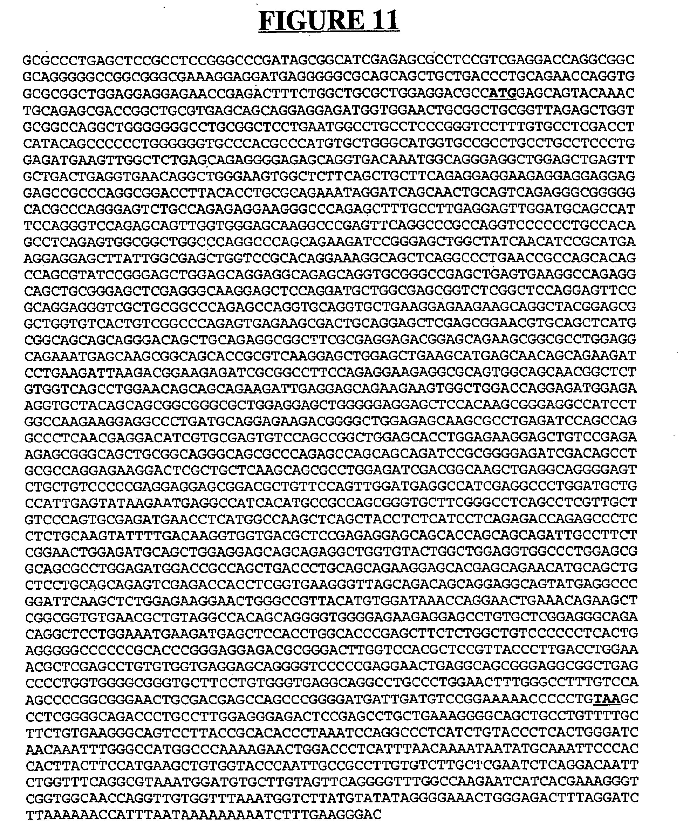

RNA for construction of the cDNA libraries was isolated from human fetal kidney tissue. DNA sequencing of the clones isolated as described above gave the full-length DNA sequence for PRO539 (designated herein as DNA47465-1561 [Figure 11, SEQ ID NO:11]; and the derived protein sequence for PRO539.

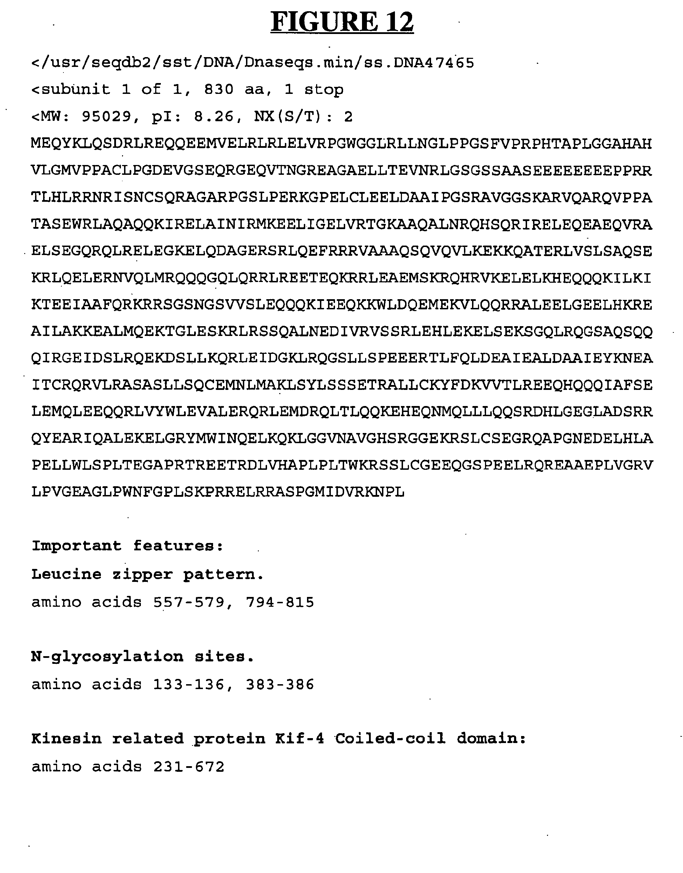

The entire nucleotide sequence of DNA47465-1561 is shown in Figure 11 (SEQ ID NO:11). Clone DNA47465-1561 contains a single open reading frame with an apparent translational initiation site at nucleotide positions 186-188 and ending at the stop codon at nucleotide positions 2676-2678 (Figure 11). The predicted polypeptide precursor is 830 amino acids long (Figure 12). The full-length PRO539 protein shown in Figure 12 has an estimated molecular weight of about 95,029 daltons and a pI of about 8.26. Analysis of the full-length PRO539 sequence shown in Figure 12 (SEQ ID NO:12) evidences the presence of the following: leucine zipper pattern sequences from about amino acid 557 to about amino acid 578 and from about amino acid 794 to about amino acid 815, potential N-glycosylation sites from about amino acid 133 to about amino acid 13 6 and from about amino acid 383 to about amino acid 386 and a kinesin-related protein Kif-4 coiled coil domain from about amino acid 231 to about amino acid 672. Clone DNA47465-1561 has been deposited with ATCC on February 9, 1999 and is assigned ATCC deposit no. 203661.

An analysis of the Dayhoff database (version 35.45 SwissProt 35), using a WU-BLAST2 sequence alignment analysis of the full-length sequence shown in Figure 12 (SEQ ID NO:12), evidenced homology between the PRO539 amino acid sequence and the following Dayhoff sequences: AF019250_1, KIF4_MOUSE, TRHY_HUMAN, A56514, G02520, MYSP_HUMAN, AF041382_1, A45592, HS125H2_1 and HS68O2_2.

### EXAMPLE 10: Isolation of cDNA clones Encoding Human PRO698 Polypeptides [UNQ362]

A yeast screening assay was employed to identify cDNA clones that encoded potential secreted proteins. Use of this yeast screening assay allowed identification of single cDNA clone whose sequence (herein designated as DNA39906). Based on the DNA39906 sequence, oligonucleotides were synthesized: 1) to identify by PCR a cDNA library that contained the sequence of interest, and 2) for use as probes to isolate a clone of the full-length coding sequence for PRO698. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

PCR primers (forward and reverse) were synthesized:
forward PCR primer 5'-AGCTGTGGTCATGGTGGTGTGGTG-3' (SEQ ID NO:110)
reverse PCR primer 5'-CTACCTTGGCCATAGGTGATCCGC-3' (SEQ ID NO:111) Additionally, a synthetic oligonucleotide hybridization probe was constructed from the consensus DNA39906 sequence which had the following nucleotide sequence
hybridization probe
   5'-CATCAGCAAACCGTCTGTGGTTCAGCTCAACTGGAGAGGGTT-3' (SEQ ID NO:112)

In order to screen several libraries for a source of a full-length clone, DNA from the libraries was screened by PCR amplification with the PCR primer pair identified above. A positive library was then used to isolate clones encoding the PRO698 gene using the probe oligonucleotide and one of the PCR primers. RNA for construction of the cDNA libraries was isolated from human bone marrow tissue (LIB255). The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defmed orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

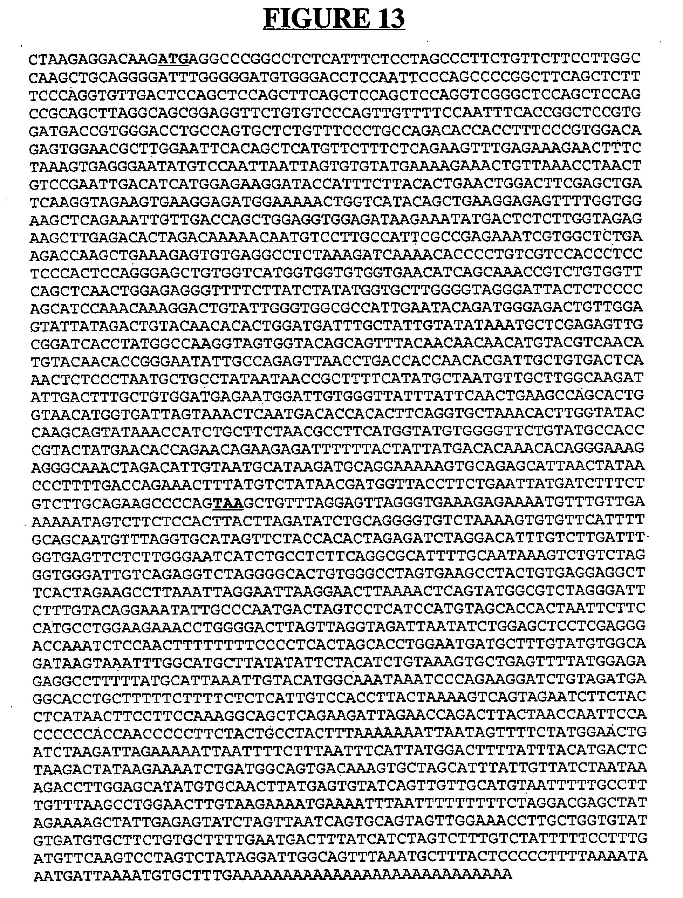

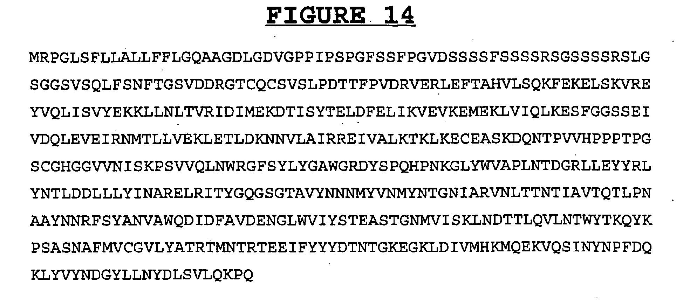

A full length clone was identified that contained a single open reading frame with an apparent translational initiation site at nucleotide positions 14-16 and ending at the stop codon found at nucleotide positions 1544-1546 (Figure 13, SEQ ID NO:13). The predicted polypeptide precursor is 510 amino acids long, has a calculated molecular weight of approximately 57,280 daltons and an estimated pI of approximately 5.61. Analysis of the full-length PRO698 sequence shown in Figure 14 (SEQ ID NO:14) evidences the presence of the following: a signal peptide from about amino acid 1 to about amino acid 20, potential N-glycosylation sites from about amino acid 72 to about amino acid 75, from about amino acid 136 to about amino acid 139, from about amino acid 193 to about amino acid 196, from about amino acid 253 to about amino acid 256, from about amino acid 352 to about amino acid 355 and from about amino acid 411 to about amino acid 414 an amino acid block having homology to legume lectin beta-chain proteins from about amino acid 20 to about amino acid 39 and an amino acid block having homology to the HBGF/FGF family of proteins from about amino acid 338 to about amino acid 365. Clone DNA48320-1433 has been deposited with ATCC on May 27, 1998 and is assigned ATCC deposit no. 209904.

Analysis of the amino acid sequence of the full-length PRO698 polypeptide suggests that it possesses significant sequence similarity to the olfactomedin protein, thereby indicating that PRO698 may be a novel olfactomedin homolog. More specifically, an analysis of the Dayhoff database (version 35.45 SwissProt 35) evidenced significant homology between the PRO698 amino acid sequence and the following Dayhoff sequences, OLFM_RANCA, I73637, AB006686S3_1, RNU78105_1, RNU72487_1, P_R98225, CELC48E7_4, CEF11C3_3, XLU85970_1 and S42257.

### EXAMPLE 11: Isolation of cDNA clones Encoding Human PRO717 Polypeptides [UNQ385]

A consensus sequence was obtained relative to a variety of EST sequences as described in Example 1 above, wherein the consensus sequence obtained is herein designated DNA42829. Based on the DNA42829 consensus sequence, oligonucleotides were synthesized: 1) to identify by PCR a cDNA library that contained the sequence of interest, and 2) for use as probes to isolate a clone of the full-length coding sequence for PRO717.

A pair of PCR primers (forward and reverse) were synthesized:
forward PCR primer 5'-AGCTTCTCAGCCCTCCTGGAGCAG-3' (SEQ ID NO:113);
reverse PCR primer 5'-CGGGTCAATAAACCTGGACGCTTGG-3' (SEQ ID NO:114).
Additionally, a synthetic oligonucleotide hybridization probe was constructed from the DNA42829 consensus sequence which had the following nucleotide sequence:
hybridization probe 5'-TATGTGGACCGGACCAAGCACTTCACTGAGGCCACCAAGATTG-3' (SEQ ID NO:115).

In order to screen several libraries for a source of a full-length clone, DNA from the libraries was screened by PCR amplification with the PCR primer pair identified above. A positive library was then used to isolate clones encoding the PRO717 gene using the probe oligonucleotide and one of the PCR primers. RNA for construction of the cDNA libraries was isolated from human fetal liver tissue (LIB229).

DNA sequencing of the clones isolated as described above gave the full-length DNA sequence for PRO717 [herein designated as UNQ385 (DNA50988-1326)] (SEQ ID NO:15) and the derived protein sequence for PRO717.

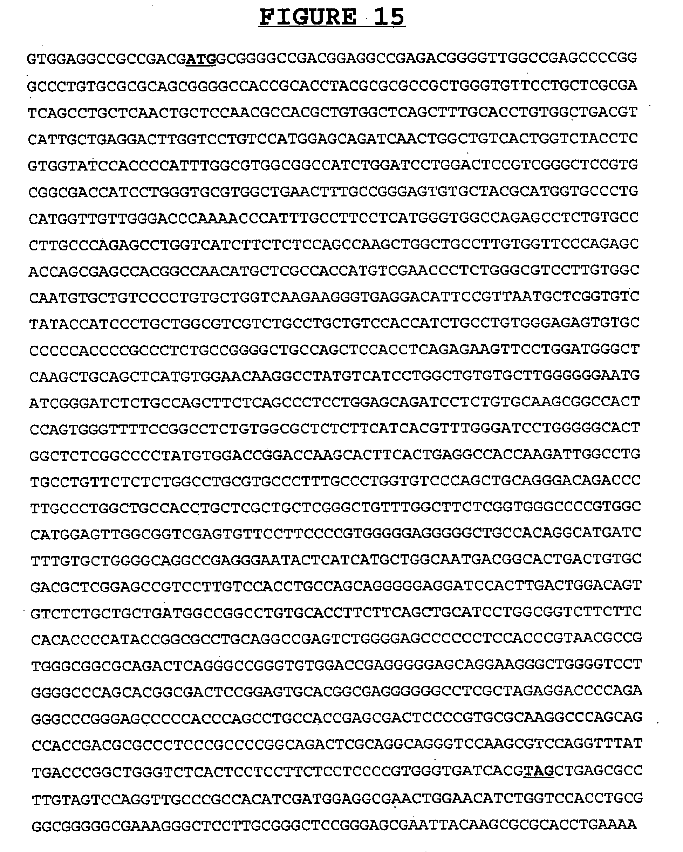

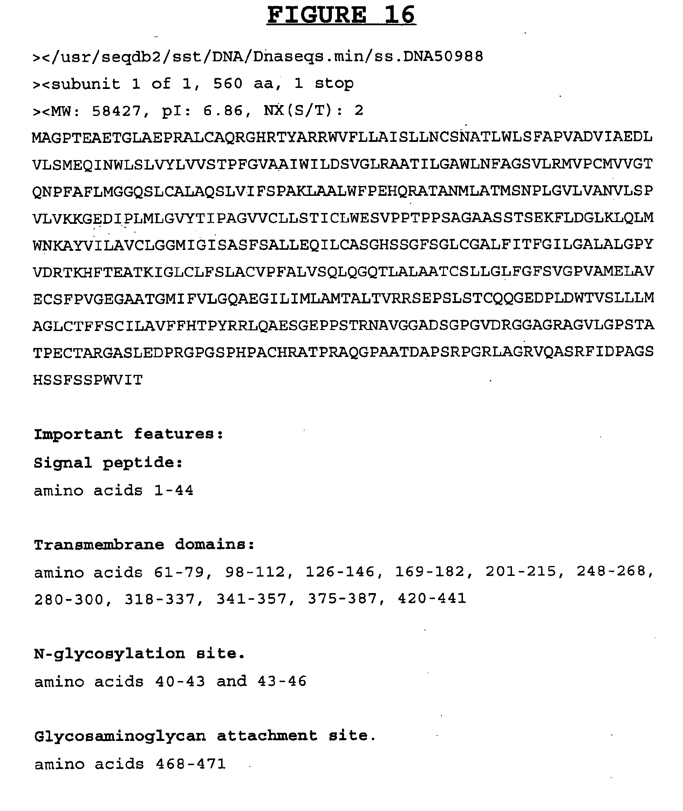

The entire nucleotide sequence of UNQ385 (DNA50988-1326) is shown in Figure 15 (SEQ ID NO:15). Clone UNQ385 (DNA50988-1326) contains a single open reading frame with an apparent translational initiation site at nucleotide positions 17-19 and ending at the stop codon at nucleotide positions 1697-1699 (Figure 15). The predicted polypeptide precursor is 560 amino acids long (Figure 16; SEQ ID NO:16). The full-length PRO717 protein shown in Figure 16 has an estimated molecular weight of about 58,427 daltons and a pI of about 6.86. Clone UNQ385 (DNA50988-1326) has been deposited with the ATCC on April 28, 1998 and is assigned ATCC deposit no.: 209814. Regarding the sequence, it is understood that the deposited clone contains the correct sequence, and the sequences provided herein are based on known sequencing techniques.

Analysis of the amino acid sequence of the full-length PRO717 polypeptide suggests that PRO717 may be a novel 12 transmembrane receptor. The reverse complement strand of DNA50988 has a stretch that matches identically with human regulatory myosin light strand.

Still analyzing the amino acid sequence of SEQ ID NO:16, transmembrane domains are at about amino acids 30-50, 61-79, 98-112, 126-146, 169-182, 201-215, 248-268, 280-300, 318-337, 341-357, 375-387, and 420-441 of SEQ ID NO:16. N-glycosylation sites are at about amino acids 40-43 and 43-46 of SEQ ID NO:16. A glycosaminoglycan attachment site is at about amino acids 468-471 of SEQ ID NO:16. The corresponding nucleotides can be routinely determined given the sequences provided herein.

### EXAMPLE 12: Isolation of cDNA clones Encoding Human PRO846 Polypeptides [UNQ422]

A consensus sequence was obtained relative to a variety of EST sequences as described in Example 1 above, wherein the consensus sequence obtained is herein designated DNA39949. Based on the DNA39949 consensus sequence, oligonucleotides were synthesized: 1) to identify by PCR a cDNA library that contained the sequence of interest, and 2) for use as probes to isolate a clone of the full-length coding sequence for PRO846.

Forward and reverse PCR primers were synthesized:
forward PCR primer 5'-CCCTGCAGTGCACCTACAGGGAAG-3' (SEQ ID NO:116)
reverse PCR primer 5'-CTGTCTTCCCCTGCTTGGCTGTGG-3' (SEQ ID NO:117) Additionally, a synthetic oligonucleotide hybridization probe was constructed from the consensus DNA39949 sequence which had the following nucleotide sequence
hybridization probe
   5'-GGTGCAGGAAGGGTGGGATCCTCTTCTCTCGCTGCTCTGGCCACATC-3' (SEQ ID NO:118)

In order to screen several libraries for a source of a full-length clone, DNA from the libraries was screened by PCR amplification with one of the PCR primer pairs identified above. A positive library was then used to isolate clones encoding the PRO846 gene using the probe oligonucleotide and one of the PCR primers. RNA for construction of the cDNA libraries was isolated from human fetal kidney tissue (LIB227).

DNA sequencing of the clones isolated as described above gave the full-length DNA sequence for PRO846 [herein designated as UNQ422 (DNA44196-1353)] (SEQ ID NO:17) and the derived protein sequence for PRO846.

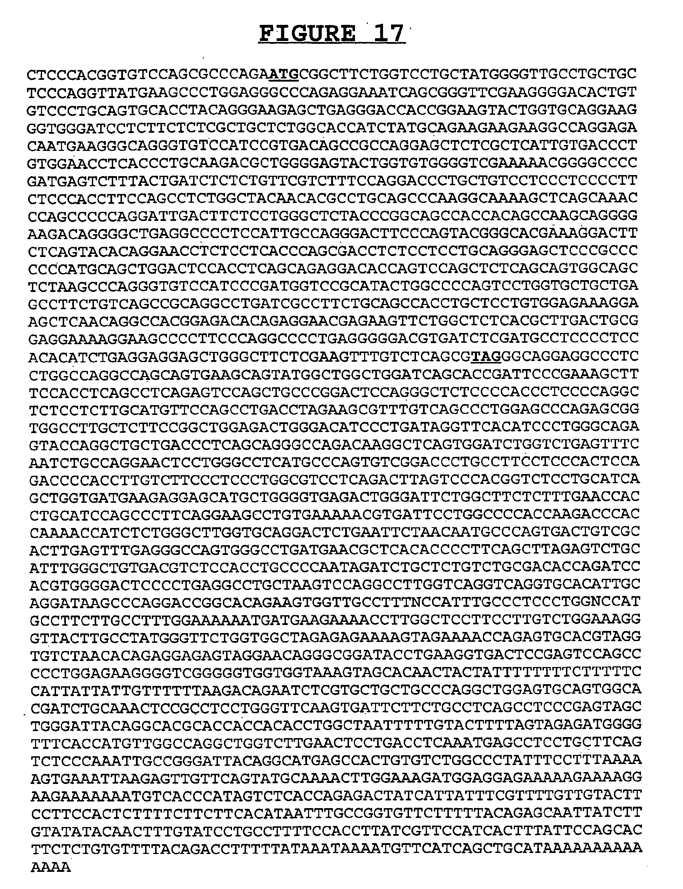

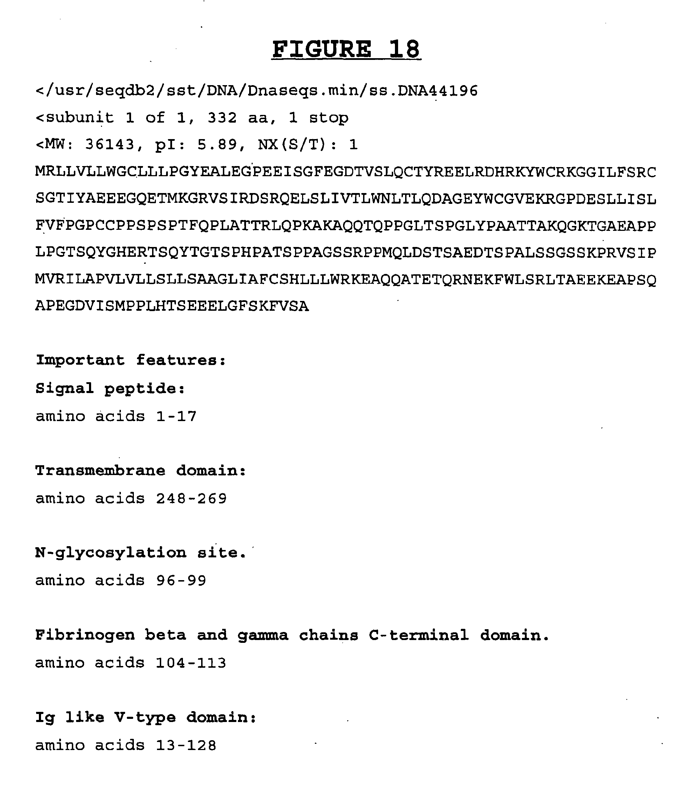

The entire nucleotide sequence of UNQ422 (DNA44196-1353) is shown in Figure 17 (SEQ ID NO:17). Clone UNQ422 (DNA44196-1353) contains a single open reading frame with an apparent translational initiation site at nucleotide positions 25-27 and ending at the stop codon at nucleotide positions 1021-1023 (Figure 17). The predicted polypeptide precursor is 332 amino acids long (Figure 18; SEQ ID NO:18). The full-length PRO846 protein shown in Figure 18 has an estimated molecular weight of about 36,143 daltons and a pI of about 5.89. Important regions of the amino acid sequence of PRO846 include the signal peptide, the transmembrane domain, an N-glycosylation site, a sequence typical of fibrinogen beta and gamma chains C-terminal domain, and a sequence typical of Ig like V-type domain as shown in Figure 18. Clone UNQ422 (DNA44196-1353) has been deposited with ATCC on May 6, 1998 and is assigned ATCC deposit no. 209847.

### EXAMPLE 13: Isolation of cDNA clones Encoding Human PRO874 Polypeptides [UNQ441]

A consensus DNA sequence designated herein as DNA36459 was identified using phrap as described in Example 1 above. Based on the DNA36459 consensus sequence, oligonucleotides were synthesized: 1) to identify by PCR a cDNA library that contained the sequence of interest, and 2) for use as probes to isolate a clone of the coding sequence for PRO874.

PCR primers (forward and reverse) were synthesized:
forward PCR primer 5'-TCGTGCCCAGGGGCTGATGTGC-3' (SEQ ID NO:119); and
reverse PCR primer 5'-GTCTTTACCCAGCCCCGGGATGCG-3' (SEQ ID NO:120).
Additionally, a synthetic oligonucleotide hybridization probe was constructed from the consensus DNA36459 sequence which had the following nucleotide sequence:
hybridization probe 5'-GGCCTAATCCAACGTTCTGTCTTCAATCTGCAAATCTATGGGGTCCTGGG-3' (SEQ ID NO:121).

In order to screen several libraries for a source of a clone, DNA from the libraries was screened by PCR amplification with the PCR primer pair identified above. A positive library was then used to isolate clones encoding the PRO874 gene using the probe oligonucleotide and one of the PCR primers. RNA for construction of the cDNA libraries was isolated from human fetal lung tissue (LIB25).

DNA sequencing ofthe clones isolated as described above gave the DNA sequence for PRO874 [herein designated as DNA40621-1440] (SEQ ID NO:19) and the derived protein sequence for PRO874.

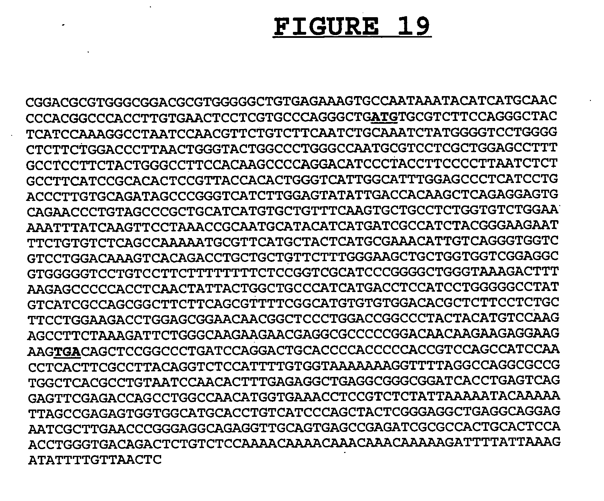

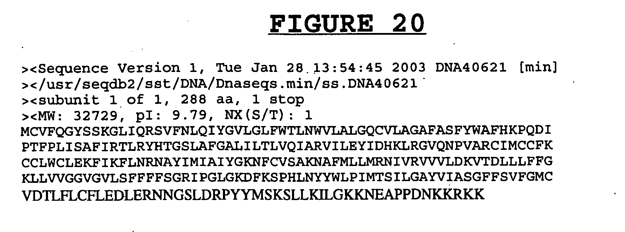

The entire nucleotide sequence of DNA40621-1440 is shown in Figure 19 (SEQ ID NO:19). Clone DNA40621-1440 contains a single open reading frame ending at the stop codon at nucleotide positions 964-966 (Figure 19). The predicted polypeptide encoded by DNA40621-1440 is 321 amino acids long (Figure 20; SEQ ID NO:20). The PRO874 protein shown in Figure 20 has an estimated molecular weight of about 36,194 daltons and a pI of about 9.85. Analysis of the PRO874 sequence shown in Figure 20 (SEQ ID NO:20) evidenced the presence of the following: a type II transmembrane domain at about amino acids 57-80; additional transmembrane domains at about amino acids 110-126,215-231, and 254-274; potential N-glycosylation sites at about amino acids 16-19, 27-30, and 289-292; sequence identity with hypothetical YBR002c family proteins at about amino acids 276-287; and sequence identity with ammonium transporter proteins at about amino acids 204-230. Clone DNA40621-1440 was deposited with the ATCC on June 2, 1998, and is assigned ATCC deposit no. 209922.

Analysis of the amino acid sequence of the PRO874 polypeptide suggests that it is a novel multi-span transmembrane protein. However, an analysis of the Dayhoff database (version 35.45 SwissProt 35) evidenced sequence identity between the PRO874 amino acid sequence and the following Dayhoff sequences: S67049, AF054839_1, S73437, S52460, and HIVU80570_1.

### EXAMPLE 14: Isolation of cDNA clones Encoding Human PRO1082 Polypeptides [UNQ539/589]

A consensus DNA sequence was assembled relative to other EST sequences using phrap as described in Example 1 above, wheein the consensus sequence is herein designated DNA38097. Based on this consensus sequence, oligonucleotides were synthesized: 1) to identify by PCR a cDNA library that contained the sequence of interest, and 2) for use as probes to isolate a clone of the full-length coding sequence for PRO1082.

A set of PCR primers (two forward and one reverse) were synthesized:
forward primer 1 5'-GTCCACAGACAGTCATCTCAGGAGCAG-3' (SEQ ID NO:122);
forward primer 2 5'-ACAAGTGTCTTCCCAACCTG-3' (SEQ ID NO:123);
reverse primer 1 5'-ATCCTCCCAGAGCCATGGTACCTC-3' (SEQ IDNO:124).
Additionally, a synthetic oligonucleotide hybridization probe was constructed from the DNA38097 consensus sequence which had the following nucleotide sequence:
hybridization probe
   5'-CCAAGGATAGCTGTTGTTTCAGAGAAAGGATCGTGTGCTGCATCTCCTCCT-3' (SEQ ID NO:125).

In order to screen several libraries for a source of a full-length clone, DNA from the libraries was screened by PCR amplification with the PCR primers identified above. A positive library was then used to isolate clones encoding the PRO1082 gene using the probe oligonucleotide and one of the PCR primers. RNA for construction of the cDNA libraries was isolated from human fetal kidney tissue (LIB227).

DNA sequencing of the clones isolated as described above gave the full-length DNA sequence for PRO1082 [herein designated as UNQ539/589 (DNA53912-1457)] (SEQ ID NO:23) and the derived protein sequence for PRO1082.

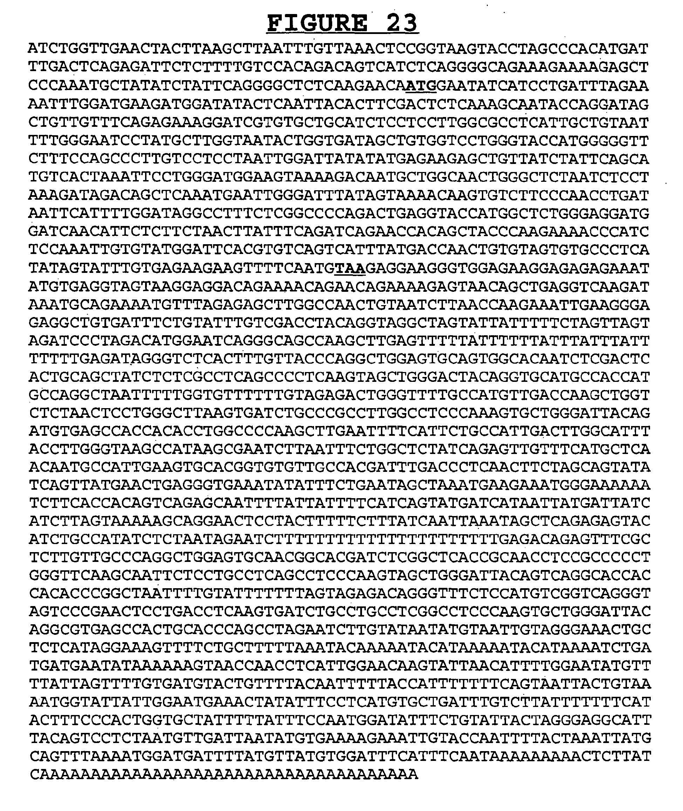

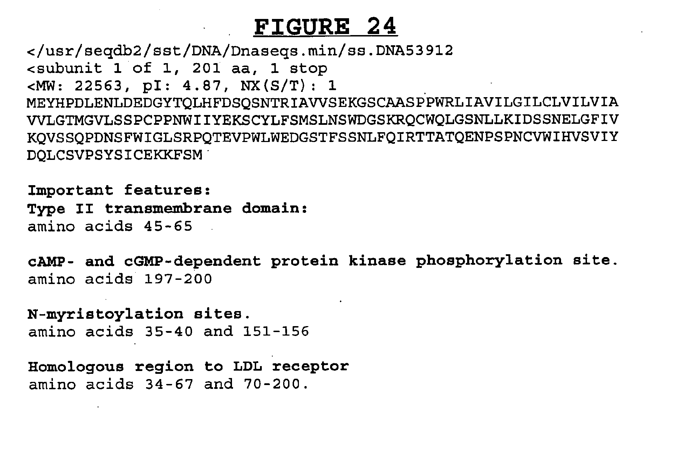

The entire nucleotide sequence of UNQ539/589 (DNA53912-1457) is shown in Figure 23 (SEQ ID NO:23). Clone UNQ539/589 (DNA53912-1457) contains a single open reading frame with an apparent translational initiation site at nucleotide positions 160-162 and ending at the stop codon at nucleotide positions 763-765 (Figure 23). The predicted polypeptide precursor is 201 amino acids long (Figure 24; SEQ ID NO:24). The full-length PRO1082 protein shown in Figure 24 has an estimated molecular weight of about 22,563 daltons and a pI of about 4.87. Clone UNQ539/589 (DNA53912-1457) has been deposited with the ATCC on May 14, 1998 and is assigned ATCC deposit no.: 209870. Regarding the sequence, it is understood that the deposited clone contains the correct sequence, and the sequences provided herein are based on known sequencing techniques.

Still analyzing the amino acid sequence of SEQ ID NO:24, the transmembrane domain is at about amino acids 45-65 of SEQ ID NO:24. A cAMP- and cGMP-dependent protein kinase phosphorylation site is at about amino acids 197-200 of SEQ ID NO:24. N-myristoylation sites are at about amino acids 35-40 and 151-156 of SEQ ID NO:24. The regions which share sequence identity with the LDL receptor are at about amino acids 34-67 and 70-200 of SEQ ID NO:24. The corresponding nucleotides of these amino acid regions and others can be routinely determined given the sequences provided herein.

### EXAMPLE 15: Isolation of cDNA clones Encoding Human PRO1097 Polypeptides [UNQ542]

Use of the signal sequence algorithm described in Example 3 above allowed identification of a single EST cluster sequence from the Incyte database. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA56006.

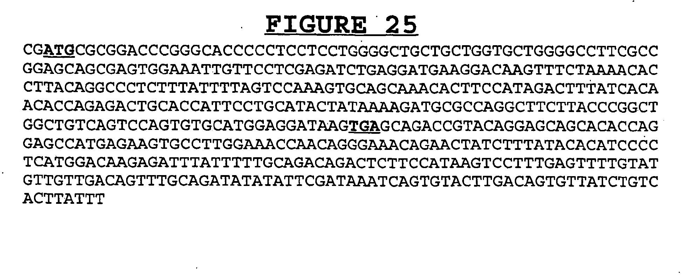

In light of an observed sequence homology between the DNA56006 consensus sequence and an EST sequence encompassed within the Incyte EST clone no. 2408105, the Incyte EST clone 2408105 was purchased and the cDNA insert was obtained and sequenced. It was found that this insert encoded a full-length protein. The sequence of this cDNA insert is shown in Figure 25 and is herein designated as DNA59841-1460.

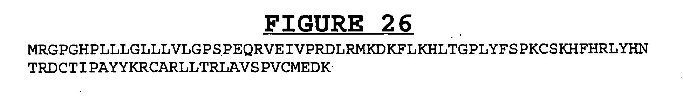

The entire nucleotide sequence of DNA59841-1460 is shown in Figure 25 (SEQ ID NO:25). Clone DNA59841-1460 contains a single open reading frame with an apparent translational initiation site at nucleotide positions 3-5 and ending at the stop codon at nucleotide positions 276-278 of SEQ ID NO:348 (Figure 25). The predicted polypeptide precursor is 91 amino acids long (Figure 26; SEQ ID NO:26). The full-length PRO1097 protein shown in Figure 26 has an estimated molecular weight of about 10,542 daltons and a pI of about 10.04. Clone DNA59841-1460 has been deposited with ATCC on July 1, 1998 and is assigned ATCC deposit no.: 203044. It is understood that the deposited clone has the actual nucleic acid sequence and that the sequences provided herein are based on known sequencing techniques.

Analyzing Figure 26, the signal peptide is at about amino acids 1-20 of SEQ ID NO:26. The glycoprotease family protein domain starts at about amino acid 56, and the acyltransferase ChoActase/COT/CPT family peptide starts at about amino acid 49 of SEQ ID NO:26.

### EXAMPLE 16: Isolation of cDNA clones Encoding Human PRO1192 Polypeptides [UNQ606]

A consensus DNA sequence was assembled relative to other EST sequences using phrap as described in Example 1 above. This consensus sequence is designated herein DNA35924. Based on the DNA35924 consensus sequence, oligonucleotides were synthesized: 1) to identify by PCR a cDNA library that contained the sequence of interest, and 2) for use as probes to isolate a clone of the full-length coding sequence for PRO1192.

PCR primers (forward and reverse) were synthesized:
forward PCR primer: 5'-CCGAGGCCATCTAGAGGCCAGAGC-3' (SEQ ID NO:126)
reverse PCR primer: 5'-ACAGGCAGAGCCAATGGCCAGAGC-3' (SEQ ID NO:127).

Additionally, a synthetic oligonucleotide hybridization probe was constructed from the consensus DNA35924 sequence which had the following nucleotide sequence:
hybridization probe:
   5'-GAGAGGACTGCGGGAGTTTGGGACCTTTGTGCAGACGTGCTCATG-3' (SEQ ID NO:128).

In order to screen several libraries for a source of a full-length clone, DNA from the libraries was screened by PCR amplification with the PCR primer pair identified above. A positive library was then used to isolate clones encoding the PRO1192 gene using the probe oligonucleotide and one of the PCR primers. RNA for construction of the cDNA libraries was isolated from human fetal liver and spleen tissue.

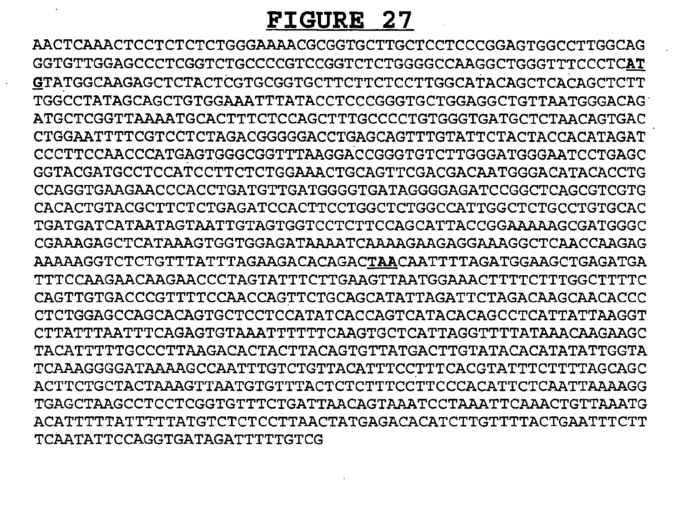

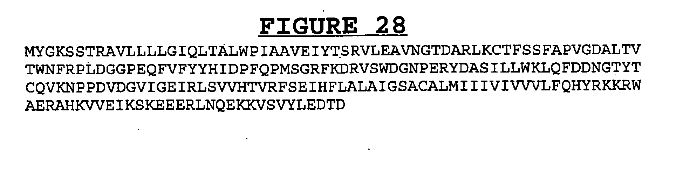

DNA sequencing of the clones isolated as described above gave the full-length DNA sequence for PRO1192 designated herein as DNA62814-1521 and is shown in Figure 27 (SEQ ID NO:27); and the derived protein sequence for PRO1192 which is shown in Figure 28 (SEQ ID NO:28).

The entire coding sequence of PRO1192 is shown in Figure 27 (SEQ ID NO:27). Clone DNA62814-1521 contains a single open reading frame with an apparent translational initiation site at nucleotide positions 121-123 and an apparent stop codon at nucleotide positions 766-768. The predicted polypeptide precursor is 215 amino acids long. The predicted polypeptide precursor has the following features: a signal peptide at about amino acids 1-21; a transmembrane domain at about amino acids 153-176; potential N-glycosylation sites at about amino acids 39-42 and 118-121; and homology with myelin P0 proteins at about amino acids 27-68 and 99-128 of Figure 28. The full-length PRO1192 protein shown in Figure 28 has an estimated molecular weight of about 24,484 daltons and a pI of about 6.98.

An analysis of the Dayhoff database (version 35.45 SwissProt 35), using a WU-BLAST2 sequence alignment analysis of the full-length sequence shown in Figure 28 (SEQ ID NO:28), revealed homology between the PRO1192 amino acid sequence and the following Dayhoff sequences: GEN12838, MYPO_HUMAN, A049498_1, GEN14531, P_W14146, HS46KDA_1, CINB_RAT, OX2G_RAT, D87018_1, and D86996_2.

Clone DNA62814-1521 was deposited with the ATCC on August 4,1998, and is assigned ATCC deposit no. 203093.

### EXAMPLE 17: Isolation of cDNA clones Encoding Human PRO1268 Polypeptides [UNQ638]

Use of the signal sequence algorithm described in Example 3 above allowed identification of an EST cluster sequence from the LIFESEQ® database, designated EST No. 8879. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). One or more of the ESTs was derived from a cDNA library constructed from human brain tumor tissue taken from a cerebral meninges lesion. The consensus sequence obtained therefrom is herein designated DNA56258.

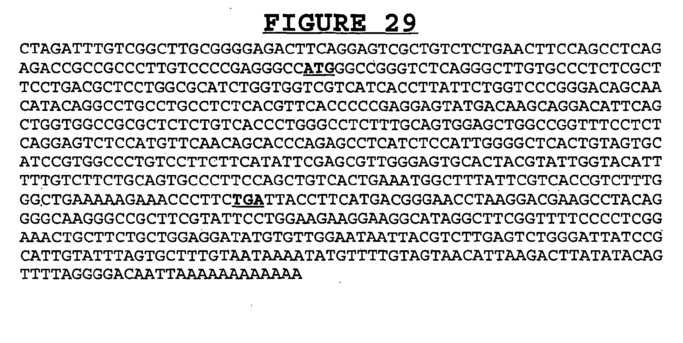

In light of the sequence homology between the DNA56258 sequence and an EST sequence contained within the Incyte EST no. 2944541, EST clone no. 2944541 was purchased and the cDNA insert was obtained and sequenced. The sequence of this cDNA insert is shown in Figure 29 and is herein designated as "DNA66519-1535".

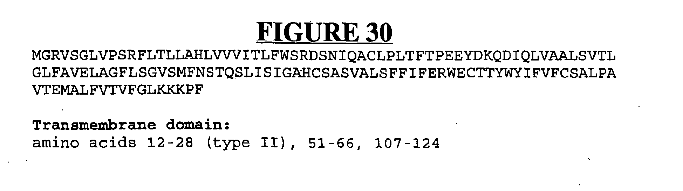

The full length clone shown in Figure 29 contained a single open reading frame with an apparent translational initiation site at nucleotide positions 89 to 91 and ending at the stop codon found at nucleotide positions 509 to 511 (Figure 29; SEQ ID NO:29). The predicted polypeptide precursor (Figure 30, SEQ ID NO:30) is 140 amino acids long. PRO1268 has a calculated molecular weight of approximately 15,503 daltons and an estimated pI of approximately 6.44. Additional features include a type II transmembrane domain at about amino acids 12-28; type I transmembrane domains at about amino acids 51-66 and 107-124; a potential N-glycosylation site at about amino acids 79-82, and a region having homology with G-protein coupled receptors at about amino acids 59-99.

An analysis of the Dayhoff database (version 35.45 SwissProt 35), using a WU-BLAST2 sequence alignment analysis of the full-length sequence shown in Figure 30 (SEQ ID NO:30), revealed some homology between the PRO1268 amino acid sequence and Dayhoff sequence no. CEF39B2_9. However, the percent sequence identity was determined to not be significant.

Clone DNA66519-1535 was deposited with the ATCC on September 15, 1998 and is assigned ATCC deposit no. 203236.

### EXAMPLE 18: Isolation of cDNA clones Encoding Human PRO1278 Polypeptides [UNQ648]

A consensus DNA sequence was assembled relative to other EST sequences using phrap as described in Example 1 above. This consensus sequence is designated herein "Consen5230". In addition, the Consen5230 consensus sequence was extended using repeated cycles of BLAST and phrap to extend the consensus sequence as far as possible using the sources of EST sequences discussed above. The extended consensus sequence is designated herein as "DNA44801". Based on the DNA44801 consensus sequence, oligonucleotides were synthesized: 1) to identify by PCR a cDNA library that contained the sequence of interest, and 2) for use as probes to isolate a clone of the full-length coding sequence for PRO1278.

PCR primers (forward and reverse) were synthesized:
forward PCR primers: GCAGGCTTTGAGGATGAAGGCTGC (44801.f1; SEQ ID NO:129) and CTCATTGGCTGCCTGGTCACAGGC (44801.f2; SEQ ID NO:130)
reverse PCR primers: CCAGTCGGACAGGTCTCTCCCCTC (44801.rl; SEQ ID NO:131) and TCAGTGACCAAGGCTGAGCAGGCG (44801.r2; SEQ ID NO:132)

Additionally, a synthetic oligonucleotide hybridization probe was constructed from the consensus DNA44801 sequence which had the following nucleotide sequence:
hybridization probe: CTACACTCGTTGCAAACTGGCAAAAATATTCTCGAGGGCTGGCCTGG (44801.p1; SEQ ID NO:33)

In order to screen several libraries for a source of a full-length clone, DNA from the libraries was screened by PCR amplification with the PCR primer pair identified above. A positive library was then used to isolate clones encoding the PRO1278 gene using the probe oligonucleotide and one of the PCR primers. RNA for construction of the cDNA libraries was isolated from human testis.

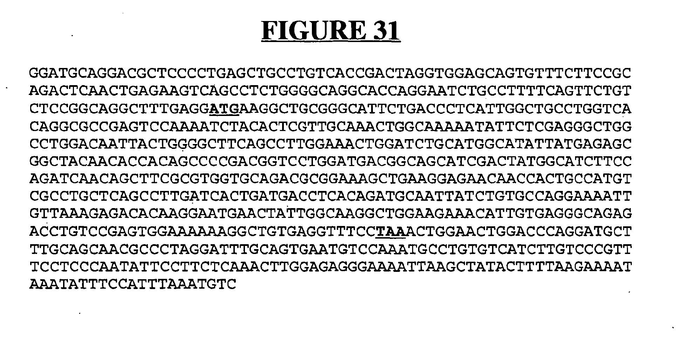

DNA sequencing of the clones isolated as described above gave the full-length DNA sequence for PRO1278 (designated herein as DNA66304-1546 [Figure 31, SEQ ID NO:31]; and the derived protein sequence for PRO1278.

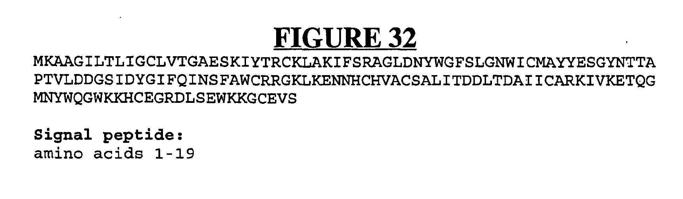

The entire coding sequence of PRO1278 is shown in Figure 31 (SEQ ID NO:31). Clone DNA66304-1546 contains a single open reading frame with an apparent translational initiation site at nucleotide positions 141-143 and an apparent stop codon at nucleotide positions 585-587. The predicted polypeptide precursor is 148 amino acids long. The full-length PRO1278 protein shown in Figure 32 has an estimated molecular weight of about 16,623 daltons and a pI of about 8.47. Additional features include a signal peptide sequence at about amino acids 1-19; a potential N-glycosylation site at about amino acids 58-61; an atpha-lactalbumin/lysozyme C signature at about amino acids 94-112; and homology with alpha-lactalbumin/lysozyme C at about amino acids 35-59, 67-59 and 112-133.

An analysis of the Dayhoff database (version 35.45 SwissProt 35), using a WU-BLAST2 sequence alignment analysis ofthe full-length sequence shown in Figure 32 (SEQ ID NO:32), revealed significant homology between the PRO1278 amino acid sequence and the following Dayhoff sequences: LYC1_ANAPL, LYC3_ANAPL, and LYC_HUMAN.

Clone DNA66304-1546 was deposited with the ATCC on October 6,1998, and is assigned ATCC deposit no. 203321.

### EXAMPLE 19: Isolation of cDNA clones Encoding Human PRO1303 Polypeptides [UNQ669]

A consensus DNA sequence was assembled relative to other EST sequences using phrap as described in Example 1 above. This consensus sequence is designated herein "DNA47347". Based on the DNA47347 consensus sequence and its homology to an Incyte EST within the assembly from which DNA47347 was derived, Incyte clone 1430305 (from an ileum tissue library) was purchased and sequenced in full. The sequence encoding PRO1303 was thereby identified.

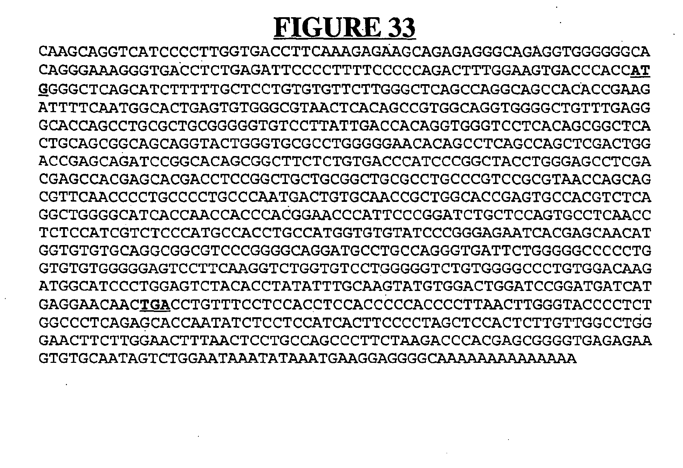

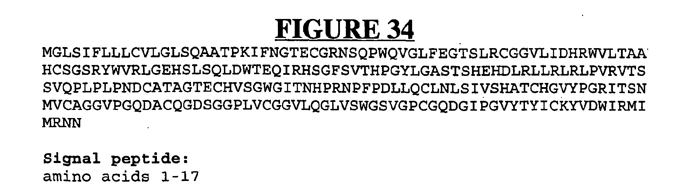

The entire coding sequence of PRO1303 is shown in Figure 33 (SEQ ID NO:33). Clone DNA65409-1566 contains a single open reading frame with an apparent translational initiation site at nucleotide positions 121-123 and an apparent stop codon at nucleotide positions 865-867. The predicted polypeptide precursor is 248 amino acids long. The signal peptide is at about amino acids 1-17 of SEQ ID NO:34. The locations ofN-glycosylation sites, active and conserved regions and domains are further indicated in Figure 34. Clone DNA65409-1566 has been deposited with ATCC on September 15, 1998 and is assigned ATCC deposit no. 203232. The full-length PRO1303 protein shown in Figure 34 has an estimated molecular weight of about 26,734 daltons and a pI of about 7.9.

An analysis of the Dayhoff database (version 35.45 SwissProt 35), using a WU-BLAST2 sequence alignment analysis of the full-length sequence shown in Figure 34 (SEQ ID NO:34), revealed sequence identity between the PRO1303 amino acid sequence and the following Dayhoff sequences (data incorporated herein): AB009849_1, P_W08475, AF024605_1, A42048_1, TRY3_RAT, MMAE00066414, TRY1_RAT, MMAE000663_4, MMAE000665_2, and MMAE00066412.

### EXAMPLE 20: Isolation of cDNA clones Encoding Human PRO1308 Polypeptides [UNQ674]

A consensus DNA sequence was assembled relative to other EST sequences using phrap as described in Example 1 above. The consensus sequence was extended then using repeated cycles of BLAST and phrap to extend the consensus sequence as far as possible using the sources of EST sequences discussed above. The extended consensus sequence is designated herein as "DNA35726". Based on the DNA35726 consensus sequence, oligonucleotides were synthesized: 1) to identify by PCR a cDNA library that contained the sequence of interest, and 2) for use as probes to isolate a clone of the full-length coding sequence for PRO1308.

The following PCR primers (forward and reverse) were synthesized:
- forward PCR primers: 5'-TCCTGTGAGCACGTGGTGTG-3' (SEQ ID NO:134);
5'-GGGTGGGATAGACCTGCG-3' (SEQ ID NO:135);
5'-AAGGCCAAGAAGGCTGCC-3' (SEQ ID NO:136); and
5'-CCAGGCCTGCAGACCCAG-3' (SEQ ID NO:137).
- reverse PCR primers: 5'-CTTCCTCAGTCCTTCCAGGATATC-3' (SEQ ID NO:138);
5'-AAGCTGGATATCCTCCGTGTTGTC-3' (SEQ ID NO:139);
5'-CCTGAAGAGGCATGACTGCTTTTCTCA-3' (SEQ ID NO:140); and
5'-GGGGATAAACCTATTAATTATTGCTAC-3' (SEQ ID NO:141).
Additionally, a synthetic oligonucleotide hybridization probe was constructed from the consensus DNA35726 sequence which had the following nucleotide sequence:
hybridization probe: 5'-AACGTCACCTACATCTCCTCGTGCCACATGCGCCAGGCCACCTG-3' (SEQ ID NO:142).

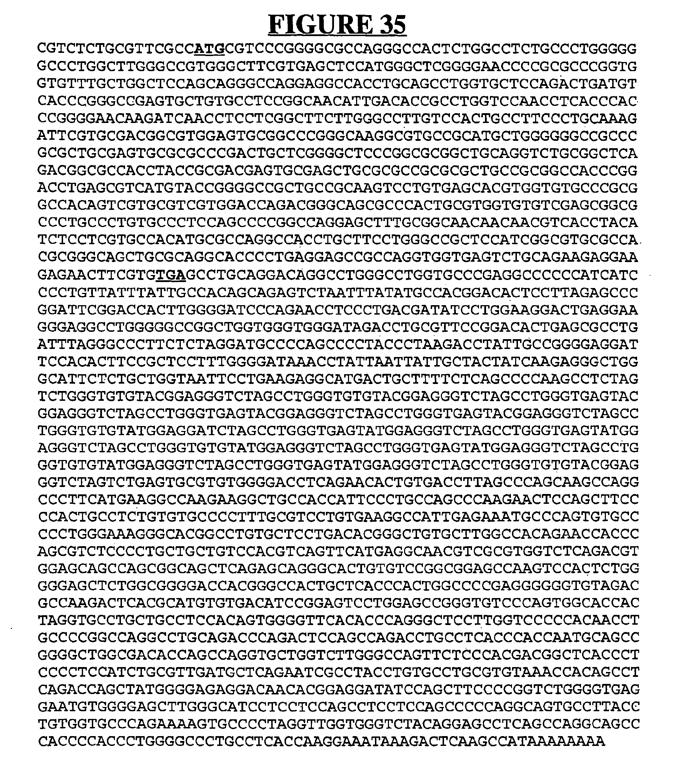

In order to screen several libraries for a source of a full-length clone, DNA from the libraries was screened by PCR amplification with the PCR primer pair identified above. A positive library was then used to isolate clones encoding the PRO1308 gene using the probe oligonucleotide and one of the PCR primers. RNA for construction of the cDNA libraries was isolated from a human SK-Lu-1 adenocarcinoma cell line. DNA sequencing of the clones isolated as described above gave the full-length DNA sequence for PRO1308 (designated herein as DNA62306-1570 [Figure 35, SEQ ID NO:35]; and the derived protein sequence for PRO1308.

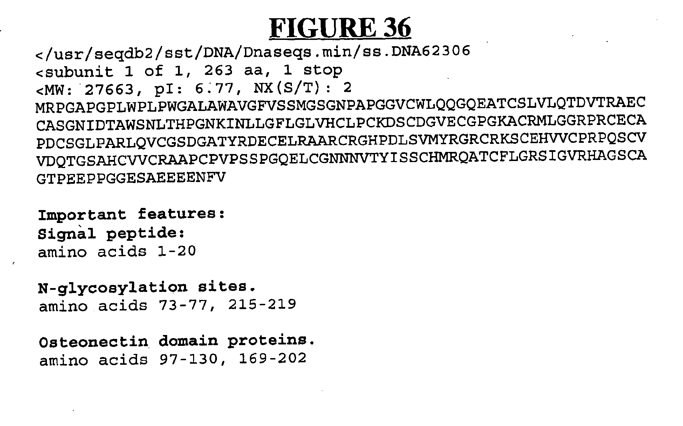

The entire coding sequence of PRO1308 is shown in Figure 35 (SEQ ID NO:35). Clone DNA62306-1570 contains a single open reading frame with an apparent translational initiation site at nucleotide positions 17-19 and an apparent stop codon at nucleotide positions 806-808. The predicted polypeptide precursor is 263 amino acids long. The full-length PRO1308 protein shown in Figure 36 has an estimated molecular weight of about 27,663 daltons and a pI of about 6.77. Additional features include a signal peptide at about amino acids 1-20, potential N-glycosylation sites at about amino acids 73-76 and 215-218, and regions of homology with osteonectin domains at about amino acids 97-129 and 169-201.

An analysis of the Dayhoff database (version 35.45 SwissProt 35), using a WU-BLAST2 sequence alignment analysis of the full-length sequence shown in Figure 36 (SEQ ID NO:36), revealed significant homology between the PRO1308 amino acid sequence and Dayhoff sequence S55369. Homology was also revealed between the PRO1308 amino acid sequence and the following Dayhoff sequences: FSA_HUMAN, P_R20063, CELT13C2_1, AGRI_RAT, p_W09406, G01639, SC1_RAT, S60062, S51362, and IOV7_CHICK.

Clone DNA62306-1570 has been deposited with ATCC on September 9, 1998 and is assigned ATCC deposit no. 203254.

### EXAMPLE 21: Isolation of cDNA clones Encoding Human PRO1338 Polypeptides [UNQ693]

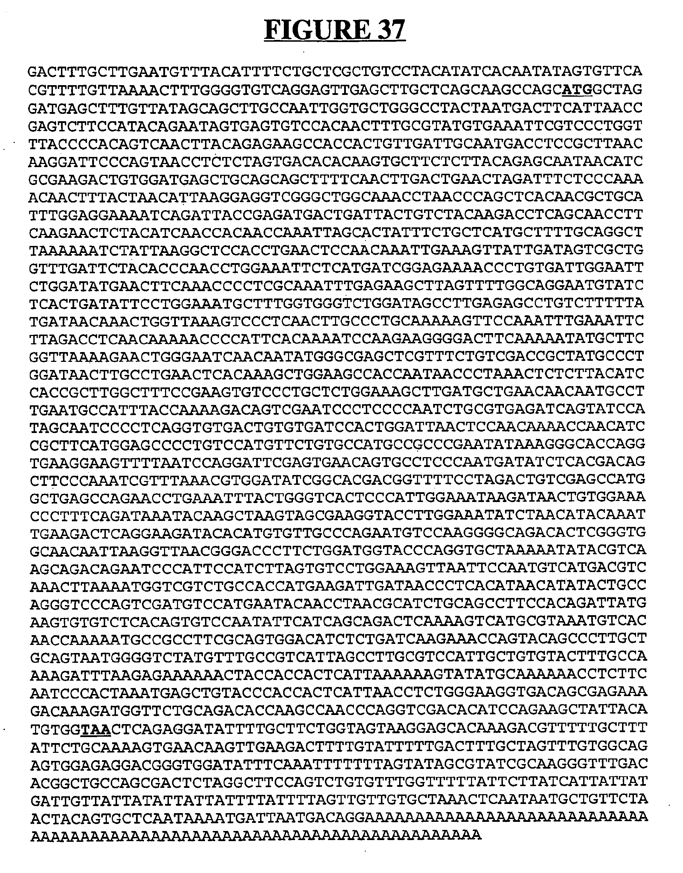

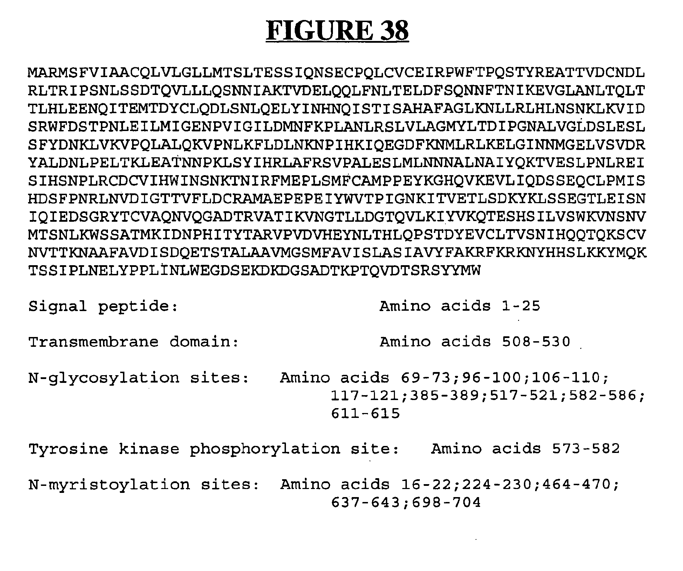

The use of yeast screens resulted in EST sequences which were then compared to various public and private EST databases in a manner similar to that described above under ECD homology (Example 1) and which resulted in the identification of Incyte EST2615184, an EST derived from cholecystitis gall bladder tissue. Analysis of the corresponding full-length sequence ultimately resulted in the isolation of DNA66667-1596 (SEQ ID NO:37, Figure 37) and the derived PRO1338 native sequence protein (SEQ ID NO:38, Figure 38).

DNA66667-1596 (SEQ ID NO:37) as shown in Figure 37 contains a single open reading frame with a translation initiation site at about nucleotide residues 115-117 and ending at the stop codon (TAA) at nucleotide positions 2263-2265, as indicated by bolded underline. The predicted PRO1338 polypeptide precursor (SEQ ID NO:38) is 716 amino acids in length (Figure 38), and has a calculated molecular weight of 80,716 daltons and a pI of 6.06.

Analysis of the PRO1338 polypeptide (SEQ ID NO:38) of Figure 38 reveals a signal sequence at about amino acid residues 1 to 25; a transmembrane domain at about amino acid residues 508 to 530; N-glycosylation sites at about amino acid residues 69-73, 96-100, 106-110, 117-121, 385-389, 517-521, 582-586 and 611-615; a tyrosine kinase phosphorylation site at about residues 573-582; and N-myristoylation sites at about amino acid residues 16-22, 224-230, 464-470, 637-643 and 698-704.

A cDNA containing DNA66667-1596 has been deposited with the ATCC under the designation DNA66667-1596 on September 22, 1998 and has been assigned ATCC deposit number 203267.

### EXAMPLE 22: Isolation of cDNA clones Encoding Human PRO1378 Polypeptides [UNQ715]

An initial DNA sequence referred to herein as DNA51941 was identified using a yeast screen, in a human bone marrow cDNA library that preferentially represents the 5' ends of the primary cDNA clones. Based on the DNA51941 sequence, the following oligonucleotides were synthesized for use as probes to isolate a clone of the full-length coding sequence for PRO1378 from a bone marrow cDNA library:
TGTCCTTTGTCCCAGACTTCTGTCC
(SEQ ID NO:143),
CTGGATGCTAATGTGTCCAGTAAATGATCCCCTTATCCCGTCGCGATGCT (SEQ ID NO:144); TTCCACTCAATGAGGTGAGCCACTC (SEQ ID NO:36); GGCGAGCCCTAACTATCCAGGAG (SEQ ID NO:145);
GGAGATCGCTGCGCTGGCCAGGTCCTCCCTGCATGGTAT (SEQ ID NO:146); and
CTGCTGCAAAGCGAGCCTCTTG (SEQ ID NO:147).

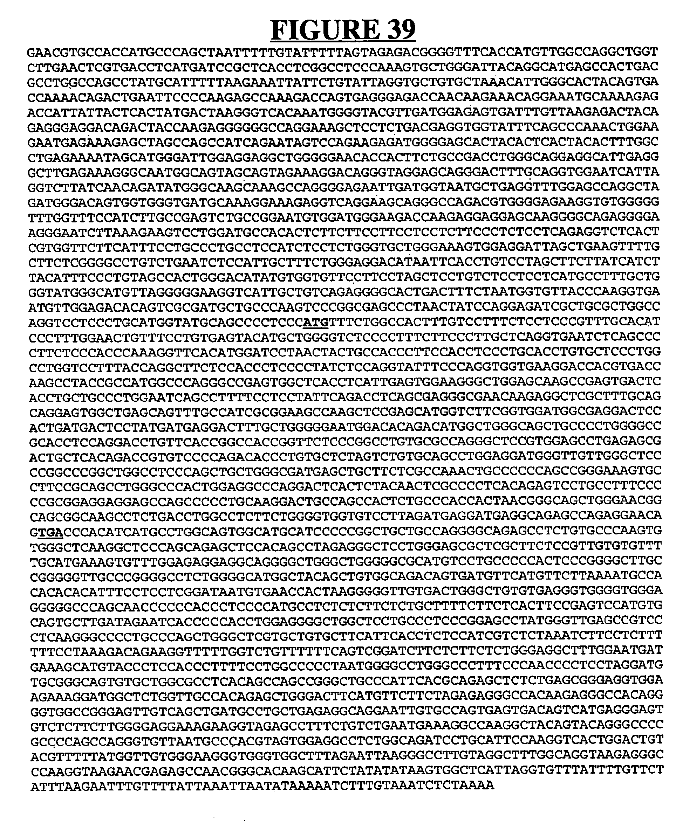

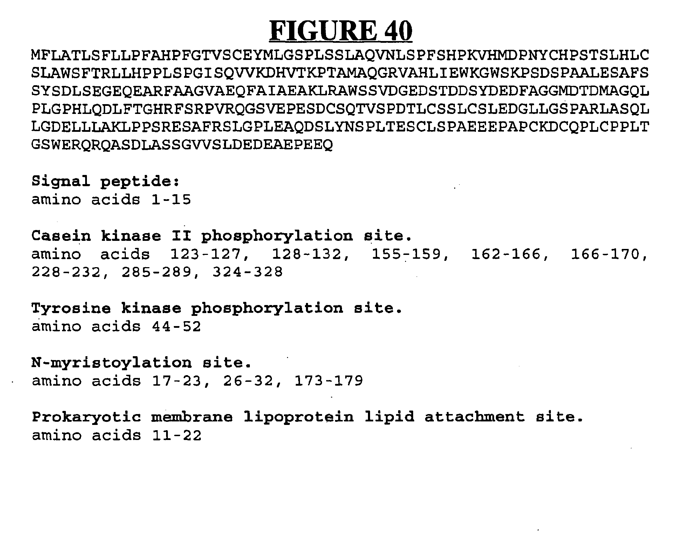

The full length DNA58730-1607 clone shown in Figure 39 contained a single open reading frame with an apparent translational initiation site at nucleotide positions 1365 to 1367 and ending at the stop codon found at nucleotide positions 2370 to 2372 (Figure 39; SEQ ID NO:39). The predicted polypeptide precursor (Figure 40, SEQ ID NO:40) is 335 amino acids long, with a signal peptide sequence at about amino acids 1-15. PRO1378 has a calculated molecular weight of approximately 36,108 daltons and an estimated pI of approximately 4.51.

An analysis of the Dayhoff database (version 35.45 SwissProt 35), using a WU-BLAST2 sequence alignment analysis of the full-length sequence shown in Figure 40 (SEQ ID NO:40), revealed some homology between the PRO1378 amino acid sequence and the following Dayhoffsequences: ICAL_RABIT, SP2_HUMAN, SHPSPRBB_1, SP23_HUMAN, P_W08158, and P_W08150.

Clone DNA58730-1607 was deposited with the ATCC on September 15, 1998, and is assigned ATCC deposit no. 203221.

### EXAMPLE 23: Isolation of cDNA clones Encoding Human PRO1415 Polypeptides [UNQ731]

Use of the signal sequence algorithm described in Example 3 above allowed identification of an EST cluster sequence from the Incyte database, designated Incyte EST cluster sequence no. 150918. This EST cluster sequence was then compared to a variety of expressed sequence tag (EST) databases which included public EST databases (e.g., GenBank) and a proprietary EST DNA database (Lifeseq®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA55720.

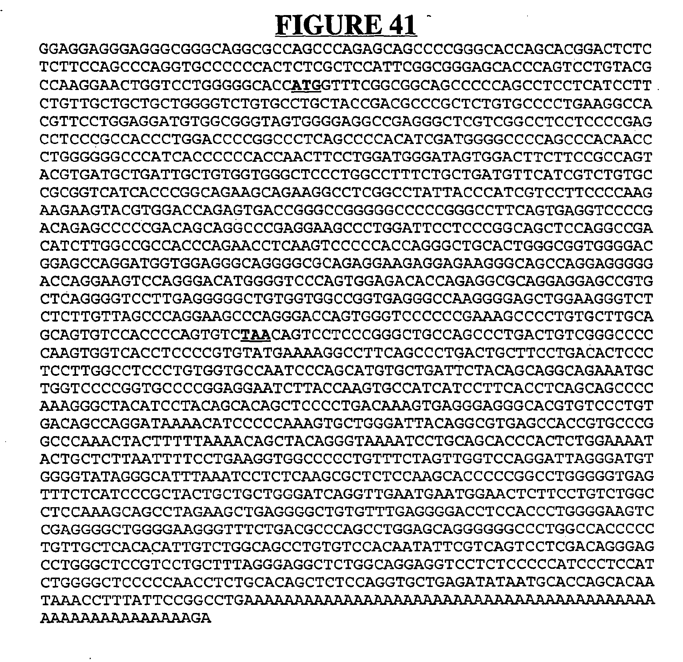

In light of the sequence homology between the DNA55720 sequence and an EST sequence contained within the Incyte EST clone no. 4081476, the Incyte EST clone no. 4081476 was purchased and the cDNA insert was obtained and sequenced. The sequence of this cDNA insert is shown in Figure 41 and is herein designated as DNA58852-1637.

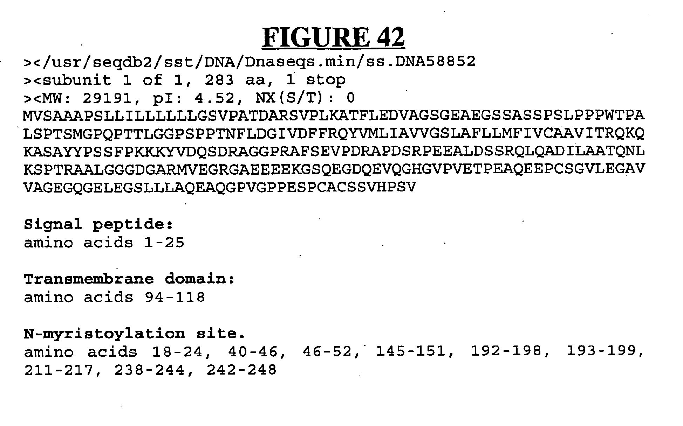

Clone DNA58852-163 7 contains a single open reading frame with an apparent translational initiation site at nucleotide positions 148-150 and ending at the stop codon at nucleotide positions 997-999 (Figure 41; SEQ ID NO:41). The predicted polypeptide precursor is 283 amino acids long (Figure 42). The full-length PRO1415 protein shown in Figure 42 has an estimated molecular weight of about 29,191 daltons and a pI of about 4.52. Analysis of the full-length PRO1415 sequence shown in Figure 42 (SEQ ID NO:42) evidences the presence of the following: a signal peptide from about amino acid 1 to about amino acid 25, a transmembrane domain from about amino acid 94 to about amino acid 118 and potential N-myristolation sites from about amino acid 18 to about amino acid 23, from about amino acid 40 to about amino acid 45, from about amino acid 46 to about amino acid 51, from about amino acid 145 to about amino acid 150, from about amino acid 192 to about amino acid 197, from about amino acid 193 to about amino acid 198, from about amino acid 211 to about amino acid 216, from about amino acid 238 to about amino acid 243 and from about amino acid 242 to about amino acid 247. Clone DNA58852-1637 has been deposited with ATCC on September 22, 1998 and is assigned ATCC deposit no. 203271.

An analysis of the Dayhoff database (version 35.45 SwissProt 35), using a WU-BLAST2 sequence alignment analysis of the full-length sequence shown in Figure 42 (SEQ ID NO:42), evidenced significant homology between the PRO1415 amino acid sequence and the following Dayhoff sequences: HSU66616_1, P_W24017_A38219, CD30_HUMAN, HSU78971_1, P_W22214, NFM_HUMAN, ADH1_ASPFL, PAU93274_5 and CENB_MOUSE.

### EXAMPLE 24: Isolation of cDNA clones Encoding Human PRO1867 Polypeptides [UNQ858]

The extracellular domain (ECD) sequences (including the secretion signal sequence, if any) from about 950 known secreted proteins from the Swiss-Prot public database were used to search EST databases. The EST databases included public EST databases (e.g., GenBank), and a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is herein designated DNA80202. Based on the DNA80202 consensus sequence, oligonucleotides were synthesized: 1) to identify by PCR a cDNA library that contained the sequence of interest, and 2) for use as probes to isolate a clone of the full-length coding sequence for PRO1867. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer (80202,f1) 5'-TGTGATTTTACAGAGTACTGCAATGGAACC-3' (SEQ ID NO:148) reverse PCR primer (80202.r1) 5'-TTCTTTAAAACAGGCAAATGGAGCACC-3' (SEQ ID NO:149) Additionally, a synthetic oligonucleotide hybridization probe was constructed from the consensus DNA80202 sequence which had the following nucleotide sequence
hybridization probe (80202.p1)
   5'-TGACACTTATGCATTGAATGGCCGTTTGTGCAAGTTGGGA-3' (SEQ ID NO:150)

RNA for construction of the cDNA libraries was isolated from human testis tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

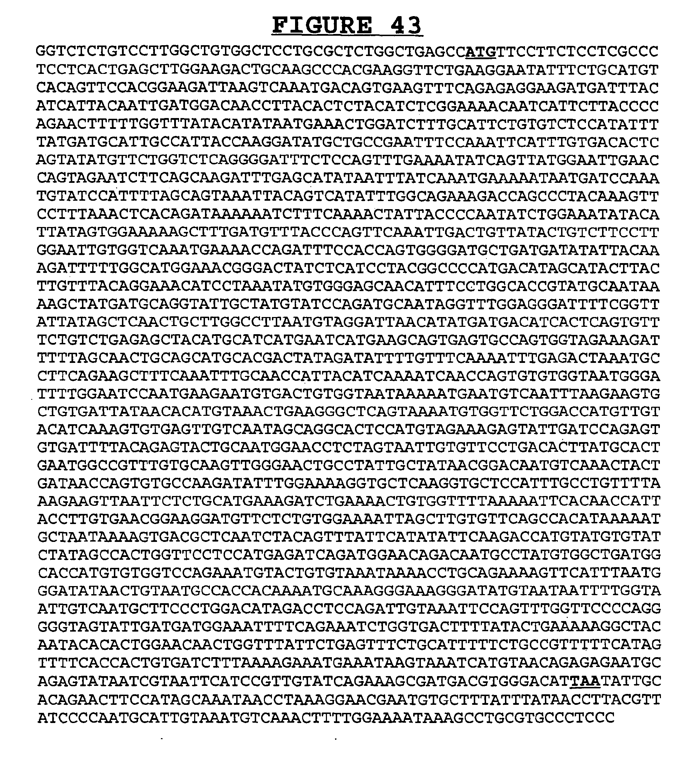

DNA sequencing of the clones isolated as described above gave the full-length DNA sequence for PRO1867 (designated herein as DNA84925-2514 [Figure 43, SEQ ID NO: 43]; (UNQ858) and the derived protein sequence for PRO1867.

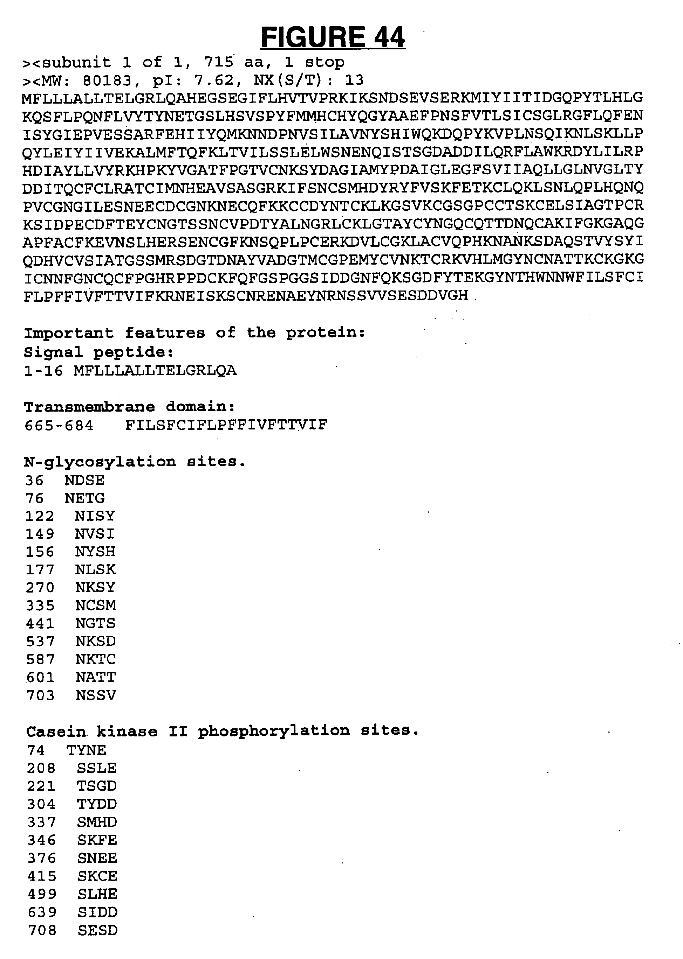

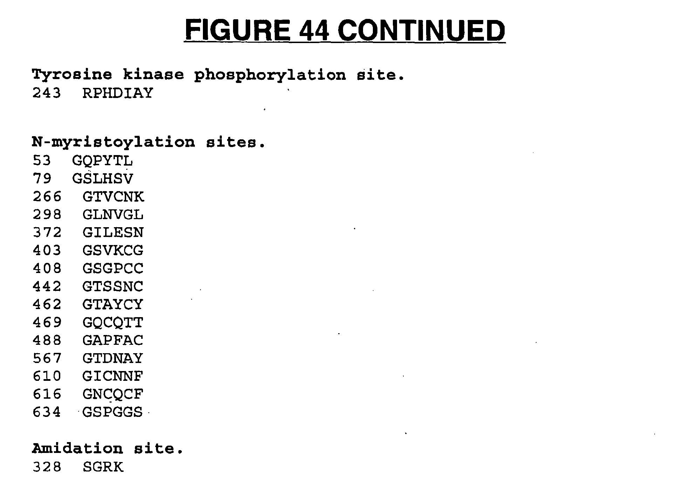

The entire nucleotide sequence of UNQ858 (DNA84925-2514) is shown in Figure 43 (SEQ ID NO:43). Clone UNQ858 (DNA84925-2514) contains a single open reading frame with an apparent translational initiation site at nucleotide positions 43-45 and ending at the stop codon at nucleotide positions 2188-2190 (Figure 43). The predicted polypeptide precursor is 715 amino acids long (Figure 44; SEQ ID NO:44). The full-length PRO1867 protein shown in Figure 44 has an estimated molecular weight of about 80,183 daltons and a pI of about 7.62. Analysis of the full-length PRO1867 sequence shown in Figure 44 (SEQ ID NO:44) evidences the presence of the following: a signal peptide from about amino acid 1 to about amino acid 16, a transmembrane domain from about amino acid 665 to about amino acid 684, potential N-glycosylation sites from about amino acid 36 to about amino acid 39, from about amino acid 76 to about amino acid 79, from about amino acid 122 to about amino acid 125, from about amino acid 149 to about amino acid 152, from about amino acid 156 to about amino acid 159, from about amino acid 177 to about amino acid 180, from about amino acid 270 to about amino acid 273, from about amino acid 335 to about amino acid 338, from about amino acid 441 to about amino acid 444, from about amino acid 537 to about amino acid 540, from about amino acid 587 to about amino acid 590, from about amino acid 601 to about amino acid 604 and from about amino acid 703 to about amino acid 706, casein kinase II phosphorylation sites from about amino acid 74 to about amino acid 77, from about amino acid 208 to about amino acid 211, from about amino acid 221 to about amino acid 224, from about amino acid 304 to about amino acid 307, from about amino acid 337 to about amino acid 340, from about amino acid 346 to about amino acid 349, from about amino acid 376 to about amino acid 379, from about amino acid 415 to about amino acid 418, from about amino acid 499 to about amino acid 502, from about amino acid 639 to about amino acid 642 and from about amino acid 708 to about amino acid 711, a tyrosine kinase phosphorylation site from about amino acid 243 to about amino acid 249, potential N-myristolation sites from about amino acid 53 to about amino acid 58, from about amino acid 79 to about amino acid 84, from about amino acid 266 to about amino acid 271, from about amino acid 298 to about amino acid 303, from about amino acid 372 to about amino acid 377, from about amino acid 403 to about amino acid 408, from about amino acid 408 to about amino acid 413, from about amino acid 442 to about amino acid 447, from about amino acid 462 to about amino acid 467, from about amino acid 469 to about amino acid 474, from about amino acid 488 to about amino acid 493, from about amino acid 567 to about amino acid 572, from about amino acid 610 to about amino acid 615, from about amino acid 616 to about amino acid 621 and from about amino acid 634 to about amino acid 639 and an amidation site from about amino acid 328 to about amino acid 331. Clone UNQ858 (DNA84925-2514) has been deposited with ATCC on December 22, 1998 and is assigned ATCC deposit no. 203548.

An analysis of the Dayhoff database (version 35.45 SwissProt 35), using a WU-BLAST2 sequence alignment analysis of the full-length sequence shown in Figure 44 (SEQ ID NO:44), evidenced significant homology between the PRO1867 amino acid sequence and the following Dayhoff sequences: MFTMDCIII_1, S47656, GEN12835, P_R87037, HSU52370_1, P_W44120, XLU78185_1, AF029899_1, AF023477_1 and GEN 14265.

### EXAMPLE 25: Isolation of cDNA clones Encoding Human PRO1890 Polypeptides [UNQ872]

A consensus DNA sequence was assembled relative to other EST sequences using repeated cycles of BLAST and phrap as described in Example 1 above. This consensus sequence is herein designated DNA52162. Based on the DNA52162 consensus sequence, oligonucleotides were synthesized: 1) to identify by PCR a cDNA library that contained the sequence of interest, and 2) for use as probes to isolate a clone of the full-length coding sequence for PRO1890.

PCR primers (forward and reverse) were synthesized:
forward PCR primer (52162.f1) 5'-CACCAACCAACTGCCAATCCTGGC-3' (SEQ ID NO:151)
reverse PCR primer (52162.r1) 5'-ACCACATTCTGATGGGTGTCTCCTGG-3' (SEQ ID NO:152) Additionally, a synthetic oligonucleotide hybridization probe was constructed from the consensus DNA52162 sequence which had the following nucleotide sequence
hybridization probe (52162.p1)
   5'-GGGTCCCTACCTTTACCAGTGGAATGATGACAGGTGTAACATGAAGCAC-3' (SEQ ID NO:153)

RNA for construction of the cDNA libraries was isolated from human fetal kidney tissue.

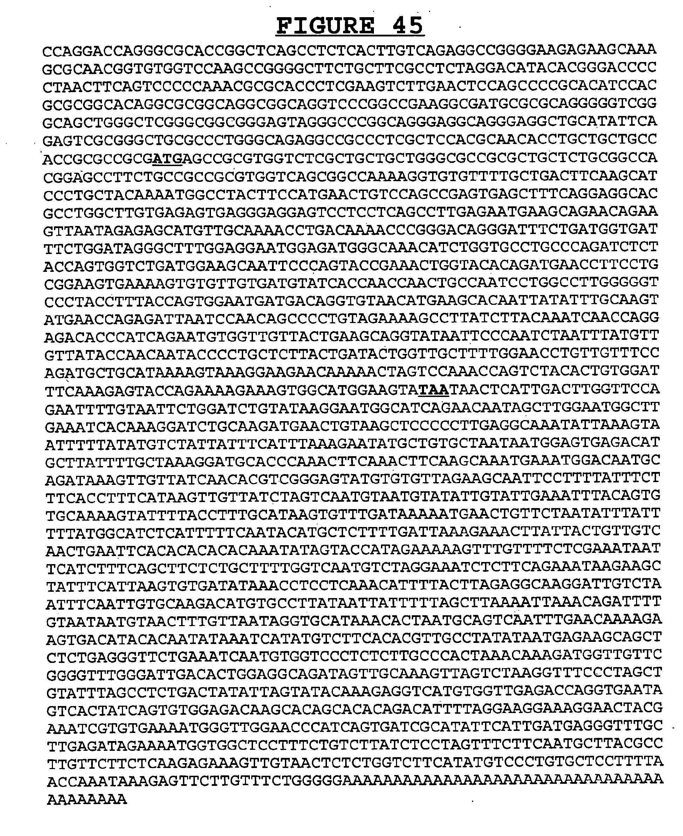

DNA sequencing of the clones isolated as described above gave the full-length DNA sequence for PRO1890 (designated herein as DNA79230-2525 [Figure 45, SEQ ID NO:45]; (UNQ872) and the derived protein sequence for PRO1890.

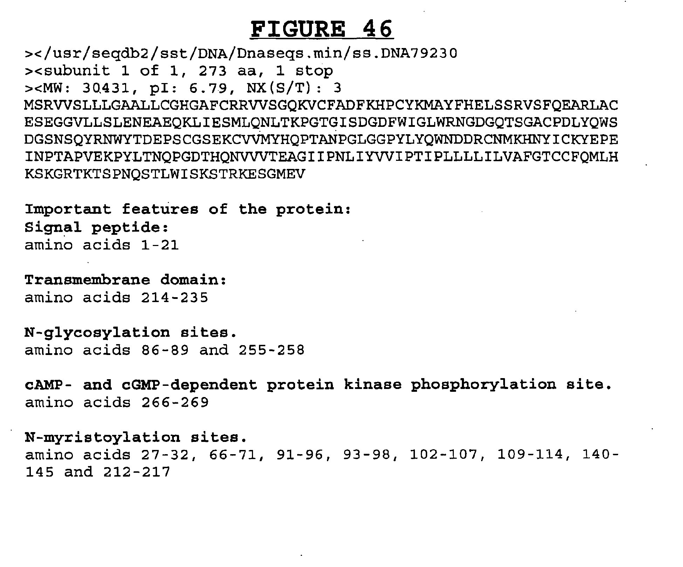

The entire nucleotide sequence of DNA79230-2525 is shown in Figure 45 (SEQ ID NO:45). Clone DNA79230-2525 contains a single open reading frame with an apparent translational initiation site at nucleotide positions 378-380 and ending at the stop codon at nucleotide positions 1197-1199 (Figure 45). The predicted polypeptide precursor is 273 amino acids long (Figure 46). The full-length PRO1890 protein shown in Figure 46 has an estimated molecular weight of about 30,431 daltons and a pI of about 6.79. Analysis of the full-length PRO1890 sequence shown in Figure 46 (SEQ ID NO:46) evidences the presence of the following: a signal peptide from about amino acid 1 to about amino acid 21, a transmembrane domain from about amino acid 214 to about amino acid 235, potential N-glycosylation sites from about amino acid 86 to about amino acid 89 and from about amino acid 255 to about amino acid 258, a cAMP- and cGMP-dependent protein kinase phosphorylation site from about amino acid 266 to about amino acid 269 and potential N-myristolation sites from about amino acid 27 to about amino acid 32, from about amino acid 66 to about amino acid 71, from about amino acid 91 to about amino acid 96, from about amino acid 93 to about amino acid 98, from about amino acid 102 to about amino acid 107, from about amino acid 109 to about amino acid 114, from about amino acid 140 to about amino acid 145 and from about amino acid 212 to about amino acid 217. Clone DNA79230-2525 has been deposited with ATCC on December 22, 1998 and is assigned ATCC deposit no. 203549.

An analysis of the Dayhoff database (version 35.45 SwissProt 35), using a WU-BLAST2 sequence alignment analysis of the full-length sequence shown in Figure 46 (SEQ ID NO:46), evidenced significant homology between the PRO1890 amino acid sequence and the following Dayhoff sequences: AF093673_1, P_W44118, AB014609_1, AC005254_1, AF026547_1, LEC2_MEGRO, PGCV_HUMAN, GEN12667, P_R06331 and CELF52E1_9.

### EXAMPLE 26: Isolation of cDNA clones Encoding Human PRO3438 Polypeptides [UNQ1825]

DNA82364-2538 was identified by applying the proprietary signal sequence finding algorithm described in Example 3 above. Use of the above described signal sequence algorithm allowed identification of an EST sequence from the LIFESEQ® database, designated Incyte EST187233H1. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA73888.

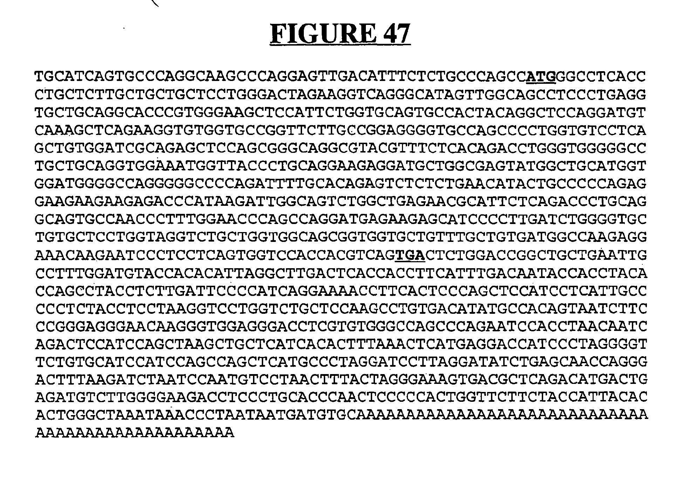

In light of the sequence homology between the DNA73888 consensus sequence and the Incyte EST187233H1, the clone including this EST was purchased and the cDNA insert was obtained and sequenced. The sequence of this cDNA insert is shown in Figure 47 (SEQ ID NO:47) and is herein designated as DNA82364-2538.

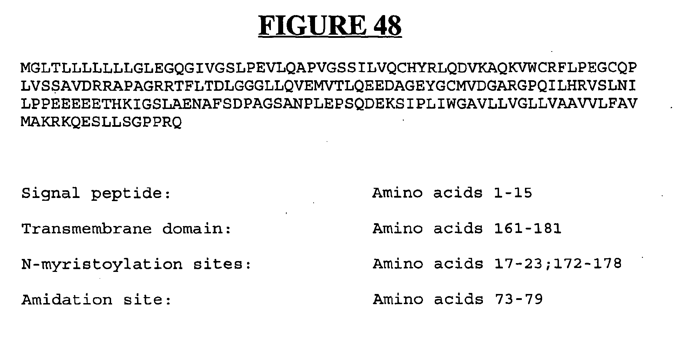

Clone DNA823 64-253 8 contains a single open reading frame with an apparent translational initiation site at nucleotide positions 50-52 and ending at the stop codon at nucleotide positions 647-649 (Figure 47). The predicted polypeptide precursor is 199 amino acids long (Figure 48; SEQ ID NO:48). The full-length PRO3438 protein shown in Figure 48 has an estimated molecular weight of about 21,323 daltons and a pI of about 5.05. Analysis of the full-length PRO3438 sequence shown in Figure 48 (SEQ ID NO:48) evidences the presence of a variety of important polypeptide domains, wherein the locations given for those important polypeptide domains are approximate as described above. Analysis of the full-length PRO3438 sequence shown in Figure 48 evidences the presence of the following: a signal peptide from about amino acid 1 to about amino acid 15; a transmembrane domain from about amino acid 161 to about amino acid 181; N-myristoylation sites from about amino acid 17 to about amino acid 23 and from about amino acid 172 to about amino acid 178; and an amidation site from about amino acid 73 to about amino acid 79. Clone DNA82364-2538 has been deposited with ATCC on January 20, 1999 and is assigned ATCC deposit no. 203603.

An analysis of the Dayhoff database (version 35.45 SwissProt 35), using a WU-BLAST2 sequence alignment analysis of the full-length sequence shown in Figure 48 (SEQ ID NO:48), evidenced homology between the PRO3438 amino acid sequence and the following Dayhoff sequences: S48841, P_W03179, P_W03178, PIGR_HUMAN, HGS_A215, AB001489_1, HGS_B471, P_W61380, P_R15068 and MML1L_1.

### EXAMPLE 27: Isolation of cDNA clones Encoding Human PRO4999 Polypeptides [UNQ2438]

A consensus DNA sequence was assembled relative to other EST sequences using phrap as described in Example 1 above. This consensus sequence is herein designated DNA86634. Based on the DNA86634 consensus sequence, oligonucleotides were synthesized: 1) to identify by PCR a cDNA library that contained the sequence of interest, and 2) for use as probes to isolate a clone of the full-length coding sequence for PRO4999.

PCR primers (forward and reverse) were synthesized:
forward PCR primer 5'-CCACTTGCCATGAACATGCCAC-3' (SEQ ID NO:154)
reverse PCR primer 5'-CCTCTTGACAGACATAGCGAGCCAC-3' (SEQ ID NO:155) Additionally, a synthetic oligonucleotide hybridization probe was constructed from the consensus DNA86634 sequence which had the following nucleotide sequence
hybridization probe
   5'-CACTCTTGTCTGTGGGAACCACACATCTTGCCACAACTGTGGC-3' (SEQ ID NO:156)

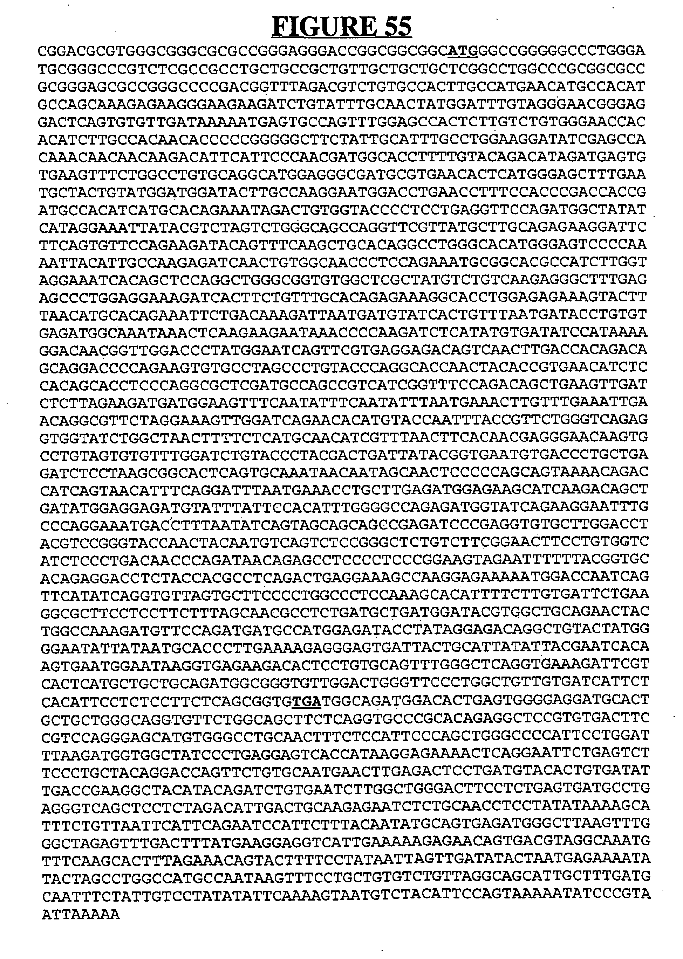

RNA for construction of the cDNA libraries was isolated from human testis tissue. DNA sequencing of the clones isolated as described above gave the full-length DNA sequence for a full-length PRO4999 polypeptide (designated herein as DNA96031-2664 [Figure 55, SEQ ID NO:55]) and the derived protein sequence for that PRO4999 polypeptide.

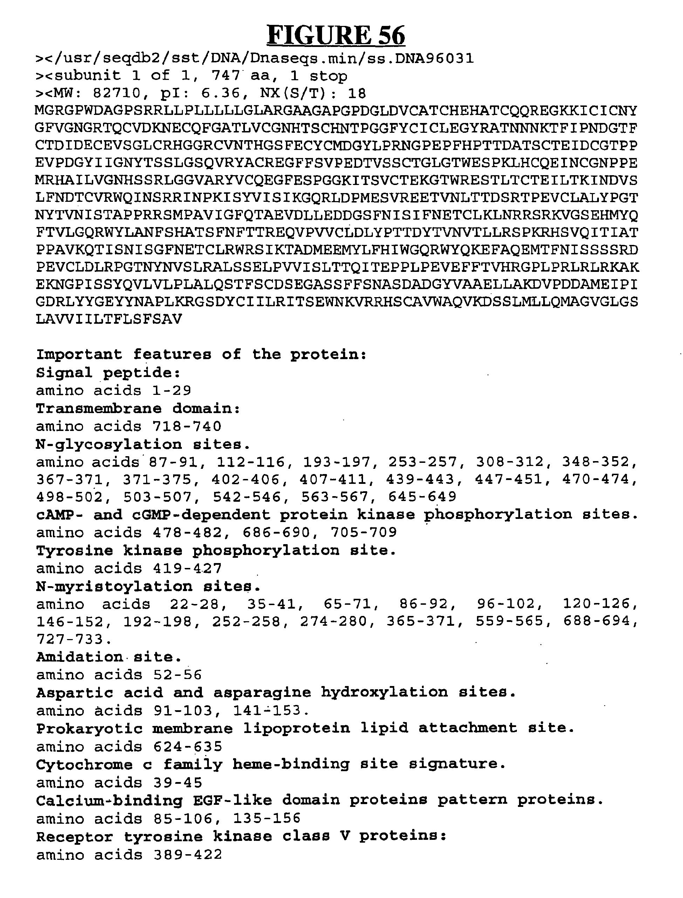

The full length clone identified above contained a single open reading frame with an apparent translational initiation site at nucleotide positions 42-44 and a stop signal at nucleotide positions 2283-2285 (Figure 55, SEQ ID NO:55). The predicted polypeptide precursor is 747 amino acids long, has a calculated molecular weight of approximately 82,710 daltons and an estimated pI of approximately 6.36. Analysis of the full-length PRO4999 sequence shown in Figure 56 (SEQ ID NO:56) evidences the presence of a variety of important polypeptide domains as shown in Figure 56, wherein the locations given for those important polypeptide domains are approximate as described above. Clone DNA96031-2664 has been deposited with ATCC on June 15, 1999 and is assigned ATCC deposit no. PTA-237.

An analysis of the Dayhoff database (version 35.45 SwissProt 35), using the ALIGN-2 sequence alignment analysis of the full-length sequence shown in Figure 56 (SEQ ID NO:56), evidenced sequence identity between the PRO4999 amino acid sequence and the following Dayhoff sequences: UROM_HUMAN; FBN1_HUMAN; GGU88872_1; S52111; GEN12408; P_R79478; P_W48756; P_R53087; P_R14584; and S78549.

### EXAMPLE 28: Isolation of cDNA clones Encoding Human PRO5778 Polypeptides [UNQ2491]

DNA96894-2675 was identified by applying a proprietary signal sequence fmding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ® database (Incyte Pharmaceuticals, Inc., Palo Alto, CA) designated herein as CLU191697. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA81206.

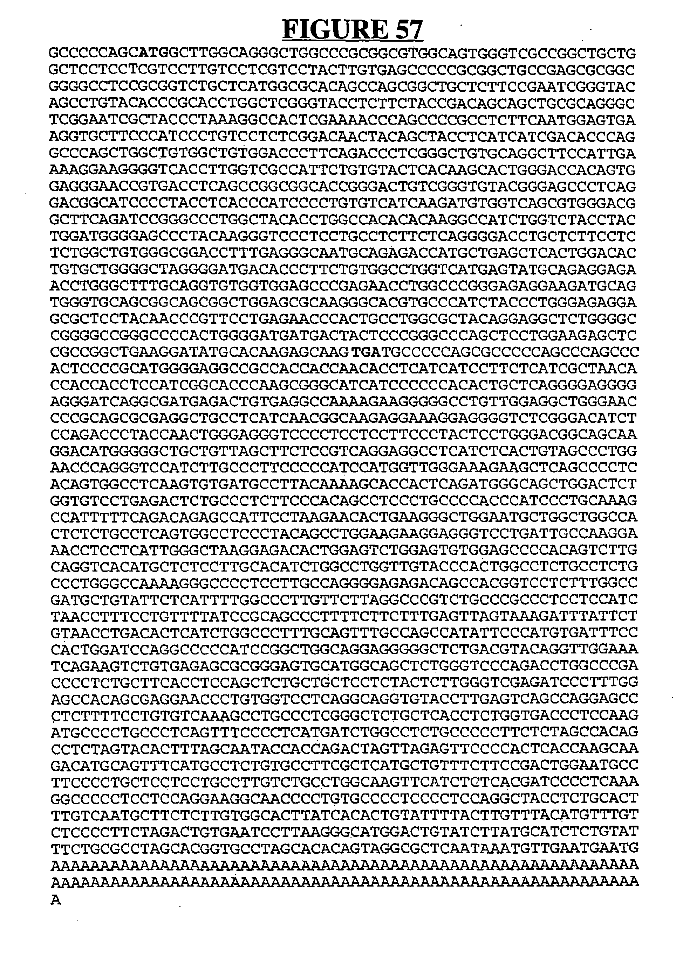

In light of an observed sequence homology between the DNA81206 sequence and an EST sequence encompassed within clone no. 3250090H1 from the LIFESEQ® database, clone no. 3250090H1 was purchased and the cDNA insert was obtained and sequenced. It was found herein that that cDNA insert encoded a full-length protein. The sequence of this cDNA insert is shown in Figure 57 and is herein designated as DNA96894-2675.

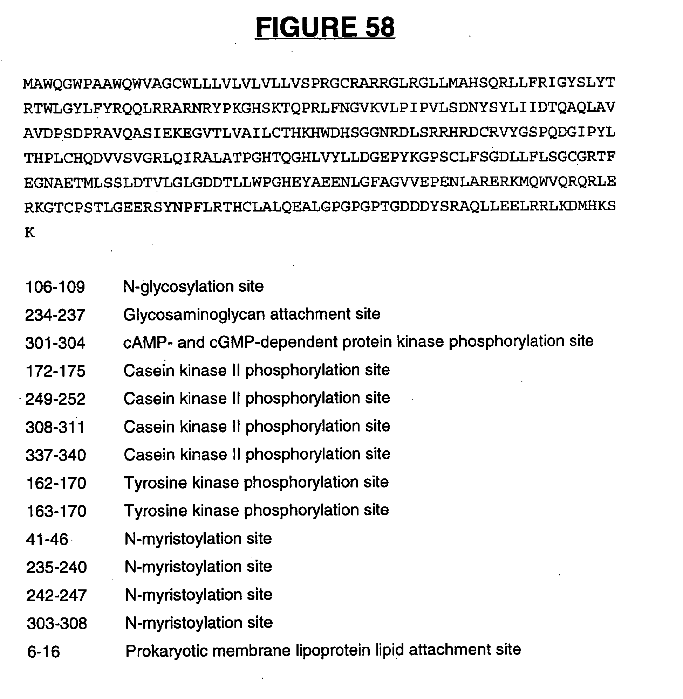

Clone DNA96894-2675 contains a single open reading frame with an apparent translational initiation site at nucleotide positions 10-12 and ending at the stop codon at nucleotide positions 1093-1095 (Figure 57; SEQ ID NO:57). The predicted polypeptide precursor is 361 amino acids long (Figure 58; SEQ ID NO:58). The full-length PRO5778 protein shown in Figure 58 has an estimated molecular weight of about 40747 daltons and a pI of about 9.20. Analysis of the full-length PRO5778 sequence shown in Figure 58 (SEQ ID NO:58) evidences the presence of a variety of important polypeptide domains as shown in Figure 58, wherein the locations given for those important polypeptide domains are approximate as described above. Clone DNA96894-2675 has been deposited with ATCC on June 22, 1999 and is assigned ATCC deposit no. PTA-260.

An analysis of the Dayhoff database (version 35.45 SwissProt 35), using the ALIGN-2 sequence alignment analysis of the full-length sequence shown in Figure 58 (SEQ ID NO:58), evidenced sequence identity between the PRO5778 amino acid sequence and the following Dayhoff sequences: JC5826; GLO2_HUMAN; GEN14048; P_W80783; GLO2_CALJA; S74799; ATU90928_1; GLO2_ECOLI; A70483; F70790.

### EXAMPLE 29: Isolation of cDNA clones Encoding Human PRO5997 Polypeptides [UNQ2509]

A cDNA clone (DNA97005-2687) encoding a native human PRO5997 polypeptide was identified using a yeast screen, in a human testis cDNA library that preferentially represents the 5' ends of the primary cDNA clones.

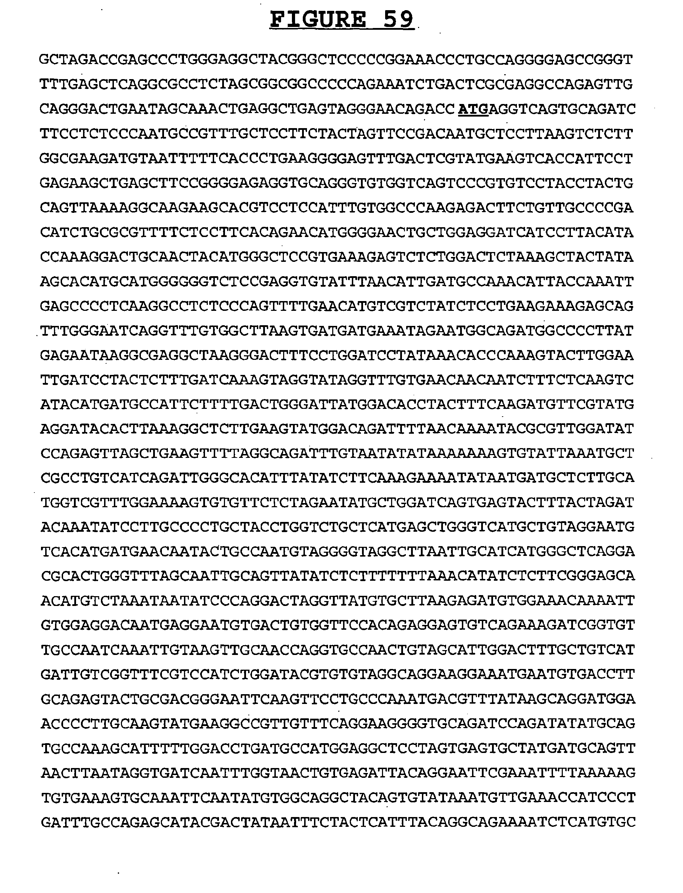

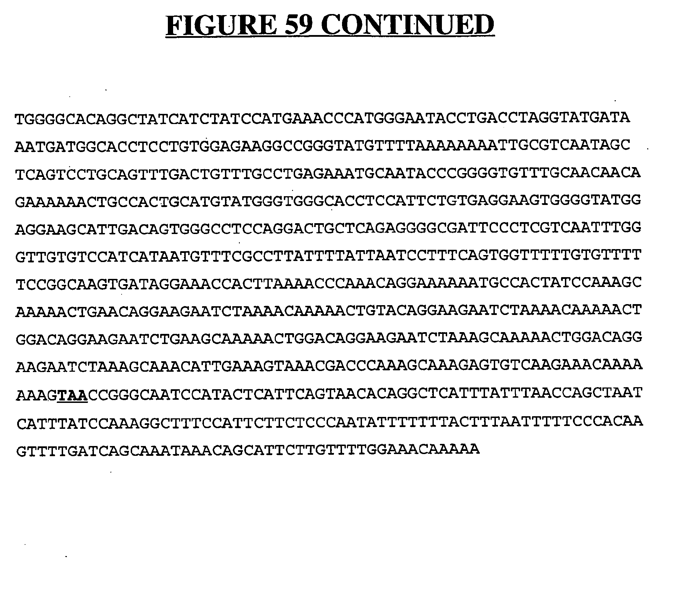

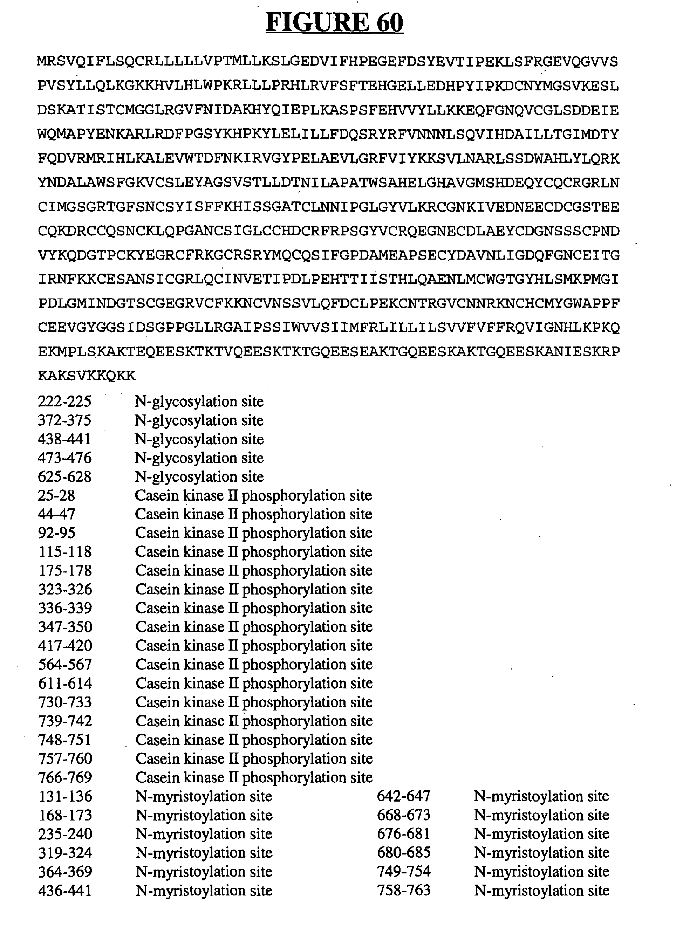

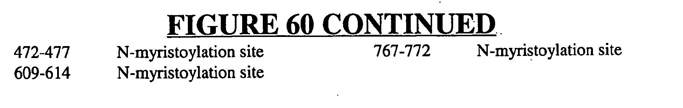

Clone DNA97005-2687 contains a single open reading frame with an apparent translational initiation site at nucleotide positions 163-165 and ending at the stop codon at nucleotide positions 2533-2535 (Figure 59; SEQ ID NO:59). The predicted polypeptide precursor is 790 amino acids long (Figure 60; SEQ ID NO:60). The full-length PRO5997 protein shown in Figure 60 has an estimated molecular weight of about 88,940 daltons and a pI ofabout 7.97. Analysis of the full-length PRO5997 sequence shown in Figure 60 (SEQ ID NO: 60) evidences the presence of a variety of important polypeptide domains as shown in Figure 60, wherein the locations given for those important polypeptide domains are approximate as described above. Clone DNA97005-2687 has been deposited with ATCC on July 20, 1999 and is assigned ATCC Deposit No. PTA-378.

An analysis of the Dayhoff database (version 35.45 SwissProt 35), using the ALIGN-2 sequence alignment analysis of the full-length sequence shown in Figure 60 (SEQ ID NO: 60), evidenced sequence identity between the PRO5997 amino acid sequence and the following Dayhoff sequences: AF029899_1, AF032382_1, P_W25717, GEN12836, P_W25719, P_W01825, MMU38806_1, GEN14265, P_R67759, and P_W73013.

### EXAMPLE 30: Isolation of cDNA clones Encoding Human PRO6079 Polypeptides [UNQ2538]

DNA 111750-2706 was identified by applying a proprietary signal sequence fmding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ®, Incyte Pharmaceuticals, Inc., Palo Alto, CA, database, designated herein as CLU63126. This EST cluster sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA57962.

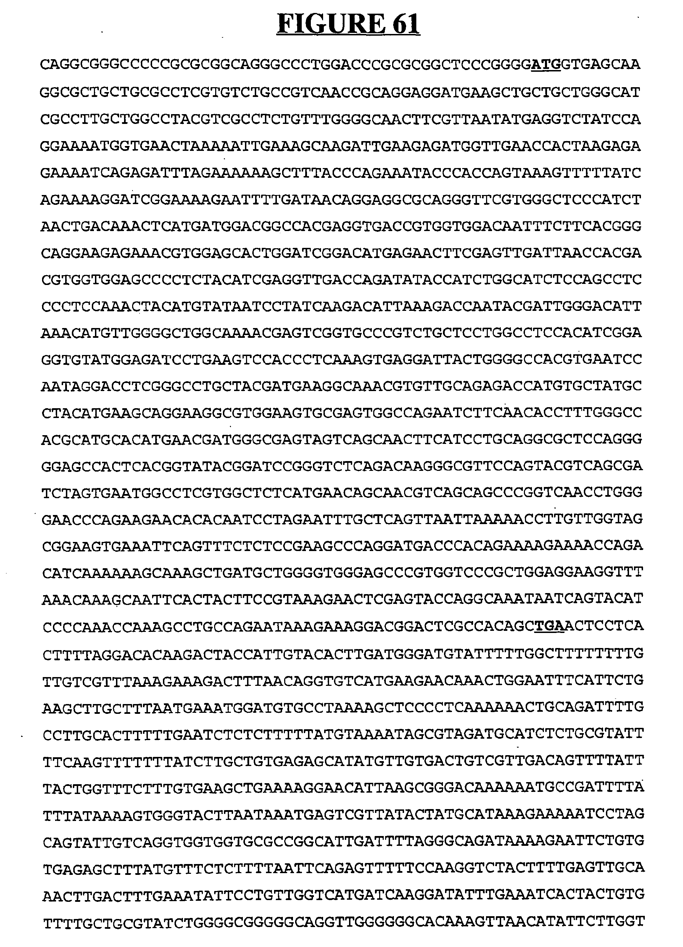

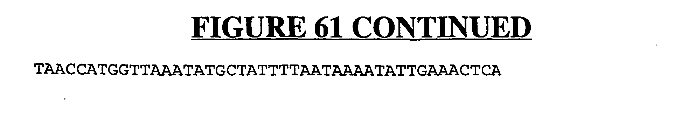

In light of an observed sequence homology between the DNA57962 sequence and an EST sequence encompassed within clone no. 3595637 from the LIFESEQ®, Incyte Pharmaceuticals, Inc., Palo Alto, CA, database, clone no. 3595637 was purchased and the cDNA insert was obtained and sequenced. It was found herein that that cDNA insert encoded a full-length protein. The sequence of this cDNA insert is shown in Figure 61 and is herein designated as DNA111750-2706.

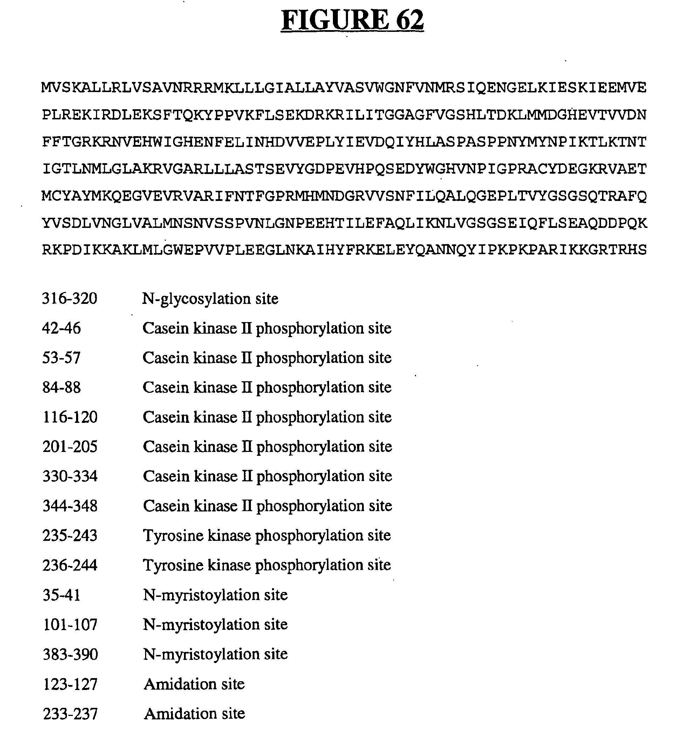

Clone DNA111750-2706 contains a single open reading frame with an apparent translational initiation site at nucleotide positions 50-52 and ending at the stop codon at nucleotide positions 1310-1312 (Figure 61; SEQ ID NO:61). The predicted polypeptide precursor is 420 amino acids long (Figure 62; SEQ ID NO:62). The full-length PRO6079 protein shown in Figure 62 has an estimated molecular weight of about 47,577 daltons and a pI of about 9.19. Analysis of the full-length PRO6079 sequence shown in Figure 62 (SEQ ID NO: 62) evidences the presence of a variety of important polypeptide domains as shown in Figure 62, wherein the locations given for those important polypeptide domains are approximate as described above. Clone DNA111750-2706 has been deposited with ATCC on August 3, 1999 and is assigned ATCC Deposit No. PTA-489.

An analysis of the Dayhoff database (version 35.45 SwissProt 35), using the ALIGN-2 sequence alignment analysis of the full-length sequence shown in Figure 62 (SEQ ID NO: 62), evidenced sequence identity between the PRO6079 amino acid sequence and the following Dayhoff sequences: P_W86312, S75550, T00419, S58282, PAU82433_1, P_W74849, D64326, G70415, AB005901_4, and F64431.

### EXAMPLE 31: Isolation of cDNA clones Encoding Human PRO6090 Polypeptides [UNQ2540]

A consensus DNA sequence was assembled relative to other EST sequences using phrap as described in Example 1 above. This consensus sequence is herein designated "DNA91346". Based on the DNA91346 consensus sequence, oligonucleotides were synthesized: 1) to identify by PCR a cDNA library that contained the sequence of interest, and 2) for use as probes to isolate a clone of the full-length coding sequence for PRO6090.

A pair of PCR primers (forward and reverse) were synthesized:
forward PCR primer 5'-GCTCTGTCTCTACCTGGAGCTACAATGTGTG-3' (SEQ ID NO:157)
reverse PCR primer 5'-TTGGCATAGATGCCAACATCAGCTCTC-3' (SEQ ID NO:158) Additionally, a synthetic oligonucleotide hybridization probe was constructed from the consensus DNA91346 sequence which had the following nucleotide sequence
hybridization probe
   5'-GAGCCCGATTCACTGCAAACTGTGAACATCTCTGTAATCTCCAAGCC-3' (SEQ ID NO:159)

In order to screen several libraries for a source of a full-length clone, DNA from the libraries was screened by PCR amplification with the PCR primer pair identified above. A positive library was then used to isolate clones encoding the PRO6090 gene using the probe oligonucleotide and one of the PCR primers.

RNA for construction of the cDNA libraries was isolated from human testis tissue.

DNA sequencing of the clones isolated as described above gave the full-length DNA sequence for PRO6090 [herein designated as DNA107781-2707] (SEQ ID NO:63) and the derived protein sequence for PRO6090.

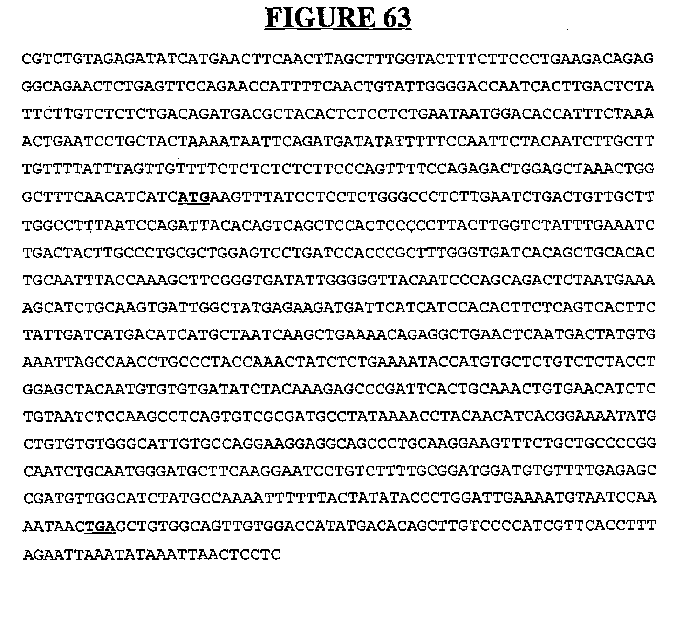

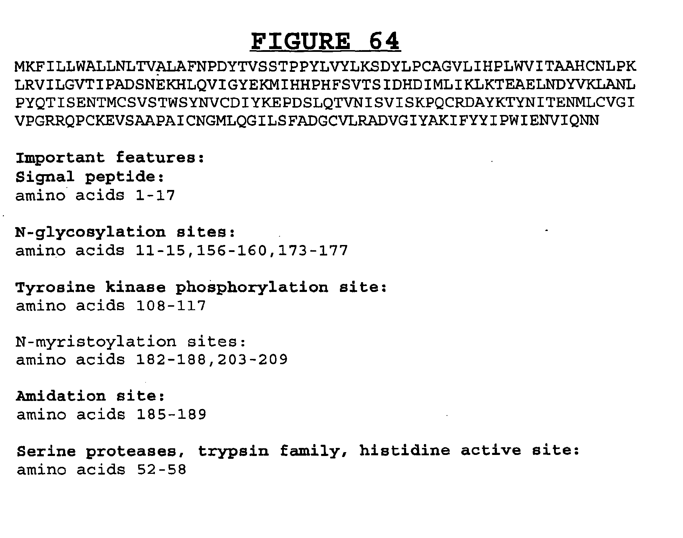

The entire nucleotide sequence of DNA107781-2707 is shown in Figure 63 (SEQ ID NO:63). Clone DNA107781-2707 contains a single open reading frame with an apparent translational initiation site at nucleotide positions 321-323 and ending at the stop codon at nucleotide positions 1044-1046 (Figure 63). The predicted polypeptide precursor is 241 amino acids long, has a calculated molecular weight of approximately 27,085 daltons and an estimated pI of approximately 6.79 (Figure 64; SEQ ID NO:64). Clone DNA107781-2707 has been deposited with ATCC on August 3, 1999 and is assigned ATCC deposit no. PTA-484.

Analysis of the Dayhoff database (version 35.45 SwissProt 35), using ALIGN-2 sequence alignment analysis of the full-length sequence shown in Figure 64 (SEQ ID NO:64), evidenced sequence identity between the PRO6090 amino acid sequence and the following Dayhoff sequences: MMAE000663_3, S55067, AB017030_1, P_W64260, P_W64261, KLK1_RAT, PSS9_HUMAN, P_R44532, P_R67888, and P_W05383.

### EXAMPLE 32: Isolation of cDNA clones Encoding Human PRO7178 Polypeptides [UNO2788]

DNA108789-2748 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the LIFESEQ® database, Incyte Pharmaceuticals, Palo Alto, designated herein as 2876494. This ESTsequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA86019.

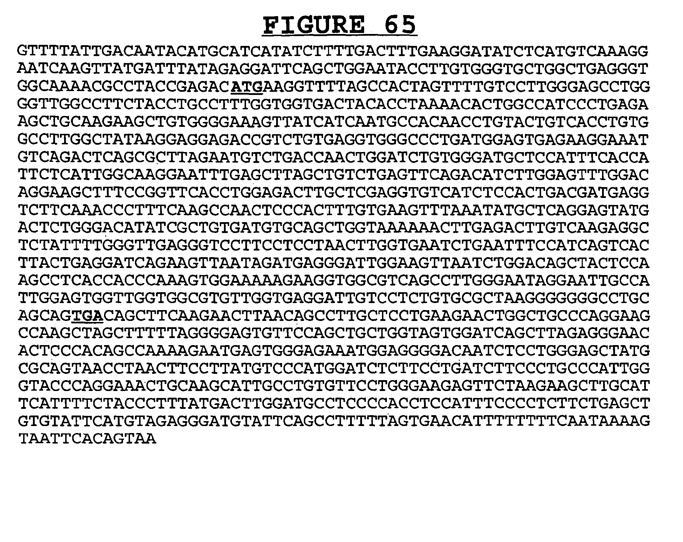

In light of an observed sequence homology between the DNA86019 sequence and an EST sequence encompassed within clone no. 2876494 from the LIFESEQ® database, Incyte Pharmaceuticals, Palo Alto, CA, clone no. 2876494 was purchased and the cDNA insert was obtained and sequenced. It was found herein that that cDNA insert encoded a full-length protein. The sequence of this cDNA insert is shown in Figure 65 and is herein designated as DNA108789-2748.

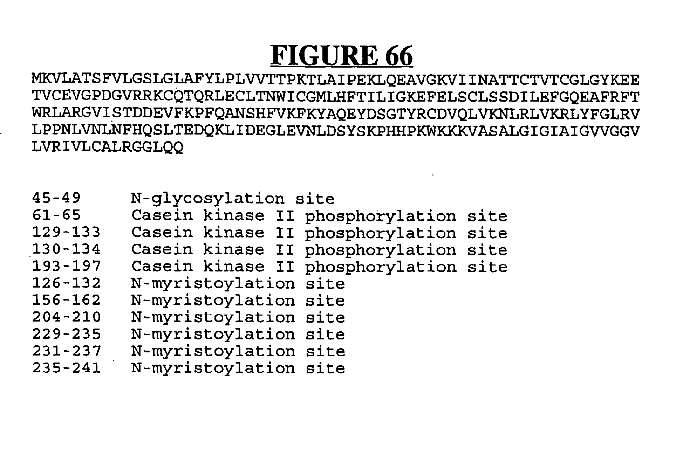

Clone DNA108789-2748 contains a single open reading frame with an apparent translational initiation site at nucleotide positions 141-143 and ending at the stop codon at nucleotide positions 906-908 (Figure 65; SEQ ID NO:65). The predicted polypeptide precursor is 255 amino acids long (Figure 66; SEQ ID NO:66). The full-length PRO7178 protein shown in Figure 66 has an estimated molecular weight of about 28,440 daltons and a pI of about 8.92. Analysis of the full-length PRO7178 sequence shown in Figure 66 (SEQ ID NO: 66) evidences the presence of a variety of important polypeptide domains as shown in Figure 66, wherein the locations given for those important polypeptide domains are approximate as described above. Clone DNA108789-2748 has been deposited with ATCC on August 17, 1999 and is assigned ATCC Deposit No. PTA-547.

An analysis of the Dayhoff database (version 35.45 SwissProt 35), using the ALIGN-2 sequence alignment analysis of the full-length sequence shown in Figure 66 (SEQ ID NO: 66), evidenced sequence identity between the PRO7178 amino acid sequence and the following Dayhoff sequences: P_W78178, HGS_RF163, CMAO2_1, MMU17793_1, AF041082_1, E27639, and LACH_SCHAM

### EXAMPLE 33: Isolation of cDNA clones Encoding Human PRO21184 Polypeptides [UNQ2945]

DNA 167678-2963 was identified by applying a proprietary signal sequence finding algorithm developed by Genentech, Inc. (South San Francisco, CA) upon genomic DNA from public (e.g., GenBank) and/or private databases. In this instance, a genomic sequence from GenBank (Accession No: BR55350) was analyzed using the gene prediction program GENSCAN, licensed from Stanford University. GENSCAN analysis predicts gene coding regions by identifying the potential exons and removing introns, creating DNA sequences which are then subjected to the signal algorithm. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. In order to determine whether the sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of a sequence from the GenBank database, designated herein as Consensus 01.

Based on the Consensus 01 sequence, oligonucleotides were synthesized: 1) to identify by PCR a cDNA library that contained the sequence of interest, and 2) for use as probes to isolate a clone of the full-length coding sequence for PRO21184. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer 5' - CCTGTCGTTCGCAGAGTCCGCTC - 3' (SEQ ID NO: 160)
reverse PCR primer 5' - CACACAGAGGTTCCGGCACCTCTC - 3' (SEQ ID NO: 161) Additionally, a synthetic oligonucleotide hybridization probe was constructed from the consensus DNA167678-2963 sequence which had the following nucleotide sequence
hybridization probe
   5' - TCCTGCAGGCGGCAGATCTGGGAGTAGGTGTGACCGTC - 3' (SEQ ID NO: 162)

RNA for construction of the cDNA libraries was isolated from human tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRK5B or pRK5D; pRK5B is a precursor of pRK5D that does not contain the SfiI site; see, Holmes et al., Science, 253:1278-1280 (1991)) in the unique XhoI and NotI sites.

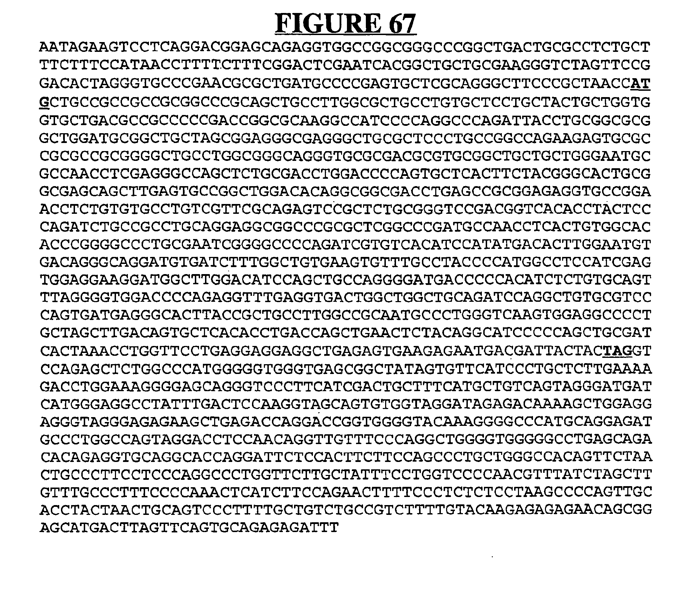

DNA sequencing of the clones isolated as described above gave the full-length DNA sequence for a full-length PRO21184 polypeptide (designated herein as DNA167678-2963 [Figure 67, SEQ ID NO: 67]) and the derived protein sequence for that PRO21184 polypeptide.

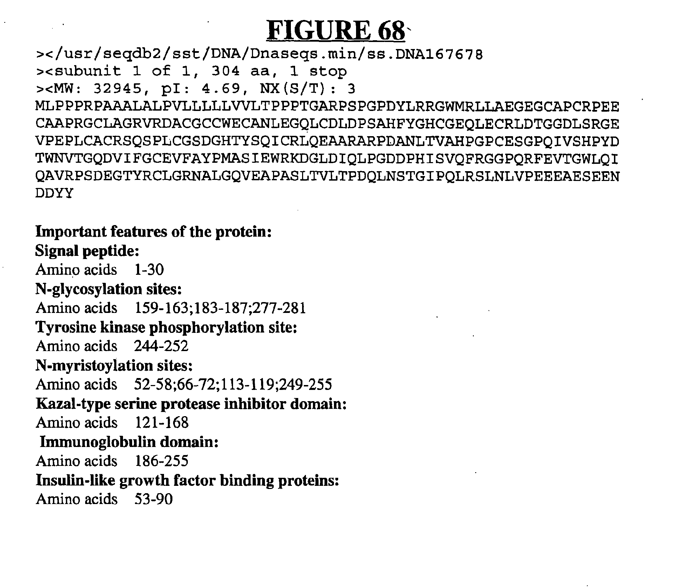

Clone DNA167678-2963 contains a single open reading frame with an apparent translational initiation site at nucleotide positions 182-184 and ending at the stop codon at nucleotide positions 1094-1096 (Figure 67). The predicted polypeptide precursor is 304 amino acids long (Figure 68; SEQ ID NO:68). The full-length PRO21184 protein shown in Figure 68 has an estimated molecular weight of about 32,945 daltons and a pI of about 4.69. Analysis of the full-length PRO21184 sequence shown in Figure 68 (SEQ ID NO:68) evidences the presence of a variety of important polypeptide domains as shown in Figure 68, wherein the locations given for those important polypeptide domains are approximate as described above. Clone DNA167678-2963 has been deposited with ATCC on July 25, 2000 and is assigned ATCC deposit no. PTA-2302.

An analysis of the Dayhoff database (version 35.45 SwissProt 35), using the ALIGN-2 sequence alignment analysis of the full-length sequence shown in Figure 68 (SEQ ID NO:68), evidenced sequence identity between the PRO21184 amino acid sequence and the following Dayhoff sequences:AL133215_1, AB012886_1, M_008048_1, P_Y06833, S50031,and I52825.

### EXAMPLE 34: Isolation of cDNA clones Encoding Human PRO7434 Polypeptides [UNQ2972]

The extracellular domain (ECD) sequences (including the secretion signal sequence, if any) from about 950 known secreted proteins from the Swiss-Prot public database were used to search EST databases. The EST databases included (1) public EST databases (e.g., Merck/Washington University), (2) a proprietary EST database (LIFESEQ®, Incyte Pharmaceuticals, Palo Alto, CA), and (3) a proprietary EST database from Genentech. The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap as described above. This consensus sequence is herein designated DNA40748. In some cases, the DNA40748 consensus sequence derives from an intermediate consensus DNA sequence which was extended using repeated cycles of BLAST and phrap to extend that intermediate consensus sequence as far as possible using the sources of EST sequences discussed above.

Based on the DNA40748 consensus sequence, oligonucleotides were synthesized: 1) to identify by PCR a cDNA library that contained the sequence of interest, and 2) for use as probes to isolate a clone of the full-length coding sequence for PRO7434. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer 5'-ACAACTGCATAGAAGCCCACAACG-3' (SEQ ID NO: 163)
forward PCR primer 5'-ACAACTGCATAGAAGCCCACAACGAATGGCG-3' (SEQ ID NO: 164)
reverse PCR primer 5'-TCTCCCCGTTGGCTTCAGAAATGG-3' (SEQ ID NO: 165)
reverse PCR primer 5'-CGCCATTCGTTGTGGGCTTCTATGCAGTTGT-3' (SEQ ID NO: 166) Additionally, a synthetic oligonucleotide hybridization probe was constructed from the consensus DNA40748 sequence which had the following nucleotide sequence
hybridization probe
   5'-GGTTAGGTGGAATAAAGTCATTCACACCAAGACATGCCATTACGGCTTGG-3' (SEQ ID NO: 167)

RNA for construction of the cDNA libraries was isolated from human testis tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

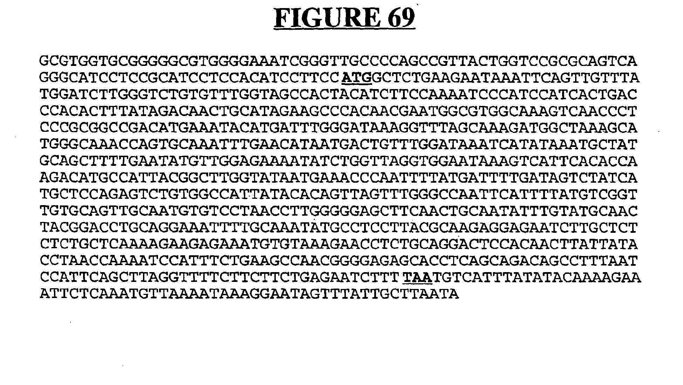

DNA sequencing of the clones isolated as described above gave the full-length DNA sequence for a full-length PRO7434 polypeptide (designated herein as DNA123430-2755 [Figure 69, SEQ ID NO: 69]) and the derived protein sequence for that PRO7434 polypeptide.

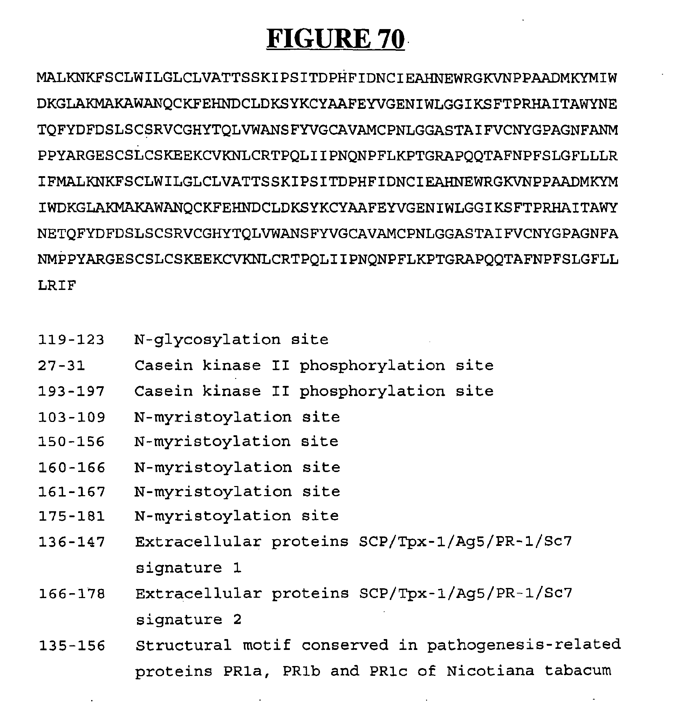

The full length clone identified above contained a single open reading frame with an apparent translational initiation site at nucleotide positions 91-93 and a stop signal at nucleotide positions 817-819 (Figure 69, SEQ ID NO: 69). The predicted polypeptide precursor is 242 amino acids long, has a calculated molecular weight of approximately 27,123 daltons and an estimated pI of approximately 8.64. Analysis of the full-length PRO7434 sequence shown in Figure 70 (SEQ ID NO: 70) evidences the presence of a variety of important polypeptide domains as shown in Figure 70, wherein the locations given for those important polypeptide domains are approximate as described above. Clone DNA123430-2755 has been deposited with ATCC on August 31, 1999 and is assigned ATCC Deposit No. PTA-614.

An analysis of the Dayhoff database (version 35.45 SwissProt 35), using the ALIGN-2 sequence alignment analysis of the full-length sequence shown in Figure 70 (SEQ ID NO: 70), evidenced sequence identity between the PRO7434 amino acid sequence and the following Dayhoff sequences: P_Y07984, GLIP_HUMAN, JC5308, P_Y07891, P_Y13392, CRS3_HUMAN, TPX1_HUMAN, VA5_DOLAR, AEG1_MOUSE, and VA51_VESCR.

### EXAMPLE 35: Isolation of cDNA clones Encoding Human PRO9822 Polypeptides [UNQ3024]

DNA108738-2767 was identified by applying a proprietary signal sequence finding algorithm developed by Genentech, Inc. (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the Incyte database, designated herein as DNA21552, (also designated DNA95709 herein).

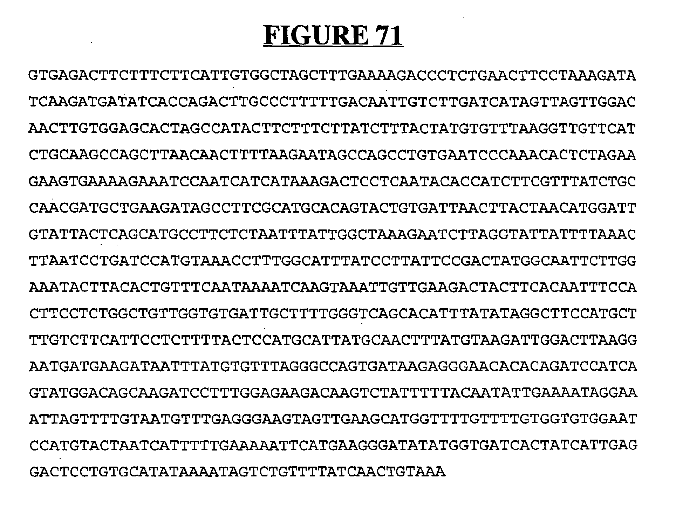

Based on the DNA95709 sequence, clone no. 399828H 1 was purchased from Incyte and the cDNA insert was obtained and sequenced. It was found herein that the cDNA insert encoded a full-length protein. The sequence of this cDNA insert is shown in Figure 71 and is herein designated as DNA108738-2767.

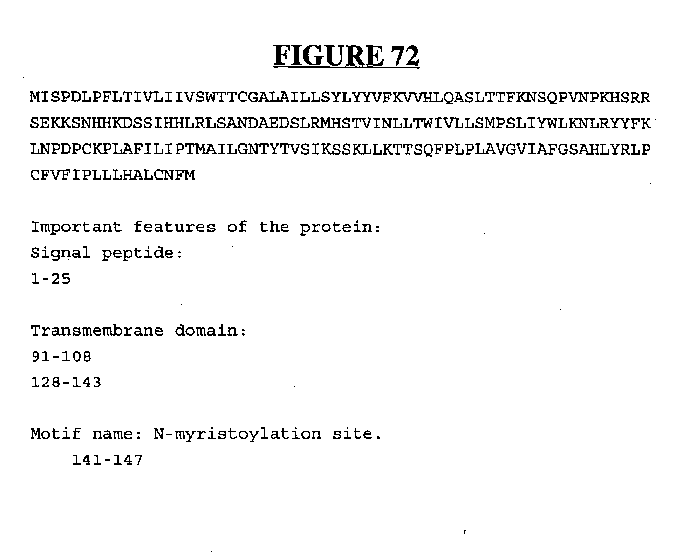

Clone DNA108738-2767 contains a single open reading frame with an apparent translational initiation site at nucleotide positions 67-69 and ending at the stop codon at nucleotide positions 655-657 (Figure 71; SEQ ID NO:71). The predicted polypeptide precursor is 196 amino acids long (Figure 72; SEQ ID NO:72). The full-length PRO9822 protein shown in Figure 72 has an estimated molecular weight of about 22225 daltons and a pI of about 9.9. Analysis of the full-length PRO9822 sequence shown in Figure 72 (SEQ ID NO:72) evidences the presence of a variety of important polypeptide domains as shown in Figure 72, wherein the locations given for those important polypeptide domains are approximate as described above. Clone DNA108738-2767 has been deposited with ATCC on October 19, 1999 and is assigned ATCC deposit no. PTA-862.

An analysis of the Dayhoff database (version 35.45 SwissProt 35), using the ALIGN-2 sequence alignment analysis of the full-length sequence shown in Figure 72 (SEQ ID NO:72), evidenced sequence identity between the PRO9822 amino acid sequence and the following Dayhoff sequences: A65056 and YGBE_ECOLI.

### EXAMPLE 36: Isolation of cDNA clones Encoding Human PRO9833 Polypeptides [UNQ3030]

The extracellular domain (ECD) sequences (including the secretion signal sequence, if any) from about 950 known secreted proteins from the Swiss-Prot public database were used to search EST databases. The EST databases included a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap as described above. This consensus sequence is herein designated DNA33781. In some cases, the DNA33781 consensus sequence derives from an intermediate consensus DNA sequence which was extended using repeated cycles of BLAST and phrap to extend that intermediate consensus sequence as far as possible using the sources of EST sequences discussed above.

An EST clone Incyte 2642942 was identified from the DNA33781 consensus sequence, and oligonucleotides were synthesized based on this clone. These oligonucleotides were used 1) to identify by PCR a cDNA library that contained the sequence of interest, and 2) for use as probes to isolate a clone of the full-length coding sequence for PRO9833. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer 5'-GAGCTGGATCTGCAGAGGAACTAC-3' (SEQ ID NO: 168)
reverse PCR primer 5'-AGGAACCACTCCAGGACGTTGTAG-3' (SEQ ID NO: 169) Additionally, a synthetic oligonucleotide hybridization probe was constructed from the consensus DNA33781 sequence which had the following nucleotide sequence
hybridization probe
   5'-CTTTCGACGGCCTGGCTGAGCTGAGGCACCTCAACCTGGCCTTCAA-3' (SEQ ID NO: 170)

RNA for construction of the cDNA libraries was isolated from human fetal liver tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

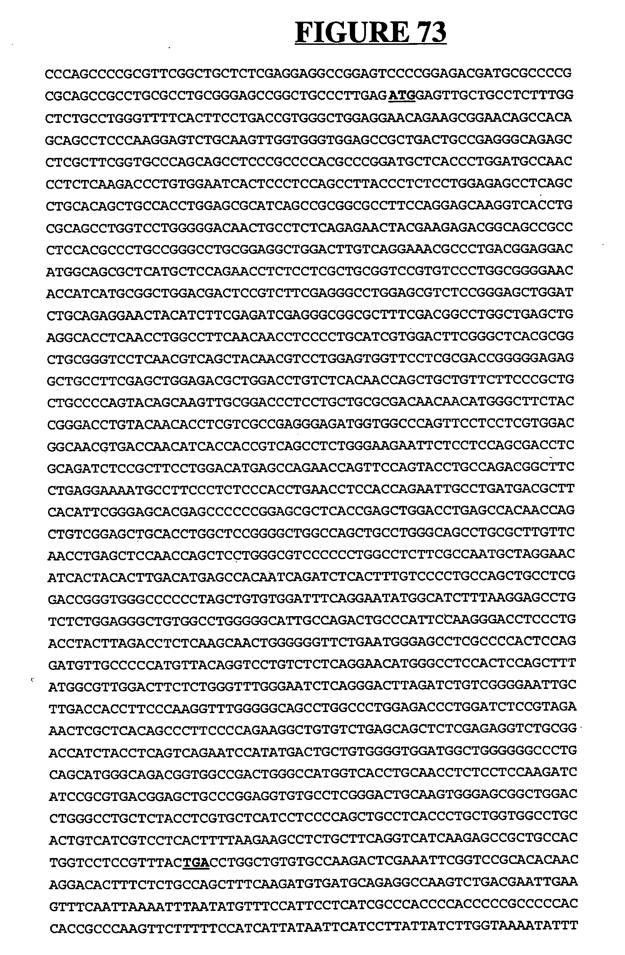

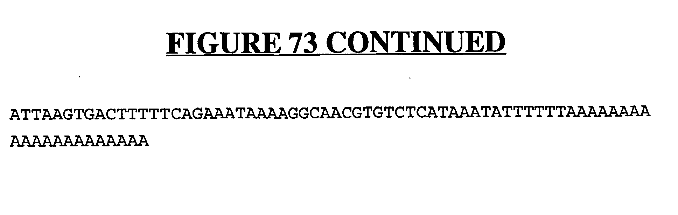

DNA sequencing of the clones isolated as described above gave the full-length DNA sequence for a full-length PRO9833 polypeptide (designated herein as DNA130809-2769 [Figure 73, SEQ ID NO: 73]) and the derived protein sequence for that PRO9833 polypeptide.

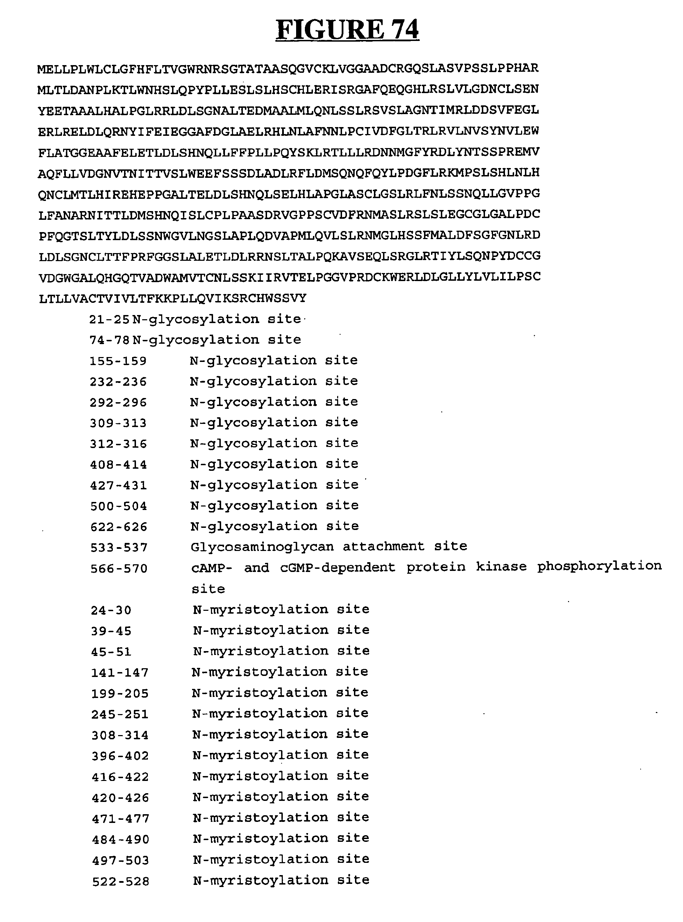

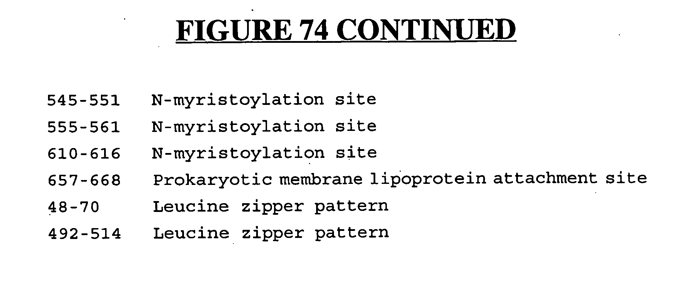

The full length clone identified above contained a single open reading frame with an apparent translational initiation site at nucleotide positions 100-102 and a stop signal at nucleotide positions 2176-2178 (Figure 73, SEQ ID NO: 73). The predicted polypeptide precursor is 692 amino acids long, has a calculated molecular weight of approximately 76366 daltons and an estimated pI of approximately 6.07. Analysis of the full-length PRO9833 sequence shown in Figure 74 (SEQ ID NO: 74) evidences the presence of a variety of important polypeptide domains as shown in Figure 74, wherein the locations given for those important polypeptide domains are approximate as described above. Clone DNA130809-2769 has been deposited with ATCC on November 9, 1999 and is assigned ATCC Deposit No. PTA-949.

An analysis of the Dayhoff database (version 35.45 SwissProt 35), using the ALIGN-2 sequence alignment analysis of the full-length sequence shown in Figure 74 (SEQ ID NO: 74), evidenced sequence identity between the PRO9833 amino acid sequence and the following Dayhoff sequences: GARP_HUMAN; ALS_HUMAN; P_R85889; GPV_MOUSE; P_W93889; AF061443_1; A58532; GPV_HUMAN; DROWHEELER_1; and HSU88879_1.

### EXAMPLE 37: Isolation of cDNA clones Encoding Human PRO9836 Polypeptides [UNQ3034]

DNA119514-2772 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the Incyte Pharmaceuticals, Palo Alto, CA database, designated herein as 3700047. This EST cluster sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA105423.

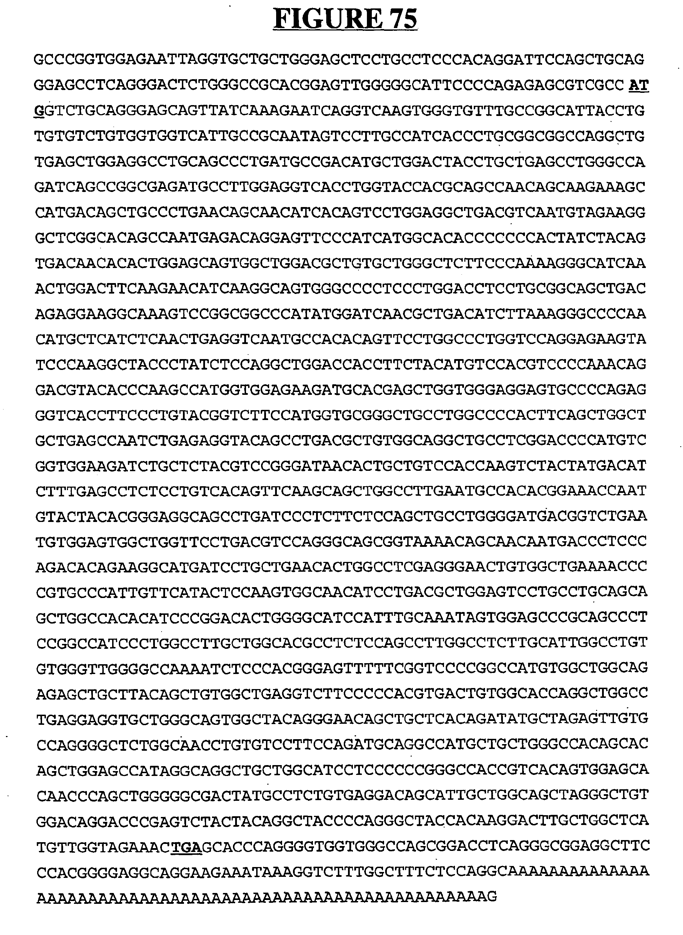

In light of an observed sequence homology between the DNA105423 sequence and an EST sequence encompassed within clone no.192197 from the Incyte Pharmaceuticals, Palo Alto, CA database, clone no.192197 was purchased and the cDNA insert was obtained and sequenced. It was found herein that that cDNA insert encoded a full-length protein. The sequence of this cDNA insert is shown in Figure 75 and is herein designated as DNA119514-2772.

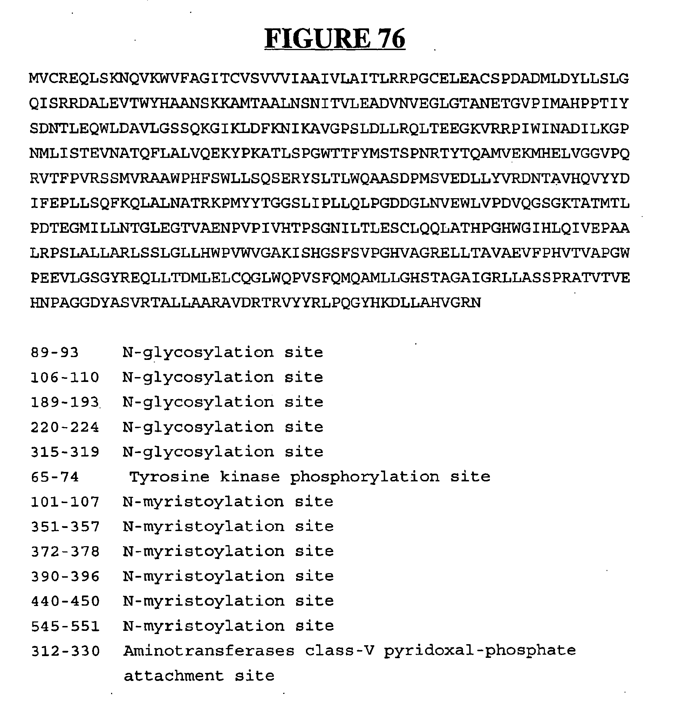

Clone DNA119514-2772 contains a single open reading frame with an apparent translational initiation site at nucleotide positions 119-121 and ending at the stop codon at nucleotide positions 1874-1876 (Figure 75; SEQ ID NO:75). The predicted polypeptide precursor is 585 amino acids long (Figure 76; SEQ ID NO:76). The full-length PRO9836 protein shown in Figure 76 has an estimated molecular weight of about 64056 daltons and a pI of about 6.58. Analysis of the full-length PRO9836 sequence shown in Figure 76 (SEQ ID NO: 76) evidences the presence of a variety of important polypeptide domains as shown in Figure 76, wherein the locations given for those important polypeptide domains are approximate as described above. Clone DNA119514-2772 has been deposited with ATCC on November 9, 1999 and is assigned ATCC Deposit No. PTA-946.

An analysis of the Dayhoff database (version 35.45 SwissProt 35), using the ALIGN-2 sequence alignment analysis of the full-length sequence shown in Figure 76 (SEQ ID NO: 76), evidenced sequence identity between the PRO9836 amino acid sequence and the following Dayhoff sequences: A30227; CEW01F3_2 W01F3.1a; CEW01F3_1 W01F3.1b; B30227; MSK_MOUSE; I49072; AF176069_1; T00259; AB020480_1; and S70434.

### EXAMPLE 38: Isolation of cDNA clones Encoding Human PRO9854 Polypeptides [UNQ3039]

The extracellular domain (ECD) sequences (including the secretion signal sequence, if any) from about 950 known secreted proteins from the Swiss-Prot public database were used to search EST databases. The EST databases included (1) public EST databases (e.g., Merck/Washington University), and (2) a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap as described above. This consensus sequence is herein designated DNA81131. In some cases, the DNA81131 consensus sequence derives from an intermediate consensus DNA sequence which was extended using repeated cycles of BLAST and phrap to extend that intermediate consensus sequence as far as possible using the sources of EST sequences discussed above.

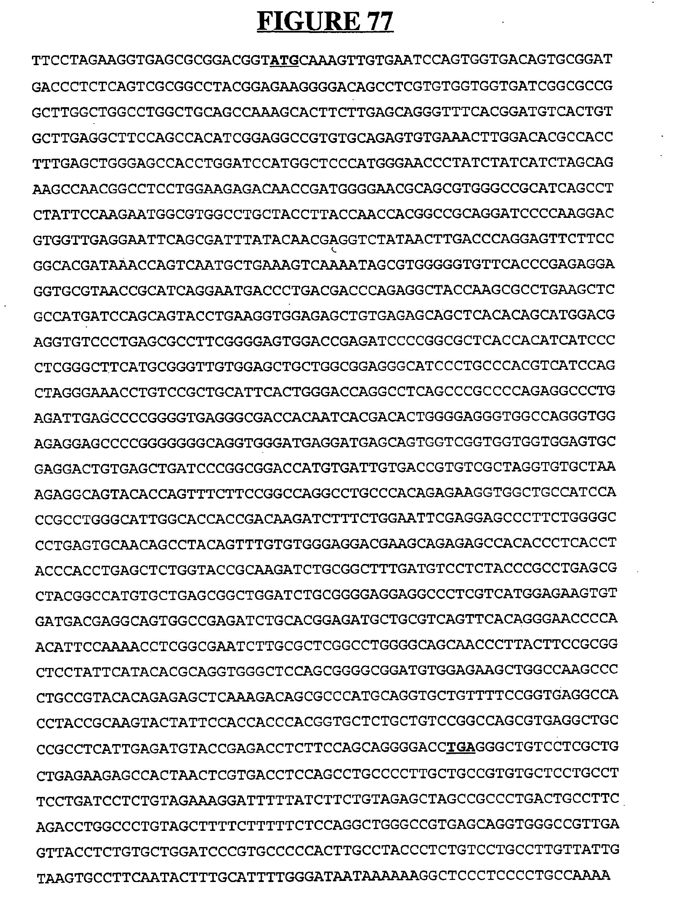

Based on the DNA81131 consensus sequence, and in light of an observed sequence homology between the DNA81131 sequence and an EST sequence encompassed within clone no. 3323937 from the LIFESEQ®, Incyte Pharmaceuticals, Palo Alto, CA database, clone no. 3323937 was purchased and the cDNA insert was obtained and sequenced. It was found herein that that cDNA insert encoded a full-length protein. The sequence of this cDNA insert is shown in Figure 77 and is herein designated as DNA108771-2776.

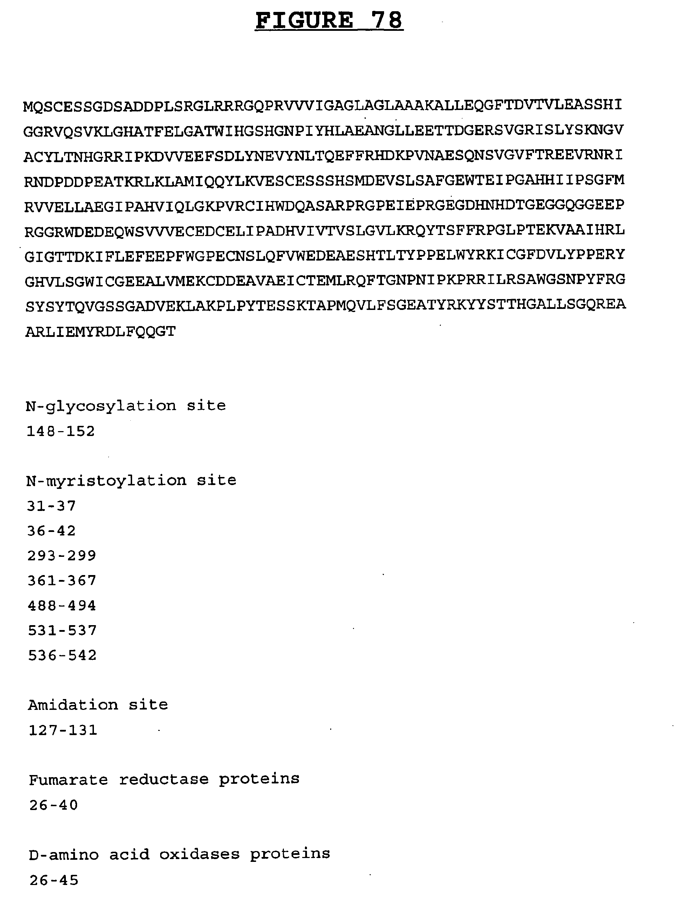

The full length clone identified above contained a single open reading frame with an apparent translational initiation site at nucleotide positions 26 to 28 and a stop signal at nucleotide positions 1691 to 1693 (Figure 77, SEQ ID NO: 77). The predicted polypeptide precursor is 555 amino acids long, has a calculated molecular weight of approximately 61845 daltons and an estimated pI of approximately 5.37. Analysis of the full-length PRO9854 sequence shown in Figure 78 (SEQ ID NO: 78) evidences the presence of a variety of important polypeptide domains as shown in Figure 78, wherein the locations given for those important polypeptide domains are approximate as described above. Clone DNA 108771-2776 has been deposited with ATCC on November 9, 1999 and is assigned ATCC Deposit No. PTA-948.

An analysis of the Dayhoff database (version 35.45 SwissProt 35), using the ALIGN-2 sequence alignment analysis of the full-length sequence shown in Figure 78 (SEQ ID NO: 78), evidenced sequence identity between the PRO9854 amino acid sequence and the following Dayhoff sequences: T09935; AF091328; AB011173_1; ATAC00622412; P_R44295; FMS1_YEAST; PY00263; P_W96805; P_Y06480; P_W61278

### EXAMPLE 39: Isolation of cDNA clones Encoding Human PRO9862 Polypeptides [UNQ3046]

DNA125148-2782 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST sequence from the LIFESEQ® database, Incyte Pharmaceuticals, Palo Alto, CA , designated herein as 304120H1. This EST sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA111060.

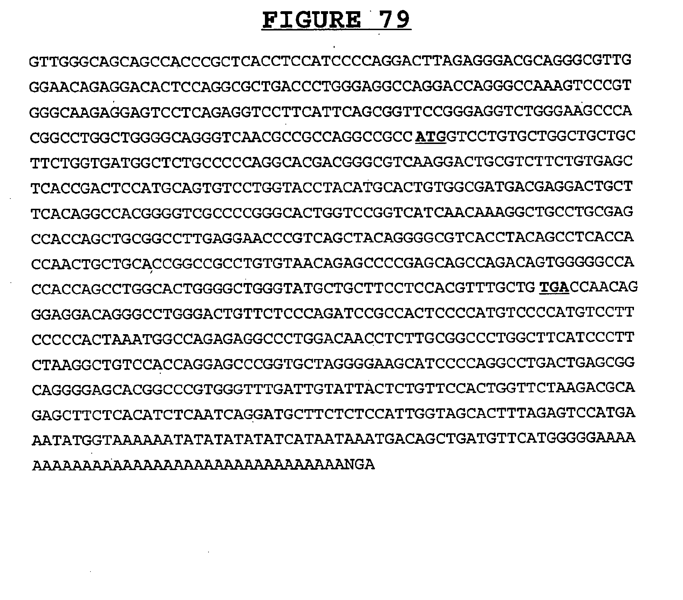

In light of an observed sequence homology between the DNA111060 sequence and an EST sequence encompassed within clone no. 5134469 from the LIFESEQ® database, Incyte Pharmaceuticals, Palo Alto, CA, clone no. 5134469 was purchased and the cDNA insert was obtained and sequenced. It was found herein that that cDNA insert encoded a full-length protein. The sequence of this cDNA insert is shown in Figure 79 and is herein designated as DNA125148-2782.

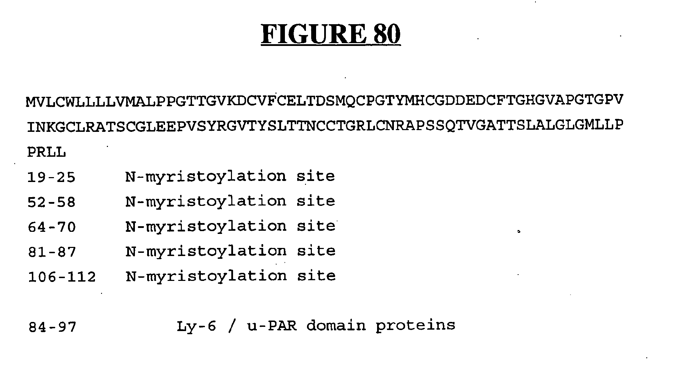

Clone DNA125148-2782 contains a single open reading frame with an apparent translational initiation site at nucleotide positions 219-221 and ending at the stop codon at nucleotide positions 591-593 (Figure 79; SEQ ID NO:79). The predicted polypeptide precursor is 124 amino acids long (Figure 80; SEQ ID NO:80). The full-length PRO9862 protein shown in Figure 80 has an estimated molecular weight of about 13,004 daltons and a pI of about 5.70. Analysis of the full-length PRO9862 sequence shown in Figure 80 (SEQ ID NO:80) evidences the presence of a variety of important polypeptide domains as shown in Figure 80, wherein the locations given for those important polypeptide domains are approximate as described above. Clone DNA125148-2782 has been deposited with ATCC on November 16, 1999 and is assigned ATCC Deposit No. PTA-955.

An analysis of the Dayhoff database (version 35.45 SwissProt 35), using the ALIGN-2 sequence alignment analysis of the full-length sequence shown in Figure 80 (SEQ ID NO: 80), evidenced sequence identity between the PRO9862 amino acid sequence and the following Dayhoff sequences: HSA245419_1, THYB_MOUSE, P_Y13938, P_W70522, P_W86024, P_W80956, AF043498_1, P_W62066, FRU90880_9, and HSA012008_5.

### EXAMPLE 40: Isolation of cDNA clones Encoding Human PRO10284 Polypeptides [UNQ3127]

A cDNA clone (DNA138039-2828) encoding a native human PRO10284 polypeptide was identified using a yeast screen, in a human testis cDNA library that preferentially represents the 5' ends of the primary cDNA clones.

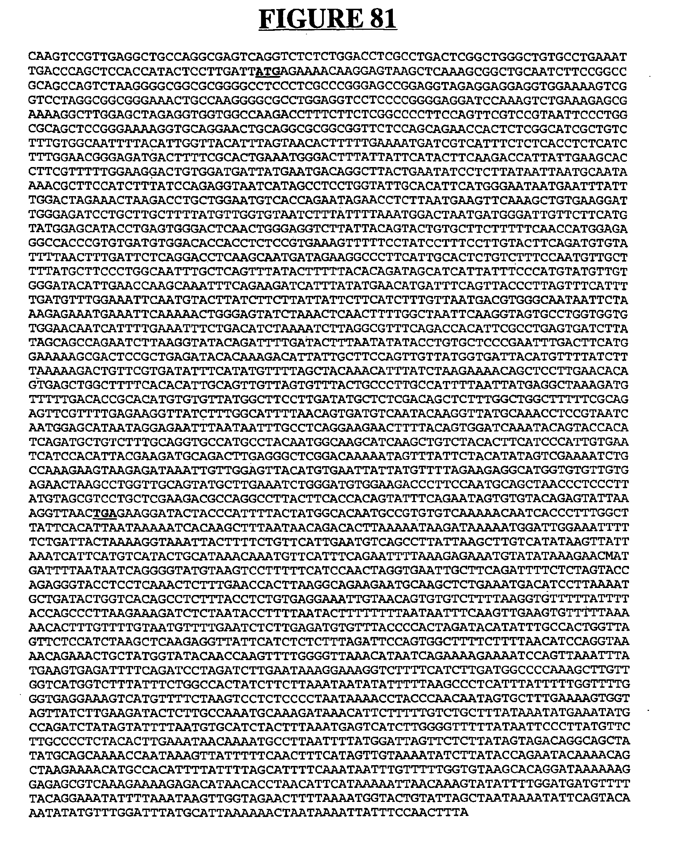

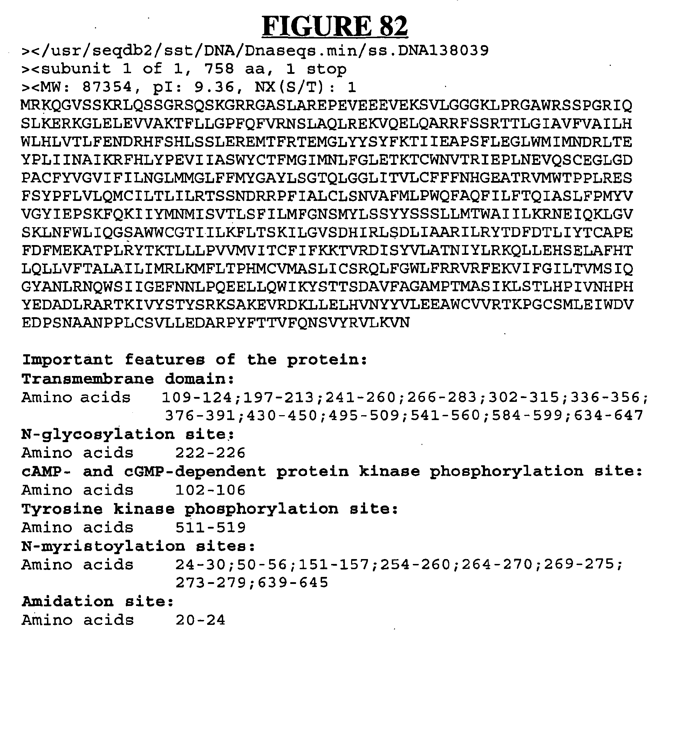

Clone DNA138039-2828 contains a single open reading frame with an apparent translational initiation site at nucleotide positions 103-105 and ending at the stop codon at nucleotide positions 2377-2379 (Figure 81; SEQ ID NO:81). The predicted polypeptide precursor is 758 amino acids long (Figure 82; SEQ ID NO:82). The full-length PRO10284 protein shown in Figure 82 has an estimated molecular weight of about 87354 daltons and a pI of about 9.36. Analysis of the full-length PRO10284 sequence shown in Figure 82 (SEQ ID NO:82) evidences the presence of a variety of important polypeptide domains as shown in Figure 82, wherein the locations given for those important polypeptide domains are approximate as described above. Clone DNA138039-2828 has been deposited with ATCC on February 8, 2000 and is assigned ATCC deposit no. PTA-1343.

An analysis of the Dayhoff database (version 35.45 SwissProt 35), using the ALIGN-2 sequence alignment analysis of the full-length sequence shown in Figure 82 (SEQ ID NO:82), evidenced sequence identity between the PRO10284 amino acid sequence and the following Dayhoff sequences: AB020684_1,DP19_CAEEL and P_W85723.

### EXAMPLE 41: Isolation of cDNA clones Encoding Human PRO20473 Polypeptides [UNQ6268]

The extracellular domain (ECD) sequences (including the secretion signal sequence, if any) from about 950 known secreted proteins from the Swiss-Prot public database were used to search EST databases. The EST databases included (1) public EST databases (e.g., Merck/Washington University), and (2) a proprietary EST database (LIFESEQ®, Incyte Pharmaceuticals, Palo Alto, CA). The search was performed using the computer program BLAST or BLAST2 [Altschul et al., Methods in Enzymology, 266:460-480 (1996)] as a comparison of the ECD protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap as described above. This consensus sequence is herein designated DNA149576. In some cases, the DNA149576 consensus sequence derives from an intermediate consensus DNA sequence which was extended using repeated cycles of BLAST and phrap to extend that intermediate consensus sequence as far as possible using the sources of EST sequences discussed above.

Based on the DNA 149576 consensus sequence, oligonucleotides were synthesized: 1) to identify by PCR a cDNA library that contained the sequence of interest, and 2) for use as probes to isolate a clone of the full-length coding sequence for PRO20473. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone, DNA from the libraries was screened by PCR amplification, as per Ausubel et al., Current Protocols in Molecular Biology, supra, with the PCR primer pair. A positive library was then used to isolate clones encoding the gene of interest using the probe oligonucleotide and one of the primer pairs.

PCR primers (forward and reverse) were synthesized:
forward PCR primer 5'-CCTATAAGGGCTACAAAAACCGCGTGG-3' (SEQ ID NO: 171)
reverse PCR primer 5'-AGCACTGCACAGACAGAGTCTCCCCTT-3' (SEQ ID NO: 172) Additionally, a synthetic oligonucleotide hybridization probe was constructed from the consensus DNA40748 sequence which had the following nucleotide sequence
hybridization probe
   5'-CAACATCACCATGGTGGCCCTCAAGCTCCAGGACTCAGG-3' (SEQ ID NO: 173)

A pool of 50 different human cDNA libraries from various tissues were used in cloning. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991)) in the unique XhoI and NotI sites.

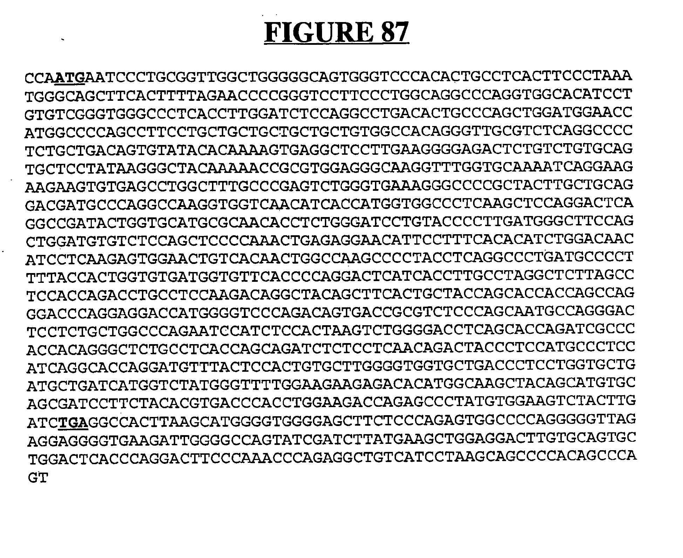

DNA sequencing of the clones isolated as described above gave the full-length DNA sequence for a full-length PRO20473 polypeptide (designated herein as DNA 163134-2917 [Figure 87, SEQ ID NO: 87]) and the derived protein sequence for that PRO20473 polypeptide.

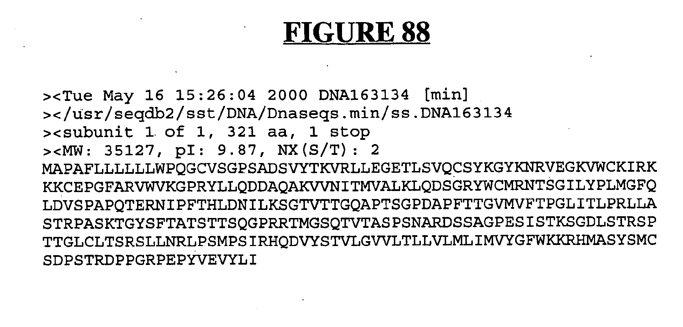

The full length clone identified above contained a single openreading frame with an apparent translational initiation site at nucleotide positions 181-183 and a stop signal at nucleotide positions 1144-1146 (Figure 87, SEQ ID NO: 87). The predicted polypeptide precursor is 321 amino acids long, has a calculated molecular weight of approximately 35,127 daltons and an estimated pI of approximately 9.87. Analysis of the full-length PRO20473 sequence shown in Figure 88 (SEQ ID NO: 88) evidences the presence of a variety of important polypeptide domains as shown in Figure 88, wherein the locations given for those important polypeptide domains are approximate as described above. Clone DNA163134-2917 has been deposited with ATCC on May 9, 1999 and is assigned ATCC Deposit No. PTA-1842.

An analysis of the Dayhoff database (version 35.45 SwissProt 35), using the ALIGN-2 sequence alignment analysis of the full-length sequence shown in Figure 88 (SEQ ID NO: 88), evidenced sequence identity between the PRO20473 amino acid sequence and the following Dayhoff sequence: HSA010099_1.

### EXAMPLE 42: Isolation of cDNA clones Encoding Human PRO21054 Polypeptides [UNQ6349]

DNA143501-2922 was identified by applying a proprietary signal sequence finding algorithm developed by Genentech, Inc. (South San Francisco, CA) upon ESTs as well as clustered and assembled EST fragments from public (e.g., Genbank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals.

Use of the above described signal sequence algorithm allowed identification of an EST cluster sequence from the LIFESEQ®, Incyte Pharmaceuticals, Palo Alto, CA database, designated herein as CLU147059. This EST cluster sequence was then compared to a variety of expressed sequence tag (EST) databases which included public EST databases (e.g., Genbank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology 266:460-480 (1996)). Those comparisons resulting in a BLAST score of 70 (or in some cases 90) or greater that did not encode known proteins were clustered and assembled into a consensus DNA sequence with the program "phrap" (Phil Green, University of Washington, Seattle, Washington). The consensus sequence obtained therefrom is herein designated DNA105254.

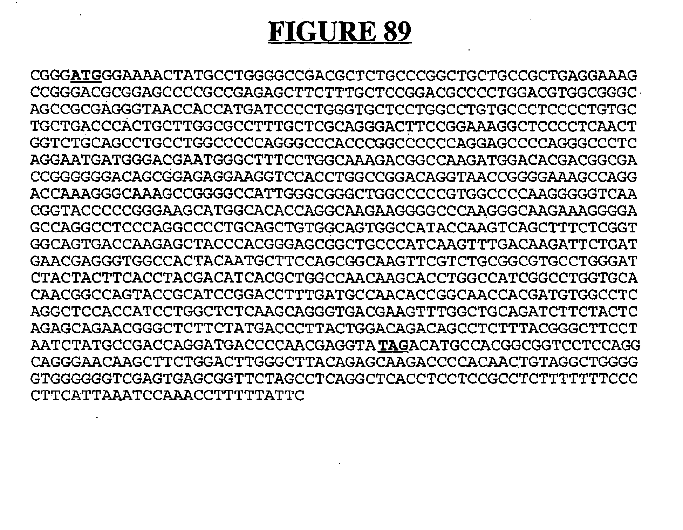

In light of an observed sequence homology between the DNA105254 sequence and an EST sequence encompassed within clone no. 2532555 from the LIFESEQ® (Incyte Pharmaceuticals, Palo Alto, CA) database, clone no. 2532555 was purchased and the cDNA insert was obtained and sequenced. It was found herein that that cDNA insert encoded a full-length protein. The sequence of this cDNA insert is shown in Figure 89 and is herein designated as DNA143501-2922.

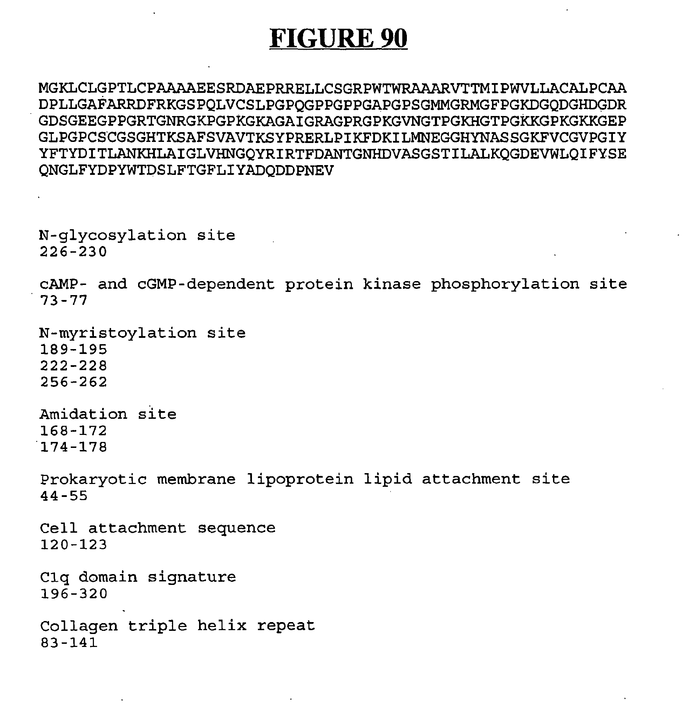

Clone DNA143501-2922 contains a single open reading frame with an apparent translational initiation site at nucleotide positions 140-142 and ending at the stop codon at nucleotide positions 995-997 (Figure 89; SEQ ID NO:89). The predicted polypeptide precursor is 330 amino acids long (Figure 90; SEQ ID NO:90). The full-length PRO21054 protein shown in Figure 90 has an estimated molecular weight of about 34833 daltons and a pI of about 9.20. Analysis of the full-length PRO21054 sequence shown in Figure 90 (SEQ ID NO: 90) evidences the presence of a variety of important polypeptide domains as shown in Figure 90, wherein the locations given for those important polypeptide domains are approximate as described above. Clone DNA 143501-2922 has been deposited with ATCC on May 23, 2000 and is assigned ATCC Deposit No. PTA-1908.

An analysis of the Dayhoff database (version 35.45 SwissProt 35), using the ALIGN-2 sequence alignment analysis of the full-length sequence shown in Figure 90 (SEQ ID NO: 90), evidenced sequence identity between the PRO21054 amino acid sequence and the following Dayhoff sequences: P_Y76039; P_Y02830; NM_000493_1; CA1A_HUMAN; CA28_HUMAN; P_Y21807; P_W09108; P_Y06481; HP25_TAMAS; P_R71701.

### EXAMPLE 43: Generation and Analysis of Mice Comprising PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Gene Disruptions

To investigate the role of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides, disruptions in PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 genes were produced by homologous recombination or retroviral insertion techniques. Specifically, transgenic mice comprising disruptions in PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 genes (i.e., knockout mice) were created by either gene targeting or gene trapping. Mutations were confirmed by southern blot analysis to confirm correct targeting on both the 5' and 3' ends. Gene-specific genotyping was also performed by genomic PCR to confirm the loss of the endogenous native transcript as demonstrated by RT-PCR using primers that anneal to exons flanking the site of insertion. Targeting vectors were electroporated into 129 strain ES cells and targeted clones were identified. Targeted clones were microinjected into host blastocysts to produce chimeras. Chimeras were bred with C57 animals to produce F1 heterozygotes. Heterozygotes were intercrossed to produce F2 wild-type, heterozygote and homozygote cohorts which were used for phenotypic analysis. Rarely, if not enough F1 heterozygotes were produced, the F1 hets were bred to wild-type C57 mice to produce sufficient heterozygotes to breed for cohorts to be analyzed for a phenotype. All phenotypic analysis was performed from 12-16 weeks after birth.

### Overall Summary of Phenotypic Results:

### 43.1. Generation and Analysis of Mice Comprising DNA28497-1130 (UNQ162) Gene Disruptions

In these knockout experiments, the gene encoding PRO188 polypeptides (designated as DNA28497-1130) (UNQ162) was disrupted. The gene specific information for these studies is as follows: the mutated mouse gene corresponds to nucleotide reference: NM_028333 Mus musculus angiopoietin-like 1 (Angptl1); protein reference: Q640P2 ACCESSION:Q640P2 NID: Mus musculus (Mouse). Angiopoietin-related protein 1 precursor (Angiopoietin-like 1); the human gene sequence reference:NM_004673 Homo sapiens angiopoietin-like 1 (ANGPTL1); the human protein sequence corresponds to reference:095841 ACCESSION:095841 NID: Homo sapiens (Human). ANGIOPOIETIN Y1 (DJ595C2.2) (ANGIOPOIETIN-RELATED PROTEIN 1 PRECURSOR).

The gene of interest is mouse Angptl1 (angiopoietin-like 1), ortholog of human ANGPTL1. Aliases include ANG3, ANGY, ARP1, ANGPT3, MGC100363, 2810039D03Rik, UNQ162, KIAA0351, and dJ595C2.2.

ANGPTL1 is a secreted protein belonging to the angiopoietin family of vascular growth factors. The protein contains a signal peptide, an N-terminal coiled-coil domain, and a fibrinogen C-terminal globular domain. Although ANGPTL 1 is structurally related to angiopoietins, ANGPTL 1 is an orphan ligand that does not bind with angiopoietin-specific receptor TIE2. ANGPTL1 is highly expressed in adrenal gland, placenta, thyroid gland, heart, skeletal muscle, and small intestine. ANGPTL1 may play a role angiogenesis as well as nonvascular processes (Kim et al, FEBS Lett 443:353-6 (1999); Oike et al, Int J Hematol 80:21-8 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 37 | 18 | 73 |
| Expected | 18.25 | 36.5 | 18.25 | 73 |

Chi-Sq.= 1.55 Significance= 0.4607038 (hom/n)= 0.22 Avg. Litter Size= 9
Mutation Information
Mutation Type: Homologous Recombination (standard)
Description: The gene consists of 5 exons, with the start codon located in exon 2 (NCBI accession NM_028333.2).
Exon 2 was targeted.
WT Panel: Expression of the target gene was detected in all 13 adult tissue samples tested by RT-PCR, except adipose.
QC Expression: Disruption of the target gene was confirmed by Southern hybridization analysis.

### 43.1.1. PHENOTYPIC ANALYSIS (for disrupted gene: DNA28497-1130 (UNQ162)

### (a) OVERALL PHENOTYPIC SUMMARY:

Mutation of the gene encoding the ortholog ofhuman angiopoietin-like 1 (ANGPTL 1) resulted in elevated cholesterol levels in the homozygous mice compared to their littermate controls. The mutant (-/-) mice also exhibited an increased platelet volume suggesting a larger than normal platelet size compared with the (+/+) wildtype littermate controls. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased mean platelet volume when compared with that of their (+/+) littermates and the historical mean, suggesting a larger than normal platelet size in the mutants.

### (c) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygous and 8 homozygous mice were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The male (-/-) mice exhibited an increased mean serum cholesterol level (approximately 2 standard deviations above littermate controls) when compared with that of their gender-matched (+/+) littermates and the historical mean.

As summarized above, the (-/-) mice exhibited increased mean serum cholesterol levels when compared with their gender-matched (+/+) littermates and the historical means. Thus, mutant mice deficient in the PRO188 gene can serve as a model for cardiovascular disease. PRO188 polypeptides or its encoding gene would be useful in regulating blood lipids such as cholesterol. Thus, PRO188 polypeptides or agonists thereof would be useful in the treatment of such cardiovascular diseases as hypertension, atherosclerosis, heart failure, stroke, various coronary diseases, hypercholesterolemia, diabetes and/or obesity.

### 43.2. Generation and Analysis of Mice Comprising DNA35558-1167 (UNQ209) Gene Disruptions

In these knockout experiments, the gene encoding PRO235 polypeptides (designated as DNA35558-1167) (UNQ209) was disrupted. The gene specific information for these studies is as follows: the mutated mouse gene corresponds to nucleotide reference: NM_008882 ACCESSION:NM_008882 NID: gi 33859838 ref NM_008882.1 Mus musculus plexin A2 (Plxna2); protein reference: P70207 ACCESSION:P70207 NID: Mus musculus (Mouse). PLEXIN 2; the human gene sequence reference: NM_025179 ACCESSION:NM_025179 NID: gi 46275829 ref NM_025179.2 Homo sapiens plexin A2 (PLXNA2); the human protein sequence corresponds to reference: Q5JRL6 ACCESSION:Q5JRL6 NID: Homo sapiens (Human). Plexin A2.

The gene of interest is mouse Plxna2 (plexin A2), ortholog of human PLXNA2. Aliases include OCT, P1xn2, mKIAA0463, 2810428A13Rik, FLJ11751, FLJ30634, and KIAA0463.

PLXNA2 is a type I integral plasma membrane protein belonging to the plexin family (Kameyama et al, Biochem Biophys Res Commun 226:396-402 (1996); Maestrini et al, Proc Natl Acad Sci U S A 93:674-8 (1996)). In general, plexins associate with neuropilins to form receptors for semaphorins (Tamagnone et al, Cell 99:71-80 (1999)), a family of chemorepellents that mediate cytoskeletal rearrangement and neuronal growth cone collapse (Castellani and Rougon, Curr Opin Neurobiol. 12:532-41 (2002); Puschel, Adv Exp Med Biol. 515:71-80 (2002)). A C-terminal domain within the cytoplasmic segment of PLXNA2 is highly conserved and is likely to function as a GTPase-activating protein (GAP) for rho-like GTPases (Castellani and Rougon, Curr Opin Neurobiol. 12:532-41 (2002); Puschel, Adv Exp Med Biol. 515:71-80 (2002)). PLXNA2 is highly expressed in brain (Maestrini et al, Proc Natl Acad Sci U S A 93:674-8 (1996)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 37 | 19 | 74 |
| Expected | 18.5 | 37 | 18.5 | 74 |

Chi-Sq.= 0.15 Significance= 0.9277435 (hom/n)= 0.26 Avg. Litter Size= 10
Mutation Information
Mutation Type: Homologous Recombination (standard)
Description: The gene consists of 32 exons, with the start codon located in exon 2 (NCBI accession NM_008882.1). Exon 2 was targeted.
1. Wild-type Expression Panel: Expression of the target gene was detected in embryonic stem (ES) cells and in all 13 adult tissue samples tested by RT-PCR, except spinal cord and bone.
2. QC Expression: Disruption of the target gene was confirmed by Southern hybridization analysis.

### 43.2.1. PHENOTYPIC ANALYSIS (for disrupted gene: DNA35558-1167 (UNQ209)

### (a) OVERALL PHENOTYPIC SUMMARY:

Mutation of the gene encoding the ortholog of human plexin A2 (PLXNA2) resulted in small (-/-) mice with decreased total tissue mass and lean body measurements and decreased bone related measurements characteristic of growth retardation. The (-/-) mice also exhibited an increased total bilirubin level and a decreased fasting serum glucose level. The (-/-) mice exhibited an increased number of RBCs with a decreased mean corpuscular volume. The mutant (-/-) exhibited several neurological abnormalities including an abnormal circadian rhythm, decreased locomotor activity during open field testing and an impaired motor coordination in the inverted screen testing. Gene disruption was confirmed by Southern blot.

### (b) Expression Patterns in Normal Human Tissues

UNQ209 has been reported to be highly expressed in the brain. However, there is notable expression in normal pancreatic tissues as well as shown by GeneLogic data. In pancreatic disease, this gene is down regulated as shown by microarray analysis. UNQ209 is also expressed on B cell subtypes.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male and female (-/-) mice exhibited decreased mean body weight (about 1 standard deviation below wildtype littermate controls) when compared with that of their gender-matched (+/+) littermates and the historical means.
Length: The male and female (-/-) mice were short exhibiting decreased mean body length when compared with that of their gender-matched (+/+) littermates and the historical means.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LVS) at 16 micrometer resolution and cortical bone parameters were analyzed in the femur midshaft at a resolution of 20 micrometers.

### Results:

DEXA: Both the male and female (-/-) mice exhibited decreased mean total tissue mass (TTM)and lean body mass (LBM) when compared with their gender matched littermate (+/+) controls and the historical means. Male knockout (-/-) mice exhibited decreased femur bone mineral density (BMD)and female (-/-) mice showed decreased total body bone mineral density (BMD) and vertebrae bone density (BMD) measurements when compared with those of their gender-matched (+/+) littermates and the historical means. micro CT: The male (-/-) mice exhibited decreased mean vertebral trabecular bone number when compared with those of their gender-matched (+/+) littermates and the historical means.

Mutant (-/-) mice deficient in the gene encoding PRO235 polypeptides show a phenotype consistent with growth retardation, marked by decreased body weight and length. Thus, antagonists or inhibitors of PRO235 polypeptides or its encoding gene would mimic these metabolic and growth related effects. On the other hand, PRO235 polypeptides or agonists thereof would be useful in the prevention and/or treatment of such metabolic disorders.

In addition, the (-/-) mice analyzed by DEXA and micro CT exhibited decreased bone related measurements and decreased body mass measurements when compared with their (+/+) littermates, suggestive of abnormal bone disorders. In addition, the decreased mean total tissue mass and lean body mass is indicative of a metabolic disorder related to growth retardation and tissue wasting disorders. The negative bone phenotype indicates that PRO235polypeptides or agonists thereof would be useful for maintaining bone homeostasis in addition to normal growth development. In addition, PRO235 polypeptides would be useful in bone healing or for the treatment of arthritis or osteoporosis, whereas antagonists (or inhibitors) of PRO235 polypeptides or its encoding gene would lead to abnormal or pathological bone disorders including inflammatory diseases associated with abnormal bone metabolism including arthritis, osteoporosis and osteopenia.

### (d) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The (-/-) mice exhibited an increased median total bilirubin level (> 2 SD above historic and littermate controls) when compared with that of their (+/+) littermates and the historical mean. In summary, the (-/-) mice showed abnormal levels of bilirubin in the blood which could be due to liver dysfunction.

The male (-/-) mice exhibited a decreased mean serum fasting glucose level (hypoglycemic) when compared with that of their gender-matched (+/+) littermates and the historical means. However, these mutants appeared unhealthy.

In these studies the mutant (-/-) mice showed a notably decreased serum glucose levels which could be due to an increased insulin sensitivity.

### (e) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased median red blood cell count and a slightly decreased mean corpuscular volume when compared with those of their (+/+) littermates and to the historical means.

These results are indicative of an abnormality in the composition of the red blood cells which could be offset by the observed decreased mean corpuscular volume in the mutant mice.

### (f) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12 ), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

The (-/-) mice exhibited a deficit in habituation to locomotor activity with an increased median sum time-in-center during open field testing when compared with their gender-matched (+/+) littermates and the historical mean, suggesting a decreased anxiety-like response in the mutants.

A notable difference was observed during open field activity testing. The male (-/-) mice exhibited an increased median sum time in the center area when compared with their gender-matched (+/+) littermates, which is indicative of a decreased anxiety-like response in the mutants. Thus, knockout mice demonstrated a phenotype consistent with depression, generalized anxiety disorders, cognitive disorders, hyperalgesia and sensory disorders and/or bipolar disorders. Thus, PRO235 polypeptides and agonists thereof would be useful for the treatment or amelioration of the symptoms associated with depressive disorders.

### Inverted Screen Testing:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Inverted Screen Test Data:

The Inverted Screen is used to measure motor strength/coordination. Untrained mice were placed individually on top of a square (7.5 cm x 7.5 cm) wire screen which was mounted horizontally on a metal rod. The rod was then rotated 180 degrees so that the mice were on the bottom of the screens. The following behavioral responses were recorded over a 1 min testing session: fell off, did not climb, and climbed up.

### Results:

| | | | |
|---|---|---|---|
| Genotype | Ratio Fell Down % | Ratio Climbed up % | |
| +/+ (n=8) | 0/8 | 8/8 | 100% |
| -/- (n=8) | 0/8 | 1/8 | 12.5% |

A motor strength deficit is apparent when there is a 50% point difference between (-/-) or (+/-) mice and (+/+) mice for the fell down response. 0/8 or 1/8 (-/-) or (+/-) mice not climbing indicates impaired motor coordination. 7/8 or 8/8(-/-) or (+/-) mice climbing up indicates enhanced motor coordination.

The Inverted Screen Test is designed to measure basic sensory & motor observations: Inverted Screen: All 8 (+/+) mice climbed up the inverted screen whereas only 1/8 (+/+) mice climbed up, indicating an impaired motor coordination in the mutants.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The (-/-) mice exhibited an augmentation or increase in activity in the early part of the dark phase when compared with that of their gender-matched (+/+) littermates and the historical mean.

Thus, the (-/-) mice exhibited increased ambulatory counts during the dark phase of home-cage activity testing resulting in a hyperactive behavior pattern or abnormal circadian rhythm when compared with their gender-matched (+/+) littermates and the historical means. These observations during home-cage activity testing is indicative of an abnormal sleep/wake cycle.

### 43.3. Generation and Analysis of Mice Comprising DNA37150-1178 (UNO233) Gene Disruptions

In these knockout experiments, the gene encoding PRO266 polypeptides (designated as DNA37150-1178) (UNQ233) was disrupted. The gene specific information for these studies is as follows: the mutated mouse gene corresponds to nucleotide reference: NM_199065 Mus musculus SLIT and NTRK-like family, member 1 (Slitrk1); protein reference: Q810C1 ACCESSION:Q810C1 NID: Mus musculus (Mouse). SLIT and NTRK-like protein 1 precursor; the human gene sequence reference: NM_052910 Homo sapiens SLIT and NTRK-like family, member 1 (SLITRK1); the human protein sequence corresponds to reference: Q5U5I6 ACCESSION:Q5U5I6 NID: Homo sapiens (Human). Slit and trk like 1 protein.

The gene of interest is mouse Slitrk1 (SLIT and NTRK-like family, member 1), ortholog of human SLITRK1. Aliases include LRRC12, KIAA0918, KIAA1910, and 3200001I04Rik.

SLITRK1 is a putative type I plasma membrane protein that likely functions as a cell adhesion molecule or signal-transducing receptor. The protein contains two leucine-rich repeat domains, a transmembrane segment, and a short cytoplasmic C-terminus. SLITRK1 is expressed primarily in neural tissue and is likely to play a role in axonogenesis (Aruga et al, Gene 315:87-94 (2003); Aruga and Mikoshiba, Mol Cell Neurosci 24:117-29 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 39 | 17 | 74 |
| Expected | 18.5 | 37 | 18.5 | 74 |

Chi-Sq.= 0.68 Significance= 0.7117703 (hom/n)= 0.23 Avg. Litter Size= 9
Mutation Information
Mutation Type: Homologous Recombination (standard)
Description: The gene consists of 1 exon (NCBI accession AK173296). Exon 1 was targeted.
1. Wild-type Expression Panel: Expression of the target gene was detected in all 13 adult tissue samples tested by RT-PCR, except liver, skeletal muscle, bone, and adipose.
2. QC Expression: Disruption of the target gene was confirmed by Southern hybridization analysis.

### 43.3.1. PHENOTYPIC ANALYSIS (for disrupted gene: DNA37150-1178 (UNO233)

### (a) OVERALL PHENOTYPIC SUMMARY:

Mutation of the gene encoding the ortholog of human SLIT and NTRK-like family, member 1 (SLITRK1) resulted in small (-/-) and (+/-) mice with decreased body mass and bone related measurements suggestive of growth and bone disorders. The mutant mice also exhibited immunological abnormalities with increased CD4 cells and decreased B cells. The (-/-) mice showed elevated mean serum IgM levels. Gene disruption was confirmed by Southern blot.

### (b) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: Male and female, knockout mice and heterozygous mice exhibited decreased mean body weight (1 SD below) when compared with that of their gender-matched (+/+) mice and the historical mean.
Length: The male (-/-) mice exhibited decreased mean body length (1-2 SD shorter) when compared with that of their gender-matched (+/+) mice and the historical mean. The heterozygous (+/-) mice exhibited intermediate shorter lengths (measurements between wildtype and homozygous mice).

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Both the male and female (-/-) mice exhibited decreased mean total tissue mass and lean body mass when compared with that of their gender-matched (+/+) littermates and the historical means. In addition, male (-/-) mice showed decreased bone mineral content; female (-/-) mice showed an increased BMC/LBM ratio due to the lowered or decreased lean body mass measurement.
micro CT: The male (-/-) mice exhibited decreased mean femoral mid-shaft cortical thickness when compared with those of their gender-matched (+/+) littermates and the historical means.

Mutant (-/-) mice deficient in the gene encoding PRO266 polypeptides show a phenotype consistent with growth retardation, marked by decreased body weight and length. Thus, antagonists or inhibitors of PRO266 polypeptides or its encoding gene would mimic these metabolic and growth related effects. On the other hand, PRO266 polypeptides or agonists thereof would be useful in the prevention and/or treatment of such metabolic disorders.

In addition, the (-/-) mice analyzed by DEXA and micro CT exhibited decreased bone measurements and decreased body mass measurements when compared with their (+/+) littermates, suggestive of abnormal bone disorders. The (-/-) mice exhibited a negative bone phenotype with abnormal decreased bone measurements reflective of bone metabolic disorders. In addition, the decreased mean total tissue mass and lean body mass is indicative of a metabolic disorder related to growth retardation and tissue wasting disorders. The negative bone phenotype indicates that PRO266 polypeptides or agonists thereof would be useful for maintaining bone homeostasis in addition to normal growth development. In addition, PRO266 polypeptides would be useful in bone healing or for the treatment of arthritis or osteoporosis, whereas antagonists (or inhibitors) of PRO266 polypeptides or its encoding gene would lead to abnormal or pathological bone disorders including inflammatory diseases associated with abnormal bone metabolism including arthritis, osteoporosis and osteopenia.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited an increased mean serum IgM level when compared with that of their (+/+) littermates and the historical mean.

Mutant (-/-) mice exhibited elevation of IgM serum immunoglobulins compared to their gender-matched (+/+) littermates. IgM immunoglobulins are the first to be produced in a humoral immune response for neutralization of bacterial toxins and are particularly important in activating the complement system. The observed phenotype suggests that the PRO266 polypeptide is a negative regulator of inflammatory responses. These immunological abnormalities suggest that inhibitors (antagonists) of PRO266 polypeptides would be useful in stimulating the immune system (such as T cell proliferation) and would find utility in the cases wherein this effect would be beneficial to the individual such as in the case of leukemia, and other types of cancer, and in immuno-compromised patients, such as AIDS sufferers. Accordingly, PRO266 polypeptides or agonists thereof would be useful in inhibiting the immune response and would be useful candidates for suppressing harmful immune responses, e.g. in the case of graft rejection or graft-versus-host diseases.

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and panNK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in peripheral blood, characterized by an increased mean percentage of CD4 cells and a decreased mean percentage of B cells when compared with those of their (+/+) littermates and the historical means.

These results show that knockout (-/-) mice exhibit immunological abnormalities compared to their wild-type (+/+) littermates. Antagonists (inhibitors) of PRO266 polypeptides would be expected to mimic this phenotype. PRO266 polypeptides or agonists thereof appear to act as a negative regulator of T cell production and a positive regulator of B cell development and would be useful in the development or maturation of B cells which could then participate in fast immune responses.

### 43.4. Generation and Analysis of Mice Comprising DNA43316-1237 (UNQ297) Gene Disruptions

In these knockout experiments, the gene encoding PRO337 polypeptides (designated as DNA43316-1237) (UNQ297) was disrupted. The gene specific information for these studies is as follows: the mutated mouse gene corresponds to nucleotide reference: NM_172290 Mus musculus neurotrimin (Hnt); protein reference: Q8BG33 ACCESSION:Q8BG33 NID: Mus musculus (Mouse). Mus musculus adult male corpora quadrigemina cDNA, RIKEN full-length enriched library, clone:B230328N06 product:NEUROTRIMIN (GP65) homolog (Mus musculus adult male corpora quadrigemina cDNA, RIKEN full-length enriched library, clone:B230377K17 product:NEUROTRIMIN (GP65) homolog); the human gene sequence reference: NM_016522 Homo sapiens neurotrimin (HNT); the human protein sequence corresponds to reference: Q9P121 ACCESSION:Q9P121 NID: Homo sapiens (Human).

The mouse gene of interest is Hnt (neurotrimin), ortholog of human HNT. Aliases include 6230410L23Rik, B230210G24Rik, NTM, and MGC60329.

HNT is a glycosylphosphatidylinositol (GPI)-anchored extracellular protein that likely functions as a cell adhesion molecule. The protein contains a signal peptide, three immunoglobulin-like domains, and a C-terminal GPI attachment site. The protein is expressed primarily in brain and is likely to participate in formation of neuronal connections in a variety of brain regions (Struyk et al, J Neurosci 15:2141-56 (1995); Liu et al, Sci China C Life Sci 47:158-64 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 37 | 14 | 62 |
| Expected | 15.5 | 31 | 15.5 | 62 |

Chi-Sq.= 2.43 Significance= 0.29671 (hom/n)= 0.2 Avg. Litter Size= 8
Mutation Information
Mutation Type: Homologous Recombination (standard)
Description: The gene consists of 7 exons, with the start codon located in exon 1 (NCBI accession BC023307). Exon 1 was targeted.
1. Wild-type Expression Panel: Expression of the target gene was detected in embryonic stem (ES) cells and in all 13 adult tissue samples tested by RT-PCR, except adipose.
2. QC Expression: Disruption of the target gene was confirmed by Southern hybridization analysis.

### 43.4.1. PHENOTYPIC ANALYSIS (for disrupted gene: DNA43316-1237 (UNQ297)

### (a) OVERALL PHENOTYPIC SUMMARY:

Mutation of the gene encoding the ortholog of human neurotrimin (HNT) resulted in the observation of increased mean total fat mass and percent of total body fat in the female (-/-) mice which is related to obesity. The female mice also exhibited increased mean serum cholesterol levels. The male knockout mice, however showed decreased lean body mass measurements. The (-/-) mice also showed immunological abnormalities related to increased percentages of CD4 cells and decreased percentages of B cells. Gene disruption was confirmed by Southern blot.

### (b) Expression in Normal Human Tissues

GeneLogic data shows UNQ297 to be highly expressed in the brain and in lung normal human tissues. In addition, UNQ297 is highly expressed on dendritic cells.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean lean body mass when compared with that of their gender-matched (+/+) littermates and the historical mean. The female (-/-) mice also exhibited increased mean total fat mass and percent total body fat.

These studies suggest that mutant female (-/-) non-human transgenic animals exhibit a negative phenotype (increased mean total fat mass and percent total body fat) that is associated with obesity. Thus, PRO337 polypeptides or agonists thereof are essential for normal growth and metabolic processes and especially would be important in the prevention and/or treatment of obesity. In addition, the male mutant (-/-) mice show signs of tissue wasting with decrease in lean body mass.

### (d) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel ofsix lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in the peripheral blood, characterized by an increased mean percentage of CD4 cells and a decreased mean percentage of B cells when compared with those of their (+/+) littermates and the historical means.

These results show that knockout (-/-) mice exhibit immunological abnormalities compared to their wild-type (+/+) littermates. Antagonists (inhibitors) of PRO337 polypeptides would be expected to mimic this phenotype. PRO337 polypeptides or agonists thereof appear to act as a negative regulator of T cell production and a positive regulator of B cell development and would be useful in the development or maturation of B cells which could then participate in fast immune responses.

### (e) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygous and 8 homozygous mice were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The female (-/-) mice exhibited an increased mean serum cholesterol level when compared with that of their gender-matched (+/+) littermates and the historical mean.

As summarized above, the (-/-) mice exhibited increased mean serum cholesterol levels when compared with their gender-matched (+/+) littermates and the historical means. Thus, mutant mice deficient in the PRO337 gene can serve as a model for cardiovascular disease. PRO337 polypeptides or its encoding gene would be useful in regulating blood lipids such as cholesterol. Thus, PRO337 polypeptides or agonists thereof would be useful in the treatment of such cardiovascular diseases as hypertension, atherosclerosis, heart failure, stroke, various coronary diseases, hypercholesterolemia, diabetes and/or obesity.

### 43.5. Generation and Analysis of Mice Comprising DNA45410-1250 (UNQ316) Gene Disruptions

In these knockout experiments, the gene encoding PRO361 polypeptides (designated as DNA45410-1250) (UNQ316) was disrupted. The gene specific information for these studies is as follows: the mutated mouse gene corresponds to nucleotide reference: NM_026345 ACCESSION:NM_026345 NID: gi 31981005 ref NM_026345.2 Mus musculus MANSC domain containing 1 (Manscl); protein reference: Q9CR33 ACCESSION:Q9CR33 NID: Mus musculus (Mouse). 9130403P13Rik protein; the human gene sequence reference: NM_018050 Homo sapiens MANSC domain containing 1 (MANSC1); the human protein sequence corresponds to reference: Q9H8J5 ACCESSION:Q9H8J5 NID: Homo sapiens (Human).

The gene of interest is mouse Mansc1 (MANSC domain containing 1), ortholog of human MANSC1. Aliases include 9130403P13Rik, FLJ10298, LOH12CR3, and 9130403P13Rik.

MANSC1 is a putative type I integral plasma membrane protein (Clark et al, Genome Res 13:2265-70 (2003)), containing a signal peptide, a MANEC (motif at N terminus with eight cysteines) domain (Pfam accession PF07502), a transmembrane segment, and a short cytoplasmic domain. Proteins with MANEC domains are typically found in extracellular proteins expressed in higher multicellular animals (Guo et al, Trends Biochem Sci 29:172-4 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 15 | 43 | 18 | 76 |
| Expected | 19 | 38 | 19 | 76 |

Chi-Sq.= 0.15 Significance= 0.9277435 (hom/n)= 0.26 Avg. Litter Size= 9
Mutation Information
Mutation Type: Homologous Recombination (standard)
Description: The gene consists of 4 exons, with the start codon located in exon 2 (NCBI accession NM 026345.2).
Exon 2 was targeted.
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 43.5.1. PHENOTYPIC ANALYSIS (for disrupted gene: DNA45410-1250 (UN0316)

### (a) OVERALL PHENOTYPIC SUMMARY:

Mutation of the gene encoding the ortholog of human MANSC domain containing 1 (MANSC 1) resulted in a decreased depressive-like response in female (-/-) mice. Gene disruption was confirmed by Southern blot.

### (b) Expression in Normal White Blood Cells

UNQ316 is highly expressed on many B cell subtypes as well as on neutrophils.

### (c) Phenotypic Analysis: CNS/Neurology

In the area of neurology, analysis focused herein on identifying in vivo validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Functional Observational Battery (FOB) Test - Tail Suspension Testing:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Tail Suspension Testing:

The tail suspension test is a procedure that has been developed as a model for depressive-like behavior in rodents. In this particular setup, a mouse is suspended by its tail for 6 minutes, and in response the mouse will struggle to escape from this position. After a certain period of time the struggling of the mouse decreases and this is interpreted as a type of learned helplessness paradigm. Animals with invalid data (i.e. climbed their tail during the testing period) are excluded from analysis.

### Results:

Tail Suspension2: The female (-/-) mice exhibited decreased median immobility time when compared with that of their gender-matched (+/+) littermates and the historical mean, suggesting a decreased depressive-like response in the mutants.

In summary, the tail suspension testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO361 polypeptides or agonists thereof would be useful in the treatment of such neurological disorders.

### 43.6. Generation and Analysis of Mice Comprising DNA47465-1561 (UNQ340) Gene Disruptions

In these knockout experiments, the gene encoding PRO539 polypeptides (designated as DNA47465-1561) (UNQ340) was disrupted. The gene specific information for these studies is as follows: the mutated mouse gene corresponds to nucleotide reference: XM_133575 ACCESSION:XM_133575 NID: gi 63558820 ref XM_133575.5 PREDICTED: Mus musculus kinesin family member 7 (Kif7); protein reference: XP_133575 kinesin family member 7 [Mus musculus]; the human gene sequence reference: NM_198525 Homo sapiens similar to kinesin family member 21A; N-5 kinesin (LOC374654); the human protein sequence corresponds to reference: Q6UXE9 ACCESSION:Q6UXE9 NID: Homo sapiens (Human).

The gene of interest is mouse Kif7 (kinesin family member 7), ortholog of human KIF7. Aliases include UNQ340.

KIF7 is a plus-end directed N-type kinesin motor protein, containing a kinesin motor catalytic domain and a myosin tail domain (Nakagawa et al, Proc Natl Acad Sci U S A 94:9654-9 (1997); Katoh and Katoh, Int J Oncol 25:1881-6 (2004a)). Like other N-type kinesins, KIF7 likely associates with microtubules at its N-terminal kinesin motor catalytic domain and with cargo at its C-terminal domain. Upon hydrolysis of A TP, KIF7 then moves along the microtubule toward the cell periphery, transporting its cargo (Miki et al, Proc Natl Acad Sci U S A 98:7004-11 (2001)). KIF7 is a paralog of KIF27, which is the ortholog of Drosophila Costal-2. Costal-2 is a hedgehog signaling regulator that interacts with hedgehog receptor smoothened, protein kinase fused, transcriptional repressor cubitus interuptus, and microtubules (Katoh and Katoh, Int J Oncol 25:1875-80 (2004b); Zhang et al, Dev Cell 8:140-1(2005); Ruel et al, Nat Cell Biol 5:907-13 (2003)). KIF7 may be involved in processes such as signal transduction, macromolecule transport, organelle transport, morphogenesis, or cell division. KIF7 expression has been detected in embryonic stem cells, melanotic melanoma cells, and Jurkat T cells (Katoh and Katoh, Int J Oncol 25:1881-6 (2004a); Miki et al, Proc Natl Acad Sci U S A 98:7004-11 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 25 | 2 | 38 |
| Expected | 9.5 | 19 | 9.5 | 38 |

Chi-Sq.= 5.62 Significance= 0.060204998 (hom/n)= 0.17 Avg. Litter Size= 7
Mutation Information
Mutation Type: Homologous Recombination (standard)
Description: The gene consists of 20 exons, with the start codon located in exon 2 (NCBI accession XM_133575.5). Exon 2 was targeted.
1. Wild-type Expression Panel: Expression of the target gene was detected in embryonic stem (ES) cells and in all 13 adult tissue samples tested by RT-PCR, except eye; skeletal muscle; bone; stomach, small intestine, and colon; and heart.
2. QC Expression: Disruption of the target gene was confirmed by Southern hybridization analysis.

### 43.6.1. PHENOTYPIC ANALYSIS (for disrupted gene: DNA47465-1561 (UNQ340)

### (a) OVERALL PHENOTYPIC SUMMARY:

Mutation of the gene encoding the ortholog of human kinesin family member 7 (KIF7) resulted in perinatal lethality of (-/-) mutants. Microscopic analysis revealed cranioschisis and exencephaly in the mutant (-/-) embryos. The female (+/-) mice exhibited an increased skin fibroblast proliferation rate and increased body fat however, no comparable elevated levels of lipids were noted in the female (+/-) mice. The male (+/-) mice exhibited abnormal bone related measurements related to such bone disorders as osteopetrosis. Gene disruption was confirmed by Southern blot.

### (b) Pathology

Microscopic: At 12.5 days, there were 52 embryos observed: 11(-/-)embryos, 16 (+/-) embryos, 13 (+/+) embryos, and 12 resorption moles. The 12.5-day (-/-) embryos exhibited cranioschisis and exencephaly, resulting in perinatal lethality of the mutants. Exencephaly is a defect caused by failed closure of the cranial neural tube during neurulation. In the 4 (-/-) embryos, there was a failure of cranial neural tube closure, leading to the cranial neuroepithelium being everted and exposed (not covered by skin). The spinal cord of the mutants was essentially normal. Neural tube defects are relatively common congenital anomalies and have multifactorial etiologies.

### (c) Expression in Human Normal and Diseased Tissues

UNQ340 is highly expressed in inflamed tissues especially in osteoarthritis patients. UNQ340 is also expressed on Th1/Th2 resting cells.

### (d) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type and heterozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and heterozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The female (+/-) mice exhibited increased mean percent total body fat and decreased mean lean body mass and bone mineral content and density measurements (total body and vertebrae BMD) when compared with those of their gender-matched (+/+) littermates and the historical means. The male (+/-) mice exhibited increased mean bone mineral content and BMC/LBM ratio when compared with those of their gender-matched (+/+) littermates. micro CT: The male (+/-) mice exhibited increased mean femoral mid-shaft cross-sectional area when compared with that of their gender-matched (+/+) littermates and the historical mean, confirming the increased DEXA bone measurements in the male (+/-) mice. Thus, male heterozygous mice showed a different bone phenotype compared to female (+/-) mice which is associated with such bone abnormalities as osteopetrosis. Osteopetrosis is a condition characterized by abnormal thickening and hardening of bone and abnormal fragility of the bones. As such, PRO539 polypeptides or agonists thereof would be beneficial for the treatment of osteopetrosis or other osteo-related diseases. On the other hand, inhibitors or antagonists of PRO539 polypeptides would be useful in bone healing.

The heterozygous female (+/-) mice showed an increased mean percent total body fat which is indicative of abnormal lipid metabolism, however no significant increase in related lipids such as cholesterol and triglycerides was noted.

### (e) Adult skin cell proliferation:

Procedure: Skin cells were isolated from 16 week old animals (2 wild type and 4 heterozygous mice). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (+/-) mice exhibited an increased mean skin fibroblast proliferation rate when compared with that of their gender-matched (+/+) littermates and the historical mean.

Thus, heterozygous mutant mice demonstrated a hyper-proliferative phenotype. As suggested by these observations, PRO539 polypeptides or agonists thereof could function as tumor suppressors and would be useful in decreasing abnormal cell proliferation.

### 43.7. Generation and Analysis of Mice Comprising DNA48320-1433 (UNQ362) Gene Disruptions

In these knockout experiments, the gene encoding PRO698 polypeptides (designated as DNA48320-1433) (UNQ362) was disrupted. The gene specific information for these studies is as follows: the mutated mouse gene corresponds to nucleotide reference: XM_354831 ACCESSION:XM_354831 NID: gi 63664155 ref XM_354831.3 PREDICTED: Mus musculus olfactomedin 4 (Olfm4); protein reference: XP_354831 ACCESSION:XP_354831 NID: gi 63664156 ref XP_354831.3 PREDICTED: olfactomedin 4 [Mus musculus]; the human gene sequence reference:NM_006418 ACCESSION:NM_006418 NID: gi 32313592 ref NM_006418.3 Homo sapiens olfactomedin 4 (OLFM4); the human protein sequence corresponds to reference:Q6UX06 ACCESSION:Q6UX06 NID: Homo sapiens (Human).

The gene of interest is mouse Olfm4 (olfactomedin 4), ortholog of human OLFM4. Aliases include GC1, OlfD, pPD4, GW112, Gm296, Gm913, KIAA4294, and bA209J19.1.

OLFM4 is a secreted glycoprotein, containing a signal peptide and a C-terminal olfactomedin-like domain. OLFM4 is expressed primarily in differentiating myeloid lineage cells (Zhang et al, Gene 283:83-93 (2002)) and in mature neutrophils from bone marrow but not in neutrophils from peripheral blood or peritoneum (Rosenbauer et al, Blood 103:4294-301 (2004)). In this context, OLFM4 may function as a component of extracellular matrix involved in trafficking of granulocytes from bone marrow into the periphery (Rosenbauer et al, Blood 103:4294-301 (2004)). OLFM4 is also expressed in small intestine, colon, and prostate, where its function is not clear (Zhang et al, Gene 283:83-93 (2002); Rosenbauer et al, Blood 103:4294-301 (2004)). OLFM4 is upregulated in crypt epithelium of inflamed colonic mucosa, suggesting that OLFM4 may be involved in inflammatory bowel disease (Shinozaki et al, Gut 48:623-9 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 10 | 16 | 11 | 37 |
| Expected | 9.25 | 18 | 9.25 | 37 |

Chi-Sq.= 4.32 Significance= 0.11532511 (hom/n)= 0.19 Avg. Litter Size= 8
Mutation Information
Mutation Type: Homologous Recombination (standard)
Description: The gene consists of 7 exons, with the start codon located in exon 1 (NCBI accession NM_001030294.1). Exon 7 was targeted.
1. Wild-type Expression Panel: Expression of the target gene was detected in embryonic stem (ES) cells and in all 13 adult tissue samples tested by RT-PCR, except skeletal muscle, bone, heart, and adipose.
2. QC Expression: Disruption of the target gene was confirmed by Southern hybridization analysis.

### 43.7.1. PHENOTYPIC ANALYSIS (for disrupted gene: DNA48320-1433 (UN0362)

### (a) OVERALL PHENOTYPIC SUMMARY:

Mutation of the gene encoding the ortholog of human olfactomedin 4 (OLFM4) resulted in numerous immunological abnormalities in (-/-) mice. The (-/-) mice also exhibited decreased alkaline phosphatase levels and decreased uric acid levels compared to their littermate controls. Male knockout mice exhibited decreased total fat mass and percent total body fat. Gene disruption was confirmed by Southern blot.

### (b) Expression in Normal and Diseased Human Tissues

UNQ362 is expressed in normal human colon, prostate, pancreas and small intestine tissues. The gene is over expressed in inflammatory bowel diseased tissues including patients with Crohns disease or ulcerative colitis compared to the normal expression pattern in the colon (microarray data). UNQ362 is also expressed on neutrophils.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean total fat mass and percent total body fat when compared with those of their gender-matched (+/+) littermates and the historical means.

Mutant (-/-) mice deficient in the gene encoding PRO698 polypeptides show a phenotype consistent with growth abnormalities and tissue wasting diseases, marked by decreased fat stores. Thus, antagonists or inhibitors of PRO698 polypeptides or its encoding gene would mimic these metabolic effects. On the other hand, PRO698 polypeptides or agonists thereof would be useful in the prevention and/or treatment of such metabolic disorders as tissue wasting diseases.

### (d) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The (-/-) mice exhibited decreased mean serum alkaline phosphatase levels which were 2 standard deviations (SD) below the littermate controls as well as decreased uric acid levels which were 1 SD below the littermate controls.

### (e) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited a decreased median hemoglobin concentration and hematocrit and an increased median platelet count when compared with those of their (+/+) littermates and the historical means.

The decreased median hemoglobin concentration and decreased hematocrit results are related to a phenotype associated with anemia. Thus, PRO698 polypeptides, agonists thereof or the encoding gene for PRO698 polypeptides must be essential for normal red blood cell production and as such would be useful in the treatment of blood disorders associated with anemia or a low hematocrit.

In addition, mutant mice deficient in the DNA48320-1433 gene resulted in a phenotype related to coagulation disorders.

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel ofsix lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in peripheral blood, characterized by decreased mean percentages of CD4 and CD8 cells and an increased mean percentage of B cells when compared with those of their (+/+) littermates and the historical means.
Tissue-Specific FACS-Pooled Tissues: The (-/-) mice exhibited a slightly decreased B1-to-B2 ratio in peritoneal lavage when compared with that of their (+/+) littermates.

In summary, the FACS results indicate that the homozygous mutant mice demonstrate immunological abnormalities marked by decreased T cell populations and increased B cell populations. From these observations, PRO698 polypeptides or the gene encoding PRO698 appears to act as a positive regulator of T cell proliferation, but a negative regulator of B cell production. PRO698 polypeptides and agonists thereof would be important for a healthy immune system and would be useful in stimulating the immune system particularly for increasing T cell proliferation.

### Ovalbumin Challenge

Procedure: This assay was carried out on 7 wild type and 8 homozygous mice. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immune-dominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited increased mean serum IgG1 and IgG2a responses to ovalbumin challenge when compared with those of their (+/+) littermates and the historical mean.

In summary, the ovalbumin challenge studies indicate that knockout homozygous mice deficient in the gene encoding PRO698 polypeptides exhibit immunological abnormalities when compared with their wild-type littermates. In particular, the mutant (-/-) mice exhibited an increased ability to elicit an immunological response when challenged with the T-cell dependent OVA antigen.

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection of kappa light chains. Any value < 6 is not significant.

### Results:

Serum Imm. 2: The (-/-) mice exhibited increased mean serum IgM, IgG2a, and IgG2b levels when compared with those of their (+/+) littermates and the historical means.

Mutant (-/-) mice exhibited elevation of IgM serum immunoglobulins compared to their gender-matched (+/+) littermates. IgM immunoglobulins are the first to be produced in a humoral immune response for neutralization of bacterial toxins and are particularly important in activating the complement system. The observed phenotype suggests that the PRO698 polypeptide is a negative regulator of inflammatory responses. These immunological abnormalities suggest that inhibitors (antagonists) of PRO698 polypeptides would be useful in stimulating the immune system and would find utility in the cases wherein this effect would be beneficial to the individual such as in the case of leukemia, and other types of cancer, and in immuno-compromised patients, such as AIDS sufferers. Accordingly, PRO698 polypeptides or agonists thereof would be useful in inhibiting the immune response and would be useful candidates for suppressing harmful immune responses, e.g. in the case of graft rejection or graft-versus-host diseases.

In addition, the mutant (-/-) mice exhibited elevation of IgG2a and IgG2b serum immunoglobulins compared to their gender-matched (+/+) littermates. These immunoglobulins have neutralization effects and to a lesser extent are important for activation of the complement system. The observed phenotype suggests that the PRO698 polypeptide is a negative regulator of inflammatory responses. These immunological abnormalities suggest that inhibitors (antagonists) of PRO698 polypeptides would be important agents which could stimulate the immune system and would find utility in the cases wherein this effect would be beneficial to the individual such as in the case of leukemia, and other types of cancer, and in immunocompromised patients, such as AIDS sufferers. Accordingly, PRO698 polypeptides or agonists thereof would be useful in inhibiting the immune response and would be useful candidates for suppressing harmful immune responses, e.g. in the case of graft rejection or graft-versus-host diseases.

### 43.8. Generation and Analysis of Mice Comprising DNA50988-1326 (UNQ385) Gene Disruptions

In these knockout experiments, the gene encoding PRO717 polypeptides (designated as DNA50988-1326) (UNQ385) was disrupted. The gene specific information for these studies is as follows: the mutated mouse gene corresponds to nucleotide reference: NM_172883 ACCESSION:NM_172883 NID: gi 27370345 ref NM_172883.1 Mus musculus RIKEN cDNA 4732482E20 gene (4732482E20Rik); protein reference: Q8CE47 ACCESSION:Q8CE47 NID: Mus musculus (Mouse). Mus musculus 10 days neonate skin cDNA, RIKEN full-length enriched library, clone:4732482E20 product:hypothetical protein, full insert sequence; the human gene sequence reference: NM_032219 ACCESSION:NM_032219NID: gi 31542730 refNM_032219.2 Homo sapiens hypothetical protein FLJ22269 (FLJ22269); the human protein sequence corresponds to reference: Q8N6H1 ACCESSION:Q8N6H1 NID: Homo sapiens (Human). Hypothetical protein FLJ22269.

The gene of interest is mouse RIKEN cDNA 4732482E20 gene, ortholog of human hypothetical protein FLJ22269. Aliases include LP2561.

Hypothetical protein FLJ22269 is a putative integral plasma membrane protein (Clark et al, Genome Res 13:2265-70 (2003)) that likely functions as a transporter. The protein contains a signal peptide, nine transmembrane segments, and an overlapping major facilitator superfamily (MFS) domain (Pfam accession PF07690). Proteins with MFS domains usually can transport only small solutes in response to chemiosmotic ion gradients (Pao et al, Microbiol Mol Biol Rev 62:1-34 (1998); Walmsley et al, Trends Biochem Sci 23:476-81 (1998)).

| | wt | het | hom | Total |
|---|---|---|---|---|
| Observed | 14 | 37 | 22 | 73 |
| Expected | 18.25 | 36.5 | 18.25 | 73 |

Chi-Sq.= 0.89 Significance= 0.64082426 (hom/n)= 0.24 Avg. Litter Size= 8
Mutation Information
Mutation Type: Homologous Recombination (standard)
Description: The gene consists of 10 exons, with the start codon located in exon 1 (NCBI accession NM_172883.1). Exons 2 through 9 were targeted.
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 43.8.1. PHENOTYPIC ANALYSIS (for disrupted gene: DNA50988-1326 (UNQ385)

### (a) OVERALL PHENOTYPIC SUMMARY:

Mutation of the gene encoding the ortholog of a putative human plasma membrane protein (FLJ22269) resulted in decreased body measurements including fat mass, body weight and length, and total tissue mass and lean body mass in female (-/-) mice. Male (-/-) mice exhibited increased bone related measurements as measured by microCT. Female (-/-) mice exhibited a decreased skin fibroblast proliferation rate. Gene disruption was confirmed by Southern blot.

### (b) Expression in Human Normal and Diseased Tissues

UNQ385 is upregulated (over expressed) in human breast cancers (infiltrating ductal carcinoma in Her-2 negative patients). Expression of this gene is also found on T7 monocytes.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight when compared with that of their gender-matched (+/+) littermates and the historical mean.
Length: The female (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Female (-/-) mice exhibited decreased mean total tissue mass and lean body mass when compared with those of their gender-matched (+/+) littermates and the historical means (consistent with decreased body weight and length). The female (-/-) mice also exhibited decreased mean total fat mass and percent total body fat. micro CT: The male (-/-) mice exhibited increased mean vertebral trabecular bone volume, number, and connectivity density when compared with those of their gender-matched (+/+) littermates and the historical means.

Mutant female (-/-) mice deficient in the gene encoding PRO717 polypeptides show a phenotype consistent with growth retardation, marked by decreased body weight and length. Thus, antagonists or inhibitors of PRO717 polypeptides or its encoding gene would mimic these metabolic and growth related effects. On the other hand, PRO717 polypeptides or agonists thereof would be useful in the prevention and/or treatment of such metabolic disorders as diabetes or other tissue wasting diseases.

In addition, the male (-/-) mice analyzed by micro CT exhibited increased bone measurements when compared with their gender-matched (+/+) littermates, suggestive of abnormal bone disorders. The (-/-) mice exhibited a negative bone phenotype with abnormal increased bone measurements reflective of bone metabolic disorders. These results indicate that the knockout mutant phenotype is associated with such bone abnormalities as osteopetrosis. Osteopetrosis is a condition characterized by abnormal thickening and hardening of bone and abnormal fragility of the bones. As such, PRO717 polypeptides or agonists thereof would be beneficial for the treatment of osteopetrosis or other osteo-related diseases. On the other hand, inhibitors or antagonists of PRO717 polypeptides would be useful in bone healing.

The decreased mean total tissue mass, lean body mass and decreased total body fat observed in female (-/-) mice is indicative of a metabolic disorder related to growth retardation and tissue wasting disorders.

### (d) Adult skin cell proliferation:

Procedure: Skin cells were isolated from 16 week old animals (2 wild type and 4 homozygous mice). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure ofrelative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited a decreased mean skin fibroblast proliferation rate when compared with that of their gender-matched (+/+) littermates and the historical mean.

Thus, homozygous mutant mice demonstrated a hypo-proliferative phenotype. As suggested by these observations, antagonists or inhibitors of PRO717 polypeptides would mimic this hypo-proliferative phenotype and could function as tumor suppressors and would be useful in decreasing abnormal cell proliferation.

### 43.9. Generation and Analysis of Mice Comprising DNA44196-1353 (UNQ422) Gene Disruptions

In these knockout experiments, the gene encoding PRO846 polypeptides (designated as DNA44196-1353) (UNQ422) was disrupted. The gene specific information for these studies is as follows: the mutated mouse gene corresponds to nucleotide reference: NM_027987 Mus musculus CD300 antigen like family member G (Cd300lg); protein reference: Q9D7B8 ACCESSION:Q9D7B8 NID: Mus musculus (Mouse). 2310016B05RIK PROTEIN; the human gene sequence reference: NM_145273 Homo sapiens CD300 antigen like family member G (CD300LG); the human protein sequence corresponds to reference: Q6UXG3 ACCESSION:Q6UXG3 NID: Homo sapiens (Human). RLLV422.

The gene of interest is mouse Cd300lg (CD300 antigen like family member G), ortholog of human CD300LG. Aliases include Clm9, D11Ertd736e, 2310016B05Rik, and TREM4.

CD300LG is a putative type I integral plasma membrane protein (Clark et al, Genome Res 13:2265-70 (2003)) likely expressed on myeloid cells. The protein contains a signal peptide, an immunoglobulin (IG) domain (InterPro accession IPRO03599), a transmembrane segment, and a cytoplasmic tail. In mouse, the CD300LG gene is found among a cluster of genes on chromosome 11 encoding proteins of similar domain organization. This gene cluster includes CLM-1 (CMRF-35-like molecule-1), a receptor expressed on myeloid lineage cells that mediates inhibition ofosteoclastogenesis (Chung et al, J Immunol 171:6541-8 (2003)). Unlike CLM-1, CD300LG does not contain immunoreceptor tyrosine-based inhibitory motifs (ITIMs) in its cytoplasmic domain.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 17 | 40 | 27 | 84 |
| Expected | 21 | 42 | 21 | 84 |

Chi-Sq.= 4.49 Significance= 0.10592755 (hom/n)= 0.27 Avg. Litter Size= 9
Mutation Information
Mutation Type: Homologous Recombination (standard)
Description: The gene consists of 7 exons, with the start codon located in exon 1 (NCBI accession NM-027987.1).
Exons 1 and 2 were targeted.
1. Wild-type Expression Panel: Expression of the target gene was detected in embryonic stem (ES) cells and in all 13 adult tissue samples tested by RT-PCR, except adipose.
2. QC Expression: Disruption of the target gene was confirmed by Southern hybridization analysis.

### 43.9.1. PHENOTYPIC ANALYSIS (for disrupted gene: DNA44196-1353 (UNQ422)

### (a) OVERALL PHENOTYPIC SUMMARY:

Mutation of the gene encoding the ortholog ofhuman CD300 antigen like family member G (CD300LG) resulted in decreased bone-related measurements in the mutant (-/-) mice. Gene disruption was confirmed by Southern blot.

### (b) Expression in Mouse Normal Tissues and Human Normal Tissues

GeneLogic data indicates that UNQ422 is highly expressed in both mouse and human normal tissues in the heart, adipose tissues, bone, lung and muscle.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type, heterozygous and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased bone mineral content, total body volumetric bone mineral density (vBMD) and vertebrae bone mineral density measurements when compared with those of their gender-matched (+/+) littermates and the historical means.
micro CT: The (-/-) mice exhibited decreased trabecular bone volume and thickness when compared with that of their gender-matched (+/+) littermates and the historical mean.

The (-/-) mice analyzed by DEXA and micro CT exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO846 polypeptides or agonists thereof would be useful for maintaining bone homeostasis and would be useful in bone healing or for the treatment of arthritis or osteoporosis, whereas antagonists (or inhibitors) of PRO846 polypeptides or its encoding gene would lead to abnormal or pathological bone disorders including inflammatory diseases associated with abnormal bone metabolism including arthritis, osteoporosis and osteopenia.

### (d) CardiologylDiagnostics - Blood Pressure

### Description:

Systolic blood pressure is measured via a noninvasive tail-cuff method for four days on the Visitech BP-2000 Blood Pressure Analysis System. The blood pressure is measured ten times each day for four days. The four days are then averaged to obtain a mouse's conscious systolic blood pressure.

### Results:

Blood Pressure: The female (-/-) mice exhibited a decreased systolic blood pressure (1 SD below) when compared with that of their gender-matched (+/+) littermates and the historical mean.

### 43.10. Generation and Analysis of Mice Comprising DNA40621-1440 (UNQ441) Gene Disruptions

In these knockout experiments, the gene encoding PRO874 polypeptides (designated as DNA40621-1440) (UNQ441) was disrupted. The gene specific information for these studies is as follows: the mutated mouse gene corresponds to nucleotide reference: NM_023557 ACCESSION:NM_023557 NID: gi 31980643 ref NM_023557.2 Mus musculus RIKEN cDNA 2210409B01 gene (2210409B01Rik): protein reference: Q91VA1 Q91VA1 Q91VA1 RIKEN CDNA 2210409B01 GENE NG22; the human gene sequence reference: NM_032794 ACCESSION:NM_032794 NID:14249467 Homo sapiens Homo sapiens chromosome 6 open reading frame 29 (C6orf29); the human protein sequence corresponds to reference: Q96K59 Q96K59 Q96K59 CDNA FLJ 14491 FIS, CLONE MAMMA1002890.

The gene of interest is mouse RIKEN cDNA 2210409B01 gene, ortholog of human C6orf29 (chromosome 6 open reading frame 29). Aliases include choline transporter-like protein 4, CTL4, NG22, and FLJ 14491.

C6orf29 is an integral plasma membrane protein that likely function as choline or choline-like transporter (O'Regan et al, Proc Natl Acad Sci U S A 97:1835-40 (2000); Traiffort et al, J Neurochem 92:1116-25 (2005); O'Regan and Meunier, Neurochem Res 28:551-5 (2003); Yuan et al, Gene 341:305-12 (2004); Inazu et al, J Neurochem 94(5):1427-37 (2005)). The protein contains at least 10 transmembrane segments mostly contained within a protein of unknown function (DUF580) domain (Pfam accession PF04515). C6orf29 is expressed primarily in peripheral tissues (Traiffort et al, J Neurochem 92:1116-25 (2005)). C6orf29 has been associated with lysosomal storage disorder sialidosis, where C6orf29 gene is fused with sialidase (NEU1) gene (Uhl et al, FEBS Lett 521:19-23 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 24 | 40 | 13 | 77 |
| Expected | 19.25 | 38.5 | 19.25 | 77 |

Chi-Sq.= 8.9 Significance= 0.011678569 (hom/n)= 0.18 Avg. Litter Size= 8
Mutation Information
Mutation Type: Homologous Recombination (standard)
Description: The gene consists of 21 exons, with the start codon located in exon 1 (NCBI accession NM_023557.2). Exons 2 through 6 were targeted.
1. Wild-type Expression Panel: Expression of the target gene was detected in brain; eye; lung; kidney; and stomach, small intestine, and colon among the 13 adult tissue samples tested by RT-PCR.
2. QC Expression: Disruption of the target gene was confirmed by Southern hybridization analysis.

### 43.10.1. PHENOTYPIC ANALYSIS (for disrupted gene: DNA40621-1440 (UN0441)

### (a) OVERALL PHENOTYPIC SUMMARY:

Mutation of the gene encoding the ortholog of human chromosome 6 open reading frame 29 (C6orf29) resulted in small male (-/-) mice, with decreased body mass, decreased body fat and decreased bone related measurements. The male (-/-) mice exhibited an increased anxiety-like response in open field testing. Multiple blood chemistry abnormalities were shown by the homozygous (-/-) mice including decreased glucose and calcium levels and increased mean serum cholesterol and alkaline phosphatase levels. Gene disruption was confirmed by Southern blot.

### (b) Expression in Human Diseased Tissues

UNQ441 is upregulated (overexpression) in pancreatic tumors, prostate cancerous tissues and breast tumors (infiltrating ductal carcinoma). UNQ441 is down regulated in colon adenocarcinomas.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight/Length: The male (-/-) mice exhibited decreased mean body weight (1 SD below) when compared with that of their gender-matched (+/+) littermates and the historical mean. Both the male and female (-/-) mice exhibited decreased body lengths (1-2 SD below) when compared with their gender matched littermate controls.

### (2) Bone Metabolism: Radiology Phenotypic Analysis

In the area ofbone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass, percent total body fat, total fat mass, and bone mineral content and density measurements when compared with those of their gender-matched (+/+) littermates and the historical means.
micro CT: The male (-/-) exhibited decreased mean vertebral trabecular bone volume and connectivity density and decreased mean femoral mid-shaft cross-sectional area when compared with those of their gender-matched (+/+) littermates and the historical means.

Mutant (-/-) mice deficient in the gene encoding PRO874 polypeptides show a phenotype consistent with growth retardation, marked by small mutant mice with decreased body weight and length. Thus, antagonists or inhibitors of PRO874 polypeptides or its encoding gene would mimic these metabolic and growth related effects. On the other hand, PRO874 polypeptides or agonists thereof would be useful in the prevention and/or treatment of such metabolic disorders.

In addition, the (-/-) mice analyzed by DEXA and micro CT exhibited decreased bone measurements and decreased body mass measurements when compared with their (+/+) littermates, suggestive of abnormal bone disorders. The (-/-) mice exhibited a negative bone phenotype with abnormal decreased bone measurements reflective ofbone metabolic disorders. In addition, the decreased mean total tissue mass and decreased total body fat is indicative of a metabolic disorder related to growth retardation and tissue wasting disorders. The negative bone and metabolic phenotype indicates that PRO874 polypeptides or agonists thereof would be useful for maintaining bone homeostasis in addition to normal growth development. In addition, PRO874 polypeptides would be useful in bone healing or for the treatment of arthritis or osteoporosis, whereas antagonists (or inhibitors) of PRO874 polypeptides or its encoding gene would lead to abnormal or pathological bone disorders including inflammatory diseases associated with abnormal bone metabolism including arthritis, osteoporosis and osteopenia.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Open field test:

Several targets of known drugs have exhibited phenotypes in the open field test. These include knockouts of the seratonin transporter, the dopamine transporter (Giros et al., Nature. 1996 Feb 15;379(6566):606-12 ), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The male (-/-) mice exhibited decreased median sum time-in-center when compared with that of their gender-matched (+/+) littermates and the historical mean, suggesting an increased anxiety-like response in the mutants.

The (-/-) mice demonstrated a decrease median sum time-in-center at intervals 2, 3, and 5 when compared to the (+/+) mice, suggesting an increased anxiety-like response in the (-/-) mice. In summary, the open field testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO874 polypeptides or agonists thereof would be useful in the treatment of such neurological disorders.

### (e) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: Multiple changes were noted in the blood chemistry panel testing. Both male and female knockout (-/-) mice showed reduced mean serum glucose levels (> 1 SD) compared with the wildtype littermate controls which could be due to increased insulin sensitivity. The heterozygous (+/-) mice showed intermediate reduced levels of mean serum glucose compared to the homozygotes and wildtype litttermates. The (-/-) mutant mice also exhibited decreased mean serum calcium levels (> 1 ST below wildtype littermate controls). The female knockout (-/-) mice showed increased alkaline phosphatase levels compared with their littermate controls (> 1 SD above).

### (f) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygous and 4 homozygous mice were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: The female (-/-) mice exhibited increased median serum cholesterol levels which were >2 SD above the wildtype littermate controls..

As summarized above, the (-/-) mice exhibited increased mean serum cholesterol levels when compared with their gender-matched (+/+) littermates and the historical means. Thus, mutant mice deficient in the PRO874 gene can serve as a model for cardiovascular disease. PRO874 polypeptides or its encoding gene would be useful in regulating blood lipids such as cholesterol. Thus, PRO874 polypeptides or agonists thereof would be useful in the treatment of such cardiovascular diseases as hypertension, atherosclerosis, heart failure, stroke, various coronary diseases, hypercholestremia, diabetes and/or obesity.

### 43.11. Generation and Analysis of Mice Comprising DNA349738 (UNQ471) Gene Disruptions

In these knockout experiments, the gene encoding PRO98346 polypeptides (designated as DNA349738) (UNQ471) was disrupted. The gene specific information for these studies is as follows: the mutated mouse gene corresponds to nucleotide reference: NM_013912 ACCESSION:NM_013912 NID: gi 31542133 ref NM_013912.2 Mus musculus apelin (Apln); protein reference: Q9R0R4 ACCESSION:Q9R0R4 NID: Mus musculus (Mouse). Apelin precursor (APJ endogenous ligand); the human gene sequence reference: NM_017413 ACCESSION:NM_017413 NID: gi 47078297 refNM_017413.3 Homo sapiens apelin, AGTRL1 ligand (APLN); the human protein sequence corresponds to reference: Q9ULZ1 ACCESSION:Q9ULZ1 NID: Homo sapiens (Human). Apelin precursor (APJ endogenous ligand) [Contains: Apelin-36; Apelin- 31; Apelin-28; Apelin-13].

The gene of interest is mouse Apln (apelin), ortholog of human APLN (apelin, AGTRL1 ligand). Aliases include Apel, 6030430G11Rik, and XNPEP2.

APLN is a secreted protein that functions as a ligand for G protein-coupled receptor AGTRL1 (angiotensin II receptor-like 1). The propeptide consists of 77 amino acids and contains a signal peptide but no other conserved domain. The mature peptide consists of the 36 C-terminal amino acids; however, the propeptide can be processed into a number of smaller peptides. APLN binds with and activates AGTRL1, inhibiting adenylyl cyclase (Tatemoto et al, Biochem Biophys Res Commun 251:471-6 (1998)); Lee et al, J Neurochem 74:34-41 (2000); Hosoya et al, J Biol Chem 275:21061-7 (2000)). APLN is expressed in several tissues, including brain, hypothalamus, stomach, and fat cells (Boucher et al, Endocrinology 146:1764-71 (2005); Medhurst et al, J Neurochem 84:1162-72 (2003)).

APLN affects a variety of physiological parameters, including water and electrolyte metabolism, cardiovascular function, energy metabolism, water and food intake, and immune function (Masri et al, Cell Signal 17:415-26 (2005)). APLN released from magnocellular neurons in the supraoptic nucleus of the hypothalamus inhibits release of antidiuretic hormone arginine-vasopressin, resulting in diuresis (De Mota et al, Proc Natl Acad Sci U S A 101:10464-9 (2004)). APLN also inhibits insulin secretion (Sorhede et al, Regul Pept.131(1-3):12-7 (2005)), improves cardiac performance (Chen et al, Circulation 108:1432-9 (2003); Ashley et al, Cardiovasc Res 65:73-82 (2005)), increases arterial and venous dilation (Cheng et al, Eur J Pharmacol 470:171-5 (2003)), decreases blood pressure (Lee et al, J Neurochem 74:34-41 (2000); Tatemoto et al, Regul Pept 99:87-92 (2001); Ishida et al, J Biol Chem 279:26274-9 (2004)), partially suppresses cytokine production (Habata et al, Biochim Biophys Acta 1452:25-35 (1999)), and inhibits HIV-1 infection (Cayabyab et al, J Virol 74:11972-6 (2000)). Genetics for Male wt x female het offspring:

| | wt | het | hemi |
|---|---|---|---|
| male | 23 | n/a | 22 |
| female | 28 | 22 | n/a |

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 13 | 16 | 31 | 60 |
| Expected | 15 | 30 | 15 | 60 |

Chi-Sq.= 26.67 Significance= 1.6168997E-6 (hom/n)= 0.49 Avg. Litter Size= 7
Mutation Information
Mutation Type: Homologous Recombination (standard)
Description: The gene consists of 3 exons, with the start codon located in exon 1 (NCBI accession NM_013912.2).
Exons 1 and 2 were targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.11.1. PHENOTYPIC ANALYSIS (for disrupted gene: DNA349738 (UNQ471)

### (a) OVERALL PHENOTYPIC SUMMARY:

Mutation of the gene encoding the ortholog of human apelin, AGTRL 1 ligand (APLN) resulted in the (0/-) mice exhibiting an increased median total white blood cell count and absolute lymphocyte counts. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (0/-) mice exhibited increased median total white blood cell and absolute lymphocyte counts when compared with those of their (+/+) and (0/+) littermates and the historical means.

These results indicate that mutant (0/-) mice show immunological abnormalities compared with their wild-type littermates. In summary, the hematology results indicate that the homozygous mutant mice exhibited an increased white blood cell count, and absolute lymphocytes count compared to their littermate controls indicating elevated levels of precursors of macrophages with increased phagocytic activity or ability to engulf or kill extracellular pathogens.

### 43.12. Generation and Analysis of Mice Comprising DNA53912-1457 (UNQ539/589) Gene Disruptions

In these knockout experiments, the gene encoding PRO1082 polypeptides (designated as DNA53912-1457) (UNQ539/589) was disrupted. The gene specific information for these studies is as follows: the mutated mouse gene corresponds to nucleotide reference: NM_020008 ACCESSION:NM_020008 NID:9910159 Mus musculus Mus musculus C-type (calcium dependent, carbohydrate recognition domain) lectin, superfamily member 12 (Clecsf12); protein reference: Q9JI50 ACCESSION:Q9JI50 NID: Mus musculus (Mouse). DENDRITIC CELL-ASSOCIATED C-TYPE LECTIN-1; the human gene sequence reference: NM_197947 ACCESSION:NM_197947 NID: gi 37675372 ref NM_197947.1 Homo sapiens C-type lectin domain family 7, member A (CLEC7A), transcript variant 1; the human protein sequence corresponds to reference:Q9BXN2 ACCESSION:Q9BXN2 NID: Homo sapiens (Human). Dendritic cell-associated C-type lectin-1 (DECTIN-1 receptor) (Lectin- like receptor 1) (Beta-glucan receptor isoform A).

The gene of interest is mouse Clec7a (C-type lectin domain family 7, member a), ortholog of human CLEC7A. Aliases include BGR, beta-GR, Clecsf12, and DECTIN1.

CLEC7A is a type II integral plasma membrane protein expressed primarily in peripheral blood leukocytes. The protein contains a cytoplasmic immunoreceptor tyrosine-based activation motif (ITAM), a transmembrane segment, and an extracellular C-type lectin domain. CLEC7A functions as a pattern recognition receptor, binding with beta-glucans from fungi and bacteria and activating Syk kinase. CLEC7A works alone or in conjunction with other receptors, such as Toll-like receptor 2 and SIGN-related 1, to initiate immune responses to pathogens (Herre et al, Crit Rev Immunol 24:193-203 (2004); Willment et al, J Biol Chem 276:43818-23 (2001); Rogers et al, Immunity 22:507-17 (2005); Willment et al, Eur J Immunol 35:1539-47 (2005); Brown et al, J Exp Med 197:1119-24 (2003); Taylor et al, J Immunol 172:1157-62 (2004); Sobanov et al, Eur J Immunol 31:3493-503 (2001); Ariizumi et al, J Biol Chem 275:20157-67 (2000); Brown and Gordon, Nature 413:36-7 (2001)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 41 | 20 | 80 |
| Expected | 20 | 40 | 20 | 80 |

Chi-Sq.= 0.48 Significance= 0.7866279 (hom/n)= 0.25 Avg. Litter Size= 9
Mutation Information
Mutation Type: Homologous Recombination (standard)
Description: The gene consists of 6 exons, with the start codon located in exon 1 (NCBI accession NM_020008.1).
Exons 1 through 3 were targeted.
1. Wild-type Expression Panel: Expression of the target gene was detected in all 13 adult tissue samples tested by RT-PCR.
2. QC Expression: Disruption of the target gene was confirmed by Southern hybridization analysis.

### 43.12.1. PHENOTYPIC ANALYSIS (for disrupted gene: DNA53912-1457 (UNQ539/589)

### (a) OVERALL PHENOTYPIC SUMMARY:

Mutation of the gene encoding the ortholog of human C-type lectin domain family 7, member a (CLEC7A) resulted in an altered distribution of leukocyte subsets in the peripheral blood of (-/-) mice. Gene disruption was confirmed by Southern blot.

### (b) Expression in Normal Human Tissues

UNQ539/589 is expressed on B cell subtypes. In addition, LPS decreased the expression of UNQ539/589 on myeloid cells.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the maj or histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in the peripheral blood, characterized by an increased mean percentage of CD4 cells and a decreased mean percentage ofB cells in the population when compared with those of their (+/+) littermates and the historical means. Tissue-Specific FACS-Pooled Tissues: The (-/-) mice exhibited an increased percentage of B220Dim/CD43Dim cells in bone marrow when compared with that of their (+/+) littermates and the historical mean.

These results show that knockout (-/-) mice exhibit immunological abnormalities compared to their wild-type (+/+) littermates. Antagonists (inhibitors) of PRO1082 polypeptides would be expected to mimic this phenotype. PRO1082 polypeptides or agonists thereof appear to act as a negative regulator of T cell production and a positive regulator of B cell development and would be useful in the development or maturation of B cells which could then participate in fast immune responses.

### 43.13. Generation and Analysis of Mice Comprising DNA59841-1460 (UNQ542) Gene Disruptions

In these knockout experiments, the gene encoding PRO1097 polypeptides (designated as DNA59841-1460) (UNQ542) was disrupted. The gene specific information for these studies is as follows: the mutated mouse gene corresponds to nucleotide reference: AK053903 Mus musculus 0 day neonate eyeball cDNA, RIKEN full-length enriched library, clone:E130319M14 product:acid phosphatase 1, soluble, full insert sequence; the human gene sequence reference: NM_001002919 Homo sapiens hypothetical protein LOC285016 (LOC285016); the human protein sequence corresponds to reference: Q6UX46 ACCESSION:Q6UX46 NID: Homo sapiens (Human). RGPG542.

The mouse gene of interest is represented by RIKEN clone E130319M14, which is orthologous with human hypothetical protein LOC285016.

Hypothetical protein LOC285016 is a putative secreted protein (Clark et al, Genome Res 13:2265-70 (2003)), containing a signal peptide.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 35 | 17 | 68 |
| Expected | 17 | 34 | 17 | 68 |

Chi-Sq.= 0.33 Significance= 0.8478937 (hom/n)= 0.24 Avg. Litter Size= 8
Mutation Information
Mutation Type: Homologous Recombination (standard)
Description: The gene consists of 5 exons, with the start codon located in exon 1 (NCBI accession AK053903). Exons 1 through 3 were targeted.
1. Wild-type Expression Panel: Expression of the target gene was detected in all 13 adult tissue samples tested by RT-PCR, except skeletal muscle, bone, and adipose.
2. QC Expression: Disruption of the target gene was confirmed by Southern hybridization analysis.

### 43.13.1. PHENOTYPIC ANALYSIS (for disrupted gene: DNA59841-1460 (UNQ542)

### (a) OVERALL PHENOTYPIC SUMMARY:

Mutation of the gene encoding the ortholog of a putative human secreted protein (LOC285016) resulted in small male (-/-) mice with decreased total tissue mass and lean body mass as well as decreased bone related measurements. The (-/-) mice also exhibited an increased serum IL-6 response to LPS challenge. Male (-/-) mice showed increased alkaline phosphatase levels. In addition, the female (-/-) mice exhibited an increased skin fibroblast proliferation rate. Gene disruption was confirmed by Southern blot.

### (b) Expression in Human Tissues/Cells

UNQ542 is expressed on natural killer (NK+) cells. In addition, GeneLogic data shows high expression of UNQ542 in both ovaries and the pancreas.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The male (-/-) mice exhibited decreased mean body weight when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean total tissue mass, lean body mass and total body bone mineral content when compared with that of their gender-matched (+/+) littermates and the historical means. micro CT: The male (-/-) mice exhibited decreased mean femoral mid-shaft cortical thickness when compared with that of their gender-matched (+/+) littermates and the historical mean.

Mutant (-/-) mice deficient in the gene encoding PRO1097 polypeptides show a phenotype consistent with growth retardation, marked by small male mutant mice with decreased body weight. Thus, antagonists or inhibitors of PRO1097 polypeptides or its encoding gene would mimic these metabolic and growth related effects. On the other hand, PRO1097 polypeptides or agonists thereof would be useful in the prevention and/or treatment of such metabolic disorders.

In addition, the (-/-) mice analyzed by DEXA and micro CT exhibited decreased bone measurements and decreased body mass measurements when compared with their (+/+) littermates, suggestive of abnormal bone disorders. The (-/-) mice exhibited a negative bone phenotype with abnormal decreased bone measurements reflective of bone metabolic disorders. In addition, the decreased mean total tissue mass, lean body mass and decreased total body fat is indicative of a metabolic disorder related to growth retardation and tissue wasting disorders. The negative bone and metabolic phenotype indicates that PRO1097 polypeptides or agonists thereof would be useful for maintaining bone homeostasis in addition to normal growth development. In addition, PRO1097 polypeptides would be useful in bone healing or for the treatment of arthritis or osteoporosis, whereas antagonists (or inhibitors) of PRO1097 polypeptides or its encoding gene would lead to abnormal or pathological bone disorders including inflammatory diseases associated with abnormal bone metabolism including arthritis, osteoporosis and osteopenia.

### (d) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: The male (-/-) mice exhibited an increased mean serum alkaline phosphatase level when compared with that of their gender-matched (+/+) littermates and the historical mean.

### (e) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100ul blood sample was then taken and analyzed for the presence of TNFa, MCP-1, and IL-6 on the FACS Calibur instrument.

### Results:

Acute Phase Response: The (-/-) mice exhibited an increased mean serum IL-6 response to LPS challenge when compared with that of their (+/+) littermates and the historical mean.

In summary, the LP S endotoxin challenge demonstrated that knockout mice deficient in the gene encoding PRO1097 polypeptides exhibit immunological abnormalities when compared with their wild-type littermates. In particular, the mutant mice exhibited an increased ability to elicit an immunological response (IL-6 production) when challenged with the LPS endotoxin indicating a proinflammatory response. IL-6 contributes to the later stages of B cell activation. In addition, IL-6 plays a critical role in inducing the acute phase response and systemic inflammation. This suggests that inhibitors or antagonists to PRO1097 polypeptides would stimulate the immune system and would find utility in the cases wherein this effect would be beneficial to the individual such as in the case of leukemia, and other types of cancer, and in immuno-compromised patients, such as AIDS sufferers. Accordingly, PRO1097 polypeptides or agonists thereof would be useful in inhibiting the immune response and would be useful candidates for suppressing harmful immune responses, e.g. in the case of graft rejection or graft-versus-host diseases.

### (f) Adult skin cell proliferation:

Procedure: Skin cells were isolated from 16 week old animals (2 wild type and 4 homozygous mice). These were developed into primary fibroblast cultures and the fibroblast proliferation rates were measured in a strictly controlled protocol. The ability of this assay to detect hyper-proliferative and hypo-proliferative phenotypes has been demonstrated with p53 and Ku80. Proliferation was measured using Brdu incorporation.

Specifically, in these studies the skin fibroblast proliferation assay was used. An increase in the number of cells in a standardized culture was used as a measure of relative proliferative capacity. Primary fibroblasts were established from skin biopsies taken from wild type and mutant mice. Duplicate or triplicate cultures of 0.05 million cells were plated and allowed to grow for six days. At the end of the culture period, the number of cells present in the culture was determined using a electronic particle counter.

### Results:

Skin Proliferation: The female (-/-) mice exhibited an increased mean skin fibroblast proliferation rate when compared with that of their gender-matched (+/+) littermates and the historical mean.

Thus, homozygous mutant mice demonstrated a hyper-proliferative phenotype. As suggested by these observations, PRO1097 polypeptides or agonists thereof could function as tumor suppressors and would be useful in decreasing abnormal cell proliferation.

### 43.14. Generation and Analysis of Mice Comprising DNA62814-1521 (UNQ606) Gene Disruptions

In these knockout experiments, the gene encoding PRO1192 polypeptides (designated as DNA62814-1521) (UNQ606) was disrupted. The gene specific information for these studies is as follows: the mutated mouse gene corresponds to nucleotide reference: NM_007962 ACCESSION:NM_007962 NID: gi 31542622 ref NM_007962.2 Mus musculus epithelial V-like antigen 1 (Eva1); protein reference: Q91WI4 ACCESSION:Q91WI4 NID: Mus musculus (Mouse). EPITHELIAL V-LIKE ANTIGEN; the human gene sequence reference: NM_005797 ACCESSION:NM_005797 NID:21536270 Homo sapiens Homo sapiens epithelial V-like antigen 1 (EVA1), transcript variant 1; the human protein sequence corresponds to reference: 060487 ACCESSION:060487 NID: Homo sapiens (Human). EPITHELIAL V-LIKE ANTIGEN 1 PRECURSOR.

The gene of interest is mouse Eva1 (epithelial V-like antigen 1), ortholog of human EVA1. Aliases include Eva and Mpz12.

EVA1 is a type I integral plasma membrane protein that likely functions as a homophilic cell adhesion molecule. The 215-amino acid protein contains a signal peptide, a V-type immunoglobulin domain (SMART accession SM00406), a transmembrane segment, and a C-terminal cytoplasmic segment. EVA1 is expressed in trophoblasts, thymus, liver, gut, skin, and testis. EVA1 may play a role in processes such as trophoblast invasion (Teesalu et al, Dev Genet 23:317-23 (1998)) and thymus organogenesis (Guttinger et al, J Cell Biol 141:1061-71 (1998)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 36 | 12 | 69 |
| Expected | 17.25 | 34.5 | 17.25 | 69 |

Chi-Sq.= 1.69 Significance= 0.42955735 (hom/n)= 0.21 Avg. Litter Size= 8
Mutation Information
Mutation Type: Homologous Recombination (standard)
Description: The gene consists of 6 exons, with the start codon located in exon 1 (NCBI accession NM_007962.2).
Exons 1 through 3 were targeted. 1. Wild-type Expression Panel: Expression of the target gene was detected in all 13 adult tissue samples tested by RT-PCR, except skeletal muscle, bone, heart, and adipose.
2. QC Expression: Disruption of the target gene was confirmed by Southern hybridization analysis.

### 43.14.1. PHENOTYPIC ANALYSIS (for disrupted gene: DNA62814-1521 (UNQ606)

### (a) OVERALL PHENOTYPIC SUMMARY:

Mutation of the gene encoding the ortholog of human epithelial V-like antigen 1 (EVA1) resulted in decreased bone mineral content and bone mineral density measurements in the (-/-) mice. Gene disruption was confirmed by Southern blot.

### (b) Expression Human Diseased Tissues

UNQ606 is overexpressed in inflammatory tissues including patients with psoriasis.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both
trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean bone mineral content and density measurements when compared with those of their gender-matched (+/+) littermates and the historical means. micro CT: The male (-/-) mice exhibited decreased mean femoral mid-shaft cortical thickness and cross sectional area when compared with those of their gender-matched (+/+) littermates and the historical means.

The (-/-) mice analyzed by DEXA and microCT exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO1192 polypeptides or agonists thereof would be useful for maintaining bone homeostasis. In addition, PRO1192 polypeptides would be useful in bone healing or for the treatment of arthritis or osteoporosis, whereas antagonists (or inhibitors) of PRO1192 polypeptides or its encoding gene would lead to abnormal or pathological bone disorders including inflammatory diseases associated with abnormal bone metabolism including arthritis, osteoporosis and osteopenia.

### 43.15. Generation and Analysis of Mice Comprising DNA66519-1535 (UNQ638) Gene Disruptions

In these knockout experiments, the gene encoding PRO1268 polypeptides (designated as DNA66519-1535) (UNQ638) was disrupted. The gene specific information for these studies is as follows: the mutated mouse gene corresponds to nucleotide reference: NM_025838 ACCESSION:NM_025838 NID: gi 21539608 ref NM_025838.1 Mus musculus RIKEN cDNA 1110004B13 gene (1110004B13Rik); protein reference: Q9CPV0 ACCESSION:Q9CPV0 NID: Mus musculus (Mouse). 1110004B13Rik protein; the human gene sequence reference: NM_183065 ACCESSION:NM_183065 NID: gi 34101277 ref NM_183065.1 Homo sapiens hypothetical protein MGC10744 (MGC10744); the human protein sequence corresponds to reference: Q6UX40 ACCESSION:Q6UX40 NID: Homo sapiens (Human).

The gene of interest is mouse RIKEN cDNA 1110004B 13 gene, ortholog of human hypothetical protein MGC10744. Aliases include GRVS638, UNQ638, MGC10744, and PRO1268.

Hypothetical protein MGC 10744 is a putative integral plasma membrane protein of about 140 amino acids (Clark et al, Genome Res 13:2265-70 (2003)). The protein contains a signal anchor and three transmembrane segments.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 25 | 35 | 3 | 63 |
| Expected | 15.75 | 31.5 | 15.75 | 63 |

Chi-Sq.= 16.31 Significance= 2.8729535E-4 (hom/n)= 0.1 Avg. Litter Size= 8
Mutation Information
Mutation Type: Homologous Recombination (standard)
Description: The gene consists of 5 exons, with the start codon located in exon 1 (NCBI accession NM_025838.1).
Exons 1 through 5 were targeted.
1. Wild-type Expression Panel: Expression of the target gene was detected in embryonic stem (ES) cells and in all 13 adult tissue samples tested by RT-PCR, except spinal cord and bone.
2. QC Expression: Disruption of the target gene was confirmed by Southern hybridization analysis.

### 43.15.1. PHENOTYPIC ANALYSIS (for disrupted gene: DNA66519-1535 (UN0638)

### (a) OVERALL PHENOTYPIC SUMMARY:

Mutation of the gene encoding the ortholog of putative human plasma membrane protein (MGC10744) resulted in lethality of the (-/-) mutants. The (-/-) embryos exhibited cardiac defects. Gene disruption was confirmed by Southern blot.

### (b) Pathology

Microscopic: At day 12.5, there were 47 embryos observed: 8 (-/-) embryos, 16 (+/-) embryos, 13 (+/+) embryos, and 10 resorption moles. The 4 (-/-) embryos analyzed exhibited cardiac malformation and dilated atria. There was complete lack of septation between the left and right atria, which were combined into a single large chamber. The opening between the left and right ventricles was also much larger than in normal littermates, also suggesting delayed or incomplete septation. At this stage of embryogenesis, atrial and ventricular septation is normally well-advanced, although still incomplete. If these cardiac defects persisted until birth, they could account for the perinatal mortality.

### 43.16. Generation and Analysis of Mice Comprising DNA66304-1546 (UNQ648) Gene Disruptions

In these knockout experiments, the gene encoding PRO1278 polypeptides (designated as DNA66304-1546) (UNQ648) was disrupted. The gene specific information for these studies is as follows: the mutated mouse gene corresponds to nucleotide reference: NM_026092 Mus musculus lysozyme-like 1 (Lyzl1); protein reference: Q9CPX3 ACCESSION:Q9CPX3 NID: Mus musculus (Mouse). 1700038F02RIK PROTEIN; the human gene sequence reference: NM_032517 Homo sapiens lysozyme-like 1 (LYZL1); the human protein sequence corresponds to reference: Q8WW16 ACCESSION:Q8WW16 NID: Homo sapiens (Human). SIMILAR TO LYSOZYME C-1 (1,4-BETA-N-ACYLMURAMIDASE C, EC 3.2.1.17).

The gene of interest is mouse Lyzl1 (lysozyme-like 1), ortholog ofhuman LYZL1. Aliases include Lyc2, 1700038F02Rik, KAAG648, PRO1278, MGC33408, and bA534G20.1.

LYZL1 is a putative secreted protein (Clark et al, Genome Res 13:2265-70 (2003)) that likely functions as an enzyme. The protein contains a signal peptide and a C-type lysozyme/alpha-lactalbumin family domain. Proteins with this domain include lysozyme C and alpha-lactalbumin. Lysozyme C catalyzes the hydrolysis of the beta-1,4-linkage betweenN-acetylmuramic acid and N-acetyl-D-glucosamine in bacterial cell wall peptidoglycans (Interpro accession IPRO00974). Alpha-lactalbumin, in contrast, has no catalytic activity. Instead, it functions as a modulator of galactosyltransferase, changing its substrate specificity from N-acetylglucosamine to glucose and enabling the synthesis of lactose from galactose and glucose (Pfam accession PF00062).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 13 | 31 | 6 | 50 |
| Expected | 12.5 | 25 | 12.5 | 50 |

Chi-Sq.= 3.23 Significance= 0.19889067 (hom/n)= 0.19 Avg. Litter Size= 8
Mutation Information
Mutation Type: Homologous Recombination (standard)
Description: The gene consists of 5 exons, with the start codon located in exon 2 (NCBI accession NM_026092.1).
Exons 2 and 3 were targeted.
1. Wild-type Expression Panel: Expression of the target gene was detected only in eye among the 13 adult tissue samples tested by RT-PCR.
2. QC Expression: Disruption of the target gene was confirmed by Southern hybridization analysis.

### 43.16.1. PHENOTYPIC ANALYSIS (for disrupted gene: DNA66304-1546 (UNQ648)

### (a) OVERALL PHENOTYPIC SUMMARY:

Mutation of the gene encoding the ortholog of human lysozyme-like 1 (LYZL1) resulted in a decreased heart rate in female (-/-) mice. One knockout mouse exhibited kyphosis of the vertebrae indicating abnormal development. Gene disruption was confirmed by Southern blot.

### (b) Pathology

CATScan: Of the 3 (-/-) mice examined, 1 (M-165) exhibited kyphosis of the thoracic vertebrae.

### (c) Cardiology - Heart Rate

### Description:

Heart rate is measured via a noninvasive tail-cuff method for four days on the Visitech BP-2000 Blood Pressure Analysis System. Heart rate is measured ten times each day for four days. The four days are then averaged to obtain a mouse's conscious heart rate. Heart Rate: The female (-/-) mice exhibited a decreased mean heart rate when compared with that of their gender-matched (+/+) littermates and the historical mean.

### 43.17. Generation and Analysis of Mice Comprising DNA65409-1566 (UNQ669) Gene Disruptions

In these knockout experiments, the gene encoding PRO1303 polypeptides (designated as DNA65409-1566) (UNQ669) was disrupted. The gene specific information for these studies is as follows: the mutated mouse gene corresponds to nucleotide reference: XM_355892 ACCESSION:XM_355892 NID: gi 51746021 ref XM_355892.2 PREDICTED: Mus musculus RIKEN cDNA 2310008B01 gene (2310008B01Rik); protein reference: XP_355892 ACCESSION:XP_355892 NID: gi 51746022 ref XP_355892.2 RIKEN cDNA 2310008B01 [Mus musculus]; the human gene sequence reference: NM_145894ACCESSION:NM_145894 NID: gi 22208986 ref NM_145894.1 Homo sapiens kallikrein 12 (KLK12); the human protein sequence corresponds to reference: Q9UKR0 ACCESSION:Q9UKR0 NID: Homo sapiens (Human). Kallikrein 12 precursor (EC 3.4.21.-) (Kallikrein-like protein 5) (KLK-L5).

The gene of interest is mouse "similar to glandular kallikrein KLK13," ortholog of human KLK12 (kallikrein 12). Aliases include LOC381881, hK12, and KLK-L5.

KLK12 is a putative secreted protein that functions as a serine protease (Shinmura et al, Hum Mutat 24:273-4 (2004)). This protein belongs to the kallikrein subfamily of serine proteases, which are encoded by 14 or 15 genes clustered on human chromosome 19. The kallikrein subfamily includes kallikrein 1, which cleaves kininogen to form bradykinin, a bioactive peptide hormone involved in the regulation of local blood flow, sodium balance, and inflammation. The kallikrein subfamily also includes kallikrein 3, better known as prostate-specific antigen (PSA) (Harvey et al, J Biol Chem 275:37397-406 (2000); Yousef et al, Biochem Biophys Res Commun 276:125-33 (2000)). KLK12 is expressed primarily in salivary gland, stomach, uterus, trachea, prostate, thymus, lung, colon, brain, breast, and thyroid gland (Yousef et al, Genomics 69:331-41 (2000)). KLK12 may play a role in the pathogenesis and progression of certain types of cancer (Yousef et al, Genomics 69:331-41 (2000); Shinmura et al, Hum Mutat 24:273-4 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 23 | 37 | 19 | 79 |
| Expected | 19.75 | 39.5 | 19.75 | 79 |

Chi-Sq.= 0.64 Significance= 0.726149 (hom/n)= 0.27 Avg. Litter Size= 8
Mutation Information
Mutation Type: Homologous Recombination (standard)
Description: The gene consists of 4 exons, with the start codon located in exon 2 (NCBI accession AK009217).
Exons 2 through 4 were targeted.
1. Wild-type Expression Panel: Expression of the target gene was detected in eye; thymus; lung; and stomach, small intestine, and colon among the 13 adult tissue samples tested by RT-PCR.
2. QC Expression: Disruption of the target gene was confirmed by Southern hybridization analysis.

### 43.17.1. PHENOTYPIC ANALYSIS (for disrupted gene: DNA65409-1566 (UNQ669)

### (a) OVERALL PHENOTYPIC SUMMARY:

Mutation of the gene encoding the ortholog of human kallikrein 12 (KLK12) resulted in an abnormal circadian rhythm marked by increased activity during the end of the light phase and beginning of the dark phase during circadian rhythm testing in the (-/-) mice. Some of the heterozygous and homozygous mice showed hematuria yet no evidence for infection or renal dysfunction was observed in these mice. Gene disruption was confirmed by Southern blot.

### (b) Expression in Human Normal Tissues

GeneLogic data shows high expression of UNQ669 in normal pancreatic tissues.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The (-/-) mice exhibited increased activity during end of light/ beginning of dark period in the circadian testing when compared with that of their gender-matched (+/+) littermates and the historical mean.

The (-/-) mice exhibited increased ambulatory counts during home-cage activity testing resulting in a hyperactive behavior pattern when compared with their gender-matched (+/+) littermates and the historical means. These observations during home-cage activity testing is also suggestive of increased anxiety which is consistent with neurological disorders such as generalized anxiety disorders, attention deficit hyperactivity disorder, obsessive compulsive disorder, schizophrenia, cognitive disorders generalized anxiety disorder. Thus, antagonists or inhibitors of PRO1303 polypeptides or the PRO1303 encoding gene would be expected to mimic this behavior. Likewise, PRO1303 polypeptides or agonists thereof, would be useful in the treatment of such neurological disorders including generalized anxiety disorders, attention deficit hyperactivity disorder, obsessive compulsive disorder, schizophrenia, cognitive disorders generalized anxiety disorder.

### 43.18. Generation and Analysis of Mice Comprising DNA62306-1570 (UNQ674) Gene Disruptions

In these knockout experiments, the gene encoding PRO1308 polypeptides (designated as DNA62306-1570) (UNQ674) was disrupted. The gene specific information for these studies is as follows: the mutated mouse gene corresponds to nucleotide reference: NM _031380 ACCESSION:NM_031380 NID:13878202 Mus musculus Mus musculus follistatin-like 3 (Fstl3); protein reference: Q9EQC7 Q9EQC7 Q9EQC7 FOLLISTATIN-LIKE PROTEIN FOLLISTATIN-RELAT; the human gene sequence reference: NM_005860 ACCESSION:NM_005860 NID:5031700 Homo sapiens Homo sapiens follistatin-like 3 (secreted glycoprotein) (FSTL3); the human protein sequence corresponds to reference: 095633 095633 095633 FOLLISTATIN-RELATED PROTEIN FLRG FOLLISTAT.

The gene of interest is mouse Fstl3 (follistatin-like 3), ortholog of human FSTL3. Aliases include Flrg, E030038F23Rik, and FSRP.

FSTL3 is a secreted glycoprotein that binds with activin, bone morphogenic proteins (BMPs), and myostatin, inhibiting their morphogenic activity (Tsuchida et al, J Biol Chem 275:40788-96 (2000); Hill et al, J Biol Chem 277:40735-41 (2002)). The protein consists of a signal peptide and two tandem segments containing a follistatin-like domain (SMART accession SM00274) and a Kazal-Like serine protease inhibitor domain (SMART accession SM00280). FSTL3 is expressed primarily in placenta, ovary, uterus, and testis but is also expressed in several other tissues, including skin, heart, lung, and kidney. FSTL3 likely plays a role in a variety of processes, such as gonadal development, gametogenesis (Xia et al, Mol Endocrinol 18:979-94 (2004)), endometrial decidualization (Wang et al, J Clin Endocrinol Metab 88:4432-9 (2003)), erythroid maturation (Maguer-Satta et al, Exp Cell Res 282:110-20 (2003)), skeletal muscle mass regulation (Hill et al, J Biol Chem 277:40735-41 (2002)), and cutaneous wound repair (Wankell et al, J Endocrinol 171:385-95 (2001)). Because FSTL3 expression is also located in the cytoplasm and nucleus of some tissues, FSTL3 may also have other intracellular functions (Tortoriello et al, Endocrinology 142:3426-34 (2001); Florio et al, Mol Cell Endocrinol 218:129-35 (2004); Wang et al, J Clin Endocrinol Metab 88:4432-9 (2003)). FSTL3 may play a role in leukemogenesis (Hayette et al, Oncogene 16:2949-54 (1998)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 32 | 17 | 67 |
| Expected | 16.75 | 33.5 | 16.75 | 67 |

Chi-Sq.= 0.09 Significance= 0.95599747 (hom/n)= 0.26 Avg. Litter Size= 9
Mutation Information
Mutation Type: Homologous Recombination (standard)
Description: The gene consists of 5 exons, with the start codon located in exon 1 (NCBI accession NM_031380.1). Exons 1 through 4 were targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.18.1. PHENOTYPIC ANALYSIS (for disrupted gene: DNA62306-1570 (UNQ674)

### (a) OVERALL PHENOTYPIC SUMMARY:

Mutation of the gene encoding the ortholog ofhuman follistatin-like 3 (FSTL3) resulted in decreased bone mineral content and density measurements in the (-/-) mice. Gene disruption was confirmed by Southern blot.

### (b) Bone Metabolism & Body Diagnostics

### Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (-/-) mice exhibited decreased mean bone mineral content and femur and vertebrae bone mineral density when compared with their gender matched (+/+) littermates and the historical means. The female (-/-) mice showed notably decreased bone mineral content, BMC/LBM and bone mineral density measurements compared with their gender-matched littermate controls. micro CT: The male (-/-) mice exhibited decreased mean femoral mid-shaft cross-sectional area when compared with that of their gender-matched (+/+) littermates and the historical means.

The (-/-) mice analyzed by DEXA and micro CT exhibited notably decreased bone mineral content and density measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO1308 polypeptides or agonists thereofwould be useful for maintaining bone homeostasis. In addition, PRO1308 polypeptides would be useful in bone healing or for the treatment of arthritis or osteoporosis, whereas antagonists (or inhibitors) of PRO1308 polypeptides or its encoding gene would lead to abnormal or pathological bone disorders including inflammatory diseases associated with abnormal bone metabolism including arthritis, osteoporosis and osteopenia.

### 43.19. Generation and Analysis of Mice Comprising DNA66667-1596 (UNQ693) Gene Disruptions

In these knockout experiments, the gene encoding PRO1338 polypeptides (designated as DNA66667-1596) (UNQ693) was disrupted. The gene specific information for these studies is as follows: the mutated mouse gene corresponds to nucleotide reference: NM_008516 ACCESSION:NM_008516 NID:6678723 Mus musculus Mus musculus leucine rich repeat protein 1, neuronal (Lrm1); protein reference: Q61809 ACCESSION:Q61809 NID: Mus musculus (Mouse). LEUCINE-RICH-REPEAT PROTEIN; the human gene sequence reference: NM_020873 Homo sapiens leucine rich repeat neuronal 1 (LRRN1); the human protein sequence corresponds to reference: Q6UXK5 ACCESSION:Q6UXK5 NID: Homo sapiens (Human).

The gene of interest is mouse Lrm1 (leucine rich repeat protein 1, neuronal), ortholog of human LRRN1 (leucine rich repeat neuronal 1). Aliases include NLRR-1, 2810047E21Rik, and KIAA1497.

LRRN1 is a type I integral plasma membrane protein that likely functions as a cell adhesion molecule (Hayata et al, Gene 221:159-66 (1998)). The protein contains a signal peptide, several leucine-rich repeats, an immunoglobulin-like C2-type domain, a fibronectin type III domain, a transmembrane segment, and a short cytoplasmic C terminus (UniProt entry LRRN1_HUMAN). The protein is expressed primarily in the central nervous system but is also expressed in other tissues, such as cartilage (Taguchi et al, Brain Res Mol Brain Res 35:31-40 (1996)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 25 | 37 | 14 | 76 |
| Expected | 19 | 38 | 19 | 76 |

Chi-Sq.= 3.48 Significance= 0.1755204 (hom/n)= 0.2 Avg. Litter Size= 9
Mutation Information
Mutation Type: Homologous Recombination (standard)
Description: The gene consists of 2 exons, with the start codon located in exon 2 (NCBI accession NM_008516.1).
Exon 2 was targeted.
1. Wild-type Expression Panel: Expression of the target gene was detected in embryonic stem (ES) cells and in all 13 adult tissue samples tested by RT-PCR, except skeletal muscle, bone, and adipose.
2. QC Expression: Disruption of the target gene was confirmed by Southern hybridization analysis.

### 43.19.1. PHENOTYPIC ANALYSIS (for disrupted gene: DNA66667-1596 (UNQ693)

### (a) OVERALL PHENOTYPIC SUMMARY:

Mutation of the gene encoding the ortholog of human leucine rich repeat neuronal 1 (LRRN1) resulted in decreased exploratory activity in (-/-) mice and hypoactivity during home caging testing. In contrast, the (-/-) mice spent less time in the center during open field testing which is usually associated with increased anxiety. The (-/-) mice also showed signs of growth retardation marked by decreased weight and length as well as decreased body mass measurements. The mutant (-/-) mice also showed an abnormal and decreased AN ratio during the fundus photography and angiogram procedures. Gene disruption was confirmed by Southern blot.

### (b) Expression in Normal and Diseased Human Tissues

UNQ693 is expressed in normal human breast, prostate, cartilage and in CNS tissues (GeneLogic data). UNQ693 is also upregulated in many tumors including brain, breast, kidney, ovarian, prostate and head and neck tumors.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The female (-/-) mice exhibited decreased mean body weight (1-2 SD lighter)when compared with that of their gender-matched (+/+) littermates and the historical mean.
Length: The female (-/-) mice exhibited decreased mean body length (∼1 SD shorter)when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: Both the male and female (-/-) mice exhibited decreased mean total tissue mass and lean body mass when compared with that of their gender-matched (+/+) littermates and the historical means. Male knockout (-/-) mice also exhibited an increased BMC/LBM index.

Mutant (-/-) mice deficient in the gene encoding PRO1338 polypeptides show a phenotype consistent with growth retardation, marked by decreased body weight and length. Thus, antagonists or inhibitors of PRO1338 polypeptides or its encoding gene would mimic these metabolic and growth related effects. On the other hand, PRO1338 polypeptides or agonists thereof would be useful in the prevention and/or treatment of such metabolic disorders.

In addition, the (-/-) mice analyzed by DEXA exhibited decreased body mass measurements when compared with their (+/+) littermates, suggestive of abnormal growth or development disorders. The decreased mean total tissue mass and lean body mass is indicative of a metabolic disorder related to growth retardation and tissue wasting disorders. The negative metabolic phenotype indicates that PRO1338 polypeptides or agonists thereof would be useful for maintaining normal growth and development.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12 ), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The female (-/-) mice exhibited a trend of hypoactivity with decreased hole-poke and rearing indicative of a decreased exploratory behavior. However, the (-/-) mice spent less time (decreased) in the center which could be interpreted as an increased anxiety response.

The (-/-) mice demonstrated a decrease median sum time-in-center at intervals 2,3, and 5 when compared to the (+/+) mice, suggesting an increased anxiety-like response in the (-/-) mice. The (-/-) mice also exhibited a decreased exploratory activity. Hypoactive (-/-) mice was confirmed during circadian rhythm testing (as shown in home-cage activity as shown below).

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

The (-/-) mice exhibited decreased ambulatory counts (hypoactivity) during the 1-hour habituation period and both light periods of home-cage activity testing when compared with their gender-matched (+/+) littermates and the historical mean. These results are consistent with lethargy or depressive disorders. Antagonists or inhibitors of PRO1338 polypeptides or the PRO1338 encoding gene would be expected to mimic this behavior. Likewise, PRO1338 polypeptides or agonists thereof, would be useful in the treatment of such neurological disorders including depressive disorders or other decreased anxiety-like symptoms such as lethargy, cognitive disorders, hyperalgesia and sensory disorders.

### (e) Cardiovascular Phenotypic Analysis:

In the area of cardiovascular biology, phenotypic testing was performed to identify potential targets for the treatment of cardiovascular, endothelial or angiogenic disorders. One such phenotypic test included optic fundus photography and angiography to determine the retinal arteriovenous ratio (A/V ratio) in order to flag various eye abnormalities. An abnormal A/V ratio signals such systemic diseases or disorders that may be related to the vascular disease of hypertension (and any disease that causes hypertension, e.g. atherosclerosis), diabetes or other ocular diseases corresponding to ophthalmological disorders. Such eye abnormalities may include but are not limited to the following: retinal abnormality is retinal dysplasia, various retinopathies, restenosis, retinal artery obstruction or occlusion; retinal degeneration causing secondary atrophy of the retinal vasculature, retinitis pigmentosa, macular dystrophies, Stargardt's disease, congenital stationary night blindness, choroideremia, gyrate atrophy, Leber's congenital amaurosis, retinoschisis disorders, Wagner's syndrome, Usher syndromes, Zellweger syndrome, Saldino-Mainzer syndrome, Senior-Loken syndrome, Bardet-Biedl syndrome, Alport's syndrome, Alstom's syndrome, Cockayne's syndrome, dysplaisa spondyloepiphysaria congentia, Flynn-Aird syndrome, Friedreich ataxia, Hallgren syndrome, Marshall syndrome, Albers-Schnoberg disease, Refsum's disease, Kearns-Sayre syndrome, Waardenburg's syndrome, Alagile syndrome, myotonic dystrophy, olivopontocerebellar atrophy, Pierre-Marie dunsdrome, Stickler syndrome, carotinemeia, cystinosis, Wolfram syndrome, Bassen-Kornzweig syndrome, abetalipoproteinemia, incontinentia pigmenti, Batten's disease, mucopolysaccharidoses, homocystinuria, or mannosidosis.

Procedure: A cohort of 4 wild type, 4 heterozygous and 8 homozygous were tested in this assay. Optic fundus photography was performed on conscious animals using a Kowa Genesis small animal fundus camera modified according to Hawes and coauthors (Hawes et al., 1999 Molecular Vision 1999; 5:22 ). Intra-peritoneal injection of fluorescein permitted the acquisition of direct light fundus images and fluorescent angiograms for each examination. In addition to direct ophthalmological changes, this test can detect retinal changes associated with systemic diseases such as diabetes and atherosclerosis or other retinal abnormalities. Pictures were provided of the optic fundus under normal light. The angiographic pictures allowed examination of the arteries and veins of the eye. In addition an artery to vein (A/V) ratio was determined for the eye.

Ophthalmology analysis was performed on generated F2 wild type, heterozygous, and homozygous mutant progeny using the protocol described above. Specifically, the AN ratio was measured and calculated according to the fundus images with Kowa COMIT+ software. This test takes color photographs through a dilated pupil: the images help in detecting and classifying many diseases. The artery to vein ratio (A/V) is the ratio of the artery diameter to the vein diameter (measured before the bifurcation of the vessels). Many diseases will influence the ratio, i.e., diabetes, cardiovascular disorders, papilledema, optic atrophy or other eye abnormalities such as retinal degeneration (known as retinitis pigmentosa) or retinal dysplasia, vision problems or blindness. Thus, phenotypic observations which result in an increased artery-to-vein ratio in homozygous (-/-) and heterozygous (+/-) mutant progeny compared to wildtype (+/+) littermates would be indicative of such pathological conditions.

Results: In this study, the (-/-) mice exhibited an increased mean artery-to-vein (A/V) ratio (1 SD above) when compared with their (+/+) littermates indicating an abnormal eye physiology. However, arteries were not clearly enlarged and the veins did not appear clearly smaller. In summary, by knocking out the gene identified as DNA66667-1596 encoding PRO1338 polypeptides, homozygous mutant progeny exhibit phenotypes which are associated with an abnormal A/V ratio. Such detected retinal changes are most commonly associated with cardiovascular systemic diseases or disorders that may be related to the vascular disease of hypertension (and any disease that causes hypertension, e.g. atherosclerosis), diabetes or other ocular diseases corresponding to ophthalmological disorders such as retinal degeneration. Thus, antagonists of PRO1338 encoding genes would lead to similar pathological retinal changes, whereas agonists would be useful as therapeutic agents in the treatment of hypertension, atherosclerosis or other opthamological disorders including retinal degeneration and diseases associated with this condition (as indicated above).

### 43.20. Generation and Analysis of Mice Comprising DNA58730-1607 (UNQ715) Gene Disruptions

In these knockout experiments, the gene encoding PRO1378 polypeptides (designated as DNA58730-1607) (UNQ715) was disrupted. The gene specific information for these studies is as follows: the mutated mouse gene corresponds to nucleotide reference: NM_133778 Mus musculus RIKEN cDNA 2900046G09 gene (2900046G09Rik); protein reference: Q8BWU3 ACCESSION:Q8BWU3 NID: Mus musculus (Mouse). Mus musculus adult male hippocampus cDNA, RIKEN full-length enriched library, clone:C630035L06 product:hypothetical Bacterial extracellular solute-binding proteins, family 3 containing protein, full insert sequence; the human gene sequence reference: NM_144635 ACCESSION:NM_144635 NID: gi 40255250 ref NM_144635.3 Homo sapiens hypothetical protein MGC21688 (MGC21688); the human protein sequence corresponds to reference: Q6UXB0 ACCESSION:Q6UXB0 NID: Homo sapiens (Human). FLAT715.

The mouse gene of interest is RIKEN cDNA 2900046G09 gene, ortholog of human hypothetical protein MGC21688. Aliases include UNQ715 and FLAT715.

Hypothetical protein MGC21688 is a putative secreted protein (Clark et al, Genome Res 13:2265-70 (2003)), consisting of 335 amino acids and containing a signal peptide.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F 1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 17 | 44 | 25 | 86 |
| Expected | 21.5 | 43 | 21.5 | 86 |

Chi-Sq.= 0.54 Significance= 0.7633795 (hom/n)= 0.25 Avg. Litter Size= 9
Mutation Information
Mutation Type: Homologous Recombination (standard)
Description: The gene consists of 6 exons, with the start codon located in exon 1 (NCBI accession NM_133778.1).
Exons 3 through 5 were targeted.
1. Wild-type Expression Panel: Expression of the target gene was detected in embryonic stem (ES) cells and in all 13 adult tissue samples tested by RT-PCR, except adipose.
2. QC Expression: Disruption of the target gene was confirmed by Southern hybridization analysis.

### 43.20.1. PHENOTYPIC ANALYSIS (for disrupted gene: DNA58730-1607 (UNQ715)

### (a) OVERALL PHENOTYPIC SUMMARY:

Mutation of the gene encoding the ortholog of a human putative secreted protein (MGC21688) resulted in the mutant (-/-) mice exhibiting decreased mean percent total body fat. Gene disruption was confirmed by Southern blot.

### (b) Expression in Human Normal Tissues

UNQ715 shows high expression in the central nervous system as shown by GeneLogic data.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The female (-/-) mice exhibited decreased mean percent total body fat and total fat mass when compared with that of their gender-matched (+/+) littermates and the historical means.

Mutant (-/-) mice deficient in the gene encoding PRO1378 polypeptides show a phenotype consistent with growth abnormalities and tissue wasting diseases, marked by decreased fat stores. Thus, antagonists or inhibitors of PRO1378 polypeptides or its encoding gene would mimic these metabolic and growth related effects. On the other hand, PRO1378 polypeptides or agonists thereof would be useful in the prevention and/or treatment of such metabolic disorders as tissue wasting diseases.

### 43.21. Generation and Analysis of Mice Comprising DNA58852-1637 (UNQ731) Gene Disruptions

In these knockout experiments, the gene encoding PRO1415 polypeptides (designated as DNA58852-1637) (UNQ731) was disrupted. The gene specific information for these studies is as follows: the mutated mouse gene corresponds to nucleotide reference: NM_146162 ACCESSION:NM_146162 NID: gi 22122696 ref NM_146162.1 Mus musculus hypothetical protein MGC38046 (MGC38046); protein reference: Q8R138 ACCESSION:Q8R138 NID: Mus musculus (Mouse). Hypothetical 29.4 kDa protein; the human gene sequence reference: NM_181724 ACCESSION:NM_181724 NID: gi 32171198 ref NM_181724.1 Homo sapiens hypothetical protein LOC338773 (LOC338773); the human protein sequence corresponds to reference: Q8N2F5 ACCESSION:Q8N2F5 NID: Homo sapiens (Human). Hypothetical protein PSEC0199.

The gene of interest is mouse cDNA sequence BC025600, ortholog of human hypothetical protein LOC338773. Aliases include MGC38046 and LOC338773.

Hypothetical protein LOC338773 is a putative type I plasma membrane protein (Clark et al, Genome Res 13:2265-70 (2003)). The protein is predicted to contain a signal peptide, a short extracellular domain, a transmembrane segment, and a cytoplasmic domain.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 25 | 35 | 13 | 73 |
| Expected | 18.25 | 36.5 | 18.25 | 73 |

Chi-Sq.= 8.81 Significance= 0.012216104 (hom/n)= 0.17 Avg. Litter Size= 9
Mutation Information
Mutation Type: Homologous Recombination (standard)
Description: The gene consists of 2 exons, with the start codon located in exon 2 (NCBI accession NM_146162.1).
Exon 2 was targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.21.1. PHENOTYPIC ANALYSIS (for disrupted gene: DNA58852-1637 (UNQ731)

### (a) OVERALL PHENOTYPIC SUMMARY:

Mutation of the gene encoding the ortholog of hypothetical human protein (LOC338773) resulted in the female (-/-) mice exhibiting a decreased depressive-like response in the tail suspension testing. The mutant (-/-) mice also exhibited increased bone mineral density measurements, and increased vertebral trabecular bone number and connectivity density. Gene disruption was confirmed by Southern blot.

### (b) Expression in Human Diseased Tissues

UNQ731 is overexpressed in many inflammatory tissues including bone disorders such as osteoarthritis; osteosarcoma; giant cell tumors and in adenocarcinomas (biopsy data).

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The female (-/-) mice exhibited increased total body volume bone mineral density (vBMD) when compared with that of their gender-matched (+/+) littermates. micro CT: The male (-/-) mice exhibited increased mean vertebral trabecular bone number and connectivity density when compared with that of their gender-matched (+/+) littermates and the historical means.

In summary, the (-/-) mice exhibited increased mean bone mineral density as well as increased microCT bone measurements when compared with their gender-matched (+/+) littermates. These results indicate that the knockout mutant phenotype may be associated with such bone abnormalities as osteopetrosis. Osteopetrosis is a condition characterized by abnormal thickening and hardening of bone and abnormal fragility of the bones. As such, PRO1415 polypeptides or agonists thereof would be beneficial for the treatment of osteopetrosis or other osteo-related diseases. On the other hand, inhibitors or antagonists of PRO1415 polypeptides would be useful in bone healing.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Functional Observational Battery (FOB) Test - Tail Suspension Testing:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Tail Suspension Testing:

The tail suspension test is a procedure that has been developed as a model for depressive-like behavior in rodents. In this particular setup, a mouse is suspended by its tail for 6 minutes, and in response the mouse will struggle to escape from this position. After a certain period of time the struggling of the mouse decreases and this is interpreted as a type of learned helplessness paradigm. Animals with invalid data (i.e. climbed their tail during the testing period) are excluded from analysis.

### Results:

Tail Suspension2: The (-/-) mice exhibited decreased median immobility time when compared with that of their (+/+) littermates and the historical mean, indicating a decreased learned helplessness and a decreased depressive-like response in the mutants.

In summary, the tail suspension testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO1415 polypeptides or agonists thereofwould be useful in the treatment of such neurological disorders.

### 43.22. Generation and Analysis of Mice Comprising DNA84925-2514 (UNQ858) Gene Disruptions

In these knockout experiments, the gene encoding PRO1867 polypeptides (designated as DNA84925-2514) (UNQ858) was disrupted. The gene specific information for these studies is as follows: the mutated mouse gene corresponds to nucleotide reference: NM_010084 ACCESSION:NM_010084 NID:6753683 Mus musculus Mus musculus a disintegrin and metalloprotease domain 18 (Adam18); protein reference: Q9R157 AD18_MOUSE Q9R157 ADAM 18 PRECURSOR A DISINTEGRIN AND ME:; the human gene sequence reference: NM_014237 ACCESSION:NM_014237 NID:7656860 Homo sapiens Homo sapiens a disintegrin and metalloproteinase domain 18 (ADAM18); the human protein sequence corresponds to reference: Q9Y3Q7 AD18_HUMAN Q9Y3Q7 ADAM 18 PRECURSOR A DISINTEGRIN AND ME.

The gene of interest is mouse Adam18 (a disintegrin and metalloprotease domain 18), ortholog of human ADAM18. Aliases include Dtgn3, disintegrin 3, Adam27, tMDCIII, and MGC88272.

ADAM 18 is a type I integral plasma membrane protein expressed primarily on the surface of developing and mature sperm. The protein is a likely inactive ADAM (A Disintegrin And Metalloprotease) family protease that functions as a cell adhesion molecule. ADAM18 contains a signal peptide, a reprolysin family propeptide domain, a reprolysin (M12B) family zinc metalloprotease domain, a disintegrin domain, an ADAM cysteine-rich (ACR) domain, and a transmembrane segment near the C terminus. The catalytic domain of ADAM18, however, does not possess the conserved zinc-dependent metalloprotease active site "HEXGHXXGXXHD. As spermatozoa transit the epididymus, ADAM18 is proteolytically processed, retaining its disintegrin and ACR domains. ADAM18 may play a role in sperm-oocyte recognition or fertilization (Frayne et al, J Reprod Fertil Suppl 53:149-55(1998); Zhu et al, Gene 234:227-37 (1999); Frayne et al, Mol Hum Reprod 8:817-22 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 14 | 44 | 19 | 77 |
| Expected | 19.25 | 38.5 | 19.25 | 77 |

Chi-Sq.= 0.34 Significance= 0.8436648 (hom/n)= 0.25 Avg. Litter Size= 9
Mutation Information
Mutation Type: Homologous Recombination (standard)
Description: The gene consists of 20 exons, with the start codon located in exon 1 (NCBI accession NM_010084.1). Exons 1 and 2 were targeted.
1. Wild-type Expression Panel: Expression of the target gene was detected in brain, spinal cord, and eye among 13 adult tissue samples tested by RT-PCR.
2. QC Expression: Disruption of the target gene was confirmed by Southern hybridization analysis.

### 43.22.1. PHENOTYPIC ANALYSIS (for disrupted gene: DNA84925-2514 (UNQ858)

### (a) OVERALL PHENOTYPIC SUMMARY:

Mutation of the gene encoding the ortholog of human a disintegrin and metalloprotease domain 18 (ADAM 18) resulted in an enhanced motor coordination in the mutant (-/-) mice. Gene disruption was confirmed by Southern blot.

### (b) Expression in Human Normal/Diseased Tissues

UNQ858 appears to be down regulated in human pancreatic diseases including adenocarcinomas and Islet cell carcinoma (microarray data). Normal pancreatic tissues show elevated expression compared to other tissues. In addition UNQ858 is expressed on neutrophils and B cell subsets.

### (c) Phenotypic Analysis: CNS/Neurology

In the area ofneurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Inverted Screen Testing:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Inverted Screen Test Data:

The Inverted Screen is used to measure motor strength/coordination. Untrained mice were placed individually on top of a square (7.5 cm x 7.5 cm) wire screen which was mounted horizontally on a metal rod. The rod was then rotated 180 degrees so that the mice were on the bottom of the screens. The following behavioral responses were recorded over a 1 min testing session: fell off, did not climb, and climbed up.

The Inverted Screen Test is designed to measure basic sensory & motor observations: Inverted Screen: All 8 (-/-) mice climbed up the inverted screen whereas only 4/8 (+/+) mice climbed up, suggesting enhanced motor coordination in the (-/-) mutants.

### Results:

| Genotype | Ratio Fell Down % | Ratio Climbed up % | |
|---|---|---|---|
| +/+ (n=8) | 0/8 | 4/8 | 50% |
| -/- (n=8) | 0/8 | 8/8 | 100% |

A motor strength deficit is apparent when there is a 50% point difference between (-/-) or (+/-) mice and (+/+) mice for the fell down response. 0/8 or 1/8 (-/-) or (+/-) mice not climbing indicates impaired motor coordination. 7/8 or 8/8(-/-) or (+/-) mice climbing up indicates enhanced motor coordination.

### 43.23. Generation and Analysis of Mice Comprising DNA79230-2525 (UNQ872) Gene Disruptions

In these knockout experiments, the gene encoding PRO890 polypeptides (designated as DNA79230-2525) (UNQ872) was disrupted. The gene specific information for these studies is as follows: the mutated mouse gene corresponds to nucleotide reference: NM_139134 ACCESSION:NM_139134 NID:20977544 Mus musculus Mus musculus chondrolectin (Chodl); protein reference: Q9CXM0 ACCESSION:Q9CXM0 NID: Mus musculus (Mouse). Chondrolectin precursor (Transmembrane protein MT75); the human gene sequence reference: NM_024944 ACCESSION:NM_024944 NID:20127635 Homo sapiens Homo sapiens chondrolectin (CHODL); the human protein sequence corresponds to reference: Q9H9P2 ACCESSION:Q9H9P2 NID: Homo sapiens (Human). TRANSMEMBRANE PROTEIN MT75 PRECURSOR (PROTEIN C21ORF68) (PRED 12 PROTEIN).

The gene of interest is mouse Chodl (chondrolectin), ortholog ofhuman CHODL. Aliases include MT75, PRED12, 3110074E07Rik, C21orf68, and FLJ12627.

CHODL is a type I integral membrane protein, containing a signal peptide, a C-type lectin domain, a transmembrane segment, and a short C-terminal tail (Weng et al, Genomics 80:62-70 (2002)). Proteins with C-type lectin domains are typically capable of binding different types of sugars in a calcium-dependent manner (InterPro accession IPRO01304). C-type animal lectins can function as cell adhesion molecules, endocytic receptors, or extracellular matrix proteins (Weis et al, Science 254:1608-15 (1991)). Although CHODL is predicted to be an extracellular protein (Clark et al, Genome Res 13:2265-70 (2003)), the subcellular location of this protein in transfected COS1 cells is primarily perinuclear (Weng et al,Genomics 80:62-70 (2002)). CHODL is expressed in vascular muscle oftestis, smooth muscle ofprostate stroma, heart muscle, skeletal muscle, crypts ofsmall intestine, and red pulp of spleen (Weng et al, Genomics 80:62-70 (2002)).

In T cells, two additional CHODL variants have been characterized. One variant lacks the signal peptide but retains the transmembrane segment near the C-terminal tail. This variant is located primarily on the endoplasmic reticulum and Golgi apparatus but not on the plasma membrane. Another variant lacks a signal peptide and contains a divergent C-terminal tail with no transmembrane segment. This variant is located on granule-like structures in the cytosol. Because the CHODL variant lacking the transmembrane segment is expressed primarily during T lymphopoiesis, it may play a role in T cell maturation (Weng et al, J Biol Chem 278:19164-70 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 30 | 41 | 15 | 86 |
| Expected | 21.5 | 43 | 21.5 | 86 |

Chi-Sq.= 3.2 Significance= 0.20189652 (hom/n)= 0.2 Avg. Litter Size= 9
Mutation Information
Mutation Type: Homologous Recombination (standard)
Description: The gene consists of 6 exons, with the start codon located in exon 1 (NCBI accession NM_139134.1).
Exon 2 was targeted.
1. Wild-type Expression Panel: Expression of the target gene was detected in all 13 adult tissue samples tested by RT-PCR, except spleen, liver, and bone.
2. QC Expression: Disruption of the target gene was confirmed by Southern hybridization analysis.

### 43.23.1. PHENOTYPIC ANALYSIS (for disrupted gene: DNA79230-2525 (UNQ872)

### (a) OVERALL PHENOTYPIC SUMMARY:

Mutation of the gene encoding the ortholog ofhuman chondrolectin (CHODL) resulted in small (-/-) pups, most dying by 2 weeks of age. The health of the (-/-) mice improved with age, but the males remained smaller. The (-/-) mice also exhibited decreased total tissue mass, lean body mass, decreased body fat and decreased vertebrae bone mineral density. The mutant (-/-) mice exhibited many alterations in the hematopoietic system. Adult (-/-) mice exhibited small islets ofLangerhans but lacked the numerous lesions observed microscopically in the mutant pups. Increased ambulation counts were observed during home cage activity testing in the mutant (-/-) mice. Gene disruption was confirmed by Southern blot.

### (b) Pathology

Gross: The (-/-) pups were small for their age and many die, but some reach maturity.
Microscopic: The (-/-) pups available for analysis were approximately one-half normal size for their age and exhibited many alterations in hematopoietic system: hypoplasia oflymphoid and hematopoietic cells in the spleen, cytoplasmic vacuolization in hepatocytes, lipid depletion in adipose tissue, and reduced hematopoiesis in bone marrow. The lesions were relatively non-specific but may be directly gene-related. Hematology results from the pups are needed to interpret the reduced hematopoiesis in the spleen and bone marrow. The 4 adult (-/-) mice analyzed exhibited smaller-than-normal islets of Langerhans. The other microscopic lesions are considered to be background changes.

### (c) Expression in Human Normal/Diseased Tissues

UNQ872 is overexpressed in ovarian diseased tissues as shown by microarray data. UNQ872 is expressed in lymph, prostate, testes and vascular heart muscle (GeneLogic data)

### (d) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited decreased mean body weight when compared with that of their gender-matched (+/+) littermates and the historical mean. However, the mean body weight for both the males and females improved with age. Length: The male (-/-) mice exhibited a decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both
trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean total tissue mass, lean body mass, total body fat mass, and vertebrae bone mineral content and density measurements when compared with those of their gender-matched wild-type littermates and the historical means. CATScan: Of the 2 male (-/-) mice examined, 1 exhibited an undescended and enlarged left testis (M-159).

Mutant (-/-) mice deficient in the gene encoding PRO1890 polypeptides show a phenotype consistent with reduced viability and growth retardation, marked by reduced number of (-/-) progeny and very small mutant mice with decreased body weight and length. Thus, antagonists or inhibitors of PRO1890 polypeptides or its encoding gene would mimic these metabolic and growth related effects. On the other hand, PRO1890 polypeptides or agonists thereof would be useful in the prevention and/or treatment of such metabolic disorders.

In addition, the (-/-) mice analyzed by DEXA exhibited decreased bone measurements and decreased body mass measurements when compared with their (+/+) littermates, suggestive of abnormal bone disorders. In addition, the decreased mean total tissue mass, lean body mass and lipid depletion (decreased body fat) is indicative of a metabolic disorder related to growth retardation and tissue wasting disorders. The negative bone and metabolic phenotype indicates that PRO1890 polypeptides or agonists thereofwould be useful for maintaining bone homeostasis in addition to normal growth development. In addition, PRO1890 polypeptides would be useful in bone healing or for the treatment of arthritis or osteoporosis, whereas antagonists (or inhibitors) of PRO1890 polypeptides or its encoding gene would lead to abnormal or pathological bone disorders including inflammatory diseases associated with abnormal bone metabolism including arthritis, osteoporosis and osteopenia.

### (e) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day oftesting in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited increased ambulatory counts during the 1-hour habituation period when compared with that of their gender-matched (+/+) littermates and the historical mean.

The female (-/-) mice exhibited increased ambulatory counts during home-cage activity testing resulting in a hyperactive behavior pattern when compared with their gender-matched (+/+) littermates and the historical means. These observations during home-cage activity testing is also suggestive of increased anxiety which is consistent with neurological disorders such as generalized anxiety disorders, attention deficit hyperactivity disorder, obsessive compulsive disorder, schizophrenia, cognitive disorders generalized anxiety disorder. Thus, antagonists or inhibitors of PRO1890 polypeptides or the PRO1890 encoding gene would be expected to mimic this behavior. Likewise, PRO1890 polypeptides or agonists thereof, would be useful in the treatment of such neurological disorders including generalized anxiety disorders, attention deficit hyperactivity disorder, obsessive compulsive disorder, schizophrenia, cognitive disorders generalized anxiety disorder.

### 43.24. Generation and Analysis of Mice Comprising DNA82364-2538 (UNQ1825) Gene Disruptions

In these knockout experiments, the gene encoding PRO3438 polypeptides (designated as DNA82364-2538) (UNQ1825) was disrupted. The gene specific information for these studies is as follows: the mutated mouse gene corresponds to nucleotide reference: NM_027763 Mus musculus triggering receptor expressed on myeloid cells-like 1 (Tremll); protein reference: Q8K558 ACCESSION:Q8K558 NID: Mus musculus (Mouse). Trem-like transcript protein; the human gene sequence reference: NM_178174 Homo sapiens triggering receptor expressed on myeloid cells-like 1 (TREML1); the human protein sequence corresponds to reference:Q8IWY2 ACCESSION:Q8IWY2 NID: Homo sapiens (Human). TREM-like transcript 1 (OTTHUMP00000017858).

The gene of interest is mouse Treml1 (triggering receptor expressed on myeloid cells-like 1), ortholog of human TREML1. Aliases include TLT-1, 5430401J17Rik, TLT1, PRO3438, GLTL1825, and dJ238O23.3.

TREML 1 is a type I integral membrane protein expressed primarily on megakaryocytes and platelets. The protein contains a signal peptide, an immunoglobulin (IG) domain (InterPro accession IPRO03599), a transmembrane segment, and a cytoplasmic immunoreceptor tyrosine-based inhibition motif (ITIM). Upon activation of platelets or megakaryocytes, TREML1 moves from intracellular alpha granules to the cell surface, where it likely functions as a signal-transducing receptor or coreceptor. TREML1 is capable of recruiting Src homology 2 domain-containing tyrosine phosphatase (SHP)-2 and enhancing calcium mobilization. TREML1 likely plays a role in vascular hemostasis, blood coagulation, and inflammation at sites of vascular injury (Washington et al, Blood 104:1042-7 (2004); Barrow et al, J Immunol. 172:5838-42 (2004); 172:5838-42 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 18 | 23 | 18 | 59 |
| Expected | 14.75 | 29.5 | 14.75 | 59 |

Chi-Sq.= 6.8 Significance= 0.033373266 (hom/n)= 0.33 Avg. Litter Size= 8
Mutation Information
Mutation Type: Homologous Recombination (standard)
Description: The gene consists of 6 exons, with the start codon located in exon 1 (NCBI accession NM_027763.1).
Exons 1 and 2 were targeted.
1. Wild-type Expression Panel: Expression of the target gene was detected in embryonic stem (ES) cells and in all 13 adult tissue samples tested by RT-PCR, except skeletal muscle; stomach, small intestine, colon and adipose.
2. QC Expression: Disruption of the target gene was confirmed by Southern hybridization analysis.

### 43.24.1. PHENOTYPIC ANALYSIS (for disrupted gene: DNA82364-2538 (UNQ1825)

### (a) OVERALL PHENOTYPIC SUMMARY:

Mutation of the gene encoding the ortholog of human triggering receptor expressed on myeloid cells-like 1 (TREML1) resulted in enhanced glucose tolerance in the male (-/-) mice. Gene disruption was confirmed by Southern blot.

### (b) Expression in Human TissueslCells

UNQ1825 shows high expression in skin tissue as shown by GeneLogic data. UNQ1825 is also expressed on monocyte and dendritic cells. UNQ1825shows down regulation in psoriasis patients as shown by microarray data.

### (c) Phenotypic Analysis: Metabolism -Blood ChemistrylGlucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of wild type and homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

Oral Glucose Tolerance: The male (-/-) mice exhibited enhanced glucose tolerance when compared with that of their gender-matched (+/+) and heterozygous (+/-) littermates and the historical means.

In these studies the mutant (-/-) mice showed a notably decreased serum glucose levels and enhanced glucose tolerance which could be due to an increased insulin sensitivity. Thus, antagonists (inhibitors) to PRO3438 polypeptides or its encoding gene would be useful in the treatment of impaired glucose homeostasis.

### 43.25. Generation and Analysis of Mice Comprising DNA164647 (UNQ2194) Gene Disruptions

In these knockout experiments, the gene encoding PRO19835 polypeptides (designated as DNA 164647) (UNQ2194) was disrupted. The gene specific information for these studies is as follows: the mutated mouse gene corresponds to nucleotide reference: AK028976 ACCESSION:AK028976 NID: gi 26324937 dbj AK028976.1 Mus musculus 10 days neonate skin cDNA, RIKEN full-length enriched library, clone:4732477B07 product:ESTROGEN REGULATED LIV-1 PROTEIN homolog [Homo sapiens], full insert sequence; protein reference: Q8C145 ACCESSION:Q8C145 NID: Mus musculus (Mouse). Zinc transporter SLC39A6 precursor (Solute carrier family 39, member 6) (Endoplamic reticulum membrane-linked protein) (Ermelin); the human gene sequence reference: NM_012319 ACCESSION:NM_012319 NID:12751474 Homo sapiens Homo sapiens LIV-1 protein, estrogen regulated (LIV-1); the human protein sequence corresponds to reference: Q13433 ACCESSION:Q13433 NID: Homo sapiens (Human). ESTROGEN REGULATED LIV-1 PROTEIN.

The gene of interest is mouse Slc39a6 (solute carrier family 39 [metal ion transporter], member 6), ortholog ofhuman SLC39A6 (solute carrier family 39 [zinc transporter], member 6). Aliases include LIV1, LIV-1, and ermelin.

SLC39A6 is an integral membrane protein located in the plasma membrane (Taylor and Nicholson, Biochim Biophys Acta 1611:16-30 (2003); Taylor et al, Biochem J 375:51-9 (2003); Chowanadisai et al, J Nutr 135:1002-7 (2005)) and the endoplasmic reticulum (Suzuki and Endo, Gene 284:31-40 (2002); Kasper et al, Int J Cancer 117(6):961-73 (2005)) and is likely to function as a zinc transporter. The protein contains a signal peptide, a long N-terminal segment, and six transmembrane segments within a ZIP family zinc transporter domain (InterPro accession IPRO03689). SLC39A6 expression is evident in breast, prostate, placenta, kidney, pituitary gland, testis, and several brain regions. Expression of SLC39A6 appears to be regulated by ubiquitin-dependent degradation and hormones, such as estrogen (Taylor e al, Biochem J 375:51-9 (2003); Taylor and Nicholson, Biochim Biolphys Acta 1611:16-30 (2003); Dressman et al, Pharmacogenomics J 1:135-41 (2001); Suzuki and Endo, Gene 284:31-40 (2002)). Because zinc is a cofactor for hundreds of different enzymes, SLC39A6 may play a role in numerous physiological processes. In zebrafish, SLC39A6 is part of a pathway that regulates epithelial-mesenchymal transition (EMT), which is central for processes such as embryonic development, organ and tissue regeneration, and cancer metastasis (Yamashita et al, Nature 429:298-302 (2004)). In humans, SLC39A6 is associated with breast cancer (Taylor et al, Biochem J 375:51-9 (2003); Yamashita et al, Nature 429:298-302 (2004); Kasper et al, Int J Cancer 117(6):961-73 (2005)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 14 | 34 | 18 | 66 |
| Expected | 16.5 | 33 | 16.5 | 66 |

Chi-Sq.= 1.31 Significance= 0.5194421 (hom/n)= 0.28 Avg. Litter Size= 7
Mutation Information
Mutation Type: Homologous Recombination (standard)
Description: The gene consists of 10 exons, with the start codon located in exon 2 (NCBI accession AK028976).
Exons 2 through 4 were targeted.
1. Wild-type Expression Panel: Expression of the target gene was detected in embryonic stem (ES) cells and in all 13 adult tissue samples tested by RT-PCR, except liver, skeletal muscle, bone, and adipose.
2. QC Expression: Disruption of the target gene was confirmed by Southern hybridization analysis.

### 43.25.1. PHENOTYPIC ANALYSIS (for disrupted gene: DNA164647 (UNQ2194)

### (a) OVERALL PHENOTYPIC SUMMARY:

Mutation of the gene encoding the ortholog of human solute carrier family 39 (zinc transporter), member 6 (SLC39A6) resulted in an increased serum TNF-alpha, MCP1 and IL6 responses to LPS challenge in the (-/-) mice. Male (-/-) mice also exhibited an increased anxiety-like response. Gene disruption was confirmed by Southern blot.

### (b) Expression in Human Diseased Tissues

UNQ2194 is overexpressed in breast tumors as shown by microarray data. In addition, the gene is upregulated in the inflammatory disease associated with psoriasis.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the maj or histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Acute Phase Response:

Test Description: Bacterial lipopolysaccharide (LPS) is an endotoxin, and as such is a potent inducer of an acute phase response and systemic inflammation. The Level I LPS mice were injected intraperitoneally (i.p.) with a sublethal dose of LPS in 200 µL sterile saline using a 26 gauge needle. The doses were based on the average weight of the mice tested at 1 µg/g body weight 3 hours after injection; a 100ul blood sample was then taken and analyzed for the presence of TNFalpha, MCP-1, and IL-6 on the FACS Calibur instrument.

### Results:

Acute Phase Response: The male (-/-) mice exhibited an increased mean serum TNF-alpha, MCP1 and IL6 responses to LPS challenge when compared with that of their (+/+) littermates and the historical mean.

In summary, the LPS endotoxin challenge demonstrated that knockout mice deficient in the gene encoding PRO19835 polypeptides exhibit immunological abnormalities when compared with their wild-type littermates. In particular, the mutant mice exhibited an increased ability to elicit an immunological response (TNF-alpha, MCP1, and IL6 production) when challenged with the LPS endotoxin indicating a proinflammatory response. TNF-alpha, MCP1 and IL6 contribute to the later stages ofB cell activation. In addition, TNF-alpha, MCP1 and IL6 play a critical role in inducing the acute phase response and systemic inflammation. This suggests that inhibitors or antagonists to PRO19835 polypeptides would stimulate the immune system and would find utility in the cases wherein this effect would be beneficial to the individual such as in the case of leukemia, and other types of cancer, and in immuno-compromised patients, such as AIDS sufferers. Accordingly, PRO19835 polypeptides or agonists thereof would be useful in inhibiting the immune response and would be useful candidates for suppressing harmful immune responses, e.g. in the case of graft rejection or graft-versus-host diseases.

### (d) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The male (-/-) mice exhibited a decreased median sum time-in-center when compared with that of their gender-matched (+/+) littermates and the historical means, suggesting an increased anxiety-like response in the mutants.

The (-/-) mice demonstrated a decrease median sum time-in-center at intervals 2, 3, and 5 when compared to the (+/+) mice, suggesting an increased anxiety-like response in the (-/-) mice. In summary, the open field testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO19835 polypeptides or agonists thereof would be useful in the treatment of such neurological disorders.

### 43.26. Generation and Analysis of Mice Comprising DNA226452 (UNQ2235) Gene Disruptions

In these knockout experiments, the gene encoding PRO36915 polypeptides (designated as DNA226452) (UNQ2235) was disrupted. The gene specific information for these studies is as follows: the mutated mouse gene corresponds to nucleotide reference:NM_011401 ACCESSION:NM_011401 NID: gi 31543725 refNM_011401.2 Mus musculus solute carrier family 2 (facilitated glucose transporter), member 3 (Slc2a3); protein reference: P32037 ACCESSION:P32037 NID: Mus musculus (Mouse). SOLUTE CARRIER FAMILY 2, FACILITATED GLUCOSE TRANSPORTER, MEMBER 3 (GLUCOSE TRANSPORTER TYPE 3, BRAIN); the human gene sequence reference: NM_006931 ACCESSION:NM_006931 NID: gi 5902089 refNM_006931.1 Homo sapiens solute carrier family 2 (facilitated glucose transporter), member 3 (SLC2A3); the human protein sequence corresponds to reference: P11169 ACCESSION:P11169 NID: Homo sapiens (Human). SOLUTE CARRIER FAMILY 2, FACILITATED GLUCOSE TRANSPORTER, MEMBER 3 (GLUCOSE TRANSPORTER TYPE 3, BRAIN).

The gene of interest is mouse Slc2a3 (solute carrier family 2 (facilitated glucose transporter), member 3), ortholog of human SLC2A3. Aliases include Glut3 and Glut-3.

SLC2A3 is an integral plasma membrane protein that functions as a facilitative glucose transporter, catalyzing the transport of glucose down its concentration gradient into cells (Watson and Pessin, Recent Prog Horm Res 56:175-93 (2001); Korgun et al., Biol Reprod 65:1364-70 (2001)). The protein is primarily expressed in neurons (Watson and Pessin, Recent Prog Horm Res 56:175-93 (2001); Nagamatsu et al, J Biol Chem 267:467-72 (1992)) but is also expressed in vascular endothelium of placenta (Hauguel-de Mouzon et al, J Clin Endocrinol Metab 82:2689-94 (1997)), in articular cartilage (Richardson et al, Osteoarthritis Cartilage 11:92-101 (2003)), and in lymphocytes, monocytes, and macrophages (Stuart et al., Metabolism 50:771-7 (2001); Fu et al, Blood Cells Mol Dis 32:182-90 (2004)). Moreover, GLUT3 is often overexpressed in malignant cells (Macheda et al, J Cell Physiol 202:654-62 (2005)). GLUT3 is likely to be important for energy metabolism in tissues or cells requiring high glucose demand (Hamlin at al., J Neurotrauma 18:1011-8 (2001); Gould and Holman, Biochem J 295 ( Pt 2):329-41 (1993)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 431 | 0 | 52 |
| Expected | 13 | 26 | 13 | 52 |

Chi-Sq.= 42.05 Significance= 7.3953493E-10 (hom/n)= 0.0 Avg. Litter Size= 8
Mutation Information
Mutation Type: Homologous Recombination (standard)
Description: The gene consists of 10 exons, with the start codon located in exon 1 (NCBI accession NM_011401.2). Exons 1 through 3 were targeted.
1. Wild-type Expression Panel: Expression of the target gene was detected in embryonic stem (ES) cells and in all 13 adult tissue samples tested by RT-PCR, except eye and bone.
2. QC Expression: Disruption of the target gene was confirmed by Southern hybridization analysis.

### 43.26.1. PHENOTYPIC ANALYSIS (for disrupted gene: DNA226452 (UNQ2235)

### (a) OVERALL PHENOTYPIC SUMMARY:

Mutation of the gene encoding the ortholog of human solute carrier family 2 (facilitated glucose transporter), member 3) (SLC2A3) resulted in lethality of (-/-) mutants. The male heterozygous (+/-) mice exhibited increased total tissue mass and total fat mass. Male (+/-) mice also exhibited an impaired glucose tolerance. Gene disruption was confirmed by Southern blot.

### (b) Expression in Human Normal/Diseased Tissues

UNQ2235 is overexpressed in kidney clear cell carcinoma as shown by microarray analysis. UNQ2235 is expressed in the lung and on white blood cells (WBCs) (GeneLogic data).

### (c) Pathology

Microscopic: Due to embryonic lethality, microscopic analysis was not performed. At 12.5 days, there were 47 embryos observed: 16 (+/-) embryos, 14 (+/+) embryos, 12 resorption moles, and 5 to-be-determined.

### (d) Bone Metabolism & Body Diagnostics

### Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type and heterozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (+/-) mice exhibited increased mean total tissue mass, total fat mass and percent total body fat when compared with that of their gender-matched (+/+) littermates and the historical means.

These studies suggest that (+/-) non-human transgenic animals exhibit a negative phenotype that is associated with obesity. Thus, PRO36915 polypeptides or agonists thereof are essential for normal growth and metabolic processes and especially would be important in the prevention and/or treatment of obesity.

### (e) Phenotypic Analysis: Metabolism -Blood ChemistrylGlucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of wild type and heterozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

Blood Glucose Levels/Glucose Tolerance Test:
Oral Glucose Tolerance: The male (+/-) mice exhibited impaired glucose tolerance when compared with that of their gender-matched (+/+) littermates and the historical mean.

The (+/-) mice also exhibited an increased mean fasting serum glucose level.

These studies indicated that (+/-) mice exhibit a decreased or impaired glucose tolerance in the presence of normal fasting glucose at all 3 intervals tested when compared with their gender-matched (+/+) littermates and the historical means. Thus, heterozygous mice exhibited the phenotypic pattern of an impaired glucose homeostasis, and therefor PRO36915 polypeptides (or agonists thereof) or its encoding gene would be useful in the treatment of conditions associated with an impaired glucose homeostasis and/or various cardiovascular diseases, including diabetes.

### 43.27. Generation and Analysis of Mice Comprising DNA225566 (UNQ2424) Gene Disruptions

In these knockout experiments, the gene encoding PRO36029 polypeptides (designated as DNA225566) (UNQ2424) was disrupted. The gene specific information for these studies is as follows: the mutated mouse gene corresponds to nucleotide reference: NM_011303 ACCESSION:NM_011303 NID: gi 31560550 ref NM_011303.2 Mus musculus retinal short-chain dehydrogenase/reductase 1 (Rsdr1-pending); protein reference: 088876 ACCESSION:088876 NID: Mus musculus (Mouse). Retinal short-chain dehydrogenase/reductase RETSDR1; the human gene sequence reference: NM_004753 ACCESSION:NM_004753 NID: gi 31543614 ref NM_004753.2 Homo sapiens short-chain dehydrogenase/reductase 1 (SDR1); the human protein sequence corresponds to reference: 075911 ACCESSION:075911 NID: Homo sapiens (Human). Retinal short-chain dehydrogenase/reductase RETSDR1.

The gene of interest is mouse Dhrs3 (dehydrogenase/reductase (SDR family) member 3), ortholog of human DHRS3. Aliases include Rsdr1, retSDR1, SDR1, and retinal short-chain dehydrogenase/reductase 1.

DHRS3 is an enzyme that catalyzes the NADPH-dependent reduction of all-trans retinal to all-trans retinol. The enzyme contains a short chain dehydrogenase catalytic domain (Pfam accession PF00106) and a hydrophobic N-terminal segment that functions to directly or indirectly anchor the protein to intracellular membranes (Haeseleer et al, J Biol Chem 273:21790-9 (1998)). The enzyme is expressed primarily in cone photoreceptor outer segments but is also expressed in inner retinal neurons as well as other tissues. DHRS3 likely plays a role in the visual system by regenerating bleached visual pigments. DHRS3 may also play a more general role in regulating vitamin A metabolism and maintaining differentiation of tissues and organs. The DHRS3 is often deleted in neuroblastomas, and loss of DHRS3 may contribute to cancer development and progression (Haeseleer et al, J Biol Chem 273:21790-9 (1998); Cerignoli et al, Cancer Res 62:1196-204 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 51 | 82 | 9 | 142 |
| Expected | 35.5 | 71 | 35.5 | 142 |

Chi-Sq.= 28.25 Significance= 7.3382154E-7 (hom/n)= 0.06 Avg. Litter Size= 8
Mutation Information
Mutation Type: Homologous Recombination (standard)
Description: The gene consists of 6 exons, with the start codon located in exon 2 (NCBI accession BC008980). Exons 1 and 2 were targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.27.1. PHENOTYPIC ANALYSIS (for disrupted gene: DNA225566 (UNQ2424)

### (a) OVERALL PHENOTYPIC SUMMARY:

Mutation of the gene encoding the ortholog of human dehydrogenase/reductase (SDR family) member 3 (DHRS3) resulted in lethality of (-/-) mutants. Female (+/-) mice exhibited enhanced sensorimotor gating/attention. Gene disruption was confirmed by Southern blot.

### (b) Pathology

Microscopic: At 12.5 days, there were 44 embryos observed: 5 (-/-) embryos, 25 (+/-) embryos, 7 (+/+) embryos, 6 resorption moles, and 1 to-be-determined. The mutant (-/-) embryos showed abnormal histology.
Obvious: The (-/-) pups died shortly after birth.
Obvious Behavior: The dead (-/-) pups at birth showed open eyes.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type and heterozygous mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The female (+/-) mice exhibited an increased prepulse inhibition resulting in an enhanced sensorimotor gating/attention at pp4, pp8, and pp 12 when compared with that of their gender-matched (+/+) littermates and the historical means.

### 43.28. Generation and Analysis of Mice Comprising DNA96031-2664 (UNQ2438) Gene Disruptions

In these knockout experiments, the gene encoding PRO4999 polypeptides (designated as DNA96031-2664) (UNQ2438) was disrupted. The gene specific information for these studies is as follows: the mutated mouse gene corresponds to nucleotide reference: AK041075 Mus musculus adult male aorta and vein cDNA, RIKEN full-length enriched library, clone:A530080P10 product:similar to CDNA FLJ32142 FIS, CLONE PLACE5000068, WEAKLY SIMILAR TO C4B-BINDING PROTEIN PRECURSOR (C4BP) [Homo sapiens], full insert sequence; the human gene sequence reference: NM_022486 Homo sapiens sushi domain containing 1 (SUSD1); the human protein sequence corresponds to reference:Q6UWL2 ACCESSION:Q6UWL2 NID: Homo sapiens (Human). GRGP2438.

The gene of interest is mouse hypothetical protein A530080P 10, ortholog of human SUSD 1 (sushi domain containing 1). Aliases include RP11-401.1 and UNQ2438.

SUSD1 is a putative type I integral plasma membrane protein (Clark et al, Genome Res 13:2265-70 (2003)), consisting of 747 amino acids and containing a signal peptide, two or three epidermal growth factor (EGF) domains (Pfam accession PF07645), two sushi domains (Pfam accession PF00084), and a C-terminal transmembrane segment. The composition and organization of these domains suggest that SUSD1 functions as a cell adhesion or signaling molecule.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F 1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 19 | 33 | 20 | 72 |
| Expected | 18 | 36 | 18 | 72 |

Chi-Sq.= 0.25 Significance= 0.8824969 (hom/n)= 0.26 Avg. Litter Size= 8
Mutation Information
Mutation Type: Homologous Recombination (standard)
Description: The gene consists of 16 exons, with the start codon located in exon 1 (Ensembl accession ENSMUST00000075856). Exon 1 was targeted.
1. Wild-type Expression Panel: Expression of the target gene was detected in embryonic stem (ES) cells and in all 13 adult tissue samples tested by RT-PCR, except skeletal muscle and bone.
2. QC Expression: Disruption of the target gene was confirmed by Southern hybridization analysis.

### 43.28.1. PHENOTYPIC ANALYSIS (for disrupted gene: DNA96031-2664 (UN02438)

### (a) OVERALL PHENOTYPIC SUMMARY:

Mutation of the gene encoding the ortholog of human sushi domain containing 1 (SUSD1) resulted in decreased mean trabecular bone measurements in the (-/-) mice. Male and female (-/-) mice exhibited an increased heart rate. Some increase in peripheral blood eosinophils and blood lymphocytes was also observed in the (-/-) mice. Gene disruption was confirmed by Southern blot.

### (b) Expression in Human Diseased Tissues

UNQ2438 is overexpressed in pancreatic diseases and bone marrow diseases as shown by microarray data.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The mutant (-/-) mice exhibited some increase in peripheral blood eosinophils and blood lymphocytes but not tissue lymphocytes when compared with their (+/+) littermates and the historical means.

### (d) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

micro CT: The male (-/-) mice exhibited decreased mean vertebral trabecular bone measurements when compared with those of their gender-matched (+/+) littermates and the historical means.

The (-/-) mice analyzed by microCT exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO4999 polypeptides or agonists thereof would be useful for maintaining bone homeostasis in addition to normal growth development. In addition, PRO4999 polypeptides would be useful in bone healing or for the treatment of arthritis or osteoporosis, whereas antagonists (or inhibitors) of PRO4999 polypeptides or its encoding gene would lead to abnormal or pathological bone disorders including inflammatory diseases associated with abnormal bone metabolism including arthritis, osteoporosis and osteopenia.

### (e) Cardiology - Heart Rate

### Description:

Heart rate is measured via a noninvasive tail-cuff method for four days on the Visitech BP-2000 Blood Pressure Analysis System. Heart rate is measured ten times each day for four days. The four days are then averaged to obtain a mouse's conscious heart rate. Heart Rate: The female (-/-) mice exhibited an increased mean heart rate (∼1 SD) when compared with that of their gender-matched (+/+) littermates and the historical mean.

### 43.29. Generation and Analysis of Mice Comprising DNA96894-2675 (UNQ2491) Gene Disruptions

In these knockout experiments, the gene encoding PRO5778 polypeptides (designated as DNA96894-2675) (UNQ2491) was disrupted. The gene specific information for these studies is as follows: the mutated mouse gene corresponds to nucleotide reference: NM_019999 ACCESSION:NM_019999 NID: gi 9910441 ref NM_019999.1 Mus musculus brain protein 17 (Brp17); protein reference: Q9JJA3 ACCESSION:Q9JJA3 NID: Mus musculus (Mouse). Brain cDNA, clone MNCb-5687, similar to Homo sapiens KIAA1184 protein; the human gene sequence reference: NM_022572 ACCESSION:NM_022572 NID: gi 21703351 ref NM_022572.1 Homo sapiens likely ortholog of mouse brain protein 17 (BRP 17); the human protein sequence corresponds to reference: Q9BU26 ACCESSION:Q9BU26 NID: Homo sapiens (Human). Similar to hypothetical protein MNCb-5687.

The gene of interest is mouse Brp17 (brain protein 17), ortholog of human MR1 (myofibrillogenesis regulator 1). Aliases include MR-1, Tahccp2, MNCb-5687, 2210013N15Rik, 2810403H05Rik, FKSG19, KIAA1184, MGC31943, and DKFZp564N1362.

MR1 is a putative enzyme structurally similar to hydroxyacylglutathione hydrolase (HAGH), which catalyzes the hydrolysis of S-D-lactoyl-glutathione to form glutathione and D-lactic acid (Lee et al, Hum Mol Genet 13:3161-70 (2004)). The 385-amino acid protein contains a metallo-beta-lactamase superfamily domain (Pfam accession PF00753). MR1 is expressed at high levels in brain, fetal brain, skeletal muscle, and ovary and at lower levels in spleen, heart, testis, lung, liver, kidney, fetal liver, and pancreas (Hirosawa et al, DNA Res 6:329-36 (1999)).

Two other variant isoforms arise from the MR1 gene by alternative splicing. A second MR1 variant consists of 361 amino acids and contains a signal peptide and a transmembrane segment in addition to a metallo-beta-lactamase superfamily domain. This variant is likely to be an extracellular membrane protein (Clark et al, Genome Res 13:2265-70 (2003)). A third MR1 variant consists of 142 amino acids and contains a transmembrane segment but no other conserved domain. This variant appears to be associated with myofibrils of skeletal and cardiac muscle and is likely to play a role in myofibrillogenesis or in regulating muscle contraction (Li et al, Acta Biochim Biophys Sin (Shanghai) 36:412-8 (2004)).

The first two coding exons of the 385-amino acid MR1 variant and the 142-amino acid MR1 variant are shared, and mutations within the first codon is associated with paroxysmal dystonic choreoathetosis. This condition is characterized by spontaneously occurring involuntary movements that sometimes follow caffeine or alcohol consumption (Lee et al, Hum Mol Genet 13:3161-70 (2004); Ranier et al, Arch Neurol 61:1025-9 (2004); Chen et al, Arch Neurol 62:597-600 (2005)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 13 | 35 | 10 | 58 |
| Expected | 14.5 | 29 | 14.5 | 58 |

Chi-Sq.= 3.3 Significance= 0.19204992 (hom/n)= 0.2 Avg. Litter Size= 9
Mutation Information
Mutation Type: Homologous Recombination (standard)
Description: The gene consists of 9 exons, with the start codon located in exon 1 (NCBI accession NM_019999.1).
Exons 2 through 6 were targeted.
1. Wild-type Expression Panel: Expression of the target gene was detected in embryonic stem (ES) cells and in all 13 adult tissue samples tested by RT-PCR, except thymus and bone.
2. QC Expression: Disruption of the target gene was confirmed by Southern hybridization analysis.

### 43.29.1. PHENOTYPIC ANALYSIS (for disrupted gene: DNA96894-2675 (UNQ2491)

### (a) OVERALL PHENOTYPIC SUMMARY:

Mutation of the gene encoding the ortholog of human myofibrillogenesis regulator 1 (MR1) resulted in an increased serum IgM level in (-/-) mice. The mutant (-/-) mice also exhibited decreased total body fat mass and percent total body fat. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Serum Immunoglobulin Isotyping Assay:

The Serum Immunoglobulin Isotyping Assay is performed using a Cytometric BeadArray (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited an increased mean serum IgM level when compared with that of their (+/+) littermates and the historical mean.

Mutant (-/-) mice exhibited elevation of IgM serum immunoglobulins compared to their gender-matched (+/+) littermates. IgM immunoglobulins are the first to be produced in a humoral immune response for neutralization of bacterial toxins and are particularly important in activating the complement system. The observed phenotype suggests that the PRO5778 polypeptide is a negative regulator of inflammatory responses.
These immunological abnormalities suggest that inhibitors (antagonists) of PRO5778 polypeptides would be useful in stimulating the immune system (such as T cell proliferation) and would fmd utility in the cases wherein this effect would be beneficial to the individual such as in the case of leukemia, and other types of cancer, and in immuno-compromised patients, such as AIDS sufferers. Accordingly, PRO5778 polypeptides or agonists thereof would be useful in inhibiting the immune response and would be useful candidates for suppressing harmful immune responses, e.g. in the case of graft rejection or graft-versus-host diseases.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean total fat mass and percent total body fat when compared with that of their gender-matched (+/+) littermates and the historical means.

Mutant (-/-) mice deficient in the gene encoding PRO5778 polypeptides show a phenotype consistent with tissue wasting diseases (decreased total body fat (% and g)). Thus, antagonists or inhibitors of PRO5778 polypeptides or its encoding gene would mimic these metabolic and growth related effects. On the other hand, PRO5778 polypeptides or agonists thereof would be useful in the prevention and/or treatment of metabolic disorders related to abnormal fat metabolism or other tissue wasting diseases.

### 43.30. Generation and Analysis of Mice Comprising DNA97005-2687 (UNQ2509) Gene Disruptions

In these knockout experiments, the gene encoding PRO5997 polypeptides (designated as DNA97005-2687) (UNQ2509) was disrupted. The gene specific information for these studies is as follows: the mutated mouse gene corresponds to nucleotide reference: NM_027665 Mus musculus RIKEN cDNA 4933424D07 gene (4933424D07Rik); protein reference: Q811Q3 ACCESSION:Q811Q3 NID: Mus musculus (Mouse). ADAM30; the human gene sequence reference: NM_021794 Homo sapiens a disintegrin and metalloproteinase domain 30 (ADAM30); the human protein sequence corresponds to reference:Q9UKF2 AD30_HUMAN Q9UKF2 ADAM 30 PRECURSOR EC 3.4.24.- A DISI.

The gene of interest is mouse Adam30 (a disintegrin and metalloproteinase domain 30), ortholog of human ADAM30. Aliases include svph4 and 4933424D07Rik.

ADAM30 is an integral plasma membrane protein that likely functions as a cell adhesion molecule or protease. Like other ADAM family members (Primakoff and Myles, Trends Genet 16:83-7 (2000)), ADAM30 contains a signal peptide, a reprolysin family propeptide domain, a metalloproteinase-like domain, a disintegrin-like domain, a cysteine-rich domain, an EGF-like domain, a transmembrane segment, and a C-terminal cytoplasmic domain (Ceretti et al, Biochem Biophys Res Commun 263:810-5 (1999)). ADAM30 is expressed in both somatic and germ cells of testis (Choi et al, Genomics 83:636-46 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 14 | 32 | 20 | 66 |
| Expected | 16.5 | 33 | 16.5 | 66 |

Chi-Sq.= 0.26 Significance= 0.87809545 (hom/n)= 0.26 Avg. Litter Size= 8
Mutation Information
Mutation Type: Homologous Recombination (standard)
Description: The single exon (NCBI accession AK077058) was targeted.
1. Wild-type Expression Panel: Expression of the target gene was detected in embryonic stem (ES) cells and in all 13 adult tissue samples tested by RT-PCR, except liver.
2. QC Expression: Disruption of the target gene was confirmed by Southern hybridization analysis.

### 43.30.1. PHENOTYPIC ANALYSIS (for disrupted gene: DNA97005-2687 (UNQ2509)

### (a) OVERALL PHENOTYPIC SUMMARY:

Mutation of the gene encoding the ortholog of human a disintegrin and metalloproteinase domain 30 (ADAM30) resulted in impaired sensorimotor gating/attention in female (-/-) mice. Gene disruption was confirmed by Southern blot.

### (b) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The female (-/-) mice exhibited impaired sensorimotor gating/attention during pp4 and pp8 when compared with that of their gender-matched (+/+) littermates and the historical mean.

### 43.31. Generation and Analysis of Mice Comprising DNA111750-2706 (UNQ2538) Gene Disruptions

In these knockout experiments, the gene encoding PRO6079 polypeptides (designated as DNA111750-2706) (UNQ2538) was disrupted. The gene specific information for these studies is as follows: the mutated mouse gene corresponds to nucleotide reference: NM_026430 Mus musculus UDP-glucuronate decarboxylase 1 (Uxs1); protein reference: Q91XL3 ACCESSION:Q91XL3 NID: Mus musculus (Mouse). UDP-GLUCURONIC ACID DECARBOXYLASE; the human gene sequence reference: NM_025076 Homo sapiens UDP-glucuronate decarboxylase 1 (UXS1); the human protein sequence corresponds to reference: Q8NBZ7 ACCESSION:Q8NBZ7 NID: Homo sapiens (Human). Hypothetical protein FLJ90639 (UDP-glucuronic acid decarboxylase) (UDP-glucuronate decarboxylase 1) (UXS1).

The mouse gene of interest is Uxs1 (UDP-glucuronate decarboxylase 1), ortholog of human UXS1. Aliases include 1600025113Rik and FLJ23591.

UXS1 is an enzyme located in the perinuclear Golgi apparatus that functions as an NAD-requiring decarboxylase, catalyzing the formation ofUDP-xylose from UDP-glucuronate. USX1 is expressed primarily in kidney, liver, and brain (Moriarity et al, J Biol Chem 277:16968-75 (2002)). USX1 may play a role in morphogenesis and development by participating in xylosylation of proteoglycan core proteins (Moriarity et al, J Biol Chem 277:16968-75 (2002); Hwang and Horvitz, Proc Natl Acad Sci U S A 99:14218-23 (2002)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 37 | 0 | 58 |
| Expected | 14.5 | 29 | 14.5 | 58 |

Chi-Sq.= 47.29 Significance= 5.3840214E-11 (hom/n)= 0.0 Avg. Litter Size= 8
Mutation Information
Mutation Type: Homologous Recombination (standard)
Description: The gene consists of 15 exons, with the start codon located in exon 1 (NCBI accession NM_026430.1). Exon 1 was targeted.
1. Wild-type Expression Panel: Expression of the target gene was detected in embryonic stem (ES) cells and in all 13 adult tissue samples tested by RT-PCR, except bone and adipose.
2. QC Expression: Disruption of the target gene was confirmed by Southern hybridization analysis.

### 43.31.1. PHENOTYPIC ANALYSIS (for disrupted gene: DNA111750-2706 (UNQ2538)

### (a) OVERALL PHENOTYPIC SUMMARY:

Mutation of the gene encoding the ortholog of human UDP-glucuronate decarboxylase 1 (UXS 1) resulted in lethality of (-/-) mutants. The heterozygous (+/-) mice exhibited increased total tissue mass and lean body mass. The male (+/-) mice also exhibited an increased mean femoral mid-shaft cross-sectional area. Gene disruption was confirmed by Southern blot.

### (b) Pathology

Microscopic: Due to embryonic lethality, microscopic analysis was not performed. At day 12.5, there were 50 embryos observed: 22 (+/-) embryos, 14 (+/+) embryos, and 14 resorption moles.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type and 4 heterozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 4 heterozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: The male (+/-) mice exhibited increased mean total tissue mass when compared with that of their gender-matched (+/+) littermates and the historical means. The female (+/-) mice also showed an increased lean body mass when compared with their gender-matched littermates. micro CT: The male (+/-) mice exhibited increased mean femoral mid-shaft cross-sectional area when compared with that of their gender-matched (+/+) littermates and the historical mean.

In summary, the (+/-) mice exhibited increased mean total tissue mass and lean body mass and increased femoral bone mid-shaft cross-sectional area when compared with their gender-matched (+/+) littermates. These results indicate that the phenotype would be associated with obesity as well as such bone abnormalities as osteopetrosis. Osteopetrosis is a condition characterized by abnormal thickening and hardening of bone and abnormal fragility of the bones. As such, PRO6079 polypeptides or agonists thereof would be beneficial for the treatment of osteopetrosis or other osteo-related diseases. On the other hand, inhibitors or antagonists of PRO6079 polypeptides would be useful in bone healing.

### 43.32. Generation and Analysis of Mice Comprising DNA107781-2707 (UNQ2540) Gene Disruptions

In these knockout experiments, the gene encoding PRO6090 polypeptides (designated as DNA107781-2707) (UNQ2540) was disrupted. The gene specific information for these studies is as follows: the mutated mouse gene corresponds to nucleotide reference: NM_175020 Mus musculus hypothetical protein 1700020H15 (1700020H15); protein reference: Q8BW11 ACCESSION:Q8BW11 NID: Mus musculus (Mouse). Mus musculus adult male testis cDNA, RIKEN full-length enriched library, clone:1700020H15 product:hypothetical Serine proteases, trypsin family containing protein, full insert sequence (Hypothetical protein 1700020H15) (Trypsinogen 1); the human gene sequence reference: NM_001001317 Homo sapiens trypsin X3 (TRY1); the human protein sequence corresponds to reference: Q8IYP2 ACCESSION:Q8IYP2 NID: Homo sapiens (Human). Trypsin X3 (KFIL2540).

The mouse gene of interest is hypothetical protein 1700020H 15, ortholog of human TRY 1 (trypsin X3). Aliases include TRYX3, UNQ2540, FLJ16649, and MGC35022.

TRY1 is a putative secreted serine protease of the trypsin family (Clark et al, Genome Res 13:2265-70 (2003); Rowen et al. Science 272:1755-62 (1996)),containing a signal peptide and a trypsin-like serine protease domain (SMART accession SM00020).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 10 | 35 | 17 | 62 |
| Expected | 15.5 | 31 | 15.5 | 62 |

Chi-Sq.= 2.84 Significance= 0.24171403 (hom/n)= 0.23 Avg. Litter Size= 8
Mutation Information
Mutation Type: Homologous Recombination (standard)
Description: The gene consists of 6 exons, with the start codon located in exon 2 (NCBI accessionNM_175020.1).
Exons 3 through 6 were targeted.
1. Wild-type Expression Panel: Expression of the target gene was detected only in adipose among the 13 adult tissue samples tested by RT-PCR.
2. QC Expression: Disruption of the target gene was confirmed by Southern hybridization analysis.

### 43.32.1. PHENOTYPIC ANALYSIS (for disrupted gene: DNA107781-2707 (UNQ2540)

### (a) OVERALL PHENOTYPIC SUMMARY:

Mutation of the gene encoding the ortholog of human trypsin X3 (TRY1) resulted in immunological abnormalities in (-/-) mice. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased mean platelet count when compared with that of their (+/+) littermates and the historical mean.

Thus, mutant mice deficient in the DNA 107781-2707 gene resulted in a phenotype related to coagulation disorders. In this regard, inhibitors or antagonists of PRO6090 polypeptides would be useful in treating disorders related to abnormal blood coagulation such as hemophilia.

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel ofsix lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

Tissue-Specific FACS-Pooled Tissues: The (-/-) mice exhibited decreased percentages of B220Med/CD23- in peritoneal lavage and a corresponding increase in percent of CD23+ cells and a slight decrease in B220dim/CD43 dim cells when compared with those of their (+/+) littermates and the historical means. Thus, the mutant (-/-) mice exhibited an increased percentages of pre-B cells in the peritoneal lavage. PRO6090 polypeptides can therefore function as a negative regulator for B cell production or maturation.

### 43.33. Generation and Analysis of Mice Comprising DNA108789-2748 (UNQ2788) Gene Disruptions

In these knockout experiments, the gene encoding PRO7178 polypeptides (designated as DNA108789-2748) (UNQ2788) was disrupted. The gene specific information for these studies is as follows: the mutated mouse gene corresponds to nucleotide reference: NM_029025 ACCESSION:NM_029025 NID: gi 21312825 ref NM_029025.1 Mus musculus RIKEN cDNA 4930429020 gene (4930429O20Rik); protein reference: Q9D5K1 ACCESSION:Q9D5K1 NID: Mus musculus (Mouse). 4930429O20Rik protein (Hypothetical Microbodies C-terminal targeting signal containing protein); the human gene sequence reference: NM_203376 ACCESSION:NM_203376 NID: gi 42794617 ref NM_203376.1 Homo sapiens similar to RIKEN cDNA 4930429020 (LOC388730); the human protein sequence corresponds to reference: Q6P7N7 ACCESSION:Q6P7N7 NID: Homo sapiens (Human). Similar to RIKEN cDNA 4930429020 (Novel protein).

The gene of interest is mouse RIKEN cDNA 4930429020 gene, ortholog of human hypothetical protein LOC388730. Aliases include LOC388730, MGC75217, and 4930429O20Rik.

Hypothetical protein LOC388730 is a putative type I integral plasma membrane protein (Clark et al, Genome Res 13:2265-70 (2003)). The protein contains a signal peptide, an extracellular Ig-like domain (InterPro accession IPRO07110), a C-terminal transmembrane segment, and a short, intracellular C-terminal domain. Ig-like domains are usually involved in protein-protein interactions.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 20 | 28 | 18 | 66 |
| Expected | 16.5 | 33 | 16.5 | 66 |

Chi-Sq.= 0.26 Significance= 0.87809545 (hom/n)= 0.24 Avg. Litter Size= 8
Mutation Information
Mutation Type: Homologous Recombination (standard)
Description: The gene consists of 2 exons, with the start codon located in exon 2 (NCBI accession NM_029025.1).
Exon 2 was targeted.
1. Wild-type Expression Panel: Expression of the target gene was detected in brain, spinal cord, and eye among the 13 adult tissue samples tested by RT-PCR.
2. QC Expression: Disruption of the target gene was confirmed by Southern hybridization analysis.

### 43.33.1. PHENOTYPIC ANALYSIS (for disrupted gene: DNA108789-2748 (UNQ2788)

### (a) OVERALL PHENOTYPIC SUMMARY.

Mutation of the gene encoding the ortholog of a hypothetical human protein (LOC388730) resulted in an increased serum IgG1 level. However, the mutant (-/-) mice exhibited a decreased serum IgG2a response to ovalbumin challenge in (-/-) mice. The mutant (-/-) mice also exhibited decreased tissue mass and lean body mass measurements along with decreased bone mineral content and density measurements. The mutant (-/-) mice exhibited an increased heart rate. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the maj or histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited an increased mean serum IgG1 level when compared with that of their (+/+) littermates, the historical mean, and the (+/+) mice within the project.

Mutant (-/-) mice exhibited elevation of IgG 1 serum immunoglobulins compared to their gender-matched (+/+) littermates. These immunoglobulins have neutralization effects and to a lesser extent are important for activation of the complement system. The observed phenotype suggests that the PRO7178 polypeptide is a negative regulator of inflammatory responses. These immunological abnormalities suggest that inhibitors (antagonists) of PRO7178 polypeptides would be important agents which could stimulate the immune system and would find utility in the cases wherein this effect would be beneficial to the individual such as in the case of leukemia, and other types of cancer, and in immunocompromised patients, such as AIDS sufferers. Accordingly, PRO7178 polypeptides or agonists thereof would be useful in inhibiting the immune response and would be useful candidates for suppressing harmful immune responses, e.g. in the case of graft rejection or graft-versus-host diseases.

### Ovalbumin Challenge

Procedure: This assay was carried out on 7 wild types and 8 homozygotes. Chicken ovalbumin (OVA) is a T-cell dependent antigen, which is commonly used as a model protein for studying antigen-specific immune responses in mice. OVA is non-toxic and inert and therefore will not cause harm to the animals even ifno immune response is induced. The murine immune response to OVA has been well characterized, to the extent that the immunodominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited a decreased mean serum IgG2a response to ovalbumin challenge when compared with that of their (+/+) littermates and the historical mean.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The female (-/-) mice exhibited decreased mean total tissue mass, lean body mass, and bone mineral content, total body vBMD and total body bone mineral density when compared with those of their gender-matched (+/+) littermates and the historical means.
Fertility: The single male (-/-) mouse produced no pups following 2 matings.

The (-/-) mice analyzed by DEXA exhibited decreased bone measurements and decreased body mass measurements when compared with their (+/+) littermates, suggestive of abnormal bone disorders. The (-/-) mice exhibited a negative bone phenotype with abnormal decreased bone measurements reflective of bone metabolic disorders. In addition, the decreased mean total tissue mass and lean body mass is indicative of a metabolic disorder related to growth retardation and tissue wasting disorders. The negative bone and metabolic phenotype indicates that PRO7178 polypeptides or agonists thereof would be useful for maintaining bone homeostasis in addition to normal growth development. In addition, PRO7178 polypeptides would be useful in bone healing or for the treatment of arthritis or osteoporosis, whereas antagonists (or inhibitors) of PRO7178 polypeptides or its encoding gene would lead to abnormal or pathological bone disorders including inflammatory diseases associated with abnormal bone metabolism including arthritis, osteoporosis and osteopenia.

### (d) Cardiology - Heart Rate

### Description:

Heart rate is measured via a noninvasive tail-cuff method for four days on the Visitech BP-2000 Blood Pressure Analysis System. Heart rate is measured ten times each day for four days. The four days are then averaged to obtain a mouse's conscious heart rate.
Heart Rate: Male and female (-/-) mice exhibited an increased mean heart rate (∼1-2 SD) when compared with that of their gender-matched (+/+) littermates and the historical mean.

### 43.34. Generation and Analysis of Mice Comprising DNA167678-2963 (UNQ2945) Gene Disruptions

In these knockout experiments, the gene encoding PRO21184 polypeptides (designated as DNA167678-2963) (UNQ2945) was disrupted. The gene specific information for these studies is as follows: the mutated mouse gene corresponds to nucleotide reference: NM_178929 ACCESSION:NM_178929 NID: gi 31341808 ref NM_178929.2 Mus musculus expressed sequence AI842353 (AI842353); protein reference: Q8BJ66 ACCESSION:Q8BJ66 NID: Mus musculus (Mouse). Mus musculus 6 days neonate head cDNA, RIKEN full-length enriched library, clone:5430425E24 product:BA108L7.1 (NOVEL INSULIN-LIKE GROWTH FACTOR BINDING TYPE PROTEIN WITH KAZAL-TYPE SERINE PROTEASE INHIBITOR DOMAIN) homolog.; the human gene sequence reference: NM_030929 ACCESSION:NM_030929 NID: gi 34147508 ref NM_030929.3 Homo sapiens Kazal-type serine protease inhibitor domain 1 (KAZALD1); the human protein sequence corresponds to reference: Q96I82 ACCESSION:Q96I82 NID: Homo sapiens (Human). HYPOTHETICAL 32.9 KDA PROTEIN.

The mouse gene of interest is Kazald1 (Kazal-type serine protease inhibitor domain 1), ortholog of human KAZALD1. Aliases include Bono1, IGFBP-rP10, FKSG28, FKSG40, and bA108L7.1.

KAZALD1 is a putative secreted protein (Clark et al, Genome Res 13:2265-70 (2003)) expressed primarily in osteoblasts of developing and regenerating bone and in odontoblasts of teeth. The protein contains a signal peptide, an insulin-like growth factor-binding protein (IGFBP) domain, a Kazal-type serine protease inhibitor domain, and an immunoglobulin-like domain. KAZALD1 is capable of stimulating osteoblast proliferation and is likely to play a role in formation and regeneration of bone and teeth (James et al, Gene Expr Patterns 4:595-9 (2004); Shibata et al, Biochem Biophys Res Commun 325:1194-200 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvE^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 44 | 23 | 83 |
| Expected | 20.75 | 41.5 | 20.75 | 83 |

Chi-Sq.= 1.24 Significance= 0.53794444 (hom/n)= 0.26 Avg. Litter Size= 9
Mutation Information
Mutation Type: Homologous Recombination (standard)
Description: The gene consists of 5 exons, with the start codon located in exon 2 (NCBI accession NM_178929.2). Exons 1 through 5 were targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.34.1. PHENOTYPIC ANALYSIS (for disrupted gene: DNA167678-2963 (UNQ2945)

### (a) OVERALL PHENOTYPIC SUMMARY:

Mutation of the gene encoding the ortholog of human Kazal-type serine protease inhibitor domain 1 (KAZALD1) resulted in immunological abnormalities in (-/-) mice. The female (-/-) mice exhibited decreased bone mineral content and density measurements. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effects. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Serum Immunoglobulin Isotyping Assay:

The Serum Immunoglobulin Isotyping Assay is performed using a Cytometric Bead Array (CBA) kit. This assay is used to rapidly identify the heavy and light chain isotypes of a mouse monoclonal antibody in a single sample. The values expressed are "relative fluorescence units" and are based on the detection ofkappa light chains. Any value < 6 is not significant.

### Results:

Serum Imm. 2: The (-/-) mice exhibited a slight increase in mean serum IgG1 and IgG2a levels when compared with that of their (+/+) littermates and the historical mean.

Mutant (-/-) mice exhibited elevation of IgG1 and IgG2a serum immunoglobulins compared to their gender-matched (+/+) littermates. These immunoglobulins have neutralization effects and to a lesser extent are important for activation of the complement system. The observed phenotype suggests that the PRO21184 polypeptide is a negative regulator of inflammatory responses. These immunological abnormalities suggest that inhibitors (antagonists) of PRO21184 polypeptides would be important agents which could stimulate the immune system and would find utility in the cases wherein this effect would be beneficial to the individual such as in the case of leukemia, and other types of cancer, and in immunocompromised patients, such as AIDS sufferers. Accordingly, PRO21184 polypeptides or agonists thereof would be useful in inhibiting the immune response and would be useful candidates for suppressing harmful immune responses, e.g. in the case of graft rejection or graft-versus-host diseases.

### Ovalbumin Challenge

Procedure: This assay was carried out on 7 wild type and 8 homozygous mice. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immuno-dominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited a slight increase in mean serum IgG2a response and a decrease in IgG1 response to ovalbumin challenge when compared with that of their (+/+) littermates and the historical mean.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The female (-/-) mice exhibited decreased mean bone mineral content and bone mineral density in total body and vertebrae when compared with that of their gender-matched (+/+) littermates and the historical means.

The (-/-) mice analyzed by DEXA exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO21184 polypeptides or agonists thereof would be useful for maintaining bone homeostasis in addition to normal growth development. In addition, PRO21184 polypeptides would be useful in bone healing or for the treatment of arthritis or osteoporosis, whereas antagonists (or inhibitors) of PRO21184 polypeptides or its encoding gene would lead to abnormal or pathological bone disorders including inflammatory diseases associated with abnormal bone metabolism including arthritis, osteoporosis and osteopenia. Basal Body Temperature: The male (-/-) mice exhibited a decreased median basal body temperature when compared with that of their gender-matched (+/+) littermates and the historical mean.

### 43.35. Generation and Analysis of Mice Comprising DNA123430-2755 (UNQ2972) Gene Disruptions

In these knockout experiments, the gene encoding PRO7434 polypeptides (designated as DNA123430-2755) (UNQ2972) was disrupted. The gene specific information for these studies is as follows: the mutated mouse gene corresponds to nucleotide reference: AK005860 Mus musculus adult male testis cDNA, RIKEN full-length enriched library, clone:1700011E04 product:hypothetical Extracellular proteins SCP/Tpx-1/Ag5/PR-1/Sc7 containing protein, full insert sequence; protein reference: Q9DAG6 Q9DAG6 Q9DAG6 1700011E04RIK PROTEIN; the human gene sequence reference: NM_152779 Homo sapiens hypothetical protein MGC26856 (MGC26856); the human protein sequence corresponds to reference: Q96L06 ACCESSION:Q96L06 NID: Homo sapiens (Human). Similar to RIKEN cDNA 1700011E04 gene.

The gene of interest is mouse RIKEN cDNA 1700011 E04 gene, ortholog of human hypothetical protein MGC26856. Aliases include MGC26856, PRO7434, and ALKN2972.

Hypothetical protein MGC26856 is a putative secreted protein (Zhang and Henzel, Protein Sci 13:2819-24 (2004); Clark et al, Genome Res 13:2265-70 (2003)), containing a signal peptide and an SCP-like extracellular protein (SCP) domain. This domain is found in a variety of proteins from diverse species (Pfam accession PF00188), including Tex31, a protease present in venom of cone snails. Structural homology modeling predicts that the SCP domain contains the catalytic site (Milne et al, J Biol Chem 278:31105-10 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 42 | 17 | 70 |
| Expected | 17.5 | 35 | 17.5 | 70 |

Chi-Sq.= 7.11 Significance= 0.028581373 (hom/n)= 0.22 Avg. Litter Size= 9
Mutation Information
Mutation Type: Homologous Recombination (standard)
Description: The gene consists of 5 exons, with the start codon located in exon 1 (NCBI accession AK005860). Exons 3 through 5 were targeted.
1. Wild-type Expression Panel: Expression of the target gene was detected in brain, spinal cord, and adipose among the 13 adult tissue samples tested by RT-PCR
2. QC Expression: Disruption of the target gene was confirmed by Southern hybridization analysis.

### 43.35.1. PHENOTYPIC ANALYSIS (for disrupted gene: DNA123430-2755 (UNQ2972)

### (a) OVERALL PHENOTYPIC SUMMARY:

Mutation of the gene encoding the ortholog of human a human hypothetical protein (MGC26856) resulted in the female (-/-) mice exhibiting decreased bone mineral density measurements. Male (-/-) mice showed decreased mean body weight. The (-/-) mice exhibited immunological abnormalities marked by a decreased subset of T cells in the peritoneal lavage. Gene disruption was confirmed by Southern blot.

### (b) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight The male (-/-) mice exhibited decreased mean body weight when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The female (-/-) mice exhibited decreased total bone mineral density and vertebrae bone mineral density measurements when compared with those of their gender-matched (+/+) littermates and the historical means.

The (-/-) mice analyzed by DEXA exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO7434 polypeptides or agonists thereof would be useful for maintaining bone homeostasis in addition to normal growth development (Note: the mutant (-/-) mice also exhibited decreased body weight). In addition, PRO7434 polypeptides would be useful in bone healing or for the treatment of arthritis or osteoporosis, whereas antagonists (or inhibitors) of PRO7434 polypeptides or its encoding gene would lead to abnormal or pathological bone disorders including inflammatory diseases associated with abnormal bone metabolism including arthritis, osteoporosis and osteopenia.

### (c) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the maj or histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Cotigan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel of six lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

Tissue-Specific FACS-Pooled Tissues: The (-/-) mice exhibited a decreased percentage of CD11 bHi cells and an increased percentage of CD11bMed cells in peritoneal lavage when compared with those of their (+/+) littermates and the historical means. Lymph nodes show an increase in CD62hiCD44dim cells.

### 43.36. Generation and Analysis of Mice Comprising DNA108738-2767 (UNQ3024) Gene Disruptions

In these knockout experiments, the gene encoding PRO9822 polypeptides (designated as DNA108738-2767) (UNQ3024) was disrupted. The gene specific information for these studies is as follows: the mutated mouse gene corresponds to nucleotide reference: ENSMUST00000076164 cdna:novel chromosome:NCBIM33:1:54867954:54879384:-1 gene:ENSMUSG00000063329; protein reference: ENSMUSP00000075521 pep:novel chromosome:NCBIM33:1:54867954:54879384:-1 gene:ENSMUSG00000063329 transcript:ENSMUST00000076164; the human gene sequence reference: NM_024989 Homo sapiens GPI deacylase (PGAP1); the human protein sequence corresponds to reference:Q75T13 ACCESSION:Q75T13 NID: Homo sapiens (Human). GPI inositol-deacylase PGAP1.

The gene of interest is mouse predicted Ensembl gene ENSMUSG00000063329, ortholog of human PGAP1 (GPI deacylase). Aliases include ISPD3024.

PGAP 1 is a putative glycosylphosphatidylinositol (GPI) inositol deacylase, which catalyzes the hydrolysis of acyl groups from inositol of mature GPI in the endoplasmic reticulum. The 922-amino acid protein contains six transmembrane segments and an esterase/lipase/thioesterase conserved domain with an active site serine (InterPro accession IPRO00379). Deacylation of GPI is important for transport of GPI-anchored proteins from the endoplasmic reticulum to the Golgi apparatus (Tanaka et al, J Biol Chem 279:14256-63 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 35 | 10 | 56 |
| Expected | 14 | 28 | 14 | 56 |

Chi-Sq.= 4.03 Significance= 0.13332039 (hom/n)= 0.19 Avg. Litter Size= 8
Mutation Information
Mutation Type: Homologous Recombination (standard)
Description: The gene consists of 27 exons, with the start codon located in exon 1 (NCBI accession NM_201990.1 [rat]). Exons 21 through 23 were targeted.
1. Wild-type Expression Panel: Expression of the target gene was detected in embryonic stem (ES) cells and in all 13 adult tissue samples tested by RT-PCR, except adipose.
2. QC Expression: Disruption of the target gene was confirmed by Southern hybridization analysis.

### 43.36.1. PHENOTYPIC ANALYSIS (for disrupted gene: DNA108738-2767 (UNQ3024)

### (a) OVERALL PHENOTYPIC SUMMARY:

Mutation of the gene encoding the ortholog of human GPI deacylase (PGAP1) resulted in a proportion of UNQ3024 embryonic lethality. The surviving (-/-) mice exhibited decreased body weight and length, decreased total tissue mass and lean body mass with decreased body fat (mass and percent) as well as decreased bone mineral density measurements. The (-/-) mice exhibited abnormally high levels of alkaline phosphatase activity. In addition, the (-/-) mice showed numerous neurological abnormalities including an abnormal circadian rhythm and impaired sensorimotor gating/attention. Craniofacial abnormalities were noted in the (-/-) pups and embryos analyzed microscopically. LPS induces expression of UNQ3024 on dendritic cells. Gene disruption was confirmed by Southern blot.

### (b) Pathology

Microscopic: The (-/-) embryos and pups available for analysis exhibited multiple craniofacial anomalies, including the absence (complete atresia) of the nares, mouth, and ear canals. All of the affected mutants lacked a lower jaw, tongue, and associated structures. The eyes and other structures of the face were frequently hypoplastic and deformed in the mutants. No lesions were detected in the thoracic and abdominal organs.

### (c) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Obvious General Pics: The (-/-) mice were notably smaller than their (+/+) littermates, and 2 were born with no facial features. Urination: Urination was abnormal in the (-/-) mice compared with their (+/+) littermate controls.
Weight: The (-/-) mice exhibited notably decreased mean body weight when compared with that of their gender-matched (+/+) littermates and the historical means.
Length: The (-/-) mice exhibited decreased mean body length when compared with that of their gender-matched (+/+) littermates and the historical means. Fertility: The male (-/-) mouse available for analysis produced no pups after 40 days of breeding.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Both the male and female (-/-) mice exhibited decreased mean total tissue mass and lean body mass when compared with those of their gender-matched (+/+) littermates and the historical means. In addition, male (-/-) mice exhibited decreased mean total fat mass, percent total body fat and decreased vertebrae bone mineral density. The female (-/-) mice exhibited decreased mean bone mineral content and density measurements (except for total body vBMD).
micro CT: The male (-/-) mice exhibited decreased mean trabecular bone volume and thickness as well as decreased mean femoral mid-shaft cross-sectional area when compared with that of their gender-matched (+/+) littermates and the historical mean.

Mutant (-/-) mice deficient in the gene encoding PRO9822 polypeptides show a phenotype consistent with growth retardation, marked by small mutant mice with decreased body weight and length. Thus, antagonists or inhibitors of PRO9822 polypeptides or its encoding gene would mimic these metabolic and growth related effects. On the other hand, PRO9822 polypeptides or agonists thereof would be useful in the prevention and/or treatment of such metabolic disorders as diabetes or other tissue wasting diseases.

In addition, the (-/-) mice analyzed by DEXA and micro CT exhibited decreased bone measurements and decreased body mass measurements when compared with their (+/+) littermates, suggestive of abnormal bone disorders. The (-/-) mice exhibited a negative bone phenotype with abnormal decreased bone measurements reflective of bone metabolic disorders. In addition, the decreased mean total tissue mass, lean body mass and decreased total body fat is indicative of a metabolic disorder related to growth retardation and tissue wasting disorders. The negative bone and metabolic phenotype indicates that PRO9822 polypeptides or agonists thereof would be useful for maintaining bone homeostasis in addition to normal growth development. In addition, PRO9822 polypeptides would be useful in bone healing or for the treatment of arthritis or osteoporosis, whereas antagonists (or inhibitors) of PRO9822 polypeptides or its encoding gene would lead to abnormal or pathological bone disorders including inflammatory diseases associated with abnormal bone metabolism including arthritis, osteoporosis and osteopenia.

### (d) Phenotypic Analysis: Metabolism -Blood Chemistry

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In addition to measuring blood glucose levels the following blood chemistry tests are also routinely performed: Alkaline Phosphatase; Alanine Amino-Transferase; Albumin; Bilirubin; Phosphorous; Creatinine; BUN = Blood Urea Nitrogen; Calcium; Uric Acid; Sodium; Potassium; and Chloride. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

### Results:

Blood Chemistry: Both the male and female (-/-) mice exhibited a notably increased mean serum alkaline phosphatase levels when compared with that of their gender-matched (+/+) littermates and the historical means. This observation is consistent with the negative bone phenotype for these mutant mice.

### (e) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defmed as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types (70 dB background noise, 120 dB alone, 74dB + 120 dB - pp4, 78 dB + 120 dB - pp8, 82 dB + 120 dB - pp12, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The (-/-) mice exhibited impaired sensorimotor gating/attention over all prepulse intensities when compared with those of their gender-matched (+/+) littermates and the historical means.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The (-/-) mice exhibited an increased dark to light activity ratio resulting in an augmentation of the normal pattern shown by their gender-matched littermate controls.

The (-/-) mice exhibited increased ambulatory counts during home-cage activity testing resulting in a hyperactive behavior pattern when compared with their gender-matched (+/+) littermates and the historical means. These observations during home-cage activity testing is also suggestive of increased anxiety which is consistent with neurological disorders such as generalized anxiety disorders, attention deficit hyperactivity disorder, obsessive compulsive disorder, schizophrenia, cognitive disorders generalized anxiety disorder. Thus, antagonists or inhibitors of PRO9822 polypeptides or the PRO9822 encoding gene would be expected to mimic this behavior. Likewise, PRO9822 polypeptides or agonists thereof, would be useful in the treatment of such neurological disorders including generalized anxiety disorders, attention deficit hyperactivity disorder, obsessive compulsive disorder, schizophrenia, cognitive disorders generalized anxiety disorder.

### 43.37. Generation and Analysis of Mice Comprising DNA130809-2769 (UNQ3030) Gene Disruptions

In these knockout experiments, the gene encoding PRO9833 polypeptides (designated as DNA130809-2769) (UNQ3030) was disrupted. The gene specific information for these studies is as follows: the mutated mouse gene corresponds to nucleotide reference: NM_146069 Mus musculus RIKEN cDNA E430025L02 gene (E430025L02Rik); proteinreference: Q8BUI7 ACCESSION:Q8BUI7 NID: Mus musculus (Mouse). Hypothetical leucine-rich repeat/leucine-rich repeat; the human gene sequence reference: NM_198565 Homo sapiens ELLP3030 (UNQ3030); the human protein sequence corresponds to reference: Q86YC3 ACCESSION:Q86YC3 NID: Homo sapiens (Human). Similar to RIKEN cDNA E430025L02 gene.

The gene of interest is mouse Lrrc33 (leucine rich repeat containing 33), ortholog of human LRRC33. Aliases include MGC36838, E430025L02Rik, GARPL1, LTNQ3030, and MGC50789.

LRRC33 is a putative integral plasma membrane protein (Clark et al, Genome Res 13:2265-70 (2003)), containing a signal peptide, several leucine-rich repeats (Pfam accession PF00560), a transmembrane segment, and a short cytoplasmic tail. This domain organization is often found in proteins that function as cell adhesion molecules or signal-transducing receptors or ligands.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 21 | 34 | 20 | 75 |
| Expected | 18.75 | 37.5 | 18.75 | 75 |

Chi-Sq.= 3.78 Significance= 0.15107182 (hom/n)= 0.26 Avg. Litter Size= 10
Mutation Information
Mutation Type: Homologous Recombination (standard)
Description: The gene consists of 4 exons, with the start codon located in exon 2 (NCBI accession NM_146069.2). Exon 4 was targeted.
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 43.37.1. PHENOTYPIC ANALYSIS (for disrupted gene: DNA130809-2769 (UNQ3030)

### (a) OVERALL PHENOTYPIC SUMMARY:

Mutation of the gene encoding the ortholog of human leucine rich repeat containing 33 (LRRC33) resulted in swollen genitals in some (-/-) mice. The (-/-) mice exhibited increased locomotor activity and an abnormal circadian rhythm pattern in the (-/-) mice. The mutant (-/-) mice showed all the signs of growth retardation with decreased mean body weight and length, decreased body mass measurements and decreased fat mass and percent body fat. The (-/-) mice also exhibited an altered distribution of leukocyte subsets in peripheral blood (decreased T cells and increase B cells). Upon ovalbumin challenge, the (-/-) mice exhibited an increased IgG2a response. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following tests were performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample oflysed peripheral blood from each mouse with a panel ofsix lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in the peripheral blood, characterized by a decreased mean percentage of T cells and an increased mean percentage of B cells when compared with those of their (+/+) littermates and the historical means.

In summary, the FACS results indicate that the homozygous mutant mice demonstrate immunological abnormalities marked by a decreased mean percentages of T cells. In addition, the mutant (-/-) mice also exhibited an increased mean percentage of B cells. Thus, PRO9833 polypeptides or agonists thereof function as a negative regulator of B cell maturation and/or production and a positive regulator of T cells.

### Ovalbumin Challenge

Procedure: This assay was carried out on 7 wild type and 8 homozygous mice. Chicken ovalbumin (OVA) is a T-cell dependent antigen, which is commonly used as a model protein for studying antigen-specific immune responses in mice. OVA is non-toxic and inert and therefore will not cause harm to the animals even if no immune response is induced. The murine immune response to OVA has been well characterized, to the extent that the immune-dominant peptides for eliciting T cell responses have been identified. Anti-OVA antibodies are detectable 8 to 10 days after immunization using enzyme-linked immunosorbent assay (ELIZA), and determination of different isotypes of antibodies gives further information on the complex processes that may lead to a deficient response in genetically engineered mice.

As noted above, this protocol assesses the ability of mice to raise an antigen-specific immune response. Animals were injected IP with 50 mg of chicken ovalbumin emulsified in Complete Freund's Adjuvant and 14 days later the serum titer of anti-ovalbumin antibodies (IgM, IgG1 and IgG2 subclasses) was measured. The amount of OVA-specific antibody in the serum sample is proportional to the Optical Density (OD) value generated by an instrument that scans a 96-well sample plate. Data was collected for a set of serial dilutions of each serum sample.

### Results of this challenge:

Ovalbumin: The (-/-) mice exhibited an increased mean serum IgG2a response to ovalbumin challenge when compared with those of their (+/+) littermates and the historical mean.

In summary, the ovalbumin challenge studies indicate that knockout homozygous mice deficient in the gene encoding PRO9833 polypeptides exhibit immunological abnormalities when compared with their wild-type littermates. In particular, the mutant (-/-) mice exhibited an increased ability to elicit an immunological response when challenged with the T-cell dependent OVA antigen. Thus, antagonists (inhibitors) of PRO9833 polypeptides would be useful for stimulating the immune system (such as T cell proliferation) and would find utility in the cases
wherein this effect would be beneficial to the individual such as in the case of leukemia, and other types of cancer, and in immuno-compromised patients, such as AIDS sufferers. Accordingly, PRO9833 polypeptides or agonists thereof, would be useful for inhibiting the immune response and thus would be useful candidates for suppressing harmful immune responses, e.g. in the case of graft rejection or graft-versus-host diseases.

### (c) Phenotypic Analysis: CNS/Neurology

In the area ofneurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Open field test:

Several targets ofknown drugs have exhibited phenotypes in the open field test. These include knockouts of the seratonin transporter, the dopamine transporter (Giros et al., Nature. 1996 Feb 15;379(6566):606-12 ), and the GABA receptor (Homanics et al., Proc Natl Acad Sci U S A. 1997 Apr 15;94(8):4143-8). An automated open-field assay was customized to address changes related to affective state and exploratory patterns related to learning. First, the field (40 X 40 cm) was selected to be relatively large for a mouse, thus designed to pick up changes in locomotor activity associated with exploration. In addition, there were 4 holes in the floor to allow for nose-poking, an activity specifically related to exploration. Several factors were also designed to heighten the affective state associated with this test. The open-field test is the first experimental procedure in which the mice are tested, and the measurements that were taken were the subjects' first experience with the chamber. In addition, the open-field was brightly lit. All these factors will heighten the natural anxiety associated with novel and open spaces. The pattern and extent of exploratory activity, and especially the center-to-total distance traveled ratio, may then be able to discern changes related to susceptibility to anxiety or depression. A large arena (40 cm x 40 cm, VersaMax animal activity monitoring system from AccuScan Instruments) with infrared beams at three different levels was used to record rearing, hole poke, and locomotor activity. The animal was placed in the center and its activity was measured for 20 minutes. Data from this test was analyzed in five, 4-minute intervals. The total distance traveled (cm), vertical movement number (rearing), number of hole pokes, and the center to total distance ratio were recorded.

The propensity for mice to exhibit normal habituation responses to a novel environment is assessed by determining the overall change in their horizontal locomotor activity across the 5 time intervals. This calculated slope of the change in activity over time is determined using normalized, rather than absolute, total distance traveled. The slope is determined from the regression line through the normalized activity at each of the 5 time intervals. Normal habituation is represented by a negative slope value.

### Results:

Openfield2: The (-/-) mice exhibited increased median sum total distance traveled when compared with that of their gender-matched (+/+) littermates and the historical means, suggesting increased locomotor activity in the mutants.

The (-/-) mice demonstrated an increased anxiety-like response with increased locomotor activity. In summary, the open field testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO9833 polypeptides or agonists thereof would be useful in the treatment of such neurological disorders.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The (-/-) mice exhibited a significantly increased dark to light activity ratio resulting in an augmentation of the normal pattern shown by their gender-matched littermate controls.

The (-/-) mice exhibited increased ambulatory counts during home-cage activity testing resulting in a hyperactive behavior pattern when compared with their gender-matched (+/+) littermates and the historical means. These observations during home-cage activity testing is also suggestive of increased anxiety which is consistent with neurological disorders such as generalized anxiety disorders, attention deficit hyperactivity disorder, obsessive compulsive disorder, schizophrenia, cognitive disorders generalized anxiety disorder. Thus, antagonists or inhibitors of PRO9833 polypeptides or the PRO9833 encoding gene would be expected to mimic this behavior. Likewise, PRO9833 polypeptides or agonists thereof, would be useful in the treatment of such neurological disorders including generalized anxiety disorders, attention deficit hyperactivity disorder, obsessive compulsive disorder, schizophrenia, cognitive disorders generalized anxiety disorder.

*(d) Bone Metabolism & Body Diagnostics*

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Obvious General: Some of the male and female (-/-) mice exhibited swollen genitials with discharge on the surrounding area. The majority of these mutants were euthanized.
Weight: The (-/-) mice exhibited decreased mean body weight when compared with that of their gender-matched (+/+) littermates and the historical mean, the difference being more notable in the males.
Length: The (-/-) mice exhibited decreased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The male (-/-) mice exhibited decreased mean total tissue mass, lean body mass, total fat mass, and percent total body fat when compared with those of their gender-matched (+/+) littermates and the historical means. Also BMC/LBM is increased in the (-/-) mice.

Mutant (-/-) mice deficient in the gene encoding PRO9833 polypeptides show a phenotype consistent with growth retardation, marked by small mutant mice with decreased body weight and length. Thus, antagonists or inhibitors of PRO9833 polypeptides or its encoding gene would mimic these metabolic and growth related effects. On the other hand, PRO9833 polypeptides or agonists thereof would be useful in the prevention and/or treatment of such metabolic disorders as diabetes or other tissue wasting diseases.

In addition, the (-/-) mice analyzed by DEXA exhibited decreased body mass measurements when compared with their (+/+) littermates, suggestive of abnormal growth disorders. The decreased mean total tissue mass, lean body mass and decreased total body fat is indicative of a metabolic disorder related to growth retardation and tissue wasting disorders. The negative metabolic phenotype indicates that PRO9833 polypeptides or agonists thereof would be useful for maintaining normal growth development and for treatment of tissue wasting diseases. The BMD/LBM was also decreased in the (-/-) mutant mice which is indicative ofbone related disorders.

### 43.38. Generation and Analysis of Mice Comprising DNA119514-2772 (UNQ3034) Gene Disruptions

In these knockout experiments, the gene encoding PRO9836 polypeptides (designated as DNA119514-2772) (UNQ3034) was disrupted. The gene specific information for these studies is as follows: the mutated mouse gene corresponds to nucleotide reference: NM_146149 ACCESSION:NM_146149 NID: gi 22165387 ref NM_146149.1 Mus musculus hypothetical protein MGC37700 (MGC37700); protein reference: Q8QZW3 ACCESSION:Q8QZW3 NID: Mus musculus (Mouse). Similar to RIKEN cDNA 2010309H15 gene (H. sapiens) (Hypothetical 66.7 kDa protein); the human gene sequence reference: NM_176782 ACCESSION:NM_176782 NID: gi 28603817 ref NM_176782.1 Homo sapiens hypothetical protein MGC27169 (MGC27169); the human protein sequence corresponds to reference: Q8NAX9 ACCESSION:Q8NAX9 NID: Homo sapiens (Human). Hypothetical protein FLJ34582.

The mouse gene of interest is cDNA sequence BC026682, ortholog of human hypothetical protein MGC27169.

Hypothetical protein MGC27169 is a putative type II membrane protein, consisting of 585 amino acids and containing a signal anchor. The protein may project from the plasma membrane into the extracellular space (Clark et al, Genome Res 13:2265-70 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 24 | 29 | 13 | 66 |
| Expected | 16.5 | 33 | 16.5 | 66 |

Chi-Sq.= 5.42 Significance= 0.06653681 (hom/n)= 0.3 Avg. Litter Size= 9
Mutation Information
Mutation Type: Homologous Recombination (standard)
Description: The gene consists of 8 exons, with the start codon located in exon 1 (NCBI accession NM_146149.1).
Exons 1 and 2 were targeted.
1. Wild-type Expression Panel: Expression of the target gene was detected in embryonic stem (ES) cells and in all 13 adult tissue samples tested by RT-PCR, except spinal cord, skeletal muscle, bone, and adipose.
2. QC Expression: Disruption of the target gene was confirmed by Southern hybridization analysis.

### 43.38.1. PHENOTYPIC ANALYSIS (for disrupted gene: DNA119514-2772 (UNQ3034)

### (a) OVERALL PHENOTYPIC SUMMARY:

Mutation of the gene encoding the ortholog of a human hypothetical protein (MGC27169) resulted in impaired sensorimotor gating/attention in female (-/-) mice as well as decreased immobility during the tail suspension testing. Gene disruption was confirmed by Southern blot.

### (b) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Prepulse inhibition of the acoustic startle reflex

Prepulse inhibition of the acoustic startle reflex occurs when a loud 120 decibel (dB) startle-inducing tone is preceded by a softer (prepulse) tone. The PPI paradigm consists of six different trial types ( 70 dB background noise, 120 dB alone, 74dB + 120 dB - pp4, 78 dB + 120 dB - pp8, 82 dB + 120 dB - pp22, and 90 dB+ 120 dB - pp20) each repeated in pseudo random order six times for a total of 36 trials. The max response to the stimulus (V max) is averaged for each trial type. Animals with a 120 dB average value equal to or below 100 are excluded from analysis. The percent that the prepulse inhibits the animal's response to the startle stimulus is calculated and graphed.

### Results:

PPI: The female (-/-) mice exhibited impaired sensorimotor gating/attention at all prepulse intensities when compared with that of their gender-matched (+/+) littermates and the historical means.

### Functional Observational Battery (FOB) Test - Tail Suspension Testing:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Tail Suspension Testing:

The tail suspension test is a procedure that has been developed as a model for depressive-like behavior in rodents. In this particular setup, a mouse is suspended by its tail for 6 minutes, and in response the mouse will struggle to escape from this position. After a certain period of time the struggling of the mouse decreases and this is interpreted as a type of learned helplessness paradigm. Animals with invalid data (i.e. climbed their tail during the testing period) are excluded from analysis.

### Results:

Tail Suspension2: The (-/-) mice exhibited decreased median immobility time when compared with that of their (+/+) littermates and the historical mean, suggesting a decreased depressive-like response in the mutants.

In summary, the tail suspension testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO9836 polypeptides or agonists thereofwould be useful in the treatment of such neurological disorders.

### 43.39. Generation and Analysis of Mice Comprising DNA108771-2776 (UNQ3039) Gene Disruptions

In these knockout experiments, the gene encoding PRO9854 polypeptides (designated as DNA108771-2776) (UNQ3039) was disrupted. The gene specific information for these studies is as follows: the mutated mouse gene corresponds to nucleotide reference: NM_145533 ACCESSION:NM_145533 NID: gi 21704049 ref NM_145533.1 Mus musculus similar to chromosome 20 open reading frame 16 (LOC228608); protein reference: Q99K82 ACCESSION:Q99K82 NID: Mus musculus (Mouse). SIMILAR TO HYPOTHETICAL PROTEIN; the human gene sequence reference: M_019025 ACCESSION:NM_019025 NID: gi 9506692 ref NM_019025.1 Homo sapiens chromosome 20 open reading frame 16 (C20orf16); the human protein sequence corresponds to reference: Q9NWM0 ACCESSION:Q9NWM0 NID: Homo sapiens (Human). CDNA FLJ20746 FIS, CLONE HEP06040.

The gene of interest is mouse Smox (spermine oxidase), ortholog ofhuman SMOX. Aliases include PAO, SMO, PAOh1, MGC1010, C20orf16, FLJ20746, dJ779E11.1, and B130066H01Rik.

SMOX is an enzyme that catalyzes the last step in polyamine catabolism, the oxidation of spermine to form spermidine, 3-aminopropanal, and hydrogen peroxide. The enzyme requires flavin adenine dinucleotide (FAD) as a cofactor and molecular oxygen and water as cosubstrates (Cervelli et al, J Biol Chem 278:5271-6 (2003)). Several spermine analogs can inhibit SMOX activity or induce SMOX expression (Vujcic et al, Biochem J 367:665-75 (2002); Wang et al, Biochem Biophys Res Commun 304:605-11 (2003); Wang et al, Cancer Res 61:5370-3 (2001)). The SMOX gene gives rise to a major variant located primarily in the cytoplasm and a minor variant equally distributed between the nucleus and the cytoplasm (Cervelli et al, Eur J Biochem 271:760-70 (2004); Bianchi et al, FEBS J 272:3052-3059 (2005)). SMOX likely plays a role in processes such as normal and neoplastic cell proliferation, differentiation, and cell survival (Wang et al, Cancer Res 61:5370-3 (2001); Cervelli et al, J Biol Chem 278:5271-6 (2003); Wang et al ,Biochem Biophys Res Commun 304:605-11 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 17 | 34 | 23 | 74 |
| Expected | 18.5 | 37 | 18.5 | 74 |

Chi-Sq.= 2.32 Significance= 0.3134862 (hom/n)= 0.3 Avg. Litter Size= 7
Mutation Information
Mutation Type: Homologous Recombination (standard)
Description: The gene consists of 7 exons, with the start codon located in exon 2 (NCBI accession NM_145533.1). Exon 2 was targeted.
1. Wild-type Expression Panel: Expression of the target gene was detected in embryonic stem (ES) cells and in all 13 adult tissue samples tested by RT-PCR.
2. QC Expression: Disruption of the target gene was confirmed by Southern hybridization analysis.

### 43.39.1. PHENOTYPIC ANALYSIS (for disrupted gene: DNA108771-2776 (UNQ3039)

### (a) OVERALL PHENOTYPIC SUMMARY:

Mutation of the gene encoding the ortholog of human spermine oxidase (SMOX) resulted in skeletal muscle inflammation and necrosis. Gene disruption was confirmed by Southern blot.

### (b) Pathology

Microscopic: Among the 6 (-/-) mice available for analysis, 4 (M-123, F-132, M-138, M-175) exhibited minimal-to-moderate necrosis, inflammation, and/or regeneration of skeletal muscle. This type of lesion could be produced by trauma but is rare in this laboratory colony.

### (c) Expression in Human Diseased Tissues

UNQ3039 is overexpressed in the inflammatory disease of psoriasis (patient biopsy). In addition, LPS causes upregulation of this gene on monocytes. UNQ3039 is also overexpressed in patients with heart disease (biopsy).

### 43.40. Generation and Analysis of Mice Comprising DNA125148-2782 (UNQ3046) Gene Disruptions

In these knockout experiments, the gene encoding PRO9862 polypeptides (designated as DNA 125148-2782) (UNQ3046) was disrupted. The gene specific information for these studies is as follows: the mutated mouse gene corresponds to nucleotide reference: NM_027055 ACCESSION:NM_027055 NID: gi 21312277 ref NM_027055.1 Mus musculus RIKEN cDNA 1700008E09 gene (1700008E09Rik); protein reference: Q9DAK7 ACCESSION:Q9DAK7 NID: Mus musculus (Mouse). 1700008E09Rik protein; the human gene sequence reference: NM_133498 ACCESSION:NM_133498 NID: gi 33563295 ref NM_133498.2 Homo sapiens sperm acrosome associated 4 (SPACA4); the human protein sequence corresponds to reference: Q8TDM5 ACCESSION:Q8TDM5 NID: Homo sapiens (Human). Sperm acrosomal membrane protein 14.

The gene of interest is mouse RIKEN cDNA 1700008E09 gene, ortholog of human SPACA4 (sperm acrosome associated 4). Aliases include SAMP14 and UNQ3046.

SPACA4 is a putative glycosylphosphatidylinositol (GPI)-anchored plasma membrane protein expressed primarily in testis. The protein is a member of the Ly-6/urokinase-type plasminogen activator receptor (uPAR) superfamily of receptors, containing a signal peptide, an LY-6/uPAR domain, and a potential C-terminal GPI anchor signal sequence. SPACA4 is loosely associated with the plasma membrane of unwashed sperm, localizing primarily on the outer acrosomal membrane of washed sperm and on the inner acrosomal membrane of acrosome-reacted sperm. SPACA4 is likely involved in sperm-egg interaction (Shetty et al, J Biol Chem 278:30506-15 (2003)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 12 | 12 | 35 |
| Expected | 8.75 | 17.5 | 8.75 | 35 |

Chi-Sq.= 2.28 Significance= 0.31981903 (hom/n)= 0.26 Avg. Litter Size= 7
Mutation Information
Mutation Type: Homologous Recombination (standard)
Description: The single exon (NCBI accession NM_027055.1) was targeted.
1. Wild-type Expression Panel: Expression of the target gene was detected in embryonic stem (ES) cells and in all 13 adult tissue samples tested by RT-PCR, except bone and adipose.
2. QC Expression: Disruption of the target gene was confirmed by Southern hybridization analysis.

### 43.40.1. PHENOTYPIC ANALYSIS (for disrupted gene: DNA125148-2782 (UNQ3046)

### (a) OVERALL PHENOTYPIC SUMMARY:

Mutation of the gene encoding the ortholog of human sperm acrosome associated 4 (SPACA4) resulted in large (-/-) mice with increased mean body weight and length, increased total tissue mass, lean body mass and increased bone mineral density measurements. Gene disruption was confirmed by Southern blot.

### (b) Bone Metabolism & Body Diagnostics

### (1) Tissue Mass & Lean Body Mass Measurements - Dexa

### Dexa Analysis - Test Description:

Procedure: A cohort of wild type, heterozygous and homozygous mice were tested in this assay. Dual Energy X-ray Absorptiometry (DEXA) has been used successfully to identify changes in total tissue mass (TTM).

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI, i.e., whole body, vertebrae, and both femurs).

### Body Measurements (Body Length & Weight):

Body Measurements : A measurement of body length and weight was performed at approximately 16 weeks of age.

### Results:

Weight: The (-/-) mice exhibited increased mean body weight when compared with that of their gender-matched (+/+) littermates and the historical mean.
Length: The (-/-) mice exhibited increased mean body length when compared with that of their gender-matched (+/+) littermates and the historical mean.

### (2) Bone Metabolism: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of wild type, heterozygous and homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight ), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Bone microCT Analysis:

Procedure: MicroCT was also used to get very sensitive measurements of BMD. One vertebra and 1 femur were taken from a cohort of wild type and homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

DEXA: Both the male and female (-/-) mice exhibited increased mean total tissue mass and lean body mass when compared with those of their gender-matched (+/+) littermates and the historical means. The male (-/-) mice also exhibited increased total body bone mineral density and femurs bone mineral density measurements.
micro CT: The male (-/-) mice exhibited increased mean femoral mid-shaft cortical thickness when compared with those of their gender-matched (+/+) littermates.

In summary, the (-/-) mice exhibited increased mean total tissue mass and lean body mass as well as increased mean bone mineral content and density measurements when compared with their gender-matched (+/+) littermates. In addition, the mutant (-/-) mice showed signs of abnormal growth since they were very large in size with increased weight and length compared to their wildtype littermates suggestive of obesity. Increased bone mineral content and bone mineral density measurements are indicative with such bone abnormalities as osteopetrosis. Osteopetrosis is a condition characterized by abnormal thickening and hardening of bone and abnormal fragility of the bones. As such, PRO9862 polypeptides or agonists thereof would be beneficial for the treatment ofosteopetrosis or other osteo-related diseases as well as conditions associated with obesity or abnormal growth and development. On the other hand, inhibitors or antagonists of PRO9862 polypeptides would be useful in bone healing.

### 43.41. Generation and Analysis of Mice Comprising DNA138039-2828 (UNQ3127) Gene Disruptions

In these knockout experiments, the gene encoding PRO10284 polypeptides (designated as DNA138039-2828) (UNQ3127) was disrupted. The gene specific information for these studies is as follows: the mutated mouse gene corresponds to nucleotide reference: AK029497 Mus musculus adult male testis cDNA, RIKEN full-length enriched library, clone:4921506L 10 product:similar to CDNA FLJ32949 FIS, CLONE TESTI2008020, WEAKLY SIMILAR TO DPY-19 PROTEIN [Homo sapiens]; the human gene sequence reference: NM_173812 Homo sapiens hypothetical protein FLJ32949 (FLJ32949); the human protein sequence corresponds to reference: Q96LZ9 ACCESSION:Q96LZ9 NID: Homo sapiens (Human). Hypothetical protein FLJ32949.

The mouse gene of interest is RIKEN cDNA 4932443J21 gene, ortholog of human hypothetical protein FLJ32949. Aliases include 4932443J21Rik.

Hypothetical protein FLJ32949 is a putative integral plasma membrane protein (Clark et al, Genome Res 13:2265-70 (2003)), containing at least nine transmembrane segments. This protein is a homolog of C. elegans dpy-19, which plays a role in neuroblast polarization and migration during development (Honigberg and Kenyon, Development 127:4655-68 (2000)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 7 | 18 | 8 | 33 |
| Expected | 8.25 | 16.5 | 8.25 | 33 |

Chi-Sq.= 1.61 Significance= 0.4470879 (hom/n)= 0.2 Avg. Litter Size= 7
Mutation Information
Mutation Type: Homologous Recombination (standard)
Description: The gene consists of 22 exons, with the start codon located in exon 1 (NCBI accession AK029497). Exons 1 was targeted.
1. Wild-type Expression Panel: Expression of the target gene was detected only in brain and eye among the 13 adult tissue samples tested by RT-PCR.
2. QC Expression: Disruption of the target gene was confirmed by Southern hybridization analysis.

### 43.41.1. PHENOTYPIC ANALYSIS (for disrupted gene: DNA138039-2828 (UNQ3127)

### (a) OVERALL PHENOTYPIC SUMMARY:

Mutation of the gene encoding the ortholog of a human hypothetical protein (FLJ32949) resulted in testicular degeneration in male (-/-) mice with infertility. The mutant (-/-) mice also exhibited increased bone mineral content and density measurements which is related to osteopetrosis. GeneLogic data show expression of UNQ3127 in normal human heart tissues. Gene disruption was confirmed by Southern blot.

### (b) Pathology

Microscopic: The male (-/-) mice exhibited testicular degeneration, characterized by failure of the spermatid nuclei to elongate in the seminiferous tubules. The abnormal sperm was more apparent in the epididymides of the mutants consistent with infertility.

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

Fertility: The male (-/-) mouse available for analysis produced no pups after 40 days of breeding. DEXA: The (-/-) mice exhibited increased bone mineral content and density measurements when compared with that of their gender-matched (+/+) littermates and the historical means.
micro CT: The male (-/-) mice exhibited increased mean femoral mid-shaft cross-sectional area when compared with that of their gender-matched (+/+) littermates and the historical mean.

In summary, the (-/-) mice exhibited increased mean bone mineral content and bone mineral density n total body and femur, and increased microCT bone measurements when compared with their gender-matched (+/+) littermates. These results indicate that the knockout mutant phenotype may be associated with such bone abnormalities as osteopetrosis. Osteopetrosis is a condition characterized by abnormal thickening and hardening of bone and abnormal fragility of the bones. As such, PRO10284 polypeptides or agonists thereof would be beneficial for the treatment of obesity as well as bone disorders such as osteopetrosis or other osteo-related diseases. On the other hand, inhibitors or antagonists of PRO10284 polypeptides would be useful in bone healing.

### 43.42. Generation and Analysis of Mice Comprising DNA227047 (UNQ4430) Gene Disruptions

In these knockout experiments, the gene encoding PRO37510 polypeptides (designated as DNA227047) (UNQ4430) was disrupted. The gene specific information for these studies is as follows: the mutated mouse gene corresponds to nucleotide reference: NM_010923 ACCESSION:NM_010923 NID: gi 6754863 ref NM_010923.1 Mus musculus neuronatin (Nnat), transcript variant 1; protein reference: Q61979 ACCESSION:Q61979 NID: Mus musculus (Mouse). Neuronatin; the human gene sequence reference: NM_005386 ACCESSION:NM_005386 NID: gi 32307134 ref NM_005386.2 Homo sapiens neuronatin (NNAT), transcript variant 1; the human protein sequence corresponds to reference: Q16517 ACCESSION:Q16517 NID: Homo sapiens (Human). Neuronatin.

The gene of interest is mouse Nnat (neuronatin), ortholog of human NNAT. Aliases include Peg5, 5730414I02Rik, and MGC1439.

NNAT is a putative extracellular protein, containing a signal peptide (Bendtsen et al, J Mol Biol 340:783-95 (2004)) and a C-terminal arginine-rich basic region (Dou and Joseph, Brain Res 723:8-22 (1996)). The 81-amino acid protein is structurally similar to proteolipid family members PMP1 and phospholamban and has been proposed to function as a regulator of ion channels in neural tissue during brain development (Dou and Joseph, Brain Res 723:8-22 (1996)). NNAT is a paternally imprinted gene. It is expressed initially in the rhombomeres and pituitary gland and later in the central and peripheral nervous system (Kikyo et al, Dev Biol 190:66-77 (1997); Kagitani e al, Nucleic Acids Res 25:3428-32 (1997); Wijnholds et al, Dev Biol 171:73-84 (1995); John et al, Dev Biol 236:387-99 (2001); Evans et al, Genomics 77:99-104 (2001)). NNAT is also expressed in pancreatic beta cells and is likely to play a role in pancreatic beta cell function and glucose-mediated insulin secretion (Chu and Tsai, Diabetes 54:1064-73 (2005)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 23 | 40 | 16 | 79 |
| Expected | 19.75 | 39.5 | 19.75 | 79 |

Chi-Sq.= 2.21 Significance= 0.33121088 (hom/n)= 0.22 Avg. Litter Size= 9
Mutation Information
Mutation Type: Homologous Recombination (standard)
Description: The gene consists of 3 exons, with the start codon located in exon 1 (NCBI accession NM_010923.1). Exons 1 through 3 were targeted.
1. Wild-type Expression Panel: Expression of the target gene was detected in embryonic stem (ES) cells and in all 13 adult tissue samples tested by RT-PCR, except skeletal muscle, bone, and adipose.
2. QC Expression: Disruption of the target gene was confirmed by Southern hybridization analysis.

### 43.42.1. PHENOTYPIC ANALYSIS (for disrupted gene: DNA227047 (UNQ4430)

### (a) OVERALL PHENOTYPIC SUMMARY:

Mutation of the gene encoding the ortholog of human neuronatin (NNAT) resulted in the (-/-) mice exhibiting decreased mean vertebral trabecular bone volume, thickness and connectivity density. Gene disruption was confirmed by Southern blot.

### (b) Expression in Normal Human Tissues

UNQ4430 shows specific expression in CNS and pancreatic tissues as well as on B cell subtypes (GeneLogic data).

### (c) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
DEXA for measurement of bone mineral density on femur and vertebra
MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Bone microCT Analysis:

Procedure: MicroCT was also used to get very sensitive measurements of BMD. One vertebra and 1 femur were taken from a cohort of 4 wild type and 8 homozygous mice. Measurements were taken of lumbar 5 vertebra trabecular bone volume, trabecular thickness, connectivity density and midshaft femur total bone area and cortical thickness. The µCT40 scans provided detailed information on bone mass and architecture. Multiple bones were placed into sample holders and scanned automatically. Instrument software was used to select regions of interest for analysis. Trabecular bone parameters were analyzed in the fifth lumbar vertebrae (LV5) at 16 micrometer resolution and cortical bone parameters were analyzed in the femur midshaft at a resolution of 20 micrometers.

### Results:

micro CT: The male (-/-) mice exhibited decreased mean vertebral trabecular bone volume, number, thickness, and connectivity density when compared with those of their gender-matched (+/+) littermates and the historical means.

The (-/-) mice analyzed by microCT exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO37510 polypeptides or agonists thereof would be useful for maintaining bone homeostasis in addition to normal growth development. In addition, PRO37510 polypeptides would be useful in bone healing or for the treatment of arthritis or osteoporosis, whereas antagonists (or inhibitors) of PRO37510 polypeptides or its encoding gene would lead to abnormal or pathological bone disorders including inflammatory diseases associated with abnormal bone metabolism including arthritis, osteoporosis and osteopenia.

### (d) Cardiology/Diagnostics - Blood Pressure

### Description:

Systolic blood pressure is measured via a noninvasive tail-cuff method for four days on the Visitech BP-2000 Blood Pressure Analysis System. The blood pressure is measured ten times each day for four days. The four days are then averaged to obtain a mouse's conscious systolic blood pressure. Blood Pressure: The (-/-) mice exhibited slightly decreased mean systolic blood pressure when compared with that of their (+/+) littermates and the historical mean.

### 43.43. Generation and Analysis of Mice Comprising DNA222653 (UNQ6114) Gene Disruptions

In these knockout experiments, the gene encoding PRO35444 polypeptides (designated as DNA222653) (UNQ6114) was disrupted. The gene specific information for these studies is as follows: the mutated mouse gene corresponds to nucleotide reference: NM_007938 ACCESSION:NM_007938 NID: gi6679660 refNM_007938.1 Mus musculus Eph receptor A6 (Epha6); protein reference: Q62413 ACCESSION:Q62413 NID: Mus musculus (Mouse). EPHRIN TYPE-A RECEPTOR 6 PRECURSOR (EC 2.7.1.112) (TYROSINE-PROTEIN KINASE RECEPTOR EHK-2) (EPH HOMOLOGY KINASE-2); the human gene sequence reference: XM_114973 PREDICTED: Homo sapiens EphA6 (EPHA6); the human protein sequence corresponds to reference: XP_114973 PREDICTED: similar to receptor tyrosine kinase [Homo sapiens].

The gene ofinterest is mouse Epha6 (Eph receptor A6), ortholog of human EPHA6. Aliases include Ehk2, Hek12, m-ehk2, FLJ35246, and DKFZp434C1418.

EPHA6 is a type I integral plasma membrane protein that functions as a receptor protein tyrosine kinase. Glycosylphosphatidylinositol (GPI)-anchored ephrin-A ligands 1 through 5 likely activate EPHA6 and culminate in signaling responses that target the actin cytoskeleton (Wilkinson, Int Rev Cytol 196:177-244 (2000)). EPHA6 is expressed primarily in cochlear ganglion neurons of the inner ear and in neurons of discrete brain regions but is also expressed in other tissues, such as testes, ovary, thymus, and spleen (Lee et al, DNA Cell Biol 15:817-25 (1996); Maisonpierre et al, Oncogene 8:3277-88 (1993)). EPHA6 likely plays a role in establishing neuronal and vascular networks during development or remodeling (Yamaguchi and Pasquale, Curr Opin Neurobiol 14:288-96 (2004); Wilkinson, Int Rev Cytol 196:177-244 (2000); Nakamoto et al, Curr Biol 14:R121-3 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 17 | 34 | 21 | 72 |
| Expected | 18 | 36 | 18 | 72 |

Chi-Sq.= 0.63 Significance= 0.7297889 (hom/n)= 0.27 Avg. Litter Size= 8
Mutation Information
Mutation Type: Homologous Recombination (standard)
Description: The gene consists of 18 exons, with the start codon located in exon 1 (NCBI accession NM_007938.1). Exon 1 was targeted.
1. Wild-type Expression Panel: Expression of the target gene was detected in brain, spinal cord, eye, kidney, and heart among 13 adult tissue samples tested by RT-PCR.
2. QC Expression: Disruption of the target gene was confirmed by Southern hybridization analysis.

### 43.43.1. PHENOTYPIC ANALYSIS (for disrupted gene: DNA222653 (UNQ6114)

### (a) OVERALL PHENOTYPIC SUMMARY:

Mutation of the gene encoding the ortholog of human Eph receptor A6 (EPHA6) resulted in a decreased depressive-like response, decreased latency during hot plate testing, immunological abnormalities marked by an increased platelet count, impaired glucose tolerance, and increased serum triglyceride and cholesterol levels in the (-/-) mice. Female (-/-) mice also exhibited increased mean total tissue mass, total body fat, total fat mass and increased bone mineral content and density measurements. Gene disruption was confirmed by Southern blot.

### (b) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the maj or histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Hematology Analysis:

Test Description: Blood tests are carried out by Abbott's Cell-Dyn 3500R, an automated hematology analyzer. Some of its features include a five-part WBC differential. 'Patient' reports can cover over 22 parameters in all.

### Results:

Hematology: The (-/-) mice exhibited an increased median platelet count when compared with that of their (+/+) littermates and the historical mean.

Thus, mutant mice deficient in the DNA222653 gene encoding PRO35444 polypeptides resulted in a phenotype related to coagulation disorders. In this regard, inhibitors or antagonists of PRO35444 polypeptides would be useful in treating disorders related to abnormal blood coagulation such as hemophilia.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Functional Observational Battery (FOB) Test - Tail Suspension Testing:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Tail Suspension Testing:

The tail suspension test is a procedure that has been developed as a model for depressive-like behavior in rodents. In this particular setup, a mouse is suspended by its tail for 6 minutes, and in response the mouse will struggle to escape from this position. After a certain period of time the struggling of the mouse decreases and this is interpreted as a type of learned helplessness paradigm. Animals with invalid data (i.e. climbed their tail during the testing period) are excluded from.analysis.

### Results:

Tail Suspension2: The (-/-) mice exhibited decreased median immobility time when compared with that of their (+/+) littermates and the historical mean, suggesting a decreased depressive-like response in the mutants.

In summary, the tail suspension testing revealed a phenotype associated with increased anxiety which could be associated with mild to moderate anxiety, anxiety due to a general medical condition, and/or bipolar disorders; hyperactivity; sensory disorders; obsessive-compulsive disorders, schizophrenia or a paranoid personality. Thus, PRO35444 polypeptides or agonists thereof would be useful in the treatment of such neurological disorders.

### Hot Plate Testing

Test Description: The hot plate test for nociception is carried out by placing each mouse on a small enclosed 55°C hot plate. Latency to a hind limb response (lick, shake, or jump) is recorded, with a maximum time on the hot plate of 30 sec. Each animal is tested once.

### Results:

The mutant (-/-) mice exhibited a reduced latency to respond (for example an increased sensitivity-difference) when compared with their gender-matched (+/+) littermate controls. These results suggest an enhanced nociception response.

### (d) Phenotypic Analysis: Cardiology

In the area of cardiovascular biology, targets were identified herein for the treatment of hypertension, atherosclerosis, heart failure, stroke, various coronary artery diseases, dyslipidemias such as high cholesterol (hypercholesterolemia)and elevated serum triglycerides (hypertriglyceridemia), diabetes and/or obesity. The phenotypic tests included the measurement of serum cholesterol and triglycerides.

### Blood Lipids

Procedure: A cohort of 4 wild type, 4 heterozygous and 8 homozygous mice were tested in this assay. High cholesterol levels and increased triglyceride blood levels are recognized risk factors in the development of cardiovascular disease and/or diabetes. Measuring blood lipids facilitates the finding of biological switches that regulate blood lipid levels. Inhibition of factors which elevate blood lipid levels may be useful for reducing the risk for cardiovascular disease. In these blood chemistry tests, measurements were recorded using the COBAS Integra 400 (mfr: Roche).

### Results:

Blood Chemistry: Both the male and female (-/-) mice exhibited increased mean serum triglyceride and cholesterol levels when compared with those of their gender-matched (+/+) littermates and the historical means.

As summarized above, the (-/-) mice exhibited increased mean serum cholesterol and triglyceride levels when compared with their gender-matched (+/+) littermates and the historical means. Thus, mutant mice deficient in the PRO35444 gene can serve as a model for cardiovascular disease. PRO35444 polypeptides or its encoding gene would be useful in regulating blood lipids such as cholesterol and triglycerides. Thus, PRO35444 polypeptides or agonists thereof would be useful in the treatment of such cardiovascular diseases as hypertension, atherosclerosis, heart failure, stroke, various coronary diseases, hypercholesterolemia, diabetes and/or obesity.

### (e) Phenotypic Analysis: Metabolism -Blood Chemistry/Glucose Tolerance

In the area of metabolism, targets may be identified for the treatment of diabetes. Blood chemistry phenotypic analysis includes blood glucose measurements. The COBAS Integra 400 (mfr: Roche) was used for running blood chemistry tests on the mice. In the area of metabolism, targets may be identified for the treatment of diabetes. Blood chemistry phenotypic analysis includes glucose tolerance tests to measure insulin sensitivity and changes in glucose metabolism. Abnormal glucose tolerance test results may indicate but may not be limited to the following disorders or conditions: Diabetes Type 1 and Type 2, Syndrome X, various cardiovascular diseases and/or obesity.

Procedure: A cohort of wild type and homozygous mice were used in this assay. The glucose tolerance test is the standard for defining impaired glucose homeostasis in mammals. Glucose tolerance tests were performed using a Lifescan glucometer. Animals were injected IP at 2g/kg with D-glucose delivered as a 20% solution and blood glucose levels were measured at 0, 30, 60 and 90 minutes after injection.

### Results:

### Blood Glucose Levels/Glucose Tolerance Test:

Oral Glucose Tolerance: The male (-/-) mice exhibited an impaired glucose tolerance when compared with that of their gender-matched (+/+) littermates and the historical means.

These studies indicated that (-/-) mice exhibit a decreased or impaired glucose tolerance in the presence of normal fasting glucose at all 3 intervals tested when compared with their gender-matched (+/+) littermates and the historical means. Thus, knockout mutant mice exhibited the phenotypic pattern of an impaired glucose homeostasis, and therefor PRO35444 polypeptides (or agonists thereof) or its encoding gene would be useful in the treatment of conditions associated with an impaired glucose homeostasis and/or various cardiovascular diseases, including diabetes.

### (f) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [ i.e., whole body, vertebrae, and both femurs].

### Results:

DEXA: The female (-/-) mice exhibited increased mean total tissue mass, percent total body fat, and total fat mass when compared with those of their gender-matched (+/+) littermates and the historical means. Also bone mineral content (BMC), BMC/LBM and total body BMD was increased in the female (-/-) mice.

In summary, the (-/-) mice exhibited increased mean total tissue mass and total body fat (mass and percent total) when compared with their gender-matched (+/+) littermates suggestive of obesity. As such, PRO35444 polypeptides or agonists thereof would be beneficial for the treatment of obesity or abnormal fat metabolism (as evidenced by the observed increase in cholesterol and triglycerides in shown in (-/-)Blood Chemistry analysis).

The female (-/-) mice also exhibited increased bone mineral content and density These results indicate that the knockout mutant phenotype may be associated with such growth abnormalities as obesity as well as such bone abnormalities as osteopetrosis. Osteopetrosis is a condition characterized by abnormal thickening and hardening of bone and abnormal fragility of the bones. As such, PRO35444 polypeptides or agonists thereofwould be beneficial for the treatment of obesity as well as bone disorders such as osteopetrosis or other osteo-related diseases. On the other hand, inhibitors or antagonists of PRO35444 polypeptides would be useful in bone healing.

### 43.44. Generation and Analysis of Mice Comprising DNA163134-2917 (UNQ6268) Gene Disruptions

In these knockout experiments, the gene encoding PRO20473 polypeptides (designated as DNA163134-2917) (UNQ6268) was disrupted. The gene specific information for these studies is as follows: the mutated mouse gene corresponds to nucleotide reference: XM_283439 PREDICTED: Mus musculus triggering receptor expressed on myeloid cells-like 2 (Treml2); protein reference: XP_283439 PREDICTED: similar to triggering receptor expressed on myeloid cells-like 2 [Mus musculus]; the human gene sequence reference: NM_024807 Homo sapiens triggering receptor expressed on myeloid cells-like 2 (TREML2); the human protein sequence corresponds to reference: Q5T2D2 ACCESSION:Q5T2D2 NID: Homo sapiens (Human). Chromosome 6 open reading frame 76.

The gene of interest is mouse Treml2 (triggering receptor expressed on myeloid cells-like 2), ortholog of human TREML2. Aliases include Gm750, TLT2, C6orf76, FLJ13693, and dJ238O23.1.

TREML2 is a putative type I plasma membrane protein (Clark et al, Genome Res 13:2265-70 (2003)) that likely functions a signal-transducing receptor. The protein contains an extracellular immunoglobulin (IG)-like domain (InterPro accession IPRO07110), a transmembrane segment, and a short cytoplasmic C terminus. TREML2 is found clustered on human chromosome 6 with triggering receptor expressed on myeloid cells (TREM)-1, TREM2, and natural killer cell triggering receptor NKp44 (Allcock et al, Eur J Immunol 33:567-77 (2003)). These receptors are involved in regulating the function of various types of immune cells.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 49 | 17 | 82 |
| Expected | 20.5 | 41 | 20.5 | 82 |

Chi-Sq.= 0.42 Significance= 0.81058425 (hom/n)= 0.26 Avg. Litter Size= 9
Mutation Information
Mutation Type: Homologous Recombination (standard)
Description: The gene consists of 6 exons, with the start codon located in exon 1 (NCBI accession XM_283439.4).
Exon 1 was targeted.
1. Wild-type Expression Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

### 43.44.1. PHENOTYPIC ANALYSIS (for disrupted gene: DNA163134-2917 (UNQ6268)

### (a) OVERALL PHENOTYPIC SUMMARY:

Mutation of the gene encoding the ortholog of human triggering receptor expressed on myeloid cells-like 2 (TREML2) resulted in the mutant (-/-) mice exhibiting decreased bone mineral content and density measurements as well as several immunological abnormalities including decreased NK+ cells (natural killer cells), decreased CD4+ and CD8+ cells with increased B cells and increased monocytes. Gene disruption was confirmed by Southern blot.

### (b) Bone Metabolism & Body Diagnostics: Radiology Phenotypic Analysis

In the area of bone metabolism, targets were identified herein for the treatment of arthritis, osteoporosis, osteopenia and osteopetrosis as well as identifying targets that promote bone healing. Tests included:
· DEXA for measurement of bone mineral density on femur and vertebra
· MicroCT for very high resolution and very high sensitivity measurements of bone mineral density for both trabecular and cortical bone.

### Dexa Analysis - Test Description:

Procedure: A cohort of 4 wild type, 4 heterozygous and 8 homozygous mice were tested in this assay. Dual Energy X-ray Absorptiometry (DEXA) has been used successfully to identify changes in bone. Anesthetized animals were examined and bone mineral content (BMC), BMC/LBM ratios, volumetric bone mineral density (vBMD), total body BMD, femur BMD and vertebra BMD were measured.

The mouse was anesthetized by intraperitoneal injection of Avertin (1.25% 2,2,2,-tribromoethanol, 20 ml/kg body weight), body length and weight were measured, and then the mouse was placed in a prone position on the platform of the PIXImusTM Densitometer (Lunar Inc.) for a DEXA scan. Using Lunar PIXImus software, the bone mineral density (BMD) and fat composition (% fat) and total tissue mass (TTM) were determined in the regions of interest (ROI) [i.e., whole body, vertebrae, and both femurs].

### Results:

DEXA: The female (-/-) mice exhibited decreased mean bone mineral content and bone mineral density in total body, femurs, and vertebrae when compared with those of their gender-matched (+/+) littermates and the historical means.

The (-/-) mice analyzed by DEXA exhibited decreased bone measurements when compared with their (+/+) littermates, suggestive of abnormal bone disorders. The (-/-) mice exhibited a negative bone phenotype with abnormal decreased bone measurements reflective of bone metabolic disorders. The negative bone phenotype indicates that PRO20473 polypeptides or agonists thereof would be useful for maintaining bone homeostasis in addition to normal growth development. In addition, PRO20473 polypeptides would be useful in bone healing or for the treatment of arthritis or osteoporosis, whereas antagonists (or inhibitors) of PRO20473 polypeptides or its encoding gene would lead to abnormal or pathological bone disorders including inflammatory diseases associated with abnormal bone metabolism including arthritis, osteoporosis and osteopenia.

### (c) Cardiology/Diagnostics - Blood Pressure

### Description:

Systolic blood pressure is measured via a noninvasive tail-cuff method for four days on the Visitech BP-2000 Blood Pressure Analysis System. The blood pressure is measured ten times each day for four days. The four days are then averaged to obtain a mouse's conscious systolic blood pressure.

### Results:

Blood Pressure: The (-/-) mice exhibited slightly decreased systolic blood pressure when compared with that of their (+/+) littermates and the historical mean.

### (d) Immunology Phenotypic Analysis

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

T lymphocytes (T cells) are an important component of a mammalian immune response. T cells recognize antigens which are associated with a self-molecule encoded by genes within the major histocompatability complex (MHC). The antigen may be displayed together with MHC molecules on the surface of antigen presenting cells, virus infected cells, cancer cells, grafts, etc. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

In many immune responses, inflammatory cells infiltrate the site of injury or infection. The migrating cells may be neutrophilic, eosinophilic, monocytic or lymphocytic as can be determined by histological examination of the affected tissues. Current Protocols in Immunology, ed. John E. Coligan, 1994, John Wiley & Sons, Inc.

Many immune related diseases are known and have been extensively studied. Such diseases include immune-mediated inflammatory diseases (such as rheumatoid arthritis, immune mediated renal disease, hepatobiliary diseases, inflammatory bowel disease (IBD), psoriasis, and asthma), non-immune-mediated inflammatory diseases, infectious diseases, immunodeficiency diseases, neoplasia, and graft rejection, etc. In the area of immunology, targets were identified for the treatment of inflammation and inflammatory disorders.

In the area of immunology, targets have been identified herein for the treatment of inflammation and inflammatory disorders. Immune related diseases, in one instance, could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

The following test was performed:

### Flourescence-activated cell-sorting (FACS) Analysis

### Procedure:

FACS analysis of immune cell composition from peripheral blood was performed including CD4, CD8 and T cell receptor to evaluate T lymphocytes, CD 19 for B lymphocytes, CD45 as a leukocyte marker and pan NK for natural killer cells. The FACS analysis was carried out on 2 wild type and 6 homozygous mice and included cells derived from thymus, spleen, bone marrow and lymph node.

In these studies, analyzed cells were isolated from thymus, peripheral blood, spleen, bone marrow and lymph nodes. Flow cytometry was designed to determine the relative proportions of CD4 and CD8 positive T cells, B cells, NK cells and monocytes in the mononuclear cell population. A Becton-Dickinson FACS Calibur 3-laser FACS machine was used to assess immune status. For Phenotypic Assays and Screening, this machine records CD4+/CD8-, CD8+/CD4-, NK, B cell and monocyte numbers in addition to the CD4+/CD8+ ratio.

The mononuclear cell profile was derived by staining a single sample of lysed peripheral blood from each mouse with apanel ofsix lineage-specific antibodies: CD45 PerCP, anti-TCRb APC, CD4 PE, CD8 FITC, pan-NK PE, and CD19 FITC. The two FITC and PE labeled antibodies stain mutually exclusive cell types. The samples were analyzed using a Becton Dickinson FACS Calibur flow cytometer with CellQuest software.

### Results:

FACS: The (-/-) mice exhibited an altered distribution of leukocyte subsets in peripheral blood, characterized by decreased mean percentage of CD4 and CD8+ cells and an increased mean percentage of B cells when compared with those of their (+/+) littermates and the historical means.

These results show that knockout (-/-) mice exhibit immunological abnormalities compared to their wild-type (+/+) littermates. Antagonists (inhibitors) of PRO20473 polypeptides would be expected to mimic this phenotype. PRO20473 polypeptides or agonists thereof appear to act as a positive regulator of T cell production and a negative regulator of B cell development and would be useful in the development or maturation of T cells. In addition, the (-/-) mice showed decreased natural killer cells and increased monocytes.

Natural killer cells are the first line of defense to viral infection since these cells have been implicated in viral immunity and in defense against tumors. Natural killer cells or NK cells act as effectors in antibody-dependent cell-mediated cytotoxicity and have been identified by their ability to kill certain lymphoid tumor cell lines in *vitro* without the need for prior immunization or activation. However, their known function in host defense is in the early phases of infection with several intracellular pathogens, particularly herpes viruses. Thus, PRO20473 polypeptides and agonists thereofwould be important for a healthy immune system and would be useful in stimulating the immune system particularly during viral infections.

### 43.45. Generation and Analysis of Mice Comprising DNA143501-2922 (UNQ6349) Gene Disruptions

In these knockout experiments, the gene encoding PRO21054 polypeptides (designated as DNA143501-2922) (UNQ6349) was disrupted. The gene specific information for these studies is as follows: the mutated mouse gene corresponds to nucleotide reference: NM_026979 ACCESSION:NM_026979 NID: gi 58037152 ref NM_026979.1 Mus musculus C1q and tumor necrosis factor related protein 2 (C1qtnf2); protein reference: Q9D8U4 ACCESSION:Q9D8U4 NID: Mus musculus (Mouse). 1810033K05Rik protein (RIKEN cDNA 1810033K05 gene); the human gene sequence reference: NM_031908 ACCESSION:NM_031908 NID: gi 34147510 ref NM_031908.3 Homo sapiens C1q and tumor necrosis factor related protein 2 (C1QTNF2); the human protein sequence corresponds to reference: Q9BXJ5 ACCESSION:Q9BXJ5 NID: Homo sapiens (Human). Complement-c1q tumor necrosis factor-related protein 2 precursor.

The gene of interest is mouse C1qtnf2 (C1q and tumor necrosis factor related protein 2), ortholog of human C1QTNF2. Aliases include CTRP2, 1810033K05Rik, and zacrp2.

C1QTNF2 is a secreted protein and paralog of ADIPOQ (adiponectin, C1Q and collagen domain containing). Like ADIPOQ, the protein contains a signal peptide, a collagen triple helix repeat (Pfam accession PF01391), and a globular C1q domain (Pfam accession PF00386). C1QTNF2 expression is evident in a wide variety of tissues but not in serum, suggesting that C1QTNF2 functions as an autacoid rather than an endocrine hormone. C 1QTNF2 is capable of mimicking the actions of ADIPOQ, enhancing glycogen accumulation and fatty acid beta-oxidation by an AMP-activated protein kinase-dependent pathway in cultured C2C12 myotubes and in isolated muscle tissue. Thus, C 1QTNF2 likely plays a role in energy homeostasis (Wong et al, Proc Natl Acad Sci U S A 101:10302-7 (2004)).

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 16 | 27 | 20 | 63 |
| Expected | 15.75 | 31.5 | 15.75 | 63 |

Chi-Sq.= 5.44 Significance= 0.065874755 (hom/n)= 0.29 Avg. Litter Size= 9
Mutation Information
Mutation Type: Homologous Recombination (standard)
Description: The gene consists of 4 exons, with the start codon located in exon 2 (NCBI accession NM_026979.1).
Exon 3 was targeted.
1. Wild-type Expression Panel: Expression of the target gene was detected in all 13 adult tissue samples tested by RT-PCR, except bone and adipose.
2. QC Expression: Disruption of the target gene was confirmed by Southern hybridization analysis.

### 43.45.1. PHENOTYPIC ANALYSIS (for disrupted gene: DNA143501-2922 (UNQ6349)

### (a) OVERALL PHENOTYPIC SUMMARY:

Mutation of the gene encoding the ortholog of human C1q and tumor necrosis factor related protein 2 (C1QTNF2) resulted in hyperactivity during home-cage testing in the mutant (-/-) mice. In addition, the (-/-) mice exhibited a decreased stress-induced hyperthermia response. Gene disruption was confirmed by Southern blot.

### (b) Expression in Human Diseased Tissues

UNQ6349 is overexpressed in certain human diseased tissues such as osteoarthritis (bone dieases) and stomach tumors (GI stromal tumors) as shown by microarray analysis. In addition, UNQ6349 is expressed in Th1 blood samples.

### (c) Phenotypic Analysis: CNS/Neurology

In the area of neurology, analysis focused herein on identifying *in vivo* validated targets for the treatment of neurological and psychiatric disorders including depression, generalized anxiety disorders, attention deficit hyperactivity disorder, obsessive compulsive disorder, schizophrenia, cognitive disorders, hyperalgesia and sensory disorders. Neurological disorders include the category defined as "anxiety disorders" which include but are not limited to: mild to moderate anxiety, anxiety disorder due to a general medical condition, anxiety disorder not otherwise specified, generalized anxiety disorder, panic attack, panic disorder with agoraphobia, panic disorder without agoraphobia, posttraumatic stress disorder, social phobia, specific phobia, substance-induced anxiety disorder, acute alcohol withdrawal, obsessive compulsive disorder, agoraphobia, bipolar disorder I or II, bipolar disorder not otherwise specified, cyclothymic disorder, depressive disorder, major depressive disorder, mood disorder, substance-induced mood disorder. In addition, anxiety disorders may apply to personality disorders including but not limited to the following types: paranoid, antisocial, avoidant behavior, borderline personality disorders, dependent, histronic, narcissistic, obsessive-compulsive, schizoid, and schizotypal.

### Procedure:

Behavioral screens were performed on a cohort of wild type, heterozygous and homozygous mice. All behavioral tests were done between 12 and 16 weeks of age unless reduced viability necessitates earlier testing. These tests included open field to measure anxiety, activity levels and exploration.

### Circadian Test Description:

Female mice are individually housed at 4 pm on the first day of testing in 48.2 cm x 26.5 cm home cages and administered food and water ad libitum. Animals are exposed to a 12-hour light/dark cycle with lights turning on at 7 am and turning off at 7 pm. The system software records the number of beam interruptions caused by the animal's movements, with beam breaks automatically divided into ambulations. Activity is recorded in 60, one-hour intervals during the three-day test. Data generated are displayed by median activity levels recorded for each hour (circadian rhythm) and median total activity during each light/dark cycle (locomotor activity) over the three-day testing period.

### Results:

Circadian: The female (-/-) mice exhibited increased ambulatory counts during the 1-hour habituation phase when compared with that of their gender-matched (+/+) littermates and the historical mean.

The female (-/-) mice exhibited increased ambulatory counts during home-cage activity testing resulting in a hyperactive behavior pattern when compared with their gender-matched (+/+) littermates and the historical means. These observations during home-cage activity testing is also suggestive of increased anxiety which is consistent with neurological disorders such as generalized anxiety disorders, attention deficit hyperactivity disorder, obsessive compulsive disorder, schizophrenia, cognitive disorders generalized anxiety disorder. Thus, antagonists or inhibitors of PRO21054 polypeptides or the PRO21054 encoding gene would be expected to mimic this behavior. Likewise, PRO21054 polypeptides or agonists thereof, would be useful in the treatment of such neurological disorders including generalized anxiety disorders, attention deficit hyperactivity disorder, obsessive compulsive disorder, schizophrenia, cognitive disorders generalized anxiety disorder.

### Functional Observational Battery (FOB) Test - Stress-induced Hyperthermia:

The FOB is a series of situations applied to the animal to determine gross sensory and motor deficits. A subset of tests from the Irwin neurological screen that evaluates gross neurological function is used. In general, short-duration, tactile, olfactory, and visual stimuli are applied to the animal to determine their ability to detect and respond normally. These simple tests take approximately 10 minutes and the mouse is returned to its home cage at the end of testing.

### Results:

Anxiety: The male (-/-) mice exhibited a trend towards a decreased response to stress-induced hyperthermia when compared with their gender-matched (+/+) littermates and the historical mean.

### 43.46. Generation and Analysis of Mice Comprising DNA129618 (UNQ3010) Gene Disruptions

In these knockout experiments, the gene encoding PRO35246 polypeptides (designated as DNA129618) (UNQ3010) was disrupted. The gene specific information for these studies is as follows: the mutated mouse gene corresponds to nucleotide reference: NM_019576 ACCESSION:NM_019576 NID:9625040 Mus musculus Mus musculus transmembrane molecule with thrombospondin module (Tmtsp-pending); protein reference: Q9JM61 ACCESSION:Q9JM61 NID: Mus musculus (Mouse). TRANSMEMBRANE MOLECULE WITH THROMBOSPONDIN MODULE PRECURSOR; the human gene sequence reference: NM_018676 ACCESSION:NM_018676 NID: gi 40805850 ref NM_018676.2 Homo sapiens thrombospondin, type I, domain containing 1 (THSD1), transcript variant 1; the human protein sequence corresponds to reference:Q9NS62 ACCESSION:Q9NS62 NID: Homo sapiens (Human). TRANSMEMBRANE MOLECULE WITH THROMBOSPONDIN MODULE PRECURSOR.

Targeted or gene trap mutations are generated in strain 129SvEv^{Brd}-derived embryonic stem (ES) cells. The chimeric mice are bred to C57BL/6J albino mice to generate F1 heterozygous animals. These progeny are intercrossed to generate F2 wild type, heterozygous, and homozygous mutant progeny. On rare occasions, for example when very few F1 mice are obtained from the chimera, F1 heterozygous mice are crossed to 129SvEv^{Brd} /C57 hybrid mice to yield additional heterozygous animals for the intercross to generate the F2 mice. Level I phenotypic analysis is performed on mice from this generation

| | wt | het | hom | Total |
|---|---|---|---|---|
| Observed | 11 | 26 | 22 | 59 |
| Expected | 14.75 | 29.5 | 14.75 | 59 |

Chi-Sq.= 3.18 Significance= 0.20392561 (hom/n)= 0.26 Avg. Litter Size= 8
Mutation Information
Mutation Type: Homologous Recombination (standard)
Description: The gene consists of 4 exons, with the start codon located in exon 1 (NCBI accessionNM_019576.1).
Exon 1 was targeted.
1. Wild-type Expression Panel: WT Panel: Expression of the target gene was detected in embryonic stem (ES) cells and in all 13 adult tissue samples tested by RT-PCR, except bone.
2. QC Expression: Disruption of the target gene was confirmed by Southern hybridization analysis.

The gene of interest is mouse Thsd1 (thrombospondin, type I, domain 1), ortholog of human THSD1. Aliases include Tmtsp, 4833423O18Rik, type I, UNQ3010, MGC74971, and domain 1. THSD1 is a putative type I integral plasma membrane protein (Clark et al, Genome Res 13:2265-70 (2003)), containing a signal peptide, a thrombospondin type 1 repeat, a transmembrane segment, and a 400-amino acid cytoplasmic domain. Thrombospondin repeats are generally involved in protein-protein interactions (SMART accession SM00209).

### 43.46.1. PHENOTYPIC ANALYSIS (for disrupted gene: DNA129618 (UNQ3010)

### (a) OVERALL PHENOTYPIC SUMMARY:

Mutation of the gene encoding the ortholog of human thrombospondin, type I, domain 1 (THSD1) resulted in decreased brain weight in the (-/-) mouse. Gene disruption was confirmed by Southern blot.

### (b) Histology

Decreased brain weight was observed in a single homozygous (-/-) male mouse suggestive of developmental abnormalities in the (-/-) mouse.

### EXAMPLE 44: Use of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 as a hybridization probe

The following method describes use of a nucleotide sequence encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide as a hybridization probe.

DNA comprising the coding sequence of full-length or mature PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides as disclosed herein is employed as a probe to screen for homologous DNAs (such as those encoding naturally-occurringvariants of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides) in human tissue cDNA libraries or human tissue genomic libraries.

Hybridization and washing of filters containing either library DNAs is performed under the following high stringency conditions. Hybridization of radiolabeled PRO188-, PRO235-, PRO266-, PRO337-, PRO361-, PRO539-, PRO698-, PRO717-, PRO846-, PRO874-, PRO98346-, PRO1082-, PRO1097-, PRO1192-, PRO1268-, PRO1278-, PRO1303-, PRO1308-, PRO1338-, PRO1378-, PRO1415-, PRO1867-, PRO1890-, PRO3438-, PRO19835-, PRO36915-, PRO36029-, PRO4999-, PRO5778-, PRO5997-, PRO6079-, PRO6090-, PRO7178-, PRO21184-, PRO7434-, PRO9822-, PRO9833-, PRO9836-, PRO9854-, PRO9862-, PRO10284-, PRO37510-, PRO35444-, PRO20473-, PRO21054- or PRO35246-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides can then be identified using standard techniques known in the art.

### EXAMPLE 45: Expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 in E. coli

This example illustrates preparation of an unglycosylated form of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides by recombinant expression in *E. coli.*

The DNA sequence encoding a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 protein can then be purified using a metal chelating column under conditions that allow tight binding of the protein.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may be expressed in *E. coli* in a poly-His tagged form, using the following procedure. The DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) Ion galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3,0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

### EXAMPLE 46: Expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 in mammalian cells

This example illustrates preparation of a potentially glycosylated form of a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by recombinant expression in mammalian cells.

The vector, pRK5 (see EP 307,247, published March 15, 1989), is employed as the expression vector. Optionally, the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 DNA is ligated into pRK5 with selected restriction enzymes to allow insertion of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO3 5246 DNA using ligation methods such as described in Sambrook et al., supra. The resulting vector is called pRK5-PRO188, pRK5-PRO235, pRK5-PRO266, pRK5-PRO337, pRK5-PRO361, pRK5-PRO539, pRK5-PRO698, pRK5-PRO717, pRK5-PRO846, pRK5-PRO874, pRK5-PRO98346, pRK5-PRO1082, pRK5-PRO1097, pRK5-PRO1192, pRK5-PRO1268, pRK5-PRO1278, pRK5-PRO1303, pRK5-PRO1308, pRK5-PRO1338, pRK5-PRO1378, pRK5-PRO1415, pRK5-PRO1867, pRK5-PRO1890, pRK5-PRO3438, pRK5-PRO19835, pRK5-PRO36915, pRK5-PRO36029, pRK5-PRO4999, pRK5-PRO5778, pRK5-PRO5997, pRK5-PRO6079, pRK5-PRO6090, pRK5-PRO7178, pRK5-PRO21184, pRK5-PRO7434, pRK5-PRO9822, pRK5-PRO9833, pRK5-PRO9836, pRK5-PRO9854, pRK5-PRO9862, pRK5-PRO10284, pRK5-PRO37510, pRK5-PRO35444, pRK5-PRO20473, pRK5-PRO21054 or pRK5-PRO35246.

The selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO188, pRK5-PRO235, pRK5-PRO266, pRK5-PRO337, pRK5-PRO361, pRK5-PRO539, pRK5-PRO698, pRK5-PRO717, pRK5-PRO846, pRK5-PRO874, pRK5-PRO98346, pRK5-PRO1082, pRK5-PRO1097, pRK5-PRO1192, pRK5-PRO1268, pRK5-PRO1278, pRK5-PRO1303, pRK5-PRO1308, pRK5-PRO1338, pRK5-PRO1378, pRK5-PRO1415, pRK5-PRO1867, pRK5-PRO1890, pRK5-PRO3438, pRK5-PRO19835, pRK5-PRO36915, pRK5-PRO36029, pRK5-PRO4999, pRK5-PRO5778, pRK5-PRO5997, pRK5-PRO6079, pRK5-PRO6090, pRK5-PRO7178, pRK5-PRO21184, pRK5-PRO7434, pRK5-PRO9822, pRK5-PRO9833, pRK5-PRO9836, pRK5-PRO9854, pRK5-PRO9862, pRK5-PRO10284, pRK5-PRO37510, pRK5-PRO35444, pRK5-PRO20473, pRK5-PRO21054 or pRK5-PRO35246 DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides. The cultures containing transfected cells may undergo further incubation (in serum free medium) and the medium is tested in selected bioassays.

In an alternative technique, PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO 188, pRK5-PRO235, pRK5-PRO266, pRK5-PRO337, pRK5-PRO361, pRK5-PRO539, pRK5-PRO698, pRK5-PRO717, pRK5-PRO846, pRK5-PRO874, pRK5-PRO98346, pRK5-PRO1082, pRK5-PRO1097, pRK5-PRO1192, pRK5-PRO1268, pRK5-PRO1278, pRK5-PRO1303, pRK5-PRO1308, pRK5-PRO1338, pRK5-PRO1378, pRK5-PRO1415, pRK5-PRO1867, pRK5-PRO1890, pRK5-PRO3438, pRK5-PRO19835, pRK5-PRO36915, pRK5-PRO36029, pRK5-PRO4999, pRK5-PRO5778, pRK5-PRO5997, pRK5-PRO6079, pRK5-PRO6090, pRK5-PRO7178, pRK5-PRO21184, pRK5-PRO7434, pRK5-PRO9822, pRK5-PRO9833, pRK5-PRO9836, pRK5-PRO9854, pRK5-PRO9862, pRK5-PRO10284, pRK5-PRO37510, pRK5-PRO35444, pRK5-PRO20473, pRK5-PRO21054 or pRK5-PRO35246 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can then be concentrated and purified by any selected method, such as dialysis and/or column chromatography.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can be expressed in CHO cells. The pRK5-PRO188, pRK5-PRO235, pRK5-PRO266, pRK5-PRO337, pRK5-PRO361, pRK5-PRO539, pRK5-PRO698, pRK5-PRO717, pRK5-PRO846, pRK5-PRO874, pRK5-PRO98346, pRK5-PRO1082, pRK5-PRO1097, pRK5-PRO1192, pRK5-PRO1268, pRK5-PRO1278, pRK5-PRO1303, pRK5-PRO1308, pRK5-PRO1338, pRK5-PRO1378, pRK5-PRO1415, pRK5-PRO1867, pRK5-PRO1890, pRK5-PRO3438, pRK5-PRO19835, pRK5-PRO36915, pRK5-PRO36029, pRK5-PRO4999, pRK5-PRO5778, pRK5-PRO5997, pRK5-PRO6079, pRK5-PRO6090, pRK5-PRO7178, pRK5-PRO21184, pRK5-PRO7434, pRK5-PRO9822, pRK5-PRO9833, pRK5-PRO9836, pRK5-PRO9854, pRK5-PRO9862, pRK5-PRO10284, pRK5-PRO37510, pRK5-PRO35444, pRK5-PRO20473, pRK5-PRO21054 or pRK5-PRO35246 can be transfected into CHO cells using known reagents such as CaPO₄ or DEAE-dextran. As described above, the cell cultures can be incubated, and the medium replaced with culture medium (alone) or medium containing aradiolabel such as ³⁵S-methionine. After determining the presence of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the culture medium may be replaced with serum free medium. Preferably, the cultures are incubated for about 6 days, and then the conditioned medium is harvested. The medium containing the expressed PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can then be concentrated and purified by any selected method.

Epitope-tagged PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may also be expressed in host CHO cells. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may be subcloned out of the pRK5 vector. The subclone insert can undergo PCR to fuse in frame with a selected epitope tag such as a poly-his tag into a Baculovirus expression vector. The poly-his tagged PRO188, PRO235, PRO266, PRO3 37, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can then be concentrated and purified by any selected method, such as by Ni²⁺-chelate affinity chromatography.

PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG 1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Qiagen), Dosper^{®} or Fugene^{®} (Boehringer Mannheim). The cells are grown as described in Lucas et al., supra. Approximately 3 x 10⁷ cells are frozen in an ampule for further growth and production as described below.

The ampules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, the cell number pH ie determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

### EXAMPLE 47: Expression of PRO188, PRO235, PRO266, PRO337, PRO361, PRO539, PRO698, PRO717, PRO846, PRO874, PRO98346, PRO1082, PRO1097. PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 in Yeast

The following method describes recombinant expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 in yeast.

First, yeast expression vectors are constructed for intracellular production or secretion of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 from the ADH2/GAPDH promoter. DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 and the promoter is inserted into suitable restriction enzyme sites in the selected plasmid to direct intracellular expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246. For secretion, DNA encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO3 6029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can be cloned into the selected plasmid, together with DNA encoding the ADH2/GAPDH promoter, a native PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 signal peptide or other mammalian signal peptide, or, for example, a yeast alpha-factor or invertase secretory signal/leader sequence, and linker sequences (if needed) for expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can subsequently be isolated and purified by removing the yeast cells from the fermentation medium by centrifugation and then concentrating the medium using selected cartridge filters. The concentrate containing PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 may further be purified using selected column chromatography resins.

### EXAMPLE 48: Expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415. PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 in Baculovirus-Infected Insect Cells

The following method describes recombinant expression of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246in Baculovirus-infected insect cells.

The sequence coding for PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 or the desired portion of the coding sequence of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 are pooled and dialyzed against loading buffer.

Alternatively, purification of the IgG tagged (or Fc tagged) PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 can be performed using known chromatography techniques, including for instance, Protein A or protein G column chromatography.

### EXAMPLE 49: Tissue Expression Profiling Using GeneExpress®

A proprietary database containing gene expression information (GeneExpress®, Gene Logic Inc., Gaithersburg, MD) was analyzed in an attempt to identify polypeptides (and their encoding nucleic acids) whose expression is significantly upregulated in a particular tumor tissue(s) of interest as compared to other tumor(s) and/or normal tissues. Specifically, analysis of the GeneExpress® database was conducted using either software available through Gene Logic Inc., Gaithersburg, MD, for use with the GeneExpress® database or with proprietary software written and developed at Genentech, Inc. for use with the GeneExpress® database. The rating of positive hits in the analysis is based upon several criteria including, for example, tissue specificity, tumor specificity and expression level in normal essential and/or normal proliferating tissues. The following is a list of molecules whose tissue expression profile as determined from an analysis of the GeneExpress® database evidences high tissue expression and significant upregulation of expression in a specific tumor or tumors as compared to other tumor(s) and/or normal tissues and optionally relatively low expression in normal essential and/or normal proliferating tissues. Tissue expression profiling was performed on several UNQ genes the results of which are disclosed in Example 43.

### EXAMPLE 50: Microarray Analysis to Detect Upregulation of UNO Genes in Cancerous Tumors

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (disease tissue) sample is greater than hybridization signal of a probe from a control (normal tissue) sample, the gene or genes overexpressed in the disease tissue are identified. The implication of this result is that an overexpressed protein in a diseased tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In the present example, cancerous tumors derived from various human tissues were studied for upregulated gene expression relative to cancerous tumors from different tissue types and/or non-cancerous human tissues in an attempt to identify those polypeptides which are overexpressed in a particular cancerous tumor(s). In certain experiments, cancerous human tumor tissue and non-cancerous human tumor tissue of the same tissue type (often from the same patient) were obtained and analyzed for UNQ polypeptide expression. Additionally, cancerous human tumor tissue from any of a variety of different human tumors was obtained and compared to a "universal" epithelial control sample which was prepared by pooling non-cancerous human tissues of epithelial origin, including liver, kidney, and lung. mRNA isolated from the pooled tissues represents a mixture of expressed gene products from these different tissues. Microarray hybridization experiments using the pooled control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the pooled "universal control" sample not only allowed effective relative gene expression determinations in a simple 2-sample comparison, it also allowed multi-sample comparisons across several experiments.

In the present experiments, nucleic acid probes derived from the herein described UNQ polypeptide-encoding nucleic acid sequences were used in the creation of the microarray and RNA from various tumor tissues were used for the hybridization thereto. Below is shown the results of these experiments, demonstrating that various UNQ polypeptides of the present invention are significantly overexpressed in various human tumor tissues as compared to their normal counterpart tissue(s). Moreover, all of the molecules shown below are significantly overexpressed in their specific tumor tissue(s) as compared to in the "universal" epithelial control. As described above, these data demonstrate that the UNQ polypeptides of the present invention are useful not only as diagnostic markers for the presence of one or more cancerous tumors, but also serve as therapeutic targets for the treatment of those tumors. Microarray analysis was performed on several UNQ genes the results of which are disclosed in Example 43.

### EXAMPLE 51: Quantitative Analysis of UNQ mRNA Expression

In this assay, a 5' nuclease assay (for example, TaqMan®) and real-time quantitative PCR (for example, ABI Prizm 7700 Sequence Detection System® (Perkin Elmer, Applied Biosystems Division, Foster City, CA)), were used to fmd genes that are significantly overexpressed in a cancerous tumor or tumors as compared to other cancerous tumors or normal non-cancerous tissue. The 5' nuclease assay reaction is a fluorescent PCR-based technique which makes use of the 5' exonuclease activity of Taq DNA polymerase enzyme to monitor gene expression in real time. Two oligonucleotide primers (whose sequences are based upon the gene or EST sequence of interest) are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the PCR amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

The 5' nuclease procedure is run on a real-time quantitative PCR device such as the ABI Prism 7700TM Sequence Detection. The system consists of a thermocycler, laser, charge-coupled device (CCD) camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

The starting material for the screen was mRNA isolated from a variety of different cancerous tissues. The mRNA is quantitated precisely, e.g., fluorometrically. As a negative control, RNA was isolated from various normal tissues of the same tissue type as the cancerous tissues being tested.

5' nuclease assay data are initially expressed as Ct, or the threshold cycle. This is defined as the cycle at which the reporter signal accumulates above the background level of fluorescence. The ACt values are used as quantitative measurement of the relative number of starting copies of a particular target sequence in a nucleic acid sample when comparing cancer mRNA results to normal human mRNA results. As one Ct unit corresponds to 1 PCR cycle or approximately a 2-fold relative increase relative to normal, two units corresponds to a 4-fold relative increase, 3 units corresponds to an 8-fold relative increase and so on, one can quantitatively measure the relative fold increase in mRNA expression between two or more different tissues. Using this technique, the molecules have been identified as being significantly overexpressed in a particular tumor(s) as compared to their normal non-cancerous counterpart tissue(s) (from both the same and different tissue donors) and thus, represent excellent polypeptide targets for the diagnosis and therapy of cancer in mammals.

### EXAMPLE 52: In situ Hybridization

*In situ* hybridization is a powerful and versatile technique for the detection and localization ofnucleic acid sequences within cell or tissue preparations. It may be useful, for example, to identify sites of gene expression, analyze the tissue distribution of transcription, identify and localize viral infection, follow changes in specific mRNA synthesis and aid in chromosome mapping.

*In situ* hybridization was performed following an optimized version of the protocol by Lu and Gillett, Cell Vision 1:169-176 (1994), using PCR-generated ³³P-labeled riboprobes. Briefly, formalin-fixed, paraffin-embedded human tissues were sectioned, deparaffinized, deproteinated in proteinase K (20 g/ml) for 15 minutes at 37°C, and further processed for *in situ* hybridization as described by Lu and Gillett, *supra*. A [³³-P] UTP-labeled antisense riboprobe was generated from a PCR product and hybridized at 55°C overnight. The slides were dipped in Kodak NTB2 nuclear track emulsion and exposed for 4 weeks.

### ³³P-Riboprobe synthesis

6.0 µl (125 mCi) of ³³P-UTP (Amersham BF 1002, SA<2000 Ci/mmol) were speed vac dried. To each tube containing dried ³³P-UTP, the following ingredients were added:
2.0 µl 5x transcription buffer
1.0 µl DTT (100 mM)
2.0 µl NTP mix (2.5 mM : 10 µ; each of 10 mM GTP, CTP & ATP + 10 µl H₂O)
1.0 µl UTP (50 µM)
1.0 µl Rnasin
1.0 µl DNA template (1µg)
1.0 µl H₂O
1.0 µl RNA polymerase (for PCR products T3 = AS, T7 = S, usually)

The tubes were incubated at 37°C for one hour. 1.0 µl RQ1 DNase were added, followed by incubation at 37°C for 15 minutes. 90 µl TE (10 mM Tris pH 7.6/1mM EDTA pH 8.0) were added, and the mixture was pipetted onto DE81 paper. The remaining solution was loaded in a Microcon-50 ultrafiltration unit, and spun using program 10 (6 minutes). The filtration unit was inverted over a second tube and spun using program 2 (3 minutes). After the final recovery spin, 100 µl TE were added. 1 µl of the final product was pipetted on DE81 paper and counted in 6 ml of Biofluor II.

The probe was run on a TBE/urea gel. 1-3 µl of the probe or 5 µl of RNA Mrk III were added to 3 µl of loading buffer. After heating on a 95°C heat block for three minutes, the probe was immediately placed on ice. The wells of gel were flushed, the sample loaded, and run at 180-250 volts for 45 minutes. The gel was wrapped in saran wrap and exposed to XAR film with an intensifying screen in -70°C freezer one hour to overnight.

### ³³P-Hybridization

### A. Pretreatment of frozen sections

The slides were removed from the freezer, placed on aluminium trays and thawed at room temperature for 5 minutes. The trays were placed in 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNase-free RNAse buffer), the sections were washed in 0.5 x SSC for 10 minutes at room temperature. The sections were dehydrated in 70%, 95%, 100% ethanol, 2 minutes each.

### B. Pretreatment of Daraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37°C, 15 minutes) - human embryo, or 8 x proteinase K (100 pl in 250 ml Rnase buffer, 37°C, 30 minutes) - formalin tissues. Subsequent rinsing in 0.5 x SSC and dehydration were performed as described above.

### C. Prehybridization

The slides were laid out in a plastic box lined with Box buffer (4 x SSC, 50% formamide) - saturated filter paper.

### D. Hybridization

1.0 x 10⁶ cpm probe and 1.0 µl tRNA (50 mg/ml stock) per slide were heated at 95°C for 3 minutes. The slides were cooled on ice, and 48 µl hybridization buffer were added per slide. After vortexing, 50 µl ³³P mix were added to 50 µl prehybridization on slide. The slides were incubated overnight at 55°C.

### E. Washes

Washing was done 2 x 10 minutes with 2xSSC, EDTA at room temperature (400 ml 20 x SSC + 16 ml 0.25M EDTA, V_{f}=4L), followed by RNaseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55°C, 0.1 x SSC, EDTA (20 ml 20 x SSC + 16 ml EDTA, V_{f}=4L).

### F. Oligonucleotides

*In situ* analysis was performed on a variety of DNA sequences disclosed herein. The oligonucleotides employed for these analyses were obtained so as to be complementary to the nucleic acids (or the complements thereof) as shown in the accompanying figures.

### EXAMPLE 53: Preparation of Antibodies that Bind PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246

This example illustrates preparation of monoclonal antibodies which can specifically bind PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308. PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246.

Techniques for producing the monoclonal antibodies are known in the art and are described, for instance, in Goding, supra. Immunogens that may be employed include purified PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides, fusion proteins containing PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides, and cells expressing recombinant PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides on the cell surface. Selection of the immunogen can be made by the skilled artisan without undue experimentation.

Mice, such as Balb/c, are immunized with the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 antibodies.

After a suitable antibody titer has been detected, the animals "positive" for antibodies can be injected with a final intravenous injection of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246. Determination of "positive" hybridoma cells secreting the desired monoclonal antibodies against PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 is within the skill in the art.

The positive hybridoma cells can be injected intraperitoneally into syngeneic Balb/c mice to produce ascites containing the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 54: Purification of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 Polypeptides Using Specific Antibodies

Native or recombinant PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides may be purified by a variety of standard techniques in the art of protein purification. For example, pro-PRO188, pro-PRO235, pro-PRO266, pro-PRO337, pro-PRO361, pro-PRO539, pro-PRO698, pro-PRO717, pro-PRO846, pro-PRO874, pro-PRO98346, pro-PRO1082, pro-PRO1097, pro-PRO1192, pro-PRO1268, pro-PRO1278, pro-PRO1303, pro-PRO1308, pro-PRO1338, pro-PRO1378, pro-PRO1415, pro-PRO1867, pro-PRO1890, pro-PRO3438, pro-PRO19835, pro-PRO36915, pro-PRO36029, pro-PRO4999, pro-PRO5778, pro-PRO5997, pro-PRO6079, pro-PRO6090, pro-PRO7178, pro-PRO21184, pro-PRO7434, pro-PRO9822, pro-PRO9833, pro-PRO9836, pro-PRO9854, pro-PRO9862, pro-PRO10284, pro-PRO37510, pro-PRO35444, pro-PRO20473, pro-PRO21054 or pro-PRO35246polypeptide, mature PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, or pre-PRO188, pre-PRO235, pre-PRO266, pre-PRO337, pre-PRO361, pre-PRO539, pre-PRO698, pre-PRO717, pre-PRO846, pre-PRO874, pre-PRO98346, pre-PRO1082, pre-PRO1097, pre-PRO1192, pre-PRO1268, pre-PRO1278, pre-PRO1303, pre-PRO1308, pre-PRO1338, pre-PRO1378, pre-PRO1415, pre-PRO1867, pre-PRO1890, pre-PRO3438, pre-PRO19835, pre-PRO36915, pre-PRO36029, pre-PRO4999, pre-PRO5778, pre-PRO5997, pre-PRO6079, pre-PRO6090, pre-PRO7178, pre-PRO21184, pre-PRO7434, pre-PRO9822, pre-PRO9833, pre-PRO9836, pre-PRO9854, pre-PRO9862, pre-PRO10284, pre-PRO37510, pre-PRO35444, pre-PRO20473, pre-PRO21054 or pre-PRO35246 polypeptide is purified by immunoaffinity chromatography using antibodies specific for the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide of interest. In general, an immunoaffinity column is constructed by covalently coupling the anti-PRO188, anti-PRO235, anti-PRO266, anti-PRO337, anti-PRO361, anti-PRO539, anti-PRO698, anti-PRO717, anti-PRO846, anti-PRO874, anti-PRO98346, anti-PRO1082, anti-PRO1097, anti-PRO1192, anti-PRO1268, anti-PRO1278, anti-PRO1303, anti-PRO1308, anti-PRO1338, anti-PRO1378, anti-PRO1415, anti-PRO1867, anti-PRO1890, anti-PRO3438, anti-PRO19835, anti-PRO36915, anti-PRO36029, anti-PRO4999, anti-PRO5778, anti-PRO5997, anti-PRO6079, anti-PRO6090, anti-PRO7178, anti-PRO21184, anti-PRO7434, anti-PRO9822, anti-PRO9833, anti-PRO9836, anti-PRO9854, anti-PRO9862, anti-PRO10284, anti-PRO37510, anti-PRO35444, anti-PRO20473, anti-PRO21054 or anti-PRO35246 polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide by preparing a fraction from cells containing PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide in a soluble form. This preparation is derived by solubilization of the whole cell or of a subcellular fraction obtained via differential centrifugation by the addition of detergent or by other methods well known in the art. Alternatively, soluble polypeptide containing a signal sequence may be secreted in useful quantity into the medium in which the cells are grown.

A soluble PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-containing preparation is passed over the immunoaffinity column, and the column is washed under conditions that allow the preferential absorbance of PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide (e.g., high ionic strength buffers in the presence of detergent). Then, the column is eluted under conditions that disrupt antibody/PRO188, antibody/PRO235, antibody/PRO266, antibody/PRO337, antibody/PRO361, antibody/PRO539, antibody/PRO698, antibody/PRO717, antibody/PRO846, antibody/PRO874, antibody/PRO98346, antibody/PRO1082, antibody/PRO1097, antibody/PRO1192, antibody/PRO1268, antibody/PRO1278, antibody/PRO1303, antibody/PRO1308, antibody/PRO1338, antibody/PRO1378, antibody/PRO1415, antibody/PRO1867, antibody/PRO1890, antibody/PRO3438, antibody/PRO19835, antibody/PRO36915, antibody/PRO36029, antibody/PRO4999, antibody/PRO5778, antibody/PRO5997, antibody/PRO6079, antibody/PRO6090, antibody/PRO7178, antibody/PRO21184, antibody/PRO7434, antibody/PRO9822, antibody/PRO9833, antibody/PRO9836, antibody/PRO9854, antibody/PRO9862, antibody/PRO10284, antibody/PRO37510, antibody/PRO35444, antibody/PRO20473, antibody/PRO21054 or antibody/PRO35246 polypeptide binding (e.g., a low pH buffer such as approximately pH 2-3, or a high concentration of a chaotrope such as urea or thiocyanate ion), and PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is collected.

### EXAMPLE 55: Drug Screening

This invention is particularly useful for screening compounds by using PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptides or binding fragment thereof in any of a variety ofdrug screening techniques. The PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment. Drugs are screened against such transformed cells in competitive binding assays. Such cells, either in viable or fixed form, can be used for standard binding assays. One may measure, for example, the formation of complexes between PRO188, PRO235, PRO266, PRO3 3 7, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or a fragment and the agent being tested. Alternatively, one can examine the diminution in complex formation between the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and its target cell or target receptors caused by the agent being tested.

Thus, the present invention provides methods of screening for drugs or any other agents which can affect a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-associated disease or disorder. These methods comprise contacting such an agent with an PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment thereof and assaying (I) for the presence of a complex between the agent and the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment, or (ii) for the presence ofa complex between the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment and the cell, by methods well known in the art. In such competitive binding assays, the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment is typically labeled. After suitable incubation, free PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragment is separated from that present in bound form, and the amount of free or uncomplexed label is a measure of the ability of the particular agentto bind to PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or to interfere with the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, the peptide test compounds are reacted with PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide and washed. Bound PRO188, PRO235, PRO266, PRO3 3 7, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide is detected by methods well known in the art. Purified PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide specifically compete with a test compound for binding to PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or fragments thereof. In this manner, the antibodies can be used to detect the presence of any peptide which shares one or more antigenic determinants with PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide.

### EXAMPLE 56: Rational Drug Design

The goal of rational drug design is to produce structural analogs of biologically active polypeptide of interest (*i.e*., a PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide) or of small molecules with which they interact, e.g., agonists, antagonists, or inhibitors. Any of these examples can be used to fashion drugs which are more active or stable forms of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide or which enhance or interfere with the function of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide *in vivo* (*c*.*f*., Hodgson, Bio/Technology, 9: 19-21 (1991)).

In one approach, the three-dimensional structure of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide, orofa PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-inhibitor complex, is determined by x-ray crystallography, by computer modeling or, most typically, by a combination of the two approaches. Both the shape and charges of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide must be ascertained to elucidate the structure and to determine active site(s) of the molecule. Less often, useful information regarding the structure of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be gained by modeling based on the structure of homologous proteins. In both cases, relevant structural information is used to design analogous PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide may be made available to perform such analytical studies as X-ray crystallography. In addition, knowledge of the PRO188, PRO235, PRO266, PRO337, PRO361, PRO539, PRO698, PRO717, PRO846, PRO874, PRO98346, PRO1082, PRO1097, PRO1192, PRO1268, PRO1278, PRO1303, PRO1308, PRO1338, PRO1378, PRO1415, PRO1867, PRO1890, PRO3438, PRO19835, PRO36915, PRO36029, PRO4999, PRO5778, PRO5997, PRO6079, PRO6090, PRO7178, PRO21184, PRO7434, PRO9822, PRO9833, PRO9836, PRO9854, PRO9862, PRO10284, PRO37510, PRO35444, PRO20473, PRO21054 or PRO35246 polypeptide amino acid sequence provided herein will provide guidance to those employing computer modeling techniques in place of or in addition to x-ray crystallography.

## Claims

1. A method of identifying a phenotype associated with a disruption of a gene which encodes for a PRO9833 polypeptide, the method comprising:
(a) providing a non-human transgenic animal whose genome comprises a disruption of a gene which is an ortholog of a human gene that encodes for a PRO9833 polypeptide;
(b) measuring a physiological characteristic of the non-human transgenic animal; and
(c) comparing the measured physiological characteristic with that of a gender matched wild-type animal, wherein the physiological characteristic of the non-human transgenic animal that differs from the physiological characteristic of the wild-type animal is identified as a phenotype resulting from the gene disruption in the non-human transgenic animal.

2. The method of Claim 1, wherein the non-human transgenic animal is heterozygous for the disruption of a gene which encodes for a PRO9833 polypeptide.

3. A method of identifying an agent that modulates a phenotype associated with a disruption of a gene which encodes for a PRO9833 polypeptide, the method comprising:
(a) providing a non-human transgenic animal whose genome comprises a disruption of a gene which is an ortholog of a human gene that encodes for the PRO9833 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a phenotype resulting from the gene disruption in the non-human transgenic animal;
(d) administering a test agent to the non-human transgenic animal of (a); and
(e) determining whether the test agent modulates the identified phenotype associated with gene disruption in the non-human transgenic animal.

4. A method of identifying an agent that modulates a physiological characteristic associated with a disruption of a gene which encodes for a PRO9833 polypeptide, the method comprising:
(a) providing a non-human transgenic animal whose genome comprises a disruption of a gene which is an ortholog of a human gene that encodes for a PRO9833 polypeptide;
(b) measuring a physiological characteristic exhibited by the non-human transgenic animal of (a);
(c) comparing the measured physiological characteristic of (b) with that of a gender matched wild-type animal, wherein the physiological characteristic exhibited by the non-human transgenic animal that differs from the physiological characteristic exhibited by the wild-type animal is identified as a physiological characteristic associated with gene disruption;
(d) administering a test agent to the non-human transgenic animal of (a); and
(e) determining whether the physiological characteristic associated with gene disruption is modulated.

5. The method of Claim 1 or Claim 3, wherein the phenotype exhibited by the non-human transgenic animal as compared with gender matched wild-type littermates is at least one of the following: a neurological disorder; an immunological disorder; a bone metabolic abnormality or disorder; or a lipid metabolic disorder.

6. A method of identifying an agent that ameliorates or modulates a neurological disorder; an immunological disorder; a bone metabolic abnormality or disorder; or a lipid metabolic disorder associated with a disruption in a gene which encodes for a PRO9833 polypeptide, the method comprising:
(a) providing a non-human transgenic animal whose genome comprises a disruption of a gene which is an ortholog of a human gene that encodes for a PRO9833 polypeptide;
(b) administering a test agent to said non-human transgenic animal; and
(c) determining whether said test agent ameliorates or modulates the neurological disorder; immunological disorder; bone metabolic abnormality or disorder; or lipid metabolic disorder in the non-human transgenic animal.

7. The method of Claim 5 or Claim 6, wherein the neurological disorder is at least one of an increased anxiety-like response during open field activity testing; and an abnormal circadian rhythm during home-activity testing

8. The method of any one of Claims 1, 3, 4 and 6, wherein the non-human transgenic animal exhibits at least one of the following physiological characteristics compared with gender matched wild-type littermates: swollen genitals; increased anxiety-like response in open field testing; abnormal circadian rhythm during home-cage activity testing including increased ambulatory counts; decreased mean body weight; decreased mean body length; decreased total tissue mass; decreased lean body mass; decreased mean percent of total body fat and total fat mass; increased bone mineral density index (bone mineral content (BMC) / lean body mass(LBM)); decreased percentages of T cells and increased percentages of B cells; and increased mean serum IgG2a response to ovalbumin challenge.

9. A method of identifying an agent which modulates a behavior associated with a disruption of a gene which encodes for a PRO9833 polypeptide, the method comprising:
(a) providing a non-human transgenic animal whose genome comprises a disruption of a gene which is an ortholog of a human gene that encodes for a PRO9833 polypeptide;
(b) observing the behavior exhibited by the non-human transgenic animal of (a);
(c) comparing the observed behavior of (b) with that of a gender matched wild-type animal, wherein the observed behavior exhibited by the non-human transgenic animal that differs from the observed behavior exhibited by the wild-type animal is identified as a behavior associated with gene disruption;
(d) administering a test agent to the non-human transgenic animal of (a); and
(e) determining whether the agent modulates the behavior associated with gene disruption.

10. The method of Claim 9, wherein the behavior is an increased anxiety-like response in open field testing; or an abnormal circadian rhythm during home-cage activity testing including increased ambulatory counts.

11. A method of identifying an agent that modulates the expression of a PRO9833 polypeptide, the method comprising:
(a) contacting a test agent with a host cell expressing a PRO9833 polypeptide; and
(b) determining whether the test agent modulates the expression of the PRO9833 polypeptide by the host cell.

12. A method of evaluating a therapeutic agent capable of affecting a condition associated with a disruption of a gene which encodes for a PRO9833 polypeptide, the method comprising:
(a) providing a non-human transgenic animal whose genome comprises a disruption of a gene which is an ortholog of a human gene that encodes for the PRO9833 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the wild-type animal is identified as a condition resulting from the gene disruption in the non-human transgenic animal;
(d) administering a test agent to the non-human transgenic animal of (a); and
(e) evaluating the effects of the test agent on the identified condition associated with gene disruption in the non-human transgenic animal.

13. The method of Claim 12, wherein the condition is a neurological disorder; an immunological disorder; a bone metabolic abnormality or disorder; or a lipid metabolic disorder.

14. A method of identifying an agent that mimics a condition or phenotype associated with a disruption in a gene which encodes a PRO9833 polypeptide, the method comprising:
(a) providing a non-human transgenic animal whose genome comprises a disruption of a gene which is an ortholog of a human gene that encodes a PRO9833 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal; and
(e) determining whether said test agent mimics the condition or phenotype initially observed in the non-human transgenic animal.

15. A method of evaluating a therapeutic agent capable of mimicking a condition or phenotype associated with a disruption of a gene which encodes a PRO9833 polypeptide, the method comprising:
(a) providing a non-human transgenic animal whose genome comprises a disruption of a gene which is an ortholog of a human gene that encodes a PRO9833 polypeptide;
(b) measuring a physiological characteristic of the non-human transgenic animal of (a);
(c) comparing the measured physiological characteristic of (b) with that of a gender matched wild-type animal, wherein the physiological characteristic of the non-human transgenic animal that differs from the physiological characteristic of the gender matched wild-type animal is identified as a condition or phenotype resulting from the gene disruption in the non-human transgenic animal;
(d) administering a test agent to said gender matched wild-type animal of (c); and
(e) evaluating the ability of the test agent to mimic the condition or phenotype associated with gene disruption in the non-human transgenic animal.

16. An isolated cell derived from a non-human transgenic animal whose genome comprises a disruption of a gene which is an ortholog of a human gene that encodes for a PRO9833 polypeptide, preferably a murine cell, more preferably a murine cell embryonic stem cell.

17. The isolated cell of Claim 16, wherein the non-human transgenic animal exhibits at least one of the following phenotypes compared with gender matched wild-type littermates: a neurological disorder; or a bone metabolic abnormality or disorder.

18. An agent identified by the method of any one of Claims 3, 4, 6, 9, 11 and 14 or a therapeutic agent identified by the method of Claim 12 or Claim 15.

19. The agent or therapeutic agent of Claim 18 which is an agonist or antagonist of a PRO9833 polypeptide.

20. The agent or therapeutic agent of Claim 19, wherein the agonist or antagonist is an anti-PRO9833 antibody.

21. A therapeutic agent identified by the method of Claim 6.

22. A pharmaceutical composition comprising the therapeutic agent of Claim 18.

23. A method of
(i) treating or preventing or ameliorating a neurological disorder; or bone metabolic abnormality or disorder, the method comprising administering to a subject in need of such treatment whom may already have the disorder, or may be prone to have the disorder or may be in whom the disorder is to be prevented, a therapeutically effective amount of the therapeutic agent of Claim 21, or agonists or antagonists thereof, thereby effectively treating or preventing or ameliorating said disorder;
(ii) modulating a phenotype associated with a disruption of a gene which encodes for a PRO9833 polypeptide, the method comprising administering to a subject whom may already have the phenotype, or may be prone to have the phenotype or may be in whom the phenotype is to be prevented, an effective amount of the agent of Claim 18 as dependent from Claim 3, or agonists or antagonists thereof, thereby effectively modulating the phenotype;
(iii) modulating a physiological characteristic associated with a disruption of a gene which encodes for a PRO9833 polypeptide, the method comprising administering to a subject whom may already exhibit the physiological characteristic, or may be prone to exhibit the physiological characteristic or may be in whom the physiological characteristic is to be prevented, an effective amount of the agent of Claim 18 as dependent from Claim 4, or agonists or antagonists thereof, thereby effectively modulating the physiological characteristic;
(iv) modulating a behavior associated with a disruption of a gene which encodes for a PRO9833 polypeptide, the method comprising administering to a subject whom may already exhibit the behavior, or may be prone to exhibit the behavior or may be in whom the exhibited behavior is to be prevented, an effective amount of the agent of Claim 18 as dependent from Claim 9, or agonists or antagonists thereof, thereby effectively modulating the behavior;
(v) modulating the expression of a PRO9833 polypeptide, the method comprising administering to a host cell expressing said PRO9833 polypeptide, an effective amount of the agent of Claim 18 as dependent from Claim 11, or agonists or antagonists thereof, thereby effectively modulating the expression of said polypeptide;
(vi) modulating a condition associated with a disruption of a gene which encodes for a PRO9833 polypeptide, the method comprising administering to a subject whom may have the condition, or may be prone to have the condition or may be in whom the condition is to be prevented, a therapeutically effective amount of the therapeutic agent of Claim 18 as dependent from Claim 12, or agonists or antagonists thereof, thereby effectively modulating the condition;
(vii) mimicking a condition or phenotype associated with a disruption of a gene which encodes a PRO9833 polypeptide, the method comprising administering to a subject in whom the condition or phenotype is to be mimicked, an effective amount of the agent of Claim 18 as dependent from Claim 14 or an antagonist of a PRO9833 polypeptide, thereby effectively mimicking the condition or phenotype;
(viii) mimicking a condition or phenotype associated with a disruption of a gene which encodes a PRO9833 polypeptide, the method comprising administering to a subject in whom the condition or phenotype disorder is to be mimicked, a therapeutically effective amount of the therapeutic agent of Claim 18 as dependent from Claim 15, or an antagonist of a PRO9833 polypeptide, thereby effectively mimicking the condition or phenotype.

24. A method of identifying an agent that ameliorates or modulates a neurological disorder; an immunological disorder; a bone metabolic abnormality or disorder; or a lipid metabolic disorder associated with a disruption in the gene which encodes for a PRO9833 polypeptide, the method comprising administering to a subject who may have the neurological disorder; immunological disorder; bone metabolic abnormality or disorder; or lipid metabolic disorder a therapeutic agent of Claim 21, or agonists or antagonists thereof, thereby ameliorating or modulating the disorder.
